(19) Europäisches Patentamt
European Patent Office
Office européen des brevets

(11) EP 2 284 279 A1

(12) **EUROPEAN PATENT APPLICATION**

(43) Date of publication:
**16.02.2011 Bulletin 2011/07**

(51) Int Cl.:
***C12Q 1/68*** *(2006.01)*

(21) Application number: **10177065.9**

(22) Date of filing: **02.12.2005**

(84) Designated Contracting States:
**AT BE BG CH CY CZ DE DK EE ES FI FR GB GR HU IE IS IT LI LT LU LV MC NL PL PT RO SE SI SK TR**

(30) Priority: **02.12.2004 US 632426 P**
**02.12.2004 US 633250 P**
**14.03.2005 US 662220 P**
**03.10.2005 US 723125 P**
**03.10.2005 US 723054 P**
**30.11.2005 US 740736 P**

(62) Document number(s) of the earlier application(s) in accordance with Art. 76 EPC:
**09010002.5 / 2 213 749**
**05853011.4 / 1 831 399**

(27) Previously filed application:
**02.12.2005 EP 09010002**

(71) Applicant: **Epigenomics AG**
**10178 Berlin (DE)**

(72) Inventors:
- **Cottrell, Susan**
  **Seattle, WA 98115 (US)**
- **Model, Fabian**
  **12683, Berlin (DE)**
- **Haefliger, Carolina**
  **4052, Basel (CH)**
- **Weiss, Gunter**
  **10437, Berlin (DE)**
- **Distler, Jürgen**
  **12163, Berlin (DE)**
- **Sledziewski, Andrew Z.**
  **Shoreline, WA 98177 (US)**
- **Song, Xiaoling**
  **Woodinville, WA 98072 (US)**
- **Skillman, Thomas L.**
  **Kennydale, WA 98056 (US)**
- **Thomas, Jeffrey G.**
  **San Francisco, CA 94158 (US)**

Remarks:
•The complete document including Reference Tables and the Sequence Listing can be downloaded from the EPO website
•This application was filed on 16-09-2010 as a divisional application to the application mentioned under INID code 62.

(54) **Methods and nucleic acids for the analysis of gene expression associated with the prognosis of prostate cell proliferative disorders**

(57) Particular aspects provide novel methods and compositions (e.g., nucleic acids, kits, etc.) having substantial utility for providing a prognosis of prostate cell proliferative disorders. In particular aspects, this is achieved by the analysis of the expression status of a panel of genes, or subsets thereof.

EP 2 284 279 A1

## Description

FIELD OF THE INVENTION

Aspects of the present invention relate to human DNA sequences that exhibit heterogenous expression patterns in prostate cancer patients, and more particularly to novel compositions and methods for providing a prognosis of said patients.

CROSS-REFERENCE TO RELATED APPLICATIONS

This application claims the benefit of priority to United States Provisional Application Serial Numbers: 60/632,426, filed 02 December 2004 and entitled METHODS AND NUCLEIC ACIDS FOR THE ANALYSIS OF GENE EXPRESSION ASSOCIATED WITH THE PROGNOSIS OF PROSTATE CELL PROLIFERATIVE DISORDERS; 60/662,220, filed 14 March 2005 of same title; 60/723,125 filed 03 October 2005 of same title; 60/_,_,filed November 30, 2005 of same title; 60/633,250 filed 02 December 2004 and entitled METHODS AND NUCLEIC ACIDS FOR THE ANALYSIS OF GENE EXPRESSION ASSOCIATED WITH THE DEVELOPMENT OF PROSTATE CELL PROLIFERATIVE DISORDERS; and 60/723,054 filed 03 October 3 2005 of same title, all of which are incorporated by reference herein in their entireties.

SEQUENCE LISTING

A Sequence Listing, pursuant to PCT Administrative Instructions Part 8, Section 801(a), has been provided on compact disc (1 of 1) as a 3.25 MB text file (476_0001.txt), which is incorporated by reference herein in its entirety.

BACKGROUND

*Prostate cancer.* Prostate cancer is the most common malignancy among men in the United States (~200,000 new cases per year), and the sixth leading cause of male cancer-related deaths worldwide (~204,000 per year). Prostate cancer is primarily a disease of the elderly, with approximately 16% of men between the ages of 60 and 79 having the disease. According to some estimates at autopsy, 80% of all men over 80 years of age have some form of prostate disease (*e.g.*, cancer, BPH, prostatitis, etc). Benign prostate hypertrophy is present in about 50% of men aged 50 or above, and in 95% of men aged 75 or above. Prostate cancer, based on these reports, is often not a disease that men die from, but more typically-with. Recent evidence suggests that the incidence of prostate cancer may in fact be declining, likely as result of better treatment, better surgery, and earlier detection.

*Diagnosis of prostate cancer; molecular approaches.* Current guidelines for prostate cancer screening have been suggested by the American Cancer Society and are as follows: At 50 years of age, health care professionals should offer a blood test for prostate specific antigen (PSA) and perform a digital rectal exam (DRE). It is recommended that high risk populations, such as African Americans and those with a family history of prostate disease, should begin screening at 45 years of age. Men without abnormal prostate pathology generally have a PSA level in blood below 4ng/ml. PSA levels between 4ng/ml and 10ng/ml (called the 'Grey Zone') have a 25% chance of having prostate cancer. The result is that 75% of the time, men with an abnormal DRE and a PSA in this *grey zone* have a negative, or a seemingly unnecessary biopsy. Above the grey zone, the likelihood of having prostate cancer is significant (> 67%) and increases even further as PSA levels go up. Numerous methods exist for measuring PSA (percent-free PSA, PSA velocity, PSA density, etc.), and each has an associated accuracy for detecting the presence of cancer. Yet, even with the minor improvements in detection, and the reported drops in mortality associated with screening, the frequency of false positives remains high. Reduced specificity results in part from increased blood PSA associated with BPH, and prostatis. It has also been estimated that up to 45% of prostate biopsies under current guidelines are falsely negative, resulting in decreased sensitivity even with biopsy.

TRUS guided biopsy is considered the 'gold standard' for diagnosing prostate cancer. Recommendations for biopsy are based upon abnormal PSA levels and or an abnormal DREs For PSA there is a grey zone where a high percentage of biopsies are perhaps not necessary. Yet the ability to detect cancer in this grey zone (PSA levels of 4.0 to 10 ng/ml) is difficult without biopsy. Due to this lack of specificity, 75% of men undergoing a biopsy do not have cancer. Yet without biopsy, those with cancer would be missed, resulting in increased morbidity and mortality. Unfortunately, the risks associated with an unnecessary biopsy are also high.

Molecular markers would offer the advantage that they can be used to efficiently analyze even very small tissue samples, and samples whose tissue architecture has not been maintained. Within the last decade, numerous genes have been studied with respect to differential expression among benign hyperplasia of the prostate and different grades of prostate cancer. However, no single marker has as yet been shown to be sufficient for the prognostic classification of prostate tumors in a clinical setting.

Alternatively, high-dimensional mRNA-based approaches may, in particular instances, provide a means to distinguish between different tumor types and benign and malignant lesions. However, application of such approaches as a routine diagnostic tool in a clinical environment is impeded and substantially limited by the extreme instability of mRNA, the rapidly occurring expression changes following certain triggers (*e.g.*, sample collection), and, most importantly, by the large amount of mRNA needed for analysis which often cannot be obtained from a routine biopsy (see, *e.g.*, Lipshutz, R. J. et al., Nature Genetics 21:20-24, 1999; Bowtell, D. D. L. Nature Genetics Suppl. 21:25-32, 1999).

Aberrant genetic methylation in prostate cancer has been observed in several genes including GSTPi, AR, p16 (CDKN2a/INK4a), CD44, CDH1. Genome-wide hypomethylation for example of the LINE-1 repetitive element has also been associated with tumor progression (Santourlidis, S., et al., Prostate 39:166-74, 1999).

*Prostate Cancer Treatment Options.* There are many treatment strategies available to patients diagnosed with prostate cancer, and the decision for the patients and physicians is often unclear. Because prostate cancer can be a slowly developing disease, many men choose a treatment approach called watchful waiting, or conservative management. As the names imply, this approach does not include any radical therapy intended to cure the patient. Instead, the disease is carefully monitored using PSA tests and DREs. The ideal patient for this approach is one whose tumor is slow growing and non-invasive, and who is therefore likely to die of other causes before the prostate cancer becomes problematic.

For younger patients with localized disease, curative treatment is more appropriate. Radical prostatectomy is used to remove the prostate and hopefully all traces of the tumor. The surgical margins, seminal vesicles, and sometimes lymph nodes are tested for the presence of cancer, and in each case the presence of cancer correlates with reduced disease free survival Overall, about 70% of men remain free of disease ten years after surgery (Roehl, et al., 2004). Radical prostatectomy is a significant surgery, with side effects including blood loss, incontinence, and impotence. The rate of intraoperative and postoperative complications is estimated to be less than 2% (Lepor, et al., 2001).

Radiation therapy is also used to attempt to cure prostate cancer patients. Patients can choose either external beam radiation or brachytherapy (radioactive seed implants). The rates of survival and the side effects are similar to radical prostatectomy (D'amico, et al., 1998). For both radical prostatectomy and radiation therapy, the probability of survival is highly dependent on the stage and differentiation of the tumor. Localized indolent tumors are more likely to be cured.

Hormonal therapy is often used for patients whose cancer has spread beyond the prostate or for patients whose cancer has recurred after prostatectomy or radiation therapy. In other words, hormonal therapy is used to control cancer but not to cure it. Hormonal therapy is sometimes used in conjunction with other therapies such as radiation or as a neoadjuvant therapy prior to surgery. The goal of hormonal therapy is to reduce the stimulatory effect of androgens on the prostate tumor. The reduction in hormones is achieved through orchiectomy, lutenizing hormone-releasing hormone (LHRH) analogs, and antiandrogens. Side effects of hormonal therapy can include impotence, hot flashes, fatigue, and reduced libido. Eventually, prostate tumors become insensitive to androgens and hormonal therapy is no longer effective.

After the tumor has spread outside the capsule and hormonal therapy has failed, chemotherapy can be used to relieve pain or delay the progression of the disease. The response to chemotherapy is variable, and lives are extended for only a minority of patients. Bisphosphonates are used to reduce the osteolytic activity of tumors metastasised to the bones.

*Prostate Cancer Prognosis Estimation.* DRE, TRUS, biopsy, and PSA provide initial staging information on the tumor, but MRI, CT scans, ProstaScint scans and bone scans are used to determine the spread of the cancer beyond the prostatic capsule. These tests are not used on every prostate cancer patient, but only those with some likelihood of metastases. If metastases can be confirmed, the patient will receive treatment designed to slow the progression of the disease. If no metastases are detected, a patient is a candidate for potentially curative treatments such as prostatectomy and radiation therapy. Prior to the removal of the prostate, lymph nodes are sometimes dissected as a final test for metastases. If metastases are present in the dissected nodes, the surgery may be aborted. Analysis of the tissue surgically removed during prostatectomy is the final and gold standard staging technique for those patients who choose to undergo surgery. Frequently, analysis of the surgical specimens shows that the patient was originally *understaged* by the diagnostic tests (Bostwick, 1997).

An accurate estimation of prognosis is crucial for selection of the most appropriate treatment for each patient. Since organ confined prostate cancer cannot lead to death, estimation of prognosis is also an estimation of the presence or likelihood of development of metastases. A patient who is likely to develop cancer outside of the prostatic capsule will receive more extensive diagnostic work-up, including MRI and CT scans, and possibly more radical treatments, including surgery and radiation.

An initial prognostic assessment is made from the results of a PSA test, DRE, and biopsy analysis. The size, location, and method of detection of the tumor are combined to give a staging score on the TNM scale. Patients with higher stage tumors and high PSA values are more likely to have cancer that has spread or will spread outside of the prostate. A histological analysis of the biopsy allows a pathologist to determine the Gleason score. The Gleason score is a composite of the two most prevalent grades in the tissue sample, and the grades can range between one and five. A higher grade indicates more extreme dedifferentiation, and higher composite scores correlate with higher probability for metastasis and reduced disease free survival.

Prostate cancer nomograms have been developed and modified to predict the risk of cancer recurrence after primary therapy based on PSA levels, Gleason grading, and pre-operative staging information (Kattan et al 2003; Kattan et al 1998; Potter et al 2001). The data is derived from actual patient survival rates in cohorts of thousands of patients at multiple institutions. As with all prognostic measurements in prostate cancer, the estimate of recurrence risk by the nomogram is also an estimate of the likelihood of presence of cancer outside the prostatic capsule. Because the clinical characteristics of the cancers that patients are presenting with have changed with the widespread use of PSA, the nomograms are out of date and are not widely used. However, the general process of integrating Gleason, stage, and PSA information is still used.

Patients with cancer that has spread to lymph nodes or other metastatic sites are treated with systemic therapies such as hormonal therapies. Patients with localized disease (T1-T3) are candidates for definitive, curative treatments such as surgery or radiation. Those patients with localized disease who are thought to be low-risk are ideal candidates for watchful waiting. Those with intermediate risk are ideal for monotherapy such as surgery or radiation. Those with high risk localized disease should be considered for multimodal therapies or clinical trials.

After surgery, more prognostic information is available because the tumor spread can be directly analyzed. During some prostatectomies, the lymph nodes are directly dissected and the nodal status is confirmed. In all surgeries, the tumor spread to the seminal vesicles and the margin status are checked. Positive nodes, seminal vesicles, and margins all indicate an inferior prognosis and may suggest that the patient should receive adjuvant treatment.

*Molecular prostate cancer prognostic markers; deficiencies of prior art approaches.* As an alternative to current approaches to the prognostic classification of prostate carcinoma patients a variety of molecular approaches are currently being explored. It is anticipated that the development of suitable molecular markers will have significant advantages over current approaches in terms of accuracy, cost-effectiveness and/or patient invasiveness. A variety of molecular markers have been discovered including monoclonal antibodies. In a study by Xu et al (ICDB/95613763) 114 cases of prostate cancer showed that 57% of the bone marrow specimens had elevated OVX1 levels (greater than 7.2 U/ml). In other experiments, OVX1 levels were about 2-fold higher in serum samples from androgen-independent than from androgen-dependent prostate cancer patients (p less than 0.001), suggesting that serum OVX1 levels may be able to predict the progression of prostate cancer, since this disease when it progresses typically becomes androgen-independent. Expression of the PSCA protein and mRNA has been positively correlated with adverse tumor characteristics, such as increasing pathological grade (poor cell differentiation), worsening clinical stage and androgen-independence and speculatively with prostate carcinogenesis (Jpn J Clin Oncol, 4:414-9, 2004). Other prospective mRNA analysis markers include Hepsin. Expression of Hepsin showed significant difference between patients at lower risk (pT2, G2 and Gleason score less than 7) and higher risk (pT3/4, G3 and Gleason score 7 or greater) for relapse (J Urol, 171:187-91, 2004).

The GSTPi gene is the most well characterized prostate carcinoma diagnostic marker. Zhou et al. (J Urol, 171: 2195-8, 2004) recently correlated expression of the GSTPi gene with Gleason grade and cancer volume. Furthermore, use of the gene GSTPi as a marker for the detection of prostate carcinomas located in the peripheral zone (*i.e.*, with a high likelihood of metastasis) has also been described in U.S. patent application serial number 10/350,763, which is hereby incorporated by reference in its entirety.

Another methylation marker which may be suitable for the prognostic classification of prostate carcinomas is uPA. Rabbani et al. (The FASEB Journal 17:1081-1088, 2003) have shown that the uPA promoter is hypermethylated in hormone-responsive PrEC and LNCaP cells and hypomethylated in hormone-insensitive PC-3 cells. De-methylation of the promoter in the LNCaP cell lines resulted in increase of mRNA analysis and resulted in an increase in the invasive capacity. Singal et al., analysed methylation of a gene panel consisting of glutathione s-transferase Pi1 (GSTP1), retinoic acid receptor beta (RARB), CD44, E-cadherin (ECAD) and RAS association domain family protein 1A (RASSF1A) in prostate cancer. A methylation index (MI) was calculated as the total number of genes methylated, higher MI was noted in stage III as compared to stage II disease, and in Gleason score 7 as compared to Gleason score 6 samples. Singal et al. thus concluded that the results suggest that the methylation of the gene panel in correlated with clinicopathological features of poor prognosis.

*Pronounced need in the art.* Significantly, however, none of the heretofore mentioned markers are sufficiently developed to provide a marker for the prognosis of prostate cell proliferative disorders that is sufficiently robust and/or accurate for effective use in a clinical setting.

While accurate diagnosis of prostate carcinoma is important, the most pressing need in prostate cancer treatment is for information to guide the treatment planning decision.

Leaders in the field agree that many patients with clinically insignificant disease receive unnecessary radical treatments such as prostatectomy or radiation therapy. However, twenty percent of patients who do receive these curative treatments experience disease recurrence. A molecular test could help select patients for the optimal treatment choice and thereby reduce over and under treatment

Currently the therapy choice is made based on the likelihood of spread of the disease. Low-risk patients are candidates for watchful waiting. Medium and high risk patients should receive surgery or radiation, and the high risk patients are candidates for additional adjuvant treatments. Staging, Gleason, and PSA are currently used to estimate this risk, but the combined information from these tests is insufficient. Very few patients are recommended for watchful waiting because clinicians cannot be sure which cancers are indolent. Furthermore, many patients who receive monotherapy experience a recurrence.

Gleason grading currently plays a primary role in prognostic assessment. Patients with localized disease and high Gleason scores (8-10) always undergo radical treatments. Patients with low Gleason scores (2-5) have the option of deferring curative treatment and opting for watchful waiting, however many chose to undergo curative therapy soon after diagnosis. For patients with mid-range Gleason scores, which is the majority of patients diagnosed today, clinicians must use other less-reliable prognostic indicators for further information.

Accordingly there is a pronounced need in the art for a novel, effective prognostic test, and in particular one that would predict the probability that a cancer has or is likely to spread outside of the prostate based on the methylation patterns of biopsy samples. This type of information is highly valuable in the diagnostic and treatment planning processes. This information would initially be used in reaching the decision about whether imaging tests are necessary to check for metastasis for a complete diagnostic work-up. Surgery is unnecessary for any patient whose cancer has already spread, but if a patient is not selected for imaging the metastases will not be detected until surgery or later.

Furthermore there is a pronounced need in the art for a novel and effective prostate cell proliferative disorder molecular classification test, and in particular one that would be suitable for the analysis of biopsy samples to improve the stratification of patients into low, intermediate, and high risk categories so that optimal treatment plans can be selected for each patient. With accurate stratification, patients and doctors can choose watchful waiting with confidence that there is little risk for early recurrence. This test would therefore reduce the number of unnecessary surgeries and radiation treatments.

Additionally, with improved estimations of which patients are likely to recur with monotherapy, physicians can make better use of available adjuvant treatments. If a patient chooses to undergo surgery, the test can be repeated on prostatectomy samples to verify the assessment of his need for adjuvant therapy. The benefits of different adjuvant therapy approaches are still being worked out in clinical trials, and a molecular test could provide valuable information to stratify patients for this additional treatment or for clinical trials.

PITX2 (Paired-like homeodomain transcription factor 2), also known as PTX2, RIEG1, or ARP1, encodes a member of the RIEG/PITX homeobox family, which is in the bicoid class of homeodomain proteins. PITX2 encodes several alternative transcripts, and mutations in the gene lead to the autosomal-dominant disorder Rieger's syndrome, a developmental disorder predominantly affecting the eye (Semina *et al.,* 1996). The protein acts as a transcription factor and is involved in the development of several major organs. It is induced by the WNT pathway, and mediates cell-type specific proliferation by inducing growth-regulating genes (Kioussi et al. 2002).

Toyota et al. (2001) found hypermethylation of the gene in a large proportion of acute myeloid leukemias. Several studies by the applicant (see WO 2005/059172) have demonstrated that hypermethylation of PITX2 is associated with poor prognosis for breast cancer patients.

The GPR7 marker is located in a CpG island in the promoter region of an intronless gene on chromosome 10. GPR7, or G-protein receptor 7, is a receptor for neuropeptide W and neuropeptide B (Shimomura *et al.* 2002; Tanaka *et al.* 2003). The expression of GPR7 has been studied in the brain, and is expressed mainly in the cerebellum and frontal cortex (O'Dowd *et al*. 1995). Ishii *et al*. (2003) studied the phenotype of mice lacking a functional copy of GPR7. The mice developed adult-onset obesity and metabolic defects such as decreased energy expenditure and increased blood levels of glucose and insulin. Interestingly, these phenotypes were only detected in male mice. The GPR7 ligands, neuropeptides W and B, have also been implicated in metabolism and obesity in separate studies (Samson *et al.* 2004; Levine *et al.* 2005). GPR7, which is similar in sequence to opioid receptors, may also have a role in pain signaling (Zaratin *et al*. 2005).

SEQ ID NO: 63 is located within the regulatory region of HIST2H2BF on chromosome 1 in a region with several histone genes. The histone content and status of chromatin can influence the expression of the encoded gene. Methylation and altered expression of a histone gene in prostate cancer could cause chromatin changes throughout the genome that alter gene expression in ways that result in more aggressive tumor properties. There are no published articles on the function of this particular histone.

The marker referred to as SEQ ID NO: 35 is located on chromosome 3 downstream of the FOXL2 (Forkhead transcription factor) gene and within or near predicted genes or ESTs. Although it is downstream, it is anticipated that methylation of this marker effects the expression of FOXL2, which is mutated in the blepharophimosis-ptosis epicanthus inversus syndrome (BPES). This syndrome is characterized by eye, craniofacial, and ovarian abnormalities. Methylation of the marker may also affect the expression of the EST, or the EST may be shown to be an alternative exon for the FOXL2 gene.

SUMMARY OF THE INVENTION

The present invention provides novel and efficacious methods and nucleic acids for providing a prognosis of prostate cell proliferative disorders.

The invention solves this longstanding need in the art by providing genes, genomic sequences and/or regulatory regions thereof according to Table 11 (or to one or more of those), the expression thereof being indicative of the prognosis of prostate cell proliferative disorders or features thereof. It is particularly preferred that said genes, genomic sequences and/or regulatory regions are selected from the group consisting PITX2, SEQ ID NO: 63, GPR7 and SEQ ID NO: 35. Further preferred is the gene PITX2. In a particularly preferred embodiment of the invention, the methylation status of CpG positions of genes, genomic sequences and/or regulatory regions thereof according to Table 11 (or to one or more of those) is indicative of the prognosis of prostate cell proliferative disorders or features thereof. It is particularly preferred that said genes, genomic sequences and/or regulatory regions are selected from the group consisting PITX2, SEQ ID NO: 63, GPR7 and SEQ ID NO: 35.Further preferred is the gene PITX2. It is particularly preferred that said prostate cell proliferative disorder is a prostate carcinoma or prostate neoplasm.

To enable this analysis the invention provides a method for the analysis of biological samples for genomic methylation associated with the development of prostate cell proliferative disorders. Said method is **characterised in that** at least one nucleic acid, or a fragment thereof, from the group consisting of SEQ ID NO:1 to SEQ ID NO:64 and SEQ ID NO: 961 (preferably SEQ ID Nos: 35, 63, 19 and most preferably SEQ ID NO: 961) is/are contacted with a reagent or series of reagents capable of distinguishing between methylated and non methylated CpG dinucleotides within the genomic sequence, or sequences of interest.

It is particularly preferred that the method and nucleic acids according to the invention are utilised for at least one of: prognosis of; treatment of; monitoring of; and treatment and monitoring of prostate cell proliferative disorders.

The present invention provides a method for ascertaining genetic and/or epigenetic parameters of genomic DNA. The method has utility for the improved prognostic classification of prostate cell proliferative disorders, more specifically by enabling the improved identification of and differentiation between aggressive and non-aggressive forms of said disorder. The invention presents several substantial improvements over the state of the art. Although some methylation assays for the detection of cancer are known, there is currently no molecular classification system for the *prognostic* classification of tumours.

The DNA source may be any suitable source. Preferably, the source of the DNA sample is selected from the group consisting of cells or cell lines, histological slides, biopsies, paraffin-embedded tissue, bodily fluids, ejaculate, urine, blood, and combinations thereof. Preferably, the source is biopsies, bodily fluids, ejaculate, urine, or blood.

Specifically, the present invention provides a method for providing a prognosis of prostate cell proliferative disorders, comprising: obtaining a biological sample comprising genomic nucleic acid(s); contacting the nucleic acid(s), or a fragment thereof, with one reagent or a plurality of reagents sufficient for distinguishing between methylated and non methylated CpG dinucleotide sequences within a target sequence of the subject nucleic acid, wherein the target sequence comprises, or hybridizes under stringent conditions to, a sequence comprising at least 16 contiguous nucleotides of SEQ ID NO:1 to SEQ ID NO:64 and SEQ ID NO: 961, (preferably SEQ ID Nos: 35, 63, 19 and most preferably SEQ ID NO: 961) said contiguous nucleotides comprising at least one CpG dinucleotide sequence; and determining, based at least in part on said distinguishing, the methylation state of at least one target CpG dinucleotide sequence, or an average, or a value reflecting an average methylation state of a plurality of target CpG dinucleotide sequences. Preferably, distinguishing between methylated and non methylated CpG dinucleotide sequences within the target sequence comprises methylation state-dependent conversion or non-conversion of at least one such CpG dinucleotide sequence to the corresponding converted or non-converted dinucleotide sequence within a sequence selected from the group consisting of SEQ ID NO:65 to SEQ ID NO:320 and SEQ ID NO: 962 to SEQ ID NO: 965, and contiguous regions thereof corresponding to the target sequence. Preferably said sequence is selected from the goup consisting of SEQ ID Nos: 133,134,261,262, 189,190,317,318, 101,102,229,230 and most preferably said sequence is selected from the goup consisting of SEQ ID Nos: 962 - 965

Additional embodiments provide a method for providing a prognosis of prostate cell proliferative disorders, comprising: obtaining a biological sample having subject genomic DNA; extracting the genomic DNA; treating the genomic DNA, or a fragment thereof, with one or more reagents to convert 5-position unmethylated cytosine bases to uracil or to another base that is detectably dissimilar to cytosine in terms of hybridization properties; contacting the treated genomic DNA, or the treated fragment thereof, with an amplification enzyme and at least two primers comprising, in each case a contiguous sequence at least 9 nucleotides in length that is complementary to, or hybridizes under moderately stringent or stringent conditions to a sequence selected from the group consisting SEQ ID NO:65 to SEQ ID NO:320 and SEQ ID NO: 962 to SEQ ID NO: 965 (preferably said group consists of SEQ ID Nos: 133,134,261,262, 189,190,317,318, 101,102,229,230 and most preferably said group consists of SEQ ID Nos: 962 - 965) and complements thereof, wherein the treated DNA or the fragment thereof is either amplified to produce an amplificate, or is not amplified; and determining, based on a presence or absence of, or on a property of said amplificate, the methylation state of at least one CpG dinucleotide sequence selected from the group consisting of SEQ ID NO:1 to SEQ ID NO:64 and SEQ ID NO: 961 (preferably said group consists of SEQ ID Nos: 35, 63, 19 and most preferably is SEQ ID NO: 961), or an average, or a value reflecting an average methylation state of a plurality of CpG dinucleotide sequences thereof.

Preferably, at least one such hybridizing nucleic acid molecule or peptide nucleic acid molecule is bound to a solid phase. Preferably, determining comprises use of at least one method selected from the group consisting of: hybridizing at least one nucleic acid molecule comprising a contiguous sequence at least 9 nucleotides in length that is complementary to, or hybridizes under moderately stringent or stringent conditions to a sequence selected from the group consisting of SEQ ID NO:65 to SEQ ID NO:320, (preferably said group consists of SEQ ID Nos: 133,134,261,262, 189,190,317,318, 101,102,229,230 and most preferably said group consists of SEQ ID Nos: 962 - 965) and complements thereof; hybridizing at least one nucleic acid molecule, bound to a solid phase, comprising a contiguous sequence at least 9 nucleotides in length that is complementary to, or hybridizes under moderately stringent or stringent conditions to a sequence selected from the group consisting of SEQ ID NO:65 to SEQ ID NO:320 and SEQ ID NO: 962 to SEQ ID NO: 965, (preferably said group consists of SEQ ID Nos: 133,134,261,262, 189,190,317,318, 101,102,229,230 and most preferably said group consists of SEQ ID Nos: 962 - 965), and complements thereof; hybridizing at least one nucleic acid molecule comprising a contiguous sequence at least 9 nucleotides in length that is complementary to, or hybridizes under moderately stringent or stringent conditions to a sequence selected from the group consisting of SEQ ID NO:65 to SEQ ID NO:320 and SEQ ID NO: 962 to SEQ ID NO: 965 (preferably said group consists of SEQ ID Nos: 133,134,261,262, 189,190,317,318, 101,102,229,230 and most preferably said group consists of SEQ ID Nos: 962 - 965), and complements thereof, and extending at least one such hybridized nucleic acid molecule by at least one nucleotide base; and sequencing of the amplificate.

Further embodiments provide a method for providing a prognosis of prostate cell proliferative disorders, comprising: obtaining a biological sample having subject genomic DNA; extracting the genomic DNA; contacting the genomic DNA, or a fragment thereof, comprising one or more sequences selected from the group consisting of SEQ ID NO:1 to SEQ ID NO:64 and SEQ ID NO: 961 (preferably said group consists of SEQ ID Nos: 35, 63, 19 and most preferably said sequence is SEQ ID NO: 961) or a sequence that hybridizes under stringent conditions thereto, with one or more methylation-sensitive restriction enzymes, wherein the genomic DNA is either digested thereby to produce digestion fragments, or is not digested thereby; and determining, based on a presence or absence of, or on property of at least one such fragment, the methylation state of at least one CpG dinucleotide sequence of one or more sequences selected from the group consisting of SEQ ID NO:1 to SEQ ID NO:64 and SEQ ID NO: 961 , or an average, or a value reflecting an average methylation state of a plurality of CpG dinucleotide sequences thereof. Preferably said group consists of SEQ ID Nos: 35, 63, 19 and most preferably said sequence is SEQ ID NO: 961. Preferably, the digested or undigested genomic DNA is amplified prior to said determining.

Additional embodiments provide novel genomic and chemically modified nucleic acid sequences, as well as oligonucleotides and/or PNA-oligomers for analysis of cytosine methylation patterns within sequences from the group consisting of SEQ ID NO:1 to SEQ ID NO:64 and SEQ ID NO: 961.

## BRIEF DESCRIPTION OF THE DRAWINGS

Figure 1 shows a ROC plot for a SEQ ID NO:19 MSP assay run on 26 frozen radical prostatectomy samples from patients with early PSA recurrence, and on 30 samples from patients with no PSA recurrence after at least 48 months.

Figure 2 shows a ROC plot for a SEQ ID NO:35 MSP assay run on 26 frozen radical prostatectomy samples from patients with early PSA recurrence and 30 samples from patients with no PSA recurrence after at least 48 months.

Figure 3 shows a ROC plot for a SEQ ID NO: 37 MSP assay run on 26 frozen radical prostatectomy samples from patients with early PSA recurrence and 30 samples from patients with no PSA recurrence after at least 48 months.

Figure 4 shows a ROC plot for a SEQ ID NO: 7 MSP assay run on 26 frozen radical prostatectomy samples from patients with early PSA recurrence and 30 samples from patients with no PSA recurrence after at least 48 months.

Figure 5 shows a ROC plot for a SEQ ID NO: 63 MSP assay run on 26 frozen radical prostatectomy samples from patients with early PSA recurrence and 30 samples from patients with no PSA recurrence after at least 48 months.

Figure 6 shows a ROC plot for a SEQ ID NO: 8 MSP assay run on 26 frozen radical prostatectomy samples from patients with early PSA recurrence and 30 samples from patients with no PSA recurrence after at least 48 months.

Figure 7 shows a ROC plot for a SEQ ID NO: 64 MSP assay run on 26 frozen radical prostatectomy samples from patients with early PSA recurrence and 30 samples from patients with no PSA recurrence after at least 48 months.

Figure 8 shows a gel electrophoresis analysis on 12 DNA samples. 200 ng per DNA was applied to a 0.8 % agarose gel. The gel was run for 4 hours at 80 Volt. The size marker (Invitrogen, No.: 10496-016) contains the following fragments: 10.000 bp, 6.000 bp.

Figure 9 shows ALF Express analyses of multiplex PCR products (8plex, mPCR SetD2) compared to single PCR products (sPCR Set D2). The size standard (lanes 1,4) contained fragments of the following lengths: 50, 100, 150, 200, 250, 300, 350, 400, 450, 500 bp. All fragments could be amplified. An undesired side product (220 bp) was observed.

Figure 10 shows performance of multiplex PCR. Lane 1: 100bp marker. Lanes 2-11: multiplex PCR performance of 10 test samples, lane 12: positive control, lane 13: H2O control.

Figure 11 shows Tumor vs. Lymphocyte samples, ranked by Wilcoxon statistics. Bonferroni corrected p-values (upper) and AUCs (lower) are shown to the right of the data matrix. Each column represents one sample; each row one oligonucleotide. Oligonucleotides are grouped per marker candidate. The indicated markers are ordered from top to bottom with increasing AUC. On the right side of each marker Bonferroni corrected Wilcoxon p-value and AUC are given. Below the AUC sensitivity at a specificity of ~ 0.75 are given enclosed in brackets. Methylation data are centered and normalized to one standard deviation for individual oligonucleotides. The color represents the relative distance of the oligonucleotide methylation status from the mean value. Light grey represents hypomethylated CpGs within an oligonucleotide while dark grey indicates hypermethylated CpGs within an oligonucleotide.

Figure 12 shows high Gleason vs. Low Gleason marker rankings. The plot displays uncorrected p-values from the genewise Wilcoxon rank statistics analysis. Lower and upper dotted lines show 5% Bonferroni and FDR limits, respectively.

Figure 13 shows high Gleason vs. Low Gleason methylation matrix of the 10 markers with best AUC. Gleason scores are shown above each group of samples. Each column represents one sample; each row one oligonucleotide (1, 2, or 3 CpG sites each). Oligonucleotides are grouped per marker candidate. The indicated markers are ordered from top to bottom with increasing AUC. On the right side of each marker Bonferroni corrected Wilcoxon p-value and AUC are given. Below the AUC sensitivity at a specificity of ~ 0.75 are given enclosed in brackets. Methylation data are centered and normalized to one standard deviation for individual oligonucleotides. The color represents the relative distance of the oligonucleotide methylation status from the mean value. Light grey represents hypomethylated CpGs within an oligonucleotide while dark grey indicates hypermethylated CpGs within an oligonucleotide.

Figure 14 shows Early Recurrence vs. No recurrence marker rankings. The plot gives uncorrected p-values from the genewise Wilcoxon rank test analysis. Lower and upper dotted lines show 5% Bonferroni and FDR limits, respectively.

Figure 15 shows Early Recurrence vs. No recurrence methylation matrix of the 10 markers with best AUC. Each column represents one sample; each row one oligonucleotide (1, 2, or 3 CpG sites each). Oligonucleotides are grouped per marker candidate. The indicated markers are ordered from top to bottom with increasing AUC. On the right side of each marker Bonferroni corrected Wilcoxon p-value and AUC are given. Below the AUC sensitivity at a specificity of ~ 0.75 are given enclosed in brackets. Methylation data are centered and normalized to one standard deviation for individual oligonucleotides. The color represents the relative distance of the oligonucleotide methylation status from the mean value. Light grey represents hypomethylated CpGs within an oligonucleotide while dark grey indicates hypermethylated CpGs within an oligonucleotide.

For Figures 16-88, each figure shows the sequence of the analysed amplificate of each respective SEQ ID NO. In each figure, an analyzed amplificate is displayed in a 'wrapped' series of panels, where the first row (top row) in each panel shows the *genomic* sequence being amplified (the genomic sequence row), and where forward and reverse amplification primers (defining an 'amplicon') are shown in the panel row (the primer display row) immediately below the first row. The row below the primer display row (or, in panels not displaying a primer, the row below the genomic display row) is the bisulfite converted sequence of the amplificate (the bisulfite converted sequence row; wherein CpG positions are marked red). The remaining rows displayed in some panels, show the sequences of detection oligonucleotides (CG and TG oligos) used to analyze the amplificate.

Figure 16 shows an Amplificate of SEQ ID NO:14.
Figure 17 shows an Amplificate of SEQ ID NO:15.
Figure 18 shows an Amplificate of SEQ ID NO:16.
Figure 19 shows an Amplificate of SEQ ID NO:17.
Figure 20 shows an Amplificate of SEQ ID NO:18
Figure 21 shows an Amplificate of SEQ ID NO:19
Figure 22 shows an Amplificate of SEQ ID NO:20
Figure 23 shows an Amplificate of SEQ ID NO:21
Figure 24 shows an Amplificate of SEQ ID NO:22
Figure 25 shows an Amplificate of SEQ ID NO:23
Figure 26 shows an Amplificate of SEQ ID NO:24
Figure 27 shows an Amplificate of SEQ ID NO:25
Figure 28 shows an Amplificate of SEQ ID NO:26

Figure 29 shows an Amplificate of SEQ ID NO:27
Figure 30 shows an Amplificate of SEQ ID NO:28
Figure 31 shows an Amplificate of SEQ ID NO:29
Figure 32 shows an Amplificate of SEQ ID NO:30
Figure 33 shows an Amplificate of SEQ ID NO:31
Figure 34 shows an Amplificate of SEQ ID NO:32 (amplificate A)
Figure 35 shows an Amplificate of SEQ ID NO:32 (amplificate B)
Figure 36 shows an Amplificate of SEQ ID NO:33
Figure 37 shows an Amplificate of SEQ ID NO:34
Figure 38 shows an Amplificate of SEQ ID NO:35
Figure 39 shows an Amplificate of SEQ ID NO:13
Figure 40 shows an Amplificate of SEQ ID NO:36
Figure 41 shows an Amplificate of SEQ ID NO:37
Figure 42 shows an Amplificate of SEQ ID NO:1
Figure 43 shows an Amplificate of SEQ ID NO:2
Figure 44 shows an Amplificate of SEQ ID NO:3
Figure 45 shows an Amplificate of SEQ ID NO:4
Figure 46 shows an Amplificate of SEQ ID NO:5
Figure 47 shows an Amplificate of SEQ ID NO:6
Figure 48 shows an Amplificate of SEQ ID NO:7
Figure 49 shows an Amplificate of SEQ ID NO:38
Figure 50 shows an Amplificate of SEQ ID NO:39
Figure 60 shows an Amplificate of SEQ ID NO:40
Figure 61 shows an Amplificate of SEQ ID NO:41
Figure 62 shows an Amplificate of SEQ ID NO:42
Figure 63 shows an Amplificate of SEQ ID NO:43
Figure 64 shows an Amplificate of SEQ ID NO:44
Figure 65 shows an Amplificate of SEQ ID NO:45
Figure 66 shows an Amplificate of SEQ ID NO:46
Figure 67 shows an Amplificate of SEQ ID NO:47
Figure 68 shows an Amplificate of SEQ ID NO:48
Figure 69 shows an Amplificate of SEQ ID NO:49
Figure 70 shows an Amplificate of SEQ ID NO:50
Figure 71 shows an Amplificate of SEQ ID NO:51
Figure 72 shows an Amplificate of SEQ ID NO:52
Figure 73 shows an Amplificate of SEQ ID NO:53
Figure 74 shows an Amplificate of SEQ ID NO:54
Figure 75 shows an Amplificate of SEQ ID NO:55
Figure 76 shows an Amplificate of SEQ ID NO:56
Figure 77 shows an Amplificate of SEQ ID NO:57
Figure 78 shows an Amplificate of SEQ ID NO:58
Figure 79 shows an Amplificate of SEQ ID NO:59
Figure 80 shows an Amplificate of SEQ ID NO:60
Figure 81 shows an Amplificate of SEQ ID NO:61
Figure 82 shows an Amplificate of SEQ ID NO:62
Figure 83 shows an Amplificate of SEQ ID NO:8
Figure 84 shows an Amplificate of SEQ ID NO:9
Figure 85 shows an Amplificate of SEQ ID NO:10
Figure 86 shows an Amplificate of SEQ ID NO:11
Figure 87 shows an Amplificate of SEQ ID NO:12 (amplificate A)
Figure 88 shows an Amplificate of SEQ ID NO:12 (amplificate B)
Figure 89 shows the distribution of follow up times of patients as analysed in Example 5. The white bars represent the distribution of all censored (no PSA relapse) patients. The grey bars show the distribution of the PSA-free survival time for all of the relapse patients. Frequency is shown on the Y-axis and time (months) is shown on the X-axis.
Figure 90 shows Kaplan-Meier survival analysis of the PITX2 marker (A & B) and ROC curve analysis (C) of the marker PITX2 in differentiating between prostate cancer patients according to Example 5. Proportion of recurrence-free patients is shown on the Y-axis, time in years is shown on the x-axis.
Figure 91 shows Kaplan-Meier survival analysis of the GPR7 marker (A & B) and ROC curve analysis (C) of the marker PITX2 in differentiating between prostate cancer patients according to Example 5. Proportion of recurrence-free patients is shown on the Y-axis, time in years is shown on the x-axis. Figure 92 shows Kaplan-Meier survival analysis of the SEQ ID NO: 63 marker (A & B) and ROC curve analysis (C) of the marker PITX2 in differentiating between prostate cancer patients according to Example 5. Proportion of recurrence-free patients is shown on the Y-axis, time in years is shown on the x-axis.

Figure 93 shows Kaplan-Meier survival analysis of the SEQ ID NO: 35 marker (A & B) and ROC curve analysis (C) of the marker PITX2 in differentiating between prostate cancer patients according to Example 5. Proportion of recurrence-free patients is shown on the Y-axis, time in years is shown on the x-axis.

Figure 94 shows Kaplan-Meier survival analysis of the ABHD9 marker (A & B) and ROC curve analysis (C) of the marker PITX2 in differentiating between prostate cancer patients according to Example 5. Proportion of recurrence-free patients is shown on the Y-axis, time in years is shown on the x-axis.

Figure 95 shows Kaplan-Meier survival analysis of the CCND2 marker (A & B) and ROC curve analysis (C) of the marker PITX2 in differentiating between prostate cancer patients according to Example 5. Proportion of recurrence-free patients is shown on the Y-axis, time in years is shown on the x-axis.

Figure 96 shows Kaplan-Meier survival analysis of PITX2 performance on sub-populations based on stage according to Example 5.

Figure 97 shows Kaplan-Meier survival analysis of PITX2 performance on sub-populations based on Gleason score according to Example 5.

Figure 98 shows Kaplan-Meier survival analysis of PITX2 performance on sub-populations based on nomogram score according to Example 5.

Figure 99 shows Kaplan-Meier survival analysis of SEQ ID NO: 63 performance on sub-populations based on High Gleason score according to Example 5.

Figure 100 shows Kaplan-Meier survival analysis of SEQ ID NO: 63 performance on sub-populations based on poor nomogram score according to Example 5.

Figure 101 shows Kaplan-Meier survival analysis of SEQ ID NO: 35 performance on T2 sub-populations according to Example 5.

Figure 102 shows the detected amplificate in both frozen and PET samples in the early biochemical relapse vs. no biochemical relapse comparisons using the assay of SEQ ID NO: 19 shown in Table 12 as detailed in Example 6.

Figure 103 shows the detected amplificate in both frozen and PET samples in the early biochemical relapse vs. no biochemical relapse comparisons using the assay of SEQ ID NO: 63 shown in Table 12 as detailed in Example 6.

Figure 104 shows the detected amplificate in both frozen and PET samples in the early biochemical relapse vs. no biochemical relapse comparisons using the assay of SEQ ID NO: 35 shown in Table 12 as detailed in Example 6.

Figure 105 shows the detected amplificate in both frozen and PET samples in the early biochemical relapse vs. no biochemical relapse comparisons using the assay of SEQ ID NO: 37 shown in Table 12 as detailed in Example 6.

Figure 106 shows the detected amplificate in PET samples only in the early biochemical relapse vs. no biochemical relapse comparisons using the assay of SEQ ID NO: 19 shown in Table 12 as detailed in Example 6.

Figure 107 shows the detected amplificate in PET samples only in the early biochemical relapse vs. no biochemical relapse comparisons using the assay of SEQ ID NO: 63 shown in Table 12 as detailed in Example 6.

Figure 108 shows the detected amplificate in PET samples only in the early biochemical relapse vs. no biochemical relapse comparisons using the assay of SEQ ID NO: 35 shown in Table 12 as detailed in Example 6.

Figure 109 shows the detected amplificate in PET samples only in the early biochemical relapse vs. no biochemical relapse comparisons using the assay of SEQ ID NO: 37 shown in Table 12 as detailed in Example 6.

Figure 110 shows the detected amplificate in frozen samples only in the early biochemical relapse vs. no biochemical relapse comparisons using the assay of SEQ ID NO: 19 shown in Table 12 as detailed in Example 6.

Figure 111 shows the detected amplificate in frozen samples only in the early biochemical relapse vs. no biochemical relapse comparisons using the assay of SEQ ID NO: 63 shown in Table 12 as detailed in Example 6.

Figure 112 shows the detected amplificate in frozen samples only in the early biochemical relapse vs. no biochemical relapse comparisons using the assay of SEQ ID NO: 35 shown in Table 12 as detailed in Example 6.

Figure 113 shows the detected amplificate in frozen samples only in the early biochemical relapse vs. no biochemical relapse comparisons using the assay of SEQ ID NO: 37 shown in Table 12 as detailed in Example 6.

Figure 114 shows the detected amplificate in both frozen and PET samples in the High Gleason vs. Low Gleason comparisons using the assay of SEQ ID NO: 19 shown in Table 12 as detailed in Example 6.

Figure 115 shows the detected amplificate in both frozen and PET samples in the High Gleason vs. Low Gleason comparisons using the assay of SEQ ID NO: 63 shown in Table 12 as detailed in Example 6.

Figure 116 shows the detected amplificate in both frozen and PET samples in the High Gleason vs. Low Gleason comparisons using the assay of SEQ ID NO: 35 shown in Table 12 as detailed in Example 6.

Figure 117 shows the detected amplificate in both frozen and PET samples in the High Gleason vs. Low Gleason comparisons using the assay of SEQ ID NO: 37 shown in Table 12 as detailed in Example 6.

Figure 118 shows the detected amplificate in PET samples only in the High Gleason vs. Low Gleason comparisons using the assay of SEQ ID NO: 19 shown in Table 12 as detailed in Example 6.
Figure 119 shows the detected amplificate in PET samples only in the High Gleason vs. Low Gleason comparisons using the assay of SEQ ID NO: 63 shown in Table 12 as detailed in Example 6.
Figure 120 shows the detected amplificate in PET samples only in the High Gleason vs. Low Gleason comparisons using the assay of SEQ ID NO: 35 shown in Table 12 as detailed in Example 6.
Figure 121 shows the detected amplificate in PET samples only in the High Gleason vs. Low Gleason comparisons using the assay of SEQ ID NO: 37 shown in Table 12 as detailed in Example 6.
Figure 122 shows the detected amplificate in frozen samples only in the High Gleason vs. Low Gleason comparisons using the assay of SEQ ID NO: 19 shown in Table 12 as detailed in Example 6.
Figure 123 shows the detected amplificate in frozen samples only in the High Gleason vs. Low Gleason comparisons using the assay of SEQ ID NO: 63 shown in Table 12 as detailed in Example 6.
Figure 124 shows the detected amplificate in frozen samples only in the High Gleason vs. Low Gleason comparisons using the assay of SEQ ID NO: 35 shown in Table 12 as detailed in Example 6.
Figure 125 shows the detected amplificate in frozen samples only in the High Gleason vs. Low Gleason comparisons using the assay of SEQ ID NO: 37 shown in Table 12 as detailed in Example 6.

DETAILED DESCRIPTION OF THE INVENTION

Definitions:

As used herein the term expression shall be taken to mean the transcription and translation of a gene. The level of expression of a gene may be determined by the analysis of any factors associated with or indicative of the level of transcription and translation of a gene including but not limited to methylation analysis, loss of heterozygosity (hereinafter also referred to as LOH), RNA expression levels and protein expression levels.

Furthermore the activity of the transcribed gene may be affected by genetic variations such as but not limited genetic mutations (including but not limited to SNPs, point mutations, deletions, insertions, repeat length, rearrangements and other polymorphisms).

As used herein the term "prognosis" shall be taken to mean a prediction of the progression of the disease (for example but not limited to regression, stasis and metastasis), in particular aggressiveness and metastatic potential of a prostate tumor.

As used herein the term "prognostic marker" shall be taken to mean an indicator of a prediction of the progression of the disease, in particular aggressiveness and metastatic potential of a prostate tumor.

As used herein the term "prognostic classification" shall be taken to mean the classification of a prostate cell proliferative disorder according to a prediction of the progression of the disease, in particular aggressiveness and metastatic potential of a prostate tumor.

It is preferably used to define patients with high, low and intermediate risks of death or recurrence after treatment that result from the inherent heterogeneity of the disease process. As used herein the term "aggressive" as used with respect to prostate tumor shall be taken to mean a prostate cell proliferative disorder that has the biological capability to rapidly spread outside of the prostate. Indicators of tumor aggressivness standard in the art include but are not limited to tumor stage, tumor grade, Gleason grade, nodal status and survival. As used herein the term "survival" shall not be limited to mean survival until mortality (wherein said mortality may be either irrespective of cause or prostate cell proliferative disorder related) but may be used in combination with other terms to define clinical terms, for example but not limited to "recurrence-free survival" (wherein the term recurrence shall include both localized and distant recurrence); metastasis free survival; disease free survival (wherein the term disease shall include prostate cancer and diseases associated therewith). The length of said survival may be calculated by reference to a defined start point (e.g. time of diagnosis or start of treatment) and a defined end point (e.g. death, recurrence or metastasis).

The term "Observed/Expected Ratio" ("O/E Ratio") refers to the frequency of CpG dinucleotides within a particular DNA sequence, and corresponds to the [number of CpG sites / (number of C bases x number of G bases)].

The term "CpG island" refers to a contiguous region of genomic DNA that satisfies the criteria of (1) having a frequency of CpG dinucleotides corresponding to an "Observed/Expected Ratio" >0.6, and (2) having a "GC Content" >0.5. CpG islands are typically, but not always, between about 0.2 to about 1 kb, or to about 2kb in length.

The term "methylation state" or "methylation status" refers to the presence or absence of 5-methylcytosine ("5-mCyt") at one or a plurality of CpG dinucleotides within a DNA sequence. Methylation states at one or more particular CpG methylation sites (each having two CpG CpG dinucleotide sequences) within a DNA sequence include "unmethylated," "fully-methylated" and "hemi-methylated."

The term "hemi-methytation" or "hemimethylation" refers to the methylation state of a palindromic CpG methylation site, where only a single cytosine in one of the two CpG dinucleotide sequences of the palindromic CpG methylation site is methylated (e.g., 5'-CC$^{M}$GG-3' (top strand): 3'-GGCC-5' (bottom strand)).

The term 'AUC' as used herein is an abbreviation for the area under a curve. In particular it refers to the area under a Receiver Operating Characteristic (ROC) curve. The ROC curve is a plot of the true positive rate against the false positive rate for the different possible cutpoints of a diagnostic test. It shows the tradeoff between sensitivity and specificity depending on the selected cutpoint (any increase in sensitivity will be accompanied by a decrease in specificity).The area under an ROC curve (AUC) is a measure for the accuracy of a diagnostic test (the larger the area the better, optimum is 1, a random test would have a ROC curve lying on the diagonal with an area of 0.5; for reference: J.P. Egan. Signal Detection Theory and ROC Analysis, Academic Press. New York, 1975)

The term "hypermethylation" refers to the average methylation state corresponding to an *increased* presence of 5-mCyt at one or a plurality of CpG dinucleotides within a DNA sequence of a test DNA sample, relative to the amount of 5-mCyt found at corresponding CpG dinucleotides within a normal control DNA sample.

The term "hypomethylation" refers to the average methylation state corresponding to a *decreased* presence of 5-mCyt at one or a plurality of CpG dinucleotides within a DNA sequence of a test DNA sample, relative to the amount of 5-mCyt found at corresponding CpG dinucleotides within a normal control DNA sample.

The term "microarray" refers broadly to both "DNA microarrays," and DNA chip(s),' as recognized in the art, encompasses all art-recognized solid supports, and encompasses all methods for affixing nucleic acid molecules thereto or synthesis of nucleic acids thereon.

"Genetic parameters" are mutations and polymorphisms of genes and sequences further required for their regulation. To be designated as mutations are, in particular, insertions, deletions, point mutations, inversions and polymorphisms and, particularly preferred, SNPs (single nucleotide polymorphisms).

"Epigenetic parameters" are, in particular, cytosine methylations. Further epigenetic parameters include, for example, the acetylation of histones which, however, cannot be directly analyzed using the described method but which, in turn, correlate with the DNA methylation.

The term "bisulfite reagent" refers to a reagent comprising bisulfite, disulfite, hydrogen sulfite or combinations thereof, useful as disclosed herein to distinguish between methylated and unmethylated CpG dinucleotide sequences.

The term "Methylation assay" refers to any assay for determining the methylation state of one or more CpG dinucleotide sequences within a sequence of DNA.

The term "MS.AP-PCR" (Methylation-Sensitive Arbitrarily-Primed Polymerase Chain Reaction) refers to the art-recognized technology that allows for a global scan of the genome using CG-rich primers to focus on the regions most likely to contain CpG dinucleotides, and described by Gonzalgo et al., Cancer Research 57:594-599, 1997.

The term "MethyLight™" refers to the art-recognized fluorescence-based real-time PCR technique described by Eads et al., Cancer Res. 59:2302-2306, 1999.

The term "HeavyMethyl™" assay, in the embodiment thereof implemented herein, refers to an assay, wherein methylation specific *blocking* probes (also referred to herein as *blockers*) covering CpG positions between, or covered by the amplification primers enable methylation-specific selective amplification of a nucleic acid sample.

The term "Heavymethyl™ MethyLight" assay, in the embodiment thereof implemented herein, refers to a HeavyMethyl™ MethyLight™ assay, which is a variation of the MethyLight™ assay, wherein the MethyLight™ assay is combined with methylation specific *blocking* probes covering CpG positions between the amplification primers.

The term "Ms-SNuPE" (Methylation-sensitive Single Nucleotide Primer Extension) refers to the art-recognized assay described by Gonzalgo & Jones, Nucleic Acids Res. 25:2529-2531, 1997.

The term "MSP" (Methylation-specific PCR) refers to the art-recognized methylation assay described by Herman et al. Proc. Natl. Acad. Sci. USA 93:9821-9826, 1996, and by US Patent No. 5,786,146.

The term "COBRA" (Combined Bisulfite Restriction Analysis) refers to the art-recognized methylation assay described by Xiong & Laird, Nucleic Acids Res. 25:2532-2534, 1997.

The term "MCA" (Methylated CpG Island Amplification) refers to the methylation assay described by Toyota et al., Cancer Res. 59:2307-12, 1999, and in WO 00/26401A1.

The term "hybridization" is to be understood as a bond of an oligonucleotide to a complementary sequence along the lines of the Watson-Crick base pairings in the sample DNA, forming a duplex structure.

"Stringent hybridization conditions," as defined herein, involve hybridizing at 68°C in 5x SSC/5x Denhardt's solution/1.0% SDS, and washing in 0.2x SSC/0.1 % SDS at room temperature, or involve the art-recognized equivalent thereof (e.g., conditions in which a hybridization is carried out at 60°C in 2.5 x SSC buffer, followed by several washing steps at 37°C in a low buffer concentration, and remains stable). Moderately stringent conditions, as defined herein, involve including washing in 3x SSC at 42°C, or the art-recognized equivalent thereof. The parameters of salt concentration and temperature can be varied to achieve the optimal level of identity between the probe and the target nucleic acid. Guidance regarding such conditions is available in the art, for example, by Sambrook et al., 1989, Molecular Cloning, A Laboratory Manual, Cold Spring Harbor Press, N.Y.; and Ausubel et al. (eds.), 1995, Current Protocols in Molecular Biology, (John Wiley & Sons, N.Y.) at Unit 2.10.

The terms "array SEQ ID NO," "composite array SEQ ID NO," or "composite array sequence" refer to a sequence, hypothetical or otherwise, consisting of a head-to-tail (5' to 3') linear composite of all individual contiguous sequences of a subject array (e.g., a head-to-tail composite of SEQ ID NO:1-71, in that order).

The terms "array SEQ ID NO node," "composite array SEQ ID NO node," or "composite array sequence node" refer to a *junction* between any two individual contiguous sequences of the "array SEQ ID NO," the "composite array SEQ ID NO," or the "composite array sequence."

In reference to composite array sequences, the phrase "contiguous nucleotides" refers to a contiguous sequence region of any individual contiguous sequence of the composite array, but does not include a region of the composite array sequence that includes a "node," as defined herein above.

Overview:

The present invention provides for molecular genetic markers that have novel utility for providing a prognosis of prostate cell proliferative disorders. In particular embodiments said markers may be used for classifying the tumor according to aggressiveness and/or invasiveness. It is particularly preferred that the method and nucleic acids according to the invention are utilised for at least one of: prognosis of; treatment of; monitoring of; and treatment and monitoring of prostate cell proliferative disorders.

The term 'prognosis' is taken to mean a prediction of outcome of disease progression (wherein the term progression shall be taken to also include recurrence after treatment). Prognosis may be expressed in terms of overall patient survival, disease- or relapse-free survival, increased tumor-related complications and rate of progression of tumour or metastases, wherein a decrease in any of said factors (with the exception of increased tumor-related complications rate of progression) as relative to a pre-determined level, is a 'negative' outcome and increase thereof is a 'positive' outcome. A decrease in tumor-related complications and/or rate of progression of tumour or metastases as relative to a pre-determined level, is considered a 'positive' outcome and increase thereof is a 'negative' outcome.

Hereinafter prognosis may also be referred to in terms of 'aggressiveness' wherein an aggressive cancer is determined to have a high risk of negative outcome and wherein a non-aggressive cancer has a low risk of negative outcome.

In one aspect the prognostic marker according to the present invention is used to provide an estimate of the risk of negative outcome. Characterisation of a prostate cancer in terms of predicted outcome enables the physician to determine the risk of recurrence and/or death. This aids in treatment selection as the absolute reduction of risk of recurrence and death after treatments such as adjuvant hormonal, chemo-, and radiation therapy can be determined based on the predicted negative outcome. The absolute reduction in risk attributable to treatment may then be compared to the drawbacks of said treatment (e.g. side effects, cost) in order to determine the suitability of said treatment for the patient.

Conversely, wherein a cancer is characterised as non-aggressive (i.e. positive outcome with low risk of death and/or recurrence) the patient will derive low absolute benefit from adjuvant or other treatment and may be appropriately treated by watchful waiting. Therein lies a great advantage of the present invention. By providing a means for determining which patients will not significantly benefit from treatment the present invention identifies suitable candidates for watchful waiting and prevents the over-prescription of therapies.

According to the predicted outcome (i.e. prognosis) of the disease an appropriate treatment or treatments may be selected. Wherein a cancer is characterised as aggressive it is particularly preferred that adjuvant treatment such as, but not limited to, hormonal, chemo- or radiation therapy is provided in addition to or instead of further treatments.

The herein described markers have further utility in predicting outcome of a patient after surgical treatment. This will hereinafter also be referred to as a 'predictive' marker. Over expression of the genes according to Table 11 (in particular FOXL2, SEQ ID NO: 35, SEQ ID NO: 63, HIST2H2BF, GPR7 and most preferably PITX2), are associated with negative outcome of prostate cancer patients. Patients with predicted positive outcome (i.e. hypomethylation or over-expression) after said treatment will accordingly have a decreased absolute reduction of risk of recurrence and death after treatment with post surgical adjuvant therapies. Patients with predicted negative outcome (i.e. hypermethylation) after said treatment will accordingly have a relatively larger absolute reduction of risk of recurrence and death after post surgical adjuvant treatment. Accordingly patients with a negative outcome after said treatment will be considered more suitable candidates for adjuvant treatment than patients with a positive outcome. Patients with a positive outcome may accordingly be prevented from over prescription of adjuvant treatment.

*Bisulfite modification of DNA is an art-recognized tool used to assess CpG methylation status.* 5-methylcytosine is the most frequent covalent base modification in the DNA of eukaryotic cells. It plays a role, for example, in the regulation of the transcription, in genetic imprinting, and in tumorigenesis. Therefore, the identification of 5-methylcytosine as a component of genetic information is of considerable interest. However, 5-methylcytosine positions cannot be identified by sequencing, because 5-methylcytosine has the same base pairing behavior as cytosine. Moreover, the epigenetic information carried by 5-methylcytosine is completely lost during, *e.g.*, PCR amplification.

The most frequently used method for analyzing DNA for the presence of 5-methylcytosine is based upon the specific reaction of bisulfite with cytosine whereby, upon subsequent alkaline hydrolysis, cytosine is converted to uracil, which corresponds to thymine in its base pairing behavior. Significantly, however, 5-methylcytosine remains unmodified under these conditions. Consequently, the original DNA is *converted* in such a manner that methylcytosine, which originally could not be distinguished from cytosine by its hybridization behavior, can now be detected as the only remaining cytosine using standard, art-recognized molecular biological techniques, for example, by amplification and hybridization, or by sequencing. All of these techniques are based on differential base pairing properties, which can now be fully exploited.

The prior art, in terms of sensitivity, is defined by a method comprising enclosing the DNA to be analyzed in an agarose matrix, thereby preventing the diffusion and renaturation of the DNA (bisulfite only reacts with single-stranded DNA), and replacing all precipitation and purification steps with fast dialysis (Olek A, et al., A modified and improved method for bisulfite based cytosine methylation analysis, Nucleic Acids Res. 24:5064-6, 1996). It is thus possible to analyze individual cells for methylation status, illustrating the utility and sensitivity of the method. An overview of art-recognized methods for detecting 5-methylcytosine is provided by Rein, T., et al., Nucleic Acids Res., 26:2255, 1998.

The bisulfite technique, barring few exceptions (*e.g.*, Zeschnigk M, et al., Eur J Hum Genet. 5:94-98, 1997), is currently only used in research. In all instances, short, specific fragments of a known gene are amplified subsequent to a bisulfite treatment, and either completely sequenced (Olek. & Walter, Nat Genet. 1997 17:275-6, 1997), subjected to one or more primer extension reactions (Gonzalgo & Jones, Nucleic Acids Res., 25:2529-31, 1997; WO 95/00669; U.S. Patent No. 6,251,594) to analyze individual cytosine positions, or treated by enzymatic digestion (Xiong & Laird, Nucleic Acids Res., 25:2532-4, 1997). Detection by hybridization has also been described in the art (Olek et al., WO 99/28498). Additionally, use of the bisulfite technique for methylation detection with respect to individual genes has been described (Grigg & Clark, Bioessays, 16:431-6, 1994; Zeschnigk M, et al., Hum Mol Genet., 6:387-95, 1997; Feil R, et al., Nucleic Acids Res., 22:695-, 1994; Martin V, et al., Gene, 157:261-4, 1995; WO 9746705 and WO 9515373).

The present invention provides for the use of the bisulfite technique , in combination with one or more methylation assays, for determination of the methylation status of CpG dinuclotide sequences within sequences from the group consisting of SEQ ID NO:1 to SEQ ID NO:64 and SEQ ID NO: 961. Preferably said group consists of SEQ ID Nos: 35, 63, 19 and most preferably said sequence is SEQ ID NO: 961 According to the present invention, determination of the methylation status of CpG dinuclotide sequences within sequences from the group consisting of SEQ ID NO:1 to SEQ ID NO:64 and SEQ ID NO: 961 and SEQ ID NO: 961 has prognostic utility.

*Methylation Assay Procedures.* Various methylation assay procedures are known in the art, and can be used in conjunction with the present invention. These assays allow for determination of the methylation state of one or a plurality of CpG dinucleotides (*e.g.*, CpG islands) within a DNA sequence. Such assays involve, among other techniques, DNA sequencing of bisulfite-treated DNA, PCR (for sequence-specific amplification), Southern blot analysis, and use of methylation-sensitive restriction enzymes.

For example, genomic sequencing has been simplified for analysis of DNA methylation patterns and 5-methylcytosine distribution by using bisulfite treatment (Frommer et al., Proc. Natl. Acad. Sci. USA 89:1827-1831, 1992). Additionally, restriction enzyme digestion of PCR products amplified from bisulfite-converted DNA is used, *e.g.*, the method described by Sadri & Hornsby (Nucl. Acids Res. 24:5058-5059, 1996), or COBRA (Combined Bisulfite Restriction Analysis) (Xiong & Laird, Nucleic Acids Res. 25:2532-2534, 1997).

COBRA. COBRA analysis is a quantitative methylation assay useful for determining DNA methylation levels at specific gene loci in small amounts of genomic DNA (Xiong & Laird, Nucleic Acids Res. 25:2532-2534, 1997). Briefly, restriction enzyme digestion is used to reveal methylation-dependent sequence differences in PCR products of sodium bisulfite-treated DNA. Methylation-dependent sequence differences are first introduced into the genomic DNA by standard bisulfite treatment according to the procedure described by Frommer et al. (Proc. Natl. Acad. Sci. USA 89:1827-1831, 1992). PCR amplification of the bisulfite converted DNA is then performed using primers specific for the CpG islands of interest, followed by restriction endonuclease digestion, gel electrophoresis, and detection using specific, labeled hybridization probes. Methylation levels in the original DNA sample are represented by the relative amounts of digested and undigested PCR product in a linearly quantitative fashion across a wide spectrum of DNA methylation levels. In addition, this technique can be reliably applied to DNA obtained from microdissected paraffin-embedded tissue samples. Typical reagents (*e.g.*, as might be found in a typical COBRA-based kit) for COBRA analysis may include, but are not limited to: PCR primers for specific gene (or bisulfite treated DNA sequence or CpG island); restriction enzyme and appropriate buffer; gene-hybridization oligo; control hybridization oligo; kinase labeling kit for oligo probe; and labelled nucleotides. Additionally, bisulfite conversion reagents may include: DNA denaturation buffer; sulfonation buffer; DNA recovery reagents or kits (*e.g.*, precipitation, ultrafiltration, affinity column); desulfonation buffer; and DNA recovery components.

Preferably, assays such as "MethyLight™" (a fluorescence-based real-time PCR technique) (Eads et al., Cancer Res. 59:2302-2306, 1999), Ms-SNuPE (Methylation-sensitive Single Nucleotide Primer Extension) reactions (Gonzalgo & Jones, Nucleic Acids Res. 25:2529-2531, 1997), methylation-specific PCR ("MSP"; Herman et al., Proc. Natl. Acad. Sci. USA 93:9821-9826, 1996; US Patent No. 5,786,146), and methylated CpG island amplification ("MCA"; Toyota et al., Cancer Res. 59:2307-12, 1999) are used alone or in combination with other of these methods.

*MethyLight™.* The MethyLight™ assay is a high-throughput quantitative methylation assay that utilizes fluorescence-based real-time PCR (TaqMan™) technology that requires no further manipulations after the PCR step (Eads et al., Cancer Res. 59:2302-2306, 1999). Briefly, the MethyLight™ process begins with a mixed sample of genomic DNA that is converted, in a sodium bisulfite reaction, to a mixed pool of methylation-dependent sequence differences according to standard procedures (the bisulfite process converts unmethylated cytosine residues to uracil). Fluorescence-based PCR is then performed either in an "unbiased" (with primers that do not overlap known CpG methylation sites) PCR reaction, or in a "biased" (with PCR primers that overlap known CpG dinucleotides) reaction. Sequence discrimination can occur either at the level of the amplification process or at the level of the fluorescence detection process, or both.

The MethyLight™ assay may be used as a quantitative test for methylation patterns in the genomic DNA sample, wherein sequence discrimination occurs at the level of probe hybridization. In this quantitative version, the PCR reaction provides for unbiased amplification in the presence of a fluorescent probe that overlaps a particular putative methylation site. An unbiased control for the amount of input DNA is provided by a reaction in which neither the primers, nor the probe overlie any CpG dinucleotides. Alternatively, a qualitative test for genomic methylation is achieved by probing of the biased PCR pool with either control oligonucleotides that do not "cover" known methylation sites (a fluorescence-based version of the "MSP" technique), or with oligonucleotides covering potential methylation sites.

The MethyLight™ process can by used with a "TaqMan®" probe in the amplification process. For example, double-stranded genomic DNA is treated with sodium bisulfite and subjected to one of two sets of PCR reactions using TaqMan® probes; *e.g.*, with either biased primers and TaqMan® probe, or unbiased primers and TaqMan® probe. The TaqMan® probe is dual-labeled with fluorescent "reporter" and "quencher" molecules, and is designed to be specific for a relatively high GC content region so that it melts out at about 10°C higher temperature in the PCR cycle than the forward or reverse primers. This allows the TaqMan® probe to remain fully hybridized during the PCR annealing/extension step. As the Taq polymerase enzymatically synthesizes a new strand during PCR, it will eventually reach the annealed TaqMan® probe. The Taq polymerase 5' to 3' endonuclease activity will then displace the TaqMan® probe by digesting it to release the fluorescent reporter molecule for quantitative detection of its now unquenched signal using a real-time fluorescent detection system.

Typical reagents (*e.g.*, as might be found in a typical MethyLightTM-based kit) for MethyLightTM analysis may include, but are not limited to: PCR primers for specific gene (or bisulfite treated DNA sequence or CpG island); TaqMan® probes; optimized PCR buffers and deoxynucleotides; and Taq polymerase.

*Ms-SNuPE.* The Ms-SNuPE technique is a quantitative method for assessing methylation differences at specific CpG sites based on bisulfite treatment of DNA, followed by single-nucleotide primer extension (Gonzalgo & Jones, Nucleic Acids Res. 25:2529-2531, 1997). Briefly, genomic DNA is reacted with sodium bisulfite to convert unmethylated cytosine to uracil while leaving 5-methylcytosine unchanged. Amplification of the desired target sequence is then performed using PCR primers specific for bisulfite-converted DNA, and the resulting product is isolated and used as a template for methylation analysis at the CpG site(s) of interest. Small amounts of DNA can be analyzed (*e.g.*, microdissected pathology sections), and it avoids utilization of restriction enzymes for determining the methylation status at CpG sites.

Typical reagents (*e.g.*, as might be found in a typical Ms-SNuPE-based kit) for Ms-SNuPE analysis may include, but are not limited to: PCR primers for specific gene (or bisulfite treated DNA sequence or CpG island); optimized PCR buffers and deoxynucleotides; gel extraction kit; positive control primers; Ms-SNuPE primers for specific gene; reaction buffer (for the Ms-SNuPE reaction); and labelled nucleotides. Additionally, bisulfite conversion reagents may include: DNA denaturation buffer; sulfonation buffer; DNA recovery regents or kit (*e.g.*, precipitation, ultrafiltration, affinity column); desulfonation buffer; and DNA recovery components.

*MSP.* MSP (methylation-specific PCR) allows for assessing the methylation status of virtually any group of CpG sites within a CpG island, independent of the use of methylation-sensitive restriction enzymes (Herman et al. Proc. Natl. Acad. Sci. USA 93:9821-9826, 1996; US Patent No. 5,786,146). Briefly, DNA is modified by sodium bisulfite converting all unmethylated, but not methylated cytosines to uracil, and subsequently amplified with primers specific for methylated versus unmethylated DNA. MSP requires only small quantities of DNA, is sensitive to 0.1 % methylated alleles of a given CpG island locus, and can be performed on DNA extracted from paraffin-embedded samples. Typical reagents (*e.g.*, as might be found in a typical MSP-based kit) for MSP analysis may include, but are not limited to: methylated and unmethylated PCR primers for specific gene (or bisulfite treated DNA sequence or CpG island), optimized PCR buffers and deoxynucleotides, and specific probes.

MCA. The MCA technique is a method that can be used to screen for altered methylation patterns in genomic DNA, and to isolate specific sequences associated with these changes (Toyota et al., Cancer Res. 59:2307-12, 1999). Briefly, restriction enzymes with different sensitivities to cytosine methylation in their recognition sites are used to digest genomic DNAs from primary tumors, cell lines, and normal tissues prior to arbitrarily primed PCR amplification. Fragments that show differential methylation are cloned and sequenced after resolving the PCR products on high-resolution polyacrylamide gels. The cloned fragments are then used as probes for Southern analysis to confirm differential methylation of these regions. Typical reagents (*e.g.*, as might be found in a typical MCA-based kit) for MCA analysis may include, but are not limited to: PCR primers for arbitrary priming Genomic DNA; PCR buffers and nucleotides, restriction enzymes and appropriate buffers; gene-hybridization oligos or probes; control hybridization oligos or probes.

Genomic Sequences According to SEQ ID NOS:1-64 and SEQ ID NO: 961 (preferably SEQ ID Nos: 35, 63, 19 and most preferably SEQ ID NO: 961), and Non-naturally Occuring Treated Variants Thereof According to SEQ ID NOS:65-320 and SEQ ID Nos: 962 - 965 (preferably SEQ ID Nos: 133,134,261,262, 189,190,317,318, 101,102,229,230 and most preferably SEQ ID Nos: 962-965), were Determined to have Utility for Providing a Prognosis and/or Treatment of Prostate Cell Proliferative Disorders.

In one embodiment the invention provides a method for providing a prognosis of prostate cell proliferative disorders in a subject. In a particularly preferred embodiment the invention provides a method for the classification based on aggressiveness of a prostate cell proliferative disorder.

Said method comprises the following steps:

i) determining the expression levels of one or more genes or gene sequences according to Table 11 and/or regulatory regions thereof; and
ii) determining the prognosis of said prostate cell proliferative disorders according to said level of expression.

Said expression level may be determined by any means standard in the art including but not limited to methylation analysis, loss of heterozygosity (hereinafter also referred to as LOH), RNA expression levels and protein expression levels.

Accordingly said method may be enabled by means of any analysis of the expression of a RNA transcribed therefrom or polypeptide or protein translated from said RNA, preferably by means of mRNA expression analysis or polypeptide expression analysis. Accordingly the present invention also provides prognostic assays and methods, both quantitative and qualitative for detecting the expression of the genes, genomic sequences and/or regulatory regions according to Table 11 in a subject with a prostate carcinoma or neoplasms and determining therefrom upon the prognosis and/or prediction of treatment outcome in said subject. It is particularly preferred that said genes, genomic sequences and/or regulatory regions are selected from the group consisting PITX2, SEQ ID NO: 63, GPR7 and SEQ ID NO: 35. Further preferred is the gene PITX2.

Aberrant expression of mRNA transcribed from the genes or genomic regions according to Table 11, in particular SEQ ID NO: 35, SEQ ID NO: 63 and GPR7 and most preferably PITX2 are associated with prognosis and/or prediction of treatment outcome of prostate carcinoma. To detect the presence of mRNA encoding a gene or genomic sequence, a sample is obtained from a patient. The sample may be any suitable sample comprising cellular matter of the tumour, most preferably the primary tumour. Suitable sample types include tumours cells or cell lines, histological slides, paraffin embedded tissues, biopsies, tissue embedded in paraffin, bodily fluids (such as but not limited to prostatic massage fluid and urine) or any other suitable biological sample and all possible combinations thereof.

In a particularly preferred embodiment of the method said source is primary tumour tissue. The sample may be treated to extract the RNA contained therein. The resulting nucleic acid from the sample is then analysed. Many techniques are known in the state of the art for determining absolute and relative levels of gene expression, commonly used techniques suitable for use in the present invention include in situ hybridisation (e.g. FISH), Northern analysis, RNase protection assays (RPA), microarrays and PCR-based techniques, such as quantitative PCR and differential display PCR or any other nucleic acid detection method.

Particularly preferred is the use of the reverse transcription/polymerisation chain reaction technique (RT-PCR). The method of RT-PCR is well known in the art (for example, see Watson and Fleming, supra).

The RT-PCR method can be performed as follows. Total cellular RNA is isolated by, for example, the standard guanidium isothiocyanate method and the total RNA is reverse transcribed. The reverse transcription method involves synthesis of DNA on a template of RNA using a reverse transcriptase enzyme and a 3' end oligo dT primer and/or random hexamer primers. The cDNA thus produced is then amplified by means of PCR. (Belyavsky et al, Nucl Acid Res 17:2919-2932, 1989; Krug and Berger, Methods in Enzymology, Academic Press, N.Y., Vol.152, pp. 316-325, 1987 which are incorporated by reference). Further preferred is the "Real-time" variant of RT- PCR, wherein the PCR product is detected by means of hybridisation probes (E.g TaqMan, Lightcycler, Molecular Beacons & Scorpion) or SYBR green. The detected signal from the probes or SYBR green is then quantitated either by reference to a standard curve or by comparing the Ct values to that of a calibration standard. Analysis of housekeeping genes is often used to normalize the results.

In Northern blot analysis total or polv(A)+ mRNA is run on a denaturing agarose gel and detected by hybridization to a labelled probe in the dried gel itself or on a membrane. The resulting signal is proportional to the amount of target RNA in the RNA population.

Comparing the signals from two or more cell populations or tissues reveals relative differences in gene expression levels. Absolute quantitation can be performed by comparing the signal to a standard curve generated using known amounts of an in vitro transcript corresponding to the target RNA. Analysis of housekeeping genes, genes whose expression levels are expected to remain relatively constant regardless of conditions, is often used to normalize the results, eliminating any apparent differences caused by unequal transfer of RNA to the membrane or unequal loading of RNA on the gel.

The first step in Northern analysis is isolating pure, intact RNA from the cells or tissue of interest. Because Northern blots distinguish RNAs by size, sample integrity influences the degree to which a signal is localized in a single band. Partially degraded RNA samples will result in the signal being smeared or distributed over several bands with an overall loss in sensitivity and possibly an erroneous interpretation of the data. In Northern blot analysis, DNA, RNA and oligonucleotide probes can be used and these probes are preferably labelled (e.g. radioactive labels, massa labels or fluorescent labels). The size of the target RNA, not the probe, will determine the size of the detected band, so methods such as random-primed labeling, which generates probes of variable lengths, are suitable for probe synthesis. The specific activity of the probe will determine the level of sensitivity, so it is preferred that probes with high specific activities, are used..

In an RNase protection assay, the RNA target and an RNA probe of a defined length are hybridized in solution. Following hybridization, the RNA is digested with RNases specific for single-stranded nucleic acids to remove any unhybridized, single-stranded target RNA and probe. The RNases are inactivated, and the RNA is separated e.g. by denaturing polyacrylamide gel electrophoresis. The amount of intact RNA probe is proportional to the amount of target RNA in the RNA population. RPA can be used for relative and absolute quantitation of gene expression and also for mapping RNA structure, such as intron/exon boundaries and transcription start sites. The RNase protection assay is preferable to Northern blot analysis as it generally has a lower limit of detection.

The antisense RNA probes used in RPA are generated by in vitro transcription of a DNA template with a defined endpoint and are typically in the range of 50-600 nucleotides. The use of RNA probes that include additional sequences not homologous to the target RNA allows the protected fragment to be distinguished from the full-length probe. RNA probes are typically used instead of DNA probes due to the ease of generating single-stranded RNA probes and the reproducibility and reliability of RNA:RNA duplex digestion with RNases (Ausubel *et al.* 2003), particularly preferred are probes with high specific activities.

Particularly preferred is the use of microarrays. The microarray analysis process can be divided into two main parts. First is the immobilization of known gene sequences onto glass slides or other solid support followed by hybridization of the fluorescently labelled cDNA (comprising the sequences to be interrogated) to the known genes immobilized on the glass slide. After hybridization, arrays are scanned using a fluorescent microarray scanner. Analyzing the relative fluorescent intensity of different genes provides a measure of the differences in gene expression.

DNA arrays can be generated by immobilizing presynthesized oligonucleotides onto prepared glass slides. In this case, representative gene sequences are manufactured and prepared using standard oligonucleotide synthesis and purification methods. These synthesized gene sequences are complementary to the genes of interest (most preferably PITX2) and tend to be shorter sequences in the range of 25-70 nucleotides. Alternatively, immobilized oligos can be chemically synthesized in-situ on the surface of the slide. In situ oligonucleotide synthesis involves the consecutive addition of the appropriate nucleotides to the spots on the microarray; spots not receiving a nucleotide are protected during each stage of the process using physical or virtual masks.

In expression profiling microarray experiments, the RNA templates used are representative of the transcription profile of the cells or tissues under study. RNA is first isolated from the cell populations or tissues to be compared. Each RNA sample is then used as a template to generate fluorescently labelled cDNA via a reverse transcription reaction. Fluorescent labeling of the cDNA can be accomplished by either direct labeling or indirect labeling methods. During direct labeling, fluorescently modified nucleotides (e.g., Cy®3- or Cy®5-dCTP) are incorporated directly into the cDNA during the reverse transcription. Alternatively, indirect labeling can be achieved by incorporating aminoallyl-modified nucleotides during cDNA synthesis and then conjugating an N-hydroxysuccinimide (NHS)-ester dye to the aminoallyl-modified cDNA after the reverse transcription reaction is complete. Alternatively, the probe may be unlabelled, but may be detectable by specific binding with a ligand which is labelled, either directly or indirectly. Suitable labels and methods for labelling ligands (and probes) are known in the art, and include, for example, radioactive labels which may be incorporated by known methods (e.g., nick translation or kinasing). Other suitable labels include but are not limited to biotin, fluorescent groups, chemiluminescent groups (e.g., dioxetanes, particularly triggered dioxetanes), enzymes, antibodies, and the like.

To perform differential gene expression analysis, cDNA generated from different RNA samples are labelled with Cy®3. The resulting labelled cDNA is purified to remove unincorporated nucleotides, free dye and residual RNA. Following purification, the labeled cDNA samples are hybridised to the microarray. The stringency of hybridisation is determined by a number of factors during hybridisation and during the washing procedure, including temperature, ionic strength, length of time and concentration of formamide. These factors are outlined in, for example, Sambrook et al. (Molecular Cloning: A Laboratory Manual, 2nd ed., 1989). The microarray is scanned post-hybridization using a fluorescent microarray scanner. The fluorescent intensity of each spot indicates the level of expression for that gene; bright spots correspond to strongly expressed genes, while dim spots indicate weak expression..

Once the images are obtained, the raw data must be analyzed. First, the background fluorescence must be subtracted from the fluorescence of each spot. The data is then normalized to a control sequence, such as an exogenously added RNA, or a housekeeping gene panel to account for any nonspecific hybridization, array imperfections or variability in the array setup, cDNA labeling, hybridization or washing. Data normalization allows the results of multiple arrays to be compared.

The present invention further provides for methods for the detection of the presence of the polypeptide encoded by said gene sequences in a sample obtained from a patient.

Aberrant levels of polypeptide expression of the polypeptides encoded by the genes and/or genomic regions according to Table 11 (in particular SEQ ID NO: 35, SEQ ID NO: 63, GPR7 and most preferably PITX2) are associated with prostate cancer and neoplasms prognosis and/or treatment outcome.

Any method known in the art for detecting polypeptides can be used. Such methods include, but are not limited to masss-spectrometry, immunodiffusion, immunoelectrophoresis, immunochemical methods, binder-ligand assays, immunohistochemical techniques, agglutination and complement assays (e.g., see Basic and Clinical Immunology, Sites and Terr, eds., Appleton & Lange, Norwalk, Conn. pp 217-262, 1991 which is incorporated by reference). Preferred are binder-ligand immunoassay methods including reacting antibodies with an epitope or epitopes and competitively displacing a labelled polypeptide or derivative thereof.

Certain embodiments of the present invention comprise the use of antibodies specific to the polypeptide encoded by the genes and/or genomic regions according to Table 11 (in particular SEQ ID NO: 35, SEQ ID NO: 63, GPR7 and most preferably PITX2).

Such antibodies are useful for prostate cancer prognostic and/or predictive applications. In certain embodiments production of monoclonal or polyclonal antibodies can be induced by the use of the coded polypeptide as an antigene. Such antibodies may in turn be used to detect expressed polypeptides as markers for prostate cancer prognosis. The levels of such polypeptides present may be quantified by conventional methods. Antibody-polypeptide binding may be detected and quantified by a variety of means known in the art, such as labelling with fluorescent or radioactive ligands. The invention further comprises kits for performing the above-mentioned procedures, wherein such kits contain antibodies specific for the investigated polypeptides.

Numerous competitive and non-competitive polypeptide binding immunoassays are well known in the art. Antibodies employed in such assays may be unlabelled, for example as used in agglutination tests, or labelled for use a wide variety of assay methods. Labels that can be used include radionuclides, enzymes, fluorescers, chemiluminescers, enzyme substrates or co-factors, enzyme inhibitors, particles, dyes and the like. Preferred assays include but are not limited to radioimmunoassay (RIA), enzyme immunoassays, e.g., enzyme-linked immunosorbent assay (ELISA), fluorescent immunoassays and the like. Polyclonal or monoclonal antibodies or epitopes thereof can be made for use in immunoassays by any of a number of methods known in the art.

In an alternative embodiment of the method the proteins may be detected by means of western blot analysis. Said analysis is standar in the art, briefly proteins are separated by means of electrophoresis e.g. SDS-PAGE. The separated proteins are then transferred to a suitable membrane (or paper) e.g. nitrocellulose, retaining the spacial separation achieved by electrophoresis. The membrane is then incubated with a generic protein (e.g. milk protein) to bind remaining sticky places on the membrane. An antibody specific to the protein of interest is then added, said antibody being detectably labelled for example by dyes or enzymatic means (e.g. alkaline phosphatase or horseradish peroxidase) . The location of the antibody on the membrane is then detected.

In an alternative embodiment of the method the proteins may be detected by means of immunohistochemistry (the use of antibodies to probe specific antigens in a sample). Said analysis is standard in the art, wherein detection of antigens in tissues is known as immunohistochemistry, while detection in cultured cells is generally termed immunocytochemistry. Briefly the primary antibody to be detected by binding to its specific antigen. The antibody-antigen complex is then bound by a secondary enzyme conjugated antibody. In the presence of the necessary substrate and chromogen the bound enzyme is detected according to colored deposits at the antibody-antigen binding sites. There is a wide range of suitable sample types, antigen-antibody affinity, antibody types, and detection enhancement methods. Thus optimal conditions for immunohistochemical or immunocytochemical detection must be determined by the person skilled in the art for each individual case.

One approach for preparing antibodies to a polypeptide is the selection and preparation of an amino acid sequence of all or part of the polypeptide, chemically synthesising the amino acid sequence and injecting it into an appropriate animal, usually a rabbit or a mouse (Milstein and Kohler Nature 256:495-497, 1975; Gulfre and Milstein, Methods in Enzymology: Immunochemical Techniques 73:1-46, Langone and Banatis eds., Academic Press, 1981 which are incorporated by reference). Methods for preparation of the polypeptides or epitopes thereof include, but are not limited to chemical synthesis, recombinant DNA techniques or isolation from biological samples.

In the final step of the method the prognosis of the patient is determined, whereby overexpression is indicative of negative prognosis. The term overexpression shall be taken to mean expression at a detected level greater than a pre-determined cut off which may be selected from the group consisting of the mean, median or an optimised threshold value.

Another aspect of the invention provides a kit for use in providing a prognosis of a subject with prostate cancer, comprising: a means for detecting polypeptides of a gene or genomic region according to Table 11 (in particular SEQ ID NO: 35, SEQ ID NO: 63 and GPR7 and most preferably PITX2). The means for detecting the polypeptides comprise preferably antibodies, antibody derivatives, or antibody fragments. The polypeptides are most preferrably detected by means of Western blotting utilizing a labelled antibody. In another embodiment of the invention the kit further comprising means for obtaining a biological sample of the patient. Preferred is a kit, which further comprises a container suitable for containing the means for detecting the polypeptides in the biological sample of the patient, and most preferably further comprises instructions for use and interpretation of the kit results. In a preferred embodiment the kit for use in determining treatment strategy for a patient with prostate cancer or neoplasms, comprises: (a) a means for detecting polypeptides of a gene or genomic region according to Table 11 (in particular SEQ ID NO: 35, SEQ ID NO: 63 and GPR7 and most preferably PITX2); (b) a container suitable for containing the said means and the biological sample of the patient comprising the polypeptides wherein the means can form complexes with the polypeptides; (c) a means to detect the complexes of (b); and optionally (d) instructions for use and interpretation of the kit results.

The kit may also contain other components such as buffers or solutions suitable for blocking, washing or coating , packaged in a separate container.

Another aspect of the invention relates to a kit for use in providing a prognosis of a subject with prostate cancer, said kit comprising: a means for measuring the level of transcription of a gene or genomic region according to Table 11 (in particular SEQ ID NO: 35, SEQ ID NO: 63 and GPR7 and most preferably PITX2). In a preferred embodiment the means for measuring the level of transcription comprise oligonucleotides or polynucleotides able to hybridise under stringent or moderately stringent conditions to the transcription products of a gene or genomic region according to Table 11 (in particular SEQ ID NO: 35, SEQ ID NO: 63 and GPR7 and most preferably PITX2). In a most preferred embodiment the level of transcription is determined by techniques selected from the group of Northern blot analysis, reverse transcriptase PCR, real-time PCR, RNAse protection, and microarray. In another embodiment of the invention the kit further comprises means for obtaining a biological sample of the patient. Preferred is a kit, which further comprises a container suitable for containing the means for measuring the level of transcription and the biological sample of the patient, and most preferably further comprises instructions for use and interpretation of the kit results.

In a preferred embodiment the kit for use in determining treatment strategy for a patient with prostate cancer comprises (a) a plurality of oligonucleotides or polynucleotides able to hybridise under stringent or moderately stringent conditions to the transcription products of a gene or genomic region according to Table 11 (in particular SEQ ID NO: 35, SEQ ID NO: 63 and GPR7 and most preferably PITX2); (b) a container suitable for containing the oligonucleotides or polynucleotides and a biological sample of the patient comprising the transcription products wherein the oligonucleotides or polynucleotide can hybridise under stringent or moderately stringent conditions to the transcription products, (c) means to detect the hybridisation of (b); and optionally, (d) instructions for use and interpretation of the kit results.

The kit may also contain other components such as hybridization buffer (where the oligonucleotides are to be used as a probe) packaged in a separate container. Alternatively, where the oligonucleotides are to be used to amplify a target region, the kit may contain, packaged in separate containers, a polymerase and a reaction buffer optimized for primer extension mediated by the polymerase, such as PCR.

Most preferably a kit according to the embodiments of the present invention is used for the determination of expression step of the methods according to other aspects of the invention.

In a further aspect, the invention provides a further method for providing a prognosis of a subject with prostate cancer comprising the following steps. In the first step of the method a sample is obtained from the subject. Suitable sample types include tumours cells or cell lines, histological slides, paraffin embedded tissues, biopsies, tissue embedded in paraffin, bodily fluids (such as but not limited to prostatic massage fluid and urine) or any other suitable biological sample and all possible combinations thereof.. Commonly used techniques suitable for use in the present invention include in situ hybridisation (e.g. FISH), Northern analysis, RNase protection assays (RPA), microarrays and PCR-based techniques, such as quantitative PCR and differential display PCR or any other nucleic acid detection method.

Particularly preferred is the use of the reverse transcription/polymerisation chain reaction technique (RT-PCR). The method of RT-PCR is well known in the art (for example, see Watson and Fleming, supra).

The RT-PCR method can be performed as follows. Total cellular RNA is isolated by, for example, the standard guanidium isothiocyanate method and the total RNA is reverse transcribed. The reverse transcription method involves synthesis of DNA on a template of RNA using a reverse transcriptase enzyme and a 3' end oligo dT primer and/or random hexamer primers. The cDNA thus produced is then amplified by means of PCR. (Belyavsky et al, Nucl Acid Res 17:2919-2932, 1989; Krug and Berger, Methods in Enzymology, Academic Press, N.Y., Vol.152, pp. 316-325, 1987 which are incorporated by reference). Further preferred is the "Real-time" variant of RT- PCR, wherein the PCR product is detected by means of hybridisation probes (E.g TaqMan, Lightcyder. Molecular Beacons & Scorpion) or SYBR green. The detected signal from the probes or SYBR green is then quantitated either by reference to a standard curve or by comparing the Ct values to that of a calibration standard. Analysis of housekeeping genes is often used to normalize the results.

In Northern blot analysis total or poly(A)+ mRNA is run on a denaturing agarose gel and detected by hybridization to a labelled probe in the dried gel itself or on a membrane. The resulting signal is proportional to the amount of target RNA in the RNA population.

Comparing the signals from two or more cell populations or tissues reveals relative differences in gene expression levels. Absolute quantitation can be performed by comparing the signal to a standard curve generated using known amounts of an in vitro transcript corresponding to the target RNA. Analysis of housekeeping genes, genes whose expression levels are expected to remain relatively constant regardless of conditions, is often used to normalize the results, eliminating any apparent differences caused by unequal transfer of RNA to the membrane or unequal loading of RNA on the gel.

The first step in Northern analysis is isolating pure, intact RNA from the cells or tissue of interest. Because Northern blots distinguish RNAs by size, sample integrity influences the degree to which a signal is localized in a single band. Partially degraded RNA samples will result in the signal being smeared or distributed over several bands with an overall loss in sensitivity and possibly an erroneous interpretation of the data. In Northern blot analysis, DNA, RNA and oligonucleotide probes can be used and these probes are preferably labelled (e.g. radioactive labels, massa labels or fluorescent labels). The size of the target RNA, not the probe, will determine the size of the detected band, so methods such as random-primed labeling, which generates probes of variable lengths, are suitable for probe synthesis. The specific activity of the probe will determine the level of sensitivity, so it is preferred that probes with high specific activities, are used..

In an RNase protection assay, the RNA target and an RNA probe of a defined length are hybridized in solution. Following hybridization, the RNA is digested with RNases specific for single-stranded nucleic acids to remove any unhybridized, single-stranded target RNA and probe. The RNases are inactivated, and the RNA is separated e.g. by denaturing polyacrylamide gel electrophoresis. The amount of intact RNA probe is proportional to the amount of target RNA in the RNA population. RPA can be used for relative and absolute quantitation of gene expression and also for mapping RNA structure, such as intron/exon boundaries and transcription start sites. The RNase protection assay is preferable to Northern blot analysis as it generally has a lower limit of detection.

The antisense RNA probes used in RPA are generated by in vitro transcription of a DNA template with a defined endpoint and are typically in the range of 50-600 nucleotides. The use of RNA probes that include additional sequences not homologous to the target RNA allows the protected fragment to be distinguished from the full-length probe. RNA probes are typically used instead of DNA probes due to the ease of generating single-stranded RNA probes and the reproducibility and reliability of RNA:RNA duplex digestion with RNases (Ausubel *et al.* 2003), particularly preferred are probes with high specific activities.

Particularly preferred is the use of microarrays. The microarray analysis process can be divided into two main parts. First is the immobilization of known gene sequences onto glass slides or other solid support followed by hybridization of the fluorescently labelled cDNA (comprising the sequences to be interrogated) to the known genes immobilized on the glass slide. After hybridization, arrays are scanned using a fluorescent microarray scanner. Analyzing the relative fluorescent intensity of different genes provides a measure of the differences in gene expression.

DNA arrays can be generated by immobilizing presynthesized oligonucleotides onto prepared glass slides. In this case, representative gene sequences are manufactured and prepared using standard oligonucleotide synthesis and purification methods. These synthesized gene sequences are complementary to the genes of interest (such as PITX2 or other genes according to Table 11) and tend to be shorter sequences in the range of 25-70 nucleotides. Alternatively, immobilized oligos can be chemically synthesized in situ on the surface of the slide. In situ oligonucleotide synthesis involves the consecutive addition of the appropriate nucleotides to the spots on the microarray; spots not receiving a nucleotide are protected during each stage of the process using physical or virtual masks.

In expression profiling microarray experiments, the RNA templates used are representative of the transcription profile of the cells or tissues under study. RNA is first isolated from the cell populations or tissues to be compared. Each RNA sample is then used as a template to generate fluorescently labelled cDNA via a reverse transcription reaction. Fluorescent labeling of the cDNA can be accomplished by either direct labeling or indirect labeling methods. During direct labeling, fluorescently modified nucleotides (e.g., Cy®3- or Cy®5-dCTP) are incorporated directly into the cDNA during the reverse transcription. Alternatively, indirect labeling can be achieved by incorporating aminoallyl-modified nucleotides during cDNA synthesis and then conjugating an N-hydroxysuccinimide (NHS)-ester dye to the aminoallyl-modified cDNA after the reverse transcription reaction is complete. Alternatively, the probe may be unlabelled, but may be detectable by specific binding with a ligand which is labelled, either directly or indirectly. Suitable labels and methods for labelling ligands (and probes) are known in the art, and include, for example, radioactive labels which may be incorporated by known methods (e.g., nick translation or kinasing). Other suitable labels include but are not limited to biotin, fluorescent groups, chemiluminescent groups (e.g., dioxetanes, particularly triggered dioxetanes), enzymes, antibodies, and the like.

To perform differential gene expression analysis, cDNA generated from different RNA samples are labelled with Cy®3. The resulting labelled cDNA is purified to remove unincorporated nucleotides, free dye and residual RNA. Following purification, the labeled cDNA samples are hybridised to the microarray. The stringency of hybridisation is determined by a number of factors during hybridisation and during the washing procedure, including temperature, ionic strength, length of time and concentration of formamide.

These factors are outlined in, for example, Sambrook et al. (Molecular Cloning: A Laboratory Manual, 2nd ed., 1989). The microarray is scanned post-hybridization using a fluorescent microarray scanner.

The fluorescent intensity of each spot indicates the level of expression for that gene; bright spots correspond to strongly expressed genes, while dim spots indicate weak expression.

Once the images are obtained, the raw data must be analyzed. First, the background fluorescence must be subtracted from the fluorescence of each spot. The data is then normalized to a control sequence, such as an exogenously added RNA, or a housekeeping gene panel to account for any nonspecific hybridization, array imperfections or variability in the array setup, cDNA labeling, hybridization or washing. Data normalization allows the results of multiple arrays to be compared.

The present invention further provides for methods for the detection of the presence of the polypeptide encoded by said gene sequences in a sample obtained from a patient.

Aberrant levels of polypeptide expression of the polypeptides encoded by the gene according to Table 11 (in particular FOXL2, HIST2H2BF and GPR7 and most preferably PITX2) are associated with prostate cancer prognosis and/or treatment outcome.

Any method known in the art for detecting polypeptides can be used. Such methods include, but are not limited to masss-spectrometry, immunodiffusion, immunoelectrophoresis, immunochemical methods, binder-ligand assays, immunohistochemical techniques, agglutination and complement assays (e.g., see Basic and Clinical Immunology, Sites and Terr, eds., Appleton & Lange, Norwalk, Conn. pp 217-262, 1991 which is incorporated by reference). Preferred are binder-ligand immunoassay methods including reacting antibodies with an epitope or epitopes and competitively displacing a labelled polypeptide or derivative thereof.

Certain embodiments of the present invention comprise the use of antibodies specific to the polypeptide encoded by the a gene selected from Table 11, preferably FOXL2, HIST2H2BF or GPR7 and most preferably PITX2.

Such antibodies are useful for prostate cancer prognostic and/or predictive applications. In certain embodiments production of monoclonal or polyclonal antibodies can be induced by the use of the coded polypeptide as an antigene. Such antibodies may in turn be used to detect expressed polypeptides as markers for prostate cancer prognosis. The levels of such polypeptides present may be quantified by conventional methods. Antibody-polypeptide binding may be detected and quantified by a variety of means known in the art, such as labelling with fluorescent or radioactive ligands. The invention further comprises kits for performing the above-mentioned procedures, wherein such kits contain antibodies specific for the investigated polypeptides.

Numerous competitive and non-competitive polypeptide binding immunoassays are well known in the art. Antibodies employed in such assays may be unlabelled, for example as used in agglutination tests, or labelled for use a wide variety of assay methods. Labels that can be used include radionuclides, enzymes, fluorescers, chemiluminescers, enzyme substrates or co-factors, enzyme inhibitors, particles, dyes and the like. Preferred assays include but are not limited to radioimmunoassay (RIA), enzyme immunoassays, e.g., enzyme-linked immunosorbent assay (ELISA), fluorescent immunoassays and the like. Polyclonal or monoclonal antibodies or epitopes thereof can be made for use in immunoassays by any of a number of methods known in the art.

In an alternative embodiment of the method the proteins may be detected by means of western blot analysis. Said analysis is standar in the art, briefly proteins are separated by means of electrophoresis e.g. SDS-PAGE. The separated proteins are then transferred to a suitable membrane (or paper) e.g. nitrocellulose, retaining the spacial separation achieved by electrophoresis. The membrane is then incubated with a generic protein (e.g. milk protein) to bind remaining sticky places on the membrane. An antibody specific to the protein of interest is then added, said antibody being detectably labelled for example by dyes or enzymatic means (e.g. alkaline phosphatase or horseradish peroxidase) . The location of the antibody on the membrane is then detected.

In an alternative embodiment of the method the proteins may be detected by means of immunohistochemistry (the use of antibodies to probe specific antigens in a sample). Said analysis is standard in the art, wherein detection of antigens in tissues is known as immunohistochemistry, while detection in cultured cells is generally termed immunocytochemistry. Briefly the primary antibody to be detected by binding to its specific antigen. The antibody-antigen complex is then bound by a secondary enzyme conjugated antibody. In the presence of the necessary substrate and chromogen the bound enzyme is detected according to coloured deposits at the antibody-antigen binding sites. There is a wide range of suitable sample types, antigen-antibody affinity, antibody types, and detection enhancement methods. Thus optimal conditions for immunohistochemical or immunocytochemical detection must be determined by the person skilled in the art for each individual case.

One approach for preparing antibodies to a polypeptide is the selection and preparation of an amino acid sequence of all or part of the polypeptide, chemically synthesising the amino acid sequence and injecting it into an appropriate animal, usually a rabbit or a mouse (Milstein and Kohler Nature 256:495-497, 1975; Gulfre and Milstein, Methods in Enzymology: Immunochemical Techniques 73:1-46, Langone and Banatis eds., Academic Press, 1981 which are incorporated by reference). Methods for preparation of the polypeptides or epitopes thereof include, but are not limited to chemical synthesis, recombinant DNA techniques or isolation from biological samples.

In a particularly preferred embodiment the expression level of the genes, genomic sequences and/or regulatory regions according to Table 11 is determined by analysis of the level of methylation of said genes, genomic sequences and/or regulatory regions thereof. It is preferred that the level of methylation of said genes, genomic sequences and/or regulatory regions thereof is determined by determing the methylaiton status or level of at least one CpG dinucleotide thereof. It is further preferred that the level of methylation of said genes, genomic sequences and/or regulatory regions thereof is determined by determing the methylaiton status or level of a plurality of CpG dinucleotides thereof. It is particularly preferred that said genes, genomic sequences and/or regulatory regions are selected from the group consisting PITX2, SEQ ID NO: 63, GPR7 and SEQ ID NO: 35. Further preferred is the gene PITX2. Said analysis comprises the following steps:

i) contacting genomic DNA obtained from the subject with at least one reagent, or series of reagents that distinguishes between methylated and non-methylated CpG dinucleotides within at least one target region of the genomic DNA, wherein said contiguous nucleotides comprise at least one CpG dinucleotide sequence; and
ii) classifying the prostate cell proliferative disorders according to its prognosis as determined from the methylation status of said target regions analysed in i).

Genomic DNA may be isolated by any means standard in the art, including the use of commercially available kits. Briefly, wherein the DNA of interest is encapsulated in by a cellular membrane the biological sample must be disrupted and lysed by enzymatic, chemical or mechanical means. The DNA solution may then be cleared of proteins and other contaminants e.g. by digestion with proteinase K. The genomic DNA is then recovered from the solution. This may be carried out by means of a variety of methods including salting out, organic extraction or binding of the DNA to a solid phase support. The choice of method will be affected by several factors including time, expense and required quantity of DNA. Preferably, the source of the DNA sample is selected from the group consisting of cells or cell lines, histological slides, biopsies, paraffin-embedded tissue, bodily fluids, ejaculate, urine, blood, and combinations thereof. Preferably, the source is biopsies, bodily fluids, ejaculate, urine, or blood. The genomic DNA sample is then treated in such a manner that cytosine bases which are unmethylated at the 5'-position are converted to uracil, thymine, or another base which is dissimilar to cytosine in terms of hybridization behavior. This will be understood as 'treatment' herein.

The above described treatment of genomic DNA is preferably carried out with bisulfite (hydrogen sulfite, disulfite) and subsequent alkaline hydrolysis which results in a conversion of non-methylated cytosine nucleobases to uracil or to another base which is dissimilar to cytosine in terms of base pairing behavior.

The treated DNA is then analysed in order to determine the methylation state of one or more target gene sequences (prior to the treatment) associated with the development of prostate carcinoma. It is particularly preferred that the target region comprises, or hybridizes under stringent conditions to at least 16 contiguous nucleotides of at least one gene or genomic sequence selected from the group consisting the genes and genomic sequences as listed in Table 11. It is particularly preferred that said gene or genomic sequence is selected from the group consisting PITX2, SEQ ID NO: 63, GPR7 and SEQ ID NO: 35. Further preferred is the gene PITX2. It is further preferred that the sequences of said genes as described in the accompanying sequence listing are analysed. The method of analysis may be selected from those known in the art, including those listed herein. Particularly preferred are MethyLight™, MSP™ and the use of blocking oligonucleotides as will be described herein. It is further preferred that any oligonucleotides used in such analysis (including primers, blocking oligonucleotides and detection probes) should be reverse complementary, identical, or hybridize under stringent or highly stringent conditions to an at least 16-base-pair long segment of the base sequences of one or more of SEQ ID NO:65 to SEQ ID NO:320 and SEQ ID NO: 962 to SEQ ID NO: 965 and sequences complementary thereto.It is preferred that any oligonucleotides used in such analysis (including primers, blocking oligonucleotides and detection probes) should be reverse complementary, identical, or hybridize under stringent or highly stringent conditions to an at least 16-base-pair long segment of the base sequences of one or more of SEQ ID Nos: 133,134,261,262, 189,190,317,318, 101,102,229,230 and most preferably SEQ ID Nos: 962 - 965 and sequences complementary thereto

Aberrant methylation, of one or more genes or genomic sequences taken from those listed in Table 11, and more preferably the genes or genomic sequences according to PITX2, SEQ ID NO: 63, GPR7 and SEQ ID NO: 35 are associated with the prognosis of prostate cell proliferative disorders. Analysis of one or a plurality of the sequences enables the prognostic classification of prostate cell proliferative disorders. More preferably hypermethylation of one or more of said genes or genomic sequences is associated with poor prognosis. Hypermethylation is in general associated with under expression of mRNA and accordingly polpeptides.

In one embodiment the method discloses the use of one or more genes or genomic sequences selected from the group consisting the genes according to Table 11 as markers for providing a prognosis of prostate cell proliferative disorders. It is particularly preferred that said gene or genomic sequence is selected from the group consisting PITX2, SEQ ID NO: 63, GPR7 and SEQ ID NO: 35. Further preferred is the gene PITX2.

Said use of the genes and/or sequences may be enabled by means of any analysis of the expression of the gene, by means of mRNA expression analysis or protein expression analysis. However, in the most preferred embodiment of the invention, the detection of prostate cell proliferative disorders is enabled by means of analysis of the methylation status of said genes or genomic sequences and their promoter or regulatory elements. Methods for the methylation analysis of genes are described herein.

In one embodiment the method discloses the use of one or more genes or genomic sequences selected from the group consisting of the genes according to Table 11 as markers for providing a prognosis of prostate cell proliferative disorders. It is particularly preferred that said gene or genomic sequence is selected from the group consisting PITX2, SEQ ID NO: 63, GPR7 and SEQ ID NO: 35. Further preferred is the gene PITX2.

Said use of the genes and/or sequences may be enabled by means of any analysis of the expression of the gene, by means of mRNA expression analysis or protein expression analysis. However, in the most preferred embodiment of the invention, the detection of prostate cell proliferative disorders is enabled by means of analysis of the methylation status of said genes or genomic sequences and their promoter or regulatory elements. Methods for the methylation analysis of genes are described herein.

Aberrant levels of mRNA expression of the genes, genomic sequences or genes regulated by genomic sequences according to Table 11 are associated with prognosis of prostate cell proliferative disorders. Accordingly, increased or decreased levels of expression of said genes or sequences are associable with factors associated with the prognosis of prostate cell proliferative disorders, including but not limited to disease agressivity and progression. It is particularly preferred that said gene or genomic sequence is selected from the group consisting PITX2, SEQ ID NO: 63, GPR7 and SEQ ID NO: 35. Further preferred is the gene PITX2.

To detect the presence of mRNA encoding a gene or genomic sequence in a prognostic classification of prostate cell proliferative disorders, a sample is obtained from a patient. Preferably, the source of the sample is selected from the group consisting of cells or cell lines, histological slides, biopsies, paraffin-embedded tissue, bodily fluids, ejaculate, urine, blood, and combinations thereof. Preferably, the source is biopsies, bodily fluids, ejaculate, urine, or blood. The sample may be treated to extract the nucleic acids contained therein. The resulting nucleic acid from the sample is subjected to gel electrophoresis or other separation techniques. Detection involves contacting the nucleic acids and in particular the mRNA of the sample with a DNA sequence serving as a probe to form hybrid duplexes. The stringency of hybridisation is determined by a number of factors during hybridisation and during the washing procedure, including temperature, ionic strength, length of time and concentration of formamide. T hese factors are outlined in, for example, Sambrook et al. (Molecular Cloning: A Laboratory Manual, 2d ed., 1989). Detection of the resulting duplex is usually accomplished by the use of labelled probes. Alternatively, the probe may be unlabeled, but may be detectable by specific binding with a ligand which is labelled, either directly or indirectly. Suitable labels and methods for labelling probes and ligands are known in the art, and include, for example, radioactive labels which may be incorporated by known methods (e.g., nick translation or kinasing), biotin, fluorescent groups, chemiluminescent groups (e.g., dioxetanes, particularly triggered dioxetanes), enzymes, antibodies, and the like.

To increase the sensitivity of the detection in a sample of mRNA transcribed from the gene or genomic sequence, the technique of reverse transcription/polymerisation chain reaction can be used to amplify cDNA transcribed from the mRNA. The method of reverse transcription /PCR is well known in the art (for example, see Watson and Fleming, supra).

The reverse transcription /PCR method can be performed as follows. Total cellular RNA is isolated by, for example, the standard guanidium isothiocyanate method and the total RNA is reverse transcribed. The reverse transcription method involves synthesis of DNA on a template of RNA using a reverse transcriptase enzyme and a 3' end primer. Typically, the primer contains an oligo(dT) sequence. The cDNA thus produced is then amplified using the PCR method and EYA4 specific primers. (Belyavsky et al, Nucl Acid Res 17:2919-2932, 1989; Krug and Berger, Methods in Enzymology, Academic Press,N.Y., Vol.152, pp. 316-325, 1987 which are incorporated by reference).

The present invention may also be described in certain embodiments as a *kit* for use in providing a prognosis of a prostate cell proliferative disorder state through testing of a biological sample. A representative kit may comprise one or more nucleic acid segments that selectively hybridise to the mRNA and a container for each of the one or more nucleic acid segments. In certain embodiments the nucleic acid segments may be combined in a single tube. In further embodiments, the nucleic acid segments may also include a pair of primers for amplifying the target mRNA. Such kits may also include any buffers, solutions, solvents, enzymes, nucleotides, or other components for hybridisation, amplification or detection reactions. Preferred kit components include reagents for reverse transcription-PCR, in situ hybridisation, Northern analysis and/or RPA.

Particular embodiments of the present invention provide a novel application of the analysis of methylation levels and/or patterns within said sequences that enables a prognostic classification and thereby improved treatment of prostate cell proliferative disorders. Treatment of prostate cell proliferative disorders is directly linked with disease prognosis in particular aggressiveness, and the disclosed method thereby enables the physician and patient to make better and more informed treatment decisions.

FURTHER IMPROVEMENTS

The present invention provides novel uses for genomic sequences selected- from the group consisting of SEQ ID NO:1 TO SEQ ID NO:64 AND SEQ ID NO: 961 (preferably SEQ ID Nos: 35, 63, 19 and most preferably SEQ ID NO: 961). Additional embodiments provide modified variants of SEQ ID NO:1 TO SEQ ID NO:64 AND SEQ ID NO: 961, as well as oligonucleotides and/or PNA-oligomers for analysis of cytosine methylation patterns within the group consisting SEQ ID NO:1 TO SEQ ID NO:64 AND SEQ ID NO: 961 (preferably SEQ ID Nos: 35, 63, 19 and most preferably SEQ ID NO: 961).

An objective of the invention comprises analysis of the methylation state of one or more CpG dinucleotides within at least one of the genomic sequences selected from the group consisting of SEQ ID NO:1 TO SEQ ID NO:64 AND SEQ ID NO: 961 and sequences complementary thereto. Preferably said group consists of SEQ ID Nos: 35, 63, 19 and most preferably said sequence is SEQ ID NO: 961.

The disclosed invention provides treated nucleic acids, derived from genomic SEQ ID NO:1 to SEQ ID NO:64 and SEQ ID NO: 961 (preferably SEQ ID Nos: 35, 63, 19 and most preferably SEQ ID NO: 961), wherein the treatment is suitable to convert at least one unmethylated cytosine base of the genomic DNA sequence to uracil or another base that is detectably dissimilar to cytosine in terms of hybridization. The genomic sequences in question may comprise one, or more, consecutive or random methylated CpG positions. Said treatment preferably comprises use of a reagent selected from the group consisting of bisulfite, hydrogen sulfite, disulfite, and combinations thereof. In a preferred embodiment of the invention, the objective comprises analysis of a non-naturally occurring modified nucleic acid comprising a sequence of at least 16 contiguous nucleotide bases in length of a sequence selected from the group consisting of SEQ ID NO: 65 TO SEQ ID NO: 320 AND SEQ ID Nos: 962 - 965 (preferably said group consists of SEQ ID Nos: 133,134,261,262, 189,190,317,318, 101,102,229,230 and most preferably said group consists of SEQ ID Nos: 962 - 965). Particularly preferred is a non-naturally occurring modified nucleic acid comprising a sequence of at least 16 contiguous nucleotide bases in length of a sequence selected from the group consisting of SEQ ID NO: 65 to SEQ ID NO: 320 and SEQ ID NO: 962 to SEQ ID NO: 965 that is not identical to or complementary to SEQ ID NO: 1 to SEQ ID NO: 64 and SEQ ID NO: 961 or other human genomic DNA. Further preferred is a non-naturally occurring modified nucleic acid comprising a sequence of at least 16 contiguous nucleotide bases in length of a sequence selected from the group consisting of SEQ ID Nos: 133,134,261,262, 189,190,317,318, 101,102,229,230, 962 - 965 that is not identical to or complementary to SEQ ID Nos: 961, 35, 63 and 19 or other human genomic DNA.

It is further preferred that said sequence comprises at least one CpG, TpA or CpA dinucleotide and sequences complementary thereto. The sequences of SEQ ID NO:65 TO SEQ ID NO:320 AND SEQ ID NO: 962 TO SEQ ID NO: 965 provide non-naturally occurring modified versions of the nucleic acid according to SEQ ID NO:1 TO SEQ ID NO: 64 AND SEQ ID NO: 961, wherein the modification of each genomic sequence results in the synthesis of a nucleic acid having a sequence that is unique and distinct from said genomic sequence as follows. For each sense strand genomic DNA, e.g., SEQ ID NO:1, four converted versions are disclosed. A first version wherein "C" is converted to "T," but "CpG" remains "CpG" (*i.e.,* corresponds to case where, for the genomic sequence, all "C" residues of CpG dinucleotide sequences are methylated and are thus not converted); a second version discloses the complement of the disclosed genomic DNA sequence (*i.e.* antisense strand), wherein "C" is converted to "T," but "CpG" remains "CpG" (*i.e*., corresponds to case where, for all "C" residues of CpG dinucleotide sequences are methylated and are thus not converted). The 'upmethylated' converted sequences of SEQ ID NO:1 to SEQ ID NO:64 and SEQ ID NO: 961 correspond to SEQ ID NO:65 to SEQ ID NO:728. A third chemically converted version of each genomic sequences is provided, wherein "C" is converted to "T' for all "C" residues, including those of "CpG" dinucleotide sequences *(i.e.,* corresponds to case where, for the genomic sequences, all "C" residues of CpG dinucleotide sequences are unmethylated); a final chemically converted version of each sequence, discloses the complement of the disclosed genomic DNA sequence (*i.e.* antisense strand), wherein "C" is converted to "T' for all "C" residues, including those of "CpG" dinucleotide sequences (*i.e*., corresponds to case where, for the complement (antisense strand) of each genomic sequence, all "C" residues of CpG dinucleotide sequences are unmethylated). The 'downmethylated' converted sequences of SEQ ID NO:1 to SEQ ID NO:64 and SEQ ID NO: 961 correspond to SEQ ID NO:65 to SEQ ID NO:320 and SEQ ID NO: 962 to SEQ ID NO: 965.

In an alternative preferred embodiment, such analysis comprises the use of an oligonucleotide or oligomer for detecting the cytosine methylation state within genomic or treated (chemically modified) DNA, according to SEQ ID NO: 1 to SEQ ID NO:320 and SEQ ID NO: 961 to SEQ ID NO: 965. Said oligonucleotide or oligomer comprising a nucleic acid sequence having a length of at least nine (9) nucleotides which hybridizes, under moderately stringent or stringent conditions (as defined herein above), to a treated nucleic acid sequence according to SEQ ID NO:1 to SEQ ID NO:320 and SEQ ID NO: 961 to SEQ ID NO: 965 and/or sequences complementary thereto, or to a genomic sequence according to SEQ ID NO:1 to SEQ ID NO:64 and SEQ ID NO: 961 and/or sequences complementary thereto.

Thus, the present invention includes nucleic acid molecules (e.g., oligonucleotides and peptide nucleic acid (PNA) molecules (PNA-oligomers)) that hybridize under moderately stringent and/or stringent hybridization conditions to all or a portion of the sequences SEQ ID NO: 1 to SEQ ID NO: 320 and SEQ ID NO: 961 to 965 , or to the complements thereof. Particularly preferred is a nucleic acid molecule that hybridizes under moderately stringent and/or stringent hybridization conditions to all or a portion of the sequences SEQ ID NO: 65 to SEQ ID NO: 320 and SEQ ID NO: 962 to SEQ ID NO: 965 but is not identical to or complementary to SEQ ID NO: 1 to SEQ ID NO: 64 and SEQ ID NO: 961 or other human genomic DNA. Further preferred is a nucleic acid molecule that hybridizes under moderately stringent and/or stringent hybridization conditions to all or a portion of the sequences SEQ ID Nos: 133,134,261,262, 189,190,317,318, 101,102,229,230, 962 - 965 but is not identical to or complementary to SEQ ID Nos: 961, 35, 63 and 19 or other human genomic DNA.

The hybridizing portion of the hybridizing nucleic acids is typically at least 9, 15, 20, 25, 30 or 35 nucleotides in length. However, longer molecules have inventive utility, and are thus within the scope of the present invention.

Preferably, the hybridizing portion of the inventive hybridizing nucleic acids is at least 95%, or at least 98%, or 100% identical to the sequence, or to a portion thereof of SEQ ID NO: 1 to SEQ ID NO: 320 and SEQ ID NO: 961 to 965, or to the complements thereof.

Hybridizing nucleic acids of the type described herein can be used, for example, as a primer (e.g., a PCR primer), or a diagnostic and/or prognostic probe or primer. Preferably, hybridization of the oligonucleotide probe to a nucleic acid sample is performed under stringent conditions and the probe is 100% identical to the target sequence. Nucleic acid duplex or hybrid stability is expressed as the melting temperature or Tm, which is the temperature at which a probe dissociates from a target DNA. This melting temperature is used to define the required stringency conditions.

For target sequences that are related and substantially identical to the corresponding sequence of SEQ ID NO:1 to SEQ ID NO:64 and SEQ ID NO: 961 (such as allelic variants and SNPs), rather than identical, it is useful to first establish the lowest temperature at which only homologous hybridization occurs With a particular concentration of salt (e.g., SSC or SSPE). Then, assuming that 1% mismatching results in a 1˚C decrease in the Tm, the temperature of the final wash in the hybridization reaction is reduced accordingly (for example, if sequences having > 95% identity with the probe are sought, the final wash temperature is decreased by 5˚C). In practice, the change in Tm can be between 0.5˚C and 1.5˚C per 1% mismatch.

Examples of inventive oligonucleotides of length X (in nucleotides), as indicated by polynucleotide positions with reference to, e.g., SEQ ID NO:1, include those corresponding to sets (sense and antisense sets) of consecutively overlapping oligonucleotides of length X, where the oligonucleotides within each consecutively overlapping set (corresponding to a given X value) are defined as the finite set of Z oligonucleotides from nucleotide positions:

n to (n + (X-1));

where n=1, 2, 3,...(Y-(X-1));

where Y equals the length (nucleotides or base pairs) of SEQ ID NO: 1 (7572);

where X equals the common length (in nucleotides) of each oligonucleotide in the set (e.g., X=20 for a set of consecutively overlapping 20-mers); and

where the number (Z) of consecutively overlapping oligomers of length X for a given SEQ ID NO of length Y is equal to Y-(X-1). For example Z= 7572-19= 7553 for either sense or antisense sets of SEQ ID NO:1, where X=20.

Preferably, the set is limited to those oligomers that comprise at least one CpG, TpG or CpA dinucleotide.

Examples of inventive 20-mer oligonucleotides include the following set of oligomers (and the antisense set complementary thereto), indicated by polynucleotide positions with reference to SEQ ID NO:1:

1-20, 2-21, 3-22, 4-23, 5-24.......7553 -7572.

Preferably, the set is limited to those oligomers that comprise at least one CpG, TpG or CpA dinucleotide.

Likewise, examples of inventive 25-mer oligonucleotides include the following set of 2,256 oligomers (and the antisense set complementary thereto), indicated by polynucleotide positions with reference to SEQ ID NO:1:

1-25, 2-26, 3-27, 4-28, 5-29, ...... 7553 -7572.

Preferably, the set is limited to those oligomers that comprise at least one CpG, TpG or CpA dinucleotide.

The present invention encompasses, for each of SEQ ID NO:1 to SEQ ID NO:320 and SEQ ID NO: 961 to SEQ ID NO: 965 (sense and antisense), multiple consecutively overlapping sets of oligonucleotides or modified oligonucleotides of length X, where, e.g., X= 9, 10, 17, 20, 22, 23, 25, 27, 30 or 35 nucleotides.

The oligonucleotides or oligomers according to the present invention constitute effective tools useful to ascertain genetic and epigenetic parameters of the genomic sequence corresponding to SEQ ID NO:1 to SEQ ID NO:64 and SEQ ID NO: 961 . Preferred sets of such oligonucleotides or modified oligonucleotides of length X are those consecutively overlapping sets of oligomers corresponding to SEQ ID NO:1 to SEQ ID NO:320 and SEQ ID NO: 961 to SEQ ID NO: 965 (and to the complements thereof). Preferably, said oligomers comprise at least one CpG, TpG or CpA dinucleotide.

Particularly preferred oligonucleotides or oligomers according to the present invention are those in which the cytosine of the CpG dinucleotide (or of the corresponding converted TpG or CpA dinculeotide) sequences is within the middle third of the oligonucleotide; that is, where the oligonucleotide is, for example, 13 bases in length, the CpG, TpG or CpA dinucleotide is positioned within the fifth to ninth nucleotide from the 5'-end.

The oligonucleotides of the invention can also be modified by chemically linking the oligonucleotide to one or more moieties or conjugates to enhance the activity, stability or detection of the oligonucleotide. Such moieties or conjugates include chromophores, fluorophors, lipids such as cholesterol, cholic acid, thioether, aliphatic chains, phospholipids, polyamines, polyethylene glycol (PEG), palmityl moieties, and others as disclosed in, for example, United States Patent Numbers 5,514,758, 5,565,552, 5,567,810, 5,574,142, 5,585,481, 5,587,371, 5,597,696 and 5,958,773. The probes may also exist in the form of a PNA (peptide nucleic acid) which has particularly preferred pairing properties. Thus, the oligonucleotide may include other appended groups such as peptides, and may include hybridization-triggered cleavage agents (Krol et al., BioTechniques 6:958-976, 1988) or intercalating agents (Zon, Pharm. Res. 5:539-549, 1988). To this end, the oligonucleotide may be conjugated to another molecule, e.g., a chromophore, fluorophor, peptide, hybridization-triggered cross-linking agent, transport agent, hybridization-triggered cleavage agent, etc.

The oligonucleotide may also comprise at least one art-recognized modified sugar and/or base moiety, or may comprise a modified backbone or non-natural internucleoside linkage.

The oligonucleotides or oligomers according to particular embodiments of the present invention are typically used in 'sets,' which contain at least one oligomer for analysis of at least one of the CpG dinucleotides of genomic sequences SEQ ID NO:1 to SEQ ID NO:64 and SEQ ID NO: 961 and sequences complementary thereto, or to the corresponding CpG, TpG or CpA dinucleotide within a sequence of the treated nucleic acids according to SEQ ID NO: 65 to SEQ ID NO:320 and SEQ ID NO: 962 to SEQ ID NO: 965 and sequences complementary thereto. However, it is anticipated that for economic or other factors it may be preferable to analyze a limited selection of the CpG dinucleotides within said sequences, and the content of the set of oligonucleotides is altered accordingly.

Therefore, in particular embodiments, the present invention provides a set of at least two (2) (oligonucleotides and/or PNA-oligomers) useful for detecting the cytosine methylation state in treated genomic DNA (SEQ ID NO: 65 to SEQ ID NO:320 and SEQ ID NO: 962 to SEQ ID NO: 965), or in genomic DNA (SEQ ID NO:1 to SEQ ID NO:64 and SEQ ID NO: 961 and sequences complementary thereto). These probes enable diagnosis and/or classification of genetic and epigenetic parameters of prostate cell proliferative disorders. The set of oligomers may also be used for detecting single nucleotide polymorphisms (SNPs) in treated genomic DNA (SEQ ID NO:65 to SEQ ID NO:320 and SEQ ID NO: 962 to SEQ ID NO: 965), or in genomic DNA (SEQ ID NO:1 to SEQ ID NO:64 and SEQ ID NO: 961 and sequences complementary thereto).

In preferred embodiments, at least one, and more preferably all members of a set of oligonucleotides is bound to a solid phase.

In further embodiments, the present invention provides a set of at least two (2) oligonucleotides that are used as 'primer' oligonucleotides for amplifying DNA sequences of one of SEQ ID NO:1 to SEQ ID NO:320 and SEQ ID NO: 961 to SEQ ID NO: 965 and sequences complementary thereto, or segments thereof.

It is anticipated that the oligonucleotides may constitute all or part of an "array" or "DNA chip" (*i.e.,* an arrangement of different oligonucleotides and/or PNA-oligomers bound to a solid phase). Such an array of different oligonucleotide- and/or PNA-oligomer sequences can be characterized, for example, in that it is arranged on the solid phase in the form of a rectangular or hexagonal lattice. The solid-phase surface may be composed of silicon, glass, polystyrene, aluminum, steel, iron, copper, nickel, silver, or gold. Nitrocellulose as well as plastics such as nylon, which can exist in the form of pellets or also as resin matrices, may also be used. An overview of the Prior Art in oligomer array manufacturing can be gathered from a special edition of Nature Genetics (Nature Genetics Supplement, Volume 21, January 1999, and from the literature cited therein). Fluorescently labeled probes are often used for the scanning of immobilized DNA arrays. The simple attachment of Cy3 and Cy5 dyes to the 5'-OH of the specific probe are particularly suitable for fluorescence labels. The detection of the fluorescence of the hybridized probes may be carried out, for example, via a confocal microscope. Cy3 and Cy5 dyes, besides many others, are commercially available.

It is also anticipated that the oligonucleotides, or particular sequences thereof, may constitute all or part of an "virtual array" wherein the oligonucleotides, or particular sequences thereof, are used, for example, as 'specifiers' as part of, or in combination with a diverse population of unique labeled probes to analyze a complex mixture of analytes. Such a method, for example is described in US 2003/0013091 (United States serial number 09/898,743, published 16 January 2003). In such methods, enough labels are generated so that each nucleic acid in the complex mixture (*i.e.,* each analyte) can be uniquely bound by a unique label and thus detected (each label is directly counted, resulting in a digital read-out of each molecular species in the mixture).

It is particularly preferred that the oligomers according to the invention are utilised for at least one of: prognosis of; treatment of; monitoring of; and treatment and monitoring of prostate cell proliferative disorders. This is enabled by use of said sets for providing a prognosis of a biological sample isolated from a patient. Particularly preferrred are those sets of oligomer that comprise at least two oligonucleotides selected from one of the following sets of oligonucleotides.

In one embodiment of the method, this is achieved by analysis of the methylation status of at least one target sequence comprising, or hybridizing under stringent conditions to at least 16 contiguous nucleotides of a gene or sequence selected from the group consisting the genes and sequences according to Table 11 and complements thereof. It is particularly preferred that said gene or genomic sequence is selected from the group consisting PITX2, SEQ ID NO: 63, GPR7 and SEQ ID NO: 35 and their sequences as listed in the accompanying sequence listing. Further preferred is the gene PITX2.

The present invention further provides a method for ascertaining genetic and/or epigenetic parameters of the genomic sequences according to SEQ ID NO:1 to SEQ ID NO:64 and SEQ ID NO: 961 within a subject by analyzing cytosine methylation and single nucleotide polymorphisms. In a preferred embodiment the present invention further provides a method for ascertaining genetic and/or epigenetic parameters of the genomic sequences according to SEQ ID Nos: 35, 63, 19 and most preferably SEQ ID NO: 961 within a subject by analyzing cytosine methylation and single nucleotide polymorphisms. Said method comprising contacting a nucleic acid comprising one or more of SEQ ID NO:1 to SEQ ID NO:64 and SEQ ID NO: 961 (preferably one or more of SEQ ID Nos: 35, 63, 19 and most preferably SEQ ID NO: 961) in a biological sample obtained from said subject with at least one reagent or a series of reagents, wherein said reagent or series of reagents, distinguishes between methylated and non-methylated CpG dinucleotides within the target nucleic acid.

Preferably, said method comprises the following steps: In the *first step,* a sample of the tissue to be analysed is obtained. The source may be any suitable source. Preferably, the source of the DNA sample is selected from the group consisting of cells or cell lines, histological slides, biopsies, paraffin-embedded tissue, bodily fluids, ejaculate, urine, blood, and combinations thereof. Preferably, the source is biopsies, bodily fluids, ejaculate, urine, or blood.

The genomic DNA is then isolated from the sample. Genomic DNA may be isolated by any means standard in the art, including the use of commercially available kits. Briefly, wherein the DNA of interest is encapsulated in by a cellular membrane the biological sample must be disrupted and lysed by enzymatic, chemical or mechanical means. The DNA solution may then be cleared of proteins and other contaminants e.g. by digestion with proteinase K. The genomic DNA is then recovered from the solution. This may be carried out by means of a variety of methods including salting out, organic extraction or binding of the DNA to a solid phase support. The choice of method will be affected by several factors including time, expense and required quantity of DNA.

Once the nucleic acids have been extracted, the genomic double stranded DNA is used in the analysis.

In the second *step* of the method, the genomic DNA sample is treated in such a manner that cytosine bases which are unmethylated at the 5'-position are converted to uracil, thymine, or another base which is dissimilar to cytosine in terms of hybridization behavior. This will be understood as 'pretreatment' or 'treatment' herein.

The above-described treatment of genomic DNA is preferably carried out with bisulfite (hydrogen sulfite, disulfite) and subsequent alkaline hydrolysis which results in a conversion of non-methylated cytosine nucleobases to uracil or to another base which is dissimilar to cytosine in terms of base pairing behavior.

In the *third step* of the method, fragments of the treated DNA are amplified, using sets of primer oligonucleotides according to the present invention, and an amplification enzyme. The amplification of several DNA segments can be carried out simultaneously in one and the same reaction vessel. Typically, the amplification is carried out using a polymerase chain reaction (PCR). The set of primer oligonucleotides includes at least two oligonucleotides whose sequences are each reverse complementary, identical, or hybridize under stringent or highly stringent conditions to an at least 16-base-pair long segment of the base sequences of one of SEQ ID NO:65 to SEQ ID NO:320 and SEQ ID NO: 962 to SEQ ID NO: 965 (preferably one of SEQ ID Nos: 133,134,261,262, 189,190,317,318, 101,102,229,230 and most preferably one of SEQ ID Nos: 962 - 965) and sequences complementary thereto.

In an alternate embodiment of the method, the methylation status of preselected CpG positions within the nucleic acid sequences comprising one or more of SEQ ID NO:1 to SEQ ID NO:64 and SEQ ID NO:961 (preferably one or more of SEQ ID Nos: 35, 63, 19 and most preferably SEQ ID NO: 961) may be detected by use of methylation-specific primer oligonucleotides. This technique (MSP) has been described in United States Patent No. 6,265,171 to Herman. The use of methylation status specific primers for the amplification of bisulfite treated DNA allows the differentiation between methylated and unmethylated nucleic acids. MSP primers pairs contain at least one primer which hybridizes to a bisulfite treated CpG dinucleotide. Therefore, the sequence of said primers comprises at least one CpG dinucleotide. MSP primers specific for non-methylated DNA contain a "T' at the position of the C position in the CpG. Preferably, therefore, the base sequence of said primers is required to comprise a sequence having a length of at least 9 nucleotides which hybridizes to a treated nucleic acid sequence according to one of SEQ ID NO: 65 to SEQ ID NO: 320 and SEQ ID NO: 961 to 965 (preferably SEQ ID Nos: 133,134,261,262, 189,190,317,318, 101,102,229,230 and most preferably SEQ ID Nos: 962 - 965) and sequences complementary thereto, wherein the base sequence of said oligomers comprises at least one CpG dinucleotide.

A further preferred embodiment of the method comprises the use of blocker oligonucleotides. The use of such blocker oligonucleotides has been described by Yu et al., BioTechniques 23:714-720, 1997. Blocking probe oligonucleotides are hybridized to the bisulfite treated nucleic acid concurrently with the PCR primers. PCR amplification of the nucleic acid is terminated at the 5' position of the blocking probe, such that amplification of a nucleic acid is suppressed where the complementary sequence to the blocking probe is present. The probes may be designed to hybridize to the bisulfite treated nucleic acid in a methylation status specific manner. For example, for detection of methylated nucleic acids within a population of unmethylated nucleic acids, suppression of the amplification of nucleic acids which are unmethylated at the position in question would be carried out by the use of blocking probes comprising a 'CpA' or 'TpA' at the position in question, as opposed to a 'CpG' if the suppression of amplification of methylated nucleic acids is desired.

For PCR methods using blocker oligonucleotides, efficient disruption of polymerase-mediated amplification requires that blocker oligonucleotides not be elongated by the polymerase. Preferably, this is achieved through the use of blockers that are 3'-deoxyoligonucleotides, or oligonucleotides derivitized at the 3' position with other than a "free" hydroxyl group. For example, 3'-O-acetyl oligonucleotides are representative of a preferred class of blocker molecule.

Additionally, polymerase-mediated decomposition of the blocker oligonucleotides should be precluded. Preferably, such preclusion comprises either use of a polymerase lacking 5'-3' exonuclease activity, or use of modified blocker oligonucleotides having, for example, thioate bridges at the 5'-terminii thereof that render the blocker molecule nuclease-resistant. Particular applications may not require such 5' modifications of the blocker. For example, if the blocker- and primer-binding sites overlap, thereby precluding binding of the primer (*e.g.*, with excess blocker), degradation of the blocker oligonucleotide will be substantially precluded. This is because the polymerase will not extend the primer toward, and through (in the 5'-3' direction) the blocker-a process that normally results in degradation of the hybridized blocker oligonucleotide.

A particularly preferred blocker/PCR embodiment, for purposes of the present invention and as implemented herein, comprises the use of peptide nucleic acid (PNA) oligomers as blocking oligonucleotides. Such PNA blocker oligomers are ideally suited, because they are neither decomposed nor extended by the polymerase.

Preferably, therefore, the base sequence of said *blocking oligonucleotides* is required to comprise a sequence having a length of at least 9 nucleotides which hybridizes to a treated nucleic acid sequence according to one of SEQ ID NO:65 to SEQ ID NO:320 and SEQ ID NO: 962 to SEQ ID NO: 965 and sequences complementary thereto, wherein the base sequence of said oligonucleotides comprises at least one CpG, TpG or CpA dinucleotide. More preferablythe base sequence of said *blocking oligonucleotides* is required to comprise a sequence having a length of at least 9 nucleotides which hybridizes to a treated nucleic acid sequence according to one of preferably SEQ ID Nos: 133,134,261,262, 189,190,317,318, 101,102,229,230 and most preferably SEQ ID Nos: 962 - 965 and sequences complementary thereto, wherein the base sequence of said oligonucleotides comprises at least one CpG, TpG or CpA dinucleotide.

The fragments obtained by means of the amplification can carry a directly or indirectly detectable label. Preferred are labels in the form of fluorescence labels, radionuclides, or detachable molecule fragments having a typical mass which can be detected in a mass spectrometer. Where said labels are mass labels, it is preferred that the labeled amplificates have a single positive or negative net charge, allowing for better detectability in the mass spectrometer. The detection may be carried out and visualized by means of, *e.g.*, matrix assisted laser desorption/ionization mass spectrometry (MALDI) or using electron spray mass spectrometry (ESI).

Matrix Assisted Laser Desorption/Ionization Mass Spectrometry (MALDI-TOF) is a very efficient development for the analysis of biomolecules (Karas & Hillenkamp, Anal Chem., 60:2299-301, 1988). An analyte is embedded in a light-absorbing matrix. The matrix is evaporated by a short laser pulse thus transporting the analyte molecule into the vapour phase in an unfragmented manner. The analyte is ionized by collisions with matrix molecules. An applied voltage accelerates the ions into a field-free flight tube. Due to their different masses, the ions are accelerated at different rates. Smaller ions reach the detector sooner than bigger ones. MALDI-TOF spectrometry is well suited to the analysis of peptides and proteins. The analysis of nucleic acids is somewhat more difficult (Gut & Beck, Current Innovations and Future Trends, 1:147-57, 1995). The sensitivity with respect to nucleic acid analysis is approximately 100-times less than for peptides, and decreases disproportionately with increasing fragment size. Moreover, for nucleic acids having a multiply negatively charged backbone, the ionization process via the matrix is considerably less efficient. In MALDI-TOF spectrometry, the selection of the matrix plays an eminently important role. For desorption of peptides, several very efficient matrixes have been found which produce a very fine crystallisation. There are now several responsive matrixes for DNA, however, the difference in sensitivity between peptides and nucleic acids has not been reduced. This difference in sensitivity can be reduced, however, by chemically modifying the DNA in such a manner that it becomes more similar to a peptide. For example, phosphorothioate nucleic acids, in which the usual phosphates of the backbone are substituted with thiophosphates, can be converted into a charge-neutral DNA using simple alkylation chemistry (Gut & Beck, Nucleic Acids Res. 23: 1367-73, 1995). The coupling of a charge tag to this modified DNA results in an increase in MALDI-TOF sensitivity to the same level as that found for peptides. A further advantage of charge tagging is the increased stability of the analysis against impurities, which makes the detection of unmodified substrates considerably more difficult.

In the *fourth step* of the method, the amplificates obtained during the third step of the method are analysed in order to ascertain the methylation status of the CpG dinucleotides prior to the treatment.

In embodiments where the amplificates were obtained by means of MSP amplification, the presence or absence of an amplificate is in itself indicative of the methylation state of the CpG positions covered by the primer, according to the base sequences of said primer.

Amplificates obtained by means of both standard and methylation specific PCR may be further analyzed by means of hybridization-based methods such as, but not limited to, array technology and probe based technologies as well as by means of techniques such as sequencing and template directed extension.

In one embodiment of the method, the amplificates synthesised in *step* three are subsequently hybridized to an array or a set of oligonucleotides and/or PNA probes. In this context, the hybridization takes place in the following manner: the set of probes used during the hybridization is preferably composed of at least 2 oligonucleotides or PNA-oligomers; in the process, the amplificates serve as probes which hybridize to oligonucleotides previously bonded to a solid phase; the non-hybridized fragments are subsequently removed; said oligonucleotides contain at least one base sequence having a length of at least 9 nucleotides which is reverse complementary or identical to a segment of the base sequences specified in the present Sequence Listing; and the segment comprises at least one CpG , TpG or CpA dinucleotide.

In a preferred embodiment, said dinucleotide is present in the central third of the oligomer. For example, wherein the oligomer comprises one CpG dinucleotide, said dinucleotide is preferably the fifth to ninth nucleotide from the 5'-end of a 13-mer. One oligonucleotide exists for the analysis of each CpG dinucleotide within the sequence according to SEQ ID NO:1 to SEQ ID NO:64 and SEQ ID NO: 961, and the equivalent positions within SEQ ID NO:65 to SEQ ID NO:320 and SEQ ID NO: 962 to SEQ ID NO: 965. Said oligonucleotides may also be present in the form of peptide nucleic acids. The non-hybridized amplificates are then removed.The hybridized amplificates are then detected. In this context, it is preferred that labels attached to the amplificates are identifiable at each position of the solid phase at which an oligonucleotide sequence is located.

In yet a further embodiment of the method, the genomic methylation status of the CpG positions may be ascertained by means of oligonucleotide probes that are hybridised to the bisulfite treated DNA concurrently with the PCR amplification primers (wherein said primers may either be methylation specific or standard).

A particularly preferred embodiment of this method is the use of fluorescence-based Real Time Quantitative PCR (Heid et al., Genome Res. 6:986-994, 1996; also see United States Patent No. 6,331,393) employing a dual-labeled fluorescent oligonucleotide probe (TaqMan™ PCR, using an ABI Prism 7700 Sequence Detection System, Perkin Elmer Applied Biosystems, Foster City, Califomia). The TaqMan™ PCR reaction employs the use of a nonextendible interrogating oligonucleotide, called a TaqMan™ probe, which, in preferred embodiments, is designed to hybridize to a GpC-rich sequence located between the forward and reverse amplification primers. The TaqMan™ probe further comprises a fluorescent "reporter moiety" and a "quencher moiety" covalently bound to linker moieties (*e.g.*, phosphoramidites) attached to the nucleotides of the TaqMan™ oligonucleotide. For analysis of methylation within nucleic acids subsequent to bisulfite treatment, it is required that the probe be methylation specific, as described in United States Patent No. 6,331,393, (hereby incorporated by reference in its entirety) also known as the MethyLight" assay. Variations on the Taqman™ detection methodology that are also suitable for use with the described invention include the use of dual-probe technology (Lightcycler™) or fluorescent amplification primers (Sunrise™ technology). Both these techniques may be adapted in a manner suitable for use with bisulfite treated DNA, and moreover for methylation analysis within CpG dinucleotides.

A further suitable method for the use of probe oligonucleotides for the assessment of methylation by analysis of bisulfite treated nucleic acids In a further preferred embodiment of the method, the *fifth step* of the method comprises the use of template-directed oligonucleotide extension, such as MS-SNuPE as described by Gonzalgo & Jones, Nucleic Acids Res. 25:2529-2531, 1997.

In yet a further embodiment of the method, the *fourth step* of the method comprises sequencing and subsequent sequence analysis of the amplificate generated in the *third step* of the method (Sanger F., et al., Proc Natl Acad Sci USA 74:5463-5467, 1977).

<u>Best mode</u>

In the most preferred embodiment of the method the genomic nucleic acids are isolated and treated according to the first three steps of the method outlined above, namely:

a) obtaining, from a subject, a biological sample having subject genomic DNA;
b) extracting or otherwise isolating the genomic DNA;
c) treating the genomic DNA of b), or a fragment thereof, with one or more reagents to convert cytosine bases that are unmethylated in the 5-position thereof to uracil or to another base that is detectably dissimilar to cytosine in terms of hybridization properties; and wherein

d) amplifying subsequent to treatment in c) is carried out in a methylation specific manner, namely by use of methylation specific primers or *blocking oligonucleotides,* and further wherein

e) detecting of the amplificates is carried out by means of a real-time detection probe, as described above.

Preferably, where the subsequent amplification of d) is carried out by means of methylation specific primers, as described above, said methylation specific primers comprise a sequence having a length of at least 9 nucleotides which hybridizes to a treated nucleic acid sequence according to one of SEQ ID NO:65 to SEQ ID NO:320 and SEQ ID NO: 962 to SEQ ID NO: 965 and sequences complementary thereto, wherein the base sequence of said oligomers comprises at least one CpG dinucleotide. More preferably, where the subsequent amplification of d) is carried out by means of methylation specific primers, as described above, said methylation specific primers comprise a sequence having a length of at least 9 nucleotides which hybridizes to a treated nucleic acid sequence according to one of SEQ ID Nos: 133,134,261,262, 189,190,317,318, 101,102,229,230 and most preferably SEQ ID Nos: 962 - 965 and sequences complementary thereto, wherein the base sequence of said oligomers comprises at least one CpG dinucleotide.

In an alternative most preferred embodiment of the method, the subsequent amplification of d) is carried out in the presence of *blocking oligonucleotides,* as described above. Said *blocking oligonucleotides* comprising a sequence having a length of at least 9 nucleotides which hybridizes to a treated nucleic acid sequence according to one of SEQ ID NO:65 to SEQ ID NO: 320 and SEQ ID NO: 961 to 965 and sequences complementary thereto, wherein the base sequence of said oligomers comprises at least one CpG, TpG or CpA dinucleotide. Preferably said blocking oligonucleotides comprising a sequence having a length of at least 9 nucleotides which hybridizes to a treated nucleic acid sequence according to one of SEQ ID Nos: 133,134,261,262, 189,190,317,318, 101,102,229,230 and most preferably SEQ ID Nos: 962 - 965 and sequences complementary thereto, wherein the base sequence of said oligomers comprises at least one CpG, TpG or CpA dinucleotide.

Step e) of the method, namely the detection of the specific amplificates indicative of the methylation status of one or more CpG positions according to SEQ ID NO:1 to SEQ ID NO:64 and SEQ ID NO: 961 (preferably SEQ ID Nos: 35, 63, 19 and most preferably SEQ ID NO: 961) is carried out by means of real-time detection methods as described above.

Additional embodiments of the invention provide a method for the analysis of the methylation status of genomic DNA according to the invention SEQ ID NO:1 to SEQ ID NO:64 and SEQ ID NO: 961, (preferably SEQ ID Nos: 35, 63, 19 and most preferably SEQ ID NO: 961) and complements thereof without the need for pretreatment.

In the *first step* of such additional embodiments, the genomic DNA sample is isolated from tissue or cellular sources. Preferably, such sources include cell lines, histological slides, body fluids, or tissue embedded in paraffin. In the *second step,* the genomic DNA is extracted. Extraction may be by means that are standard to one skilled in the art, including but not limited to the use of detergent lysates, sonification and vortexing with glass beads. Once the nucleic acids have been extracted, the genomic double-stranded DNA is used in the analysis.

In a preferred embodiment, the DNA may be cleaved prior to the treatment, and this may be by any means standard in the state of the art, in particular with methylation-sensitive restriction endonucleases.

In the *third step,* the DNA is then digested with one or more methylation sensitive restriction enzymes. The digestion is carried out such that hydrolysis of the DNA at the restriction site is informative of the methylation status of a specific CpG dinucleotide.

In the *fourth step*, which is optional but a preferred embodiment, the restriction fragments are amplified. This is preferably carried out using a polymerase chain reaction, and said amplificates may carry suitable detectable labels as discussed above, namely fluorophore labels, radionuclides and mass labels.

In the *fifth step* the amplificates are detected. The detection may be by any means standard in the art, for example, but not limited to, gel electrophoresis analysis, hybridization analysis, incorporation of detectable tags within the PCR products, DNA array analysis, MALDI or ESI analysis.

Subsequent to the determination of the methylation state of the genomic nucleic acids the prognosis of the prostate cancer is deduced based upon the methylation state of at least one CpG dinucleotide sequence of SEQ ID NO: 1 to SEQ ID NO:64 and SEQ ID NO: 961, or an average, or a value reflecting an average methylation state of a plurality of CpG dinucleotide sequences of SEQ ID NO:1 to SEQ ID NO:64 and SEQ ID NO: 961. Preferably said prognosis is based upon the methylation state of at least one CpG dinucleotide sequence of the genes PITX2, SEQ ID NO: 63, GPR7 and SEQ ID NO: 35 (SEQ ID Nos: 35, 63, 19 and most preferably SEQ ID NO: 961), or an average, or a value reflecting an average methylation state of a plurality of CpG dinucleotide sequences of SEQ ID Nos: 35, 63, 19 and most preferably SEQ ID NO: 961. Hypomethylation of said CpG positions are associated with good prognosis, and hypermethylation is associated with poor prognosis. Hypermethylation is in general associated with under expression of mRNA and accordingly polpeptides. The cut-off point for determining hypo and hyper methylation is may be the median methylation level for a given population, or is preferably an optimized cut-off level. For the analysis of SEQ ID NO: 35 it is preferred that the cut-off is between 30% and 40% methylation, and most preferably 36.42%. For the analysis of SEQ ID NO: 63 it is preferred that the cut-off is between 2% and 10% methylation, and most preferably 5.96%. For the analysis of GPR7 it is preferred that the cut-off is between 20% and 10% methylation, and most preferably 16.65%. For the analysis of PITX2 it is preferred that the cut-off is between 20% and 10% methylation, and most preferably 14.27%.

Wherein the methods according to the present invention of expression analysis (most preferably by means of methylation analysis), of the herein described markers (preferably PITX2, SEQ ID NO: 63, GPR7 and SEQ ID NO: 35 ) are used to determine the prognosis of a prostate cancer said methods are preferably used in combination with other clinical prognostic variables used to determine prognosis most preferably Gleason score but also including nomogram score and PSA level (i.e. that said variables are factored in or taken into account). In a preferred embodiment of the invention prognostic expression analysis of each of the markers PITX2 and SEQ ID NO: 35 as herein described is carried out on patients who present with organ confined (T2) prostate cancer. PITX2 and SEQ ID NO: 35 have a distinct advantage in determinig the prognosis of T2 prostate cancer, whereas it is current clinical practise that all patients presenting with T2 prostate cancer are deemed to have a good prognosis. According to the present invetion said T2 patients with poor prognosis (hypermethylation) would have a prognosis more typical of patients presenting with T3 (non organ-confined) prostate cancer and may be treated appropriately. Furthermore prognostic expression analysis of each of the markers PITX2 and SEQ ID NO: 63 have further utility in the analysis of patients presenting high Gleason score (8 or higher). Said patients are currently considered to have a poor prognosis, however by means of the herein described method of PITX2 and SEQ ID NO: 63 expression analysis it is for the first time possible to differentiate high Gleason score patients with a poor prognosis (hypermethylation) from those with a good prognosis. Currently all patients with high Gleason score are considered candidates for post-surgical adjuvant treatment, accordingly the method according to the invention enables the prevention of over-treatment of said patients. Furthermore prognostic expression analysis of the marker SEQ ID NO: 63 has further utility in the analysis of patients presenting with poor prognosis based on nomogram score.

Kits

Moreover, an additional aspect of the present invention is a kit comprising, for example: a bisulfite-containing reagent; a set of primer oligonucleotides containing at least two oligonucleotides whose sequences in each case correspond, are complementary, or hybridize under stringent or highly stringent conditions to a 16-base long segment of the sequences SEQ ID NO: 1 to SEQ ID NO: 320 and SEQ ID NO: 961 to 965; oligonucleotides and/or PNA-oligomers; as well as instructions for carrying out and evaluating the described method. In a further preferred embodiment, said kit may further comprise standard reagents for performing a CpG position-specific methylation analysis, wherein said analysis comprises one or more of the following techniques: MS-SNuPE. MSP, MethyLight ™, HeavyMethyl™ , COBRA, and nucleic acid sequencing. However, a kit along the lines of the present invention can also contain only part of the aforementioned components.

Preferably said kit comprises a bisulfite-containing reagent; a set of primer oligonucleotides containing at least two oligonucleotides whose sequences in each case correspond, are complementary, or hybridize under stringent or highly stringent conditions to a 16-base long segment of the sequences SEQ ID Nos: 133,134,261,262, 189,190,317,318, 101,102,229,230 and most preferably SEQ ID Nos: 962-965; oligonucleotides and/or PNA-oligomers; as well as instructions for carrying out and evaluating the described method. In a further preferred embodiment, said kit may further comprise standard reagents for performing a CpG position-specific methylation analysis, wherein said analysis comprises one or more of the following techniques: MS-SNuPE, MSP, MethyLight ™, HeavyMethyl™ , COBRA, and nucleic acid sequencing. However, a kit along the lines of the present invention can also contain only part of the aforementioned components.

The described invention further provides a composition of matter useful for providing a prognosis of prostate cancer patients. Said composition comprising at least one nucleic acid 18 base pairs in length of a segment of a nucleic acid sequence selected from the group consisting SEQ ID Nos: 133,134,261,262, 189,190,317,318, 101,102,229,230, 962 - 965, and one or more substances taken from the group comprising : magnesium chloride, dNTP, taq polymerase, bovine serum albumen, an oligomer in particular an oligonucleotide or peptide nucleic acid (PNA)-oligomer, said oligomer comprising in each case at least one base sequence having a length of at least 9 nucleotides which is complementary to, or hybridizes under moderately stringent or stringent conditions to a pretreated genomic DNA according to one of the SEQ ID Nos: 133,134,261,262, 189,190,317,318, 101,102,229,230, 962 - 965 and sequences complementary thereto. It is preferred that said composition of matter comprises a buffer solution appropriate for the stabilization of said nucleic acid in an aqueous solution and enabling polymerase based reactions within said solution. Suitable buffers are known in the art and commercially available.

In one embodiment the invention provides a method for providing a diagnosis of prostate carcinoma or neoplasm in a subject. Said method comprises the following steps:

i) determining the expression levels of one or more genes or gene sequences of or according to CDRN2A, ELK1, GSTP1, RARB, PTGS2, RASSF1, ESR2, ONECUT2, BTG4, SLC35F2, HOXB5, LIMK1, HIST1H4J, SEQ ID NO: 35, EPAS1, NOTCH1, SEQ ID NO: 55, PTPRN2, Q9NP73, MX1, DOCK10, CCND2, ISL1, SNAPC2, GRN, H2AFY2, WDFY3, FOS, FAT, Q86SP6, SLC38A1, SNRPN, GPRK5, FBN2, ARHGEF18, RHOC, KBTBD6, NR2E1, PSD, DRG1, Q8N365, SEQ ID NO: 44, Q96S01, CD37, CMYA3, SEQ ID NO: 61, Q8NCX8 and ZNF566 and/or regulatory regions thereof; and

ii) determining the presence or absence of said prostate carcinoma or neoplasm according to said level of expression.

Said expression level may be determined by any means standard in the art including but not limited to methylation analysis, loss of heterozygosity (hereinafter also referred to as LOH), RNA expression levels and protein expression levels.

Accordingly said method ma be enabled by means of any analysis of the expression of a RNA transcribed therefrom or polypeptide or protein translated from said RNA, preferably by means of mRNA expression analysis or polypeptide expression analysis.

Accordingly the prognostic assays and methods, both quantitative and qualitative for detecting the expression of the genes, genomic sequences and/or regulatory regions according to Table 11 as applied to CDRN2A, ELK1, GSTP1, RARB, PTGS2, RASSF1, ESR2, ONECUT2, BTG4, SLC35F2, HOXB5, LIMK1, HIST1H4J, SEQ ID NO: 35, EPAS1, NOTCH1, SEQ ID NO: 55, PTPRN2, Q9NP73, MX1, DOCK10, CCND2, ISL1, SNAPC2, GRN, H2AFY2, WDFY3, FOS, FAT, Q86SP6, SLC38A1, SNRPN, GPRK5, FBN2, ARHGEF18, RHOC, KBTBD6, NR2E1, PSD, DRG1, Q8N365, SEQ ID NO: 44, Q96S01, CD37, CMYA3, SEQ ID NO: 61, Q8NCX8 and ZNF566 are suitable for said diagnostic purposes. Furthermore the compositions of matter, kits and nucleic acids as described above for analysis of the genes and sequences according to Table 11 above are also of use in the analysis of CDRN2A, ELK1, GSTP1, RARB, PTGS2, RASSF1, ESR2, ONECUT2, BTG4, SLC35F2, HOXB5, LIMK1, HIST1H4J, SEQ ID NO: 35, EPAS1, NOTCH1, SEQ ID NO: 55, PTPRN2, Q9NP73, MX1, DOCK10, CCND2, ISL1, SNAPC2, GRN, H2AFY2, WDFY3, FOS, FAT, Q86SP6, SLC38A1, SNRPN, GPRK5, FBN2, ARHGEF18, RHOC, KBTBD6, NR2E1, PSD, DRG1, Q8N365, SEQ ID NO: 44, Q96S01, CD37, CMYA3, SEQ ID NO: 61, Q8NCX8 and ZNF566 and therefore are also applicable in the detection of prostate carcinoma or neoplasms.

While the present invention has been described with specificity in accordance with certain of its preferred embodiments, the following EXAMPLES and TABLES serve only to illustrate the invention and are not intended to limit the invention within the principles and scope of the broadest interpretations and equivalent configurations thereof.

## TABLES 1-12

TABLE 1: Experimental set-up of MeST screening with number of samples in pools.

| Experiment | Number of comparisons | "Aggressive" group | "Nonaggressive" group |
|---|---|---|---|
| PSA recurrence vs. no recurrence | 3 | 5 tumors from patients who had PSA recurrences <2yr after surgery | 5 tumors from patients without PSA recurrence after at least 4 years follow up |
| Late stage vs Early stage | 1 | 5 stage III and IV tumors | 5 stage I and II tumors |
| High Gleason vs. Low Gleason | 1 | 5 grade 4 or 5 tumors | 5 grade 1, 2, or 3 tumors |
| NAT (PSA recurrence) vs. NAT (no recurrence) | 1 | 4 normal samples from tissue adjacent to tumors from patients who had PSA recurrences <2yr after surgery | 4 normal tissue samples adjacent to tumors from patients without PSA recurrence after at least 4 years follow up |
| Peripheral Zone vs. Transition Zone | 1 | 5 peripheral zone tumors | 5 transition zone tumors |

TABLE 2. Summarized results of MeST Screening experiments

| Experiment: | |
|---|---|
| Total number of MeSTs<br>scoring > 0 | 441<br>278 |
| MeSTs from MCA<br>scoring > 0 | 242<br>126 |
| MeSTs from AP-PCR<br>scoring > 0 | 199<br>152 |

TABLE 3: Total number of MeSTs and number of positive MeSTs from each screening comparison.

| Experiment | Number of MeSTs | Number of Positive MeSTs | Percent Positive MeSTs |
|---|---|---|---|
| PSA recurrence vs no recurrence I | 41 | 28 | 68% |
| FSA recurrence vs. no recurrence II | 113 | 74 | 65% |
| PSA recurrence vs. no recurrence II | 69 | 43 | 62% |
| Late stage vs Early stage | 64 | 39 | 61 % |
| High Gteason vs. Low Gleason | 63 | 48 | 76% |
| NAT (PSA recurrence) vs: NAT (no recurrence) | 103 | 66 | 64% |
| Peripheral Zone vs. Transition Zone | 76 | 48 | 63% |

TABLE 4 Summary of Real-time PCR data for seven candidates. P values are from a Wilcoxon test and are uncorrected for multiple comparisons. The sensitivity is calculated when the specificity is set at 0.85 or greater.

| Candidate | Gene-Name | AUC | Sensitivity | Specificity | P value (Wilcoxon) |
|---|---|---|---|---|---|
| SEO ID NO: 19 | GPR7 | 0.76 | 0.50 | 0.87 | 0.0008 |
| SEQ ID NO: 35 | Genomic region downstream of FOXL2 | 0.75 | 0.54 | 0.87 | 0.0016 |
| SEQ ID NO: 37 | ABHD9 | 0.7 | 0.38 | 0.86 | 0.0115 |
| SEQ ID NO: 7 | GSTP1 | 0.69 | 0.38 | 0.87 | 0.0176 |
| SEQ ID NO: 63 | HIST2H2BF regulatory region | 0.68 | 0.35 | 0.87 | 0.0241 |
| SEQ ID NO: 8 | RARB | 0.68 | 0.50 | 0.85 | 0.0214 |
| SEQ ID NO: 64 | PMF1 | 0.47 | 0.15 | 0.87 | 0.7138 |

TABLE 5 A summary of the samples used in the chip study. For the outcome categories, "No recurrence" means the patient did not have a relapse and at least 48 months follow up information was available. "Early recurrence" indicates that the patient experienced PSA relapse in less than 2 years after surgery. "Other" includes patients with no follow up information and patients who did not fit the criteria for the recurrence categories.

| Gleason Categories | Sample Number | Outcome categories |
|---|---|---|
| A) Low Gleason (1+2, 2+1, 2+2, 2+3, 3+2, and 3+3) | 135 | 1. No recurrence (n=42) |
| | | 2. Early recurrence (n=6) |
| | | 3. Other (n=87) |
| B) Intermediate Gleason (2+4, 4+2, 3+4, 4+3, 2+5, 5+2) | 73 | 1. No recurrence (n=34) |
| | | 2. Early recurrence (n=33) |
| | | 3. Other (n=6) |
| C) High Gleason (3+5, 5+3, 4+4, 4+5, 5+4, and 5+5) | 99 | 1. No recurrence (n=9) |
| | | 2. Early recurrence (n=26) |
| | | 3. Other (n=64) |
| D) No Gleason information | 4 | 1. No recurrence (n=3) |
| | | 2. Early recurrence (n=0) |
| | | 3. Other (n=1) |

TABLE 6 Corrected Wilcoxon p-value / AUC / Sensitivity / Specificity of the amplificates. Sensitivity is reported at a fixed specificity of -0.75.

| Marker | p-value | AUC | Sensitivity | Specificity |
|---|---|---|---|---|
| SEQ ID NO: 19 | 2.05E-06 | 0.72 | 0.58 | 0.75 |
| SEQ ID NO: 41 | 2.17E-06 | 0.71 | 0.51 | 0.75 |
| SEQ ID NO: 37 | 4.15E-06 | 0.71 | 0.55 | 0.75 |
| SEQ ID NO: 51 | 8.68E-06 | 0.71 | 0.51 | 0.75 |
| SEQ ID NO: 35 | 2.60E-05 | 0.70 | 0.48 | 0.75 |
| SEQ ID NO: 4 | 5.52E-05 | 0.69 | 0.51 | 0.75 |
| SEQ ID NO: 17 | 5.89E-05 | 0.69 | 0.51 | 0.75 |
| SEQ ID NO: 16 | 6.82E-05 | 0.69 | 0.47 | 0.75 |
| SEQ ID NO: 9 | 9.30E-05 | 0.69 | 0.57 | 0.75 |
| SEQ ID NO: 47 | 1.80E-04 | 0.68 | 0.46 | 0.75 |
| SEQ ID NO: 49 | 2.05E-04 | 0.68 | 0.49 | 0.75 |
| SEQ ID NO: 42 | 3.84E-04 | 0.68 | 0.45 | 0.75 |
| SEQ ID NO: 57 | 4.34E-04 | 0.68 | 0.49 | 0.75 |
| SEQ ID NO: 1 | 7.44E-04 | 0.67 | 0.51 | 0.75 |
| SEQ ID NO: 7 | 8.06E-04 | 0.67 | 0.43 | 0.75 |
| SEQ ID NO: 62 | 8.06E-04 | 0.67 | 0.49 | 0.75 |
| SEQ ID NO: 8 | 9.30E-04 | 0.67 | 0.47 | 0.75 |
| SEQ ID NO: 58 | 1.24E-03 | 0.67 | 0.46 | 0.75 |
| SEQ ID NO: 3 | 2.79E-03 | 0.66 | 0.48 | 0.75 |

(continued)

| Marker | p-value | AUC | Sensitivity | Specificity |
|---|---|---|---|---|
| SEQ ID NO: 13 | 3.84E-03 | 0.66 | 0.45 | 0.75 |
| SEQ ID NO: 23 | 8.68E-03 | 0.65 | 0.43 | 0.75 |
| SEQ ID NO: 29 | 9.30E-03 | 0.65 | 0.41 | 0.75 |
| SEQ ID NO: 39 | 9.30E-03 | 0.65 | 0.41 | 0.75 |
| SEQ ID NO: 5 | 1.05E-02 | 0.65 | 0.47 | 0.75 |
| SEQ ID NO: 56 | 0.03 | 0.64 | 0.46 | 0.75 |
| SEQ ID NO: 10 | 0.10 | 0.62 | 0.41 | 0.75 |
| SEQ ID NO: 2 | 0.11 | 0.62 | 0.40 | 0.75 |
| SEQ ID NO: 6 | 0.13 | 0.62 | 0.40 | 0.75 |
| SEQ ID NO: 50 | 0.25 | 0.61 | 0.42 | 0.75 |
| SEQ ID NO: 33 | 0.36 | 0.61 | 0.47 | 0.75 |
| SEQ ID NO: 15 | 0.37 | 0.61 | 0.35 | 0.75 |
| SEQ ID NO: 60 | 0.39 | 0.61 | 0.43 | 0.75 |
| SEQ ID NO: 55 | 0.46 | 0.60 | 0.31 | 0.75 |
| SEQ ID NO: 52 | 0.47 | 0.60 | 0.38 | 0.75 |
| SEQ ID NO: 20 | 0.87 | 0.60 | 0.33 | 0.75 |
| SEQ ID NO: 21 | 0.87 | 0.60 | 0.42 | 0.75 |
| SEQ ID NO: 24 | 1.00 | 0.59 | 0.35 | 0.75 |
| SEQ ID NO: 54 | 1.00 | 0.59 | 0.36 | 0.75 |
| SEQ ID NO: 30 | 1.00 | 0.59 | 0.36 | 0.75 |
| SEQ ID NO: 43 | 1.00 | 0.42 | 0.22 | 0.75 |
| SEQ ID NO: 48 | 1.00 | 0.58 | 0.35 | 0.75 |
| SEQ ID NO: 45 | 1.00 | 0.58 | 0.32 | 0.75 |
| SEQ ID NO: 26 | 1.00 | 0.58 | 0.38 | 0.75 |
| SEQ ID NO: 31 | 1.00 | 0.57 | 0.42 | 0.75 |
| SEQ ID NO: 38 | 1.00 | 0.56 | 0.34 | 0.75 |
| SEQ ID NO: 28 | 1.00 | 0.56 | 0.36 | 0.75 |
| SEQ ID NO: 32 | 1.00 | 0.56 | 0.33 | 0.75 |
| SEQ ID NO: 40 | 1.00 | 0.56 | 0.30 | 0.75 |
| SEQ ID NO: 27 | 1.00 | 0.55 | 0.34 | 0.75 |
| SEQ ID NO: 61 | 1.00 | 0.45 | 0.21 | 0.75 |
| SEQ ID NO: 11 | 1.00 | 0.54 | 0.34 | 0.75 |
| SEQ ID NO: 44 | 1.00 | 0.54 | 0.28 | 0.75 |
| SEQ ID NO: 53 | 1.00 | 0.54 | 0.21 | 0.75 |
| SEQ ID NO: 22 | 1.00 | 0.54 | 0.28 | 0.75 |
| SEQ ID NO: 18 | 1.00 | 0.47 | 0.20 | 0.75 |
| SEQ ID NO: 59 | 1.00 | 0.47 | 0.25 | 0.75 |
| SEQ ID NO: 36 | 1.00 | 0.47 | 0.22 | 0.75 |

(continued)

| Marker | p-value | AUC | Sensitivity | Specificity |
|---|---|---|---|---|
| SEQ ID NO: 12 | 1.00 | 0.53 | 0.24 | 0.75 |
| SEQ ID NO: 34 | 1.00 | 0.52 | 0.27 | 0.75 |
| SEQ ID NO: 46 | 1.00 | 0.48 | 0.19 | 0.75 |
| SEQ ID NO: 25 | 1.00 | 0.51 | 0.25 | 0.75 |
| SEQ ID NO: 14 | 1.00 | 0.51 | 0.24 | 0.75 |

TABLE 7: Corrected Wilcoxon p-value / AUC / Sensitivity / Specificity of the amplificates. Sensitivity is reported at a fixed specificity of -0.75.

| Marker | p-value | AUC | Sensitivity | Specificity |
|---|---|---|---|---|
| SEQ ID NO: 19 | 2.42E-04 | 0.72 | 0.59 | 0.76 |
| SEQ ID NO: 35 | 2.48E-04 | 0.72 | 0.59 | 0.76 |
| SEQ ID NO: 37 | 0.05 | 0.66 | 0.37 | 0.76 |
| SEO ID NO: 20 | 0.07 | 0.66 | 0.44 | 0.76 |
| SEQ ID NO: 13 | 0.08 | 0.65 | 0.46 | 0.76 |
| SEQ ID NO: 9 | 0.11 | 0.65 | 0.37 | 0.76 |
| SEQ ID NO: 41 | 0.11 | 0.65 | 0.37 | 0.76 |
| SEQ ID NO: 57 | 0.11 | 0.65 | 0.41 | 0.76 |
| SEQ ID NO: 51 | 0.12 | 0.65 | 0.49 | 0.76 |
| SEQ ID NO: 4 | 0.22 | 0.64 | 0.40 | 0.76 |
| SEQ ID NO: 1 | 0.24 | 0.64 | 0.41 | 0.76 |
| SEQ ID NO: 10 | 0.57 | 0.62 | 0.38 | 0.76 |
| SEQ ID NO: 15 | 0.61 | 0.62 | 0.38 | 0.76 |
| SEQ ID NO: 17 | 0.74 | 0.62 | 0.32 | 0.76 |
| SEQ ID NO: 58 | 0.93 | 0.62 | 0.40 | 0.76 |
| SEQ ID NO: 62 | 1.00 | 0.61 | 0.49 | 0.76 |
| SEQ ID NO: 43 | 1.00 | 0.39 | 0.11 | 0.76 |
| SEQ ID NO: 29 | 1.00 | 0.61 | 0.37 | 0.76 |
| SEQ ID NO: 16 | 1.00 | 0.60 | 0.35 | 0.76 |
| SEQ ID NO: 23 | 1.00 | 0.60 | 0.40 | 0.76 |
| SEQ ID NO: 5 | 1.00 | 0.60 | 0.44 | 0.76 |
| SEQ ID NO: 42 | 1.00 | 0.60 | 0.32 | 0.76 |
| SEQ ID NO: 49 | 1.00 | 0.59 | 0.37 | 0.76 |
| SEQ ID NO: 3 | 1.00 | 0.59 | 0.40 | 0.76 |
| SEQ ID NO: 47 | 1.00 | 0.59 | 0.35 | 0.76 |
| SEQ ID NO: 7 | 1.00 | 0.59 | 0.27 | 0.76 |
| SEQ ID NO: 28 | 1.00 | 0.59 | 0.37 | 0.76 |
| SEQ ID NO: 2 | 1.00 | 0.59 | 0.29 | 0.76 |
| SEQ ID NO: 18 | 1.00 | 0.42 | 0.14 | 0.76 |

(continued)

| Marker | p-value | AUC | Sensitivity | Specificity |
|---|---|---|---|---|
| SEQ ID NO: 45 | 1.00 | 0.58 | 0.38 | 0.76 |
| SEQ ID NO: 8 | 1.00 | 0.58 | 0.25 | 0.76 |
| SEQ ID NO: 46 | 1.00 | 0.42 | 0.13 | 0.76 |
| SEQ ID NO: 30 | 1.00 | 0.58 | 0.32 | 0.76 |
| SEQ ID NO: 39 | 1.00 | 0.58 | 0.35 | 0.76 |
| SEQ ID NO: 54 | 1.00 | 0.57 | 0.32 | 0.76 |
| SEQ ID NO: 25 | 1.00 | 0.43 | 0.17 | 0.76 |
| SEQ ID NO: 6 | 1.00 | 0.57 | 0.30 | 0.76 |
| SEQ ID NO: 11 | 1.00 | 0.57 | 0.41 | 0.76 |
| SEQ ID NO: 36 | 1.00 | 0.57 | 0.33 | 0.76 |
| SEQ ID NO: 60 | 1.00 | 0.57 | 0.32 | 0.76 |
| SEO ID NO: 61 | 1.00 | 0.43 | 0.13 | 0.76 |
| SEO ID NO: 55 | 1.00 | 0.56 | 0.27 | 0.76 |
| SEQ ID NO: 33 | 1.00 | 0.56 | 0.27 | 0.76 |
| SEQ ID NO: 21 | 1.00 | 0.56 | 0.21 | 0.76 |
| SEQ ID NO: 56 | 1.00 | 0.56 | 0.32 | 0.76 |
| SEQ ID NO: 24 | 1.00 | 0.56 | 0.27 | 0.76 |
| SEQ ID NO: 38 | 1.00 | 0.55 | 0.37 | 0.76 |
| SEQ ID NO: 48 | 1.00 | 0.55 | 0.33 | 0.76 |
| SEQ ID NO: 26 | 1.00 | 0.45 | 0.22 | 0.76 |
| SEQ ID NO: 59 | 1.00 | 0.54 | 0.38 | 0.76 |
| SEQ ID NO: 34 | 1.00 | 0.47 | 0.22 | 0.76 |
| SEQ ID NO: 52 | 1.00 | 0.53 | 0.22 | 0.76 |
| SEQ ID NO: 50 | 1.00 | 0.53 | 0.27 | 0.76 |
| SEQ ID NO: 14 | 1.00 | 0.47 | 0.21 | 0.76 |
| SEQ ID NO: 22 | 1.00 | 0.47 | 0.27 | 0.76 |
| SEQ ID NO: 32 | 1.00 | 0.52 | 0.29 | 0.76 |
| SEQ ID NO: 53 | 1.00 | 0.52 | 0.25 | 0.76 |
| SEQ ID NO: 44 | 1.00 | 0.48 | 0.27 | 0.76 |
| SEQ ID NO: 40 | 1.00 | 0.49 | 0.13 | 0.76 |
| SEQ ID NO: 27 | 1.00 | 0.50 | 0.33 | 0.76 |
| SEQ ID NO: 12 | 1.00 | 0.50 | 0.21 | 0.76 |
| SEQ ID NO: 31 | 1.00 | 0.50 | 0.16 | 0.76 |

TABLE 8: Corrected Wilcoxon p-value / AUC / Sensitivity / Specificity of the amplificates. Sensitivity is reported at a fixed specificity of ~0.75.

| Marker | p-value | AUC | Sensitivity | Specificity |
|---|---|---|---|---|
| SEQ ID NO: 19 | 2.42E-04 | 0.72 | 0.59 | 0.76 |

(continued)

| Marker | p-value | AUC | Sensitivity | Specificity |
|---|---|---|---|---|
| SEQ ID NO: 35 | 2.48E-04 | 0.72 | 0.59 | 0.76 |
| SEQ ID NO: 37 | 0.05 | 0.66 | 0.37 | 0.76 |
| SEQ ID NO: 20 | 0.07 | 0.66 | 0.44 | 0.76 |
| SEQ ID NO: 13 | 0.08 | 0.65 | 0.46 | 0.76 |
| SEQ ID NO: 9 | 0.11 | 0.65 | 0.37 | 0.76 |
| SEQ ID NO: 41 | 0.11 | 0.65 | 0.37 | 0.76 |
| SEQ ID NO: 57 | 0.11 | 0.65 | 0.41 | 0.76 |
| SEQ ID NO: 51 | 0.12 | 0.65 | 0.49 | 0.76 |
| SEQ ID NO: 4 | 0.22 | 0.64 | 0.40 | 0.76 |
| SEQ ID NO: 1 | 0.24 | 0.64 | 0.41 | 0.76 |
| SEQ ID NO: 10 | 0.57 | 0.62 | 0.38 | 0.76 |
| SEQ ID NO: 15 | 0.61 | 0.62 | 0.38 | 0.76 |
| SEQ ID NO: 17 | 0.74 | 0.62 | 0.32 | 0.76 |
| SEQ ID NO: 58 | 0.93 | 0.62 | 0.40 | 0.76 |
| SEQ ID NO: 62 | 1.00 | 0.61 | 0.49 | 0.76 |
| SEQ ID NO: 43 | 1.00 | 0.39 | 0.11 | 0.76 |
| SEQ ID NO: 29 | 1.00 | 0.61 | 0.37 | 0.76 |
| SEQ ID NO: 16 | 1.00 | 0.60 | 0.35 | 0.76 |
| SEQ ID NO: 23 | 1.00 | 0.60 | 0.40 | 0.76 |
| SEQ ID NO: 5 | 1.00 | 0.60 | 0.44 | 0.76 |
| SEQ ID NO: 42 | 1.00 | 0.60 | 0.32 | 0.76 |
| SEQ ID NO: 49 | 1.00 | 0.59 | 0.37 | 0.76 |
| SEQ ID NO: 3 | 1.00 | 0.59 | 0.40 | 0.76 |
| SEQ ID NO: 47 | 1.00 | 0.59 | 0.35 | 0.76 |
| SEQ ID NO: 7 | 1.00 | 0.59 | 0.27 | 0.76 |
| SEQ ID NO: 28 | 1.00 | 0.59 | 0.37 | 0.76 |
| SEQ ID NO: 2 | 1.00 | 0.59 | 0.29 | 0.76 |
| SEQ ID NO: 18 | 1.00 | 0.42 | 0.14 | 0.76 |
| SEQ ID NO: 45 | 1.00 | 0.58 | 0.38 | 0.76 |
| SEQ ID NO: 8 | 1.00 | 0.58 | 0.25 | 0.76 |
| SEQ ID NO: 46 | 1.00 | 0.42 | 0.13 | 0.76 |
| SEQ ID NO: 30 | 1.00 | 0.58 | 0.32 | 0.76 |
| SEQ ID NO: 39 | 1.00 | 0.58 | 0.35 | 0.76 |
| SEQ ID NO: 54 | 1.00 | 0.57 | 0.32 | 0.76 |
| SEQ ID NO: 25 | 1.00 | 0.43 | 0.17 | 0.76 |
| SEQ ID NO: 6 | 1.00 | 0.57 | 0.30 | 0.76 |
| SEQ ID NO: 11 | 1.00 | 0.57 | 0.41 | 0.76 |
| SEQ ID NO: 36 | 1.00 | 0.57 | 0.33 | 0.76 |

(continued)

| Marker | p-value | AUC | Sensitivity | Specificity |
|---|---|---|---|---|
| SEQ ID NO: 60 | 1.00 | 0.57 | 0.32 | 0.76 |
| SEQ ID NO: 61 | 1.00 | 0.43 | 0.13 | 0.76 |
| SEQ ID NO: 55 | 1.00 | 0.56 | 0.27 | 0.76 |
| SEQ ID NO: 33 | 1.00 | 0.56 | 0.27 | 0.76 |
| SEQ ID NO: 21 | 1.00 | 0.56 | 0.21 | 0.76 |
| SEQ ID NO: 56 | 1.00 | 0.56 | 0.32 | 0.76 |
| SEQ ID NO: 24 | 1.00 | 0.56 | 0.27 | 0.76 |
| SEQ ID NO: 38 | 1.00 | 0.55 | 0.37 | 0.76 |
| SEQ ID NO: 48 | 1.00 | 0.55 | 0.33 | 0.76 |
| SEQ ID NO: 26 | 1.00 | 0.45 | 0.22 | 0.76 |
| SEQ ID NO: 59 | 1.00 | 0.54 | 0.38 | 0.76 |
| SEQ ID NO: 34 | 1.00 | 0.47 | 0.22 | 0.76 |
| SEQ ID NO: 52 | 1.00 | 0.53 | 0.22 | 0.76 |
| SEQ ID NO: 50 | 1.00 | 0.53 | 0.27 | 0.76 |
| SEQ ID NO: 14 | 1.00 | 0.47 | 0.21 | 0.76 |
| SEQ ID NO: 22 | 1.00 | 0.47 | 0.27 | 0.76 |
| SEQ ID NO: 32 | 1.00 | 0.52 | 0.29 | 0.76 |
| SEQ ID NO: 53 | 1.00 | 0.52 | 0.25 | 0.76 |
| SEQ ID NO: 44 | 1.00 | 0.48 | 0.27 | 0.76 |
| SEQ ID NO: 40 | 1.00 | 0.49 | 0.13 | 0.76 |
| SEQ ID NO: 27 | 1.00 | 0.50 | 0.33 | 0.76 |
| SEQ ID NO: 12 | 1.00 | 0.50 | 0.21 | 0.76 |
| SEQ ID NO: 31 | 1.00 | 0.50 | 0.16 | 0.76 |

TABLE 9: Primers according to EXAMPLE 3.

| ***Gene*** | ***Primers:*** | ***Amplificate Length:*** |
|---|---|---|
| CCND2 (SEQ ID NO: 1) | AAAAACAACCTTAACTC AAACAT (SEQ ID NO: 322)<br>TTTGGAGGGATAGAAT GTGA (SEQ ID NO: 321) | 504 |
| CDKN2A (SEQ ID NO: 2) | AGATTATTAGTTTTTATT TGAGGGATT (SEQ ID NO: 323) | |

(continued)

| Gene | Primers: | Amplificate Length: |
|---|---|---|
| | CCACCCTAACTCTAACC ATTC (SEQ ID NO: 324) | |
| CD44 (SEQ ID NO: 3) | TTTTTGTTTGGGTGTGT TTT | 403 |
| | (SEQ ID NO: 325) CACTTAACTCCAATCCC CC (SEQ ID NO: 326) | |
| EDNRB1 (SEQ ID NO: 4) | CAAAAACTTCTCAAATC AACAA (SEQ ID NO: 328) GAAGGGATGAATGAAT AAAAGT (SEQ ID NO: 327) | 446 |
| ELK1 (SEQ ID NO: 5) | TCCAATAAACACAAACC TAAATC (SEQ ID NO: 330) ATATGGGATTGATGGA AGATAG (SEQ ID NO: 329) | |
| FOS (SEQ ID NO: 6) | TTTTTATTTAGGATGAG GGATATT (SEQ ID NO: 331) ACTACTTCCCACCCAAC C (SEQ ID NO: 332) | |
| GSTP1 (SEQ ID NO: 7) | CTACTATCTATTTACTC CCTAAAC (SEQ ID NO: 334) TTGGTTTTATGTTGGGA GTTTTG (SEQ ID NO: 333) | 347 |

(continued)

| Gene | Primers: | Amplificate Length: |
|---|---|---|
| RARB (SEQ ID NO: 8) | GGGAGTTTTTAAGTTTT GTGAG (SEQ ID NO: 335) TTAATCTTTTTCCCAAC CC (SEQ ID NO: 336) | 488 |
| PTGS2 (SEQ ID NO: 9) | AACATATCAACCTTTCT TAACCTT (SEQ ID NO: 338) AGAGGGGGTAGTTTTT ATTTTT | 344 |
| | (SEQ ID NO: 337) | |
| RASSF1 (SEQ ID NO: 10) | AGTGGGTAGGTTAAGT GTGTTG (SEQ ID NO: 339) CCCCAAAATCCAAACTA AA (SEQ ID NO: 340) | 319 |
| ESR2 (SEQ ID NO: 11) | AGTTGGAGAAATTGAAA AGATTA (SEQ ID NO: 341) TAACAAACCCAAAACCT CTCTA (SEQ ID NO: 342) | 441 |
| DRG1 (SEQ ID NO: 12) | TTTAGTTGTGAAAAAGG GATTT (SEQ ID NO: 343) CCCTATAACCTCCACAC TATCTC (SEQ ID NO: 344) | 436 |
| DRG1 (SEQ ID NO: 12) | CCCATCCCACAATTAAA A (SEQ ID NO: 346) | |

(continued)

| Gene | Primers: | Amplificate Length: |
|---|---|---|
| | GTTTTGGAGGGAGTAG AGATT (SEQ ID NO: 345) | |
| CMYA3 (SEQ ID NO: 13) | ACTCCCCAAAATCCCA CT (SEQ ID NO: 348) TGTTTTAGGTTTGATGG ATTAGA (SEQ ID NO: 347) | 488 |
| ONECUT2 (SEQ ID NO: 14) | GAAGAGGTGTTGAGAA ATTAAAA (SEQ ID NO: 349) CCCACCCTAACTTACCA TAAA (SEQ ID NO: 350) | 462 |
| MX1 (SEQ ID NO: 15) | TGTAGGAGAGGTTGGG AAG | 341 |
| | (SEQ ID NO: 351) CCAAACATAACATCCAC TAAAA (SEQ ID NO: 352) | |
| DOCK10 (SEQ ID NO: 16) | TACCTCTTCCCTCTACC AAAC (SEQ ID NO: 354) GTTTTTAAGTGTTGGGT GATTT (SEQ ID NO: 353) | 459 |
| BTG4 (SEQ ID NO: 17) | AAGAGTTTTAGGAAATG TGTTTTT (SEQ ID NO: 355) TTCTACTCACCAAACCC TCTAC (SEQ ID NO: 356) | 199 |

(continued)

| *Gene* | *Primers:* | *Amplificate Length:* |
|---|---|---|
| DMRTC2 (SEQ ID NO: 18) | TAAGGTAAGGGAAGGT TAGAAA (SEQ ID NO: 357) ATTACCACAACCTCCAA TAAAA (SEQ ID NO: 358) | 477 |
| GPR7 (SEQ ID NO: 19) | TTATTATTTTAGATGGA GTGAGGTT (SEQ ID NO: 359) ACCCAAATTACCCCACA A (SEQ ID NO: 360) | 442 |
| FAT (SEQ ID NO: 20) | TACTCACCCCAATCTTC ACTA (SEQ ID NO: 362) AGATGTTTTATATTTGT TTGGGA (SEQ ID NO: 361) | 444 |
| ISL1 (SEQ ID NO: 21) | ATCTCCCAAAAACAATC ACA (SEQ ID NO: 364) TAAAAATGGAAGGGAA GATAGA | 412 |
| | (SEQ ID NO: 363) | |
| GPRK5 (SEQ ID NO: 22) | TTGTGGTTATTTTGAGA TGGTA (SEQ ID NO: 365) CCCTCCCCCTAACTAAA A (SEQ ID NO: 366) | 416 |
| SLC35F2 (SEQ ID NO: 23) | TTTATTTATTAGGTGAA GAGTTTGTT (SEQ ID NO: 367) | 366 |

(continued)

| Gene | Primers: | Amplificate Length: |
|---|---|---|
| | TCCTCCTACCACCCTAA AA (SEQ ID NO: 368) | |
| C14orf59 (SEQ ID NO: 24) | AAAACTCCTCCCCTCTA TAAAT (SEQ ID NO: 370) TTGGAGAGATGTGTTG GTTAG (SEQ ID NO: 369) | 492 |
| SNRPN (SEQ ID NO: 25) | CCCCTCTCATTACAACA ATACT (SEQ ID NO: 372) TTTTTAGAATAAAGGAT TTTAGGG (SEQ ID NO: 371) | 407 |
| ARHGEF18 (SEQ ID NO: 26) | TTTTAGGAATGTAGGAT ATAAGGG (SEQ ID NO: 373) CCCCACATAAAAACCTA TCC (SEQ ID NO: 374) | 454 |
| SNX8 (SEQ ID NO: 27) | TCCCTCCTAACTCAACA CTAA (SEQ ID NO: 376) TAGGGTTTGATGATGT GATTTT (SEQ ID NO: 375) | 273 |
| FBN2 (SEQ ID NO: 28) | GAGAGGGAGGGTTAAG GTT | 193 |
| | (SEQ ID NO: 377) ACTACTACCTCCTTTCC CAAAT (SEQ ID NO: 378) | |

(continued)

| Gene | Primers: | Amplificate Length: |
|---|---|---|
| HOXB5 (SEQ ID NO: 29) | CTCCTCAATTCTCACCA AAA (SEQ ID NO: 380) GTGGAAAAAGGAGAGT AAATTG (SEQ ID NO: 379) | 356 |
| LIMK1 (SEQ ID NO: 30) | AAACCCTACTTCCTACA AACAA (SEQ ID NO: 382) AGGGAGGTTTGGTGTA TTTT (SEQ ID NO: 381) | |
| PSD/Q9H469 (SEQ ID NO: 31) | AAGGTATTATTTTTGGG GTTTT (SEQ ID NO: 383) AACTATCCAACCTCTTC CACTT (SEQ ID NO: 384) | 333 |
| SLC38A1 (SEQ ID NO: 32) | GGGTGTTGGGGAGTTT TA (SEQ ID NO: 385) CTTACAATAACTCACTA TCCTTTCC (SEQ ID NO: 386) | 334 |
| SLC38A1 (SEQ ID NO: 32) | ACAAACATCCTTTAATA ATTTCTCC (SEQ ID NO: 388) AGAGTGTGGTATTAGAT TTGGTTTT (SEQ ID NO: 387) | 317 |
| HIST1H4J (SEQ ID NO: 33) | TTAGTTGAGAAAGTGG GGGT (SEQ ID NO: 389) | 421 |

(continued)

| *Gene* | *Primers:* | *Amplificate Length:* |
|---|---|---|
| | CTACCTCAAACCAAAAT | |
| | (SEQ ID NO: 390) | |
| Q96S01 (SEQ ID NO: 34) | ACCCCAATCAACTACAT AACTAA (SEQ ID NO: 392) GTGAGAGTGGGTGTTG AAAT (SEQ ID NO: 391) | 498 |
| Genomic region downstream of FOXL2 (SEQ ID NO: 35) | ATTTTAGGTGTAAGTTT AAGGTTGT (SEQ ID NO: 393) ATCTACCTTTCCCCACC C (SEQ ID NO: 394) | 473 |
| ORC4L (SEQ ID NO: 36) | GTTGAGAGGTAAGGTA TGAAGG (SEQ ID NO: 395) TTAATTCCCCTCTTTAA CCTAATAA (SEQ ID NO: 396) | 477 |
| ABHD9 (SEQ ID NO: 37) | GTGTTAGGGTTTAGGG GTTT (SEQ ID NO: 397) CCTTTCCAACCTCTTCC T (SEQ ID NO: 398) | 209 |
| CD37 (SEQ ID NO: 38) | CCTCATCAACCAACCCT A (SEQ ID NO: 400) TAGATGGGGATAGGAA GTTGT (SEQ ID NO: 399) | 466 |

(continued)

| Gene | Primers: | Amplificate Length: |
|---|---|---|
| GRN (SEQ ID NO: 39) | CATTCCAAACTAACCCCA (SEQ ID NO: 402) TTATTAGGAGAGGGGAAGAAGT (SEQ ID NO: 401) | 466 |
| EPAS1 (SEQ ID NO: 40) | TATTGGATGTTTTTGGTAGGTT | 479 |
| | (SEQ ID NO: 403) AATCACCTCCCTCCCTTA (SEQ ID NO: 404) | |
| NOTCH1 (SEQ ID NO: 41) | CCCACCCTAAAAACTCACTA (SEQ ID NO: 406) GGAGGGGTTTGAGTAATTG (SEQ ID NO: 405) | 424 |
| MLLT3 (SEQ ID NO: 42) | GATTAGGTTTGGAGTTGTTTTT (SEQ ID NO: 407) AATCACATCCATCTTTCACTTT (SEQ ID NO: 408) | |
| SOLH (SEQ ID NO: 43) | CCCAACTCCCCAAATAAA (SEQ ID NO: 410) GGGGATAAGTGGTTAATGAGT (SEQ ID NO: 409) | 389 |
| SEQ ID NO: 44 (SEQ ID NO: 44) | ATAGTGTGGATTTTTAGGGATATT (SEQ ID NO: 411) | 448 |

(continued)

| Gene | Primers: | Amplificate Length: |
|---|---|---|
| | CAAAACCTATTCCCCTA CCT (SEQ ID NO: 412) | |
| Q8N365 (SEQ ID NO: 45) | GTAGTAAATGGGTTATG GATTTTAG (SEQ ID NO: 413) CCATACCACCCACAAA CA (SEQ ID NO: 414) | 475 |
| Q9NWV0 (SEQ ID NO: 46) | TTTTAGTAGTGGATTTG GTTAGTATT (SEQ ID NO: 415) CATCTCTTAACCCCACT TTCA | 330 |
| | (SEQ ID NO: 416) | |
| SEQ ID NO: 47 (SEQ ID NO: 47) | TCCCTCTAAAAACCAAA AATC (SEQ ID NO: 418) GAGGAAAGAAGGAGGA TATAGG (SEQ ID NO: 417) | 335 |
| H2AFY2 (SEQ ID NO: 48) | GTTAAAGGGGTATTGG TTTTT (SEQ ID NO: 419) TTTCTTTTTCTCTCACC TATTAAAC (SEQ ID NO: 420) | 490 |
| RHOC (SEQ ID NO: 49) | GTAAGAGGGATAGGGA ATTGG (SEQ ID NO: 421) AACACCCAAACCAAAAT AAAA (SEQ ID NO: 422) | 440 |

(continued)

| Gene | Primers: | Amplificate Length: |
|---|---|---|
| NR2E1 (SEQ ID NO: 50) | GGAGTTTGTGAAAAGT GGG (SEQ ID NO: 423) ACTCAACAAATACAATA ATCTAAACC (SEQ ID NO: 424) | 429 |
| KBTBD6 (SEQ ID NO: 51) | ACTATACCAACAAAACT ACAAAATAAA (SEQ ID NO: 426) GAAGGTTGAGGAGGAG TTAGA (SEQ ID NO: 425) | 228 |
| TRPM4 (SEQ ID NO: 52) | GGTTGGAAAGTGGAGG ATT (SEQ ID NO: 427) CCAACTCTAAAAACAAA AACAA (SEQ ID NO: 428) | 438 |
| TCEB3BP1 (SEQ ID NO: 53) | GAGTTGGTTTTGTTGAG GTGT | 325 |
| | (SEQ ID NO: 429) AAAATACCTTCCCACTA ACCTT (SEQ ID NO: 430) | |
| Q8NCX8 (SEQ ID NO: 54) | TAGTGGAATTATGAGG GGG (SEQ ID NO: 431) CCAAATACACCTCTACC AAAA (SEQ ID NO: 432) | 300 |
| SEQ ID NO: 55 (SEQ ID NO: 55) | CCTTACCCTCCTCTCCT AAA (SEQ ID NO: 434) | 384 |

(continued)

| Gene | Primers: | Amplificate Length: |
|---|---|---|
| | TAGGATTTGTGGTTGGT GTT (SEQ ID NO: 433) | |
| SNAPC2 (SEQ ID NO: 56) | GGTTTAGGGTATTTTAA GGGG (SEQ ID NO: 435) AAACTAAATCCAACTCC CAAA (SEQ ID NO: 436) | 362 |
| PTPRN2 (SEQ ID NO: 57) | CCCTCTACTCACTTTAC CAAAA (SEQ ID NO: 438) GGGGAGGTGTTTAGTG GTT (SEQ ID NO: 437) | 378 |
| WDFY3 (SEQ ID NO: 58) | TGTTGGTGGTTATTTTT AATTTTT (SEQ ID NO: 439) ACCCAATTATCCTTTCT CAAC (SEQ ID NO: 440) | 435 |
| ZNF566 (SEQ ID NO: 59) | GAGGATTTGTGTTAAG GTTTTT (SEQ ID NO: 441) CCAACTCCACTATCTAC CACAT | 457 |
| | (SEQ ID NO: 442) | |
| Q9NP73 (SEQ ID NO: 60) | GGGGTTTTAAGAGTTG GTTTT (SEQ ID NO: 443) CCTCCCTCACTCACTTA CAA (SEQ ID NO: 444) | |

(continued)

| Gene | Primers: | Amplificate Length: |
|---|---|---|
| SEQ ID NO: 61 (SEQ ID NO: 61) | TGGGGATATTGGATGT TTTT (SEQ ID NO: 445) CCTTCCAACCTAACCTC C (SEQ ID NO: 446) | 497 |
| Q86SP6 (SEQ ID NO: 62) | CCCAACACTCATTTACA CTATCT (SEQ ID NO: 448) GGAGTTTTAATTTTTGG GATTT (SEQ ID NO: 447) | 342 |

TABLE 10: Detection oligonucleotides according to Example 3.

| No: | Gene | Oligo: |
|---|---|---|
| 1 | ONECUT2 (SEQ ID NO: 14) | TAGAGGCGCGGGTTAT (SEQ ID NO: 449) |
| 2 | ONECUT2 (SEQ ID NO: 14) | TAGAGGTGTGGGTTAT (SEQ ID NO: 450) |
| 3 | ONECUT2 (SEQ ID NO: 14) | TTGCGATTGGTACGTA (SEQ ID NO: 451) |
| 4 | ONECUT2 (SEQ ID NO: 14) | TGTGATTGGTATGTAGT (SEQ ID NO: 452) |
| 5 | ONECUT2 (SEQ ID NO: 14) | TTTTGTGCGTACGGAT (SEQ ID NO: 453) |
| 6 | ONECUT2 (SEQ ID NO: 14) | TTTTTGTGTGTATGGAT (SEQ ID NO: 454) |
| 7 | ONECUT2 (SEQ ID NO: 14) | TTAAGCGGGCGTTGAT (SEQ ID NO: 455) |
| 8 | ONECUT2 (SEQ ID NO: 14) | TTAAGTGGGTGTTGAT (SEQ ID NO: 456) |
| 9 | MX1 (SEQ ID NO: 15) | GTTACGAGTTTATTCGA (SEQ ID NO: 457) |
| 10 | MX1 (SEQ ID NO: 15) | GGTTATGAGTTTATTTGAA (SEQ ID NO: 458) |
| 11 | MX1 (SEQ ID NO: 15) | AACGCGCGAAAGTAAA (SEQ ID NO: 459) |
| 12 | MX1 (SEQ ID NO: 15) | TTGGGAATGTGTGAAA (SEQ ID NO: 460) |
| 13 | MX1 (SEQ ID NO: 15) | GAGTTTTCGTCGATTT (SEQ ID NO: 461) |
| 14 | MX1 (SEQ ID NO: 15) | AGGAGTTTTTGTTGATT (SEQ ID NO: 462) |
| 15 | MX1 (SEQ ID NO: 15) | TATGCGCGGGAAGATT (SEQ ID NO: 463) |
| 16 | MX1 (SEQ ID NO: 15) | GTATGTGTGGGAAGAT (SEQ ID NO: 464) |
| 17 | DOCK10 (SEQ ID NO: 16) | GATCGGAATTCGGGTT (SEQ ID NO: 465) |
| 18 | DOCK10 (SEQ ID NO: 16) | ATTGGAATTTGGGTTG (SEQ ID NO: 466) |
| 19 | DOCK10 (SEQ ID NO: 16) | TAGTAGTCGCGTTTTT (SEQ ID NO: 467) |
| 20 | DOCK10 (SEQ ID NO: 16) | AGTAGTTGTGTTTTTGG (SEQ ID NO: 468) |
| 21 | DOCK10 (SEQ ID NO: 16) | ATTTTCGCGGGAAGTT (SEQ ID NO: 469) |

(continued)

| No: | Gene | Oligo: |
|---|---|---|
| 22 | DOCK10 (SEQ ID NO: 16) | GTGATTTTTGTGGGAA (SEQ ID NO: 470) |
| 23 | BTG4 (SEQ ID NO: 17) | AGTTAGCGTTTGTCGG (SEQ ID NO: 471) |
| 24 | BTG4 (SEQ ID NO: 17) | TAGTTAGTGTTTGTTGG (SEQ ID NO: 472) |
| 25 | BTG4 (SEQ ID NO: 17) | TTCGGCGTCGATGTAT (SEQ ID NO: 473) |
| 26 | BTG4 (SEQ ID NO: 17) | TTTGGTGTTGATGTATT (SEQ ID NO: 474) |
| 27 | DMRTC2 (SEQ ID NO: 18) | GGGTATTCGACGTTTT (SEQ ID NO: 475) |
| 28 | DMRTC2 (SEQ ID NO: 18) | AGGGGTATTTGATGTTT (SEQ ID NO: 476) |
| 29 | DMRTC2 (SEQ ID NO: 18) | ATTCGAAGCGTTATTG (SEQ ID NO: 477) |
| 30 | DMRTC2 (SEQ ID NO: 18) | TTTGAAGTGTTATTGGT (SEQ ID NO: 478) |
| 31 | DMRTC2 (SEQ ID NO: 18) | AAATCGTATTGGTCGT (SEQ ID NO: 479) |
| 32 | DMRTC2 (SEQ ID NO: 18) | AAATTGTATTGGTTGTTT (SEQ ID NO: 480) |
| 33 | DMRTC2 (SEQ ID NO: 18) | AATTCGTGTATAGATCGG (SEQ ID NO: 481) |
| 34 | DMRTC2 (SEQ ID NO: 18) | ATTTGTGTATAGATTGGG (SEQ ID NO: 482) |
| 35 | DMRTC2 (SEQ ID NO: 18) | AAAAGTAGCGCGAGTT (SEQ ID NO: 483) |
| 36 | DMRTC2 (SEQ ID NO: 18) | AGTAGTGTGAGTTTGG (SEQ ID NO: 484) |
| 37 | GPR7 (SEQ ID NO: 19) | GAACGTAGTCGCGGTT (SEQ ID NO: 485) |
| 38 | GPR7 (SEQ ID NO: 19) | GGAATGTAGTTGTGGT (SEQ ID NO: 486) |
| 39 | GPR7 (SEQ ID NO: 19) | ATTTTGGCGAATTCGG (SEQ ID NO: 487) |
| 40 | GPR7 (SEQ ID NO: 19) | TGGTGAATTTGGGGGA (SEQ ID NO: 488) |
| 41 | GPR7 (SEQ ID NO: 19) | TTAGTCGGTAGGCGTT (SEQ ID NO: 489) |
| 42 | GPR7 (SEQ ID NO: 19) | ATTTAGTTGGTAGGTGT (SEQ ID NO: 490) |
| 43 | GPR7 (SEQ ID NO: 19) | TTTTCGTAGTCGGCGG (SEQ ID NO: 491) |
| 44 | GPR7 (SEQ ID NO: 19) | TTTTTTGTAGTTGGTGG (SEQ ID NO: 492) |
| 45 | FAT (SEQ ID NO: 20) | TAAGCGTTAATAGAACGA (SEQ ID NO: 493) |
| 46 | FAT (SEQ ID NO: 20) | AGTGTTAATAGAATGAAAT (SEQ ID NO: 494) |
| 47 | FAT (SEQ ID NO: 20) | TTGTATTTCGTCGTTAT (SEQ ID NO: 495) |
| 48 | FAT (SEQ ID NO: 20) | TTTTGTTGTTATAGGAGT (SEQ ID NO: 496) |
| 49 | FAT (SEQ ID NO: 20) | ATAGGAGTCGTCGAGA (SEQ ID NO: 497) |
| 50 | FAT (SEQ ID NO: 20) | ATAGGAGTTGTTGAGAG (SEQ ID NO: 498) |
| 51 | ISL1 (SEQ ID NO: 21) | TTAATGCGGTCGGTTA (SEQ ID NO: 499) |
| 52 | ISL1 (SEQ ID NO: 21) | AGTTAATGTGGTTGGT (SEQ ID NO: 500) |
| 53 | GPRK5 (SEQ ID NO: 22) | TTTGATTCGCGGTCGG (SEQ ID NO: 501) |
| 54 | GPRK5 (SEQ ID NO: 22) | ATTTGATTTGTGGTTGG (SEQ ID NO: 502) |
| 55 | GPRK5 (SEQ ID NO: 22) | TATCGTCGGTCGAGTT (SEQ ID NO: 503) |
| 56 | GPRK5 (SEQ ID NO: 22) | TTTATTGTTGGTTGAGT (SEQ ID NO: 504) |
| 57 | GPRK5 (SEQ ID NO: 22) | ATGTCGAGAGTTCGTA (SEQ ID NO: 505) |
| 58 | GPRK5 (SEQ ID NO: 22) | GTTGAGAGTTTGTATGT (SEQ ID NO: 506) |
| 59 | SLC35F2 (SEQ ID NO: 23) | TTTCGGCGTTTAAAAT (SEQ ID NO: 507) |

(continued)

| No: | Gene | Oligo: |
|---|---|---|
| 60 | SLC35F2 (SEQ ID NO: 23) | TTTTTGGTGTTTAAAATTT (SEQ ID NO: 508) |
| 61 | SLC35F2 (SEQ ID NO: 23) | ATTTTCGAAGTGTCGG (SEQ ID NO: 509) |
| 62 | SLC35F2 (SEQ ID NO: 23) | TTTGAAGTGTTGGGTT (SEQ ID NO: 510) |
| 63 | SLC35F2 (SEQ ID NO: 23) | TTTCGGAAGACGGGAG (SEQ ID NO: 511) |
| 64 | SLC35F2 (SEQ ID NO: 23) | TTTTGGAAGATGGGAG (SEQ ID NO: 512) |
| 65 | SLC35F2 (SEQ ID NO: 23) | AATTCGGTCGTCGTTT (SEQ ID NO: 513) |
| 66 | SLC35F2 (SEQ ID NO: 23) | AGAATTTGGTTGTTGTT (SEQ ID NO: 514) |
| 67 | C14orf59 (SEQ ID NO: 24) | GACGTAGGGACGGAGA (SEQ ID NO: 515) |
| 68 | C14orf59 (SEQ ID NO: 24) | GATGTAGGGATGGAGA (SEQ ID NO: 516) |
| 69 | C14orf59 (SEQ ID NO: 24) | TATCGTGGTTTTTTACGTAT (SEQ ID NO: 517) |
| 70 | C14orf59 (SEQ ID NO: 24) | ATTGTGGTTTTTTATGTATA (SEQ ID NO: 518) |
| 71 | C14orf59 (SEQ ID NO: 24) | GTGTTCGAGAGCGAGT (SEQ ID NO: 519) |
| 72 | C14orf59 (SEQ ID NO: 24) | TGTTTGAGAGTGAGTGT (SEQ ID NO: 520) |
| 73 | C14orf59 (SEQ ID NO: 24) | TTTATTCGGTGTTCGA (SEQ ID NO: 521) |
| 74 | C14orf59 (SEQ ID NO: 24) | TATTTGGTGTTTGAGAG (SEQ ID NO: 522) |
| 75 | SNRPN (SEQ ID NO: 25) | TTTTTGCGGTCGCGTA (SEQ ID NO: 523) |
| 76 | SNRPN (SEQ ID NO: 25) | TTTTGTGGTTGTGTAGG (SEQ ID NO: 524) |
| 77 | SNRPN (SEQ ID NO: 25) | AGTATGCGCGTTAGTT (SEQ ID NO: 525) |
| 78 | SNRPN (SEQ ID NO: 25) | TGAGTATGTGTGTTAGT (SEQ ID NO: 526) |
| 79 | SNRPN (SEQ ID NO: 25) | TAGCGGTAGGTTTCGTA (SEQ ID NO: 527) |
| 80 | SNRPN (SEQ ID NO: 25) | TAGTGGTAGGTTTTGTA (SEQ ID NO: 528) |
| 81 | SNRPN (SEQ ID NO: 25) | TTTGCGTTAGATTCGT (SEQ ID NO: 529) |
| 82 | SNRPN (SEQ ID NO: 25) | TGTGTTAGATTTGTTGT (SEQ ID NO: 530) |
| 83 | ARHGEF18 (SEQ ID NO: 26) | GTCGATTCGGTTGATT (SEQ ID NO: 531) |
| 84 | ARHGEF18 (SEQ ID NO: 26) | AAGGTTGATTTGGTTG (SEQ ID NO: 532) |
| 85 | ARHGEF18 (SEQ ID NO: 26) | GGCGGTTTCGAAGATT (SEQ ID NO: 533) |
| 86 | ARHGEF18 (SEQ ID NO: 26) | AGATGGGTGGTTTTGA (SEQ ID NO: 534) |
| 87 | ARHGEF18 (SEQ ID NO: 26) | AAGTCGGTTATGAGCGA (SEQ ID NO: 535) |
| 88 | ARHGEF18 (SEQ ID NO: 26) | AAGTTGGTTATGAGTGA (SEQ ID NO: 536) |
| 89 | ARHGEF18 (SEQ ID NO: 26) | TGGTCGATACGGTATT (SEQ ID NO: 537) |
| 90 | ARHGEF18 (SEQ ID NO: 26) | TTGTGGTTGATATGGT (SEQ ID NO: 538) |
| 91 | SNX8 (SEQ ID NO: 27) | AGGACGCGATAGGGAT (SEQ ID NO: 539) |
| 92 | SNX8 (SEQ ID NO: 27) | AGGATGTGATAGGGAT (SEQ ID NO: 540) |
| 93 | SNX8 (SEQ ID NO: 27) | ATTTCGTCGTATGTGA (SEQ ID NO: 541) |
| 94 | SNX8 (SEQ ID NO: 27) | ATTTTGTTGTATGTGAAG (SEQ ID NO: 542) |
| 95 | SNX8 (SEQ ID NO: 27) | GTCGTTTGCGTATTTA (SEQ ID NO: 543) |
| 96 | SNX8 (SEQ ID NO: 27) | GGTTGTTTGTGTATTTAA (SEQ ID NO: 544) |
| 97 | SNX8 (SEQ ID NO: 27) | ATTGTATACGCGCGTT (SEQ ID NO: 545) |

(continued)

| No: | Gene | Oligo: |
|---|---|---|
| 98 | SNX8 (SEQ ID NO: 27) | TTGTATATGTGTGTTGG (SEQ ID NO: 546) |
| 99 | FBN2 (SEQ ID NO: 28) | TAAAGCGAGTAGACGG (SEQ ID NO: 547) |
| 100 | FBN2 (SEQ ID NO: 28) | TATAAAGTGAGTAGATGG (SEQ ID NO: 548) |
| 101 | HOXB5 (SEQ ID NO: 29) | ATAGTTTTCGGCGGGT (SEQ ID NO: 549) |
| 102 | HOXB5 (SEQ ID NO: 29) | TATAGTTTTTGGTGGGT (SEQ ID NO: 550) |
| 103 | HOXB5 (SEQ ID NO: 29) | TTTTTCGGCGTAGATA (SEQ ID NO: 551) |
| 104 | HOXB5 (SEQ ID NO: 29) | TGTTTTTTGGTGTAGAT (SEQ ID NO: 552) |
| 105 | HOXB5 (SEQ ID NO: 29) | AGTCGAGGGCGTTAGA (SEQ ID NO: 553) |
| 106 | HOXB5 (SEQ ID NO: 29) | AGTTGAGGGTGTTAGA (SEQ ID NO: 554) |
| 107 | HOXB5 (SEQ ID NO: 29) | TTTTCGAGGAATTCGT (SEQ ID NO: 555) |
| 108 | HOXB5 (SEQ ID NO: 29) | TTTTTTGAGGAATTTGTT (SEQ ID NO: 556) |
| 109 | LIMK1 (SEQ ID NO: 30) | TATCGGATTATCGCGG (SEQ ID NO: 557) |
| 110 | LIMK1 (SEQ ID NO: 30) | ATTGGATTATTGTGGGG (SEQ ID NO: 558) |
| 111 | LIMK1 (SEQ ID NO: 30) | GTCGGTAGTTTATCGGAT (SEQ ID NO: 559) |
| 112 | LIMK1 (SEQ ID NO: 30) | GTTGGTAGTTTATTGGAT (SEQ ID NO: 560) |
| 113 | LIMK1 (SEQ ID NO: 30) | TAGGAGACGTTACGTT (SEQ ID NO: 561) |
| 114 | LIMK1 (SEQ ID NO: 30) | AGATGTTATGTTAGGGT (SEQ ID NO: 562) |
| 115 | PSD/Q9H469 (SEQ ID NO: 31) | TTTTTCGAAGCGGATT (SEQ ID NO: 563) |
| 116 | PSD/Q9H469 (SEQ ID NO: 31) | TTTGAAGTGGATTTTGG (SEQ ID NO: 564) |
| 117 | PSD/Q9H469 (SEQ ID NO: 31) | GAAACGCGGTTTAAAT (SEQ ID NO: 565) |
| 118 | PSD/Q9H469 (SEQ ID NO: 31) | GGAAATGTGGTTTAAATT (SEQ ID NO: 566) |
| 119 | SLC38A1 (SEQ ID NO: 32) | TTTGCGGTAACGTTTA (SEQ ID NO: 567) |
| 120 | SLC38A1 (SEQ ID NO: 32) | TTGTGGTAATGTTTAGG (SEQ ID NO: 568) |
| 121 | SLC38A1 (SEQ ID NO: 32) | TAGCGGTCGCGGATTA (SEQ ID NO: 569) |
| 122 | SLC38A1 (SEQ ID NO: 32) | GTAGTGGTTGTGGATT (SEQ ID NO: 570) |
| 123 | SLC38A1 (SEQ ID NO: 32) | TTAGGGACGCGAATTA (SEQ ID NO: 571) |
| 124 | SLC38A1 (SEQ ID NO: 32) | AGGGATGTGAATTAGG (SEQ ID NO: 572) |
| 125 | SLC38A1 (SEQ ID NO: 32) | TGCGTTTAAGATCGCGT (SEQ ID NO: 573) |
| 126 | SLC38A1 (SEQ ID NO: 32) | TGTGTTTAAGATTGTGT (SEQ ID NO: 574) |
| 127 | SLC38A1 (SEQ ID NO: 32) | ATTTCGGTTTTCGAAA (SEQ ID NO: 575) |
| 128 | SLC38A1 (SEQ ID NO: 32) | ATATTTTGGTTTTTGAAAA (SEQ ID NO: 576) |
| 129 | SLC38A1 (SEQ ID NO: 32) | AGAGCGTAGTTGATTCGA (SEQ ID NO: 577) |
| 130 | SLC38A1 (SEQ ID NO: 32) | AGAGTGTAGTTGATTTGA (SEQ ID NO: 578) |
| 131 | SLC38A1 (SEQ ID NO: 32) | AGGAATTACGTACGTT (SEQ ID NO: 579) |
| 132 | SLC38A1 (SEQ ID NO: 32) | TATGTATGTTTGGAGGG (SEQ ID NO: 580) |
| 133 | SLC38A1 (SEQ ID NO: 32) | TTTGTAACGCGGGGAA (SEQ ID NO: 581) |
| 134 | SLC38A1 (SEQ ID NO: 32) | TTTTGTAATGTGGGGA (SEQ ID NO: 582) |
| 135 | HIST1H4J (SEQ ID NO: 33) | TATGGCGGTGATCGTT (SEQ ID NO: 583) |

(continued)

| No: | Gene | Oligo: |
|---|---|---|
| 136 | HIST1H4J (SEQ ID NO: 33) | TTTATGGTGGTGATTGT (SEQ ID NO: 584) |
| 137 | HIST1H4J (SEQ ID NO: 33) | TTACGGCGTTTCGGAT (SEQ ID NO: 585) |
| 138 | HIST1H4J (SEQ ID NO: 33) | TTATGGTGTTTTGGATT (SEQ ID NO: 586) |
| 139 | HIST1H4J (SEQ ID NO: 33) | ATGCGTTTTACGTCGT (SEQ ID NO: 587) |
| 140 | HIST1H4J (SEQ ID NO: 33) | AGATGTGTTTTATGTTGT (SEQ ID NO: 588) |
| 141 | HIST1H4J (SEQ ID NO: 33) | TATTGTCGCGTAGTAT (SEQ ID NO: 589) |
| 142 | HIST1H4J (SEQ ID NO: 33) | GGATATTGTTGTGTAGT (SEQ ID NO: 590) |
| 143 | HIST1H4J (SEQ ID NO: 33) | ATCGAAATCGTAGAGG (SEQ ID NO: 591) |
| 144 | HIST1H4J (SEQ ID NO: 33) | ATTGAAATTGTAGAGGG (SEQ ID NO: 592) |
| 145 | Q96S01 (SEQ ID NO: 34) | ATCGGTTTTTCGAGGT (SEQ ID NO: 593) |
| 146 | Q96S01 (SEQ ID NO: 34) | ATTGGTTTTTTGAGGTT (SEQ ID NO: 594) |
| 147 | Q96S01 (SEQ ID NO: 34) | GGTCGATTTTCGCGTA (SEQ ID NO: 595) |
| 148 | Q96S01 (SEQ ID NO: 34) | TGGTTGATTTTTGTGTA (SEQ ID NO: 596) |
| 149 | GENOMIC REGION DOWNSTREAM FROM FOXL2 (SEQ ID NO: 35) | AATCGTGCGGTTGATA (SEQ ID NO: 597) |
| 150 | GENOMIC REGION DOWNSTREAM FROM FOXL2 (SEQ ID NO: 35) | TGTAGAATTGTGTGGT (SEQ ID NO: 598) |
| 151 | GENOMIC REGION DOWNSTREAM FROM FOXL2 (SEQ ID NO: 35) | AAAATTCGAGGTCGGG (SEQ ID NO: 599) |
| 152 | GENOMIC REGION DOWNSTREAM FROM FOXL2 (SEQ ID NO: 35) | AAAATTTGAGGTTGGG (SEQ ID NO: 600) |
| 153 | GENOMIC REGION DOWNSTREAM FROM FOXL2 (SEQ ID NO: 35) | TTTTCGCGGTTCGGAG (SEQ ID NO: 601) |
| 154 | GENOMIC REGION DOWNSTREAM FROM FOXL2 (SEQ ID NO: 35) | TTTGTGGTTTGGAGAA (SEQ ID NO: 602) |
| 155 | GENOMIC REGION DOWNSTREAM FROM FOXL2 (SEQ ID NO: 35) | AATAGGCGATGTACGG (SEQ ID NO: 603) |
| 156 | GENOMIC REGION DOWNSTREAM FROM FOXL2 (SEQ ID NO: 35) | TAGGTGATGTATGGGT (SEQ ID NO: 604) |
| 157 | GENOMIC REGION DOWNSTREAM FROMFOXL2 (SEQ ID NO: 35) | TTGGTCGGTTAATCGA (SEQ ID NO: 605) |
| 158 | GENOMIC REGION DOWNSTREAM FROM FOXL2 (SEQ ID NO: 35) | TTTGGTTGGTTAATTGA (SEQ ID NO: 606) |
| 159 | GENOMIC REGION DOWNSTREAM FROM FOX2 (SEQ ID NO: 35) | TAGCGGTCGCGAAAAT (SEQ ID NO: 607) |
| 160 | GENOMIC REGION DOWNSTREAM FROM FOXL2 (SEQ ID NO: 35) | AGTTTAGTGGTTGTGA (SEQ ID NO: 608) |
| 161 | CMYA3 (SEQ ID NO: 13) | TTTACGCGGGGTTTTA (SEQ ID NO: 609) |
| 162 | CMYA3 (SEQ ID NO: 13) | TTTATGTGGGGTTTTAG (SEQ ID NO: 610) |
| 163 | CMYA3 (SEQ ID NO: 13) | TTACGTCGTTATTAGGT (SEQ ID NO: 611) |
| 164 | CMYA3 (SEQ ID NO: 13) | TTTTATGTTGTTATTAGGT (SEQ ID NO: 612) |

(continued)

| No: | Gene | Oligo: |
|---|---|---|
| 165 | CMYA3 (SEQ ID NO: 13) | TATTTGGACGTCGGGT (SEQ ID NO: 613) |
| 166 | CMYA3 (SEQ ID NO: 13) | TATTTGGATGTTGGGT (SEQ ID NO: 614) |
| 167 | CMYA3 (SEQ ID NO: 13) | TTTGTCGGAAAGCGGA (SEQ ID NO: 615) |
| 168 | CMYA3 (SEQ ID NO: 13) | TTTGTTGGAAAGTGGA (SEQ ID NO: 616) |
| 169 | ORC4L (SEQ ID NO: 36) | ATTCGGATCGTTACGT (SEQ ID NO: 617) |
| 170 | ORC4L (SEQ ID NO: 36) | ATTTGGATTGTTATGTTT (SEQ ID NO: 618) |
| 171 | ORC4L (SEQ ID NO: 36) | ATAAGACGGAGTTCGT (SEQ ID NO: 619) |
| 172 | ORC4L (SEQ ID NO: 36) | AAGATGGAGTTTGTTTG (SEQ ID NO: 620) |
| 173 | ORC4L (SEQ ID NO: 36) | TTGCGTTATCGACGTT (SEQ ID NO: 621) |
| 174 | ORC4L (SEQ ID NO: 36) | ATTTTGTGTTATTGATGT (SEQ ID NO: 622) |
| 175 | ORC4L (SEQ ID NO: 36) | GAGTAACGCGTGTGAT (SEQ ID NO: 623) |
| 176 | ORC4L (SEQ ID NO: 36) | TATGAGTAATGTGTGTG (SEQ ID NO: 624) |
| 177 | ABHD9 (SEQ ID NO: 37) | TATTTGGGCGCGATAG (SEQ ID NO: 625) |
| 178 | ABHD9 (SEQ ID NO: 37) | ATTTGGGTGTGATAGG (SEQ ID NO: 626) |
| 179 | ABHD9 (SEQ ID NO: 37) | GTGGGACGCGTTGAAG (SEQ ID NO: 627) |
| 180 | ABHD9 (SEQ ID NO: 37) | TGTGGGATGTGTTGAA (SEQ ID NO: 628) |
| 181 | ABHD9 (SEQ ID NO: 37) | GGCGGTTTCGATAGAA (SEQ ID NO: 629) |
| 182 | ABHD9 (SEQ ID NO: 37) | GGGTGGTTTTGATAGA (SEQ ID NO: 630) |
| 183 | CCND2 (SEQ ID NO: 1) | ATAAGTCGTTCGAGGT (SEQ ID NO: 631) |
| 184 | CCND2 (SEQ ID NO: 1) | AAGTTGTTTGAGGTGT (SEQ ID NO: 632) |
| 185 | CCND2 (SEQ ID NO: 1) | TAGCGGTTACGTAGGA (SEQ ID NO: 633) |
| 186 | CCND2 (SEQ ID NO: 1) | AGTGGTTATGTAGGAAA (SEQ ID NO: 634) |
| 187 | CCND2 (SEQ ID NO: 1) | TACGTGTTTTAACGTAT (SEQ ID NO: 635) |
| 188 | CCND2 (SEQ ID NO: 1) | TGATATGTGTTTTAATGTA (SEQ ID NO: 636) |
| 189 | CCND2 (SEQ ID NO: 1) | TTAGGGTCGTCGTAGGT (SEQ ID NO: 637) |
| 190 | CCND2 (SEQ ID NO: 1) | TTAGGGTTGTTGTAGGT (SEQ ID NO: 638) |
| 191 | CCND2 (SEQ ID NO: 1) | TTAGTACGGTCGGTTT (SEQ ID NO: 639) |
| 192 | CCND2 (SEQ ID NO: 1) | GTTAGTATGGTTGGTTT (SEQ ID NO: 640) |
| 193 | CDKN2A (SEQ ID NO: 2) | TAGGTATCGCGTACGT (SEQ ID NO: 641) |
| 194 | CDKN2A (SEQ ID NO: 2) | TTAGGTATTGTGTATGTT (SEQ ID NO: 642) |
| 195 | CDKN2A (SEQ ID NO: 2) | TTCGCGTCGTGGAGTA (SEQ ID NO: 643) |
| 196 | CDKN2A (SEQ ID NO: 2) | TTTTGTGTTGTGGAGTA (SEQ ID NO: 644) |
| 197 | CDKN2A (SEQ ID NO: 2) | TAGTCGCGCGTAGGTA (SEQ ID NO: 645) |
| 198 | CDKN2A (SEQ ID NO: 2) | TAGTTGTGTGTAGGTAT (SEQ ID NO: 646) |
| 199 | CDKN2A (SEQ ID NO: 2) | TAGGTATCGTGCGATA (SEQ ID NO: 647) |
| 200 | CDKN2A (SEQ ID NO: 2) | GTAGGTATTGTGTGATAT (SEQ ID NO: 648) |
| 201 | CD44 (SEQ ID NO: 3) | TAGGTTCGGTTCGTTAT (SEQ ID NO: 649) |
| 202 | CD44 (SEQ ID NO: 3) | TAGGTTTGGTTTGTTATT (SEQ ID NO: 650) |

(continued)

| No: | Gene | Oligo: |
|---|---|---|
| 203 | CD44 (SEQ ID NO: 3) | GTTTCGCGTTTAGGGA (SEQ ID NO: 651) |
| 204 | CD44 (SEQ ID NO: 3) | GTTTTGTGTTTAGGGAT (SEQ ID NO: 652) |
| 205 | CD44 (SEQ ID NO: 3) | GTTCGTTTCGGATATTA (SEQ ID NO: 653) |
| 206 | CD44 (SEQ ID NO: 3) | TTTGTTTTGGATATTATGG (SEQ ID NO: 654) |
| 207 | CD44 (SEQ ID NO: 3) | TTTGGCGTAGATCGGT (SEQ ID NO: 655) |
| 208 | CD44 (SEQ ID NO: 3) | TTTGGTGTAGATTGGT (SEQ ID NO: 656) |
| 209 | EDNRB1 (SEQ ID NO: 4) | TTCGTTTTTCGGGAAG (SEQ ID NO: 657) |
| 210 | EDNRB1 (SEQ ID NO: 4) | TTTGTTTTTTGGGAAGG (SEQ ID NO: 658) |
| 211 | EDNRB1 (SEQ ID NO: 4) | TAGAGTCGGATTCGTT (SEQ ID NO: 659) |
| 212 | EDNRB1 (SEQ ID NO: 4) | AGTTGGATTTGTTTGTA (SEQ ID NO: 660) |
| 213 | EDNRB1 (SEQ ID NO: 4) | GTATTTTCGTAGCGTT (SEQ ID NO: 661) |
| 214 | EDNRB1 (SEQ ID NO: 4) | TGGTATTTTTGTAGTGTT (SEQ ID NO: 662) |
| 215 | EDNRB1 (SEQ ID NO: 4) | ATTTCGAGTAAACGGT (SEQ ID NO: 663) |
| 216 | EDNRB1 (SEQ ID NO: 4) | TTTGAGTAAATGGTGGA (SEQ ID NO: 664) |
| 217 | ELK1 (SEQ ID NO: 5) | TGTCGTACGTTATGTT (SEQ ID NO: 665) |
| 218 | ELK1 (SEQ ID NO: 5) | GGTGTTGTATGTTATGT (SEQ ID NO: 666) |
| 219 | ELK1 (SEQ ID NO: 5) | TGGGCGTAGTAGTCGG (SEQ ID NO: 667) |
| 220 | ELK1 (SEQ ID NO: 5) | ATGGGTGTAGTAGTTGG (SEQ ID NO: 668) |
| 221 | ELK1 (SEQ ID NO: 5) | ATTGGGTTTCGCGTAGG (SEQ ID NO: 669) |
| 222 | ELK1 (SEQ ID NO: 5) | ATTGGGTTTTGTGTAGG (SEQ ID NO: 670) |
| 223 | ELK1 (SEQ ID NO: 5) | TTGATTGGCGGACGAG (SEQ ID NO: 671) |
| 224 | ELK1 (SEQ ID NO: 5) | TTGATTGGTGGATGAG (SEQ ID NO: 672) |
| 225 | FOS (SEQ ID NO: 6) | TACGGATTTGGTCGTTT (SEQ ID NO: 673) |
| 226 | FOS (SEQ ID NO: 6) | TATGGATTTGGTTGTTT (SEQ ID NO: 674) |
| 227 | FOS (SEQ ID NO: 6) | TTCGATTAGTTCGGAT (SEQ ID NO: 675) |
| 228 | FOS (SEQ ID NO: 6) | TATTTTGATTAGTTTGGAT (SEQ ID NO: 676) |
| 229 | FOS (SEQ ID NO: 6) | TTTCGTGGTTTTATCGTA (SEQ ID NO: 677) |
| 230 | FOS (SEQ ID NO: 6) | TTTTGTGGTTTTATTGTA (SEQ ID NO: 678) |
| 231 | FOS (SEQ ID NO: 6) | GTCGAGCGTAGAGTAT (SEQ ID NO: 679) |
| 232 | FOS (SEQ ID NO: 6) | TAGGAGGTTGAGTGTA (SEQ ID NO: 680) |
| 233 | GSTP1 (SEQ ID NO: 7) | GTCGGTCGTAGAGGGG (SEQ ID NO: 681) |
| 234 | GSTP1 (SEQ ID NO: 7) | GTTGGTTGTAGAGGGG (SEQ ID NO: 682) |
| 235 | GSTP1 (SEQ ID NO: 7) | TTCGCGGTTTTCGAGT (SEQ ID NO: 683) |
| 236 | GSTP1 (SEQ ID NO: 7) | TTTGTGGTTTTTGAGTT (SEQ ID NO: 684) |
| 237 | GSTP1 (SEQ ID NO: 7) | GTCGCGCGTATTTATT (SEQ ID NO: 685) |
| 238 | GSTP1 (SEQ ID NO: 7) | GGGTTGTGTGTATTTAT (SEQ ID NO: 686) |
| 239 | GSTP1 (SEQ ID NO: 7) | GAGTCGTCGCGTAGTT (SEQ ID NO: 687) |
| 240 | GSTP1 (SEQ ID NO: 7) | GGAGTTGTTGTGTAGTT (SEQ ID NO: 688) |

(continued)

| No: | Gene | Oligo: |
|---|---|---|
| 241 | CD37 (SEQ ID NO: 38) | ATCGAGAGCGTTATGA (SEQ ID NO: 689) |
| 242 | CD37 (SEQ ID NO: 38) | AGGAATATTGAGAGTGT (SEQ ID NO: 690) |
| 243 | CD37 (SEQ ID NO: 38) | TATCGAGCGAGTCGGT (SEQ ID NO: 691) |
| 244 | CD37 (SEQ ID NO: 38) | TATTGAGTGAGTTGGTT (SEQ ID NO: 692) |
| 245 | CD37 (SEQ ID NO: 38) | TTAGCGTACGTGACGG (SEQ ID NO: 693) |
| 246 | CD37 (SEQ ID NO: 38) | AGTGTATGTGATGGGG (SEQ ID NO: 694) |
| 247 | CD37 (SEQ ID NO: 38) | GGGTACGTAGTTACGT (SEQ ID NO: 695) |
| 248 | CD37 (SEQ ID NO: 38) | TATGTAGTTATGTGGGT (SEQ ID NO: 696) |
| 249 | GRN (SEQ ID NO: 39) | TAGCGCGATGATTCGT (SEQ ID NO: 697) |
| 250 | GRN (SEQ ID NO: 39) | AGTGTGATGATTTGTTT (SEQ ID NO: 698) |
| 251 | GRN (SEQ ID NO: 39) | TAATCGGGTAGCGTTT (SEQ ID NO: 699) |
| 252 | GRN (SEQ ID NO: 39) | TAGTAATTGGGTAGTGT (SEQ ID NO: 700) |
| 253 | GRN (SEQ ID NO: 39) | TTCGATTTCGCGGTTT (SEQ ID NO: 701) |
| 254 | GRN (SEQ ID NO: 39) | GTTTGATTTTGTGGTTT (SEQ ID NO: 702) |
| 255 | GRN (SEQ ID NO: 39) | TTTAACGGGGCGTTAT (SEQ ID NO: 703) |
| 256 | GRN (SEQ ID NO: 39) | AATGGGGTGTTATTGT (SEQ ID NO: 704) |
| 257 | EPAS1 (SEQ ID NO: 40) | TTCGGCGTTATTCGAG (SEQ ID NO: 705) |
| 258 | EPAS1 (SEQ ID NO: 40) | GGTTTGGTGTTATTTGA (SEQ ID NO: 706) |
| 259 | EPAS1 (SEQ ID NO: 40) | TATTCGTGCGGTTTTA (SEQ ID NO: 707) |
| 260 | EPAS1 (SEQ ID NO: 40) | ATTTGTGTGGTTTTAGT (SEQ ID NO: 708) |
| 261 | EPAS1 (SEQ ID NO: 40) | GAATTTAACGCGCGGT (SEQ ID NO: 709) |
| 262 | EPAS1 (SEQ ID NO: 40) | GGAATTTAATGTGTGGT (SEQ ID NO: 710) |
| 263 | EPAS1 (SEQ ID NO: 40) | TTCGCGAGTTTTTCGG (SEQ ID NO: 711) |
| 264 | EPAS1 (SEQ ID NO: 40) | TTTGTGAGTTTTTTGGTA (SEQ ID NO: 712) |
| 265 | NOTCH1 (SEQ ID NO: 41) | TTTACGGGCGGGAGTT (SEQ ID NO: 713) |
| 266 | NOTCH1 (SEQ ID NO: 41) | TTTTATGGGTGGGAGT (SEQ ID NO: 714) |
| 267 | NOTCH1 (SEQ ID NO: 41) | TAGCGGGCGAGTAGTT (SEQ ID NO: 715) |
| 268 | NOTCH1 (SEQ ID NO: 41) | TAGTGGGTGAGTAGTT (SEQ ID NO: 716) |
| 269 | NOTCH1 (SEQ ID NO: 41) | AGGCGGTTTCGATTTT (SEQ ID NO: 717) |
| 270 | NOTCH1 (SEQ ID NO: 41) | TGGAGGTGGTTTTGAT (SEQ ID NO: 718) |
| 271 | NOTCH1 (SEQ ID NO: 41) | ATTTCGGCGGTTGGAT (SEQ ID NO: 719) |
| 272 | NOTCH1 (SEQ ID NO: 41) | AGGTAATTTTGGTGGTT (SEQ ID NO: 720) |
| 273 | MLLT3 (SEQ ID NO: 42) | TAATTCGGTTAGATTTTCGG (SEQ ID NO: 721) |
| 274 | MLLT3 (SEQ ID NO: 42) | TAATTTGGTTAGATTTTTGG (SEQ ID NO: 722) |
| 275 | MLLT3 (SEQ ID NO: 42) | TTTAGAGTCGCGTTTT (SEQ ID NO: 723) |
| 276 | MLLT3 (SEQ ID NO: 42) | TAGAGTTGTGTTTTTGT (SEQ ID NO: 724) |
| 277 | MLLT3 (SEQ ID NO: 42) | TAGAATAGCGCGGTTA (SEQ ID NO: 725) |
| 278 | MLLT3 (SEQ ID NO: 42) | GATAGAATAGTGTGGTTA (SEQ ID NO: 726) |

(continued)

| No: | Gene | Oligo: |
|---|---|---|
| 279 | SOLH (SEQ ID NO: 43) | TAGGGGACGTGTACGA (SEQ ID NO: 727) |
| 280 | SOLH (SEQ ID NO: 43) | TAGGGGATGTGTATGA (SEQ ID NO: 728) |
| 281 | SOLH (SEQ ID NO: 43) | GACGGAACGTATGTTT (SEQ ID NO: 729) |
| 282 | SOLH (SEQ ID NO: 43) | TGGGGATGGAATGTAT (SEQ ID NO: 730) |
| 283 | SOLH (SEQ ID NO: 43) | ATTCGTGGGGACGGAA (SEQ ID NO: 731) |
| 284 | SOLH (SEQ ID NO: 43) | ATTTGTGGGGATGGAA (SEQ ID NO: 732) |
| 285 | SEQ ID NO: 44 (SEQ ID NO: 44) | TAGGGTTCGTTCGTATT (SEQ ID NO: 733) |
| 286 | SEQ ID NO: 44 (SEQ ID NO: 44) | TTTAGGGTTTGTTTGTAT (SEQ ID NO: 734) |
| 287 | SEQ ID NO: 44 (SEQ ID NO: 44) | TTACGATTTTCGGGGT (SEQ ID NO: 735) |
| 288 | SEQ ID NO: 44 (SEQ ID NO: 44) | TTTATGATTTTTGGGGT (SEQ ID NO: 736) |
| 289 | SEQ ID NO: 44 (SEQ ID NO: 44) | AAGTAGACGTCGGAGA (SEQ ID NO: 737) |
| 290 | SEQ ID NO: 44 (SEQ ID NO: 44) | TAGATGTTGGAGAGGG (SEQ ID NO: 738) |
| 291 | SEQ ID NO: 44 (SEQ ID NO: 44) | TGTCGTTACGTTTAGG (SEQ ID NO: 739) |
| 292 | SEQ ID NO: 44 (SEQ ID NO: 44) | GTTGTTATGTTTAGGGT (SEQ ID NO: 740) |
| 293 | Q8N365 (SEQ ID NO: 45) | AGATTCGGAGAGACGG (SEQ ID NO: 741) |
| 294 | Q8N365 (SEQ ID NO: 45) | TAGATTTGGAGAGATGG (SEQ ID NO: 742) |
| 295 | Q8N365 (SEQ ID NO: 45) | AGATCGGCGTAGGGAT (SEQ ID NO: 743) |
| 296 | Q8N365 (SEQ ID NO: 45) | AGATTGGTGTAGGGAT (SEQ ID NO: 744) |
| 297 | Q8N365 (SEQ ID NO: 45) | TACGTGTTATCGGCGA (SEQ ID NO: 745) |
| 298 | Q8N365 (SEQ ID NO: 45) | TATGTGTTATTGGTGATA (SEQ ID NO: 746) |
| 299 | Q8N365 (SEQ ID NO: 45) | TACGTGTCGGGTCGTA (SEQ ID NO: 747) |
| 300 | Q8N365 (SEQ ID NO: 45) | GTATGTGTTGGGTTGTA (SEQ ID NO: 748) |
| 301 | Q9NWV0 (SEQ ID NO: 46) | GTCGCGTGGATACGTG (SEQ ID NO: 749) |
| 302 | Q9NWV0 (SEQ ID NO: 46) | GGTTGTGTGGATATGT (SEQ ID NO: 750) |
| 303 | Q9NWV0 (SEQ ID NO: 46) | TTGGCGATTTTTACGA (SEQ ID NO: 751) |
| 304 | Q9NWV0 (SEQ ID NO: 46) | TTGGTGATTTTTATGAGA (SEQ ID NO: 752) |
| 305 | 09NWV0 (SEQ ID NO: 46) | TGTCGTAGCGTTATGT (SEQ ID NO: 753) |
| 306 | Q9NWV0 (SEQ ID NO: 46) | AGGTGTTGTAGTGTTAT (SEQ ID NO: 754) |
| 307 | SEQ ID NO: 47 (SEQ ID NO: 47) | GTAACGCGTTTGGTTT (SEQ ID NO: 755) |
| 308 | SEQ ID NO: 47 (SEQ ID NO: 47) | TGGTAATGTGTTTGGT (SEQ ID NO: 756) |
| 309 | SEQ ID NO: 47 (SEQ ID NO: 47) | TTCGAGCGTTTTACGT (SEQ ID NO: 757) |
| 310 | SEQ ID NO: 47 (SEQ ID NO: 47) | TTTTGAGTGTTTTATGTT (SEQ ID NO: 758) |
| 311 | SEQ ID NO: 47 (SEQ ID NO: 47) | TTACGTGGGAGCGTTT (SEQ ID NO: 759) |
| 312 | SEQ ID NO: 47 (SEQ ID NO: 47) | TTATGTGGGAGTGTTT (SEQ ID NO: 760) |
| 313 | SEQ ID NO: 47 (SEQ ID NO: 47) | TAGTTTTACGCGGGTA (SEQ ID NO: 761) |
| 314 | SEQ ID NO: 47 (SEQ ID NO: 47) | AGTTTTATGTGGGTATG (SEQ ID NO: 762) |
| 315 | H2AFY2 (SEQ ID NO: 48) | ATGAAAGGCGCGAGAA (SEQ ID NO: 763) |
| 316 | H2AFY2 (SEQ ID NO: 48) | ATGAAAGGTGTGAGAA (SEQ ID NO: 764) |

(continued)

| No: | Gene | Oligo: |
|---|---|---|
| 317 | H2AFY2 (SEQ ID NO: 48) | GAATCGTGGTTTCGTT (SEQ ID NO: 765) |
| 318 | H2AFY2 (SEQ ID NO: 48) | GGAATTGTGGTTTTGT (SEQ ID NO: 766) |
| 319 | H2AFY2 (SEQ ID NO: 48) | TAGTTTATCGCGGTAA (SEQ ID NO: 767) |
| 320 | H2AFY2 (SEQ ID NO: 48) | TTTATTGTGGTAAATGGT (SEQ ID NO: 768) |
| 321 | RHOC (SEQ ID NO: 49) | TGCGGTTCGAAGATTA (SEQ ID NO: 769) |
| 322 | RHOC (SEQ ID NO: 49) | GGTGTGGTTTGAAGAT (SEQ ID NO: 770) |
| 323 | RHOC (SEQ ID NO: 49) | GAACGCGTTTTAGCGT (SEQ ID NO: 771) |
| 324 | RHOC (SEQ ID NO: 49) | GGAATGTGTTTTAGTGT (SEQ ID NO: 772) |
| 325 | RHOC (SEQ ID NO: 49) | TTTTAGCGTCGGGGAT (SEQ ID NO: 773) |
| 326 | RHOC (SEQ ID NO: 49) | TTTTAGTGTTGGGGAT (SEQ ID NO: 774) |
| 327 | NR2E1 (SEQ ID NO: 50) | TAGTCGTATTAGCGGT (SEQ ID NO: 775) |
| 328 | NR2E1 (SEQ ID NO: 50) | TAGTTGTATTAGTGGTTT (SEQ ID NO: 776) |
| 329 | NR2E1 (SEQ ID NO: 50) | TGCGTTTTATTCGCGG (SEQ ID NO: 777) |
| 330 | NR2E1 (SEQ ID NO: 50) | TGTGTTTTATTTGTGGT (SEQ ID NO: 778) |
| 331 | NR2E1 (SEQ ID NO: 50) | TTTCGAAGTTTCGCGG (SEQ ID NO: 779) |
| 332 | NR2E1 (SEQ ID NO: 50) | TTTGAAGTTTTGTGGG (SEQ ID NO: 780) |
| 333 | NR2E1 (SEQ ID NO: 50) | TAGCGCGAATCGTTTA (SEQ ID NO: 781) |
| 334 | NR2E1 (SEQ ID NO: 50) | AGTGTGAATTGTTTAGT (SEQ ID NO: 782) |
| 335 | KBTBD6 (SEQ ID NO: 51) | AGACGTTTCGCGTTTT (SEQ ID NO: 783) |
| 336 | KBTBD6 (SEQ ID NO: 51) | GAAGATGTTTTGTGTTT (SEQ ID NO: 784) |
| 337 | KBTBD6 (SEQ ID NO: 51) | TTTCGGGAAGACGTTT (SEQ ID NO: 785) |
| 338 | KBTBD6 (SEQ ID NO: 51) | AGTTTTGGGAAGATGT (SEQ ID NO: 786) |
| 339 | KBTBD6 (SEQ ID NO: 51) | TATTAGCGTTCGTCGT (SEQ ID NO: 787) |
| 340 | KBTBD6 (SEQ ID NO: 51) | GTTATTAGTGTTTGTTGT (SEQ ID NO: 788) |
| 341 | TRPM4 (SEQ ID NO: 52) | TAGGTGAGCGTCGGAT (SEQ ID NO: 789) |
| 342 | TRPM4 (SEQ ID NO: 52) | TAGGTGAGTGTTGGAT (SEQ ID NO: 790) |
| 343 | TRPM4 (SEQ ID NO: 52) | AGTAGAGTCGGCGGAG (SEQ ID NO: 791) |
| 344 | TRPM4 (SEQ ID NO: 52) | AGTAGAGTTGGTGGAG (SEQ ID NO: 792) |
| 345 | TCEB3BP1 (SEQ ID NO: 53) | GACGTTAGCGAGTATT (SEQ ID NO: 793) |
| 346 | TCEB3BP1 (SEQ ID NO: 53) | AGGGATGTTAGTGAGT (SEQ ID NO: 794) |
| 347 | TCEB3BP1 (SEQ ID NO: 53) | TGTCGGGTCGAGTAGT (SEQ ID NO: 795) |
| 348 | TCEB3BP1 (SEQ ID NO: 53) | TGTTGGGTTGAGTAGT (SEQ ID NO: 796) |
| 349 | Q8NCX8 (SEQ ID NO: 54) | TTACGGCGGGTTTTTA (SEQ ID NO: 797) |
| 350 | Q8NCX8 (SEQ ID NO: 54) | TTATGGTGGGTTTTTATT (SEQ ID NO: 798) |
| 351 | Q8NCX8 (SEQ ID NO: 54) | TTCGGTTTATACGATTT (SEQ ID NO: 799) |
| 352 | Q8NCX8 (SEQ ID NO: 54) | ATTTGGTTTATATGATTTTT (SEQ ID NO: 800) |
| 353 | Q8NCX8 (SEQ ID NO: 54) | TTACGTCGATTATCGG (SEQ ID NO: 801) |
| 354 | Q8NCX8 (SEQ ID NO: 54) | TATGTTGATTATTGGGGT (SEQ ID NO: 802) |

(continued)

| No: | Gene | Oligo: |
|---|---|---|
| 355 | SEQ ID NO: 55 (SEQ ID NO: 55) | AATTTACGCGGGTGTT (SEQ ID NO: 803) |
| 356 | SEQ ID NO: 55 (SEQ ID NO: 55) | GGAATTTATGTGGGTG (SEQ ID NO: 804) |
| 357 | SEQ ID NO: 55 (SEQ ID NO: 55) | TAGTTCGTTTCGGTTA (SEQ ID NO: 805) |
| 358 | SEQ ID NO: 55 (SEQ ID NO: 55) | AGTTTGTTTTGGTTAGT (SEQ ID NO: 806) |
| 359 | SEQ ID NO: 55 (SEQ ID NO: 55) | GTCGTGTACGAATTCGT (SEQ ID NO: 807) |
| 360 | SEQ ID NO: 55 (SEQ ID NO: 55) | GTTGTGTATGAATTTGTT (SEQ ID NO: 808) |
| 361 | SEQ ID NO: 55 (SEQ ID NO: 55) | AGCGTTTAAGTCGCGG (SEQ ID NO: 809) |
| 362 | SEQ ID NO: 55 (SEQ ID NO: 55) | TAGTGTTTAAGTTGTGG (SEQ ID NO: 810) |
| 363 | SNAPC2 (SEQ ID NO: 56) | TTACGATTTCGGTGTT (SEQ ID NO: 811) |
| 364 | SNAPC2 (SEQ ID NO: 56) | TAGAGTTATGATTTTGGT (SEQ ID NO: 812) |
| 365 | SNAPC2 (SEQ ID NO: 56) | AGGCGTGCGTCGATAT (SEQ ID NO: 813) |
| 366 | SNAPC2 (SEQ ID NO: 56) | AGGTGTGTGTTGATATT (SEQ ID NO: 814) |
| 367 | SNAPC2 (SEQ ID NO: 56) | GTAGCGTCGAGGGTTT (SEQ ID NO: 815) |
| 368 | SNAPC2 (SEQ ID NO: 56) | GTAGTGTTGAGGGTTT (SEQ ID NO: 816) |
| 369 | SNAPC2 (SEQ ID NO: 56) | ATACGTTGACGTAGTT (SEQ ID NO: 817) |
| 370 | SNAPC2 (SEQ ID NO: 56) | TTGTATATGTTGATGTAGT (SEQ ID NO: 818) |
| 371 | PTPRN2 (SEQ ID NO: 57) | GACGTAGTTCGTACGT (SEQ ID NO: 819) |
| 372 | PTPRN2 (SEQ ID NO: 57) | GGATGTAGTTTGTATGT (SEQ ID NO: 820) |
| 373 | PTPRN2 (SEQ ID NO: 57) | TTAGTCGTTTCGAGAT (SEQ ID NO: 821) |
| 374 | PTPRN2 (SEQ ID NO: 57) | GTTTTAGTTGTTTTGAGA (SEQ ID NO: 822) |
| 375 | PTPRN2 (SEQ ID NO: 57) | TTACGCGTATCGGGAT (SEQ ID NO: 823) |
| 376 | PTPRN2 (SEQ ID NO: 57) | TATGTGTATTGGGATTTA (SEQ ID NO: 824) |
| 377 | PTPRN2 (SEQ ID NO: 57) | TTCGCGTTTCGTAAGA (SEQ ID NO: 825) |
| 378 | PTPRN2 (SEQ ID NO: 57) | TTTGTGTTTTGTAAGATAT (SEQ ID NO: 826) |
| 379 | WDFY3 (SEQ ID NO: 58) | TATTGAGTCGGTCGTA (SEQ ID NO: 827) |
| 380 | WDFY3 (SEQ ID NO: 58) | ATTGAGTTGGTTGTAGA (SEQ ID NO: 828) |
| 381 | WDFY3 (SEQ ID NO: 58) | TAGATAGCGTCGTTGG (SEQ ID NO: 829) |
| 382 | WDFY3 (SEQ ID NO: 58) | TAGATAGTGTTGTTGGA (SEQ ID NO: 830) |
| 383 | ZNF566 (SEQ ID NO: 59) | ATAAAATACGACGTTGA (SEQ ID NO: 831) |
| 384 | ZNF566 (SEQ ID NO: 59) | ATAAAATATGATGTTGAATT (SEQ ID NO: 832) |
| 385 | ZNF566 (SEQ ID NO: 59) | AGCGTTTTTTACGAAT (SEQ ID NO: 833) |
| 386 | ZNF566 (SEQ ID NO: 59) | TAGTGTTTTTTATGAATGA (SEQ ID NO: 834) |
| 387 | Q9NP73 (SEQ ID NO: 60) | TATTACGGATTTTCGTT (SEQ ID NO: 835) |
| 388 | Q9NP73 (SEQ ID NO: 60) | GTTATTATGGATTTTTGTT (SEQ ID NO: 836) |
| 389 | Q9NP73 (SEQ ID NO: 60) | TGCGTTATATTCGGTT (SEQ ID NO: 837) |
| 390 | Q9NP73 (SEQ ID NO: 60) | AGTTGTGTTATATTTGGT (SEQ ID NO: 838) |
| 391 | Q9NP73 (SEQ ID NO: 60) | AATTCGCGTTATGAAG (SEQ ID NO: 839) |
| 392 | Q9NP73 (SEQ ID NO: 60) | TTGAGGAATTTGTGTTAT (SEQ ID NO: 840) |

(continued)

| No: | Gene | Oligo: |
|---|---|---|
| 393 | Q9NP73 (SEQ ID NO: 60) | GTCGGCGTTCGATAGT (SEQ ID NO: 841) |
| 394 | Q9NP73 (SEQ ID NO: 60) | TGTTGGTGTTTGATAGT (SEQ ID NO: 842) |
| 395 | SEQ ID NO: 61 (SEQ ID NO: 61) | AGAATTCGTAAACGGG (SEQ ID NO: 843) |
| 396 | SEQ ID NO: 61 (SEQ ID NO: 61) | ATTTGTAAATGGGGGT (SEQ ID NO: 844) |
| 397 | SEQ ID NO: 61 (SEQ ID NO: 61) | TTCGGTTATCGACGGT (SEQ ID NO: 845) |
| 398 | SEQ ID NO: 61 (SEQ ID NO: 61) | TTTGGTTATTGATGGTT (SEQ ID NO: 846) |
| 399 | Q86SP6 (SEQ ID NO: 62) | ATCGTGAGCGTAGCGT (SEQ ID NO: 847) |
| 400 | Q86SP6 (SEQ ID NO: 62) | ATTGTGAGTGTAGTGTA (SEQ ID NO: 848) |
| 401 | Q86SP6 (SEQ ID NO: 62) | TAGGCGTGAGAATCGG (SEQ ID NO: 849) |
| 402 | Q86SP6 (SEQ ID NO: 62) | TAGGTGTGAGAATTGG (SEQ ID NO: 850) |
| 403 | Q86SP6 (SEQ ID NO: 62) | ATCGTGTTCGGATCGG (SEQ ID NO: 851) |
| 404 | Q86SP6 (SEQ ID NO: 62) | TATTGTGTTTGGATTGG (SEQ ID NO: 852) |
| 405 | Q86SP6 (SEQ ID NO: 62) | AGGTTCGAGTTTGCGG (SEQ ID NO: 853) |
| 406 | Q86SP6 (SEQ ID NO: 62) | TAGGTTTGAGTTTGTGG (SEQ ID NO: 854) |
| 407 | Q86SP6 (SEQ ID NO: 62) | TATAAAGCGCGAGTGT (SEQ ID NO: 855) |
| 408 | Q86SP6 (SEQ ID NO: 62) | TATAAAGTGTGAGTGTG (SEQ ID NO: 856) |
| 409 | RARB (SEQ ID NO: 8) | ATGTCGAGAACGCGAG (SEQ ID NO: 857) |
| 410 | RARB (SEQ ID NO: 8) | GGATGTTGAGAATGTGA (SEQ ID NO: 858) |
| 411 | RARB (SEQ ID NO: 8) | AGCGATTCGAGTAGGG (SEQ ID NO: 859) |
| 412 | RARB (SEQ ID NO: 8) | AGTGATTTGAGTAGGG (SEQ ID NO: 860) |
| 413 | RARB (SEQ ID NO: 8) | TATCGTCGGGGTAGGA (SEQ ID NO: 861) |
| 414 | RARB (SEQ ID NO: 8) | TATTGTTGGGGTAGGA (SEQ ID NO: 862) |
| 415 | RARB (SEQ ID NO: 8) | AACGTATTCGGAAGGT (SEQ ID NO: 863) |
| 416 | RARB (SEQ ID NO: 8) | GAATGTATTTGGAAGGT (SEQ ID NO: 864) |
| 417 | PTGS2 (SEQ ID NO: 9) | AGCGTTTTCGAGAGTT (SEQ ID NO: 865) |
| 418 | PTGS2 (SEQ ID NO: 9) | GAGTGTTTTTGAGAGTT (SEQ ID NO: 866) |
| 419 | PTGS2 (SEQ ID NO: 9) | TTACGTCGGGATAGAT (SEQ ID NO: 867) |
| 420 | PTGS2 (SEQ ID NO: 9) | GGAAGTTATGTTGGGA (SEQ ID NO: 868) |
| 421 | PTGS2 (SEQ ID NO: 9) | TATCGTTTTAGGCGTA (SEQ ID NO: 869) |
| 422 | PTGS2 (SEQ ID NO: 9) | TTTATTGTTTTAGGTGTAT (SEQ ID NO: 870) |
| 423 | RASSF1 (SEQ ID NO: 10) | TACGGGTATTTTCGCGT (SEQ ID NO: 871) |
| 424 | RASSF1 (SEQ ID NO: 10) | ATATGGGTATTTTTGTGT (SEQ ID NO: 872) |
| 425 | RASSF1 (SEQ ID NO: 10) | AGAGCGCGTTTAGTTT (SEQ ID NO: 873) |
| 426 | RASSF1 (SEQ ID NO: 10) | GAGAGTGTGTTTAGTTT (SEQ ID NO: 874) |
| 427 | ESR2 (SEQ ID NO: 11) | TAGGAACGTTCGACGT (SEQ ID NO: 875) |
| 428 | ESR2 (SEQ ID NO: 11) | TTTAGGAATGTTTGATGT (SEQ ID NO: 876) |
| 429 | ESR2 (SEQ ID NO: 11) | ATGGCGTTTTTCGTAG (SEQ ID NO: 877) |
| 430 | ESR2 (SEQ ID NO: 11) | TAGATGGTGTTTTTTGT (SEQ ID NO: 878) |

(continued)

| No: | Gene | Oligo: |
|---|---|---|
| 431 | ESR2 (SEQ ID NO: 11) | ATAAGCGATTTAACGAT (SEQ ID NO: 879) |
| 432 | ESR2 (SEQ ID NO: 11) | AGTGATTTAATGATAAGTT (SEQ ID NO: 880) |
| 433 | ESR2 (SEQ ID NO: 11) | ATTTCGAGGATTACGT (SEQ ID NO: 881) |
| 434 | ESR2 (SEQ ID NO: 11) | ATATTTTGAGGATTATGTT (SEQ ID NO: 882) |
| 435 | DRG1 (SEQ ID NO: 12) | TAGTCGTTAAAACGTAG (SEQ ID NO: 883) |
| 436 | DRG1 (SEQ ID NO: 12) | AGTTGTTAAAATGTAGATT (SEQ ID NO: 884) |
| 437 | DRG1 (SEQ ID NO: 12) | ATCGTATTGGTTCGCGG (SEQ ID NO: 885) |
| 438 | DRG1 (SEQ ID NO: 12) | ATTGTATTGGTTTGTGG (SEQ ID NO: 886) |
| 439 | DRG1 (SEQ ID NO: 12) | TTTACGTTTTGCGATT (SEQ ID NO: 887) |
| 440 | DRG1 (SEQ ID NO: 12) | AGTTTTATGTTTTGTGAT (SEQ ID NO: 888) |
| 441 | DRG1 (SEQ ID NO: 12) | ATTTTTACGCGGAATT (SEQ ID NO: 889) |
| 442 | DRG1 (SEQ ID NO: 12) | GTGATTTTTATGTGGAAT (SEQ ID NO: 890) |
| 443 | DRG1 (SEQ ID NO: 12) | ATTCGAAGTATCGCGT (SEQ ID NO: 891) |
| 444 | DRG1 (SEQ ID NO: 12) | ATTTGAAGTATTGTGTTT (SEQ ID NO: 892) |
| 445 | DRG1 (SEQ ID NO: 12) | ATTTCGAATTTCGAGTA (SEQ ID NO: 893) |
| 446 | DRG1 (SEQ ID NO: 12) | TTTGAATTTTGAGTAGAG (SEQ ID NO: 894) |
| 447 | DOCK10 (SEQ ID NO: 16) | TATATTGTCGGGGAGA (SEQ ID NO: 895) |
| 448 | DOCK10 (SEQ ID NO: 16) | ATATTGTTGGGGAGAG (SEQ ID NO: 896) |
| 449 | BTG4 (SEQ ID NO: 17) | GTGTTGCGTAGAGATA (SEQ ID NO: 897) |
| 450 | BTG4 (SEQ ID NO: 17) | GGTGTTGTGTAGAGAT (SEQ ID NO: 898) |
| 451 | BTG4 (SEQ ID NO: 17) | TTGGTTTTTCGGAATAA (SEQ ID NO: 899) |
| 452 | BTG4 (SEQ ID NO: 17) | GGTTTTTTGGAATAAGAT (SEQ ID NO: 900) |
| 453 | GPR7 (SEQ ID NO: 19) | ATTGAGGGCGTATAGA (SEQ ID NO: 901) |
| 454 | GPR7 (SEQ ID NO: 19) | TGAGGGTGTATAGATTT (SEQ ID NO: 902) |
| 455 | GPR7 (SEQ ID NO: 19) | GGAGATCGAAGTTTGT (SEQ ID NO: 903) |
| 456 | GPR7 (SEQ ID NO: 19) | GGGGAGATTGAAGTTT (SEQ ID NO: 904) |
| 457 | ISL1 (SEQ ID NO: 21) | AGATTTTGCGAAAGATA (SEQ ID NO: 905) |
| 458 | ISL1 (SEQ ID NO: 21) | TTAGATTTTGTGAAAGATA (SEQ ID NO: 906) |
| 459 | ISL1 (SEQ ID NO: 21) | AAGATATCGAAATTAAGTT (SEQ ID NO: 907) |
| 460 | ISL1 (SEQ ID NO: 21) | AAGATATTGAAATTAAGTTT (SEQ ID NO: 908) |
| 461 | GPRK5 (SEQ ID NO: 22) | AGATTTTCGAGGGAGA (SEQ ID NO: 909) |
| 462 | GPRK5 (SEQ ID NO: 22) | AGATTTTTGAGGGAGAT (SEQ ID NO: 910) |
| 463 | FBN2 (SEQ ID NO: 28) | AATTGGTCGTTAGTTTT (SEQ ID NO: 911) |
| 464 | FBN2 (SEQ ID NO: 28) | GTTAATTGGTTGTTAGTT (SEQ ID NO: 912) |
| 465 | FBN2 (SEQ ID NO: 28) | TTAGGGATCGGATTTG (SEQ ID NO: 913) |
| 466 | FBN2 (SEQ ID NO: 28) | ATTAGGGATTGGATTTG (SEQ ID NO: 914) |
| 467 | LIMK1 (SEQ ID NO: 30) | TTTAATTCGAAAGGGAA (SEQ ID NO: 915) |
| 468 | LIMK1 (SEQ ID NO: 30) | TTAATTTGAAAGGGAAAG (SEQ ID NO: 916) |

(continued)

| No: | Gene | Oligo: |
|---|---|---|
| 469 | PSD/Q9H469 (SEQ ID NO: 31) | AGGTTAGTCGAGAAGT (SEQ ID NO: 917) |
| 470 | PSD/Q9H469 (SEQ ID NO: 31) | AGGTTAGTTGAGAAGTA (SEQ ID NO: 918) |
| 471 | PSD/Q9H469 (SEQ ID NO: 31) | ATTGTTACGAAGTGGA (SEQ ID NO: 919) |
| 472 | PSD/Q9H469 (SEQ ID NO: 31) | TTGTTATGAAGTGGAAG (SEQ ID NO: 920) |
| 473 | Q96S01 (SEQ ID NO: 34) | TATTGATCGGTTGGAG (SEQ ID NO: 921) |
| 474 | Q96S01 (SEQ ID NO: 34) | TATTGATTGGTTGGAGG (SEQ ID NO: 922) |
| 475 | ABHD9 (SEQ ID NO: 37) | AGTTAGAGCGTATTATTT (SEQ ID NO: 923) |
| 476 | ABHD9 (SEQ ID NO: 37) | AATAGTTAGAGTGTATTATT (SEQ ID NO: 924) |
| 477 | MLLT3 (SEQ ID NO: 42) | TTAGTGGTCGGAGATA (SEQ ID NO: 925) |
| 478 | MLLT3 (SEQ ID NO: 42) | AGTGGTTGGAGATAGA (SEQ ID NO: 926) |
| 479 | SOLH (SEQ ID NO: 43) | TATATTTCGTGAGGGTA (SEQ ID NO: 927) |
| 480 | SOLH (SEQ ID NO: 43) | TATATTTTGTGAGGGTAG (SEQ ID NO: 928) |
| 481 | Q9NWV0 (SEQ ID NO: 46) | ATTTTTACGAGAAGGTT (SEQ ID NO: 929) |
| 482 | Q9NWV0 (SEQ ID NO: 46) | GATTTTTATGAGAAGGTT (SEQ ID NO: 930) |
| 483 | H2AFY2 (SEQ ID NO: 48) | ATGGGAATCGTGGTTT (SEQ ID NO: 931) |
| 484 | H2AFY2 (SEQ ID NO: 48) | ATGGGAATTGTGGTTT (SEQ ID NO: 932) |
| 485 | RHOC (SEQ ID NO: 49) | AGGTTTACGGAAAAGG (SEQ ID NO: 933) |
| 486 | RHOC (SEQ ID NO: 49) | AAGGTTTATGGAAAAGG (SEQ ID NO: 934) |
| 487 | KBTBD6 (SEQ ID NO: 51) | TAGAGTCGGGTTTTGTA (SEQ ID NO: 935) |
| 488 | KBTBD6 (SEQ ID NO: 51) | TAGAGTTGGGTTTTGTA (SEQ ID NO: 936) |
| 489 | TRPM4 (SEQ ID NO: 52) | ATGGGGGTCGAGATTT (SEQ ID NO: 937) |
| 490 | TRPM4 (SEQ ID NO: 52) | ATGGGGGTTGAGATTT (SEQ ID NO: 938) |
| 491 | TCEB3BP1 (SEQ ID NO: 53) | GGTAGTCGGTATTAGG (SEQ ID NO: 939) |
| 492 | TCEB3BP1 (SEQ ID NO: 53) | TGGTAGTTGGTATTAGG (SEQ ID NO: 940) |
| 493 | TCEB3BP1 (SEQ ID NO: 53) | TGTAGTCGAAGGTTAG (SEQ ID NO: 941) |
| 494 | TCEB3BP1 (SEQ ID NO: 53) | TGTAGTTGAAGGTTAGT (SEQ ID NO: 942) |
| 495 | Q8NCX8 (SEQ ID NO: 54) | GGAGTTTATTCGGTTTAT (SEQ ID NO: 943) |
| 496 | Q8NCX8 (SEQ ID NO: 54) | GGAGTTTATTTGGTTTATA (SEQ ID NO: 944) |
| 497 | WDFY3 (SEQ ID NO: 58) | AATTGTAGTCGTTTAGTT (SEQ ID NO: 945) |
| 498 | WDFY3 (SEQ ID NO: 58) | AAATTGTAGTTGTTTAGTT (SEQ ID NO: 946) |
| 499 | ZNF566 (SEQ ID NO: 59) | TATTTTTTCGGTAGAGAT (SEQ ID NO: 947) |
| 500 | ZNF566 (SEQ ID NO: 59) | TTTTTTTGGTAGAGATTG (SEQ ID NO: 948) |
| 501 | ZNF566 (SEQ ID NO: 59) | ATGGGTTGCGTTTATA (SEQ ID NO: 949) |
| 502 | ZNF566 (SEQ ID NO: 59) | TGGGTTGTGTTTATAAG (SEQ ID NO: 950) |
| 503 | SEQ ID NO: 61 (SEQ ID NO: 61) | TTTAGGGCGAGGTTAT (SEQ ID NO: 951) |
| 504 | SEQ ID NO: 61 (SEQ ID NO: 61) | TTTAGGGTGAGGTTATT (SEQ ID NO: 952) |
| 505 | SEQ ID NO: 61 (SEQ ID NO: 61) | TGTATATTTCGTAGGGT (SEQ ID NO: 953) |
| 506 | SEQ ID NO: 61 (SEQ ID NO: 61) | TTGTATATTTTGTAGGGT (SEQ ID NO: 954) |

(continued)

| No: | Gene | Oligo: |
|---|---|---|
| 507 | PTGS2 (SEQ ID NO: 9) | ATTTGAGCGGTTTTGA (SEQ ID NO: 955) |
| 508 | PTGS2 (SEQ ID NO: 9) | AATTTGAGTGGTTTTGA (SEQ ID NO: 956) |
| 509 | RASSF1 (SEQ ID NO: 10) | AGTAAATCGGATTAGGA (SEQ ID NO: 957) |
| 510 | RASSF1 (SEQ ID NO: 10) | AGTAAATTGGATTAGGAG (SEQ ID NO: 958) |
| 511 | DRG1 (SEQ ID NO: 12) | TGTATGAACGTGTAGTT (SEQ ID NO: 959) |
| 512 | DRG1 (SEQ ID NO: 12) | GTGTATGAATGTGTAGT (SEQ ID NO: 960) |

TABLE 11: Genes and sequences according to sequence listing.

| Gene (HUGO or SPTREMBL ID or EST Gene ID) | Ref.-Seq | Genomic SEQ ID NO: | Sense methylated converted SEQ ID NO: | Antisense methylated converted SEQ ID NO: | Sense unmethylated converted SEQ ID NO: | Antisense unmethylated converted SEQ ID NO: |
|---|---|---|---|---|---|---|
| CCND2 | NM_001759 | 1 | 65 | 66 | 193 | 194 |
| CDRN2A | NM_000077 | 2 | 67 | 68 | 195 | 196 |
| CD44 | NM_000610 | 3 | 69 | 70 | 197 | 198 |
| EDNRB1 | NM_000115 | 4 | 71 | 72 | 199 | 200 |
| ELK1 | NM_005229 | 5 | 73 | 74 | 201 | 202 |
| FOS | NM_005252 | 6 | 75 | 76 | 203 | 204 |
| GSTP1 | NM_000852 | 7 | 77 | 78 | 205 | 206 |
| RARB | NM_000965 | 8 | 79 | 80 | 207 | 208 |
| PTGS2 | NM_000963 | 9 | 81 | 82 | 209 | 210 |
| RASSF1 | NM_170715 | 10 | 83 | 84 | 211 | 212 |
| ESR2 | NM_001437 | 11 | 85 | 86 | 213 | 214 |
| DRG1 | NM_004147 | 12 | 87 | 88 | 215 | 216 |
| CMYA3 | | 13 | 89 | 90 | 217 | 218 |
| ONECUT2 | NM_004852 | 14 | 91 | 92 | 219 | 220 |
| MX1 | NM_002462 | 15 | 93 | 94 | 221 | 222 |
| DOCK10 | | 16 | 95 | 96 | 223 | 224 |
| BTG4 | NM_017589 | 17 | 97 | 98 | 225 | 226 |
| DMRTC2 | NM_033052 | 18 | 99 | 100 | 227 | 228 |
| GPR7 | NM_005285 | 19 | 101 | 102 | 229 | 230 |
| FAT | NM_005245 | 20 | 103 | 104 | 231 | 232 |
| ISL1 | NM_002202 | 21 | 105 | 106 | 233 | 234 |
| GPRK5 | NM_005308 | 22 | 107 | 108 | 235 | 236 |
| SLC35F2 | NM_017515 | 23 | 109 | 110 | 237 | 238 |

(continued)

| Gene (HUGO or SPTREMBL ID or EST Gene ID) | Ref.-Seq | Genomic SEQ ID NO: | Sense methylated converted SEQ ID NO: | Antisense methylated converted SEQ ID NO: | Sense unmethylated converted SEQ ID NO: | Antisense unmethylated converted SEQ ID NO: |
|---|---|---|---|---|---|---|
| C14orf59 | NM_174976 | 24 | 111 | 112 | 239 | 240 |
| SNRPN | NM_003097 | 25 | 113 | 114 | 241 | 242 |
| ARHGEF18 | NM_015318 | 26 | 115 | 116 | 243 | 244 |
| SNX8 | NM_013321 | 27 | 117 | 118 | 245 | 246 |
| FBN2 | NM_001999 | 28 | 119 | 120 | 247 | 248 |
| HOXB5 | NM_002147 | 29 | 121 | 122 | 249 | 250 |
| LIMK1 | NM_002314 | 30 | 123 | 124 | 251 | 252 |
| PSD; Q9H469 | NM_002779; NM_ 024326 | 31 | 125 | 126 | 253 | 254 |
| SLC38A1 | NM_030674 | 32 | 127 | 128 | 255 | 256 |
| HIST1H4J | NM_003495 | 33 | 129 | 130 | 257 | 258 |
| Q96S01 | Not applicable | 34 | 131 | 132 | 259 | 260 |
| Genomic region downstream of FOXL2 | NM_023067 | 35 | 133 | 134 | 261 | 262 |
| ORC4L | NM_002552 | 36 | 135 | 136 | 263 | 264 |
| ABHD9 | NM_024794 | 37 | 137 | 138 | 265 | 266 |
| CD37 | NM_001774 | 38 | 139 | 140 | 267 | 268 |
| GRN | NM_002087 | 39 | 141 | 142 | 269 | 270 |
| EPAS1 | NM_001430 | 40 | 143 | 144 | 271 | 272 |
| NOTCH1 | NM_017617 | 41 | 145 | 146 | 273 | 274 |
| MLLT3 | NM_004529 | 42 | 147 | 148 | 275 | 276 |
| SOLH | NM_005632 | 43 | 149 | 150 | 277 | 278 |
| ENSESTG00002636932 | Not applicable | 44 | 151 | 152 | 279 | 280 |
| Q8N365 | NM_144697 | 45 | 153 | 154 | 281 | 282 |

(continued)

| Gene (HUGO or SPTREMBL ID or EST Gene ID) | Ref.-Seq | Genomic SEQ ID NO: | Sense methylated converted SEQ ID NO: | Antisense methylated converted SEQ ID NO: | Sense unmethylated converted SEQ ID NO: | Antisense unmethylated converted SEQ ID NO: |
|---|---|---|---|---|---|---|
| Q9NWV0 | NM_017891 | 46 | 155 | 156 | 283 | 284 |
| ENST00000339569 | Not applicable | 47 | 157 | 158 | 285 | 286 |
| H2AFY2 | NM_018649 | 48 | 159 | 160 | 287 | 288 |
| RHOC | NM_005167 | 49 | 161 | 162 | 289 | 290 |
| NR2E1 | NM_003269 | 50 | 163 | 164 | 291 | 292 |
| KBTBD6 | NM_152903 | 51 | 165 | 166 | 293 | 294 |
| TRPM4 | NM_017636 | 52 | 167 | 168 | 295 | 296 |
| TCEB3BP1 | NM_020695 | 53 | 169 | 170 | 297 | 298 |
| Q8NCX8 | NM_178564 | 54 | 171 | 172 | 299 | 300 |
| Genomic sequence | Genomic sequence | 55 | 173 | 174 | 301 | 302 |
| SNAPC2 | NM_003083 | 56 | 175 | 176 | 303 | 304 |
| PTPRN2 | NM_002847 | 57 | 177 | 178 | 305 | 306 |
| WDFY3 | NM_014991 | 58 | 179 | 180 | 307 | 308 |
| ZNF566 | NM_032838 | 59 | 181 | 182 | 309 | 310 |
| Q9NP73 | NM_018466 | 60 | 183 | 184 | 311 | 312 |
| | NM_173660 | 61 | 185 | 186 | 313 | 314 |
| Q86SP6 | Not applicable | 62 | 187 | 188 | 315 | 316 |
| HIST2H2BF regulatory region | NM_003529 | 63 | 189 | 190 | 317 | 318 |
| PMF1 | NM_000711 | 64 | 191 | 192 | 319 | 320 |
| PITX2 | NM_000325 | 961 | 962 | 963 | 964 | 965 |

TABLE 12: Primers and Probes for MSP-MethyLight™ Assays.

| | Forward | Reverse | Probe |
|---|---|---|---|
| SEQ ID NO: 19 | tgcggattttggcgaattc | aaaaacccgccgactacga a | aactaaaacgcctaccgactaaatatccg cct |
| SEQ ID NO: 35 | gagttttcgcggttcgga | cgcgaccgctaaactcg | cgaccaaatccgaacccgtacatcg |
| SEQ ID NO: 37 | cggtatgtcgtcgcgtttc | ctaacaccgcttcgccg | cctaaccgacaacgccgccgtaat |
| SEQ ID NO: 7 | agttgcgcggcgatttc | gccccaatactaaatcacga cg | cggtcgacgttcggggtgtagcg |
| SEQ ID NO: 63 | atggcgtataggttcgtgttttc | cttccaacgactaatacgcga a | cgcttccaaaactcgaccgtaataacgc |
| SEQ ID NO: 8 | atataaactaaaaaacgaaacgataaac ga | ttttacgttttttatttgcggc | cgaacgaacgcaaacgaaacaccg |
| SEQ ID NO: 64 | ccactaactccgtaccgtacgtat | ggttagcgagtcgatcggtt | acgttctcgtctccgctaaattatccgc |
| SEQ ID NO: 43 | tcgtttttttagtcgtttgggtc | tatcgaaaccccgaaccg | caccgtcgcctcccacgaca |
| SEQ ID NO: 40 | ttttcgtttttttcggtcgtt | gaccgcgcaaaaaaactcg | cgctcgaataacgccgaacccg |
| SEQ ID NO: 32 | ctcgcacaaaaacgaaaatacg | agttcgcgtttttaaacgttgtc | ccgacaccgacccacgcgt |
| SEQ ID NO: 37 | cggtatgtcgtcgcgtttc | ctaacaccgcttcgccg | cctaaccgacaacgccgccgtaat |
| SEQ ID NO: 9 | aaacgaaaactctacccgaatacg | cgcggttttggcgttt | ccgaaatccccgatacgcgacg |
| SEQ ID NO: 19 | tgcggattttggcgaattc | aaaaacccgccgactacga a | aactaaaacgcctaccgactaaatatccg cct |
| SEQ ID NO: 34 | gtatttatttggtaatttcgtattataattcgag | gactaaaaacgcgaaatccg a | acgatccgatctaaaaaccgactcttcgaa |
| SEQ ID NO: 35 | gagttttcgcggttcgga | cgcgaccgctaaactcg | cgaccaaatccgaacccgtacatcg |

Table 13: Components for all QM assays according to Example 5

| Component | Company | Stock conc. |
|---|---|---|
| Reaction buffer ROX | Eurogentec | 10x |
| MgCl2 | Eurogentec | 50mM |
| DNTPs | MBI | 25mM each |
| Forward primer | TIB Molbiol | 6,25μM |
| Reverse primer | TIB Molbiol | 6,25μM |
| cg Probe | Eurogentec | 4μM |
| tg Probe | Eurogentec | 4μM |
| HotGoldStar-Taq | Eurogentec | 5U/μl |
| Water | Fluka | |

Table 14: Optimized Reaction conditions for all QM assays according to Example 5

| Gene | dNTPs | Buffer | $MgCl_2$ | Primers | Probes | Taq | Baseline | Threshold | Annealing |
|---|---|---|---|---|---|---|---|---|---|
| PITX2 | 250μM | 1 x | 3mM | 625 nM | 200 nM | 1U | 3/23 | 0,05 | 62°C |
| Chr3-EST | 200μM | 1 x | 3.5mM | 625 nM | 200 nM | 1U | 6/22 | 0,05 | 60°C |
| ABHD9 | 200μM | 1 x | 2.5mM | 625 nM | 200 nM | 1U | 6/25 | 0,08 | 60°C |
| GPR7 | 250μM | 1 x | 3mM | 625nM | 150 nM | 1U | 6/24 | 0,05 | 60°C |
| HIST 2H2BF | 250μM | 1 x | 3mM | 625 nM | 250 nM | 1U | 3/22 | 0,05 | 60°C |
| CCND2 | 250μM | 1 x | 3mM | 625 nM | 250 nM | 1 U | 3/22 | 0,08 | 60°C |

Table 15: Cycle program for QM assays according to Example 5. For annealing temperatures see Table 14.

| | T [°C] | t | Cycles |
|---|---|---|---|
| Initial denat. | 95.0 | 10min | |
| Denaturation | 95.0 | 15sec | 45x (PITX2 50x) |
| Annealing | Variable | 60sec | |

Table 16: Clinical characteristics of the patient population according to Example 5. Age is given as the mean, and all other variables are given as the number of patients. Not all information was available for all patients.

| Clinical Variable | | Baylor | Stanford | VMMC | Total |
|---|---|---|---|---|---|
| Age (mean) | | 61.1 | 61.7 | 61.1 | 61.3 |
| PSA | 0-4 | 25 | 33 | 18 | 76 |
| | 4-10 | 120 | 139 | 99 | 358 |
| | >10 | 60 | 72 | 30 | 162 |
| Gleason Score | 5-6 | 137 | 164 | 118 | 419 |
| | 7 | 37 | 44 | 19 | 100 |
| | 8-10 | 26 | 31 | 25 | 82 |

(continued)

| Clinical Variable | | Baylor | Stanford | VMMC | Total |
|---|---|---|---|---|---|
| Stage | Organ- confined | 110 | 211 | 113 | 434 |
| | Not organ- confined | 94 | 33 | 35 | 162 |
| PSA-based recurrence | | 22 | 10 | 13 | 45 |
| Decision to treat based recurrence | | 3 | 14 | 4 | 21 |
| Total Samples | | 206 | 244 | 162 | 612 |

Table 17: Performance of the six markers according to Example 5 using the median methylation level as a cut-off.

| | P value | Events in hypomethylated group | Events in hypermethylated group | AUC (5 years) |
|---|---|---|---|---|
| PITX2 | 0.000017 | 15 | 49 | 0.64 |
| GPR7 | 0.0016 | 20 | 45 | 0.64 |
| HIST2H2BF regulatory region | 0.018 | 22 | 43 | 0.60 |
| SEQ ID NO: 35 | 0.0059 | 21 | 44 | 0.61 |
| ABHD9 | 0.018 | 22 | 43 | 0.58 |
| CCND2 | 0.22 | 27 | 38 | 0.61 |

Table 18: Results of the Cox regression analysis for PITX2 according to Example 5. Using stepwise regression the marker remains in the model. P-values refer to the null-hypothesis "hazard ratio equals zero".

| Variable | P value | Hazard Ratio | Lower Confidence Interval | Upper Confidence Interval |
|---|---|---|---|---|
| PITX2 | 0.0043 | 2.222 | 1.284 | 3.845 |
| Disease stage | 0.0692 | 1.713 | 0.965 | 3.061 |
| Gleason category | 0.0107 | 1.798 | 1.146 | 2.821 |
| PSA | 0.075 | 1.254 | 0.977 | 1.609 |
| Nomogram category | 0.0866 | 2.187 | 0.894 | 5.353 |

Table 19: Results of the Cox regression analysis for SEQ ID NO: 63 according to Example 5. The marker remains in the model.

| Variable | P value | Hazard Ratio | Lower Confidence Interval | Upper Confidence Interval |
|---|---|---|---|---|
| SEQ ID NO: 63 | 0.0239 | 2.918 | 1.152 | 7.393 |
| Disease stage | 0.0599 | 1.735 | 0.977 | 3.081 |
| Gleason category | 0.0106 | 1.799 | 1.146 | 2.822 |
| PSA | 0.0732 | 1.25 | 0.979 | 1.596 |
| Nomogram category | 0.0384 | 2.526 | 1.051 | 6.071 |

Table 20: Primers and probe sequences of assays according to Example 5.

| CCND2 | | | |
|---|---|---|---|
| Primers + Probes | Sequence | Label 5' | Label 3' |
| Forward primer | Ttttgtaaagatagttttgatttaagtat (SEQ ID NO: 983) | | |
| Reverse primer | caaactttctccctaaaaacc (SEQ ID NO: 984) | | |
| CG-probe | Cgccgccaacacgatcg (SEQ ID NO: 985) | FAM | BHQ1 |
| TG-probe | Caccaccaacacaatcaaccctaacac (SEQ ID NO: 986) | HEX | BHQ1 |
| | | | |
| SEQ ID NO: 63 | | | |
| Primers + Probes | Sequence | Label 5' | Label 3' |
| Forward primer | tgattattatgtttaaggatatttagttg (SEQ ID NO: 987) | | |
| Reverse primer | caataactctaaaaaaaacctttaaatc (SEQ ID NO: 988) | | |
| CG-probe | Cgctccccgcgaatacgacg (SEQ ID NO: 989) | FAM | BHQ1 |
| TG-probe | Taaacccactccccacaaatacaacaaac (SEQ ID NO: 990) | HEX | BHQ1 |
| | | | |
| GRP7 | | | |
| Primers + Probes | Sequence | Label 5' | Label 3' |
| Forward primer | Catccctacacttccaaac (SEQ ID NO: 991) | | |
| Reverse primer | Ggagttgttaggagaaaagtt (SEQ ID NO: 992) | | |
| CG-probe | Cgaacacccaaccgacaaacg (SEQ ID NO: 993) | FAM | BHQ1 |
| TG-probe | Caaacacccaaccaacaaacatctca (SEQ ID NO: 994) | HEX | BHQ1 |
| | | | |
| Chr3_EST | | | |
| Primers + Probes | Sequence | Label 5' | Label 3' |
| Forward primer | ttgtagggttttttgggtt (SEQ ID NO: 995) | | |
| Reverse primer | Ctcaaaacccttaaaaacataaa (SEQ | | |
| | ID NO: 996) | | |
| CG-probe | Ataaccacactacgcgcctcc (SEQ ID NO: 997) | FAM | BHQ1 |
| TG-probe | Ataaccacactacacacctcccaca (SEQ ID NO: 998) | Yakima Yellow | BHQ1 |
| | | | |
| ABHD9 | | | |
| Primers + Probes | Sequence | Label 5' | Label 3' |
| Forward primer | Ggtgttagggtttaggggtt (SEQ ID NO: 999) | | |
| Reverse primer | Ccaaatatttacctaacactcaaata (SEQ ID NO: 1000) | | |
| CG-probe | Aactattttctatcgaaaccgcccg (SEQ ID NO: 1001) | FAM | BHQ1 |
| TG-probe | Aactattttctatcaaaaccacccacctct (SEQ ID NO: 1002) | Yakima Yellow | BHQ1 |

(continued)

| ABHD9 | | | |
|---|---|---|---|
| Primers + Probes | Sequence | Label 5' | Label 3' |
| | | | |
| PITX2 | | | |
| Primers + Probes | Sequence | Label 5' | Label 3' |
| Forward primer | Gtaggggagggaagtagatgtt (SEQ ID NO: 1003) | | |
| Reverse primer | Ttctaatcctcctttccacaataa (SEQ ID NO: 1004) | | |
| CG-probe | Agtcggagtcgggagagcga (SEQ ID NO: 1005) | FAM | TAMRA |
| TG-probe | Agttggagttgggagagtgaaaggaga (SEQ ID NO: 1006) | VIC | TAMRA |
| CCND2 | | | |
| Primers + Probes | Sequence | Label 5' | Label 3' |
| Forward primer | tttttgtaaagatagttttgatttaagtat | | |
| Reverse primer | caaactttctccctaaaaacc | | |
| CG-probe | cgccgccaacacgatcg | FAM | BHQ1 |
| TG-probe | caccaccaacacaatcaaccctaacac | HEX | BHQ1 |
| | | | |
| SEQ ID NO: 63 | | | |
| Primers + Probes | Sequence | Label 5' | Label 3' |
| Forward primer | tgattattatgtttaaggatatttagttg | | |
| Reverse primer | caataactctaaaaaaaacctttaaatc | | |
| CG-probe | cgctccccgcgaatacgacg | FAM | BHQ1 |
| TG-probe | taaacccactccccacaaatacaacaaac | HEX | BHQ1 |
| | | | |
| GRP7 | | | |
| Primers + Probes | Sequence | Label 5' | Label 3' |
| Forward primer | catccctacacttccaaac | | |
| Reverse primer | ggagttgttaggagaaaagtt | | |
| CG-probe | cgaacacccaaccgacaaacg | FAM | BHQ1 |
| TG-probe | caaacacccaaccaacaaacatctca | HEX | BHQ1 |
| | | | |
| Chr3_EST | | | |
| Primers + Probes | Sequence | Label 5 | Label 3' |
| Forward primer | ttgtagggtttttttgggtt | | |
| Reverse primer | ctcaaaacccttaaaaacataaa | | |

(continued)

| Chr3_EST | | | |
|---|---|---|---|
| Primers + Probes | Sequence | Label 5 | Label 3’ |
| CG-probe | ataaccacactacgcgcctcc | FAM | BHQ1 |
| TG-probe | ataaccacactacacacctcccaca | Yakima Yellow | BHQ1 |
| | | | |
| ABHD9 | | | |
| Primers + Probes | Sequence | Label 5’ | Label 3’ |
| Forward primer | ggtgttagggtttaggggtt | | |
| Reverse primer | ccaaatatttacctaacactcaaata | | |
| CG-probe | aactattttctatcgaaaccgcccg | FAM | BHQ1 |
| TG-probe | aactattttctatcaaaaccacccacctct | Yakima Yellow | BHQ1 |
| | | | |
| PITX2 | | | |
| Primers + Probes | Sequence | Label 5’ | Label 3’ |
| Forward primer | gtaggggagggaagtagatgtt | | |
| Reverse primer | ttctaatcctcctttccacaataa | | |
| CG-probe | agtcggagtcgggagagcga | FAM | TAMRA |
| TG-probe | agttggagttgggagagtgaaaggaga | VIC | TAMRA |

Table 21

| SEQ ID NO | Classification | Tissue Type | AUC | Sensitivity | Specificity |
|---|---|---|---|---|---|
| 19 | Recurrance | Frozen & PET | 0.62 | 0.35 | 0.86 |
| 63 | Recurrance | Frozen & PET | 0.68 | 0.34 | 0.85 |
| 35 | Recurrance | Frozen & PET | 0.6 | 0.27 | 0.85 |
| 37 | Recurrance | Frozen & PET | 0.61 | 0.18 | 0.85 |
| 19 | Recurrance | PET | 0.58 | 0.5 | 0.88 |
| 63 | Recurrance | PET | 0.63 | 0.17 | 0.89 |
| 35 | Recurrance | PET | 0.69 | 0.33 | 0.89 |
| 37 | Recurrance | PET | 0.69 | 0.17 | 0.89 |
| 19 | Recurrance | Frozen | 0.62 | 0.34 | 0.86 |
| 63 | Recurrance | Frozen | 0.68 | 0.31 | 0.86 |
| 35 | Recurrance | Frozen | 0.6 | 0.28 | 0.85 |
| 37 | Recurrance | Frozen | 0.6 | 0.2 | 0.85 |
| 19 | Gleason | Frozen & PET | 0.72 | 0.49 | 0.86 |
| 63 | Gleason | Frozen & PET | 0.73 | 0.39 | 0.86 |
| 35 | Gleason | Frozen & PET | 0.62 | 0.21 | 0.86 |
| 37 | Gleason | Frozen & PET | 0.76 | 0.48 | 0.86 |
| 19 | Gleason | PET | 0.72 | 0.36 | 0.89 |

(continued)

| SEQ ID NO | Classification | Tissue Type | AUC | Sensitivity | Specificity |
|---|---|---|---|---|---|
| 63 | Gleason | PET | 0.7 | 0.38 | 0.86 |
| 35 | Gleason | PET | 0.56 | 0.12 | 0.86 |
| 37 | Gleason | PET | 0.77 | 0.5 | 0.86 |
| 19 | Gleason | Frozen | 0.74 | 0.56 | 0.87 |
| 63 | Gleason | Frozen | 0.76 | 0.58 | 0.86 |
| 35 | Gleason | Frozen | 0.65 | 0.28 | 0.87 |
| 37 | Gleason | Frozen | 0.77 | 0.47 | 0.87 |

## EXAMPLES

Investigation Overview

The objective of the present investigation is to develop genetic markers that can identify prostate cancer patients that have aggressive tumors with metastatic potential. It was decided to use methylation analysis to identify differentially expressed genomic markers.

The investigation started with a genome-wide screening step to discover novel markers. This approach utilizes molecular biology methods for the determination of differential methylation between predefined groups of patient samples. Differentially methylated sequences identified using said genomic screening methods are herein referred to as Methylation Sequence Tags (also referred to as MeSTs). The genome-wide screening step identifies differentially methylated CpG sites. Additional information concerning the area surrounding the identified CpG site is obtained by BLAST analysis of the sequence found in the screening step (MeST or Methylation Sequence Tag) and mapping to the human genome.

Following identification of candidates by genome-wide screening, MSP MethyLight™ assays were developed for a subset of the promising candidates. These assays were used to analyze methylation in 56 prostatectomy samples with clinical outcome information. MSP MethyLight™ assays are sensitive and quantitative and they rely on CpG co-methylation; therefore, this assay format provides complementary data to DNA array technology.

All of the promising MeST candidates and some additional candidates were then analyzed by methylation oligonucleotide array using the applicant's proprietary chip technology as described in further detail below. This process provides information concerning the preliminary performance of the MeSTs or candidate genes if an appropriate sample set is used. Candidate markers will be selected based on data obtained from the chip data analysis. Selection is mainly based on the AUC of the marker in the discrimination between the desired classes.

To complete the development process, the candidate markers selected for assay development from the chip study were tested in real-time PCR assays for further validation on the target population and on the sample material used for a potential diagnostic or prognostic test (paraffin embedded tissues).

## EXAMPLE 1: MeST Screening

Experimental Design

Pooled genomic DNA from prostate cancer samples was used for genome-wide screening of markers associated with tumor aggressiveness. Two different methods were applied: Methylation Specific-Arbitrarily Primed Polymerase Chain Reaction (MS-APPCR) (Liang *et al*., 1998) and Methylated CpG Island Amplification (MCA) (Toyota *et al*., 1999). These technologies distinguish between methylated and unmethylated CpG sites through the use of methylation sensitive enzymes n general, genomic DNA is cut with a methylation sensitive restriction enzyme. Methylated fragments are preferentially amplified because cleavage at unmethylated sites prevents amplification of the unmethylated targets. Methylated sequence tag (MeST) fragments obtained using these techniques are sequenced and mapped to the human genome using the BLAST utility in the Ensembl database (www.ensembl.org).

The primary definition of tumor aggressiveness for the screening phase was based on PSA recurrence after radical prostatectomy. An aggressive tumor was defined as one that recurred in less than 24 months. A non-aggressive tumor was defined as one that did not recur after at least 48 months of follow up with regular PSA testing. Five samples in each category were pooled, and there were three pools for each category. The median time to PSA recurrence for the patients with aggressive tumors was 5.1 months. The median follow up time for patients without recurrence was 60.3 months. None of these patients received neo-adjuvant or adjuvant therapies before PSA relapse.

We also included four other comparisons with alternative indicators of aggressiveness. Samples with Gleason grades four and five were compared to samples with Gleason grades one, two, and three. Late stage tumors III and IV) were compared to early stage tumors (I and II). Peripheral zone tumors were compared to transition zone tumors. Lastly, normal tissues adjacent to tumors in patients with early PSA recurrence (<2 years) were compared to normal tissues adjacent to tumors in patients with no PSA recurrence (>4 years follow up).

Screening

The MeST screening process usually results in a large number of sequences that represent potential markers. Some of them are redundant or cannot be matched to the genome. The remaining sequences are selected using a scoring procedure assessing:

Appearance using multiple methods
Appearance in multiple pool comparisons of the same type
Location in CpG island
Location in promoter region
Location near or within gene sequence
Association of nearby gene with cancer
Class of gene (transcription factor, growth factor, etc.)
Repetitive element (negative score)

In this scoring scheme, a MeST sequence receives one point for each of the positive criteria (first seven criteria), and receives a score of minus 8 for having repetitive sequence content greater than 50 % (negative score). In the latter case the MeST always has an overall negative score. Tables 2 and 3 summarize the results of the MeST screening experiments.

Using the scoring criteria and literature based investigation of potential gene function, 80 genes were selected for chip amplicon design. MeSTs from the comparisons based on PSA recurrence were prioritized, but many of the MeSTs from the other comparisons were included in the amplicon design list.

**EXAMPLE 2: Real-time PCR Study**

Experimental Design

MSP assays were developed on the Taqman™ 7900 for strong candidates from MeST screening in order to obtain early data on the performance of the MeSTs as markers of prostate cancer aggressiveness.

As used herein the term MSP-MethyLight shall be taken to mean an assay comprising the amplification of a bisulfite treated sequence by means of methylation specific primers and the detection of resultant amplificates by means of MethyLight detection oligonucleotides (also referred to as 'probes').

MSP-MethyLight assays were developed for a number of MeSTs (see Table 12). The assays were tested on artificially methylated DNA and dilutions of methylated DNA in unmethylated DNA to ensure assay performance. All assays were able to amplify as little as 100 picograms of methylated DNA in the presence or absence of 20 to 100 nanograms of unmethylated DNA. Most of the assays were quantitative between 0.1 and 100% methylation.

Of the 36 assays, 21 were methylated in a pool of prostate tumor DNAs. These 21 assays were first tested on 46 samples from the screening process. These 46 samples included 14 prostatectomies from patients who recurred in less than 24 months and 18 prostatectomies from patients who did not recur after at least 48 months. In addition, there were fourteen patients without follow up information; nine were high Gleason (Score 8-10) and 5 were low Gleason (Score 2-6). The data from this experiment were used to choose seven assays for an independent sample set.

The second sample set consisted of 26 frozen radical prostatectomy samples from patients with early PSA recurrence, with a median time to PSA recurrence of 6 months, and 30 samples from patients with no PSA recurrence after at least 48 months (median follow up time was 60 months). The MethyLight assays were used to measure the amount of DNA methylated at each locus by comparing the threshold cycle (Ct) to a standard curve of methylated DNA. A control assay was used to measure the total amount of DNA. All samples were run in triplicate for all assays. The primer and probe sequences are listed in Table 12. The ratio of the methylated DNA to the total amount of DNA was used to indicate the methylation status of each candidate in each sample.

Results

The methylation values for each assay were used to construct ROC curves (Figures 2 to 8) and calculate sensitivity, specificity, and p values. The data are summarized in Table 4. The AUC values for some of the candidates suggest that the methylation of the marker could have prognostic value. Six candidates had an AUC of 0.68 or greater. The strongest candidates, SEQ ID NO: 19 (GPR7) and SEQ ID NO: 35 (genomic region downstream of FOXL2), were significant by a Wilcoxon test after Bonferroni correction. When the specificity is set to 87% for these two assays, the sensitivity is around 50% for each. (SEQ ID NOs correlated to gene names in table 11.)

**EXAMPLE 3: Chip Study**

In the chip study, a gene panel composed of candidate genes and selected MeSTs were analyzed on 329 samples using the applicant's microarray technology.

Sample Set

The sample set included 329 frozen samples obtained from radical prostatectomies. Only samples with an estimated percent tumor of at least 70% were used, and the median estimated percent tumor (by volume) was 90%. Some sample providers achieved high percent tumor either by coring out a section of the frozen prostate known to contain tumor or by dissecting normal tissue away from the tumor. Patients who received neo-adjuvant therapy were not excluded from the study. Clinical information on disease free survival was not used for any patient receiving adjuvant therapy prior to disease recurrence.

Gleason scores were available for almost all prostatectomies. For some samples, the Gleason score of the portion of the tumor provided to the applicant was also available. The sample set consisted of samples that qualified for one of the two extreme Gleason categories (high or low), samples from patients that qualified for the two relapse categories (early or no relapse), or samples that fell into multiple categories (e.g., high Gleason and early recurrence). Within the sample set, there were 135 samples with low Gleason scores (1+2, 2+1, 2+2, 2+3, 3+2, and 3+3). There were 99 samples with high Gleason scores (3+5, 5+3, 4+4, 4+5, 5+4, and 5+5). For some of the samples, clinical follow up information was available. Sixty-five patients experienced PSA recurrence in less than two years, and 88 patients did not recur after at least 4 years follow up.

For control purposes additional samples were included. In order to control the quality and the functionality of oligos, unmethylated (phi-29 DNA) and artificially methylated DNAs (Promega) were used. Additionally, 16 DNA samples from lymphocytes were processed in parallel to the test samples.

Array

The array contained oligos representing 62 different candidates. Fifty-one of the candidates were MeSTs. One MeST, SEQ ID NO:32, was represented by two non-overlapping amplicons, both near exon one of the gene. For all of the MeSTs, the amplicon was designed as close to the MeST sequence as possible in a CpG rich region. An amplicon for the X chromosome gene ELK1 was included for analysis of male and female control lymphocyte samples.

Other analysed genes included CCND2 (Cyclin D2 Padar et al 2003), CD44 (Woodson et al 2004), EDNRB1 (endothelin receptor B; Woodson et al 2004; Nelson et al 1997), GSTP1 (glutathione S-transferase pi; Maruyama et al 2002), RARB (retinoic acid receptor, beta; Singal et al 2004), PTGS2 (prostaglandin-endoperoxide synthase 2; Yegnasubramanian et al 2004), RASSF1 (Ras association domain family 1; Liu et al 2002), ESR2 (estrogen receptor 2; Zhu et al 2004), DRG1 (developmentally regulated GTP binding protein 1; Bandyopadhyay et al 2003), and CDKN2A (p16; Halvorsen et al 2000). DRG1 was represented with two amplicons. In all cases, CpG rich areas near the promoter or exon 1 were targeted. For p16, the CpG rich area encompassing exon 2 was used because higher methylation rates have been noted in the literature (Nguyen et al 2000).

A complete overview of all analyzed genes can be found in Table 11.

Statistical Methods; Analysis of Chip Data

From Raw Hybridization Intensities to Methylation Ratios

The log methylation ratio (log(CG/TG)) at each CpG position is determined according to a standardized preprocessing pipeline that includes the following steps:

- For each spot the median background pixel intensity is subtracted from the median foreground pixel intensity. This gives a good estimate of background corrected hybridization intensities;
- For both CG and TG detection oligonucleotides of each CpG position, the background corrected median of the 4 redundant spot intensities is taken;

- For each chip and each CG/TG oligo pair, the log(CG/TG) ratio is calculated; and
- For each sample, the median of log(CG/TG) intensities over the redundant chip repetitions is taken.

This log ratio has the property that the hybridization noise has approximately constant variance over the full range of possible methylation rates (Huber *et al*., 2002).

Principle Component Analysis

The principle component analysis (PCA) projects measurement vectors (e.g. chip data, methylation profiles on several CpG sites etc.) onto a new coordinate system. The new coordinate axes are referred to as principal components. The first principal component spans the direction of the largest variance of the data. Subsequent components are ordered by decreasing variance and are orthogonal and uncorrelated to each other. Different CpG positions contribute with different weights to the extension of the data cloud along different components. PCA is an unsupervised technique, i.e. it does not take into account any group or label information of the data points (for further details see e.g. Ripley, 1996).

PCA is typically used to project high dimensional data (in our case methylation-array data) onto lower dimensional subspaces in order to visualize or extract features with high variance from the data. In the present report we used 2 dimensional projections for statistical quality control of the data. We investigated the effect or different process parameters on the chip data and excluded that changing process parameters caused large alterations in the measurement values.

A robust version of PCA was used to detect single outlier chips and exclude them from further analysis (Model *et al.,* 2002).

$T^2$ Control Charts

To control the general stability of the chip production process we use methods from the field of multivariate statistical process control (MVSPC). Our major tool is the $T^2$ control chart, which is used to detect significant deviations of the chip process from normal working conditions (Model *et al.,* 2002).

The $T^2$ chart is constructed as follows:

1. Order the chip data with respect to a process parameter (e.g. hybridization date or spotting robot);
2. Define a historic data set, which describes the chip process under normal working conditions (e.g. the first 75 hybridized chips). In the chart, data from the historical data set are indicated by a speciai pioi symbol; and
3. Compute the distance of every new chip to the historic data set. If the distance of several consecutive chips exceeds a given control limit the process has to be regarded as out of control.

Use of $T^2$ charts to monitor the chip production process allows us to efficiently detect and eliminate most systematic error sources.

Hypothesis testing

Our main task is to identify markers that can make a significant contribution to the class prediction of samples. A significant contribution is detected when the null-hypothesis that a prediction model including the marker does not improve classification performance over a model without the marker can be rejected with $p<0.05$. Because we apply this test to a whole set of potential markers, we have to correct the p-values for multiple testing. We do this by applying the conservative Bonferroni correction, which simply multiplies the single marker p-values with the number of potential markers tested. We also give results with the less conservative False Screening Rate (FDR) method (Dudoit et al 2002).

Throughout this report a marker (sometimes also simply refered to as gene or amplicon) is a genomic region of interest (ROI). It usually consists of several CpG positions in the respective area. For testing the null hypothesis that a marker has no predictive power we use the Wilcoxon rank sum tests to compare groups. A significant test result ($p<0.05$) indicates a shift between the distributions of the respective methylation logratios, i.e. $ln(CG/TG)$. The mean of all oligos for each mrker was used to combine CpGs before Wilcoxon statistics were generated. This approach has the advantage that it favors markers showing co-methylation.

A significant p-value for a marker means that the methylation of this ROI has some systematic correlation to the question of interest as given by the two classes. In general a significant p-value using the Wilcoxon rank sum test also implies good classification performance.

Class prediction by ROC analysis

Receiver Operation Characteristic (ROC) analysis was used to estimate how well the CpG ensemble of a selected marker can differentiate between different tissue classes. An ROC curve is a plot of true positive rate (sensitivity) versus false positive rate (1 - specificity) for a marker over all possible test thresholds. The Area Under the Curve (AUC) of an ROC curve gives the probability of properly classifying a random sample and can thus be used to evaluate overall marker performance. The AUC is related to the Wilcoxon test statistic and comparative ranking of markers by AUCs or Wilcoxon p-values is equivalent. The mean of all oligos for each marker was used to combine CpGs before ROC analysis. This approach has the advantage that it favors markers showing co-methylation.

Experimental Performance

DNA Extraction

Samples were received from external collaborators either as frozen tissues or extracted genomic DNA. DNA from tissue samples was isolated at Epigenomics Berlin using the Qiagen DNA Mini Kit.

The DNA quality of all delivered and extracted samples was first assessed by photometrical measurements. Extinctions at 260 nm and 280 nm as well as A260/280 ratios were determined and the resulting concentrations were calculated. For most of the DNAs used, A260/280 ratios between 1.6 and 1.9 were determined indicating sufficient purity. For some samples ratios in the range of 1.2-1.5 were calculated. Nevertheless, these DNAs were processed as well.

After photometrical measurements 200 ng of the genomic DNAs were applied to a 0.8% agarose gel and gel electrophoresis was performed. Figure 8 shows a typical gel image. No or only minor signs of degradation were observed, indicating a good overall quality of the DNAs used.

Bisulfite Treatment and Multiplex PCR

Total genomic DNAs from all selected samples as well as control DNAs were bisulfite treated converting unmethylated cytosines to uracil. Methylated cytosines are conserved. Bisulfite treatment was performed using Epigenomics' dioxane bisulfite treatment process. In order to avoid a joint processing of all samples with the same biological background resulting in a potential process-bias in the data later on, the samples were randomly grouped into processing batches. Batches of 50 samples were randomized for the Gleason score and PSA outcome. Two independent bisulfite reactions were performed per DNA sample. After bisulfitation, 11.25 ng of each sample was used in 8 subsequent multiplex PCR (mPCR) reactions containing 8 primer pairs each.

For monitoring the mPCR results, gel electrophoresis was performed for all PCR products. To find the best composition of eight primer pairs in a mPCR-set, ALF analysis was used, comparing a mixture of single PCR products with different variants of mPCR-sets.

ALF Express Analyses:

For evaluation of the amplified fragments, mPCR products of Promega DNA were analyzed using the ALF Express-technology. The results for those mPCRs were compared to the mixture of single PCR products. Figure 9 illustrates the result for an 8-plex PCR. All 64 fragments (eight 8-plex-PCRs) selected for the study could be amplified in the performed mPCR experiments. In some cases undesired side products were obtained.

Agarose Gel Electrophoresis: As mentioned above two independent bisulfite reactions and PCRs were performed per DNA sample and the PCR products obtained were applied to a 2% agarose gel. In Figure 10 a typical gel image is shown illustrating the mPCR performance for 10 samples. No visible PCR product implies failure of bisulfite treatment or PCR amplification. The PCR was then repeated with twice the amount of DNA (22.5 ng). Bisulfite treated DNAs that failed again were excluded from the study. f we obtained only one hybridization probe (64 pooled PCR products) from a sample, 4 chips were hybridized using this single probe. If the probes from two independent bisulfite treatments of a sample were successfully amplified, both probes were hybridized onto two chips each.

Four (4) out of 331 samples processed (including control samples) could not be amplified, despite several attempts. These samples were not further processed.

Results of Chip Study

Our primary analysis was a comparison of samples with high Gleason scores (8-10) and low Gleason scores (2-6 with no grade 4 or 5 component). These classes are categories A and C in Table 5. For our second comparison, we used a group of samples from patients with early PSA recurrence after surgery (<2 years) and a group with no recurrence (>4 years follow up). These classes are A1, B1, C1, and D1 for the no recurrence group and A2, B2, and C2 for the recurrence group (see Table 5). These two sample sets (Gleason and clinical outcome) overlapped somewhat. Our third comparison analyzed only patients with intermediate Gleason (3+4, 4+3, 2+5, 5+2, 2+4, 4+2), to determine whether methylation of our candidates sequences correlates with early recurrence in these patients. Therefore, only categories B1 and B2 were included.

*Tumor Tissue vs. Lymphocytes*

In order to evaluate the diagnostic value of the chip, sixteen lymphocyte samples were included into the study. Prostate cancer tissues and lymphocytes were compared using Wilcoxon rank sum statistics. A ranked display for the ten best amplificates is given in Figure 12. Whereas the lymphocyte group is somewhat homogeneous, the figure displays larger variability for the prostate cancer samples. Differences between groups are significant at the 0.05 level (after 5% false discovery rate correction) for amplificates of CDRN2A, ELK1, GSTP1, RARB, PTGS2, RASSF1, ESR2, ONECUT2, BTG4, SLC35F2, HOXB5, LIMK1, HIST1H4J, SEQ ID NO: 35, EPAS1, NOTCH1, SEQ ID NO: 55, PTPRN2, Q9NP73, MX1, DOCK10, CCND2, ISL1, SNAPC2, GRN, H2AFY2, WDFY3, FOS, FAT, Q86SP6, SLC38A1, SNRPN, GPRK5, FBN2, ARHGEF18, RHOC, KBTBD6, NR2E1, PSD, DRG1, Q8N365, SEQ ID NO: 44, Q96S01, CD37, CMYA3, SEQ ID NO: 61, Q8NCX8 and ZNF566

Candidate markers for Gleason

High Gleason vs. Low Gleason Comparison

Wilcoxon rank statistics were used to analyze differences in methylation profiles of patients classified as high Gleason (Score 8-10) and low Gleason (Score 2-6, no grade 4 or 5 component). The high Gleason class consists of 98 samples and the low Gleason class consists of 135 samples. Figure 12 shows the results of this analysis.

For 25 amplificates, the Bonferroni corrected p-value of the Wilcoxon test is below 0.05. For a discussion of biological relevance see section below. Figure 13 displays the methylation matrix of the 10 best markers.

The AUC/sensitivity/specificity of the candidate marker amplificates are given in Table 6.

Figure 13 shows the High Gleason vs. Low Gleason methylation matrix of the 10 markers with best AUC. Each column represents one sample; each row one oligonucleotide (1, 2, or 3 CpG sites each). Oligonucleotides are grouped per marker candidate. The indicated markers are ordered from top to bottom with increasing AUC. On the right side of each marker Bonferroni corrected Wilcoxon p-value and AUC are given. Below the AUC sensitivity at a specificity of -0.75 are given enclosed in brackets. Methylation data are centered and normalized to one standard deviation for individual oligonucleotides. The coior represents the relative distance of the oiigonucieotide methylation status from the mean value. Light grey represents hypomethylated CpGs within an oligonucleotide while dark grey indicates hypermethylated CpGs within an oligonucleotide.

Candidate markers for PSA recurrence

Early Recurrence vs. No Recurrence Comparison

We next analyzed differences in methylation profiles of patients classified as early recurrence (PSA relapse in less than 24 months) and no recurrence (no PSA relapse after at least 4 years).

For three (3) amplificates the Bonferroni corrected p-value of the likelihood-ratio (LR) test is below 0.05. For a discussion of biological relevance see below.

The AUC/sensitivity/specificity of the top marker amplificates are given in Table 7.

Figure 15 shows Early Recurrence vs. No recurrence methylation matrix of the 10 markers with best AUC. Each column represents one sample; each row one oligonucleotide (1, 2, or 3 CpG sites each). Oligonucleotides are grouped per marker candidate. The indicated markers are ordered from top to bottom with increasing AUC. On the right side of each marker Bonferroni corrected Wilcoxon p-value and AUC are given. Below the AUC sensitivity at a specificity of ~ 0.75 are given enclosed in brackets. Methylation data are centered and normalized to one standard deviation for individual oligonucleotides. The color represents the relative distance of the oligonucleotide methylation status from the mean value. Light grey represents hypomethylated CpGs within an oligonucleotide while dark grey indicates hypermethylated CpGs within an oligonucleotide.

Candidate markers for PSA recurrence in patients with intermediate Gleason Scores

Early Recurrence vs. No Recurrence Comparison

Finally, we analyzed differences in methylation profiles of intermediate Gleason samples from patients classified as early recurrence (PSA relapse in less than 24 months) and no recurrence (no PSA relapse after at least 4 years). Intermediate Gleason included all patients with scores 2+5, 5+2, 3+4, 4+3, 2+4, 4+2, 1+5, 5+1, and these patients are a subset of the group used in section 6.6.2. The majority were Gleason 3+4 or 4+3. Although no amplificates displayed a Bonferroni corrected p-value below 0.05 several markers showed promising AUCs (see Table 8). It is likely this comparison was underpowered due to the small sample set for this comparison.

For a discussion of biological relevance see below.

Co-methylation revealed by microarray analysis

Due to the design of the current chip study, we were able to determine areas within marker fragments that were co-methylated. In this design, at least two oligo pairs, each containing 1, 2 or 3 CpG sites, were included for each marker fragments analyzed. Details of CpG sites targeted in the array analysis can be found in figures 16 onwards. Evidence of co-methylation is apparent in the ranked matrix figures. In the ranked matrix figures from the microarray analysis each marker fragment is grouped horizontally. Since each marker fragment represents one to three amplicons and a minimum of four and up to thirty individual CpG sites, extensive information concerning the methylation status of the fragment can be determined. Consecutive dark grey boxes within the grouping of a fragment in a vertical direction indicate co-methylation of the oligonucleotides (and CpGs within that oligo). These data will be further analysed for the most discriminatory areas within a fragment and this information will be utilized for real-time PCR assay design.

Results Summary

In the primary analysis, a comparison of high and low Gleason samples, 25 markers met the criteria for statistical significance using very conservative statistical methodology. This comparison relies on Gleason as a surrogate indicator of aggressiveness, but it was used as the primary analysis because Gleason information was available for nearly all tumors. Fewer samples were available for the additional analysis, based on time to PSA relapse, but still two markers reached statistical significance.

Discussion

Biological Aspects

MeST Screening

The MeST Screening process was very successful, yielding over 400 candidates. In the real-time PCR and chip studies, the MeST candidates performed well. In the real-time PCR study, three MeSTs outperformed GSTP1. In the chip study, the top five candidates in the Gleason comparison are all MeSTs. Therefore, the screening process contributed valuable candidate markers for distinguishing aggressive and non-aggressive tumors.

Furthermore, MeSTs from all of the screening comparisons were represented in the list of top scoring candidates. The top candidate marker in the Gleason comparison, GPR7, was discovered in two outcome comparisons, the Gleason comparison, and the comparison based on stage. Another top performer, DOCK10, was discovered in the comparison based on prostatic zone. Of the top markers, only ABHD9 was discovered in the comparison of normal tissue adjacent to tumors from patients in the two outcome categories. We conclude that all of the screening genome-wide screening comparisons yielded important candidate markers.

Candidate evaluation by MethyLight

Many candidate MeSTs were chosen for real-time PCR assay development while samples were being collected for the chip study. The assays were pre-screened on a pooled DNA sample from many prostate tumor samples. This step allowed pre-selecting only those assays that could potentially be informative Over one-third of the assays were ruled out at this step. Next, the remaining assays were tested on the DNA from the screening samples in order to prioritize the assays. Then, on the final set of 56 independent samples, six of the seven prioritized assays performed well.

The success of the real-time PCR experiment suggests that there is significant co-methylation in these markers.

Therefore, real-time PCR assays, which all require some degree of co-methylation, will be suitable candidates for a final assay choice. The real-time PCR experiment relied heavily on quantification of methylation differences: For nearly all of the assays, the difference between the early recurrence group and the non-recurrence group was a quantitative methylation difference. The final assay type will need strong quantitative abilities.

Candidate evaluation by Methylation Array

The chip experiment was highly successful, demonstrating marker potential for many candidate sequences. In the comparison of high and low Gleason samples, 25 amplicons were significantly different. The vast majority of these are hypermethylated in high Gleason samples. The three candidates analyzed by real-time PCR were among the top six markers in this Gleason comparison. Therefore there is consistency between the two methods of measuring methylation.

The comparison based on patient PSA relapse characteristics had lower sample numbers. Despite these low numbers, we were still able to prove that at least three (3) of our candidate markers can significantly distinguish patients experiencing early relapse from patients not experiencing relapse. In general, methylation is higher in patients experiencing early recurrence. In this comparison, the top three candidates in the real-time PCR study were the top three most significant markers in the chip analysis. The AUCs for GPR7, SEQ ID NO: 35 (downstream of FOXL2), and ABHD9 were 0.72, 0.72, and 0.66 respectively in the chip clinical outcome data and 0.76, 0.75, and 0.70 respectively in the real-time PCR clinical outcome data.

Treatment for patients with high and low Gleason is often clear. Anyone with high Gleason will be recommended for aggressive treatment, including definitive treatment (surgery or radiation) and possibly adjuvant therapy. Patients with low Gleason have the option of deferring definitive treatment. While there are still some uncertainties for these patients, the best options are even less clear for patients with intermediate Gleason levels. Furthermore, the majority of patients being diagnosed with prostate cancer today have intermediate Gleason scores of 6 or 7. These are the patients that can be helped the most by a molecular classification test. The amplicons with the highest AUCs in the comparison based on clinical outcome were GPR7 (AUC=0.72) and SEQ ID NO: 35 (AUC=0.72). When this comparison was restricted to patients with intermediate Gleason scores (1+5, 5+1, 2+4, 4+2, 3+4, 4+3, 2+5, 5+2), the AUC for both of these markers was still 0.72 or greater. These results suggest that a methylation-based assay will provide information even for patients with middle range Gleason scores.

Biology of Marker Genes

several interesting markers were identified by the real-time PCR and chip studies. One of the real-time PCR markers is a G protein coupled receptor (GPR7; SEQ ID NO:19), however very little is known about the gene product. A second marker from the real-time study is located in a CpG island in the promoter of the gene Abhydrolase Domain containing 9 (ABHD9; SEQ ID NO:37). The closest gene to SEQ ID NO:35 is FOXL2. The MeST is in a CpG island several kilobases downstream of this gene. SEQ ID NO:63 is in an area with several histone genes.

Additional markers emerged in the chip study. NOTCH1 (SEQ ID NO:41) controls a signalling pathway that regulates interactions between adjacent cells. Many labs have studied the role of this gene in carcinogenesis and metastasis. Little is known about many of the candidates, including DOCK10 (SEQ ID NO:16), SEQ ID NO:51, which is in the promoter of a gene called Kelch repeat and BTB (POZ) domain-containing 6, and SEQ ID NO:17, which is located between an EST and B-cell Translocation Gene 4. BTG4 has been shown to have growth inhibitory properties (Buanne et al 2000).

PTGS2 (SEQ ID NO:9) is the only gene previously shown in the literature to be more methylated in prostate tumors of patients who recurred soon after prostatectomy (Yegnasubramanian et al 2004). PTGS2, also known as cyclo-oxygenase (COX2), is a further promising candidate in both the Gleason and the clinical outcome analyses, with AUCs of 0.69 and 0.65 respectively. GSTP1 is the most highly studied methylation marker in prostate cancer, and while there are no published data directly demonstrating its prognostic value, there is some evidence that its methylation correlates with Gleason grade (Maruyama et al 2002). However, this correlation was not confirmed in another study (Woodson et al 2004). In the instant data, GSTP1 methylation significantly correlates with Gleason grade, but the AUC in the clinical outcome comparison is only 0.58.

Medical Aspects

The methylation candidates that have emerged from our Gleason comparison are informative prognostic markers. We have shown that some of these candidates that correlate with Gleason categories can also predict PSA relapse, even in patients with intermediate Gleason scores. Therefore it is likely that our analysis based on Gleason will provide markers that provide additional information to Gleason.

As individual markers, the chip candidates reach 40-60% sensitivity when the specificity is set at 75%. In the real-time study, the sensitivity of three of the markers was higher, reaching 50-60% at a specificity of 85%. The enhanced performance in the real-time might be due to the quantitative abilities of MSP-MethyLight. Approximately 20% of patients experience relapses within 5-10 years after surgery. If this were set as the prevalence of aggressive tumors in the radical prostatectomy population, then a marker such as ours with 50% sensitivity and 85% specificity would have a negative predictive value of 0.87 and a positive predictive value of 0.45. Therefore, a marker with this performance would define a group of patients with only a 13% chance of recurrence after surgery and a group of patients with a 45% chance of recurrence. The first group could just be monitored for PSA rise, and the second group would be candidates for adjuvant therapies.

While these candidates have been studied in prostatectomy samples, they will also be useful for analysis of biopsies. A marker that predicts outcome after prostatectomy correlates with the aggressiveness and metastatic potential of the tumor, and these properties will also be present in the biopsy. After biopsy and staging tests, patients opt for watchful waiting, definitive curative therapy, or a combination of treatments (such as surgery plus radiation or androgen ablation). A molecular test with high negative predictive value would allow more patients to choose watchful waiting. A molecular test with sufficiently high positive predictive value would select a subset of patients who should not receive radiation or surgery only. Thus, these candidate methylation markers have the potential to reduce both under and over treatment of prostate cancer.

**EXAMPLE 4: Real time quantitative methylation analysis**

Genomic DNA was analyzed using the Real Time PCR technique after bisulfite conversion.

The QM assay (= Quantitative Methylation Assay) is a Real-time PCR based method for quantitative DNA methylation detection. The assay principle is based on non-methylation specific amplification of the target region and a methylation specific detection by competitive hybridization of two different probes specific for the CG or the TG status, respectively. For the present study, TaqMan probes were used that were labeled with two different fluorescence dyes ("FAM" for CG specific probes, "VIC" for TG specific probes) and were further modified by a quencher molecule ("TAMRA" or "Minor Groove Binder/non-fluorescent quencher").

Evaluation of the QM assay raw data is possible with two different methods:

1. Measuring absolute fluorescence intensities (FI) in the logarithmic phase of amplification Difference in threshold cycles (Ct) of CG and TG specific probe.

In the following series of quantitative methylation assays the amount of sample DNA amplified is quantified by reference to the gene GSTP1 to normalize for input DNA. For standardization, the primers and the probe for analysis of the GSTP1 gene lack CpG dinucleotides so that amplification is possible regardless of methylation levels. As there are no methylation variable positions, only one probe oligonucleotide is required.

The reactions are calibrated by reference to DNA standards of known methylation levels in order to quantify the levels of methylation within the sample. The DNA standards were composed of bisulfite treated phi29 amplified genomic DNA (i.e. unmethlyated), and/or phi29 amplified genomic DNA treated with Sss1 methylase enzyme (thereby methylating each CpG position in the sample), which is then treated with bisulfite solution. Seven different reference standards were used with 0%, (i.e. phi29 amplified genomic DNA only), 5%, 10%, 25%, 50%, 75% and 100% (i.e. phi29 Sss1 treated genomic only).

<u>Bisulfite treatment</u>

Bisulfite treatment was carried out based on the method disclosed by Olek et al. Nucleic Acids Res. 1996 Dec 15;24(24):5064-6, and optimized to the applicant's laboratory workflow.

<u>Quantification Standards</u>

The reactions are calibrated by reference to DNA standards of known methylation levels in order to quantify the levels of methylation within the sample. The DNA standards were composed of bisulfite treated phi29 amplified human genomic DNA (Promega) (i.e. unmethlyated), and/or phi29 amplified genomic DNA treated with Sss1 Methylase enzyme (thereby methylating each CpG position in the sample), which is then treated with bisulfite solution. Seven different reference standards were used with 0%, (i.e. phi29 amplified genomic DNA only), 5%, 10%, 25%, 50%, 75% and 100% (i.e. phi29 Sss1 treated genomic only). 2000 ng batches of human genomic DNA (Promega) were treated with bisulfite. To generate methylated MDA DNA, 13 tubes of 4.5 μg MDA-DNA (700 ng/μl) was treated with Sss1.

Control assay

The GSTP1-C3 assay design makes it suitable for quantitating DNAs from different sources, including fresh/frozen samples, remote samples such as plasma or serum, and DNA obtained from archival specimen such as paraffin embedded material. The following oligonucleotides were used in the reaction to amplify the control amplificate:

Control Primer1: GGAGTGGAGGAAATTGAGAT (SEQ ID NO:966)
Control Primer2: CCACACAACAAATACTCAAAAC (SEQ ID NO:967)
Control Probe: FAM-TGGGTGTTTGTAATTTTTGTTTTGTGTTAGGTT-TAMRA (SEQ ID NO:968)
Cycle program (40 cycles):

95˚C, 10 min
95˚C, 15 sec
58˚C, 1 min

Assay design and reaction conditions

Two assays were developed for the analysis of the gene PITX2(SEQ ID NO:961)

Assay 1:

Primers: GTAGGGGAGGGAAGTAGATGTT (SEQ ID NO:969)
TTCTAATCCTCCTTTCCACAATAA (SEQ ID NO:970)
Probes: FAM-AGTCGGAGTCGGGAGAGCGA-TAMRA (SEQ ID NO:971)
VIC-AGTTGGAGTTGGGAGAGTGAAAGGAGA -TAMRA (SEQ ID NO:972)
Amplicon (SEQ ID NO:973):

GtAGGGGAGGGAAGtAGATGttAGCGGGtCGAAGAGTCGGGAGtCGGAGtCGGGAGAGCGAAAGGAG
AGGGGAttTGGCGGGGtAtTTAGGAGttAAtCGAGGAGtAGGAGtACGGAtTtttAtTGTGGAAAGGAGGAttA
GAA

Length of fragment: 143 bp

Positions of primers, probes and CpG dinucleotides ar highlighted.

PCR components (supplied by Eurogentec) : 3 mM MgCl2 buffer, 10x buffer, Hotstart TAQ, 200 μM dNTP, 625 nM each primer, 200 nM each probe

Cycle program (45 cycles):

95˚C, 10 min
95˚C, 15 sec
62˚C, 1 min

Assay 2 :

Primers: AACATCTACTTCCCTCCCCTAC (SEQ ID NO:974)
GTTAGTAGAGATTTTATTAAATTTTATTGTAT (SEQ ID NO:975)
Probes: FAM-TTCGGTTGCGCGGT-MGBNQF (SEQ ID NO:976)
VIC-TTTGGTTGTGTGGTTG- MGBNQF (SEQ ID NO:977)
Amplicon (SEQ ID NO:978):

GTtAGtAGAGATTttAttAAAtTttAtTGtAtAGTGGCGCGGGCGGtCGGtCGAGtCGGtTGCGCGtTGGC GATttAGGAGCGAGtAtAGCGttCGGGCGAGCGtCGGGGGGAGCGAGtAGGGGCGACGAGAAACGAGG tAGGGGAGGGAAGtAGATGtt

Length of fragment: 164 bp

The positions of probes, primers and CpG positions are highlighted.

The probes cover three co-methylated CpG positions.

PCR components (supplied by Eurogentec): 2,5 mM MgCl2 buffer, 10x buffer, Hotstart TAQ, 200 μM dNTP, 625 nM each primer, 200 nM each probe

Program (45 cycles):

95˚C, 10 min
95˚C, 15 sec
60˚C, 1 min

The extent of methylation at a specific locus was determined by the following formulas:

Using absolute fluorescence intensity: methylation rate= 100 * I(CG) / (I(CG) + I(TG))

(I = Intensity of the fluorescence of CG-probe or TG-probe)

Using threshold cycle Ct: methylation rate= 100*CG/(CG+TG)= 100/(1+TG/CG)= 100/(1+2^delta(ct))

(assuming PCR efficiency E=2; delta (Ct)= Ct (methylated) - Ct (unmethylated))

**Example 5: Validation**

The main goal of this phase of the investigation was to confirm the significance of previously identified marker candidates and optimize methylation cut-offs. The markers should be suitable to split patients who undergo prostatectomy into two groups: one with a high chance of PSA recurrence and one with a low chance of PSA recurrence. In addition, the markers should provide additional information to Gleason grade analysis. Markers meeting these criteria will have an important clinical role in selection of prostatectomy patients for adjuvant therapy.

The applicant had previously identified several markers with significantly higher methylation levels in patients who experienced PSA recurrence within 24 months of surgery compared to patients who did not experience PSA recurrence (see above Examples 1 to 4). Six of these markers were transferred to a real-time platform (QM Assay). These assays were used to analyze the methylation levels of 612 paraffin embedded prostatectomy samples from a cohort of node-negative patients from three institutions.

The primary aim of the invention was to provide markers that can differentiate between patients with low chance for PSA recurrence after surgery and those with a high chance for PSA recurrence. The performance of these markers as compared to traditional prognostic indicators such as Gleason grading and stage information is also provided.

It is a further aim of the present invention to determine where the markers are most informative in relation to current clinical prognostic assessment and accordingly provide particularly preferred use embodiments of the present invention. It is particularly preferred that a molecular test according to the present invention is combined, either formally or informally, with information from other prognostic sources, in particular Gleason grading.

Methods: QM Assay Description

Each QM-assay was developed to enhance performance without drastically altering standard conditions in order to allow future multiplexing. Primer and probe concentrations, $MgCl_2$ concentration and annealing temperature were optimized under fixed buffer and polymerase conditions. The assays were designed and optimized to ensure quantitative methylation analysis of each marker between 10 and 100 percent methylation. The assay products were checked on an agarose gel and no undesired products were detected. The results of the optimization procedure are shown in the following tables.

Sample Set

Paraffin-embedded prostatectomy tissue samples from 605 patients were analyzed. The samples were provided by the Baylor College of Medicine SPORE, Stanford University Department of Urology, and Virginia Mason Hospital in Seattle. The samples from Stanford and Virginia Mason were prepared by first finding the surgical block with the highest percent tumor, then sectioning the block. Three tubes were prepared, each with three 10 micron thick sections. The procedure was slightly different at Baylor. A core of tissue was removed from the tumor within the prostatectomy block, and then this core was cut into 10 micron sections. Ten sections were included into each of three tubes.

An adjacent section was mounted on a slide and H&E stained for histological analysis. A pathologist reviewed these slides for an independent determination of Gleason grading and percent tumor. The Gleason results were used for all analyses in this report. The original provider Gleason values are available, but they were not used for analysis due to known and hypothetical biases among the providers. Stanford, for instance, uses a percentage Gleason 4/5 for reporting grade, while the other two providers use the traditional system. The measured Gleason values provided an independent and uniform measurement.

A few samples were found to have no tumor cells on the H&E slide, and these patients were omitted from the analysis. In addition, we found a few patients that did not have a PSA nadir after surgery. These patients were also excluded from the study. In total, 612 patients were included in the data analysis.

Due to their coring technique, the percent tumor of the samples provided by Baylor were higher than the other providers.

All patients, aged 40-80, undergoing surgery at the three institutions during certain years were included in the study, with the exception of patients who received neo-adjuvant or adjuvant therapy (before PSA rise) and patients with positive nodes at the time of surgery. For Baylor, the time period was 1993-1998, for Virginia Mason it was 1996-2000, and for Stanford it was 1996-1999.

The overall cohort is similar to other prostatectomy cohorts described in the literature, such as the cohort collected by William Catalona and described in 2004 (Roehl *et al*.). The patient cohorts from each provider are similar for nearly all clinical parameters. One exception is the type of recurrence. While other institutions typically wait until the patient's PSA rises to 0.2 ng/ml or higher after surgery, the Stanford Department of Urology treats many patients when their PSA rises to 0.05. Therefore, Stanford has a higher rate of recurrence based on the decision to treat criteria and a lower rate of recurrence based on the PSA level (0.2ng/ml) criteria. See section 6.1 for a summary of the event definition criteria. Figure 89 provides a histogram of follow-up times for the patient cohort (all three providers included). The white bars consist of the patients who did not have a recurrence before they were censored, and the shaded bars consist of the patients who experienced recurrence. By selecting patients who received surgery from 1993-2000, we have ensured that the median follow-up time of the cohort (66 months) is long enough to have a significant number of patients who have relapsed.

For deparaffination, the 627 provided PET samples were processed directly in the tube in which they were delivered by the providers. One ml (Virginia Mason and Baylor) or 1.8 ml (Stanford) of limonene was added to each tube and incubated at room temperature for 10 minutes in a thermomixer with occasional vortexing. The samples were centrifuged at 16,000 x g for 5 minutes. The limonene supernatant was removed, and if no pellet was detected, centrifugation was repeated at higher speed and the remaining limonene was removed. For samples from Stanford, the deparaffination process was repeated once with 1.6 ml of limonene to get rid of residual paraffin.

For lysis of the tissue, 190 μl lysis buffer and 20 μl proteinase K was added to each deparaffinated sample. For Stanford samples, 570 μl lysis buffer and 60 μl proteinase K was used. After vortexing, samples were centrifuged briefly and incubated on a thermoshaker at 60˚C for 40 hours. After the incubation, samples were checked to ensure that lysis was complete, and the proteinase was then inactivated at 95˚C for 10 minutes. If the lysed samples were not directly used for DNA extraction, they were stored at -20˚C.

The lysates were randomized based on the sample provider and PSA recurrence. The DNA was isolated using a QIAGEN DNeasy Tissue kit with a few modifications. 400 μl buffer AUE was distributed to collection tubes and 200 μl of lysate were added. The samples were mixed by shaking for 15 seconds. The lysate/buffer mixtures were applied to the 96-well DNeasy plate columns. The plate was sealed and centrifuged at 5790xg for 10 minutes. The columns were washed once with 500 μl of AW1 and then 500 μl AW2. The DNA was eluted with 120 μl buffer AE. Therefore, the final volume of extracted DNA was approximately 120μl. The DNA was stored at -20˚C.

Bisulfite Treatment

The CFF real-time PCR assay was used to quantify the DNA concentration of the samples after extraction.

CFF sequence:

TAAGAGTAATAATGGATGGATGATGGATAGATGAATGGATGAAGAAAGAAAGGATGAGTGAGAGAA AGGAAGGGAGATGGGAGG (84bp) (SEQ ID NO: 979)
CFF-Forward primer TAAGAGTAATAATGGATGGATGATG (SEQ ID NO: 980)
CFF-Reverse primer CCTCCCATCTCCCTTCC (SEQ ID NO: 981)
CFF TaqMan probe ATGGATGAAGAAAGAAAGGATGAGT (SEQ ID NO: 982)

We adjusted the concentration of each genomic DNA sample so that 1ug of CFF1 measured DNA was present in 44 μl. The bisulfite treatment of genomic DNA derived from paraffin embedded tissue was performed using a 96 well protocol. Forty-four μl genomic DNA (with approximately 1μg of amplifiable DNA), 83 μl 4.9M bisulfite solution (pH 5.45-5.5), and 13 μL DME solution were pipetted into the wells of the plate. The samples were thoroughly mixed then placed in a thermocycler with the following program:

- 5:00 min denaturation of DNA at 99°C
- 22:00 min incubation at 60°C
- 3:00 min denaturation of DNA at 99°C
- 1:27:00 hours incubation at 60°C
- 3:00 min denaturation of DNA at 99°C
- 2:57:00 hours incubation at 60°C
- Cooling at 20°C

After the incubations, each sample was divided into two 70 μL aliquots. Each aliquot was combined with 280 μL of prepared Buffer AVL/Carrier RNA and 280 μL ethanol. The wells were sealed and the samples were mixed vigorously for 15 seconds. The plate was incubated for 10 minutes at room temperature. The first aliquot was applied to the QIAamp 96 plate and the plate was centrifuged for four minutes at 5790 x g. The process was repeated with the second aliquot so that both aliquots were applied to the same binding column. The columns were washed with 500 μL buffer AW1, then 500 μL 0.2 M NaOH, and then twice with 500 μL buffer AW2. The UNA was eluted with 100 μL elution buffer (Qiagen) pre-heated to 70 deg C. The bisDNAs were stored at -20°C.
The bisulfite treated DNA samples were stored in 8 x 96 well plates (plate 01-08). The samples and controls were combined onto two 384-well PCR reaction plates for each QM assay. Each QM assay plate contained the samples of 4 x 96 well plates (85 wells actually used per plate) and 1x96 well plate with standard DNA (7 mixtures of the calibration DNA and water for the no template control PCR reaction). The QM assay plates were run three times.
The 384-well PCR plates were pipetted with the TECAN workstation. The pipetting program transferred first 10 μl of the mastermix and then 10 μl of the respective DNA into the designated well. The master mix was pipetted in a falcon tube and distributed to 8 x 500 μl screw cap vials for automatic pipetting with TECAN workstation.
All QM assays were run on an ABI TAQMAN 7900HT real-time device (SDS 2.2. software) with a reaction volume of 20 μl. PITX2 and CCND2 assays were run with 9600 emulation, and the other assays were not. An automatic sample setup was used to transfer the correct sample names and detector/reporter dyes to the TAQMAN software. The cycling conditions were manually adjusted and ROX was used as passive reference dye. All 384 well PCR plates we analyzed with the SDS2.2 software using the manual analysis settings (baseline setting with start and stop values and manual threshold) to produce results files for each run individually.

Methods: Evaluation of Marker Performance

Definition of Events

After a successful prostatectomy on a patient with non-metastatic disease, there should be no prostate cells left in his body and therefore his PSA levels should drop to zero. A patient's PSA levels are typically measured every 6-12 months after surgery to ensure that the patient remains free of prostate cancer. If PSA becomes detectable and rises to a certain level, the doctor and patient may decide on additional therapy. Therefore, the return and rise of PSA levels are the primary indication of disease recurrence.
A post-surgical PSA relapse is typically indicated by either a gradual or rapid rise in levels over a series of sequential tests. Depending on the clinical characteristics of the patient or the approach of the institution, patients may be treated as soon as PSA is detected, when it reaches a certain threshold, or when clinical symptoms accompany the PSA rise. Most institutions consider a PSA level of 0.2 ng/ml to be significant, and if a patient's PSA reaches this level and is confirmed to be rising in subsequent tests, he will be offered additional therapy. Stanford Department of Urology, one of the sample providers, considers 0.05 ng/ml to be a PSA recurrence, and considers treatment for patients when their PSA reaches this level.

An event in this study includes all PSA-based recurrences. A PSA level of 0.2 ng/ml, confirmed in subsequent tests, has been demonstrated to provide the best sensitivity and specificity for detection of recurrence (Freedland *et al.* 2003). Rise of PSA to this level normally precedes any development of clinical recurrence; therefore, nearly all of the patients in this study are free of clinical recurrence at the time of PSA recurrence. Because Stanford often treats patients with PSA recurrence before they reach this cut-off of 0.2ng/ml, many of their recurrence patients would be censored in the present study if the PSA level of 0.2ng/ml was the only considered event. Therefore, patients from any of the three institutions who receive therapy due to PSA levels are also considered an event in this study.

To summarize, an event is defined in the present study as any rise in PSA to 0.2 ng/ml (confirmed in subsequent test) OR a decision to treat the patient based on PSA criteria.

Raw QM Data Processing

All analyses in this report are based on the CT evaluation. Assuming optimal real-time PCR conditions in the exponential amplification phase, the concentration of methylated DNA ($C_{meth}$) can be determined by

$$C_{meth} = \frac{100}{1+2^{(CT_{CG}-CT_{TG})}}[\%],$$

where

$CT_{CG}$ denotes the threshold cycle of the CG reporter (FAM channel) and

$CT_{TG}$ denotes the threshold cycle of the TG reporter (VIC channel).

The thresholds for the cycles were determined by visual inspection of the amplification plots (ABI PRISM 7900 HT Sequence Detection System User Guide). The values for the cycles ($CT_{CG}$ and $CT_{TG}$) were calculated with these thresholds by the ABI 7900 software. Whenever the amplification curve did not exceed the threshold, the value of the cycle was set to the maximum cycle e.g. 50.

The R software package, version 2.2. (Gentleman and Ihaka 1997), was used for the statistical analysis.

In addition, we used the "survival" package, version 2.11-5 (http://cran.at.r-project.org/src/contrib/Descriptions/survival.html), for survival analysis.

Proprietary code was used for k-fold-cross validation, ROC analysis and plot functions.

Each dataset is represented in a proprietary data object, called "Annotated Data Matrix" (ADM). This data object contains the measurements after quality control and averaging, as well as all necessary annotations for the samples and assays.

QM Assay calibration curves

A series of mixtures of methylated MDA-DNA and unmethylated MDA-DNA, ranging from 0 to 100 percent methylated, were included in triplicate on each QM PCR plate. These DNAs were used to ensure uniform QM assay performance on all PCR plates. All assays showed strong quantitative abilities between 10 and 100%, and some assays were able to consistently distinguish 5% methylated DNA from unmethylated DNA.

Statistical Methods

After quality control, each assay was statistically analyzed.

Cox Regression

The relation between recurrence-free survival times (RFS) and covariates were analyzed using Cox Proportional Hazard models (Cox and Oates 1984; Harrel 2001).

The hazard, i.e. the instantaneous risk of a relapse, is modeled as

$$h(t \mid x) = h_0\,(t) \cdot exp(\beta x) \qquad (3)$$

and

$$h(t \mid x_1,\ldots,x_k) = h_0\,(t) \cdot exp(\beta_1 x_1 + \ldots + \beta_k x_k) \qquad (4)$$

for univariate and multiple regression analyses, respectively, where *k* is 10, *m* is 100*t* is the time measured in months after surgery, $h_0(t)$ is the (unspecified) baseline hazard, $x_i$ are the covariates (e.g. measurements of the assays) and $\beta_i$ are the regression coefficients (parameters of the model). $\beta_i$ will be estimated by maximizing the partial likelihood of the Cox Proportional Hazard model Likelihood ratio tests are performed to test whether methylation is related to the hazard. The difference between 2Log(Likelihood) of the full model and the null-model is approximately $\chi^2$-distributed with k degrees of freedom under the nuii hypotheses $\beta_1 = ... = \beta_k = 0$.

The assumption of proportional hazards wre evaluated by scaled Schoenfeld residuals (Themau and Grambsch 2000). For the calculation, analysis and diagnostics of the Cox Proportional Hazard Model the R functions "coxph" and "coxph.zph" of the "survival" package are used.

Stepwise Regression Analysis

For multiple Cox regression models a stepwise procedure (Venables and Ripley 1999; Harrel 2001) was used in order to find sub-models including only relevant variables. Two effects are usually achieved by these procedures:

- Variables (methylation rates) that are basically unrelated to the dependent variable (DFS/MFS) are excluded as they do not add relevant information to the model.
- Out of a set of highly correlated variables, only the one with the best relation to the dependent variable is retained.

Inclusion of both types of variables can lead to numerical instabilities and a loss of power. Moreover, the predictor's performance can be low due to over-fitting.

The applied algorithm aims at minimizing the Akaike information criterion (AIC).

The AIC is related to the performance of a model, smaller values promise better performance. Whereas the inclusion of additional variables always improves the model fit and thus increases the likelihood, the second term penalizes the estimation of additional parameters. The best model will present a compromise model with good fit and usually a small or moderate number of variables. Stepwise regression calculation with AIC are done with the R function "step".

Kaplan-Meier Survival Curves and Log-Rank Tests

Survival curves were estimated from RFS data using Kaplan-Meier estimator for survival (Kaplan and Meier, 1958). Log-rank tests (Cox and Oates 1984) are used to test for differences of two survival curves, e.g. survival in hyper- vs. hypomethylated groups. In addition, a variant of the Log-rank test usually referred to as the Generalized Wilcoxon test was applied (for description see Hosmer and Lemeshow 1999). For the Kaplan-Meier analysis the functions "survfit" and "survdiff" of the "survival" package are used.

Independence of single markers and marker panels from other covariates

To check whether the present markers give additional and independent information, other relevant clinical factors were included in the Cox Proportional Hazard model and the p-values for the weights for every factor were calculated (Wald-Test) (Thernau *et al.* 2000). For the analysis of additional factors in the Cox Proportional Hazard model, the R function "coxph" is used.

Multiple Test Corrections

No correction for multiple testing was done.

Density Estimation

For numerical variables, kernel density estimation was performed with a Gaussian kernel and variable bandwidth. The bandwidth is determined using Silverman's "rule-of-thumb" (Silverman 1986). For the calculation of the densities the R function "density" is used.

Analysis of Sensitivity and Specificity

The method of calculating sensitivity and specificity using the Bayes-formula was based on the Kaplan-Meier estimates (Heagerty *et al.* 2000) for the survival probabilities in the marker positive and marker negative groups for a given time $T_{Threshold}$. The ROCs were calculated for different reference times $T_{Threshold}$ (3 year, 4 years, 5 years, 6 years).

k-fold Crossvalidation

For the analysis of model selection and model robustness k-fold crossvalidation (Hastie *et al.* 2001) was used. The set of observations is randomly split into k chunks. In turn, every chunk was used as a test set, whereas the remaining k-1 chunks constitute the training set. This procedure is repeated m times.

<u>Results</u>

The 605 samples were processed as described above. All samples were analyzed with six marker QM assays with three replicates. The data were filtered for quality control, and analyzed as described in the methods section. The clinical performance of each marker is summarized below and the Kaplan-Meier survival curves and ROC curves according to figures 90 to 95. P-values for comparison of survival curves reported in the graphs are based on the ordinary Log-rank test. The results of using the Generalized Wilcoxon test are essentially the same (data not shown).

The performance of the markers was first examined using the median methylation level as a cut-off. Since this cut-off was fixed before looking at the data, the p values can be used to judge the performance of the markers. Any marker with a significant p value using the median methylation as a cut-off is considered to be validated. The median methylation level might not be the best cut-off for all markers, and for these markers the prognostic separation can be further optimized by choosing the methylation cut-off that results in the lowest p value. Since the cut-off is optimized specifically for p value, the p value no longer can be used to indicate statistical significance.

For judging the significance of the marker performance using the median methylation as a cut-off, we used a p value of 0.005 (assuming correction for 6 comparisons). Based on p-value (less than 0.008) and event separation, PITX2 is the strongest candidate. GPR7along with SEQ ID NO:35.

Therefore, these two markers are considered validated markers of post-surgical prostate cancer prognosis., SEQ ID NO: 63 was not significant using the median methylation level as a cut-off (p values 0.018 and 0.0059), but perform well when the methylation cut-off is optimized. See Table 17 for results.

Figure 90A shows the Kaplan-Meier survival analysis of the PITX2 marker of the 585 patient samples that passed the quality control filter using the optimized methylation cut-off value (13.5%). Figure 90B shows the Kaplan-Meier survival analysis of the PITX2 marker using the predefined median methylation value as a cut-off, the p-value was 0.000017. Figure 90C shows the ROC curve analysis of the PITX2 marker after 5 years of follow-up. The median methylation cut-off is marked as a triangle, and the optimized methylation cut-off is shown as a diamond. The AUC was 0.64.

Figure 91A shows the Kaplan-Meler survival analysis of the GrFC7 marker of the 596 patient samples that passed the quality control filter using the optimized methylation cut-off value (18.06%). Figure 91B shows the Kaplan-Meier survival analysis of said marker using the predefined median methylation value as a cut-off, the p-value was 0.0016. Figure 91C shows the ROC curve analysis of said marker after 5 years of follow-up. The median methylation cut-off is marked as a triangle, and the optimized methylation cut-off is shown as a diamond. The AUC was 0.64.

Figure 92A shows the Kaplan-Meier survival analysis of the SEQ ID NO:63 marker of the 599 patient samples that passed the quality control filter using the optimized methylation cut-off value (5.79%). Figure 92B shows the Kaplan-Meier survival analysis of said marker using the predefined median methylation value as a cut-off, the p-value was 0.018. Figure 92C shows the ROC curve analysis of said marker after 5 years of follow-up. The median methylation cut-off is marked as a triangle, and the optimized methylation cut-off is shown as a diamond. The AUC was 0.60.

Figure 93A shows the Kaplan-Meier survival analysis of the SEQ ID NO:35 marker of the 598 patient samples that passed the quality control filter using the optimized methylation cut-off value (36.77%). Figure 93B shows the Kaplan-Meier survival analysis of said marker using the predefined median methylation value as a cut-off, the p-value was 0.059. Figure 93C shows the ROC curve analysis of said marker after 5 years of follow-up. The median methylation cut-off is marked as a triangle, and the optimized methylation cut-off is shown as a diamond. The AUC was 0.61.

Figure 94A shows the Kaplan-Meier survival analysis of the ABHD9 marker of the 592 patient samples that passed the quality control filter using the optimized methylation cut-off value (28.41%). Figure 94B shows the Kaplan-Meier survival analysis of said marker using the predefined median methylation value as a cut-off, the p-value was 0.018. Figure 94C shows the ROC curve analysis of said marker after 5 years of follow-up. The median methylation cut-off is marked as a triangle, and the optimized methylation cut-off is shown as a diamond. The AUC was 0.58.

Figure 95A shows the Kaplan-Meier survival analysis of the CCND2 marker of the 604 patient samples that passed the quality control filter using the optimized methylation cut-off value (2.22%). Figure 95B shows the Kaplan-Meier survival analysis of said marker using the predefined median methylation value as a cut-off, the p-value was 0.22. Figure 95C shows the ROC curve analysis of said marker after 5 years of follow-up. The median methylation cut-off is marked as a triangle, and the optimized methylation cut-off is shown as a diamond. The AUC was 0.61.

Evaluation of Markers on Clinical Subsets of the Patients

Several clinical prognostic factors are commonly used for assessing prostate cancer. Histological analysis of the tumor with quantification of the tumor differentiation state using the Gleason grading system is a particularly important prognostic indicator in current clinical practice. The analysis was continued by determining whether the markers could improve Gleason analysis by subdividing patients within a Gleason category. We also investigated whether the markers could add information to other prognostic indicators, such as nomogram risk estimation (Han *et al.* 2003) and disease stage.

For these analyses, we used Kaplan-Meier analysis to determine whether our markers are still informative on population sub-groups, and Cox regression analysis to determine whether the markers provide information independent of the prognostic clinical variables. Gleason score (using Charite Gleason calls) was divided into three groups (6 or lower, 7, and 8 through 10), stage was divided into two groups (T2/organ-confined and T3/non-organ confined), PSA was divided into four groups (0 to 4 ng/ml, 4 to 10 ng/ml, 10 to 20 ng/ml, and greater than 20 ng/ml), and nomogram estimation of 5 year PSA-free survival was divided into two groups (90 to 100% and 0 to 89%).

PITX2

With Cox regression modeling, PITX2 is a valuable prognostic marker independent of other clinical prognostic information (Table 18). In other words, PITX2 methylation adds more information to Gleason than either PSA or disease stage. The hazard ratio for PITX2 is 2.2. In the survival analysis of sub-groups, PITX2 has the potential to be a significant marker for all prostate cancer patients.

It is particularly interesting to see strong separation within the patient sub-group with organ-confined disease (Figure 96). Patients with organ-confined disease (T2) should be cured by surgery. Those that are not cured by surgery must have had some cells leave the prostate before surgery, and therefore had tumor cells with aggressive characteristics early in the development. PITX2 can separate the T2 group into a hypomethylated group with a very small chance for recurrence (~5%) and a hyper-methylated group with a prognosis more like T3 patients.

Figure 96 shows the survival analysis of PITX2 performance on sub-populations based on stage. The upper left plot shows the performance of disease stage as a prognostic marker. The upper right plot shows the performance of PITX2 on pT2 patients. The lower left plot shows the performance of PITX2 on pT3 patients.

PITX2 is also capable of stratifying patients within Gleason sub-categories. Figure 97 shows that survival analysis on low Gleason patients (Score 5 or 6) and high Gleason patients (Score 8, 9, or 10) results in low p values. Patients with high Gleason scores are currently candidates for clinical trials on post-surgical adjuvant therapies. But the PITX2 values suggest that this is not a uniform group. PITX2 hypomethylated, high Gleason patients have 85% probability of disease free survival at ten years, while hypermethylated high Gleason patients have a very low chance (-35%). These patients with high likelihood for disease recurrence are the patients who should be selected for adjuvant therapy or clinical trials.

Figure 97 shows the survival analysis of PITX2 performance on sub-populations based on Gleason score categories. The upper left plot shows the performance of Gleason score as a prognostic marker. Gleason 5 and 6 patients are marked A, Gleason 7 patients are marked B, and Gleason 8, 9, and 10 patients are marked C. The upper right plot shows the performance of PITX2 on Gleason 5 and 6 patients. The lower left plot shows the performance of PITX2 on Gleason 7 patients. The lower right plot shows the performance of PITX2 on Gleason 8, 9, and 10 patients.

Prostate cancer nomograms are created based on large cohorts of patients. They mathematically combine information from stage, Gleason, and pre-operative PSA levels into one prognostic indicator. As

Figure 98 shows, the nomogram by itself is very strong. But PITX2 is capable of further sub-dividing the patients.

Figure 98 shows the survival analysis of PITX2 performance on sub-populations based on nomogram risk estimation. The upper left plot shows the performance of the nomogram as a prognostic marker. The upper right plot shows the performance of PITX2 on patients with a 90% chance of 5-year PSA-free survival according to the nomogram.

The lower left plot shows the performance of PITX2 on patients with less than 90% chance of 5-year PSA-free survival according to the nomogram.

SEQ ID NO:63

With Cox regression analysis, SEQ ID NO:63 is a valuable prognostic marker independent of other clinical prognostic information (Table 19). The hazard ratio is 2.9. In the survival analysis of sub-groups, SEQ ID NO:63 seems to have the potential to be a significant marker for some sub-groups, such as high Gleason patients (Figure 99) and patients with poor nomogram-based prognosis (Figure 100).

Figure 99 shows the survival analysis of SEQ ID NO:63 performance on Gleason score 8, 9, and 10 patients.

Figure 100 shows the survival analysis of SEQ ID NO:63 performance on patients with less than 90% chance of 5-year PSA-free survival according to the nomogram.

SEQ ID NO:35 is a marker for some sub-groups, such as pT2 patients (Figure 101).

Discussion

PITX2, SEQ ID NO:35, and GPR7 all show significant prognostic information when the median methylation level is used as a cut-off. Setting the methylation cut-off even higher than the median improves the performance of these three markers. This has the effect of decreasing the marker positive group and increasing the specificity of the test. The median methylation level is not optimal for SEQ ID NO: 63. Instead, a lower cut-off more clearly separates the good and bad prognosis groups for this marker. The optimized methylation cut-off values for these four markers all fall in the range for which their respective assays are technically well suited.

The patients whose samples were analyzed in this study are representative of the population who would be targeted for a prostatectomy test. Therefore, it is possible to speculate on the information these markers could provide for future patients. PITX2, for example, has a sensitivity of around 60% and a specificity of 70%. In the Kaplan-Meier analysis in Figure 90, the marker positive group has approximately three times the risk of recurrence after ten years that the marker negative group has. In

Figure 97, Gleason 8-10 patients that are positive for PITX2 have a 65% chance for PSA recurrence in 10 years. In contrast, the Gleason 8-10 patients who were marker negative had only a 15% chance of PSA relapse. The addition of the methylation marker information to the Gleason stratification will allow clinicians to identify a poor prognosis sub-group who can most benefit from adjuvant therapy. If these methylation markers are incorporated into the patient selection procedure for adjuvant therapy clinical trials, clinicians may begin to see a clear benefit to the addition of early adjuvant treatments for poor prognosis patients.

In addition to adding information to Gleason, PITX2 and some of the other markers can also stratify patients with organ-confined disease. Patients with disease that is truly confined to the organ will be cured by complete removal of the organ. Patients with disease that appears to be confined to the organ, but have undetected micrometastases, will not be cured by surgery. These two groups of patients, both with small operable lesions, have tumors with very different capacities for metastases. PITX2 and some of the other markers seem to be detecting these underlying differences in basic tumor aggressiveness.

The ability of these markers to add information to currently used markers is essential. Gleason and staging already provide significant prognostic information, a new test that would not replace but complement these traditional sources of information is both more valuable and more likely to be readily adopted in clinical practice.

In the analysis of the markers on sub-groups of patients, the markers often seemed strongest on patients with poor prognosis based on traditional clinical variables. Gleason 8-10 patients and patients with low nomogram probability for PSA free survival are well stratified by the present markers into good and poor prognosis groups. For a prostatectomy test, these are the ideal patients to target, since the test would be used to select a group of poor prognosis patients who can most benefit from adjuvant therapy. For T3 patients (non-organ-confined disease), the marker SEQ ID NO: 63 is preferred. Overall, this analysis demonstrates that the present markers are especially well suited for identifying poor prognosis patients.

**Example 6: Test of assays on paraffin embedded tissue.**

In the following analysis, methylation within paraffin embedded prostate tissue samples was analysed by means of the assays shown in Table 12 for the analysis of SEQ ID NO: 19, 35, 37 and 63 . This was then compared to the same measurement carried out upon the frozen samples described in Example 2.

Samples

The samples were paraffin embedded prostatectomy samples or fresh frozen tissues as described in Example 3. The samples were sectioned, the tissue was lysed, the DNA was then extracted and bisulfite converted.

309 paraffin embedded samples were available, of these all samples with at least 1ng of DNA per PCR were included in the analysis, with between 1 and 10ng of DNA per PCR being used.

Reagents:

1 x Taqman PCR Buffer A
0.25mM dNTPs
3.5mM MgCl2
900nM each primer
300nM probe
1 unit AmpliTaq Gold

Thermal Cycling profile:

Step 1. 95˚C->10min (Taq Activation)
Repititions:1
Step 2. 95˚C->15s (Denaturation)
63,0˚C->1 min (Annealing/Extension)
Repititions:50

The following categories were compared

1. Early biochemical relapse (PSA relapse after prostatectomy in less than 24 months) vs. no biochemical relapse (no significant rise in PSA during at least 4 years of PSA monitoring after surgery)
132 samples were available in the non-relapse group and 59 samples available in the early relapse group.

2. High Gleason (score =8-10) vs Low Gleason (Score =2-6 with no grade 4 or 5 component) 59 samples were available in the high Gleason group and 64 samples available in the low Gleason group.

Results

Results are shown in Figures 102 to 125, and were calculated using the Wilcoxon test as described above.

Figure 102 shows the detected amplificate in both frozen and PET samples in the early biochemical relapse vs. no biochemical relapse comparisons using the assay of SEQ ID N0:19 shown in Table 12.
Figure 103 shows the detected amplificate in both frozen and PET samples in the early biochemical relapse vs. no biochemical relapse comparisons using the assay of SEQ ID NO:63 shown in Table 12.
Figure 104 shows the detected amplificate in both frozen and PET samples in the early biochemical relapse vs. no biochemical relapse comparisons using the assay of SEQ ID NO:35 shown in Table 12.
Figure 105 shows the detected amplificate in both frozen and PET samples in the early biochemical relapse vs. no biochemical relapse comparisons using the assay of SEQ ID NO:37 shown in Table 12.
Figure 106 shows the detected amplificate in PET samples only in the early biochemical relapse vs. no biochemical relapse comparisons using the assay of SEQ ID N0:19 shown in Table 12.
Figure 107 shows the detected amplificate in PET samples only in the early biochemical relapse vs. no biochemical relapse comparisons using the assay of SEQ ID NO:63 shown in Table 12.
Figure 108 shows the detected amplificate in PET samples only in the early biochemical relapse vs. no biochemical relapse comparisons using the assay of SEQ ID NO:35 shown in Table 12.
Figure 109 shows the detected amplificate in PET samples only in the early biochemical relapse vs. no biochemical relapse comparisons using the assay of SEQ ID NO:37 shown in Table 12.
Figure 110 shows the detected amplificate in frozen samples only in the early biochemical relapse vs. no biochemical relapse comparisons using the assay of SEQ ID N0:19 shown in Table 12.
Figure 111 shows the detected amplificate in frozen samples only in the early biochemical relapse vs. no biochemical relapse comparisons using the assay of SEQ ID NO:63 shown in Table 12.
Figure 112 shows the detected amplificate in frozen samples only in the early biochemical relapse vs. no biochemical relapse comparisons using the assay of SEQ ID NO:35 shown in Table 12.
Figure 113 shows the detected amplificate in frozen samples only in the early biochemical relapse vs. no biochemical relapse comparisons using the assay of SEQ ID NO:37 shown in Table 12.
Figure 114 shows the detected amplificate in both frozen and PET samples in the High Gleason vs. Low Gleason comparisons using the assay of SEQ ID NO:19 shown in Table 12.

Figure 115 shows the detected amplificate in both frozen and PET samples in the High Gleason vs. Low Gleason comparisons using the assay of SEQ ID NO:63 shown in Table 12.
Figure 116 shows the detected amplificate in both frozen and PET samples in the High Gleason vs. Low Gleason comparisons using the assay of SEQ ID NO:35 shown in Table 12.
Figure 117 shows the detected amplificate in both frozen and PET samples in the High Gleason vs. Low Gleason comparisons using the assay of SEQ ID NO:37 shown in Table 12.
Figure 118 shows the detected amplificate in PET samples only in the High Gleason vs. Low Gleason comparisons using the assay of SEQ ID NO:19 shown in Table 12.
Figure 119 shows the detected amplificate in PET samples only in the High Gleason vs. Low Gleason comparisons using the assay of SEQ ID NO:63 shown in Table 12.
Figure 120 shows the detected amplificate in PET samples only in the High Gleason vs. Low Gleason comparisons using the assay of SEQ ID NO:35 shown in Table 12.
Figure 121 shows the detected amplificate in PET samples only in the High Gleason vs. Low Gleason comparisons using the assay of SEQ ID NO:37 shown in Table 12.
Figure 122 shows the detected amplificate in frozen samples only in the High Gleason vs. Low Gleason comparisons using the assay of SEQ ID NO:19 shown in Table 12.
Figure 123 shows the detected amplificate in frozen samples only in the High Gleason vs. Low Gleason comparisons using the assay of SEQ ID NO:63 shown in Table 12.
Figure 124 shows the detected amplificate in frozen samples only in the High Gleason vs. Low Gleason comparisons using the assay of SEQ ID NO:35 shown in Table 12.
Figure 125 shows the detected amplificate in frozen samples only in the High Gleason vs. Low Gleason comparisons using the assay of SEQ ID NO:37 shown in Table 12.

## Claims

**1.** A method for providing a prognosis of a subject with a prostate cell proliferative disorder comprising the following steps of:

(a) obtaining a biological sample from said subject;
(b) determining the expression status of the HIST2H2BF gene or the genomic sequence of SEQ ID NO: 63 in said sample; and
(c) determining therefrom the prognosis of said subject whereby expression is indicative of prognosis.

**2.** A method according to claim 1, wherein at least one further prognostic variable is factored in when determining the prognosis of c).

**3.** A method according to claim 2, wherein said prognostic variable is selected from the group consisting of nomogram, PSA level and Gleason score.

**4.** A method according to any of claims 1 to 3, wherein the expression is determined by determining the level of methylation or methylation status of one or more CpG positions within said gene or by measuring the level of at least one of mRNA, cDNA or polypeptide.

**5.** A method according to claim 1 to 4, comprising the following steps of:

a) isolating genomic DNA from a biological sample taken from said subject;
b) treating the genomic DNA, or a fragment thereof, with one or more reagents to convert 5-position unmethylated cytosine bases to uracil or to another base that is detectably dissimilar to cytosine in terms of hybridization properties;
c) contacting the treated genomic DNA, or the treated fragment thereof, with an amplification enzyme and at least two primers comprising, in each case a contiguous sequence at least 9 nucleotides in length that is complementary to, or hybridizes under moderately stringent or stringent conditions to a sequence selected from the group consisting of SEQ ID NOs 189, 190, 317, 318, and complements thereof, wherein the treated DNA or a fragment thereof is either amplified to produce one or more amplificates, or is not amplified;
d) determining, based on the presence or absence of, or on the quantity or on a property of said amplificate, the methylation state of at least one CpG dinucleotide sequence of the HIST2H2BF gene or the genomic sequence of SEQ ID NO: 63 or an average, or a value reflecting an average methylation state of a plurality of CpG dinucleotide sequences of the HIST2H2BF gene or the genomic sequence of SEQ ID NO: 63; and
e) determining from said methylation state the prognosis of said subject

**6.** A treated nucleic acid derived from SEQ ID NOs: 63, wherein the treatment is suitable to convert at least one unmethylated cytosine base of the genomic DNA sequence to uracil or another base that is detectably dissimilar to cytosine in terms of hybridization.

**7.** A nucleic acid, comprising at least 16 contiguous nucleotides of a treated genomic DNA sequence selected from the group consisting of SEQ ID NOs: 189, 190, 317, 318, and sequences complementary thereto, wherein said nucleic acid is not identical or complementary to SEQ ID NOS: 63, wherein the treatment is suitable to convert at least one unmethylated cytosine base of the genomic DNA sequence to uracil or another base that is detectably dissimilar to cytosine in terms of hybridization, preferably the contiguous base sequence comprises at least one CpG, TpG or CpA dinucleotide sequence.

**8.** The nucleic acid of any of claims 6 to 7, wherein the treatment comprises use of a reagent selected from the group consisting of bisulfite, hydrogen sulfite, disulfite, and combinations thereof.

**9.** An oligomer, comprising a sequence of at least 9 contiguous nucleotides that is complementary to, or hybridizes under moderately stringent or stringent conditions to a treated genomic DNA sequence selected from the group consisting of SEQ ID NOs: 189, 190, 317, 318, and sequences complementary thereto, wherein said nucleic acid is not identical or complementary to SEQ ID NO: 63, preferably the oligomer comprises at least one CpG, CpA or TpG dinucleotide.

**10.** A kit for use in for use in providing a prognosis of a subject with a prostate cell proliferative disorder, comprising: a means for detecting the polypeptides of the gene HIST2H2BF or the genomic sequence of SEQ ID NO: 63.

**11.** A kit for use in for use in providing a prognosis of a subject with a prostate cell proliferative disorder, comprising: a means for measuring the level of mRNA transcription of the HIST2H2BFgene or the genomic sequence of SEQ ID NO: 63.

**12.** A kit comprising:

- a bisulfite reagent; and
- at least two nucleic acid molecules comprising, in each case a contiguous sequence at least 16 nucleotides that is complementary to, or hybridizes under moderately stringent or stringent conditions to a sequence selected from the group consisting of SEQ ID NOS: 189, 190, 317, 318, and complements thereof.

**13.** A composition comprising the following:

a) a nucleic acid comprising a sequence at least 18 bases in length of a segment of the chemically pretreated genomic DNA according to one of the sequences taken from the group consisting of SEQ ID NOs: 189, 190, 317, 318, and sequences complementary thereto, and
b) a buffer comprising at least one of the following substances: magnesium chloride, dNTP, of taq polymerase, an oligomer, in particular an oligonucleotide or peptide nucleic acid (PNA)-oligomer, said oligomer comprising in each case at least one base sequence having a length of at least 9 contiguous nucleotides which is complementary to, or hybridizes under moderately stringent or stringent conditions to a pre-treated genomic DNA according to one of the SEQ ID NOs: 189, 190, 317, 318, and sequences complementary thereto.

**14.** The use of a method according to claims 1 to 18, a nucleic acid according to claims 19 to 22, an oligomer according to claims 23 to 24, a kit according to claims 25 to 29 or a composition according to claim 30 for providing the diagnosis of, prognosis of, treatment of, monitoring of, and treatment and monitoring of a subject with a prostate cell proliferative disorder.

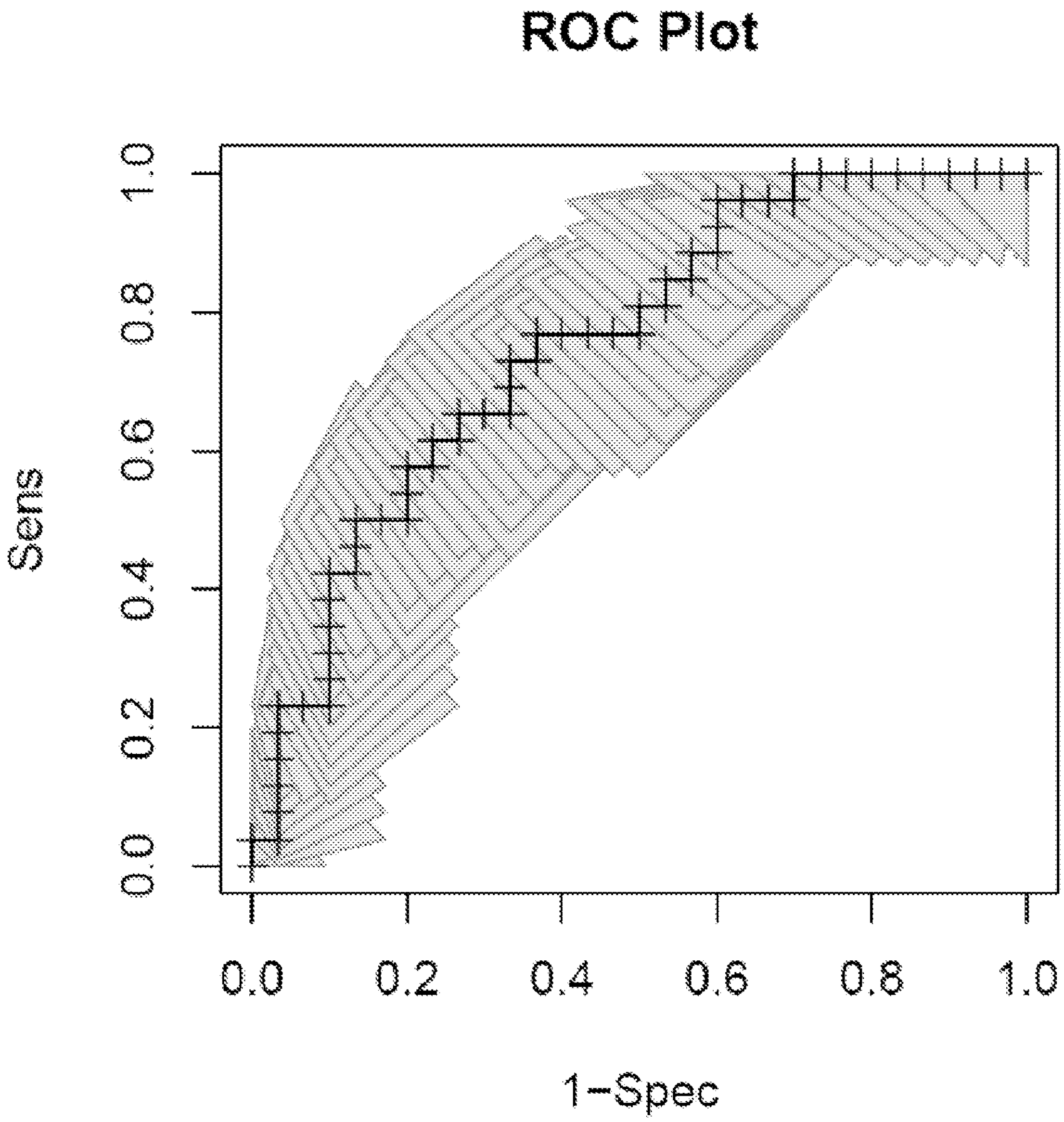


**Figure 1**

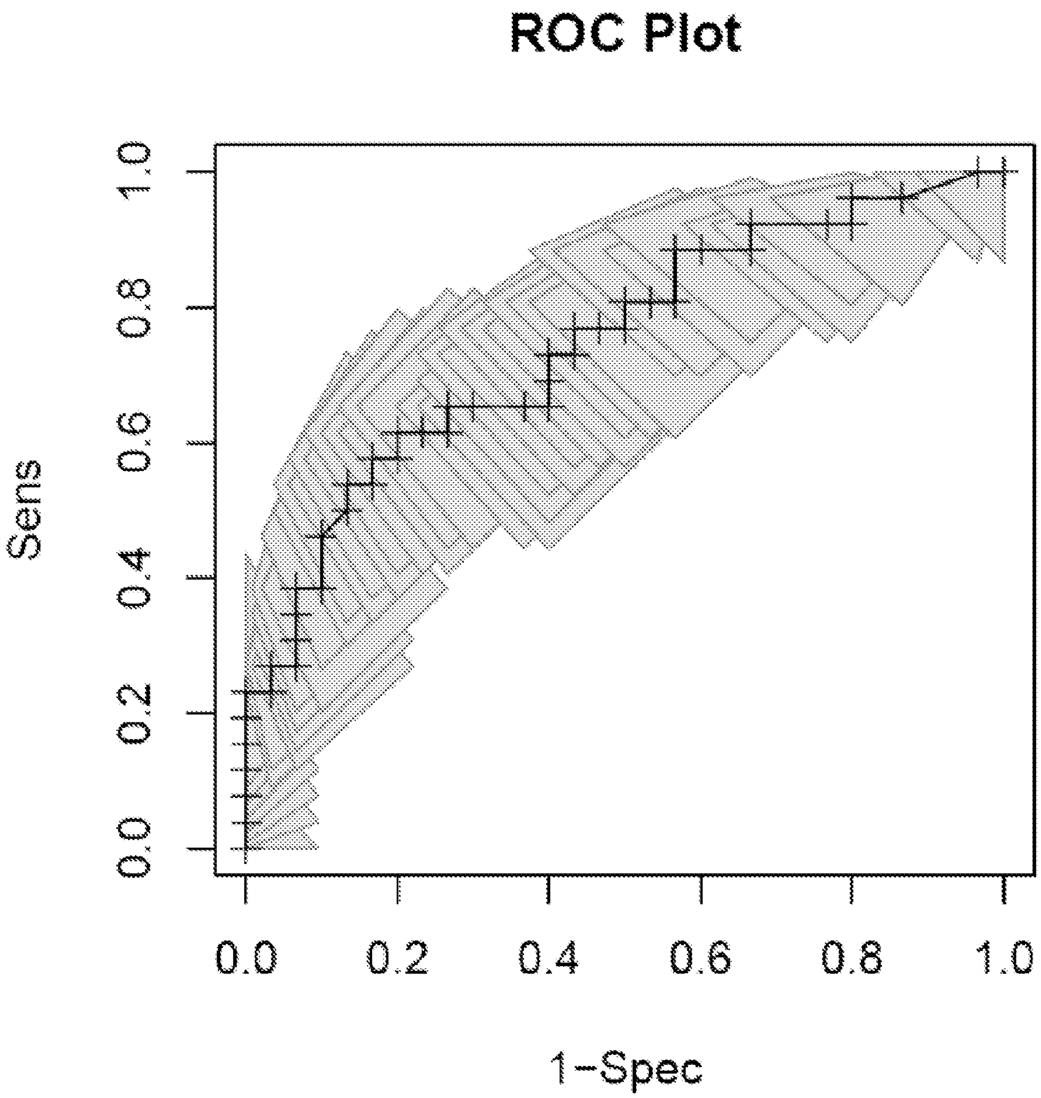


Figure 2

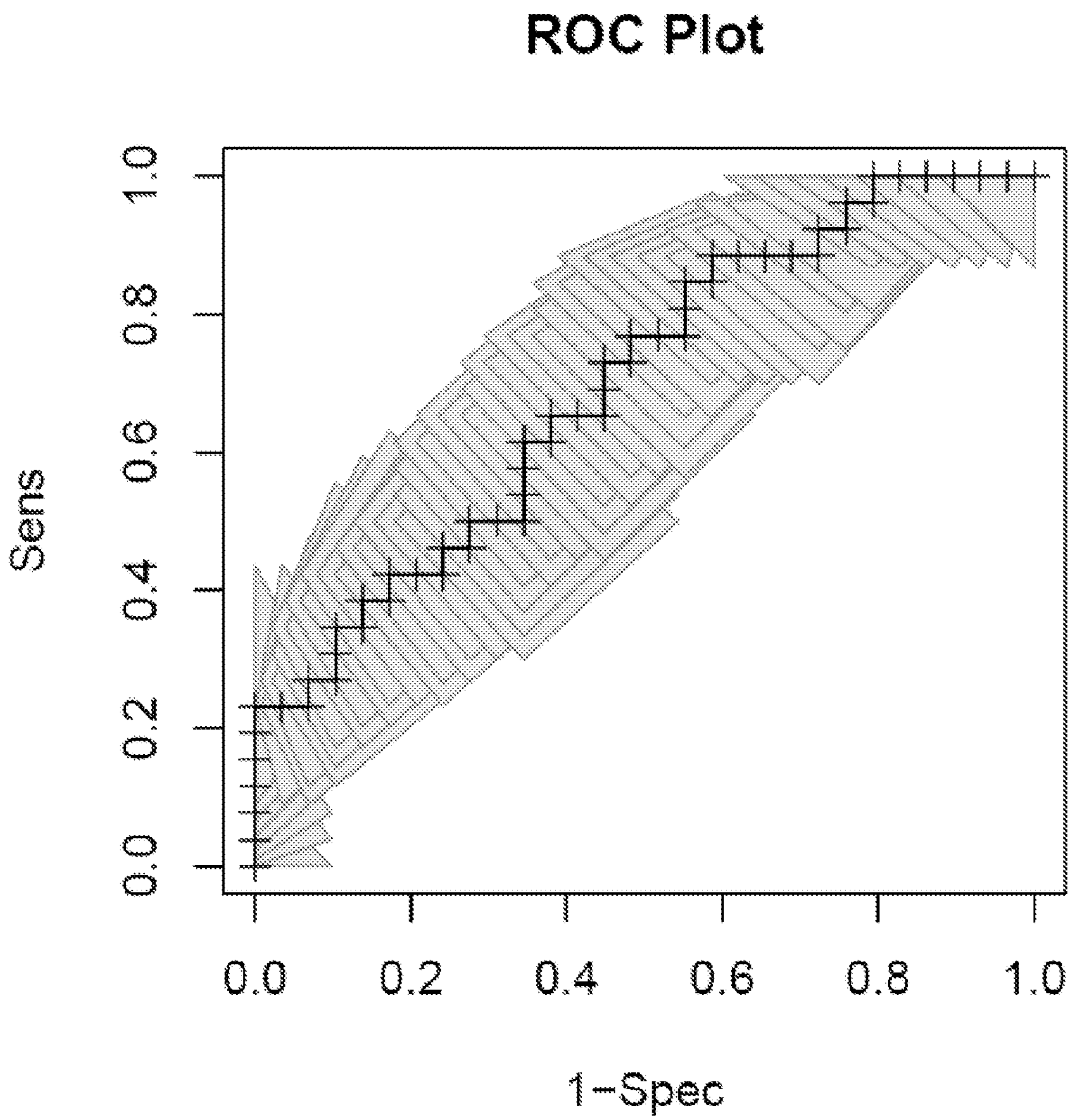


Figure 3

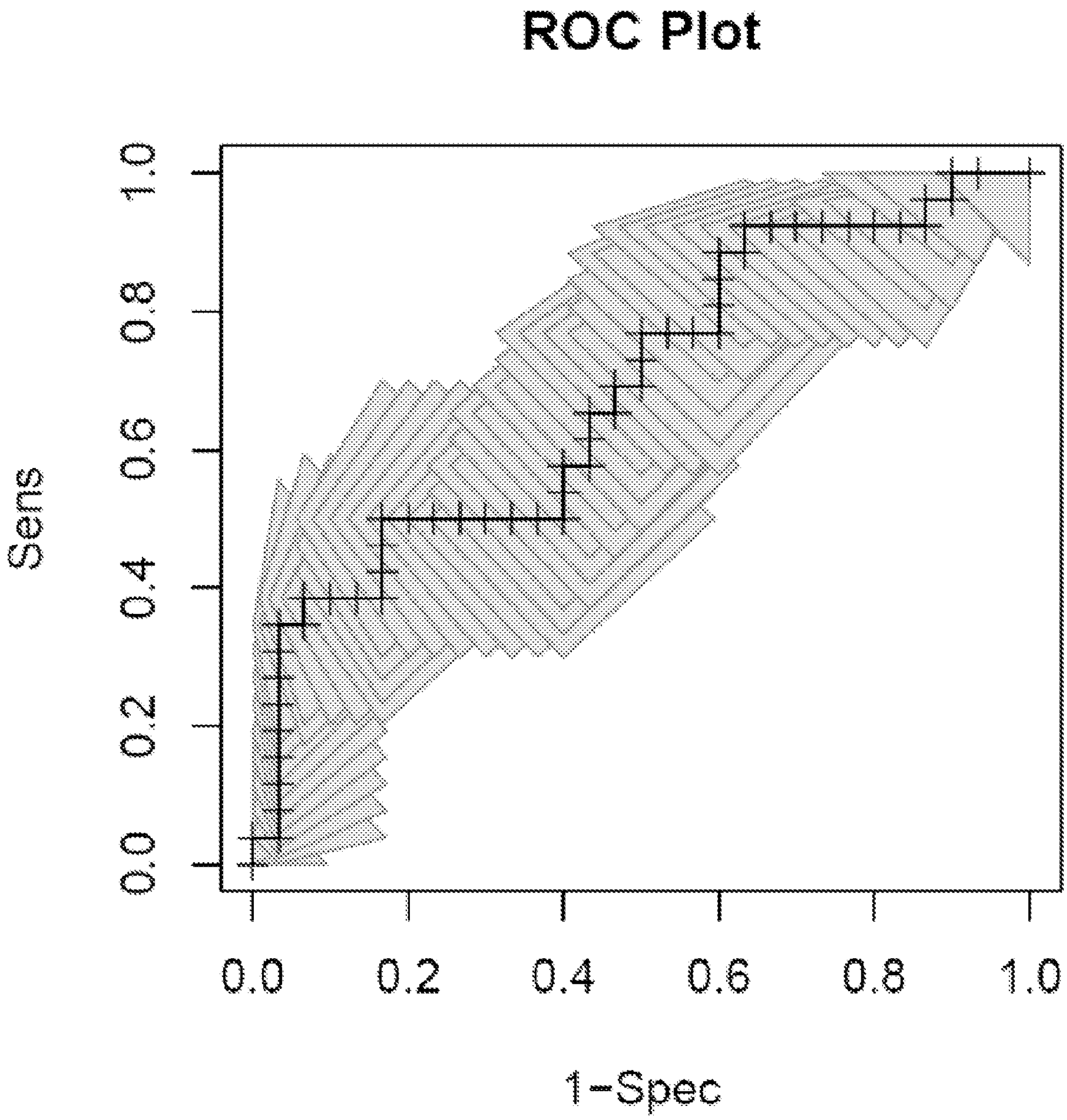


**Figure 4**

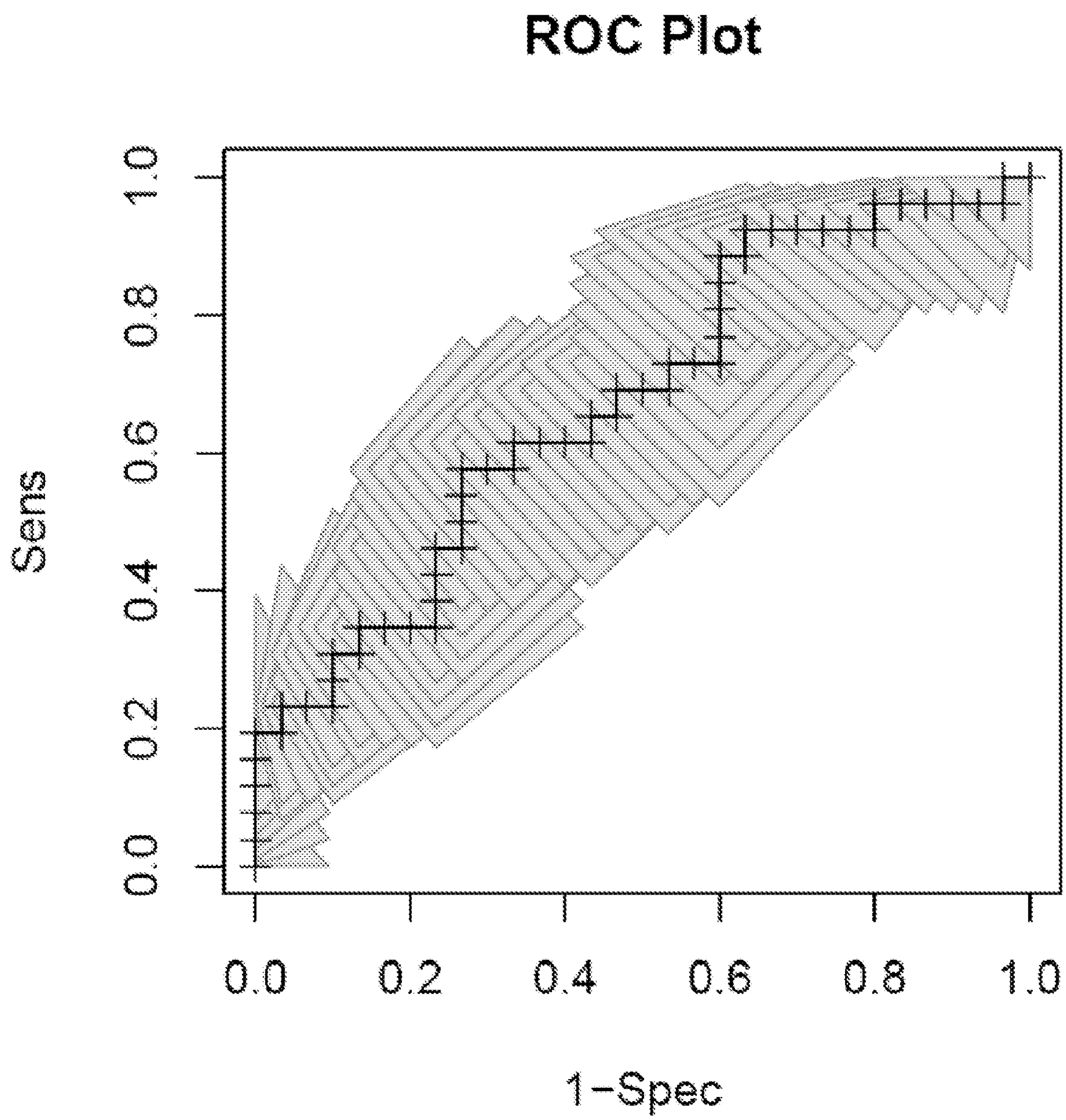


Figure 5

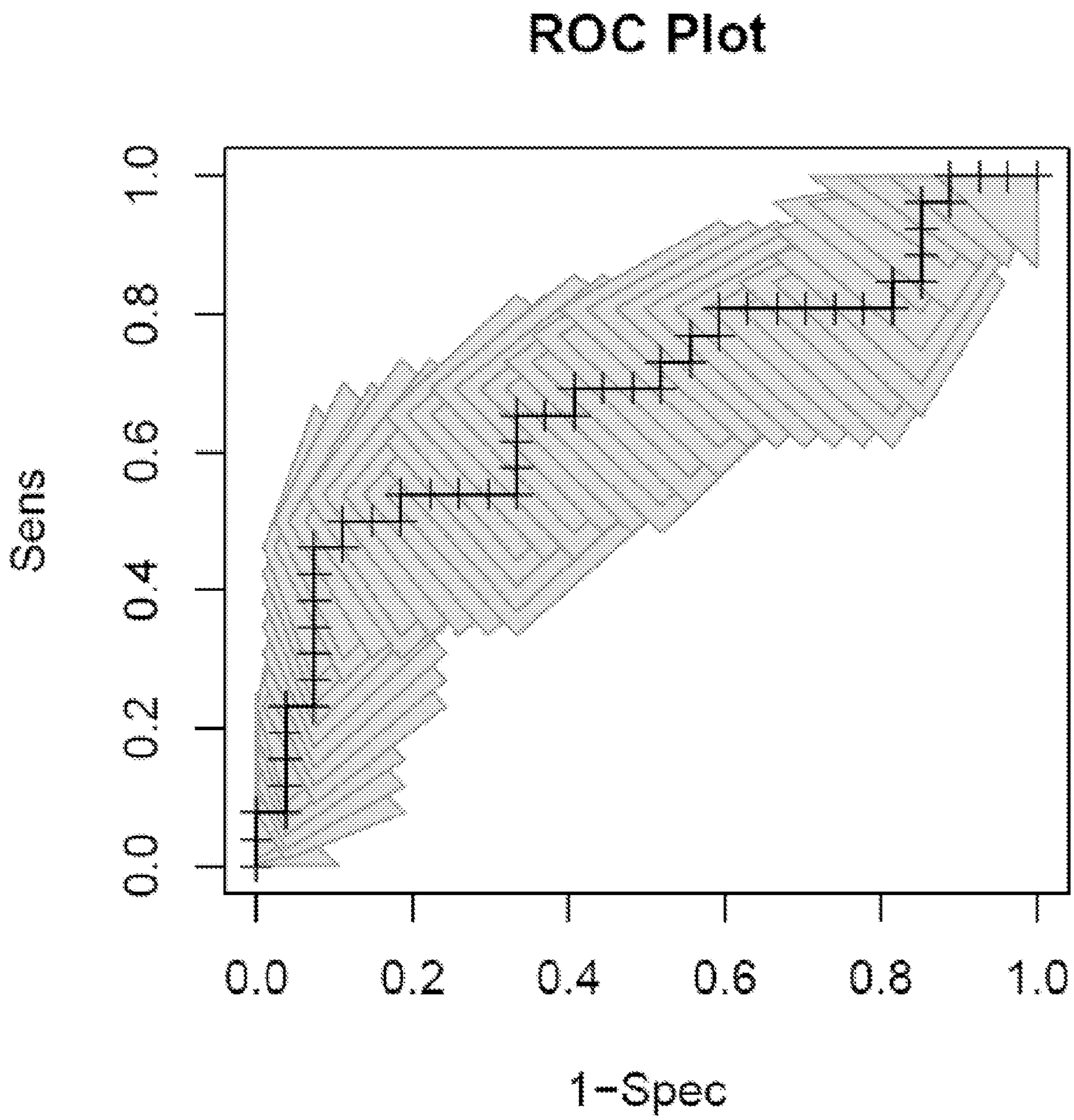


Figure 6

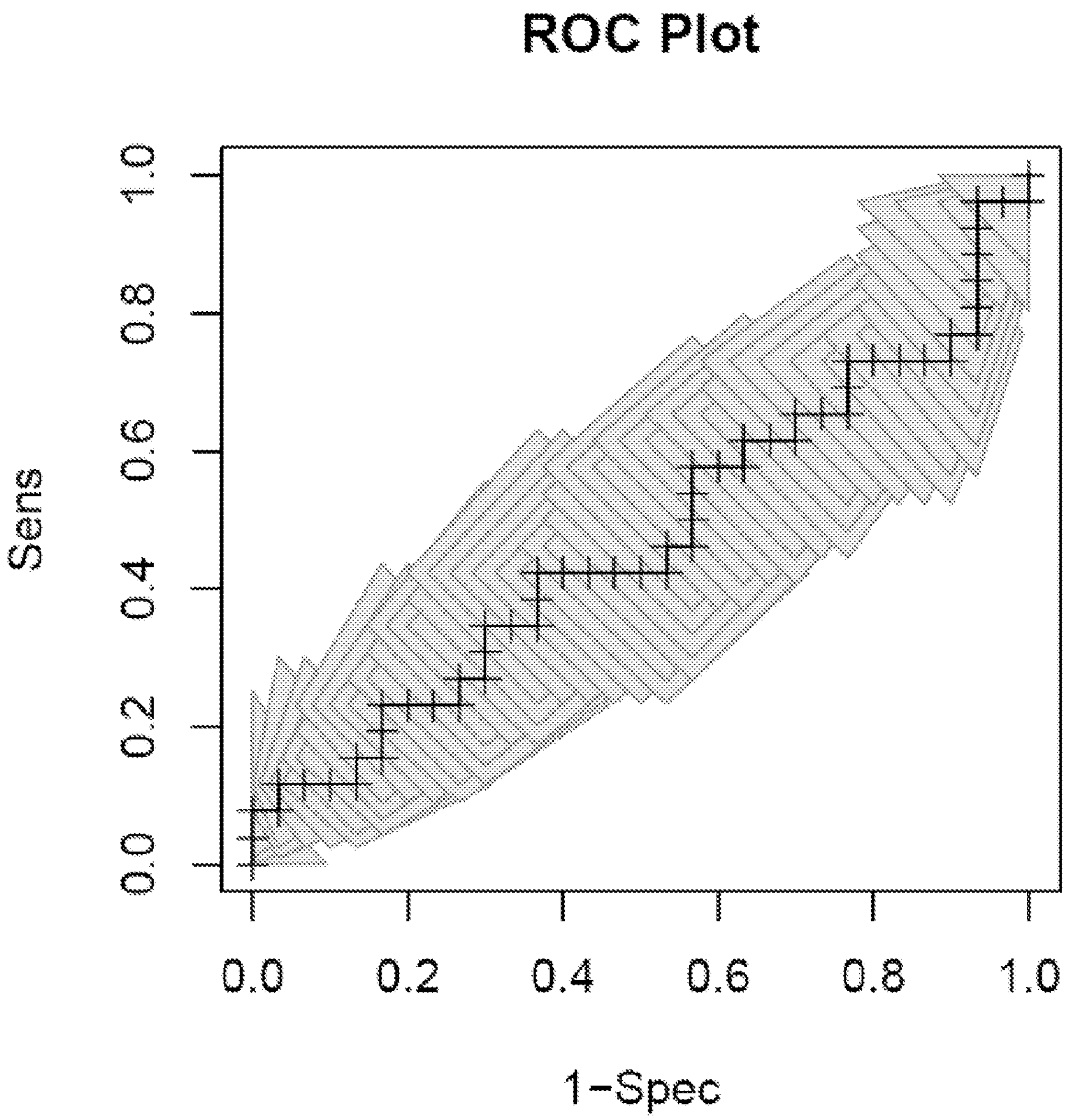


Figure 7

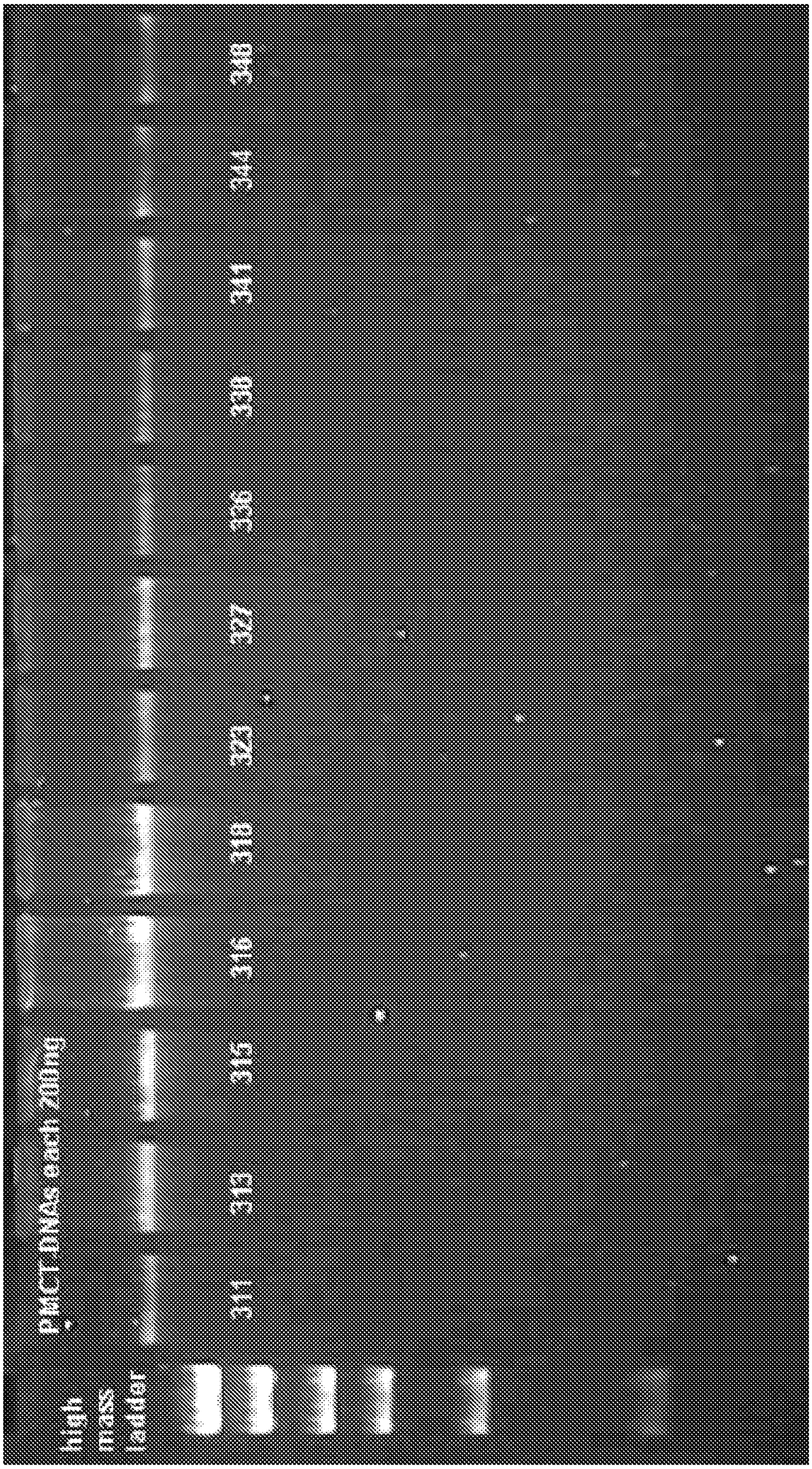
high
mass
ladder
PMCT DNAs each 200ng
311
313
315
316
318
323
327
336
338
341
344
348

Figure 8

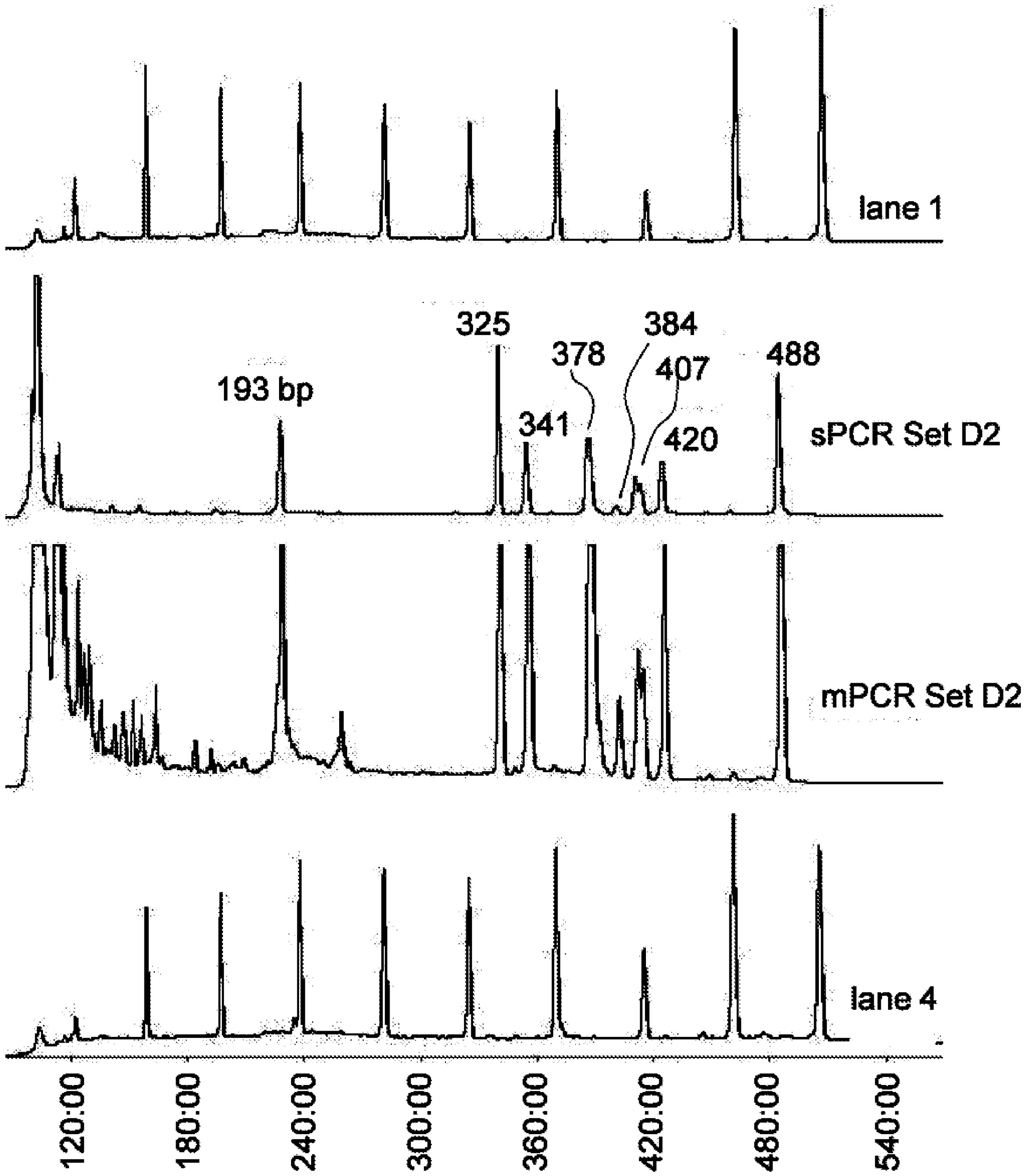
lane 1
193 bp
325
341
378
384
407
420
488
sPCR Set D2
mPCR Set D2
lane 4
120:00
180:00
240:00
300:00
360:00
420:00
480:00
540:00

Figure 9

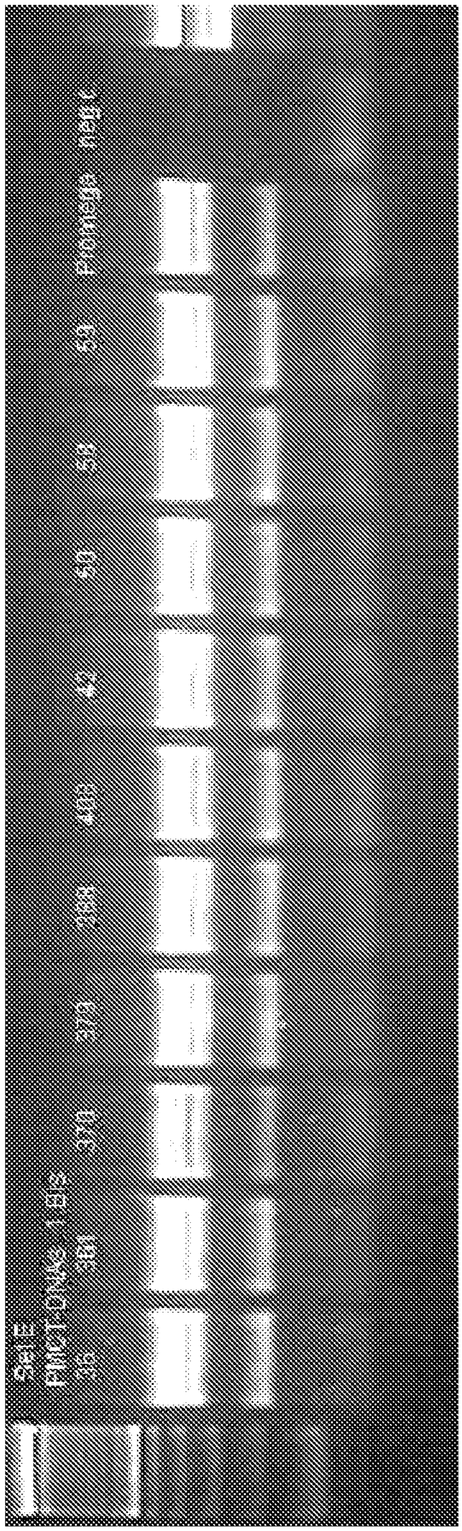


Figure 10

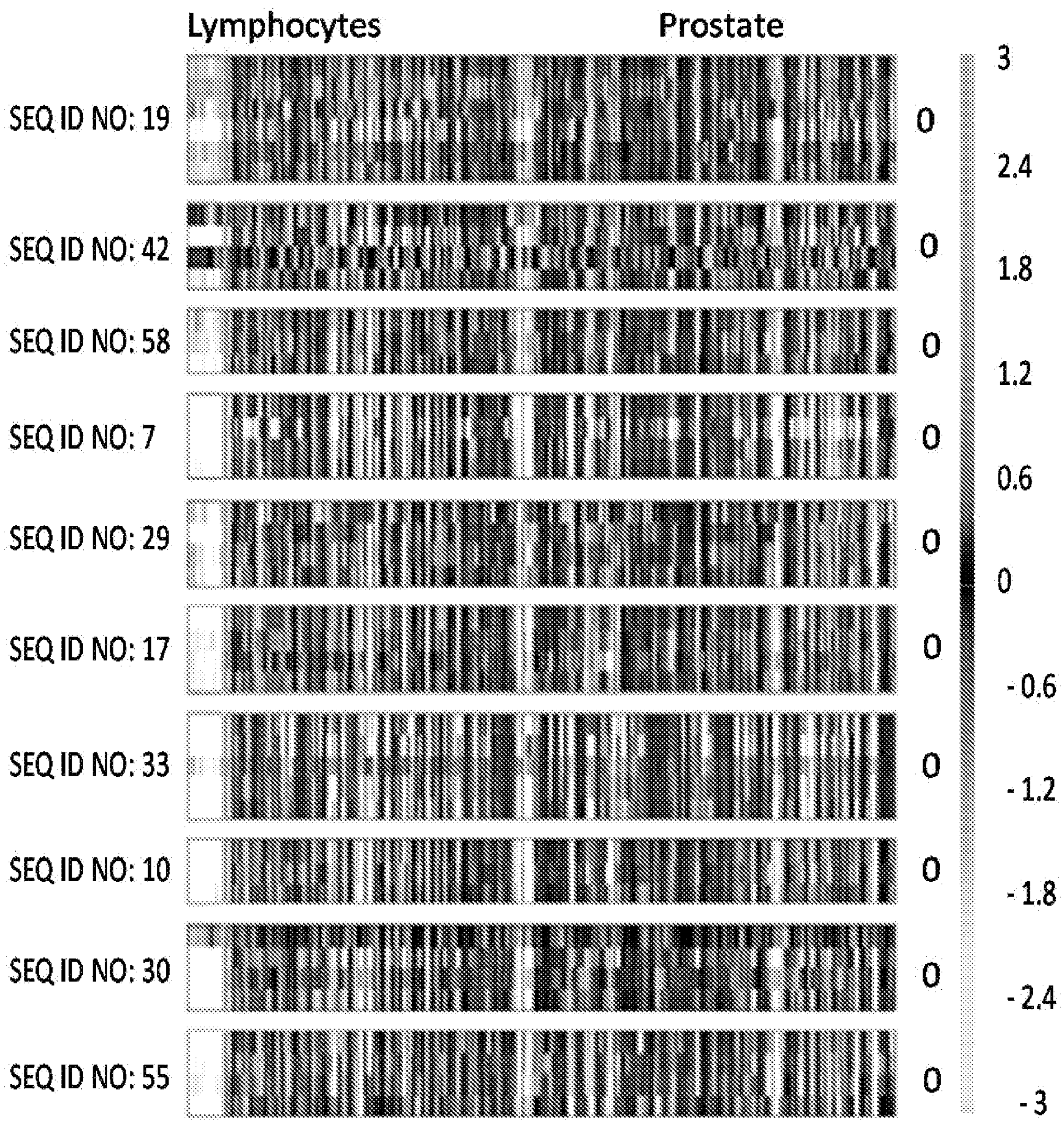
Lymphocytes
Prostate
SEQ ID NO: 19
SEQ ID NO: 42
SEQ ID NO: 58
SEQ ID NO: 7
SEQ ID NO: 29
SEQ ID NO: 17
SEQ ID NO: 33
SEQ ID NO: 10
SEQ ID NO: 30
SEQ ID NO: 55
0
0
0
0
0
0
0
0
0
0
3
2.4
1.8
1.2
0.6
0
- 0.6
- 1.2
- 1.8
- 2.4
- 3

Figure 11

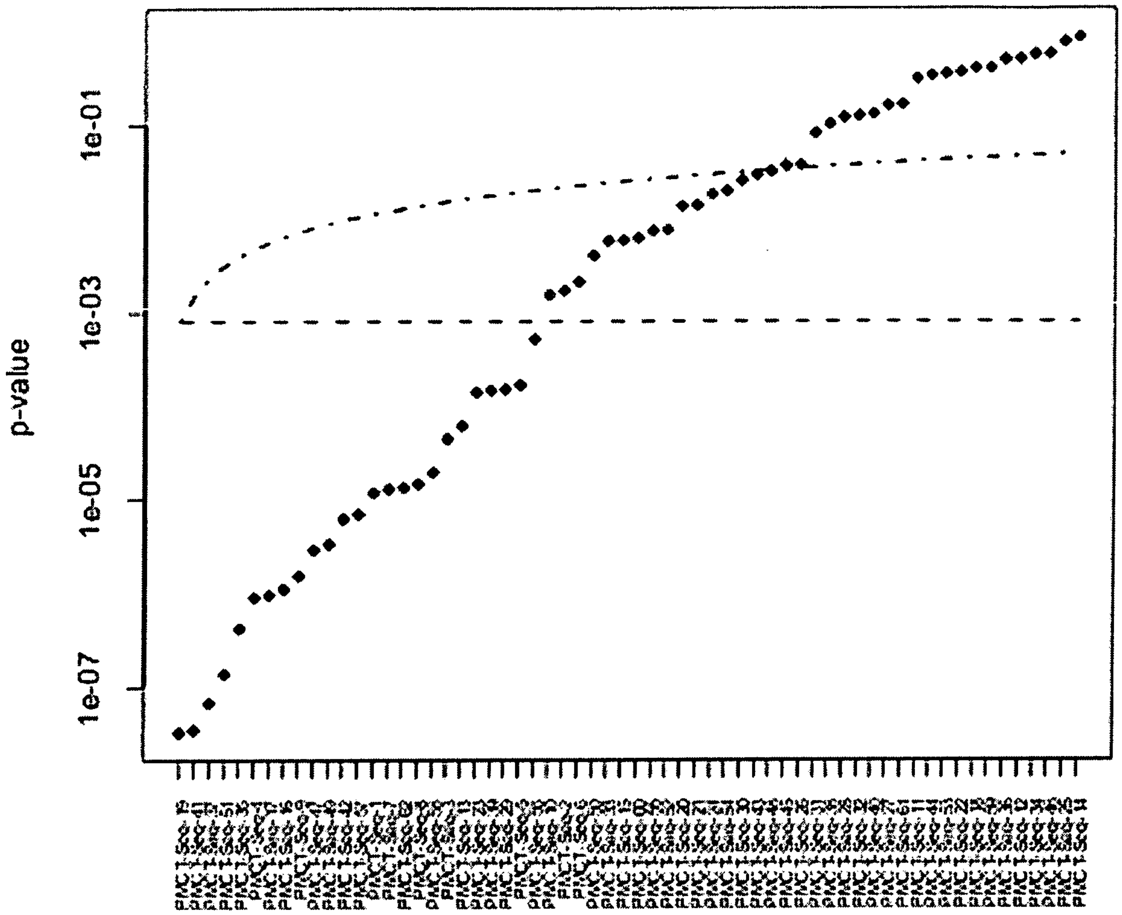


Figure 12

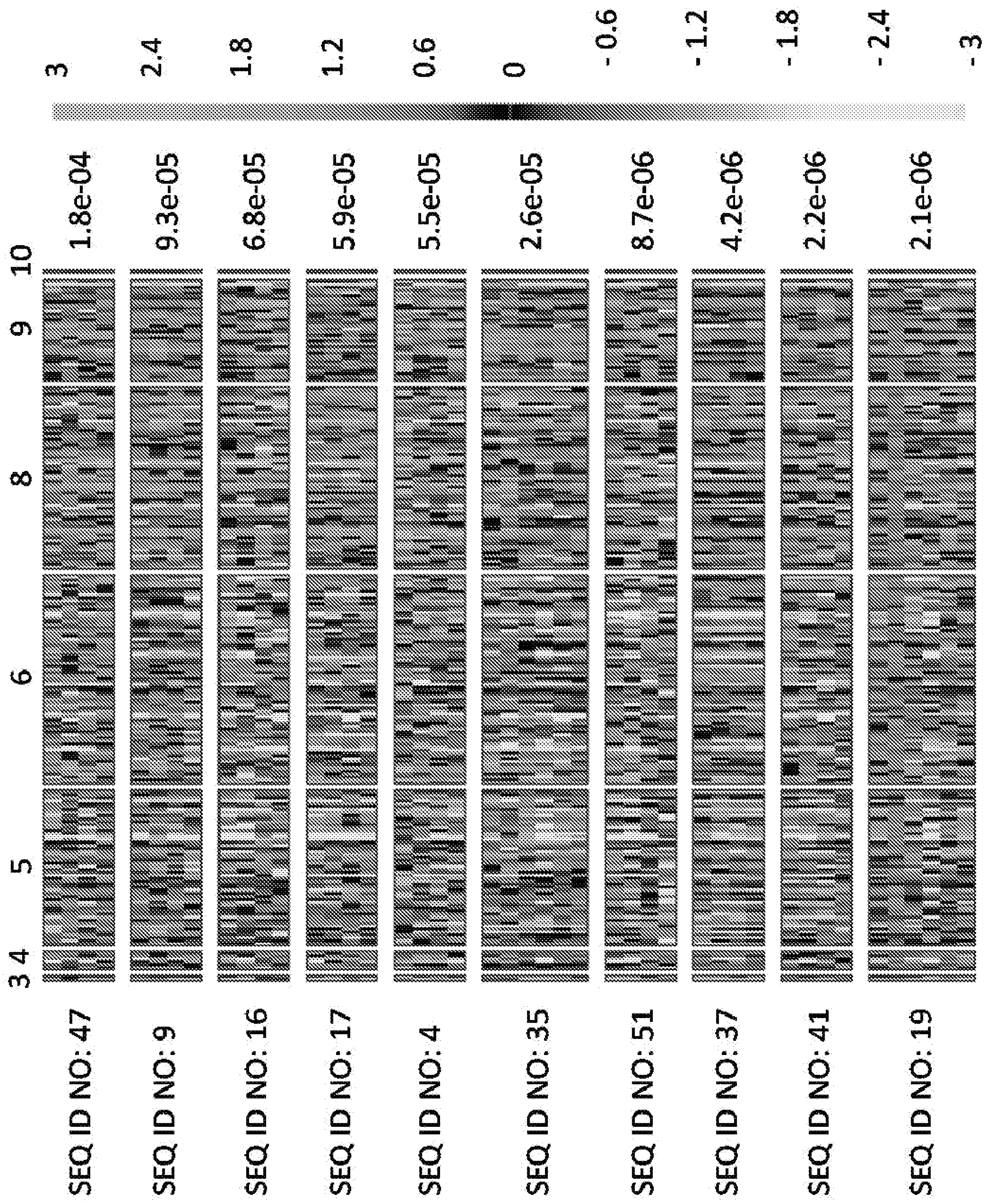
3
2.4
1.8
1.2
0.6
0
- 0.6
- 1.2
- 1.8
- 2.4
- 3
1.8e-04
9.3e-05
6.8e-05
5.9e-05
5.5e-05
2.6e-05
8.7e-06
4.2e-06
2.2e-06
2.1e-06
3 4
5
6
8
9
10
SEQ ID NO: 47
SEQ ID NO: 9
SEQ ID NO: 16
SEQ ID NO: 17
SEQ ID NO: 4
SEQ ID NO: 35
SEQ ID NO: 51
SEQ ID NO: 37
SEQ ID NO: 41
SEQ ID NO: 19

Figure 13

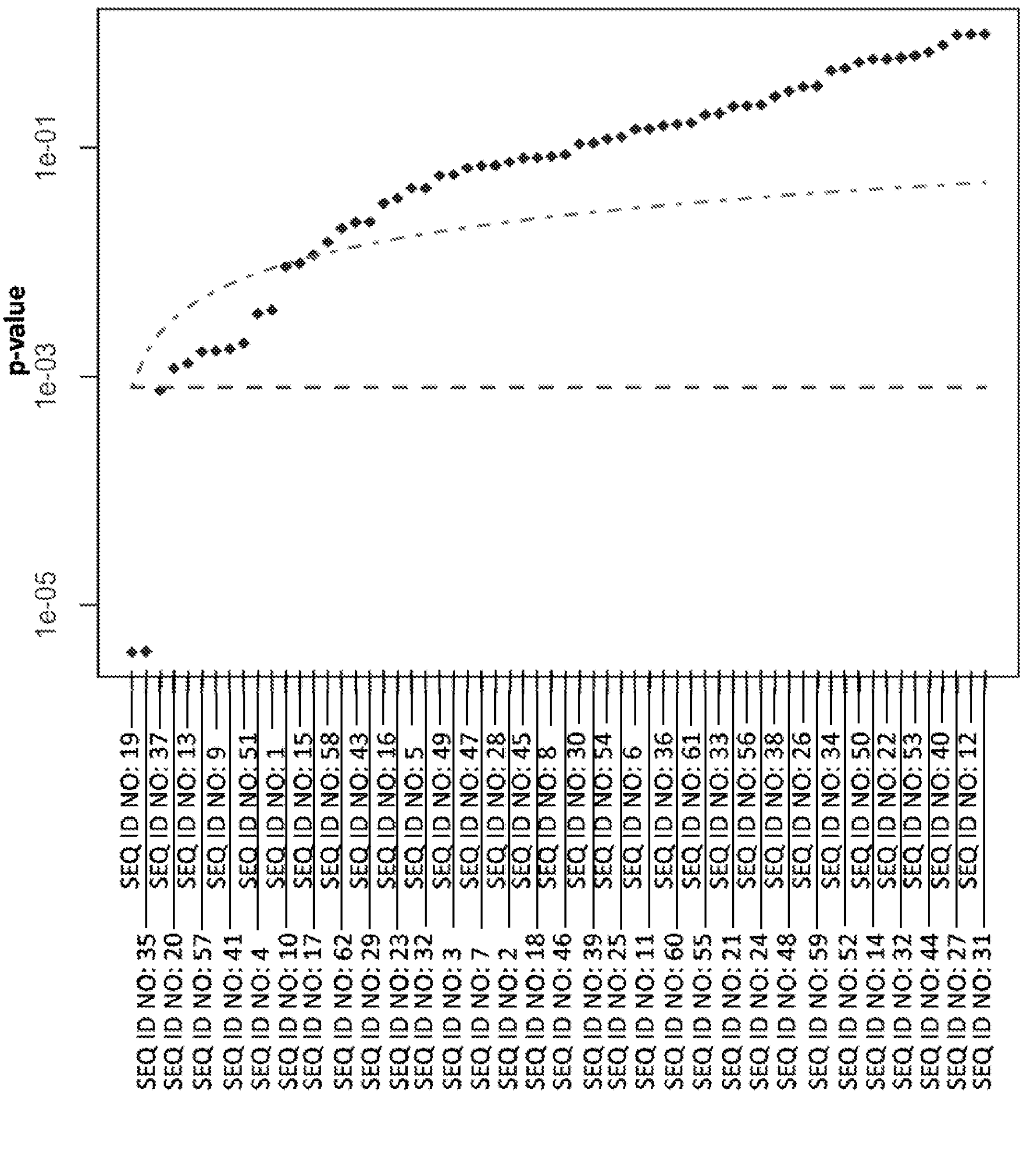
p-value
1e-01
1e-03
1e-05
SEQ ID NO: 19
SEQ ID NO: 35
SEQ ID NO: 37
SEQ ID NO: 20
SEQ ID NO: 13
SEQ ID NO: 57
SEQ ID NO: 9
SEQ ID NO: 41
SEQ ID NO: 51
SEQ ID NO: 4
SEQ ID NO: 1
SEQ ID NO: 10
SEQ ID NO: 15
SEQ ID NO: 17
SEQ ID NO: 58
SEQ ID NO: 62
SEQ ID NO: 43
SEQ ID NO: 29
SEQ ID NO: 16
SEQ ID NO: 23
SEQ ID NO: 5
SEQ ID NO: 32
SEQ ID NO: 49
SEQ ID NO: 3
SEQ ID NO: 47
SEQ ID NO: 7
SEQ ID NO: 28
SEQ ID NO: 2
SEQ ID NO: 45
SEQ ID NO: 18
SEQ ID NO: 8
SEQ ID NO: 46
SEQ ID NO: 30
SEQ ID NO: 39
SEQ ID NO: 54
SEQ ID NO: 25
SEQ ID NO: 6
SEQ ID NO: 11
SEQ ID NO: 36
SEQ ID NO: 60
SEQ ID NO: 61
SEQ ID NO: 55
SEQ ID NO: 33
SEQ ID NO: 21
SEQ ID NO: 56
SEQ ID NO: 24
SEQ ID NO: 38
SEQ ID NO: 48
SEQ ID NO: 26
SEQ ID NO: 59
SEQ ID NO: 34
SEQ ID NO: 52
SEQ ID NO: 50
SEQ ID NO: 14
SEQ ID NO: 22
SEQ ID NO: 32
SEQ ID NO: 53
SEQ ID NO: 44
SEQ ID NO: 40
SEQ ID NO: 27
SEQ ID NO: 12
SEQ ID NO: 31

Figure 14

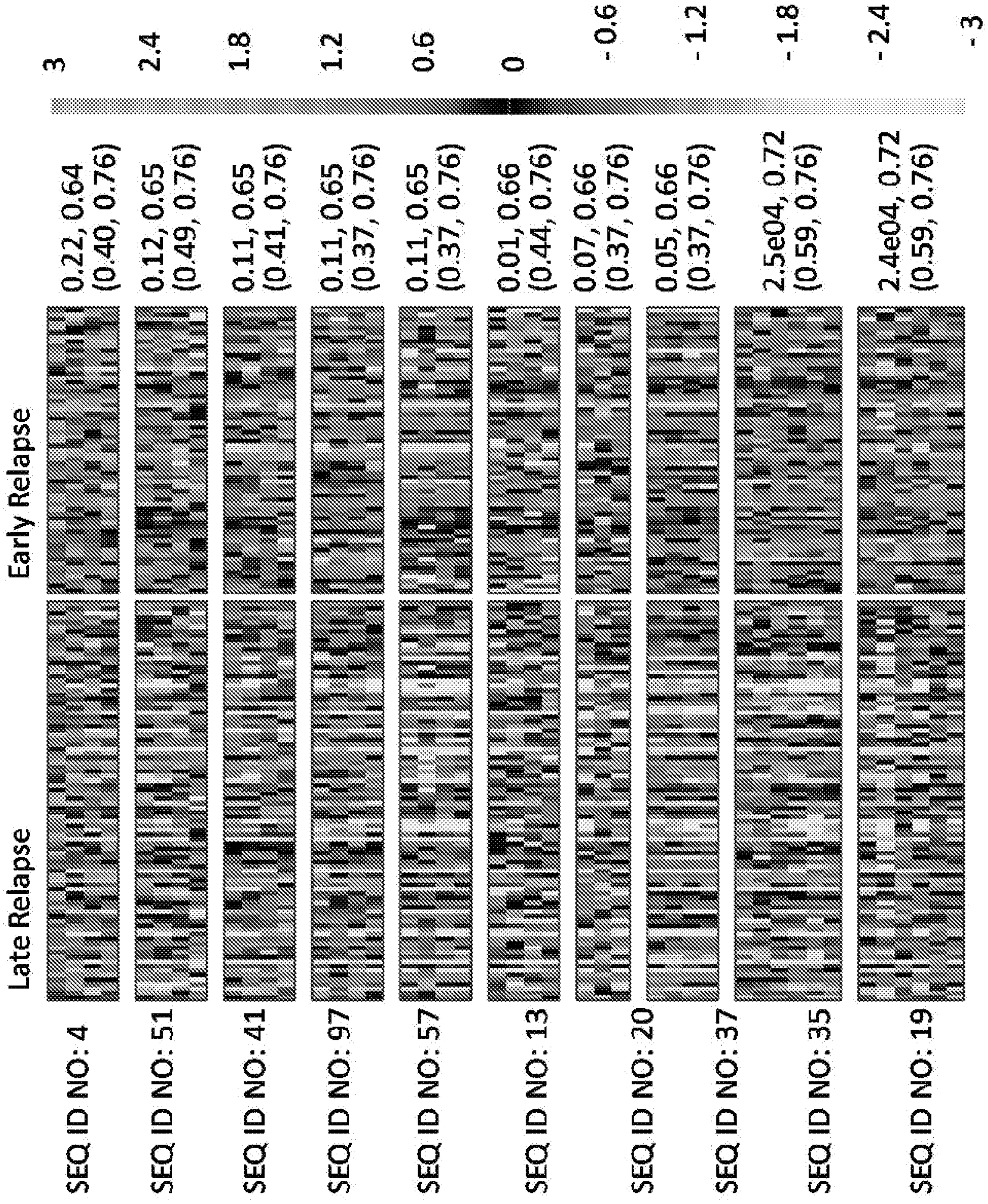
Late Relapse
Early Relapse
SEQ ID NO: 4
0.22, 0.64 (0.40, 0.76)
SEQ ID NO: 51
0.12, 0.65 (0.49, 0.76)
SEQ ID NO: 41
0.11, 0.65 (0.41, 0.76)
SEQ ID NO: 97
0.11, 0.65 (0.37, 0.76)
SEQ ID NO: 57
0.11, 0.65 (0.37, 0.76)
SEQ ID NO: 13
0.01, 0.66 (0.44, 0.76)
SEQ ID NO: 20
0.07, 0.66 (0.37, 0.76)
SEQ ID NO: 37
0.05, 0.66 (0.37, 0.76)
SEQ ID NO: 35
2.5e04, 0.72 (0.59, 0.76)
SEQ ID NO: 19
2.4e04, 0.72 (0.59, 0.76)
3
2.4
1.8
1.2
0.6
0
-0.6
-1.2
-1.8
-2.4
-3

Figure 15

Figure 16 Amplificate of SEQ ID NO: 14

```
  1 GAAGAGGTGCTGAGAAATTAAAAATTCAGGTTAGTTAATGCATCCCTGCCGCCGGCTGCAGGCTCCGCCTTTGCATTAAG  80
  1 GAAGAGGTGTTGAGAAATTAAAA                                                            80
  1 GAAGAGGTGtTGAGAAATTAAAAATTtAGGTTAGTTAATGtATttttTGtCGtCGGtTGtAGGtTtCGttTTTGtATTAAG  80
  1                                                                            TTAAG  80
  1                                                                            TTAAG  80

 81 CGGGCGCTGATTGTGCGCGCCTGGCGACCGCGGGGAGGACTGGCGGCCCGCGGGAGGGGACGGGTAGAGGCGCGGGTTAC 160
 81 CGGGCGtTGATTGTGCGCGttTGGCGAtCGCGGGGAGGAtTGGCGGttCGCGGGAGGGGACGGGTAGAGGCGCGGGTTAt 160
 81 CGGGCGTTGAT                                                       TAGAGGCGCGGGTTAT 160
 81 TGGGTGTTGAT                                                       TAGAGGTGTGGGTTAT 160

161 ATTGTTCTGGAGCCGGCTCGGCTCTTTGTGCCTCCTCTAGCGGCCAAGCTGCGAGGTACAGCCCTCTATTGTTCTAGGAG 240
161 ATTGTTtTGGAGtCGGtTCGGtTtTTTGTGttTttTtTAGCGGttAAGtTGCGAGGTAtAGtttTtTATTGTTtTAGGAG 240

241 CACAGAAACCTCCTGTGTGGGCGGCGGGTGCGCGAGCTAGAGGGAAAGATGCAGTAGTTACTGCGACTGGCACGCAGTTG 320
241 tAtAGAAAttTttTGTGTGGGCGGCGGGTGCGCGAGtTAGAGGGAAAGATGtAGTAGTTAtTGCGAtTGGtACGtAGTTG 320
241                                                             TTGCGATTGGTACGTA     320
241                                                              TGTGATTGGTATGTAGT   320
```

```
321 CGCGCTTTTGTGCGCACGGACCCCGCGCGGTGTGCGTGGCGACTGCGCTGCCCCTAGGAGCAAGCCACGGGCCCAGAGGG 400
321 CGCGtTTTTGTGCGtACGGAtttCGCGCGGTGTGCGTGGCGAtTGCGtTGttttTAGGAGtAAGttACGGGtttAGAGGG 400
321     TTTTTGTGTGTATGGAT                                                               400
321      TTTTGTGCGTACGGAT

401 GCAAAATGTCCAGGTCCCCCGCTGGGAAGGACACACTATACCCTATGGCAAGCCAGGGTGGG                    480
401                                                      TTTATGGTAAGTTAGGGTGGG            480
401 GtAAAATGTttAGGTttttCGtTGGGAAGGAtAtAtTATAtttTATGGtAAGttAGGGTGGG                    480
```

Figure 17: Amplificate of SEQ ID NO: 15

```
  1 TGCAGGAGAGGTTGGGAAGGGGTGGGGGACGGGGCTCGGGGGAGGTCTCCGAGGGACTCTAGTAAGCGGGGAAGGGCGCC  80
  1 TGTAGGAGAGGTTGGGAAG                                                                80
  1 TGtAGGAGAGGTTGGGAAGGGGTGGGGGACGGGGtTCGGGGGAGGTtTtCGAGGGAtTtTAGTAAGCGGGGAAGGGCGtC  80

 81 GGGAAAGTTTCAGATCCACGGCTGCGCGGGCCACGAGCCCACCCGAACGCCGACCACTGCTTTCCGTCGACTTCTATTTC 160
 81 GGGAAAGTTTtAGATttACGGtTGCGCGGGttACGAGtttAttCGAACGtCGAttAtTGtTTTtCGTCGAtTTtTATTTt 160
 81                                GGTTATGAGTTTATTTGAA                               160
 81                                 GTTACGAGTTTATTCGA                                160

161 CTGGGAACGCGCGAAAGCAAACCCAAGTCAGACTGCGGAGGTCGCTGGGGAGGGAAGGTTCAAGGAGTTCTCGCCGATCC 240
161 tTGGGAACGCGCGAAAGtAAAtttAAGTtAGAtTGCGGAGGTCGtTGGGGAGGGAAGGTTtAAGGAGTTtTCGtCGATtt 240
161 TTGGGAATGTGTGAAA                                                 AGGAGTTTTTGTTGATT 240
161     AACGCGCGAAAGTAAA                                               GAGTTTTCGTCGATTT 240

241 TGCTGAATAAAGGGGGTTCCGAGCTGGGCCGAGATGGGGCATGCGCGGGAAGACCCCTGCCCGCTGTTCCCCCCCACCGC 320
241                                                                                T 320
241 TGtTGAATAAAGGGGGTTtCGAGtTGGGtCGAGATGGGGtATGCGCGGGAAGAttttTGttCGtTGTTtttttttAtCGt 320
241                                        GTATGTGTGGGAAGAT                          320
241                                         TATGCGCGGGAAGATT
```

```
321 CCCAGTGGATGCCATGCCTGG    400
321 TTTAGTGGATGTTATGTTTGG    400
321 tttAGTGGATGttATGttTGG    400
```

Figure 18: Amplificate of SEQ ID NO: 16

```
459 GCTTCTAAGTGCTGGGTGATTTCCGCGGGAAGTCCCTGGGGCAGCAGCCAGCATCTGATGCTAGGATCCTATCCATACCA 380
459 GTTTTTAAGTGTTGGGTGATTT                                                           380
459 GtTTtTAAGTGtTGGGTGATTTtCGCGGGAAGTtttTGGGGtAGtAGttAGtATtTGATGtTAGGATttTATttATAttA 380
459                     ATTTTCGCGGGAAGTT                                             380
459                   GTGATTTTTGTGGGAA                                               380

379 ACCCAGATGTTCCTGCGTTGCAGTAGGTCCACACTGCCGGGGAGAGGTGGCAAGAGACAAATCAACTCTAGTGGAAACGG 300
379 AtttAGATGTTttTGCGTTGtAGTAGGTttAtAtTGtCGGGGAGAGGTGGtAAGAGAtAAATtAAtTtTAGTGGAAACGG 300
379                                ATATTGTTGGGGAGAG                                  300
379                               TATATTGTCGGGGAGA                                   300

299 GAAGCGAGTGGGAACAACAGCCTCACAGCAAGACCGGGGCTCAGCAGCCGCGCTCTTGGCCTGCGTTTGGTGGACCGGAA 220
299 GAAGCGAGTGGGAAtAAtAGttTtAtAGtAAGAtCGGGGtTtAGtAGtCGCGtTtTTGGttTGCGTTTGGTGGAtCGGAA 220
299                                             AGTAGTTGTGTTTTTGG                ATTGGAA 220
299                                            TAGTAGTCGCGTTTTT                 GATCGGAA 220

219 TCCGGGTTGTGTGTTTCATTTTCTTTTTTGCCAGAAAATATCATTAGGAACTAAACGGCTCTTTTTAAGAGTGTGAAAGT 140
219 TtCGGGTTGTGTGTTTtATTTTtTTTTTTGttAGAAAATATtATTAGGAAtTAAACGGtTtTTTTTAAGAGTGTGAAAGT 140
219 TTTGGGTTG                                                                        140
219 TTCGGGTT
```

```
139 GTTACTCTTGCCAGTCACCAATGAGGAGGGCGCCAAAAGCTTCCCAGAGCCAGTTCCCACAGCCAGGACTATTCTCTAAA 60
139 GTTAtTtTTGttAGTtAttAATGAGGAGGGCGttAAAAGtTTtttAGAGttAGTTtttAtAGttAGGAtTATTtTtTAAA 60

59 GCCAGAGGTCGGAAAGTTGAACAGAACGGAAGCAAATTGCCTGGCAGAGGGAAGAGGCA -20
59                                       GTTTGGTAGAGGGAAGAGGTA -20
59 GttAGAGGTCGGAAAGTTGAAtAGAACGGAAGtAAATTGttTGGtAGAGGGAAGAGGtA -20
```

Figure 19: Amplificate of SEQ ID NO: 17

```
  1 AAGAGTCCCAGGAAATGTGCCCCCCGGGATTACTGGTATTTGCTGGCTCCTCGGAACAAGATGCCAACTTGGCTAAGCAG  80
  1 AAGAGTTTTAGGAAATGTGTTTTT                                                          80
  1 AAGAGTtttAGGAAATGTGtttttCGGGATTAtTGGTATTTGtTGGtTttTCGGAAtAAGATGttAAtTTGGtTAAGtAG  80
  1                                                 TTGGTTTTTCGGAATAA                 80
  1                                                   GGTTTTTTGGAATAAGAT              80

 81 TTCTGGATCTCGGCGTCGATGTATCCCCCTAGCGAATCTCAGCTGGTGCTGCGCAGAGACAGCAGTCAGCGTCTGCCGGT 160
 81 TTtTGGATtTCGGCGTCGATGTATtttttTAGCGAATtTtAGtTGGTGtTGCGtAGAGAtAGtAGTtAGCGTtTGtCGGT 160
 81       TTCGGCGTCGATGTAT                                  GGTGTTGTGTAGAGAT  TAGTTAGTGTTTGTTGG 160
 81       TTTGGTGTTGATGTATT                                  GTGTTGCGTAGAGATA  AGTTAGCGTTTGTCGG

161 GGCGCGGCCCAGGAGGAGCAGAGGGTCTGGTGAGTAGAA                                         240
161               GTAGAGGGTTTGGTGAGTAGAA                                            240
161 GGCGCGGtttAGGAGGAGtAGAGGGTtTGGTGAGTAGAA                                         240
```

Figure 20: Amplificate of SEQ ID NO: 18

```
1 CAAGGCAAGGGAAGGCCAGAAACGAGGCAACCCGTGCACAGACCGGGCTTGGTCAACGCCCCAGGGGCGGGGCCAGGCGG 80
1 TAAGGTAAGGGAAGGTTAGAAA 80
1 tAAGGtAAGGGAAGGttAGAAACGAGGtAAttCGTGtAtAGAtCGGGtTTGGTtAACGttttAGGGGCGGGGttAGGCGG 80
1 AATTCGTGTATAGATCGG 80
1 ATTTGTGTATAGATTGGG 80

81 GCCGTTCCTGGGGGGCGGGGCCAGCCTCGGTCCAATAAGGGGTACTCGACGCCCCATTGGCCACCTGCCTCGAAAGGGGG 160
81 GtCGTTttTGGGGGGCGGGGttAGttTCGGTttAATAAGGGGTAtTCGACGttttATTGGttAttTGttTCGAAAGGGGG 160
81 AGGGGTATTTGATGTTT 160
81 GGGTATTCGACGTTTT 160

161 AAGACAGTCTGGGCGGGCAGGACGTGCGGAGGGAGAGGCAGCGGTGGCGCGAGGCTCAACTCGAAGCGCTATTGGTGGGA 240
161 AAGAtAGTtTGGGCGGGtAGGACGTGCGGAGGGAGAGGtAGCGGTGGCGCGAGGtTtAAtTCGAAGCGtTATTGGTGGGA 240
161 ATTCGAAGCGTTATTG 240
161 TTTGAAGTGTTATTGGT 240

241 CTGATAGTCTTGTGCGCCAAGAAGCTGGCAGAAGGGAAGGGGCGGGACATCAGTTCAGGATCTCAAACCGCATTGGTCGT 320
241 tTGATAGTtTTGTGCGttAAGAAGtTGGtAGAAGGGAAGGGGCGGGAtATtAGTTtAGGATtTtAAAtCGtATTGGTCGT 320
241 AAATCGTATTGGTCGT 320
241 AAATTGTATTGGTTGT 320
```

```
321 CTGCCTCGGCTGAGAGAGGCGATGCTTGAAGTTCATTGGTCTTTGTGAGACAGGGTAAGAAAAGCAGCGCGAGCTTGGCG 400
321 tTGttTCGGtTGAGAGAGGCGATGtTTGAAGTTtATTGGTtTTTGTGAGAtAGGGTAAGAAAAGtAGCGCGAGtTTGGCG 400
321                                                                   AAAAGTAGCGCGAGTT        400
321 TT                                                                   AGTAGTGTGAGTTTGG

401 GTTCCTCTGGCCACTTTCTCACAGTGTCTTTGGGCGTCTTCTTGACTGAATCTGACTCCATTGGAGGCTGTGGTAAC    480
401                                                              TTTTATTGGAGGTTGTGGTAAT    480
401 GTTttTtTGGttAtTTTtTtAtAGTGTtTTTGGGCGTtTTtTTGAtTGAATtTGAtTttATTGGAGGtTGTGGTAAt    480
```

Figure 21: Amplificate of SEQ ID NO: 19

```
  1 CTATTATTTCAGATGGAGTGAGGTTGCACGACTGGGATGGAAGAAAGGAATCCCTTAAATTTGGGGGAATTTCTGTTCTC  80
  1 TTATTATTTTAGATGGAGTGAGGTT                                                        80
  1 tTATTATTTtAGATGGAGTGAGGTTGtACGAtTGGGATGGAAGAAAGGAATtttTTAAATTTGGGGGAATTTtTGTTtTt  80

 81 TGTTCTAAGACCATTTTACTTGGGGTGTGGGGGTGGGCGCGGCGGTCAGGGCAGTGGAACGCAGTCGCGGCTGCGCCATC 160
 81 TGTTtTAAGAttATTTTAtTTGGGGTGTGGGGGTGGGCGCGGCGGTtAGGGtAGTGGAACGtAGTCGCGGtTGCGttATt 160
 81                                                                GGAATGTAGTTGTGGT  160
 81                                                                 GAACGTAGTCGCGGTT 160

161 CCTGCACTTCCAGGCGCGCGGGAGGGACCGGCGGGGACGCGAGCTGCGGACTCTGGCGAACTCGGGGGAGGCAGACAGGG 240
161 ttTGtAtTTttAGGCGCGCGGGAGGGAtCGGCGGGGACGCGAGtTGCGGAtTtTGGCGAAtTCGGGGGAGGtAGAtAGGG 240
161                                                           ATTTTGGCGAATTCGG       240
161                                                               TGGTGAATTTGGGGGA   240

241 GGAGGCGGACACCCAGCCGGCAGGCGTCTCAGCCTCCCCGCAGCCGGCGGGCTTTTCTCCTGACAGCTCCAGGAAAGGCA 320
241 GGAGGCGGAtAtttAGtCGGtAGGCGTtTtAGttTtttCGtAGtCGGCGGGtTTTTtTttTGAtAGtTttAGGAAAGGtA 320
241          ATTTAGTTGGTAGGTGT     TTTTTTGTAGTTGGTGG                                 320
241            TTAGTCGGTAGGCGTT     TTTTCGTAGTCGGCGG                                 320
```

321 GACCCCTTCCCCAGCCAGCCAGGTAAGGTAAAGACTGCTGTTGAGCTTGCTGTTACTGAGGGCGCACAGACCCTGGGGAG 400
321 GAttttTTttttAGttAGttAGGTAAGGTAAAGAtTGtTGTTGAGtTTGtTGTTAtTGAGGGCGtAtAGAtttTGGGGAG 400
321 ATTGAGGGCGTATAGA GGGGAG 400
321 TGAGGGTGTATAGATTT GGAG 400

401 ACCGAAGCTTGCCACTGCGGGATTCTGTGGGGTAACCTGGGT 480
401 **TTGTGGGGTAATTTGGGT** 480
401 AtCGAAGtTTGttAtTGCGGGATTtTGTGGGGTAAttTGGGT 480
401 ATTGAAGTTT 480
401 ATCGAAGTTTGT

Figure 22: Amplificate of SEQ ID NO: 20

```
  1 AGATGTTTTACATCTGTCTGGGAATCCCGATTTTCCAGGCTCCCTGAGAAATCTATCTGCCTGGAAGAAGTTGCACTTCG  80
  1 AGATGTTTTATATTTGTTTGGGA                                                          80
  1 AGATGTTTTAtATTtGTtTGGGAATttCGATTTTttAGGtTtttTGAGAAATtTATtTGttTGGAAGAAGTTGtAtTTCG  80
  1                                                                       TTGTATTTCG  80
  1                                                                            TTTTG  80

 81 TCGCCACAGGAGCCGCCGAGAGCCAGGGATGGAGATTTCAGACTTAGGACTCTGCCACGATTCTACTGATCCTTAAGCGC 160
 81 TCGttAtAGGAGtCGtCGAGAGttAGGGATGGAGATTTtAGAtTTAGGAtTtTGttACGATTtTAtTGATttTTAAGCGt 160
 81 TCGTTAT                                                                  TAAGCGT 160
 81 TTGTTATAGGAGT                                                              AGTGT 160
 81       ATAGGAGTCGTCGAGA                                                           160
 81       ATAGGAGTTGTTGAGAG                                                          160

161 CAACAGAACGAAATGGACATGCCTATTAGCTACCACAAATCGAATTATTTATATGAGAGAGAGAAATGTGGTTCAAGTT 240
161 tAAtAGAACGAAATGGAtATGttTATTAGtTAttAtAAATCGAATTATTTATATGAGAGAGAGAAATGTGGTTtAAGTT 240
161 TAATAGAACGA                                                                      240
161 TAATAGAATGAAAT
```

```
241 CCACATTTGTCCTTTAAATAATCGACCCTCCCTAACCCTGTCCCCAAAGGCTCGTGGAAATTAGGGAGGGGGTTGGGGAG 320
241 ttAtATTTGTttTTTAAATAATCGAtttTtttTAAtttTGTttttAAAGGtTCGTGGAAATTAGGGAGGGGGTTGGGGAG 320

321 ACGGTTCCAGAAACAAAACGCTTTCCTCCAGCACGGCCTCTACATCCAGTCCACCCAAGCTGATTTTTGTAACTCATATA 400
321 ACGGTTttAGAAAtAAAACGtTTTttTttAGtACGGttTtTAtATttAGTttAtttAAGtTGATTTTTGTAAtTtATATA 400

401 AAGAATGGGCCCGGGTGGGTGGTCAGTGAAGACTGGGGTGAGCA                                     480
401                         TAGTGAAGATTGGGGTGAGTA                                    480
401 AAGAATGGGttCGGGTGGGTGGTtAGTGAAGAtTGGGGTGAGtA                                     480
```

Figure 23: Amplificate of SEQ ID NO: 21

```
  1 TAAAAATGGAAGGGAAGACAGATCCAATTTGAAATCCTCCTTGAGAAAAAACAAATCAAAACTAGTTCCTGGGGAAGAAA 80
  1 TAAAAATGGAAGGGAAGATAGA                                                           80
  1 TAAAAATGGAAGGGAAGAtAGATttAATTTGAAATttTttTTGAGAAAAAAtAAATtAAAAtTAGTTttTGGGGAAGAAA 80

 81 GCCTCAGCTAGGTCAGGAGGAAACTTACGCATCTTTGATTCCCTTTCCGCTTTGGAGAGGCATTACCCGTTCAAGCCCAG 160
 81 GttTtAGtTAGGTtAGGAGGAAAtTTACGtATtTTTGATTtttTTTtCGtTTTGGAGAGGtATTAttCGTTtAAGtttAG 160
 81                                                                               AG 160
 81                                                                                  160

161 CCAATGCGGCCGGCCAGGATTTACAGCTCTTATCAAGGGTTAGATTTTGCGAAAGATATAAAGAAAGGAGTTTCCTAACA 240
161 ttAATGCGGtCGGttAGGATTTAtAGtTtTTATtAAGGGTTAGATTTTGCGAAAGATATAAAGAAAGGAGTTTttTAAtA 240
161 TTAATGTGGTTGGT                         TTAGATTTTGTGAAAGATA                       240
161 TTAATGCGGTCGGTTA                         AGATTTTGCGAAAGATA                       240

241 CACCGGGTCTTCTCACAGCAACAAGACACCGAAATTAAGCCTTCTATGGTTTGTGTCTGTAGGACTTTTTAGATAAAACT 320
241 tAtCGGGTtTTtTtAtAGtAAtAAGAtAtCGAAATTAAGttTTtTATGGTTTGTGTtTGTAGGAtTTTTTAGATAAAAtT 320
241                       AAGATATCGAAATTAAGTT                                        320
241                       AAGATATTGAAATTAAGTTT
```

```
321 GGCAGCCCCATTAGATAAGAAATGGCTTTTTAGAAGCTTTGGGGGAAGCGCGGGGGTTCTTCGTGGCCTTCCTGTGACTG 400
321                                                                         TGTGATTG 400
321 GGtAGttttATTAGATAAGAAATGGtTTTTTAGAAGtTTTGGGGGAAGCGCGGGGGTTtTTCGTGGttTTttTGTGAtTG 400

401 TCTTTGGGAGAC                                                                     480
401 TTTTTGGGAGAT                                                                     480
401 TtTTTGGGAGAt                                                                     480
```

Figure 24: Amplificate of SEQ ID NO: 22

```
  1 TTGTGGTCACTCTGAGATGGCAGATTGGGGTCCCTGTTGTCCTTGGACAGATGAGAATGCCGAGAGCTCGTATGCCTTGC 80
  1 TTGTGGTTATTTTGAGATGGTA                                                           80
  1 TTGTGGTtAtTtTGAGATGGtAGATTGGGGTtttTGTTGTttTTGGAtAGATGAGAATGtCGAGAGtTCGTATGttTTGt 80
  1                                                             ATGTCGAGAGTTCGTA     80
  1                                                               GTTGAGAGTTTGTATGT  80

 81 CCAAGGGCACACAGCAAGGCCGGGTGCCCATGCGGCTGTCCCAGGGACCCACTGACCCTGCTGTCCCCCTCAGGCCACAT 160
 81 ttAAGGGtAtAtAGtAAGGtCGGGTGtttATGCGGtTGTtttAGGGAtttAtTGAtttTGtTGTtttttTtAGGttAtAT 160

161 TAGGATCTCAGACCTGGGCTTGGCTGTGAAGATCCCCGAGGGAGACCTGATCCGCGGCCGGGTGGGCACTGTTGGCTACA 240
161 TAGGATtTtAGAttTGGGtTTGGtTGTGAAGATtttCGAGGGAGAttTGATtCGCGGtCGGGTGGGtAtTGTTGGtTAtA 240
161                               AGATTTTCGAGGGAGA                                   240
161                               AGATTTTTGAGGGAGAT                                  240
161                                               ATTTGATTTGTGGTTGG                  240
161                                                TTTGATTCGCGGTCGG                  240
```

```
241 TGGGTGAGTGCTGGGCTGCCTGTGTCAATGCACCTTGAGACCCACCACCTGCTCACCGCCGGCCGAGCCCCCAGAGCCTG 320
241 TGGGTGAGTGtTGGGtTGttTGTGTtAATGtAttTTGAGAtttAttAttTGtTtAtCGtCGGtCGAGttttAGAGttTG 320
241                                                    TTTATTGTTGGTTGAGT          320
241                                                     TATCGTCGGTCGAGTT

321 CCCGCAGAGCCTCTCGCCTCTTCATGCACTGCTGATCACACACAGAGTCACAGTCTCTGCAGGGCTGCTGGGGGGCGGCC 400
321                                                                              TT 400
321 ttCGtAGAGttTtTCGttTtTTtATGtAtTGtTGATtAtAtAtAGAGTtAtAGTtTtTGtAGGGtTGtTGGGGGGCGGtt 400

401 TTAGCCAGGGGGAGGG                                                                 480
401 TTAGTTAGGGGGAGGG                                                                 480
401 TTAGttAGGGGGAGGG                                                                 480
```

Figure 25: Amplificate of SEQ ID NO: 23

```
  1 TCCACTCACCAGGTGAAGAGTTTGCCTTTTATCCTGCGCAGCAGGCTGGAGAACTCGGCCGCCGCTCCCTCCGCGAGGGG  80
  1 TTTATTTATTAGGTGAAGAGTTTGTT                                                        80
  1 TttAtTtAttAGGTGAAGAGTTTGttTTTTATttTGCGtAGtAGGtTGGAGAAtTCGGtCGtCGtTtttTtCGCGAGGGG  80
  1                                                  AGAATTTGGTTGTTGTT                80
  1                                                    AATTCGGTCGTCGTTT               80

 81 CTCTGGGGCGCCGGGGCCCGCTGGCGAGTCTGCCTCCATCGGCGCCCTTGCGCGTCTCCGGCGCCCAAAACCCCCGAAGT 160
 81 tTtTGGGGCGtCGGGGttCGtTGGCGAGTtTGttTttATCGGCGtttTTGCGCGTtTtCGGCGtttAAAAttttCGAAGT 160
 81                                                         TTTTTGGTGTTTAAAATTT      160
 81                                                          TTTCGGCGTTTAAAAT        160
 81                                                                       ATTTTCGAAGT 160
 81                                                                          TTTGAAGT 160

161 GCCGGGTCCCGGAAGACGGGAGGCGGTGCCTGTGCGGCCGGCTGGGGCTCCGCTCTGGCCCTGCGCTCCCTCCCTCGCTC 240
161 GtCGGGTttCGGAAGACGGGAGGCGGTGttTGTGCGGtCGGtTGGGGtTtCGtTtTGGtttTGCGtTtttTtttTCGtTt 240
161       TTTCGGAAGACGGGAG                                                           240
161       TTTTGGAAGATGGGAG                                                           240
161 GTCGG                                                                            240
161 GTTGGGTT
```

```
241 TCTTGCTCGCGTCCCTCTCGCTCCTCCGCTCGGCTCCTTCAGCGACCAATCATTAACTGTCAAGCCCTAAGGGCCAGACA 320
241 TtTTGtTCGCGTtttTtTCGtTttTtCGtTCGGtTttTTtAGCGAttAATtATTAAtTGTtAAGtttTAAGGGttAGAtA 320
```

```
321 ATACATTTGCAGATTACAACCTGGCACCTCCAGGGTGGCAGGAGGA                                   400
321                                         TTTTAGGGTGGTAGGAGGA                                   400
321 ATAtATTTGtAGATTAtAAttTGGtAttTttAGGGTGGtAGGAGGA                                   400
```

Figure 26: Amplificate of SEQ ID NO: 24

```
492 TTGGAGAGATGTGCTGGCCAGGACCCGCTGACACAGGTATTCTGGTTGCGCCCCCACCTCACCCGGTGCCCGAGAGCGAG 413
492 TTGGAGAGATGTGTTGGTTAG                                                             413
492 TTGGAGAGATGTGtTGGttAGGAttCGtTGAtAtAGGTATTtTGGTTGCGtttttAttTtAttCGGTGttCGAGAGCGAG 413
492                                                                     TGTTTGAGAGTGAG 413
492                                                                    GTGTTCGAGAGCGAG 413
492                                                            TATTTGGTGTTTGAGAG       413
492                                                          TTTATTCGGTGTTCGA          413

412 TGTGAGTGTATCGATTGTTTCAATTGATTTTTTTAAAGCTAGATAGCAGAGGCTGCGTGTCTGTACACGCAGATATATAC 333
412 TGTGAGTGTATCGATTGTTTtAATTGATTTTTTTAAAGtTAGATAGtAGAGGtTGCGTGTtTGTAtACGtAGATATATAt 333
412 TGT                                                                              333
412 T                                                                                333

332 ATACACAAATACAGATACTCAAGCGCACACAGATTACACGTACAGAGTCACAGACAAGGACACACACACACACATCGTGG 253
332 ATAtAtAAATAtAGATAtTtAAGCGtAtAtAGATTAtACGTAtAGAGTtAtAGAtAAGGAtAtAtAtAtAtAtATCGTGG 253
332                                                                          ATTGTGG 253
332                                                                         TATCGTGG 253
```

252 TCCCTCACGCACACATACCTCCAAAGAGCCTTGGGTAACCTGGTGAGGTCCCTGAGTGCTGCCCAAGATGTGTGTGTGGG 173
252 TttttTtACGtAtAtATAttTttAAAGAGttTTGGGTAAttTGGTGAGGTtttTGAGTGtTGtttAAGATGTGTGTGTGGG 173
252 TTTTTTATGTATA 173
252 TTTTTTACGTAT 173

172 TGGGGACGCAGGGACGGAGAAAGGGGAGTGTGAAGCAGGATTTCCAGCAGGTCAAGTGCCGCTGATTCTCCTGCCCCCGA 93
172 TGGGGACGtAGGGACGGAGAAAGGGGAGTGTGAAGtAGGATTTttAGtAGGTtAAGTGtCGtTGATTtTttTGttttCGA 93
172 GACGTAGGGACGGAGA 93
172 GATGTAGGGATGGAGA

92 CCCCAAATCCTTCCCTTTCTCCTCCTAGGGAGGTCTGAACCAGAAATCCCAAACCTGGGCTTGTTTCCACATCCACAGAG 13
92 **ATTTATAGAG** 13
92 ttttAAATttTTttttTTTtTttTttTAGGGAGGTtTGAAttAGAAATtttAAAttTGGGtTTGTTTttAtATttAtAGAG 13

12 GGGAGGAGTCCT -67
12 **GGGAGGAGTTTT** -67
12 GGGAGGAGTttT -67

Figure 27: Amplificate of SEQ ID NO: 25

```
407 TCTCCAGAACAAAGGACTTTAGGGCCCAAATTCCGTTTATTCAGTACTCCAAGTCCTAAAAACTTGGAATATCTGATGAA 328
407 TTTTTAGAATAAAGGATTTTAGGG                                                         328
407 TtTttAGAAtAAAGGAtTTTAGGGtttAAATTtCGTTTATTtAGTAtTttAAGTttTAAAAAtTTGGAATATtTGATGAA 328

327 TAAAAGTGGCCGCTCCCCAGGCTGTCTCTTGAGAGAAGCCACCGGCACAGCTGACCTTGCCCGCTCCATCGCGTCACTGA 248
327 TAAAAGTGGtCGtTttttAGGtTGTtTtTTGAGAGAAGttAtCGGtAtAGtTGAttTTGttCGtTttATCGCGTtAtTGA 248

247 CCGCTCCTCAGACAGATGCGTCAGGCATCTCCGGCGGCCGCTCCACTCTGCGCCAGACTCGCTGCAGCAGCGGCAGGCTT 168
247 tCGtTttTtAGAtAGATGCGTtAGGtATtTtCGGCGGtCGtTttAtTtTGCGttAGAtTCGtTGtAGtAGCGGtAGGtTT 168
247                                                      TGTGTTAGATTTGTTGT   TAGCGGTAGGTTT 168
247                                                    TTTGCGTTAGATTCGT      TAGTGGTAGGTTT 168

167 CGCACACATCCCCGCCTGAGCATGCGCGCCAGCCTGCCTCTGCGGCCGCGCAGGCGTGCTTGTTTGCCGCAGTGCAGGGG 88
167 CGtAtAtATtttCGttTGAGtATGCGCGttAGttTGttTtTGCGGtCGCGtAGGCGTGtTTGTTTGtCGtAGTGtAGGGG 88
167 CGTA            AGTATGCGCGTTAGTT    TTTTGTGGTTGTGTAGG                              88
167 TGTA          TGAGTATGTGTGTTAGT    TTTTTGCGGTCGCGTA
```

```
87 TCCCAGCTCCCTCCCTCACCGGAATGACCTGGGGGGAGGGGGCTACTGGACCCCTAGGGCCCCACAGCACTGTTGCAATG  8
87                                                                AGTATTGTTGTAATG  8
87 TtttAGtTtttTtttTtAtCGGAATGAttTGGGGGGAGGGGGtTAtTGGAttttTAGGGttttAtAGtAtTGTTGtAATG  8

 7 AGAGGGG                                                                        -72
 7 AGAGGGG                                                                        -72
 7 AGAGGGG                                                                        -72
```

Figure 28: Amplificate of SEQ ID NO: 26

```
  1 TCCCAGGAATGCAGGACACAAGGGGGCAGCCTACCTCAGGGCAGGGCCAGCGGGGTTCCTCATCCTTAGGCCAGTCCCCG  80
  1 TTTTAGGAATGTAGGATATAAGGG                                                          80
  1 TtttAGGAATGtAGGAtAtAAGGGGGtAGttTAttTtAGGGtAGGGttAGCGGGGTTttTtATttTTAGGttAGTtttCG  80

 81 GGGCTCAGATGATTCCAGGGAAGGCCGACCCGGCTGACTGCCACCTCCACCATCACTCTGCAGAGCGGTTGAGCATGAAA 160
 81 GGGtTtAGATGATTttAGGGAAGGtCGAttCGGtTGAtTGttAttTttAttATtAtTtTGtAGAGCGGTTGAGtATGAAA 160
 81                     AAGGTTGATTTGGTTG                                             160
 81                        GTCGATTCGGTTGATT                                          160

161 GACCAGCTGATCGCACAGAGCCTCCTAGAGAAACAGCAGATCTACCTGGAGATGGCCGAGATGGGCGGCCTCGAAGACCT 240
161 GAttAGtTGATCGtAtAGAGttTttTAGAGAAAtAGtAGATtTAttTGGAGATGGtCGAGATGGGCGGttTCGAAGAttT 240
161                                                           AGATGGGTGGTTTTGA       240
161                                                               GGCGGTTTCGAAGATT   240

241 GCCCCAGCCCCGAGGCCTATTCCGTGGAGGGGACCCATCCGAGACCCTGCAGGGGGAGCTAATTCTCAAGTCGGCCATGA 320
241 GttttAGtttCGAGGttTATTtCGTGGAGGGGAtttATtCGAGAtttTGtAGGGGGAGtTAATTtTtAAGTCGGttATGA 320
241                                                                     AAGTCGGTTATGA 320
241                                                                     AAGTTGGTTATGA 320
```

```
321 GCGAGAGTAAGTTGGCTGCCCACACCTCAAGGGTGCAGTCTTGCCGGGGTGGGCTCCTCAGGGAACCCCAGGCCAGGCTC 400
321 GCGAGAGTAAGTTGGtTGtttAtAttTtAAGGGTGtAGTtTTGtCGGGGTGGGtTttTtAGGGAAttttAGGttAGGtTt 400
321 GCGA                                                                             400
321 GTGA                                                                             400

401 ACGGCTCATTGTGGCCGACACGGCACCCTGTCCAGGACAGGCTTCTATGTGGGG                           480
401                                                   GGATAGGTTTTTATGTGGGG           480
401 ACGGtTtATTGTGGtCGAtACGGtAtttTGTttAGGAtAGGtTTtTATGTGGGG                           480
401            TTGTGGTTGATATGGT                                                      480
401                TGGTCGATACGGTATT
```

Figure 29: Amplificate of SEQ ID NO: 27

```
273 CAGGGTCTGATGATGTGATTTTCACCCCGCCGCATGTGAAGGCCACAGAGCAGATCATTCAGCTCACGCTGAAGGGCACA 194
273 TAGGGTTTGATGATGTGATTTT                                                           194
273 tAGGGTtTGATGATGTGATTTTtAtttCGtCGtATGTGAAGGttAtAGAGtAGATtATTtAGtTtACGtTGAAGGGtAtA 194
273                                     ATTTTGTTGTATGTGAAG                            194
273                                     ATTTCGTCGTATGTGA                              194

193 CGGGCGCTCAGGGCAGCAGAGAGCAGAGGACGCGACAGGGATGTGTGATGCTGGCTCAGGCCTGGGCATCCGAGTTGATG 114
193 CGGGCGtTtAGGGtAGtAGAGAGtAGAGGACGCGAtAGGGATGTGTGATGtTGGtTtAGGttTGGGtATtCGAGTTGATG 114
193                                        AGGACGCGATAGGGAT                           114
193                                        AGGATGTGATAGGGAT                           114

113 AGCGCTGGCTGGCGTTCAGCGCCTGGAAACTGCACACGCGCGCTGGGCCGCTTGCGCATTTAAGGAAGTGCAGCCTCATC  34
113 AGCGtTGGtTGGCGTTtAGCGttTGGAAAtTGtAtACGCGCGtTGGGtCGtTTGCGtATTTAAGGAAGTGtAGttTtATC  34
113                                                          GGTTGTTTGTGTATTTAA       34
113                                          ATTGTATACGCGCGTT GTCGTTTGCGTATTTA        34
113                                           TTGTATATGTGTGTTGG

 33 GGAAGAGGCCTGCCAGTGCTGAGCCAGGAGGGA                                                -46
 33           TTAGTGTTGAGTTAGGAGGGA                                                  -46
 33 GGAAGAGGttTGttAGTGtTGAGttAGGAGGGA                                                -46
```

Figure 30: Amplificate of SEQ ID NO: 28

```
  1 GAGAGGGAGGGCCAAGGTCCGGGCGCCGAGAGGAGGGGCCCGGGGGGCCGGCTGGAGGGGGCGAGGCGCTAGGCGGCTTT  80
  1 GAGAGGGAGGGTTAAGGTT                                                               80
  1 GAGAGGGAGGGttAAGGTtCGGGCGtCGAGAGGAGGGGttCGGGGGGtCGGtTGGAGGGGGCGAGGCGtTAGGCGGtTTT  80

 81 GCTTCCCGTCCCCGTCAATTGGCCGCTAGCTCTGTTTTGACTGCCTGTACAAAGCGAGTAGACGGCGGGGGATGGGGCGA 160
 81 GtTTttCGTtttCGTtAATTGGtCGtTAGtTtTGTTTTGAtTGttTGTAtAAAGCGAGTAGACGGCGGGGGATGGGGCGA 160
 81             GTTAATTGGTTGTTAGTT                TATAAAGTGAGTAGATGG                   A 160
 81                AATTGGTCGTTAGTTTT                 TAAAGCGAGTAGACGG                    160

161 TTAGGGACCGGATTTGGGAAAGGAGGCAGCAGC                                                240
161            ATTTGGGAAAGGAGGTAGTAGT                                                240
161 TTAGGGAtCGGATTTGGGAAAGGAGGtAGtAGt                                                240
161 TTAGGGATTGGATTTG                                                                 240
161 TTAGGGATCGGATTTG
```

Figure 31: Amplificate of SEQ ID NO: 29

356 GTGGAAAAAGGAGAGCAAACTGCTCAGCGCGTCTCAGCTCAGTGCCGAGGAGGAGGAAGAAAAACAGGCCGAGTGAAGGT 277
356 **GTGGAAAAAGGAGAGTAAATTG** 277
356 GTGGAAAAAGGAGAGtAAAtTGtTtAGCGCGTtTtAGtTtAGTGtCGAGGAGGAGGAAGAAAAAtAGGtCGAGTGAAGGT 277

276 GCTGGAAAGGGAGGGAGGACGCGAGGGGAAAGGCCTGTGGGGAGCCGAGGGCGTCAGAGAGACCCGGGAAGGAAGGCTCT 197
276 GtTGGAAAGGGAGGGAGGACGCGAGGGGAAAGGtTtGTGGGGAGtCGAGGGCGTtAGAGAGAttCGGGAAGGAAGGtTtT 197
276 AGTCGAGGGCGTTAGA 197
276 AGTTGAGGGTGTTAGA 197

196 CGGGTGGGGGAGCCAGGAGACCTGCTCTCCGGCGCAGACAGGCGGGGCCCAGCGCTCTCCTGGACGCCCCCGCCCGCACA 117
196 CGGGTGGGGGAGttAGGAGAttTGtTtTtCGGCGtAGAtAGGCGGGGtttAGCGtTtTttTGGACGttttCGttCGtAtA 117
196 TTTTTCGGCGTAGATA ATA 117
196 TGTTTTTTGGTGTAGAT TATA 117

116 GCTCCCGGCGGGTGCTCTGAGGCCTCACTACTCGAGCCCACCCAGCATCCCGCGCGCCCTTCCTTCCCGAGGAACTCGCC 37
116 GtTttCGGCGGGTGtTtTGAGGtTtAtTAtTCGAGtttAtttAGtATttCGCGCGtttTTttTTttCGAGGAAtTCGtt 37
116 GTTTTCGGCGGGT TTTTTTGAGGAATTTGTT 37
116 GTTTTTGGTGGGT TTTTCGAGGAATTCGT

| | | |
|---|---|---|
| 36 | TCAGCCTGATCAGGCTTCCTGGTGAGAACTGAGGAG | -43 |
| 36 | **TTTTGGTGAGAATTGAGGAG** | -43 |
| 36 | TtAGttTGATtAGGtTTttTGGTGAGAAtTGAGGAG | -43 |

Figure 32: Amplificate of SEQ ID NO: 30

```
420 AGGGAGGCCTGGTGCATTCCTCCATGGGGGTGGGGAATGCGGAGGCTGGGCCACCGGAGGTGGTGGCGGTGGCAGGGGCG 341
420 AGGGAGGTTTGGTGTATTTT                                                             341
420 AGGGAGGttTGGTGtATTttTttATGGGGGTGGGGAATGCGGAGGtTGGGttAtCGGAGGTGGTGGCGGTGGtAGGGGCG 341

340 GCAGGAGACGCCACGTCAGGGTCACTGCCCCACTCGGTGCACAGCAGGGGGCCGGCAGTTCACCGGATCACCGCGGGGGT 261
340 GtAGGAGACGttACGTtAGGGTtAtTGttttAtTCGGTGtAtAGtAGGGGGtCGGtAGTTtAtCGGATtAtCGCGGGGGT 261
340     AGATGTTATGTTAGGGT                                          ATTGGATTATTGTGGGG    261
340  TAGGAGACGTTACGTT                                          TATCGGATTATCGCGG      261
340                                                   GTCGGTAGTTTATCGGAT                261
340                                                   GTTGGTAGTTTATTGGAT                261

260 GGGGCGGAGGACGGAAGTTGGTGAGGGGGGTGGTCTACAGGGCACCTCCCGTCCCAGGGCTCTAACCTAATTCGAAAGGG 181
260 GGGGCGGAGGACGGAAGTTGGTGAGGGGGGTGGTtTAtAGGGtAttTttCGTtttAGGGtTtTAAttTAATTCGAAAGGG 181
260                                                                   TTAATTTGAAAGGG 181
260                                                                  TTTAATTCGAAAGGG 181
```

| | | |
|---|---|---|
| 180 | AAAGGAGTGCTGCAGCCGGGAGGCCAGGCCGACTTTGGGTCCTGCCCTCGCAGAAGCCCCCACGCCTGGTGCAGACCAAT | 101 |
| 180 | AAAGGAGTGtTGtAGtCGGGAGGttAGGtCGAtTTTGGGTttTGtttTCGtAGAAGtttttACGttTGGTGtAGAttAAT | 101 |
| 180 | AAAG | 101 |
| 180 | AA | |
| 100 | GACTCGCTTCCTGCCCTTACACCGATCACACATGACCTCGGTCACAGGGGTGCCACATCACAGGAGTGCCCCTCACTCCT | 21 |
| 100 | **TT** | 21 |
| 100 | GAtTCGtTTttTGtttTTAtAtCGATtAtAtATGAttTCGGTtAtAGGGGTGttAtATtAtAGGAGTGttttTtAtTttT | 21 |
| 20 | GCCTGCAGGAAGCAGGGCCC | -59 |
| 20 | **GTTTGTAGGAAGTAGGGTTT** | -59 |
| 20 | GttTGtAGGAAGtAGGGttt | -59 |

Figure 33: Amplificate of SEQ ID NO: 31

```
  1 AAGGCACTACTCCTGGGGTTCCCCGAAGCGGATCCTGGGACTGGGACCTGGGGACGCGGTGAGAGGCAGCCCTGAAGCTG  80
  1 AAGGTATTATTTTTGGGGTTTT                                                            80
  1 AAGGtAtTAtTttTGGGGTTttCGAAGCGGATttTGGGAtTGGGAttTGGGGACGCGGTGAGAGGtAGtttTGAAGtTG  80
  1                   TTTTTCGAAGCGGATT                                                80
  1                      TTTGAAGTGGATTTTGG                                            80

 81 AGTCCAGAGGTCTGGGGCGTCATTGTCCCAGGGCGGACCCAGCCCCAGACCCTACAACTAAACCCAAGGCCAGCCGAGAA 160
 81 AGTttAGAGGTtTGGGGCGTtATTGTtttAGGGCGGAtttAGttttAGAtttTAtAAtTAAAtttAAGGttAGtCGAGAA 160
 81                                                                   AGGTTAGTCGAGAA 160
 81                                                                   AGGTTAGTTGAGAA 160

161 GCACTACCACTGGCGGGCTGAGCTGAGCTCTCATTCTTCTTAGGACTCTGGACCTTTGGAGGAAATGAAAGGGACCCAGT 240
161 GtAtTAttAtTGGCGGGtTGAGtTGAGtTtTtATTtTTtTTAGGAtTtTGGAttTTTGGAGGAAATGAAAGGGAtttAGT 240
161 GT                                                                               240
161 GTA                                                                              240
```

```
241 TGGAAATATAGTCTTTCTTCGCGCCCCCTACATCTCTCTCCCCCGGCGGAAACGCGGCCCAAACTGTTACGAAGTGGAAG 320
241                                                                         AAGTGGAAG 320
241 TGGAAATATAGTtTTTtTTCGCGtttttTAtATtTtTtTttttCGGCGGAAACGCGGtttAAAtTGTTACGAAGTGGAAG 320
241                                                   GGAAATGTGGTTTAAATT             320
241                                                    GAAACGCGGTTTAAAT              320
241                                                                ATTGTTACGAAGTGGA  320
241                                                                 TTGTTATGAAGTGGAAG

321 AGGCTGGATAGCT                                                                    400
321 AGGTTGGATAGTT                                                                    400
321 AGGtTGGATAGtT                                                                    400
```

Figure 34: Amplificate of SEQ ID NO: 32a

```
  1 GGGTGCTGGGGAGCCCCATGCGCCAGGAGGTGGATAATTTAGGGGGCAAGGGGCTCGCACAAGGGCGAAGATGCGCTCTC  80
  1 GGGTGTTGGGGAGTTTTA                                                                80
  1 GGGTGtTGGGGAGttttATGCGttAGGAGGTGGATAATTTAGGGGGtAAGGGGtTCGtAtAAGGGCGAAGATGCGtTtTt  80

 81 CTCCCGGCACCGACCCACGCGTTTAAAGGCCTCTGCGGCAACGTTTAGGGACGCGAACTAGGTGCCGGGGGTTGGGGAGG 160
 81 tTttCGGtAtCGAtttACGCGTTTAAAGGttTtTGCGGtAACGTTTAGGGACGCGAAtTAGGTGtCGGGGGTTGGGGAGG 160
 81                               TTTGCGGTAACGTTTA                                   160
 81                                TTGTGGTAATGTTTAGG                                  160
 81                                           TTAGGGACGCGAATTA                        160
 81                                             AGGGATGTGAATTAGG                      160

161 AGGTGGGACTGGAAGCGAGAGCAGTCTGCGCCCAAGACCGCGCAAGAGGTGGCCTTTTGTTCGCTGGCTCCAGCACATCC 240
161 AGGTGGGAtTGGAAGCGAGAGtAGTtTGCGtttAAGAtCGCGtAAGAGGTGGttTTTTGTTCGtTGGtTttAGtAtATtt 240
161                          TGCGTTTAAGATCGCGT                                 ATATTT 240
161                          TGTGTTTAAGATTGTGT                                   ATTT 240
```

```
241 CGGCTCTCGAAAAAACACTACCTCTGTCCCCTGCCACCCTCTGCATTTTAGAAAATCCTCTTCAGGGCAGGAAAGGACAG 320
241                                                                       GGAAAGGATAG 320
241 CGGtTtTCGAAAAAAtAtTAttTtTGTttttTGttAtttTtTGtATTTTAGAAAATttTtTTtAGGGtAGGAAAGGAtAG 320
241 TGGTTTTTGAAAA                                                                     320
241 CGGTTTTCGAAA

321 TGAGTCACTGCAAG                                                                    400
321 TGAGTTATTGTAAG                                                                    400
321 TGAGTtAtTGtAAG                                                                    400
```

Figure 35: Amplificate of SEQ ID NO: 32b

```
317 AGAGTGTGGTACCAGATCTGGTTCCATTTTTACGAGAGCCAGGAACCACGCACGTCTGGAGGGAGAGCGCAGCTGACCCG 238
317 TCCTTTCC                                                                         238
317 AGAGTGTGGTAttAGATtTGGTTtATTTTTACGAGAGttAGGAAttACGtACGTtTGGAGGGAGAGCGtAGtTGAttCG 238
317                                         AGGAATTACGTACGTT          AGAGCGTAGTTGATTCG 238
317                                                                   AGAGTGTAGTTGATTTG 238
317                                             TATGTATGTTTGGAGGG                        238

237 AGGGCGCGACCGTGTTCTGGGAGTGTTTGAATCCCCGGCCTTTGGTGAAAATTTCCTGTGTCCGCAAGGCCCAGAGGAGA 158
237 AGGGCGCGAtCGTGTTtTGGGAGTGTTTGAATtttCGGttTTTGGTGAAAATTTttTGTGTtCGtAAGGtttAGAGGAGA 158
237 A                                                                                158
237 A                                                                                158

157 TCGTGTGATGTCCGGGGGGTGCTGCGTGCGGCCGGCTGGGTAGCGGCCGCGGACCACCACTAAACCGGGGCATGTTTGCC  78
157 TCGTGTGATGTtCGGGGGGTGtTGCGTGCGGtCGGtTGGGTAGCGGtCGCGGAttAttAtTAAAtCGGGGtATGTTTGtC  78
157                                         TAGCGGTCGCGGATTA                             78
157                                        GTAGTGGTTGTGGATT                              78
```

| | | |
|---|---|---|
| 77 | GTCCTTCCTGGTGCTCTGCCCCTCTGTAACGCGGGGAAGTAGGAGGGCGCGGGGAGAAATTACTAAAGGATGCTTGC | -2 |
| 77 | **TAAAACTCCCCAACACCC** | -2 |
| 77 | GTttTTttTGGTGtTtTGttttTtTGTAACGCGGGGAAGTAGGAGGGCGCGGGGAGAAATTAtTAAAGGATGtTTGt | -2 |
| 77 | TTTGTAACGCGGGGAA | -2 |
| 77 | TTTTGTAATGTGGGGA | |

Figure 36: Amplificate of SEQ ID NO: 33

```
  1 TCAGCTGAGAAAGTGGGGGCCCTAAAAAGGGCCTTTTGTTGATAGAAAGGACGCTCAACCACCGAAACCGTAGAGGGTG 80
  1 TTAGTTGAGAAAGTGGGGGT                                                             80
  1 TtAGtTGAGAAAGTGGGGGtttTAAAAAGGGttTTTTGTTGATAGAAAGGACGtTtAAttAtCGAAAtCGTAGAGGGTG 80
  1                                                              ATCGAAATCGTAGAGG    80
  1                                                              ATTGAAATTGTAGAGGG   80

 81 CGGCCCTGGCGCTTGAGCGCGTAGACCACATCCATGGCGGTGACCGTCTTGCGCTTGGCGTGCTCTGTATAGGTCACGGC 160
 81 CGGtttTGGCGtTTGAGCGCGTAGAttAtATttATGGCGGTGAtCGTtTTGCGtTTGGCGTGtTtTGTATAGGTtACGGC 160
 81                            TTTATGGTGGTGATTGT                                TTACGGC 160
 81                              TATGGCGGTGATCGTT                               TTATGGT 160

161 GTCCCGGATCACGTTCTCCAGGAACACCTTCAGCACCCCGCGAGTCTCCTCGTAGATGAGGCCGGAGATGCGCTTCACGC 240
161 GTttCGGATtACGTTtTttAGGAAtAttTTtAGtAtttCGCGAGTtTttTCGTAGATGAGGtCGGAGATGCGtTTtACGt 240
161 GTTTCGGAT                                                         AGATGTGTTTTATGT 240
161 GTTTTGGATT                                                      ATGCGTTTTACGT 240

241 CGCCGCGGCGAGCAAGGCGCCGGATGGCCGGCTTGGTGATGCCCTGGATATTGTCGCGCAGTACTTTACGGTGGCGCTTA 320
241 CGtCGCGGCGAGtAAGGCGtCGGATGGtCGGtTTGGTGATGtttTGGATATTGTCGCGtAGTAtTTTACGGTGGCGtTTA 320
241 TGT                                       GGATATTGTTGTGTAGT                        320
241 CGT                                         TATTGTCGCGTAGTAT
```

```
321 GCGCCGCCTTTGCCAAGACCCTTCCCGCCTTTGCCGCGGCCAGACATGACGAGCAAGAGGAGTCTCACCCAACGCTTTGT 400
321 GCGtCGttTTTGttAAGAtttTTttCGttTTTGtCGCGGttAGAtATGACGAGtAAGAGGAGTtTtAtttAACGtTTTGT 400

401 GAGGACTCTGGCCTGAGGCAG 480
401 GAGGATTTTGGTTTGAGGTAG 480
401 GAGGAtTtTGGttTGAGGtAG 480
```

Figure 37: Amplificate of SEQ ID NO: 34

```
498 GTGAGAGTGGGTGCTGAAACCCCATCCCGGGCCCCAAAGGACACTGGCCGATTCTCGCGCAGAAAGCCACAGGCCTTGTC 419
498 GTGAGAGTGGGTGTTGAAAT                                                             419
498 GTGAGAGTGGGTGtTGAAAttttATttCGGGttttAAAGGAtAtTGGtCGATTtTCGCGtAGAAAGttAtAGGttTTGTC 419
498                                                   GGTCGATTTTCGCGTA               419
498                                                  TGGTTGATTTTTGTGTA               419

418 GCACTCCCTCCCTGTGGACGGGTGTGGATGTGTCATCATCTGACTGCTCCCTGGGAGGGCTGGGAGTTTCACCCGCTCCT 339
418 GtAtTttttTttTGTGGACGGGTGTGGATGTGTtATtATtTGAtTGtTtttTGGGAGGGtTGGGAGTTTtAttCGtTttT 339

338 GCCTTGCCAAGGCACTGACCGGCTGGAGGCGCGGAATCCGGCCTGGTTGTTGGCGGTCCGATCTGGGGACCGGCTCTTCG 259
338 GttTTGttAAGGtAtTGAtCGGtTGGAGGCGCGGAATtCGGttTGGTTGTTGGCGGTtCGATtTGGGGAtCGGtTtTTCG 259
338           TATTGATTGGTTGGAGG                                               ATTGGTTTTTG 259
338           TATTGATCGGTTGGAG                                                ATCGGTTTTTCG 259

258 AGGTCTGTCCTGTTCAGCCCCGCCTGGGGCATAGGGTCTTCTAGGTCTCGGGTTGTGGTGCGGGGTTGCCAGATAAATGC 179
258 AGGTtTGTttTGTTtAGtttCGttTGGGGtATAGGGTtTTtTAGGTtTCGGGTTGTGGTGCGGGGTTGttAGATAAATGt 179
258 AGGTT                                                                            179
258 AGGT
```

178 AGTAATGGCAGCAAGGAGAACCGGCATTTCCACTGGGCCTTTTACGTCTACCATGCCTGTTTTCCCATGTGGCCCTCCGA 99
178 AGTAATGGtAGtAAGGAGAAtCGGtATTTttAtTGGGttTTTTACGTtTAttATGttTGTTTTtttATGTGGtttTtCGA 99

98 CTCCTGTGAGGGAGGTGAGGCTGCACTATAGGTGAGGATGTGGGGGATCAAGGCAGGGGCATCCCCCACCCACCCTCAGC 19
98 **TTAGT** 19
98 tTttTGTGAGGGAGGTGAGGtTGtAtTATAGGTGAGGATGTGGGGGATtAAGGtAGGGGtATtttttAtttAtttTtAGt 19

18 CATGCAGCTGACTGGGGC -61
18 **TATGTAGTTGATTGGGGT** -61
18 tATGtAGtTGAtTGGGGt -61

Figure 38: Amplificate of SEQ ID NO: 35

```
  1 ACCTCAGGTGTAAGCCCAAGGCTGCTGCCTGCAAAGGCAATGCCGTGGAGACTGGGTTCCACAGCGACCTGGGTTTTTAA  80
  1 ATTTTAGGTGTAAGTTTAAGGTTGT                                                        80
  1 AttTtAGGTGTAAGtttAAGGtTGtTGttTGtAAAGGtAATGtCGTGGAGAtTGGGTTttAtAGCGAttTGGGTTTTTAA  80

 81 GTCACCGAAAGCTACAGGGCAGGGTCACAAACACTCTCCCGCCTTCTCTGCAGAACCGTGCGGCTGACAGGAGAGCGTTG 160
 81 GTtAtCGAAAGtTAtAGGGtAGGGTtAtAAAtAtTtTttCGtttTTtTtTGtAGAAtCGTGCGGtTGAtAGGAGAGCGTTG 160
 81                                                        TGTAGAATTGTGTGGT         160
 81                                                            AATCGTGCGGTTGATA     160

161 CGCAGAAAATTCGAGGCCGGGCGCTGGAGGAGTCTCCGCGGCCCGGAGAAGGCACAGAGGCGCCCCTGAGAGGCGCAGCT 240
161 CGtAGAAAATTCGAGGtCGGGCGtTGGAGGAGTtTtCGCGGttCGGAGAAGGtAtAGAGGCGttttTGAGAGGCGtAGtT 240
161     AAAATTCGAGGTCGGG          TTTTCGCGGTTCGGAG                                  240
161     AAAATTTGAGGTTGGG            TTTGTGGTTTGGAGAA                                240

241 GGAACAGGCGATGCACGGGTTCGGATCTGGCCGGCCAACCGAGCCCAGCGGTCGCGAAAACCAGAGTCGCCACAGAGGTT 320
241 GGAAtAGGCGATGtACGGGTTCGGATtTGGtCGGttAAtCGAGtttAGCGGTCGCGAAAAttAGAGTCGttAtAGAGGTT 320
241  AATAGGCGATGTACGG       TTTGGTTGGTTAATTGA   TAGCGGTCGCGAAAAT                    320
241   TAGGTGATGTATGGGT       TTGGTCGGTTAATCGA                                       320
241                                     AGTTTAGTGGTTGTGA
```

```
321 GAGCACGATTCCATTTCTGGGGATCGGGTCCGGTGGGGAGCCACTGTCCCTAACGCCTCCAGAGACTTTAAGTAGGACAA 400
321 GAGtACGATTttATTTtTGGGGATCGGGTtCGGTGGGGAGttAtTGTtttTAACGttTttAGAGAtTTTAAGTAGGAtAA 400

401 TATAATGCTGGTAGGAGCAAGGTGCAAGGAATTTAGCGGCAGAAGCCTTTCGGGGGGGTGGGGAAAGGCAGAT       480
401                                                             GGGTGGGGAAAGGTAGAT       480
401 TATAATGtTGGTAGGAGtAAGGTGtAAGGAATTTAGCGGtAGAAGttTTTCGGGGGGGTGGGGAAAGGtAGAT       480
```

Figure 39: Amplificate of SEQ ID NO: 13

```
488 TGTCCTAGGTCTGATGGACCAGACCCCAGCGGGCCTGAACTTTGTCGGAAAGCGGAGCCGCCAGGCATTTGGCAGCTGCC 409
488 TGTTTTAGGTTTGATGGATTAGA                                                          409
488 TGTttTAGGTtTGATGGAttAGAttttAGCGGGttTGAAtTTTGTCGGAAAGCGGAGtCGttAGGtATTTGGtAGtTGtt 409
488                                     TTTGTCGGAAAGCGGA                             409
488                                     TTTGTTGGAAAGTGGA                             409

408 AGAGGCGCACCAGGCCGGGTCTCAACCTGGGAGGTCAGCGGGCCAAAGCGGCCTGGGGAGGCAGTGGCCCCACCTGGACG 329
408 AGAGGCGtAttAGGtCGGGTtTtAAttTGGGAGGTtAGCGGGttAAAGCGGttTGGGGAGGtAGTGGttttAttTGGACG 329
408                                                                        TATTTGGACG 329
408                                                                        TATTTGGATG 329

328 CCGGGCCGTCGGGCGTCGGAGCCCCGCTCTGCCTCCACCAAGCTGTCCCGCTGCGCCCTACGCCGCCACCAGGCAGGAGG 249
328 tCGGGtCGTCGGGCGTCGGAGtttCGtTtTGttTttAttAAGtTGTttCGtTGCGtttTACGtCGttAttAGGtAGGAGG 249
328 TCGGGT                                                     TTACGTCGTTATTAGGT     249
328 TTGGGT                                                   TTTTATGTTGTTATTAGGT     249

248 AGCCGCTACCAGTACCTCAGAGTGGGACTCTCCATCCTCAAGTCTGCGCTCCAGAAAAGTGTAGCAAGCCTCCCACGCGG 169
248 AGtCGtTAttAGTAttTtAGAGTGGGAtTtTttATttTtAAGTtTGCGtTttAGAAAAGTGTAGtAAGttTtttACGCGG 169
248                                                                         TTTATGTGG 169
248                                                                         TTTACGCGG 169
```

```
168 GGCTTCAGACACCTAAAAGTCGCCACTCCCCCAAGAGTAACCTCCACCCCCACCCCCACCCCCAACTTCTCTTTCCTCCT  89
168 GGtTTtAGAtAttTAAAAGTCGttAtTtttttAAGAGTAAttTttAtttttAtttttAtttttAAtTTtTtTTTttTttT  89
168 GGTTTTAG                                                                           89
168 GGTTTTA

 88 TTTCTTCAGCAATGTGGACCAACAGTTACTTTTCCCTCTCTTCCCTTCCCCTCCGAAGTCCCCGGGGCAAAGTGGGACTT   9
 88                                                                       AGTGGGATTT   9
 88 TTTtTTtAGtAATGTGGAttAAtAGTTAtTTTTtttTtTtTTtttTTttttTtCGAAGTtttCGGGGtAAAGTGGGAtTT   9

  8 TGGGGAGC                                                                         -71
  8 TGGGGAGT                                                                         -71
  8 TGGGGAGt                                                                         -71
```

Figure 40: Amplificate of SEQ ID NO: 36

1 GTTGAGAGGTAAGGCATGAAGGGCGCAATATCCAATATGAGCAACGCGTGTGATGCATCTGGTCAAAATGCATACAGAGG 80
1 **GTTGAGAGGTAAGGTATGAAGG** 80
1 GTTGAGAGGTAAGGtATGAAGGGCGtAATATttAATATGAGtAACGCGTGTGATGtATtTGGTtAAAATGtATAtAGAGG 80
1 TATGAGTAATGTGTGTG 80
1 GAGTAACGCGTGTGAT 80

81 ACTTGTCTCTGTCCCTAGATAGAAGTCCTCCGTCCTGCAGTCATGAGGGTCAATTGCTGAGGCTTCACAGTTCCCTTCTC 160
81 AtTTGTtTtTGTtttTAGATAGAAGTttTtCGTttTGtAGTtATGAGGGTtAATTGtTGAGGtTTtAtAGTTtttTTtTt 160

161 TCTTACACTCGGACCGTCACGCTCCTCACCTACTACCCCGATGCAGAGGTAGACTCAGGATCCCTGCACTTGTCAAGGAT 240
161 TtTTAtAtTCGGAtCGTtACGtTttTtAttTAtTAttCGATGtAGAGGTAGAtTtAGGATtttTGtAtTTGTtAAGGAT 240
161 ATTCGGATCGTTACGT 240
161 ATTTGGATTGTTATGTTT 240

241 TCCTCGGCAAGCTCACGGGGCGGGAGTGGCCACAAGACGGAGCTCGCCTGGTCCTGGCCTTCCCGGCCTATACAAGCCTG 320
241 TttTCGGtAAGtTtACGGGGCGGGAGTGGttAtAAGACGGAGtTCGttTGGTttTGGttTTttCGGttTATAtAAGttTG 320
241 ATAAGACGGAGTTCGT 320
241 AAGATGGAGTTTGTTTG 320

```
321 CCCCCTTCCCAATTCCCAATCTCCACAGCCTTCCATCCTCCCACTTTCGATTCACCTTGCGCCACCGACGCCCCTGGCCT 400
321 tttttTTtttAATTtttAATtTttAtAGttTTttATttTtttAtTTTCGATTtAttTTGCGttAtCGACGttttTGGttT 400
321                                                                 ATTTTGTGTTATTGATGT         400
321                                                                    TTGCGTTATCGACGTT

401 TCGTTTGCAGCAAGTTTACCCCCACCATTACCTCTCGCATAAAAGCCTGCATTTACCAGGTCAAAGAGGGGAACCAA    480
401                                                              TTATTAGGTTAAAGAGGGGAATTAA    480
401 TCGTTTGtAGtAAGTTTAtttttAttATTAttTtTCGtATAAAAGttTGtATTTAttAGGTtAAAGAGGGGAAttAA    480
```

Figure 41: Amplificate of SEQ ID NO: 37

```
1 GTGTCAGGGCTCAGGGGCCCATCGCCCTTGGCCTGGGGCCCCTCCGTGCCGTCGGGATTTGTGGGACGCGCTGAAGGAAG 80
1 GTGTTAGGGTTTAGGGGTTT                                                             80
1 GTGTtAGGGtTtAGGGGtttATCGtttTTGGttTGGGGttttTtCGTGtCGTCGGGATTTGTGGGACGCGtTGAAGGAAG 80
1                                                             TGTGGGATGTGTTGAA     80
1                                                              GTGGGACGCGTTGAAG    80

81 AGGCGGGCGGCTCCGACAGAAAACAGCCAGAGCGCACCACTCACCTGAGTGCCAGGTAAACACCTGGGCGCGACAGGGAC 160
81 AGGCGGGCGGtTtCGAtAGAAAAtAGttAGAGCGtAttAtTtAttTGAGTGttAGGTAAAtAttTGGGCGCGAtAGGGAt 160
81    GGGTGGTTTTGATAGA    AGTTAGAGCGTATTATTT                          TATTTGGGCGCGATAG  160
81     GGCGGTTTCGATAGAA                                                ATTTGGGTGTGATAGG 160
81                       AATAGTTAGAGTGTATTATT

161 AGGAAACAAGGGTAGGGTGCGGAGGCTGGGGAGGAAGAGGTTGGAAAGG                              240
161                           AGGAAGAGGTTGGAAAGG                              240
161 AGGAAAtAAGGGTAGGGTGCGGAGGtTGGGGAGGAAGAGGTTGGAAAGG                              240
```

Figure 42: Amplificate of SEQ ID NO: 1

```
504 TCTGGAGGGATAGAATGTGATCCTCGAGCAGAAACCATCGTGTTTCTGGGGGCTCTTCGGGCGCCGTGGGGCGACGCGGT 425
504 TTTGGAGGGATAGAATGTGA                                                              425
504 TtTGGAGGGATAGAATGTGATttTCGAGtAGAAAttATCGTGTTTtTGGGGGtTtTTCGGGCGtCGTGGGGCGACGCGGT 425

424 GCAGCGTCTAGGGCCGCCGCAGGCCGGGGGCAGGGCTCCCAGCGGTTCCCCCGCGGCCAGCGGCCACGCAGGAAAAACCC 345
424 GtAGCGTtTAGGGtCGtCGtAGGtCGGGGGtAGGGtTtttAGCGGTTttttCGCGGttAGCGGttACGtAGGAAAAAttC 345
424          TTAGGGTCGTCGTAGGT                                       AGTGGTTATGTAGGAAA    345
424          TTAGGGTTGTTGTAGGT                                      TAGCGGTTACGTAGGA      345

344 GCTTCCTCGCCCCTGCATCTGCTGACAAGCCGCCCGAGGTGTCTGCGCTGAGCGTGGCCACACCGATGCAGCTTTCTAGG 265
344 GtTTttTCGttttTGtATtTGtTGAtAAGtCGttCGAGGTGTtTGCGtTGAGCGTGGttAtAtCGATGtAGtTTTtTAGG 265
344                           AAGTTGTTTGAGGTGT                                          265
344                          ATAAGTCGTTCGAGGT                                           265

264 AAATGGCCCGGGAGGGGGAGGGGGCGAGTGAGGGATTAGGTCCGGCTCCCCCTCGCTCCCCCTGCGCAAACACCACCACC 185
264 AAATGGttCGGGAGGGGGAGGGGGCGAGTGAGGGATTAGGTtCGGtTtttttTCGtTttttTGCGtAAAtAttAttAtt 185
```

```
184 CCTTCCTTTCCCCGGAGGCATGAGGCCTCCACGCTCGGTCCTTCGCCCGCATCCTCGCCGACCCCTCCGGTTTCGCGCCT 105
184 ttTTttTTTtttCGGAGGtATGAGGttTttACGtTCGGTttTTCGttCGtATttTCGtCGAttttTtCGGTTTCGCGttT 105

104 CACTCGCCAGGCTTTCTCCCTGGGAGCCGACTGCGGTGAAGTCGCCGCCAGCACGGTCGGCCCTGACACGTGCTCTAACG  25
104 tAtTCGttAGGtTTTtTtttTGGGAGtCGAtTGCGGTGAAGTCGtCGttAGtACGGTCGGtttTGAtACGTGtTtTAACG  25
104                                                  TTAGTACGGTCGGTTT   TACGTGTTTTAACG  25
104                                                                  TGATATGTGTTTTAATG  25
104                                                 GTTAGTATGGTTGGTTT                   25

 24 CATGCTTGAGTCAAGGCTGTCTTT                                                          -55
 24  ATGTTTGAGTTAAGGTTGTTTTT                                                          -55
 24 tATGtTTGAGTtAAGGtTGTtTTT                                                          -55
 24 TAT                                                                               -55
 24 TA
```

Figure 43: Amplificate of SEQ ID NO: 2

```
  1 AGATCATCAGTCCTCACCTGAGGGACCTTCCGCGGCATCTATGCGGGCATGGTTACTGCCTCTGGTGCCCCCCGCAGCCG  80
  1 AGATTATTAGTTTTTATTTGAGGGATT                                                       80
  1 AGATtATtAGTttTtAttTGAGGGAttTTtCGCGGtATtTATGCGGGtATGGTTAtTGttTtTGGTGttttCGtAGtCG  80
  1                                                                           TAGTCG  80
  1                                                                           TAGTTG  80

 81 CGCGCAGGTACCGTGCGACATCGCGATGGCCCAGCTCCTCAGCCAGGTCCACGGGCAGACGGCCCCAGGCATCGCGCACG 160
 81 CGCGtAGGTAtCGTGCGAtATCGCGATGGtttAGtTttTtAGttAGGTttACGGGtAGACGGttttAGGtATCGCGtACG 160
 81 CGCGTAGGTA                                                      TTAGGTATTGTGTATG 160
 81 TGTGTAGGTAT                                                     TAGGTATCGCGTACG 160
 81    GTAGGTATTGTGTGATAT                                                            160
 81     TAGGTATCGTGCGATA                                                             160

161 TCCAGCCGCGCCCCGGCCCGGTGCAGCACCACCAGCGTGTCCAGGAAGCCCTCCCGGGCAGCGTCGTGCACGGGTCGGGT 240
161 TttAGtCGCGtttCGGttCGGTGtAGtAttAttAGCGTGTttAGGAAGtttTttCGGGtAGCGTCGTGtACGGGTCGGGT 240
161 TT                                                                               240
161 T                                                                                240
```

```
241 GAGAGTGGCGGGGTCGGCGCAGTTGGGCTCCGCGCCGTGGAGCAGCAGCAGCTCCGCCACTCGGGCGCTGCCCATCATCA 320
241 GAGAGTGGCGGGGTCGGCGtAGTTGGGtTtCGCGtCGTGGAGtAGtAGtAGtTtCGttAtTCGGGCGtTGtttATtATtA 320
241                                           TTTTGTGTTGTGGAGTA                                320
241                                            TTCGCGTCGTGGAGTA

321 TGACCTGCCAGAGAGAACAGAATGGTCAGAGCCAGGGTGG                                            400
321                   GAATGGTTAGAGTTAGGGTGG                                            400
321 TGAttTGttAGAGAGAAtAGAATGGTtAGAGttAGGGTGG                                            400
```

*Figure 44: Amplificate of SEQ ID NO: 3*

```
  1 CCCTTGCTTGGGTGTGTCCTTCGCTCGCTCCCTCCCTCCGTCTTAGGTCACTGTTTTCAACCTCGAATAAAAACTGCAGC  80
  1 TTTTTGTTTGGGTGTGTTTT                                                              80
  1 tttTTGtTTGGGTGTGTttTTCGtTCGtTtttTtttTtCGTtTTAGGTtAtTGTTTTtAAttTCGAATAAAAAtTGtAGt  80

 81 CAACTTCCGAGGCAGCCTCATTGCCCAGCGGACCCCAGCCTCTGCCAGGTTCGGTCCGCCATCCTCGTCCCGTCCTCCGC 160
 81 tAAtTTtCGAGGtAGttTtATTGtttAGCGGAttttAGttTtTGttAGGTTCGGTtCGttATttTCGTttCGTttTtCGt 160
 81                                             TAGGTTCGGTTCGTTAT                    160
 81                                             TAGGTTTGGTTTGTTATT                   160

161 CGGCCCCTGCCCCGCGCCCAGGGATCCTCCAGCTCCTTTCGCCCGCGCCCTCCGTTCGCTCCGGACACCATGGACAAGTT 240
161 CGGttttTGtttCGCGtttAGGGATttTttAGtTttTTTCGttCGCGtttTtCGTTCGtTtCGGAtAttATGGAtAAGTT 240
161        GTTTCGCGTTTAGGGA                                     GTTCGTTTCGGATATTA        240
161        GTTTTGTGTTTAGGGAT                                     TTTGTTTTGGATATTATGG     240

241 TTGGTGGCACGCAGCCTGGGGACTCTGCCTCGTGCCGCTGAGCCTGGCGCAGATCGGTGAGTGCCCGCCGCAGCCTGGGC 320
241 TTGGTGGtACGtAGttTGGGGAtTtTGttTCGTGtCGtTGAGttTGGCGtAGATCGGTGAGTGttCGtCGtAGttTGGGt 320
241                                          TTTGGCGTAGATCGGT                        320
241                                          TTTGGTGTAGATTGGT
```

321 AGCAAGATGGGTGCGGGGTGCTCAGCGCGGACCCGGCGGCAGCCCCTCCGGCTGAGTCGGCCCTGGGGGACTGGAGTCAA 400
321 **GGGGGATTGGAGTTAA** 400
321 AGtAAGATGGGTGCGGGGTGtTtAGCGCGGAttCGGCGGtAGttttTtCGGtTGAGTCGGtttTGGGGGAtTGGAGTtAA 400

401 GTG 480
401 **GTG** 480
401 GTG 480

Figure 45: Amplificate of SEQ ID NO: 4

```
446 GAAGGGATGAATGAATAAAAGTACTTGTCTGATGGCAGCAGAGACCCCGAGCAAACGGTGGAGGCTACACTGTCTGGCAT 367
446 GAAGGGATGAATGAATAAAAGT 367
446 GAAGGGATGAATGAATAAAAGTAtTTGTtTGATGGtAGtAGAGAtttCGAGtAAACGGTGGAGGtTAtAtTGTtTGGtAT 367
446 TTTGAGTAAATGGTGGA GTAT 367
446 ATTTCGAGTAAACGGT TGGTAT 367

366 TCTCGCAGCGTTTCGTCAGAGCCGGACCCGCCTGCAGCTCAAGGGAGGCGTGCTCCTCTCCCAGAGCAGGCTGGAACCCA 287
366 TtTCGtAGCGTTTCGTtAGAGtCGGAttCGttTGtAGtTtAAGGGAGGCGTGtTttTtTtttAGAGtAGGtTGGAAtttA 287
366 TTTCGTAGCGTT AGTTGGATTTGTTTGTA 287
366 TTTTGTAGTGTT TAGAGTCGGATTCGTT 287

286 GCTGGGTTCCGCCTCCCGGGAAGGTGGTCTCCATTCGTCGCTCTGCATCTGGTTTGTCAGATCCGAGAGGTAAACATTCG 207
286 GtTGGGTTtCGttTttCGGGAAGGTGGTtTttATTCGTCGtTtTGtATtTGGTTTGTtAGATtCGAGAGGTAAAtATTCG 207
286 TTTGTTTTTTGGGAAGG 207
286 TTCGTTTTTCGGGAAG

206 GGCTTGGTGTTGAATTAAAATCATTGATTGAACCTTATTCTGGGGCTTCGGTTTGGCTTACTAGTTTGGGATTTTAAAAA 127
206 GGtTTGGTGTTGAATTAAAATtATTGATTGAAttTTATTtTGGGGtTTCGGTTTGGtTTAtTAGTTTGGGATTTTAAAAA 127
```

| | | |
|---|---|---|
| 126 | AATAAAAATTAAGCCTATAGAGAGGGCAAATTAAAATTAGGTTGGGTAAAGGAAGGAGCGCGAGTGTTTGAAGCCGTTTG | 47 |
| 126 | AATAAAAATTAAGttTATAGAGAGGGtAAATTAAAATTAGGTTGGGTAAAGGAAGGAGCGCGAGTGTTTGAAGtCGTTTG | 47 |

| | | |
|---|---|---|
| 46 | GAGGGAACAGCGGTTTCCAAGTTCCTGCTGACTTGAGAAGTCTCTG | -33 |
| 46 | **TTGTTGATTTGAGAAGTTTTTG** | -33 |
| 46 | GAGGGAAtAGCGGTTTttAAGTTttTGtTGAtTTGAGAAGTtTtTG | -33 |

Figure 46: Amplificate of SEQ ID NO: 5

```
  1 ACATGGGATTGATGGAAGACAGCCCCCAAGGACGGGGGTGGGTGGCCCTGTTTTTCTCTGATTGGCGGACGAGGGACTTC 80
  1 ATATGGGATTGATGGAAGATAG                                                           80
  1 AtATGGGATTGATGGAAGAtAGttttAAGGACGGGGGTGGGTGGtttTGTTTTTtTtTGATTGGCGGACGAGGGAtTTt 80
  1                                                             TTGATTGGCGGACGAG     80
  1                                                             TTGATTGGTGGATGAG     80

 81 TAGACCCCTGCCAAAACACCAAGGGGGCGGTGTCGCACGCCATGCTGCTCAGCGGAAGCAGGGCTTGTATAGAAATGGGC 160
 81 TAGAttttTGttAAAAtAttAAGGGGGCGGTGTCGtACGttATGtTGtTtAGCGGAAGtAGGGtTTGTATAGAAATGGGC 160
 81                         GGTGTTGTATGTTATGT                                 ATGGGT 160
 81                           TGTCGTACGTTATGTT                                 TGGGC 160

161 GTAGCAGCCGGCGGGAGTGGGTCGGACTGGGCTCCGCGTAGGCGGGCAGGGCAGCTCTACGGTTAGACAAGACCATAGAG 240
161 GTAGtAGtCGGCGGGAGTGGGTCGGAtTGGGtTtCGCGTAGGCGGGtAGGGtAGtTtTACGGTTAGAtAAGAttATAGAG 240
161 GTAGTAGTTGG          ATTGGGTTTCGCGTAGG                                           240
161 GTAGTAGTCGG          ATTGGGTTTTGTGTAGG

241 CTGGGTAAAGACGAATTTAGAACACAGCGGAGGTAGGAGGGCAGGATGGCTGTCAGGCACACGAAAGAGCATTGAGTGGC 320
241 tTGGGTAAAGACGAATTTAGAAtAtAGCGGAGGTAGGAGGGtAGGATGGtTGTtAGGtAtACGAAAGAGtATTGAGTGGt 320
```

```
321 AGAAACGAAATGCTTTCAGAGGGCAGGGTTGAACTGCGGCCAGAGTTAAAAAGGGGAGGAACCTGGCTCTGCATTGATTG 400
321 AGAAACGAAATGtTTTtAGAGGGtAGGGTTGAAtTGCGGttAGAGTTAAAAAGGGGAGGAAttTGGtTtTGtATTGATTG 400

401 GCGGTGGCTGGACTCAACCTAGAATAGGGGCCCGACTAGGGAGAGCGGGATCCAGGTCTGTGCCCATTGGA          480
401                                                     GATTTAGGTTTGTGTTTATTGGA          480
401 GCGGTGGtTGGAtTtAAttTAGAATAGGGGttCGAtTAGGGAGAGCGGGATttAGGTtTGTGtttATTGGA          480
```

Figure 47: Amplificate of SEQ ID NO: 6

```
  1 CTTTCATCCAGGATGAGGGACATTTAAGATGAAATGTCCGTGGCAGGATCGTTTCTCTTCACTGCTGCATGCGGCACTGG  80
  1 TTTTTATTTAGGATGAGGGATATT                                                          80
  1 tTTTtATttAGGATGAGGGAtATTTAAGATGAAATGTtCGTGGtAGGATCGTTTtTtTTtAtTGtTGtATGCGGtAtTGG  80

 81 GAACTCGCCCCACCTGTGTCCGGAACCTGCTCGCTCACGTCGGCTTTCCCCTTCTGTTTTGTTCTAGGACTTCTGCACGG 160
 81 GAAtTCGttttAttTGTGTtCGGAAttTGtTCGtTtACGTCGGtTTTttttTTtTGTTTTGTTtTAGGAtTTtTGtACGG 160
 81                                                                            TACGG 160
 81                                                                            TATGG 160

161 ACCTGGCCGTCTCCAGTGCCAACTTCATTCCCACGGTCACTGCCATCTCGACCAGTCCGGACCTGCAGTGGCTGGTGCAG 240
161 AttTGGtCGTtTttAGTGttAAtTTtATTtttACGGTtAtTGttATtTCGAttAGTtCGGAttTGtAGTGGtTGGTGtAG 240
161 ATTTGGTCGTTT                                      TATTTTGATTAGTTTGGAT            240
161 ATTTGGTTGTTT                                        TTCGATTAGTTCGGAT             240

241 CCCGCCCTCGTCTCCTCCGTGGCCCCATCGCAGACCAGAGCCCCTCACCCTTTCGGAGTCCCCGCCCCCTCCGCTGGGGC 320
241 ttCGtttTCGTtTttTtCGTGGttttATCGtAGAttAGAGttttTtAtttTTTCGGAGTtttCGtttttTtCGtTGGGGt 320
241           TTTCGTGGTTTTATCGTA                                                     320
241           TTTTGTGGTTTTATTGTA                                                     320
```

```
321 TTACTCCAGGGCTGGCGTTGTGAAGACCATGACAGGAGGCCGAGCGCAGAGCATTGGCAGGAGGGGCAAGGTGGAACAGG 400
321 TTAtTttAGGGtTGGCGTTGTGAAGAttATGAtAGGAGGtCGAGCGtAGAGtATTGGtAGGAGGGGtAAGGTGGAAtAGG 400
321                                             TAGGAGGTTGAGTGTA                       400
321                                                     GTCGAGCGTAGAGTAT

401 TGAGGAACTCTAGCGTACTCTTCCTGGGAATGTGGGGGCTGGGTGGAAGCAGC                              480
401                                                       GGTTGGGTGGGAAGTAGT          480
401 TGAGGAAtTtTAGCGTAtTtTTttTGGGAATGTGGGGGtTGGGTGGAAGtAGt                              480
```

Figure 48: Amplificate of SEQ ID NO: 7

```
347 TTGGCCCCATGCTGGGAGCTCTGAGCCCCATCCCCGGGGACGCGGGCCGCGCGTACTCACTGGTGGCGAAGACTGCGGCG 268
347 TTGGTTTTATGTTGGGAGTTTTG                                                          268
347 TTGGttttATGtTGGGAGtTtTGAGttttATtttCGGGGACGCGGGtCGCGCGTAtTtAtTGGTGGCGAAGAtTGCGGCG 268
347                                                     GTCGCGCGTATTTATT             268
347                                                   GGGTTGTGTGTATTTAT              268

267 GCGAAACTCCAGCGAAGGCCTCGCGGCCTCCGAGCCTTATAAGGGTGGTCCCGCCCCGCTCCGCCCCAGTGCTGAGTCAC 188
267 GCGAAAtTttAGCGAAGGttTCGCGGttTtCGAGttTTATAAGGGTGGTttCGtttCGtTtCGttttAGTGtTGAGTtAC 188
267                 TTTGTGGTTTTTGAGTT                                                188
267                 TTCGCGGTTTTCGAGT                                                 188

187 GGCGCCGGCCGCTCTTCTGGAGGGTCCCGCGGACTCCCGCCGGCCCCAGCCCCGGCGGCCGCTGCACCCCGGGCGTCGGC 108
187 GGCGtCGGtCGtTtTTtTGGAGGGTttCGCGGAtTttCGtCGGttttAGtttCGGCGGtCGtTGtAtttCGGGCGTCGGt 108
187                                                                           GTCGGT 108
187                                                                           GTTGGT 108

107 CGCAGAGGGGCGCCCTGGAGTCCCCGGAGTCGCCGCGCAGCTGGCCGGGGAAGCCTTTCCCTCTTTCCCAGGTCCCCAGC  28
107 CGtAGAGGGGCGtttTGGAGTtttCGGAGTCGtCGCGtAGtTGGtCGGGGAAGttTTTtttTtTTTtttAGGTttttAGC  28
107 CGTAGAGGGG              GAGTCGTCGCGTAGTT                                          28
107 TGTAGAGGGG             GGAGTTGTTGTGTAGTT
```

| | | |
|---|---|---|
| 27 | GGGGCCTAGGGAGTAAACAGACAGCAG | -52 |
| 27 | **GTTTAGGGAGTAAATAGATAGTAG** | -52 |
| 27 | GGGGttTAGGGAGTAAAtAGAtAGtAG | -52 |

Figure 49: Amplificate of SEQ ID NO: 38

```
466 CAGATGGGGACAGGAAGCTGTGGACGAAAGCCCCAGGTCCCGTGGGAGAGGTGACAGCAGCAGGGGCACGCAGCCACGTG 387
466 TAGATGGGGATAGGAAGTTGT                                                             387
466 tAGATGGGGAtAGGAAGtTGTGGACGAAAGttttAGGTttCGTGGGAGAGGTGAtAGtAGtAGGGGtACGtAGttACGTG 387
466                                                                   TATGTAGTTATGTG 387
466                                                                 GGGTACGTAGTTACGT  387

386 GGTCCCCAGGGGAATGTGAAGGCGGAGGGCTCCAGGCGAACTGGGGATTAAACAAATATTTACAGGCAGCAGGGAAGTGC 307
386 GGTttttAGGGGAATGTGAAGGCGGAGGGtTttAGGCGAAtTGGGGATTAAAtAAATATTTAtAGGtAGtAGGGAAGTGt 307
386 GGT                                                                               307
386                                                                                   307

306 CCAGCGCACGTGACGGGGGCGGGGCGGGACTTTGGGGAGGGCGGGGCCTAACGGTATCGAGCGAGCCGGTTGTAGACGTG 227
306 ttAGCGtACGTGACGGGGGCGGGGCGGGAtTTTGGGGAGGGCGGGGttTAACGGTATCGAGCGAGtCGGTTGTAGACGTG 227
306   AGTGTATGTGATGGGG                                     TATTGAGTGAGTTGGTT          227
306 TTAGCGTACGTGACGG                                       TATCGAGCGAGTCGGT           227

226 GTCCAGGTTTCTGCACAGGAATATCGAGAGCGTCATGAACCCGAGCTATAGAGAAAGGAGATGAGGTCAGAGAAGTCGAA 147
226 GTttAGGTTTtTGtAtAGGAATATCGAGAGCGTtATGAAttCGAGtTATAGAGAAAGGAGATGAGGTtAGAGAAGTCGAA 147
226                       ATCGAGAGCGTTATGA                                            147
226                   AGGAATATTGAGAGTGT
```

```
146 GGGGTGCTGGCGAGACGGGGGTGATGCCCACCCGGGCGAGAAAGGTCAGGGGTGGGGCCGTGAATTGGGCGGGAAGGGGC 67
146 GGGGTGtTGGCGAGACGGGGGTGATGtttAttCGGGCGAGAAAGGTtAGGGGTGGGGtCGTGAATTGGGCGGGAAGGGGt 67

 66 TGGAGAAGGGGATAGGTAGAAAAGGGCGGGGCTCGACGGGAACTGCCGCAGGGCTGGCTGATGAGG -13
 66                                                     TAGGGTTGGTTGATGAGG -13
 66 TGGAGAAGGGGATAGGTAGAAAAGGGCGGGGtTCGACGGGAAtTGtCGtAGGGtTGGtTGATGAGG -13
```

Figure 50: Amplificate of SEQ ID NO: 39

```
466 CCACCAGGAGAGGGGAAGAAGCCAGCACCTACCGACAGGGGTGGAGCTGGGTCAAGAATGGTGTGGTCCCTGCTTTGGGG 387
466 TTATTAGGAGAGGGGAAGAAGT                                                            387
466 ttAttAGGAGAGGGGAAGAAGttAGtAttTAtCGAtAGGGGTGGAGtTGGGTtAAGAATGGTGTGGTtttTGtTTTGGGG 387

386 GAATGCTGGGGAGGTAGAAAGCCCCTTCTAACGGGGCGTCACTGCAATTACTGCTTCCTCTTTCCCATAAAACTCCCCCT 307
386 GAATGtTGGGGAGGTAGAAAGttttTTtTAACGGGGCGTtAtTGtAATTAtTGtTTttTtTTTtttATAAAAtTtttttT 307
386                           AATGGGGTGTTATTGT                                        307
386                         TTTAACGGGGCGTTAT                                          307

306 AGTGTATCAGAACCCCCAAGGAGTTTCAGTAAGCGGTTCTTCTGTTGTCTCCGGCTGAGACTCCAGGGGAACCTCAAGCT 227
306 AGTGTATtAGAAttttAAGGAGTTTtAGTAAGCGGTTtTTtTGTTGTtTtCGGtTGAGAtTttAGGGGAAttTtAAGtT 227

226 CACATGGCCCTGGCGGGCCCCTGGGCAGGAGCAGGCGAGAGGTCTGCGCGGCCGCTCTCCTACCTGCGTCCGACTCCGCG 147
226 tAtATGGtttTGGCGGGttttTGGGtAGGAGtAGGCGAGAGGTtTGCGCGGtCGtTtTttTAttTGCGTtCGAtTtCGCG 147
226                                                                     TTCGATTTCGCG 147
226                                                                    GTTTGATTTTGTG 147
```

```
146 GTCCTTGGGCAGCAGCAACCGGGTAGCGCTCAGACTACAGACCCCAGCGCGATGACCCGCCTACCTCAGTTTCCATTGGC  67
146 GTttTTGGGtAGtAGtAAtCGGGTAGCGtTtAGAtTAtAGAttttAGCGCGATGAttCGttTAttTtAGTTTttATTGGt  67
146 GTTT               TAATCGGGTAGCGTTT              AGTGTGATGATTTGTTT                   67
146 GTTT            TAGTAATTGGGTAGTGT              TAGCGCGATGATTCGT

 66 CAGGGATCAGGGCTACCCGCTCCCATTGGCTACTTATCAATATAGAGGTGGGGCCAGCCTGGAATG         -13
 66                                                     TGGGGTTAGTTTGGAATG         -13
 66 tAGGGATtAGGGtTAttCGtTtttATTGGtTAtTTATtAATATAGAGGTGGGGttAGttTGGAATG         -13
```

Figure 60: Amplificate of SEQ ID NO: 40

```
  1 CACTGGATGTCCCTGGCAGGTCCGGGTGTCTGTCTCCGAAGAGGACAGAGAAGGGCAGCCACGATCTGCCCGCCTGCCCT  80
  1 TATTGGATGTTTTTGGTAGGTT                                                            80
  1 tAtTGGATGTtttTGGtAGGTtCGGGTGTtTGTtTtCGAAGAGGAtAGAGAAGGGtAGttACGATtTGttCGttTGtttT  80
  1                                                                               TT  80
  1                                                                               TT  80

 81 CGCGAGCCTCTCGGCACTGGGTGAGAGGCAACTCTGGCCATTTCTTGCTGCCCTCTCGCCCTCTCCCGGCCGCTCCCAGT 160
 81 CGCGAGttTtTCGGtAtTGGGTGAGAGGtAAtTtTGGttATTTtTTGtTGtttTtTCGtttTtTttCGGtCGtTtttAGT 160
 81 CGCGAGTTTTTCGG                                                                   160
 81 TGTGAGTTTTTTGGTA                                                                 160

161 CCAGCCGGGCCCGGCGCTACCCGAGCGAGGGTTCGAGCCCTCTGCGCGGCCGCGCAGAAGCAGGCAGGGCCTCAACTTCT 240
161 ttAGtCGGGttCGGCGtTAttCGAGCGAGGGTTCGAGtttTtTGCGCGGtCGCGtAGAAGtAGGtAGGGttTtAAtTTtT 240
161       GGTTTGGTGTTATTTGA                                                          240
161         TTCGGCGTTATTCGAG                                                         240

241 GCAAATGTGTGCGGCTCGCCGCCTGTCCCCTTTCTCCTCCTGTCTCCACCCGTGCGGCCCCAGTGGCCTGGAGCTTCCAG 320
241 GtAAATGTGTGCGGtTCGtCGttTGTttttTTTtTttTttTGTtTttAttCGTGCGGtttAGTGGttTGGAGtTTttAG 320
241                                            TATTCGTGCGGTTTTA                      320
241                                             ATTTGTGTGGTTTTAGT                    320
```

```
321 CCCCGCGCTTGGCCGCGGCTTGGCGAGGCTATGCTGCGGGAAGCTGGAATCCAACGCGCGGCCGGCTGAACCGCCTGAGC 400
321 tttCGCGtTTGGtCGCGGtTTGGCGAGGtTATGtTGCGGGAAGtTGGAATttAACGCGCGGtCGGtTGAAtCGttTGAGt 400
321                                                        GGAATTTAATGTGTGGT                  400
321                                                         GAATTTAACGCGCGGT

401 CGCGGGAAACCAGCGGCGGAGCGGCGGTATAGAGGTGCGAGGATGAGGAGGACCGACTCGCTAAGGGAGGGAGGTGACT 480
401                                                                   TAAGGGAGGGAGGTGATT 480
401 CGCGGGAAAttAGCGGCGGAGCGGCGGTATAGAGGTGCGAGGATGAGGAGGAtCGAtTCGtTAAGGGAGGGAGGTGAtT 480
```

igure 61: Amplificate of SEQ ID NO: 41

```
424 GGAGGGGCTTGAGTAATTGATCCCTTCTCGAGATGGGGTCGAATTCCTTCCGAATGGGGGACCTTCATCCCCCTCCTGTG 345
424 GGAGGGGTTTGAGTAATTG                                                               345
424 GGAGGGGtTTGAGTAATTGATttttTTtTCGAGATGGGGTCGAATTttTTtCGAATGGGGGAttTTtATtttttTttTGTG 345

344 GGTGTATGGGGGCTGCCCTGGAGATGCGCGCCCGCGGAGGCAGGTATTGGGTGTCGGCGGAGGCGGGGCCGCGTCCCCAG 265
344 GGTGTATGGGGGtTGtttTGGAGATGCGCGttCGCGGAGGtAGGTATTGGGTGTCGGCGGAGGCGGGGtCGCGTttttAG 265

264 GGTGCTGTCCCGGTGCCCCTGGAGGCGGCCCCGACTCCACAATGGCCGCTCTGATTCTGAGGCGGAGGCCGGCGCTTTG 185
264 GGTGtTGTttCGGTGttttTGGAGGCGGtttCGAtTttAtAATGGGtCGtTtTGATTtTGAGGCGGAGGtCGGCGtTTTG 185
264                               AGGCGGTTTCGATTTT                                  185
264                             TGGAGGTGGTTTTGAT                                    185

184 TTGGCGGGCGGGGTAGCGGGCGAGCAGCTGTCGCATTTTCCCACGGGCGGGAGCTGAGTGTGGCCACCCCCCCTCCCCCC 105
184 TTGGCGGGCGGGGTAGCGGGCGAGtAGtTGTCGtATTTTtttACGGGCGGGAGtTGAGTGTGGttAtttttttTtttttC 105
184                  TAGCGGGCGAGTAGTT                  TTTACGGGCGGGAGTT                105
184                  TAGTGGGTGAGTAGTT                 TTTTATGGGTGGGAGT                 105
```

| | | |
|---|---|---|
| 104 | GTCCAGCTCTGCCCCCTTGCAGAACGGGTTTGAACAGAGGCAATCTCGGCGGCTGGATGGGGGGCCCTGCCCTGCCAGAC | 25 |
| 104 | GTttAGtTtTGtttttTTGtAGAACGGGTTTGAAtAGAGGtAATtTCGGCGGtTGGATGGGGGGtttTGtttTGttAGAt | 25 |
| 104 | ATTTCGGCGGTTGGAT | 25 |
| 104 | AGGTAATTTTGGTGGTT | |
| 24 | TCCTCAGTGAGCCTCCAGGGTGGG | -55 |
| 24 | **TAGTGAGTTTTTAGGGTGGG** | -55 |
| 24 | TttTtAGTGAGttTttAGGGTGGG | -55 |

Figure 62: Amplificate of SEQ ID NO: 42

```
  1 GACCAGGCTTGGAGTTGTTTTCTTCGGGGGGCTTCCCCTCCTGACAGCCTACTGAGTTGCATCAGAACGTGGAGGATTGT  80
  1 GATTAGGTTTGGAGTTGTTTTT                                                            80
  1 GAttAGGtTTGGAGTTGTTTTtTTCGGGGGGtTTttttTttTGAtAGttTAtTGAGTTGtATtAGAACGTGGAGGATTGT  80

 81 CCTCTGAGGACGCGCGGGGCAACCGGGAGTCTTAAAATAGCAGATAAAGTTAATACTGACTGTAATGTGCGTAAATTGGA 160
 81 ttTtTGAGGACGCGCGGGGtAAtCGGGAGTtTTAAAATAGtAGATAAAGTTAATAtTGAtTGTAATGTGCGTAAATTGGA 160

161 TTATAATCTTTAGGCGTATTGGTGACAACCAGCGTAATCCATCTCTGAGTATTAATCCGGTTAGACTCCCGGGTGACAGC 240
161 TTATAATtTTTAGGCGTATTGGTGAtAAttAGCGTAATttATtTtTGAGTATTAATtCGGTTAGAtTttCGGGTGAtAGt 240
161                                                         TAATTCGGTTAGATTTTCGG         240
161                                                         TAATTTGGTTAGATTTTTGG         240

241 CAGTCGCAGGAGTGTTCCCAGAGTCGCGCCTCTGCAAATGTTCTCACTCGCGTTGTATTTGATCTCAGTGGCCGGAGATA 320
241 tAGTCGtAGGAGTGTTtttAGAGTCGCGttTtTGtAAATGTTtTtAtTCGCGTTGTATTTGATtTtAGTGGtCGGAGATA 320
241              TTTAGAGTCGCGTTTT                                   TTAGTGGTCGGAGATA 320
241               TAGAGTTGTGTTTTTGT                                  AGTGGTTGGAGATA 320
241                                                                             GATA 320
241                                                                               TA 320
```

```
321 GAACAGCGCGGCCACGCGTGGGGCGCGGAGAGGAGTATCAACTACTGGCTTCCTTTACAGACGCAGATAACCTTTAAAAA 400
321 GAAtAGCGCGGttACGCGTGGGGCGCGGAGAGGAGTATtAAtTAtTGGtTTttTTTAtAGACGtAGATAAttTTTAAAAA 400
321 GA                                                                               400
321 GAATAGTGTGGTTA                                                                   400
321 GAATAGCGCGGTTA

401 GGAAACGGATAAGATCTAGACGATTGATTTGAAAGTGAAAGATGGATGTGACT                            480
401                             AAAGTGAAAGATGGATGTGATT                               480
401 GGAAACGGATAAGATtTAGACGATTGATTTGAAAGTGAAAGATGGATGTGAtT                            480
```

Figure 63: Amplificate of SEQ ID NO: 43

```
389 GGGGACAAGTGGTTAATGAGCAGGGCCAACTGGCCGGTCCCTGTCCTGTATGCCACACACACACCCCGTGAGGGCAGCAA 310
389 GGGGATAAGTGGTTAATGAGT                                                              310
389 GGGGAtAAGTGGTTAATGAGtAGGGttAAtTGGtCGGTtttTGTttTGTATGttAtAtAtAtAtttCGTGAGGGtAGtAA 310
389                                                             TATATTTTGTGAGGGTAG   310
389                                                             TATATTTCGTGAGGGTA    310

309 GGAGCATCCAGCCAGGGGACGTGCACGAGAAGGGCCAGGATGATCACACTGCCCGGGGCTCAGCACAGGGCTGAGTCCCG 230
309 GGAGtATttAGttAGGGGACGTGtACGAGAAGGGttAGGATGATtAtAtTGttCGGGGtTtAGtAtAGGGtTGAGTttCG 230
309         TAGGGGACGTGTACGA                                                         230
309         TAGGGGATGTGTATGA                                                         230

229 GAGGTGACCATTGCCGCCCCGCTCCATCTGCGGCCACTGAGGTCTGCTGGGGGTCTCTGGTGCTGGCCTTGACAGCCCAC 150
229 GAGGTGAttATTGtCGtttCGtTttATtTGCGGttAtTGAGGTtTGtTGGGGGTtTtTGGTGtTGGttTTGAtAGtttAt 150

149 CCGTGGCAGGACAGGGTCTCGAGGTGCCTCTCGGTAGGTGGTGGGCTCAGTGGGGGTGACCCGTGGGGACGGAACGCATG  70
149 tCGTGGtAGGAtAGGGTtTCGAGGTGttTtTCGGTAGGTGGTGGGtTtAGTGGGGGTGAttCGTGGGGACGGAACGtATG  70
149                                                                     GACGGAACGTATG  70
149                                                                  TGGGGATGGAATGTAT   70
149                                                              ATTCGTGGGGACGGAA       70
149                                                              ATTTGTGGGGATGGAA       70
```

| | | |
|---|---|---|
| 69 | CTCCTGAGGCTCCAGTGGATGCAGGCAAAGGCACCTCCCGGGGTCAGCGTCCCTACCTGGGGAGCTGGG | -10 |
| 69 | **TTTATTTGGGGAGTTGGG** | -10 |
| 69 | tTttTGAGGtTttAGTGGATGtAGGtAAAGGtAttTttCGGGGTtAGCGTtttTAttTGGGGAGtTGGG | -10 |
| 69 | | |

Figure 64: Amplificate of SEQ ID NO: 44

```
  1 ACAGTGTGGACCCTCAGGGACACCAGAGTCTCCGTGATGTTCTTGCGTATCTGGGCTCGGCGCTGCCGCTCGTGCTTGGG  80
  1 ATAGTGTGGATTTTTAGGGATATT                                                          80
  1 AtAGTGTGGAtttTtAGGGAtAttAGAGTtTtCGTGATGTTtTTGCGTATtTGGGtTCGGCGtTGtCGtTCGTGtTTGGG  80

 81 CTCTGCCGCCACGTCCAGGGCCCGCTCGTACTGGGGCAGGCAGGGGGCACAGCAAGCTGTCAGCAGGGCAGGAGGCCGGC 160
 81 tTtTGtCGttACGTttAGGGttCGtTCGTAtTGGGGtAGGtAGGGGGtAtAGtAAGtTGTtAGtAGGGtAGGAGGtCGGt 160
 81    TGTCGTTACGTTTAGG                                                              160
 81     GTTGTTATGTTTAGGGT                                                            160
 81              TTTAGGGTTTGTTTGTAT                                                  160
 81                TAGGGTTCGTTCGTATT                                                 160

161 AGGAGGCCAGCAGATGCCCACGACTCCCGGGGTGCAGTTACGTGCTAGATGCTGTGTGATGTGGGCACTGACCCGCAACA 240
161 AGGAGGttAGtAGATGtttACGAtTttCGGGGTGtAGTTACGTGtTAGATGtTGTGTGATGTGGGtAtTGAttCGtAAtA 240
161                TTTATGATTTTTGGGGT                                                 240
161                 TTACGATTTTCGGGGT                                                 240

241 CTGAGCTGTTTCTTCATGGGCAAAACAGGGTAAGCACATGGGCCCTCCTGGGCGGGGGCTGCATTGTGGAAAGCAGACGC 320
241 tTGAGtTGTTTtTTtATGGGtAAAAtAGGGTAAGtAtATGGGtttTttTGGGCGGGGGtTGtATTGTGGAAAGtAGACGt 320
241                                                                       AAGTAGACGT 320
241                                                                          TAGATGT 320
```

```
321 CGGAGAGGGCCCGGTGGGTGTGGCTGCTGGGAGCGGAACGTCGGGGTGCTGCTTCAGGGTCACTGGGATTTATCTCTGGG 400
321 CGGAGAGGGttCGGTGGGTGTGGtTGtTGGGAGCGGAACGTCGGGGTGtTGtTTtAGGGTtAtTGGGATTTATtTtTGGG 400
321 CGGAGA                                                                            400
321 TGGAGAGGG

401 GCCCGGGATGAGCCCTCCGCAAAGCTCCAGGCAGGGGAACAGGTCTTG                                  480
401                                 AGGTAGGGGAATAGGTTTTG                              480
401 GttCGGGATGAGtttTtCGtAAAGtTttAGGtAGGGGAAtAGGTtTTG                                  480
```

Figure 65: Amplificate of SEQ ID NO: 45

```
  1 GTAGCAAATGGGCTATGGACTTCAGTAACTAGAGTCACGTGTTACCGGCGACACAACCAATGAGGGCGCGAGATCTGTGT  80
  1 GTAGTAAATGGGTTATGGATTTTAG                                                        80
  1 GTAGtAAATGGGtTATGGAtTTtAGTAAtTAGAGTtACGTGTTAtCGGCGAtAtAAttAATGAGGGCGCGAGATtTGTGT  80
  1                                             TACGTGTTATCGGCGA                     80
  1                                             TATGTGTTATTGGTGATA                   80

 81 AAGCAGGGGGAGGCACGTGCCGGGCCGCACGTGTCTGGGAGGGGGGGAAGGGGCGGGGCCCACTGAGAGAGGCGGAGGTG 160
 81 AAGtAGGGGGAGGtACGTGtCGGGtCGtACGTGTtTGGGAGGGGGGGAAGGGGCGGGGtttAtTGAGAGAGGCGGAGGTG 160
 81            GTATGTGTTGGGTTGTA                                                     160
 81             TACGTGTCGGGTCGTA                                                     160

161 GGTAGATAGACCCGGAGAGACGGCGAAGGAGCTGGAAACCGAGCCAGGGCTGAGCCGGCTGACAACAGGTAAGGAGATTC 240
161 GGTAGATAGAttCGGAGAGACGGCGAAGGAGtTGGAAAtCGAGttAGGGtTGAGtCGGtTGAtAAtAGGTAAGGAGATTC 240
161       TAGATTTGGAGAGATGG                                                          240
161        AGATTCGGAGAGACGG                                                          240

241 GGAGGACAAAGCGAGCTGTTAGGGAGTATTTGGGAGTGGATTTGGGGGCTGAATGCAGATCTTGGTATTGGGAGGGTTTG 320
241 GGAGGAtAAAGCGAGtTGTTAGGGAGTATTTGGGAGTGGATTTGGGGGtTGAATGtAGATtTTGGTATTGGGAGGGTTTG 320
```

```
321 CATCACGTGGCAGGTGCGAGCCCAGAGAGACCGGCGCAGGGATCCTGATCTTGAAAGATTGGGAGAGGGCAGGAGGCCAG 400
321 tATtACGTGGtAGGTGCGAGtttAGAGAGAtCGGCGtAGGGATttTGATtTTGAAAGATTGGGAGAGGGtAGGAGGttAG 400
321                                          AGATCGGCGTAGGGAT                                         400
321                                          AGATTGGTGTAGGGAT

401 TCTCTATGGGGGCGGTTCCCGTAGGATCACCAGGTGGGTTCGCGTGCCCAGATTGACTGCTTGTGGGTGGCATGG     480
401                                                                 TGTTTGTGGGTGGTATGG     480
401 TtTtTATGGGGGCGGTTttCGTAGGATtAttAGGTGGGTTCGCGTGtttAGATTGAtTGtTTGTGGGTGGtATGG     480
```

Figure 66. Amplificate of SEQ ID NO: 46

```
  1 CCCCAGCAGTGGACCTGGTCAGCACCTCTGGGGCTGGGACATCAGTGTCCTAAAGGTGGAAGGGTTAGACCCTCTAGGGG  80
  1 TTTTAGTAGTGGATTTGGTTAGTATT                                                        80
  1 ttttAGtAGTGGAttTGGTtAGtAttTtTGGGGtTGGGAtATtAGTGTttTAAAGGTGGAAGGGTTAGAtttTtTAGGGG  80

 81 AAGGGCCCACCGCCCTCCACAGAGGCTCTGGCTTAGGCCGCGTGGACACGTGAGGGGGGCACCTACCGTGTTCTCCATGG 160
 81 AAGGGtttAtCGtttTttAtAGAGGtTtTGGtTTAGGtCGCGTGGAtACGTGAGGGGGGtAttTAtCGTGTTtTttATGG 160
 81                                            GGTTGTGTGGATATGT                       160
 81                                             GTCGCGTGGATACGTG                      160

161 ACTTGCTGGCGACTCCCACGAGAAGGCCAGCCAGGAGGGCGAGGTGCCGCAGCGCCATGCCAGGAGCAGATGCGCAGAGC 240
161 AtTTGtTGGCGAtTtttACGAGAAGGttAGttAGGAGGGCGAGGTGtCGtAGCGttATGttAGGAGtAGATGCGtAGAGt 240
161     TTGGCGATTTTTACGA                              AGGTGTTGTAGTGTTAT                 240
161     TTGGTGATTTTTATGAGA                               TGTCGTAGCGTTATGT               240
161          GATTTTTATGAGAAGGTT                                                     240
161           ATTTTTACGAGAAGGTT

241 CTGCCACAGGGAGGAGCATGCGGAGCCAAAGAGATGGAGTGGGGCTGAGGCAGGGTGGGTGGGGCCAAATGAAAGTGGGG 320
241                                                                      TGAAAGTGGGG 320
241 tTGttAtAGGGAGGAGtATGCGGAGttAAAGAGATGGAGTGGGGtTGAGGtAGGGTGGGTGGGGttAAATGAAAGTGGGG 320
```

```
321 TCAAGAGATG                400
321 TTAAGAGATG                400
321 TtAAGAGATG                400
```

Figure 67: Amplificate of SEQ ID NO: 47

```
335 GAGGAAAGAAGGAGGATACAGGCGGCCAATGTGTGGCCTACGTGGGAGCGCCCCGAGCGCTCCACGTCCCCGGCCTGCGC 256
335 GAGGAAAGAAGGAGGATATAGG                                                            256
335 GAGGAAAGAAGGAGGATAtAGGCGGttAATGTGTGGttTACGTGGGAGCGtttCGAGCGtTttACGTtttCGGttTGCGt 256
335                                                  TTTTGAGTGTTTTATGTT               256
335                                                   TTCGAGCGTTTTACGT                256
335                                  TTACGTGGGAGCGTTT                                 256
335                                  TTATGTGGGAGTGTTT                                 256

255 CGGGCGGCCCCTTCCCCGGGCATCTTGGCAACGCGCCTGGCCCAAGCCGGAGGGGCCCCGCGGCCCCGCACGCCTCCTGG 176
255 CGGGCGGttttTTtttCGGGtATtTTGGtAACGCGttTGGtttAAGtCGGAGGGGtttCGCGGtttCGtACGttTttTGG 176
255                           GTAACGCGTTTGGTTT                                        176
255                          TGGTAATGTGTTTGGT                                         176

175 ACCAGTCAGGATCCAGGCTCCTCTTTGAGCCCGCGGGTGTTTCATGGGGGTAGAAGTCTAACCCCTCCACCCCCTCTCCT  96
175 AttAGTtAGGATttAGGtTttTtTTTGAGttCGCGGGTGTTTtATGGGGGTAGAAGTtTAAttttTttAtttttTtTttT  96
```

| | | |
|---|---|---|
| 95 | CCCCAGCAGTCCCACGCGGGTATGGGAGAGAATGAAGTTCTTTGTCTCTAAGGGATTCAAACCAGAAACGGAGGGACCTC | 16 |
| 95 | **GATTTT** | 16 |
| 95 | ttttAGtAGTtttACGCGGGTATGGGAGAGAATGAAGTTtTTTGTtTtTAAGGGATTtAAAttAGAAACGGAGGGAttTt | 16 |
| 95 | AGTTTTATGTGGGTATG | 16 |
| 95 | TAGTTTTACGCGGGTA | |
| 15 | TGGTTCCCAGAGGGA | -64 |
| 15 | **TGGTTTTTAGAGGGA** | -64 |
| 15 | TGGTTtttAGAGGGA | -64 |

Figure 68: Amplificate of SEQ ID NO: 48

```
  1 GCCAAAGGGGCATTGGTTCCCCAAGGGCCAAACGAGCTTCCTCCAGGAGTTTCCCGTCGCGTGCAGTGGACCACCGTGGG  80
  1 GTTAAAGGGGTATTGGTTTTT                                                             80
  1 GttAAAGGGGtATTGGTTttttAAGGGttAAACGAGtTTttTttAGGAGTTTttCGTCGCGTGtAGTGGAttAtCGTGGG  80

 81 CCTGACTGAGGCCTGGTCCTTGCCAGGGTTTTTTGGCAACGGCCCTGGATGAGTGAAGGGATGAAAGGCGCGAGAAGAAA 160
 81 ttTGAtTGAGGttTGGTttTTGttAGGGTTTTTTGGtAACGGtttTGGATGAGTGAAGGGATGAAAGGCGCGAGAAGAAA 160
 81                                                               ATGAAAGGCGCGAGAA    160
 81                                                               ATGAAAGGTGTGAGAA    160

161 TGGGCTGAGAAACCAAAGTGCGGGGCCCTGAGTGTTTGAGGGGACGCGATGGGAACCGTGGCTCCGCTGCCCTGATTTTA 240
161 TGGGtTGAGAAAttAAAGTGCGGGGtttTGAGTGTTTGAGGGGACGCGATGGGAAtCGTGGtTtCGtTGtttTGATTTTA 240
161                                                       ATGGGAATCGTGGTTT           240
161                                                       ATGGGAATTGTGGTTT           240
161                                                          GGAATTGTGGTTTTGT        240
161                                                           GAATCGTGGTTTCGTT       240

241 AGGGCAGTTACCCTGTCACAGCCACGCTGGAACTGAGGTGTCCTTCCAGCTTCTGTCCACCTCAGAGGAATTCAAAACAA 320
241 AGGGtAGTTAtttTGTtAtAGttACGtTGGAAtTGAGGTGTttTTttAGtTTtTGTttAttTtAGAGGAATTtAAAAtAA 320
```

```
321 GGGCCCAGTTCTAAGCCCCACAGACCCCAGGATCCCCTAGGGGCCATTAGTCCACCGCGGCAAATGGTTTCCTCACTTTG 400
321 GGGtttAGTTtTAAGttttAtAGAttttAGGATttttTAGGGGttATTAGTttAtCGCGGtAAATGGTTTttTtAtTTTG 400
321                                                                  TAGTTTATCGCGGTAA    400
321                                                                     TTTATTGTGGTAAATGGT

401 CTCATTTGTGTTTGTTTTGAAATCCAATTGTCACCTCACTGGGCTGCACAGTTGCTACTGAAACGGTTCAATAGGTGAGA 480
401                                                                 GTTTAATAGGTGAGA 480
401 tTtATTTGTGTTTGTTTTGAAATttAATTGTtAttTtAtTGGGtTGtAtAGTTGtTAtTGAAACGGTTtAATAGGTGAGA 480

481 GAAAAAGAAA                                                                       560
481 GAAAAAGAAA                                                                       560
481 GAAAAAGAAA                                                                       560
```

Figure 69: Amplificate of SEQ ID NO: 49

```
  1 GTAAGAGGGACAGGGAACTGGGGCGCTGGAGCCGGGAAGCGGGTGAGCGAGTCTTCCCAACCGCTATGTCCCCTATTAAA  80
  1 GTAAGAGGGATAGGGAATTGG                                                             80
  1 GTAAGAGGGAtAGGGAAtTGGGGCGtTGGAGtCGGGAAGCGGGTGAGCGAGTtTTtttAAtCGtTATGTttttTATTAAA  80

 81 AAGAACCCCGCTCCTTCGTGAAACCCCTTTTCCCTTAGCCTTTAAGCTGCCGGGCCGCGACGGGGAACTGGCCAACTCAT 160
 81 AAGAAtttCGtTttTTCGTGAAAttttTTTTtttTTAGttTTTAAGtTGtCGGGtCGCGACGGGGAAtTGGttAAtTtAT 160

161 CCTTCGCCGGGCTCTCCGCCCTCCGGGCTCTGGGGCAGAACAGGGTCCTGAGGCGGCGTGGGCGAGGGCAGTCAATGAGC 240
161 ttTTCGtCGGGtTtTtCGtttTtCGGGtTtTGGGGtAGAAtAGGGTttTGAGGCGGCGTGGGCGAGGGtAGTtAATGAGC 240

241 GGGAAGGACGTGAAGAAGGTGACACAGGCTGCCCCCCACTGGAAAATGGGTTGGGAGGGGGTGCGGCCCGAAGACCAGAG 320
241 GGGAAGGACGTGAAGAAGGTGAtAtAGGtTGtttttAtTGGAAAATGGGTTGGGAGGGGGTGCGGttCGAAGAttAGAG 320
241                                                                GGTGTGGTTTGAAGAT   320
241                                                                  TGCGGTTCGAAGATTA 320
```

```
321 GATAGCAAAGGTCCACGGAAAAGGGAGCTCCTGGCCCGGACGCGGCGTGGGGAACGCGTCCTAGCGCCGGGGATGCGGAG 400
321 GATAGtAAAGGTttACGGAAAAGGGAGtTttTGGttCGGACGCGGCGTGGGGAACGCGTttTAGCGtCGGGGATGCGGAG 400
321             AAGGTTTATGGAAAAGG                                          GGAATGTGTTTTAGTGT                400
321              AGGTTTACGGAAAAGG                                           GAACGCGTTTTAGCGT                400
321                                                                                TTTTAGCGTCGGGGAT         400
321                                                                                TTTTAGTGTTGGGGAT

401 CTGGGGAGAGGGGTGGGAGCCCCATCCTGGTTTGGGTGCC                                                         480
401                    TTTTATTTTGGTTTGGGTGTT                                                         480
401 tTGGGGAGAGGGGTGGGAGttttATttTGGTTTGGGTGtt                                                         480
```

Figure 70: Amplificate of SEQ ID NO: 50

```
  1 GGAGTTTGTGAAAAGTGGGGCTCGGGGCCCAGTCAATGGGGCGCCCCGCGGCGCGGGCTGAGTGGAGCTAGCGCGAACCG  80
  1 GGAGTTTGTGAAAAGTGGG                                                              80
  1 GGAGTTTGTGAAAAGTGGGGtTCGGGGtttAGTtAATGGGGCGtttCGCGGCGCGGGtTGAGTGGAGtTAGCGCGAAtCG  80
  1                                                                     TAGCGCGAATCG  80
  1                                                                      AGTGTGAATTG  80

 81 CTCAGCCGCGGCCCCAATTAATCCGCCCTTTGTGCGGCCCGCCCGGCCGCCCCCGCCGCAGCCGCACCAGCGGCCCATTG 160
 81 tTtAGtCGCGGttttAATTAATtCGtttTTTGTGCGGttCGttCGGtCGttttCGtCGtAGtCGtAttAGCGGtttATTG 160
 81 TTTA                                                           TAGTCGTATTAGCGGT     160
 81 TTTAGT                                                         TAGTTGTATTAGTGGTTT   160

161 TTCGGCCTCGCCGGGCCGCGGGATTTACCCTTTTCAAACAGCCGGTTTTGTCCAGGGCAGTTCGAGCGGAAGTTTCTCAC 240
161 TTCGGttTCGtCGGGtCGCGGGATTTAtttTTTTtAAAtAGtCGGTTTTGTttAGGGtAGTTCGAGCGGAAGTTTtTtAt 240

241 TGACAATTTGCCCCAAATAGATAGATTTGTCAGAAGCGACCTTCGGGAAGGAAGCAAAAAGCCACCGGCCCGAAGGTTGG 320
241 TGAtAATTTGttttAAATAGATAGATTTGTtAGAAGCGAttTTCGGGAAGGAAGtAAAAAGttAtCGGttCGAAGGTTGG 320
```

321 GCCCAAAACAGGGACTCTGCGTCCCACCCGCGGCCGCGCCGCCCCCCCGCGCCCCCGGCCCTGCAGTCCCGAAGCCCCGC 400
321 GtttAAAAtAGGGAtTtTGCGTtttAttCGCGGtCGCGtCGttttttCGCGttttCGGtttTGtAGTttCGAAGtttCGC 400
321 TGCGTTTTATTCGCGG TTTCGAAGTTTCGC 400
321 TGTGTTTTATTTGTGGT TTTGAAGTTTTGT 400

401 GGGGGTCCAGACCACTGCACCTGCTGAGC 480
401 **GGTTTAGATTATTGTATTTGTTGAGT** 480
401 GGGGGTttAGAttAtTGtAttTGtTGAGt 480
401 GG 480
401 GGG

Figure 71: Amplificate of SEQ ID NO: 51

```
228 GAAGGTTGAGGAGGAGCCAGAGCCGGGTCCTGCAGCGTTTCTCGCCATCAGCGCCCGTCGCCATCTCCACCATGCAGTCC 149
228 GAAGGTTGAGGAGGAGTTAGA                                                            149
228 GAAGGTTGAGGAGGAGttAGAGtCGGGTttTGtAGCGTTTtTCGttATtAGCGttCGTCGttATtTttAttATGtAGTtt 149
228                         TAGAGTCGGGTTTTGTA            TATTAGCGTTCGTCGT            149
228                         TAGAGTTGGGTTTTGTA         GTTATTAGTGTTTGTTGT             149
228                                                                              TTT 149
228                                                                            AGTTT 149

148 CGGGAAGACGCCCCGCGCTCTCGCCGCCTAGCCAGTCCCCGTGGTGGGAAGCGGCCCAAGAAGATTCACAAACCCACAGT 69
148 CGGGAAGACGtttCGCGtTtTCGtCGttTAGttAGTtttCGTGGTGGGAAGCGGtttAAGAAGATTtAtAAAtttAtAGT 69
148        AGACGTTTCGCGTTTT                                                          69
148     GAAGATGTTTTGTGTTT                                                            69
148 CGGGAAGACGTTT                                                                    69
148 TGGGAAGATGT

 68 TTCGGCCTTTTTCACGGGTCCAGAGGAATTAAAGGACACGGCCCATTCTGCAGCCCTGCTGGCACAGC         -11
 68                                                 TTTATTTTGTAGTTTTGTTGGTATAGT  -11
 68 TTCGGttTTTTTtACGGGTttAGAGGAATTAAAGGAtACGGtttATTtTGtAGtttTGtTGGtAtAGt         -11
```

Figure 72 Amplificate of SEQ ID NO: 52

```
  1 GGCTGGAAAGTGGAGGATCCGGTTTGCTCTGGGCGGGTCTGGAAGCAGAGCCGGCGGAGGGAGCGCCGGGGCCCTGGGCT  80
  1 GGTTGGAAAGTGGAGGATT                                                               80
  1 GGtTGGAAAGTGGAGGATtCGGTTTGtTtTGGGCGGGTtTGGAAGtAGAGtCGGCGGAGGGAGCGtCGGGGtttTGGGtT  80
  1                                           AGTAGAGTCGGCGGAG                       80
  1                                           AGTAGAGTTGGTGGAG                       80

 81 GCAGGAGGTTGCGGCGGCCGCGGCAGCATGGTGGTGCCGGAGAAGGAGCAGGTGAGCGCCGGACCAGGGTCTGCGGGAGC 160
 81 GtAGGAGGTTGCGGCGGtCGCGGtAGtATGGTGGTGtCGGAGAAGGAGtAGGTGAGCGtCGGAttAGGGTtTGCGGGAGC 160
 81                                                TAGGTGAGCGTCGGAT                  160
 81                                                TAGGTGAGTGTTGGAT                  160

161 GCGGAGCTGGGGACCTCGCCTCCAGGCTCCCAGAGAGGAGGGCGCTGGACACCCAGATTCCTGGGTCAGACGGAGGAGGG 240
161 GCGGAGtTGGGGAttTCGttTttAGGtTtttAGAGAGGAGGGCGtTGGAtAtttAGATTttTGGGTtAGACGGAGGAGGG 240

241 GGATGGGAGGGTCCAGGTTCCAGGGTCCAGGGCATGGGGGTCGAGACTCCTGAGTCTGGAGCGGGAGGGCGCTGGGGGCC 320
241 GGATGGGAGGGTttAGGTTttAGGGTttAGGGtATGGGGGTCGAGAtTttTGAGTtTGGAGCGGGAGGGCGtTGGGGGtt 320
241                                  ATGGGGGTCGAGATTT                                320
241                                  ATGGGGGTTGAGATTT
```

```
321 CGGACTCCTGTGTCCGTGGGGAGCGCACGGGGTGGGTGGCTCTGTGTCCCATAGGACAGAACTGGGTGCCCTCTCACCCC 400
321 CGGAtTttTGTGTtCGTGGGGAGCGtACGGGGTGGGTGGtTtTGTGTtttATAGGAtAGAAtTGGGTGtttTtTtAtttt 400

401 ACTTCCACCCCTACATTTGTTCCTGTCCCCAGAGCTGG                                           480
401                        TTGTTTTTGTTTTTAGAGTTGG                                           480
401 AtTTttAttttTAtATTTGTTttTGTttttAGAGtTGG                                           480
```

Figure 73: Amplificate of SEQ ID NO: 53

```
  1 GAGCTGGCCCTGCTGAGGTGCCGGGCCGAGTAGTTGGAGAGAGGGTCATACTCCAGGTCTGTGGGTGGCCTGGAGTTGTC  80
  1 GAGTTGGTTTTGTTGAGGTGT                                                            80
  1 GAGtTGGtttTGtTGAGGTGtCGGGtCGAGTAGTTGGAGAGAGGGTtATAtTttAGGTtTGTGGGTGGttTGGAGTTGTt  80
  1                          TGTCGGGTCGAGTAGT                                        80
  1                          TGTTGGGTTGAGTAGT                                        80

 81 CACCACGTACTTGCCACTGGGAACGGGGCGGCTGTGCCGCCGGGGCTGGCTCACAGCCTTGGGGACGTATTCCAGGGCAC 160
 81 tAttACGTAtTTGttAtTGGGAACGGGGCGGtTGTGtCGtCGGGGtTGGtTtAtAGttTTGGGGACGTATTttAGGGtAt 160

161 CGCCACCCCCTCCACCTCTGCCCTGACCCCTGTCCAGGGACGCCAGCGAGTACTTGCTGCCCGGCTCGGCAGGGGCGGCC 240
161 CGttAtttttTttAttTtTGtttTGAttttTGTttAGGGACGttAGCGAGTAtTTGtTGttCGGtTCGGtAGGGGCGGtt 240
161                                                 AGGGATGTTAGTGAGT                 240
161                                                     GACGTTAGCGAGTATT             240

241 AGTGGGGTGGGCTGGTAGCCGGCATCAGGGCTTAATAGGCCGTGGCTGCCGGGGCTGTAGTCGAAGGCCAGTGGGAAGGC 320
241                                                                AAGGTTAGTGGGAAGGT 320
241 AGTGGGGTGGGtTGGTAGtCGGtATtAGGGtTTAATAGGtCGTGGtTGtCGGGGtTGTAGTCGAAGGttAGTGGGAAGGt 320
241                   TGGTAGTTGGTATTAGG                                TGTAGTCGAAGGTTAG     320
241                    GGTAGTCGGTATTAGG                               TGTAGTTGAAGGTTAGT
```

| | | |
|---|---|---|
| 321 | ATCCT | 400 |
| 321 | **ATTTT** | 400 |
| 321 | ATttT | 400 |

Figure 74: Amplificate of SEQ ID NO: 54

```
  1 CAGTGGAACCATGAGGGGGGAGCCCACCCGGCTCACACGACCTCTCCCTCCGGAGGCCGCTCTAAGCACACCCTCCAGCC  80
  1 TAGTGGAATTATGAGGGGG                                                               80
  1 tAGTGGAAttATGAGGGGGGAGtttAttCGGtTtAtACGAttTtTttttTtCGGAGGtCGtTtTAAGtAtAtttTttAGtt  80
  1                         GGAGTTTATTCGGTTTAT                                      TT  80
  1                         GGAGTTTATTTGGTTTATA                                      T  80
  1                                    ATTTGGTTTATATGATTTTT                             80
  1                                     TTCGGTTTATACGATTT                               80

 81 ACGCCGACCACCGGGGCTGTCCCCGATTCTCACCTAAAACCCCCTTCAGCCCACAGCCCCCACGGCCTTCCTGTGGTCTC 160
 81 ACGtCGAttAtCGGGGtTGTtttCGATTtTtAttTAAAAtttttTTtAGtttAtAGtttttACGGttTTttTGTGGTtTt 160
 81 ACGTCGATTATCGG                                                                   160
 81 ATGTTGATTATTGGGGT                                                                160

161 CTCCCTCCTTCACGGCGGGTCCTCATCCTTTACCTTCTCTCTCTGGAGCCCAAATTGGAACTCCGCTCCTCCCCTCCGCG 240
161 tTtttTttTTtACGGCGGGTttTtATttTTTAttTTtTtTtTtTGGAGtttAAATTGGAAtTtCGtTttTttttTtCGCG 240
161         TTACGGCGGGTTTTTA                                                         240
161         TTATGGTGGGTTTTTATT

241 CAGCTCCATGCACTGGCCCTTTGGGACGCCTCTTTCTTTCTTTGGCAGAGGTGCATCTGG                     320
241                                                TTTTGGTAGAGGTGTATTTGG              320
241 tAGtTttATGtAtTGGtttTTTGGGACGttTtTTTtTTTtTTTGGtAGAGGTGtATtTGG                     320
```

Figure 75: Amplificate of SEQ ID NO: 55

```
384 TAGGACTTGTGGCTGGTGTTCTCGGCCATGCCCTGGCCCAAGAAGTCGTTGGTGAAGATGCCTCAGCGGAGCGGGGCGCC 305
384 TAGGATTTGTGGTTGGTGTT                                                                305
384 TAGGAtTTGTGGtTGGTGTTtTCGGttATGtttTGGtttAAGAAGTCGTTGGTGAAGATGttTtAGCGGAGCGGGGCGtt 305

304 CGCCGGGCCGCACGTTGGGGTTGTTCACGGTCGCCCACACGTCGTCGTGCACGAACTCGCCCTGCAGGGAGCGGCCTTAG 225
304 CGtCGGGtCGtACGTTGGGGTTGTTtACGGTCGtttAtACGTCGTCGTGtACGAAtTCGtttTGtAGGGAGCGGttTTAG 225
304                                         GTTGTGTATGAATTTGTT                       225
304                                         GTCGTGTACGAATTCGT                        225

224 CACAGGCAGCTCGCCCCGGCCAGCAGCACCTCTTCCTCCCCACTGCGAATGCCCCGATCATGCTCCCCCAGACCCAACAC 145
224 tAtAGGtAGtTCGtttCGGttAGtAGtAttTtTTttTttttAtTGCGAATGtttCGATtATGtTtttttAGAtttAAtAt 145
224       AGTTTGTTTTGGTTAGT                                                          145
224      TAGTTCGTTTCGGTTA                                                            145

144 ACAAACACCCTTGGGAACCCACGCGGGTGCCTTCTGTTTACCGCAGCGCTCAAGCCGCGGGCGCTTGAGTCCCAGATGGC  65
144 AtAAAtAtttTTGGGAAtttACGCGGGTGttTTtTGTTTAtCGtAGCGtTtAAGtCGCGGGCGtTTGAGTtttAGATGGC  65
144              AATTTACGCGGGTGTT                AGCGTTTAAGTCGCGG                     65
144            GGAATTTATGTGGGTG                 TAGTGTTTAAGTTGTGG
```

```
64 GAAAGTTCGCGCGCAGGTGGAGTGGGGATCCCGGCGTGCGGCTGCCCAGGAGAGGAGGGCAAGG   -15
64                                                   TTTAGGAGAGGAGGGTAAGG   -15
64 GAAAGTTCGCGCGtAGGTGGAGTGGGGATttCGGCGTGCGGtTGtttAGGAGAGGAGGGtAAGG   -15
```

Figure 76: Amplificate of SEQ ID NO: 56

```
  1 GGCTCAGGGCACCCTAAGGGGCCGGATTTCCAGGGGTCCCATCTTGGCTGTGGTGGAGAGCTGGACTCAGGTCTCTCTTG  80
  1 GGTTTAGGGTATTTTAAGGGG                                                              80
  1 GGtTtAGGGtAtttTAAGGGGtCGGATTTttAGGGGTtttATtTTGGtTGTGGTGGAGAGtTGGAtTtAGGTtTtTtTTG  80

 81 GGGGCCCTGGGGACCAGAGCCACGATCCCGGTGCCCTGATGTCCCCGTCTCTTCCCCACCTTCCTCTCCCCCAGTGTCTG 160
 81 GGGGtttTGGGGAttAGAGttACGATttCGGTGtttTGATGTtttCGTtTtTTttttAttTTttTtTttttttAGTGTtTG 160
 81                TAGAGTTATGATTTTGGT                                                160
 81                     TTACGATTTCGGTGTT                                             160

161 CCTCAGGACGAGGCGTGCGCCGACACTGGCAGTGGCAGCGCCGAGGGCCTGGCTGCTGACGGCCCCCACCTGCACACGCT 240
161 ttTtAGGACGAGGCGTGCGtCGAtAtTGGtAGTGGtAGCGtCGAGGGttTGGtTGtTGACGGttttAttTGtAtACGtT 240
161           AGGCGTGCGTCGATAT        GTAGCGTCGAGGGTTT                       TTGTATATGTT 240
161           AGGTGTGTGTTGATATT       GTAGTGTTGAGGGTTT                           ATACGTT 240

241 GACGCAGCCTATCGTGGTCACCGTGCCGCGGCCGCCCCCCAGTGAGCACGCAGTCCTCCTCCGCATGGGGCCGTGGGGCG 320
241 GACGtAGttTATCGTGGTtAtCGTGtCGCGGtCGtttttAGTGAGtACGtAGTttTttTtCGtATGGGGtCGTGGGGCG 320
241 GATGTAGT                                                                         320
241 GACGTAGTT
```

```
321 GCAGAGCGGGTGGGAAGGACGCCTGGGAGCTGGACCCAGTCT    400
321                     TTTGGGAGTTGGATTTAGTTT    400
321 GtAGAGCGGGTGGGAAGGACGttTGGGAGtTGGAtttAGTtT    400
```

Figure 77: Amplificate of SEQ ID NO: 57

```
378 GGGGAGGTGCTCAGTGGTCCTAGTCGCCCCGAGACCCTAGCTCTTCTCGCCCGCTGCGGGCCTGACTGCGCCAGGGGCCG 299
378 GGGGAGGTGTTTAGTGGTT                                                              299
378 GGGGAGGTGtTtAGTGGTttTAGTCGtttCGAGAtttTAGtTtTTtTCGttCGtTGCGGGttTGAtTGCGttAGGGGtCG 299
378                          TTAGTCGTTTCGAGAT                                        299
378                      GTTTTAGTTGTTTTGAGA                                          299

298 GGGGTTATGTGGGCAGGCTCGAACGACTGGGAGCGTGGCCTGTGATTCTGCTCCACTCCTTTCGCCCGCAGAGCGGTGGT 219
298 GGGGTTATGTGGGtAGGtTCGAACGAtTGGGAGCGTGGttTGTGATTtTGtTttAtTttTTTCGttCGtAGAGCGGTGGT 219

218 CTAGGCCAGACCATTGACCTGGGTAGGGGGAAGGGCAAGGACGCAGCTCGCACGCCTGGGTTTTCACCGCCGTCTCCCTT 139
218 tTAGGttAGAttATTGAttTGGGTAGGGGGAAGGGtAAGGACGtAGtTCGtACGttTGGGTTTTtAtCGtCGTtTttTT 139
218                                                     GACGTAGTTCGTACGT             139
218                                                    GGATGTAGTTTGTATGT             139

138 CGGCGTTTTGGGCTGGCGGCCGACATTGGCCTGGACCAAATGGTGGCAAAGTCTCACCAGTTCCGCGTCTCGCAAGACAC  59
138 CGGCGTTTTGGGtTGGCGGtCGAtATTGGttTGGAttAAATGGTGGtAAAGTtTtAttAGTTtCGCGTtTCGtAAGAtAt  59
138                                                                TTTGTGTTTTGTAAGATAT  59
138                                                                TTCGCGTTTCGTAAGA     59
```

| | | |
|---|---|---|
| 58 | TTTCTACGCGCATCGGGACCTAAACACTCGGGAGGACCCTGGTAAAGTGAGCAGAGGG | -21 |
| 58 | **TTTTGGTAAAGTGAGTAGAGGG** | -21 |
| 58 | TTTtTACGCGtATCGGGAttTAAAtAtTCGGGAGGAtttTGGTAAAGTGAGtAGAGGG | -21 |
| 58 | TATGTGTATTGGGATTTA | -21 |
| 58 | TTACGCGTATCGGGAT | |

Figure 78: Amplificate of SEQ ID NO: 58

1 TGTTGGTGGCCATCTTTAATCCTCCTCCTCCTGCTTTCTCCACCTCCCGCTGGCTGTCTGACTGACTGACTGGATCC 80
1 **TGTTGGTGGTTATTTTTAATTTTT** 80
1 TGTTGGTGGttATtTTTAATttTttTttTttTttTGtTTTtTttAttTttCGtTGGtTGTtTGAtTGAtTGAtTGGATtt 80

81 TCCTCTTTCCCCTCCTGCTCCTCCTACTGAGCCGGCCGCAGAAATTGCAGCCGCTCAGCTTCTACCCCCTCCTGCCTTTC 160
81 TttTtTTTttttTttTGtTttTttTAtTGAGtCGGtCGtAGAAATTGtAGtCGtTtAGtTTtTAtttttTttTGttTTTt 160
81 TATTGAGTCGGTCGTA AATTGTAGTCGTTTAGTT 160
81 ATTGAGTTGGTTGTAGA 160
81 AAATTGTAGTTGTTTAGTT 160

161 CTTCCTCTTTCCTTACTTCCTTCCCTTCCCTCGGCTTCCCGCTCTTGCCTCACTCTCAGCGGCTGCCTTCGCCCCTGTCT 240
161 tTTttTtTTTttTTAtTTttTTtttTTtttTCGGtTTttCGtTtTTGttTtAtTtTtAGCGGtTGttTTCGttttTGTtT 240

241 GCAGACAGCGCCGCTGGATGCTCCCAGCTGGACTTCAACCCCACTCCTCTCAGTCCCTCTCCCCACTGCCTTCCAGACGC 320
241 GtAGAtAGCGtCGtTGGATGtTtttAGtTGGAtTTtAAttttAtTttTtTtAGTtttTtTttttAtTGttTTttAGACGC 320
241 TAGATAGCGTCGTTGG 320
241 TAGATAGTGTTGTTGGA

```
321 GCCTCTTCCCCGCCCCGCGCCCCTCTCTCCTCTCCCACCCCTGCCCCTCTCCGCGGCGCTCACCCTCCTCAGTCCCAGTT 400
321 GttTtTTtttCGtttCGCGttttTtTtTttTtTtttAttttTGttttTtTtCGCGGCGtTtAtttTttTtAGTtttAGTT 400

401 TCTGAAAGGACTCAGCTGAGAAAGGACAACTGGGT                                              480
401                     GTTGAGAAAGGATAATTGGGT                                        480
401 TtTGAAAGGAtTtAGtTGAGAAAGGAtAAtTGGGT                                              480
```

Figure 79: Amplificate of SEQ ID NO: 59

```
  1 GAGGACCTGTGCTAAGGCTTTCTCATCCACCAGGCCACCATGGGCTGCGTTCACAAGGAATGCTCCCTGTCTCATCTGCT  80
  1 GAGGATTTGTGTTAAGGTTTTT                                                            80
  1 GAGGAttTGTGtTAAGGtTTTtTtATttAttAGGttAttATGGGtTGCGTTtAtAAGGAATGtTtttTGTtTtATtTGtT  80
  1                                                  ATGGGTTGCGTTTATA                 80
  1                                                   TGGGTTGTGTTTATAAG               80

 81 TTATAGTAAAGTCATTGACGAGGTGGTGGTTATGTTCATTGAGATTGCAGTGCAACGAGACACAGTCACTCTGATACAGC 160
 81 TTATAGTAAAGTtATTGACGAGGTGGTGGTTATGTTtATTGAGATTGtAGTGtAACGAGAtAtAGTtAtTtTGATAtAGt 160

161 AAACCCTGCAGGGTGTATCAGGGTCCCCTCTGCATGCCCTGGGACCTCTCTATCTTGTCCTACAAGTAGGGGTCATAAAA 240
161 AAAtttTGtAGGGTGTATTtAGGGTttttTtTGtATGtttTGGGAttTtTtTATtTTGTttTAtAAGTAGGGGTtATAAAA 240
161                                                                           ATAAAA 240
161                                                                           ATAAAA 240

241 TACGACGCTGAATCCAAAGGCCTTGGCTCAAACTGCAACCGCCTGCCTCATGCAACCGAAGCCCATGAGGCCTAGCGTCT 320
241 TACGACGtTGAATttAAAGGttTTGGtTtAAAtTGtAAtCGttTGttTtATGtAAtCGAAGtttATGAGGttTAGCGTtT 320
241 TACGACGTTGA                                                             TAGTGTTT 320
241 TATGATGTTGAATT                                                           AGCGTTT 320
```

```
321 TCCACGAATGAGGGCCACTCCCATGGCCACCTCGAGAATCTGCTCCACGCTCTGAACCCGCGCGCCTTCCCACAGTGTCT 400
321 TttACGAATGAGGGttAtTtttATGGttAttTCGAGAATtTGtTttACGtTtTGAAttCGCGCGttTTtttAtAGTGTtT 400
321 TTTATGAATGA                                                                      400
321 TTTACGAAT                                                                        400

401 GGTACAGCCACATATTCCTCCGGCAGAGATTGAGGATGTGGCAGATAGTGGAGTTGG                        480
401                                            ATGTGGTAGATAGTGGAGTTGG                    480
401 GGTAtAGttAtATATTttTtCGGtAGAGATTGAGGATGTGGtAGATAGTGGAGTTGG                        480
401             TATTTTTTCGGTAGAGAT                                                   480
401               TTTTTTTGGTAGAGATTG
```

Figure 80: Amplificate of SEQ ID NO: 60

```
  1 GGGGTCTCAAGAGCTGGCTCCTACACATAAAGGGCTTAGAACAGTGGCTGGTACCTAGGCCACCAATAGTCTTTTGCCCC  80
  1 GGGGTTTTAAGAGTTGGTTTT                                                              80
  1 GGGGTtTtAAGAGtTGGtTttTAtAtATAAAGGGtTTAGAAtAGTGGtTGGTAttTAGGttAttAATAGTtTTTTGtttt  80

 81 ACCCAACCGGTGACACAGCCCGGGGCCCCGCCGCCCCCTGGCGGCCATTACGGATTTCCGCCTCCCTCACAGAAGGCAGT 160
 81 AtttAAtCGGTGAtAtAGttCGGGGtttCGtCGtttttTGGCGGttATTACGGATTTtCGttTtttTtAtAGAAGGtAGT 160
 81                                                GTTATTATGGATTTTTGTT               160
 81                                                  TATTACGGATTTTCGTT               160

161 CACTGCAACGTGCGTGGCCTCAGTTGCGTCATATCCGGCCCTTGCGATCAGGGCTTGAGGAACCCGCGCCATGAAGTGCG 240
161 tAtTGtAACGTGCGTGGttTtAGTTGCGTtATATtCGGtttTTGCGATtAGGGtTTGAGGAAttCGCGttATGAAGTGCG 240
161                     AGTTGTGTTATATTTGGT           TTGAGGAATTTGTGTTAT               240
161                        TGCGTTATATTCGGTT               AATTCGCGTTATGAAG           240

241 TGTTTGTTACCGTAGGGACCACCAGCTTTGACGACCTCATTGCGTGTGTGTCGGCGCCCGACAGTCTGCAAGTGAGTGAG 320
241                                                                  TTGTAAGTGAGTGAG 320
241 TGTTTGTTAtCGTAGGGAttAttAGtTTTGACGAttTtATTGCGTGTGTGTCGGCGttCGAtAGTtTGtAAGTGAGTGAG 320
241                                                    TGTTGGTGTTTGATAGT             320
241                                                     GTCGGCGTTCGATAGT
```

321 GGAGG 400
321 **GGAGG** 400
321 GGAGG 400

Figure 81: Amplificate of SEQ ID NO: 61

```
1 TGGGGACACTGGATGTTTTTCCTCTGAGGCCGTGGAGGTTCACATCCCTGCCGAGGTGTGGGTGGCCCCTTTTCCGCTGG 80
1 TGGGGATATTGGATGTTTTT                                                             80
1 TGGGGAtAtTGGATGTTTTTttTtTGAGGtCGTGGAGGTTtAtATttt TGtCGAGGTGTGGGTGGttttTTTTtCGtTGG 80

81 TAAACAATCCCACACCTGGGGCTGTGCTTCTCCCCAGGGCGAGGCTACTGTGCCGTTTTCCTGGGCTGCTCAAAAGCTGG 160
81 TAAAtAATtttAtAttTGGGGtTGTGtTTtTttttAGGGCGAGGtTAtTGTGtCGTTTTttTGGGtTGtTtAAAAGtTGG 160
81                                         TTTAGGGCGAGGTTAT                         160
81                                         TTTAGGGTGAGGTTATT                        160

161 GGGCACTCTTGTGGGGCCGTCTGTCTTGCTGCTGCCTCTCCGGGGCAAGCTGTTAGAGCAGCCCCACCCGCAAACCTGCA 240
161 GGGtAtTtTTGTGGGGtCGTtTGTtTTGtTGtTGttTtTtCGGGGtAAGtTGTTAGAGtAGttttAttCGtAAAttTGtA 240

241 AACCAGGCTGGCCCCACAGAACCTGAAAACCTGCAAACCGGGCTGGCCCCACAGAACCCACAAACCTGCAGACTGGGCTG 320
241 AAttAGGtTGGttttAtAGAAttTGAAAAttTGtAAAtCGGGtTGGttttAtAGAAtttAtAAAttTGtAGAtTGGGtTG 320
```

321 GCCCCACAGAACCCGCAAACGGGGGCTGGCCCCACAGAACCCGCCTTTGCAGCTCAGTGCTGAATGTTCTCGTCCCCGGT 400
321 GttttAtAGAAttCGtAAACGGGGGtTGGttttAtAGAAttCGttTTTGtAGtTtAGTGtTGAATGTTtTCGTtttCGGT 400
321 AGAATTCGTAAACGGG 400
321 ATTTGTAAATGGGGGT 400

401 GGTACCCTGGCCTGAGAGAGGAAGGGCTCTGGGAATTCGGTCACCGACGGCTGCACACCCCGTAGGGTAGGTGGGTTCTG 480
401 **G** 480
401 GGTAtttTGGttTGAGAGAGGAAGGGtTtTGGGAATTCGGTtAtCGACGGtTGtAtAtttCGTAGGGTAGGTGGGTTtTG 480
401 TTCGGTTATCGACGGT 480
401 TTTGGTTATTGATGGTT 480
401 TTGTATATTTTGTAGGGT 480
401 TGTATATTTCGTAGGGT

481 GAGGCCAGGCTGGAAGG 560
481 **GAGGTTAGGTTGGAAGG** 560
481 GAGGttAGGtTGGAAGG 560

Figure 82: Amplificate of SEQ ID NO: 62

```
342 GGAGCTTCAATTCCTGGGACCCCTCCTACTGCGGGGAGAGTGGTTTCCCTGTCCCCAGAGGATGCTCCAGGCCCGAGTCT 263
342 GGAGTTTTAATTTTTGGGATTT                                                            263
342 GGAGtTTtAATTttTGGGAttttTttTAtTGCGGGGAGAGTGGTTTttttTGTttttAGAGGATGtTttAGGttCGAGTtT 263
342                                                                        AGGTTCGAGTTT 263
342                                                                        TAGGTTTGAGTTT 263

262 GCGGCGCTCTGGGGGATGCTCTCCGAGCTCCGACACCGTGTTCGGACCGGGTGCGCCCGCTGCCGCTGGGGCTGAAGCCT 183
262 GCGGCGtTtTGGGGGATGtTtTtCGAGtTtCGAtAtCGTGTTCGGAtCGGGTGCGttCGtTGtCGtTGGGGtTGAAGttT 183
262 GCGG                                      ATCGTGTTCGGATCGG                          183
262 GTGG                                     TATTGTGTTTGGATTGG                          183

182 GCAGGCGTGAGAACCGGGGGACTCTCTATGGCACCAGGAGCTTCACCGTGAGCGTAGCGCAGAAGCGCTTCGCTTTGATC 103
182 GtAGGCGTGAGAAtCGGGGGAtTtTtTATGGtAttAGGAGtTTtAtCGTGAGCGTAGCGtAGAAGCGtTTCGtTTTGATt 103
182  TAGGCGTGAGAATCGG                                 ATTGTGAGTGTAGTGTA                 103
182  TAGGTGTGAGAATTGG                                 ATCGTGAGCGTAGCGT                  103
```

| | | |
|---|---|---|
| 102 | CTAGCGCTTACAAAAGTCGTCCTTTGGCTGCCCATGATGGTAAAAGTGCAGTTGCTCACAAAGCGCGAGTGTGTGTGCCA | 23 |
| 102 | **A** | 23 |
| 102 | tTAGCGtTTAtAAAAGTCGTttTTTGGtTGtttATGATGGTAAAAGTGtAGTTGtTtAtAAAGCGCGAGTGTGTGTGttA | 23 |
| 102 | TATAAAGTGTGAGTGTG | 23 |
| 102 | TATAAAGCGCGAGTGT | |
| 22 | GACAGTGTAAATGAGTGTTGGG | -57 |
| 22 | **GATAGTGTAAATGAGTGTTGGG** | -57 |
| 22 | GAtAGTGTAAATGAGTGTTGGG | -57 |

Figure 83: Amplificate of SEQ ID NO: 8

```
  1 GGGAGTTTTTAAGCTCTGTGAGAATCCTGGGAGTTGGTGATGTCAGACTAGTTGGGTCATTTGAAGGTTAGCAGCCCGGG  80
  1 GGGAGTTTTTAAGTTTTGTGAG                                                            80
  1 GGGAGTTTTTAAGtTtTGTGAGAATttTGGGAGTTGGTGATGTtAGAtTAGTTGGGTtATTTGAAGGTTAGtAGttCGGG  80

 81 TAGGGTTCACCGAAAGTTCACTCGCATATATTAGGCAATTCAATCTTTCATTCTGTGTGACAGAAGTAGTAGGAAGTGAG 160
 81 TAGGGTTtAtCGAAAGTTtAtTCGtATATATTAGGtAATTtAATtTTTtATTtTGTGTGAtAGAAGTAGTAGGAAGTGAG 160

161 CTGTTCAGAGGCAGGAGGGTCTATTCTTTGCCAAAGGGGGGACCAGAATTCCCCCATGCGAGCTGTTTGAGGACTGGGAT 240
161 tTGTTtAGAGGtAGGAGGGTtTATTtTTTGttAAAGGGGGGAttAGAATTtttttATGCGAGtTGTTTGAGGAtTGGGAT 240
161                                                                             GGAT 240
161                                                                               AT 240

241 GCCGAGAACGCGAGCGATCCGAGCAGGGTTTGTCTGGGCACCGTCGGGGTAGGATCCGGAACGCATTCGGAAGGCTTTTT 320
241 GtCGAGAACGCGAGCGATtCGAGtAGGGTTTGTtTGGGtAtCGTCGGGGTAGGATtCGGAACGtATTCGGAAGGtTTTTT 320
241 GTTGAGAATGTGA                                TATCGTCGGGGTAGGA   GAATGTATTTGGAAGGT    320
241 GTCGAGAACGCGAG                               TATTGTTGGGGTAGGA    AACGTATTCGGAAGGT    320
241          AGCGATTCGAGTAGGG                                                            320
241          AGTGATTTGAGTAGGG
```

```
321 GCAAGCATTTACTTGGAAGGAGAACTTGGGATCTTTCTGGGAACCCCCCGCCCCGGCTGGATTGGCCGAGCAAGCCTGGA 400
321 GtAAGtATTTAtTTGGAAGGAGAAtTTGGGATtTTTtTGGGAAtttttCGtttCGGtTGGATTGGtCGAGtAAGttTGGA 400

401 AAATGGTAAATGATCATTTGGATCAATTACAGGCTTTTAGCTGGCTTGTCTGTCATAATTCATGATTCGGGGCTGGGAAA 480
401                                                                      GGGTTGGGAAA 480
401 AAATGGTAAATGATtATTTGGATtAATTAtAGGtTTTTAGtTGGtTTGTtTGTtATAATTtATGATTCGGGGtTGGGAAA 480

481 AAGACCAA                                                                         560
481 AAGATTAA                                                                         560
481 AAGAttAA                                                                         560
```

Figure 84: Amplificate of SEQ ID NO: 9

```
  1 AGAGGGGGTAGTCCCCACTCTCCTGTCTGATCCCTCCCTCTCCTCCCCGAGTTCCACCGCCCCAGGCGCACAGGTTTCCG  80
  1 AGAGGGGGTAGTTTTTATTTTT                                                            80
  1 AGAGGGGGTAGTttttAtTtTttTGTtTGATtttTtttTtTttTtttCGAGTTttAtCGttttAGGCGtAtAGGTTTtCG  80
  1                                                     TTTATTGTTTTAGGTGTAT           80
  1                                                       TATCGTTTTAGGCGTA            80

 81 CCAGATGTCTTTTCTTCTTCGCAGTCTTTGCCCGAGCGCTTCCGAGAGCCAGTTCTGGACTGATCGCCTTGGATGGGATA 160
 81 ttAGATGTtTTTTtTTtTTCGtAGTtTTTGttCGAGCGtTTtCGAGAGttAGTTtTGGAtTGATCGttTTGGATGGGATA 160
 81                                     GAGTGTTTTTGAGAGTT                            160
 81                                      AGCGTTTTCGAGAGTT                            160

161 CCGGGGGAGGGCAGAAGGACACTTGGCTTCCTCTCCAGGAATCTGAGCGGCCCTGAGGTCCGGGGGCGCAGGGAATCCCC 240
161 tCGGGGGAGGGtAGAAGGAtAtTTGGtTTttTtTttAGGAATtTGAGCGGtttTGAGGTtCGGGGGCGtAGGGAATtttt 240
161                                           AATTTGAGTGGTTTTGA                      240
161                                            ATTTGAGCGGTTTTGA                      240

241 TCTCCCGCCGCCGCCGCCGTGTCTGGTCTGTACGTCTTTAGAGGGTCGAGGAAGTCACGTCGGGACAGACTGGGGCGAGT 320
241 TtTttCGtCGtCGtCGtCGTGTtTGGTtTGTACGTtTTTAGAGGGTCGAGGAAGTtACGTCGGGAtAGAtTGGGGCGAGT 320
241                                                   GGAAGTTATGTTGGGA               320
241                                                       TTACGTCGGGATAGAT
```

| | | |
|---|---|---|
| 321 | AAGGTTAAGAAAGGCTGACATGTT | 400 |
| 321 | **AAGGTTAAGAAAGGTTGATATGTT** | 400 |
| 321 | AAGGTTAAGAAAGGtTGAtATGTT | 400 |

Figure 85: Amplificate of SEQ ID NO: 10

```
  1 AGTGGGTAGGCCAAGTGTGTTGCTTCAGCAAACCGGACCAGGAGGGCCAGGGCCGGATGTGGGGACCCTCTTCCTCTAGC  80
  1 AGTGGGTAGGTTAAGTGTGTTG                                                            80
  1 AGTGGGTAGGttAAGTGTGTTGtTTtAGtAAAtCGGAttAGGAGGGttAGGGtCGGATGTGGGGAtttTtTTttTtTAGt  80
  1                                       AGTAAATCGGATTAGGA                           80
  1                                       AGTAAATTGGATTAGGAG                          80

 81 ACAGTAAAGCTGGCCTCCAGAAACACGGGTATCTCCGCGTGGTGCTTTGCGGTCGCCGTCGTTGTGGCCGTCCGGGGTGG 160
 81 AtAGTAAAGtTGGttTttAGAAAtACGGGTATtTtCGCGTGGTGtTTTGCGGTCGtCGTCGTTGTGGtCGTtCGGGGTGG 160
 81                                 ATATGGGTATTTTTGTGT                                160
 81                                  TACGGGTATTTTCGCGT                                160

161 GGTGTGAGGAGGGGACGAAGGAGGGAAGGAAGGGCAAGGCGGGGGGGGCTCTGCGAGAGCGCGCCCAGCCCCGCCTTCGG 240
161 GGTGTGAGGAGGGGACGAAGGAGGGAAGGAAGGGtAAGGCGGGGGGGGtTtTGCGAGAGCGCGtttAGtttCGttTTCGG 240
161                                                               GAGAGTGTGTTTAGTTT   240
161                                                                AGAGCGCGTTTAGTTT

241 GCCCCACAGTCCCTGCACCCAGGTTTCCATTGCGCGGCTCTCCTCAGCTCCTTCCCGCCGCCCAGTCTGGATCCTGGGG 320
241                                                              TTTAGTTTGGATTTTGGGG 320
241 GttttAtAGTttttTGtAtttAGGTTTttATTGCGCGGtTtTttTtAGtTttTTttCGtCGtttAGTtTGGATttTGGGG 320
```

Figure 86: Amplificate of SEQ ID NO: 11

1 AGCTGGAGAAACTGAAAAGATCACAAGCGACTTAACGATAAGCCCCTTCTTCCTTTTAAAGACCGAGAGGAGGGTAGAGG 80

1 **AGTTGGAGAAATTGAAAAGATTA** 80

1 AGtTGGAGAAAtTGAAAAGATtAtAAGCGAtTTAACGATAAGttttTTtTTttTTTTAAAGAtCGAGAGGAGGGTAGAGG 80

1 ATAAGCGATTTAACGAT 80

1 AGTGATTTAATGATAAGTT 80

81 GGAGTAGTGCCTGAGCCCACGTGACCGAGCCAGGGAGCCCGACGGTCTCAGGAACGCCCGACGCCGCGCGTGACCTCTAA 160

81 GGAGTAGTGttTGAGtttACGTGAtCGAGttAGGGAGttCGACGGTtTtAGGAACGttCGACGtCGCGCGTGAttTtTAA 160

81 TTTAGGAATGTTTGATGT 160

81 TAGGAACGTTCGACGT 160

161 GTGGGAGCACCCTCGAACCGACTCCTGGTCCACCCACAAGGATAGTGGCGCACAGATGGCGCTCCCCGCAGCCCCAGTCT 240

161 GTGGGAGtAtttTCGAAtCGAtTttTGGTttAtttAtAAGGATAGTGGCGtAtAGATGGCGtTttTCGtAGttttAGTtT 240

161 TAGATGGTGTTTTTTGT 240

161 ATGGCGTTTTTCGTAG 240

241 CAGATTTAAGAGGTCTGGAGTAGGGCCTGAGAATATGCATTTCCAACCAGGTCCTGGGGGATGCCGACACTGATACAGCC 320

241 tAGATTTAAGAGGTtTGGAGTAGGGttTGAGAATATGtATTTttAAttAGGTttTGGGGGATGtCGAtAtTGATAtAGtt 320

```
321 AGTCTGGGGACCACACTTCGAGGATCACGTCCTCAGCCCCTGACTCACATAAGTGTCATTCAGAACAGATGTCTGATTCT 400
321 AGTtTGGGGAttAtAtTTCGAGGATtACGTttTtAGttttTGAtTtAtATAAGTGTtATTtAGAAtAGATGTtTGATTtT 400
321                 ATATTTTGAGGATTATGTT                                              400
321                    ATTTCGAGGATTACGT

401 AAGGAGCCAGTGGTGAAACCAGAGAGGCTTTGGGTTTGTCA                                        480
401                              TAGAGAGGTTTTGGGTTTGTTA                              480
401 AAGGAGttAGTGGTGAAAttAGAGAGGtTTTGGGTTTGTtA                                        480
```

Figure 87: Amplificate of SEQ ID NO: 12a

```
  1 CCCAGCTGTGAAAAAGGGACCCAGGACCCGCAGATGCTGGCGAGGAACAGGGGTGCGGAGCATGAGTCGGCTTTGCACCA  80
  1 TTTAGTTGTGAAAAAGGGATTT                                                            80
  1 tttAGtTGTGAAAAAGGGAtttAGGAttCGtAGATGtTGGCGAGGAAtAGGGGTGCGGAGtATGAGTCGGtTTTGtAttA  80

 81 CGCCCTGGGGTCTCCAGCACTGAAGCGTTCAGTACCATGCAGAGCAGCTGGGAGCGGGTCCCGCTGGGCGGGGCCGAGCT 160
 81 CGtttTGGGGTtTttAGtAtTGAAGCGTTtAGTAttATGtAGAGtAGtTGGGAGCGGGTttCGtTGGGCGGGGtCGAGtT 160

161 GCGCGCGACCCTCGGCGCGCTCGGGGAGGCCCAGACAGGGGTGGCCTCTCTGGCCTCCGCTCCCGCGGGCTCTATGACAC 240
161 GCGCGCGAtttTCGGCGCGtTCGGGGAGGtttAGAtAGGGGTGGttTtTtTGGttTtCGtTttCGCGGGtTtTATGAtAt 240
161                                                                               AT 240
161                                                                               AT 240

241 CGCACTGGCTCGCGGGACGGGGCGGGGTCGGGTGAGGGGGAGGAGACAGCCCCACGCTTTGCGACTCGCGGTGACCCCTA 320
241 CGtAtTGGtTCGCGGGACGGGGCGGGGTCGGGTGAGGGGGAGGAGAtAGttttACGtTTTGCGAtTCGCGGTGAttttTA 320
241 CGTATTGGTTCGCGG                                  AGTTTTATGTTTTGTGAT     GTGATTTTTA 320
241 TGTATTGGTTTGTGG                                     TTTACGTTTTGCGATT       ATTTTTA 320
```

```
321 CGCGGAACTCTCTCGCGGTAATTCGAAGTACCGCGCCTGCGTGCTGCAGTAGCGCCTGGTGGCGGTGGCAGTTTGCCCGC 400
321 CGCGGAAtTtTtTCGCGGTAATTCGAAGTAtCGCGttTGCGTGtTGtAGTAGCGttTGGTGGCGGTGGtAGTTTGttCGC 400
321 TGTGGAAT                      ATTCGAAGTATCGCGT                                  400
321 CGCGGAATT                     ATTTGAAGTATTGTGTTT

401 GGGTGTGTGAAGGGAGACAGTGTGGAGGCCACAGGG                                            480
401           GAGATAGTGTGGAGGTTATAGGG                                               480
401 GGGTGTGTGAAGGGAGAtAGTGTGGAGGttAtAGGG                                            480
```

Figure 88: Amplificate of SEQ ID NO: 12b

497 GTCTTGGAGGGAGTAGAGACCCATCTTCAGCCTGCCTCACCCCCTTGGATAAAGAAAATGCCCCCTTTCTATCATTCAAA 418
497 **CCCTATAACCTCCACACTA** 418
497 GTtTTGGAGGGAGTAGAGAtttATtTTtAGttTGttTtAttttTTGGATAAAGAAAATGtttttTTTtTATtATTtAAA 418

417 ATTATTTTTAGGTCGGTTTTGTTTACTCCGAACCCCGAGTAGAGCACATGGGGAGCCCAAAATAAGTGCATACTTGAATG 338
417 **TCTC** 338
417 ATTATTTTTAGGTCGGTTTTGTTTAtTtCGAAtttCGAGTAGAGtAtATGGGGAGtttAAAATAAGTGtATAtTTGAATG 338
417 TTTGAATTTTGAGTAGAG 338
417 ATTTCGAATTTCGAGTA 338

337 GATAATTCCCAGAGAACTGCTGCTTGCAACATAGTTTGTAATACCAAAAAATTGAAATCTATATTAACACCCATCAGTAG 258
337 GATAATTtttAGAGAAtTGtTGtTTGtAAtATAGTTTGTAATAttAAAAAATTGAAATtTATATTAAtAtttATtAGTAG 258

257 GAAAATGGATAAATGAATAATGCCTCTATCCTGTTTTTGAAATATGATTACAGCCGTTAAAACGTAGATCTGTGGTCTGA 178
257 GAAAATGGATAAATGAATAATGttTtTATttTGTTTTTGAAATATGATTAtAGtCGTTAAAACGTAGATtTGTGGTtTGA 178
257 AGTTGTTAAAATGTAGATT 178
257 TAGTCGTTAAAACGTAG 178

177 CTGGCTACCCAGGACATGTTAAATGGGAAAACAACTAGCAGAAGGGCACCTACGTAGTGATACCATTTATGTTATTTTTA 98
177 tTGGtTAtttAGGAtATGTTAAATGGGAAAAtAAtTAGtAGAAGGGtAttTACGTAGTGATAttATTTATGTTATTTTTA 98

97 AAATTAAATGTACATTTATATAGGTGTACGTGTATGAACGTGCAGTTTAAAGGCCTAAACAGAAAAATACATCAGAAGCT 18
97 **AAATC** 18
97 AAATTAAATGTAtATTTATATAGGTGTACGTGTATGAACGTGtAGTTTAAAGGttTAAAtAGAAAAATAtATtAGAAGtT 18
97 TGTATGAACGTGTAGTT 18
97 GTGTATGAATGTGTAGT

17 CTTAACTGTGGGATGGG -62
17 **CCTTTTTCACAACTAAA** -62
17 tTTAAtTGTGGGATGGG -62

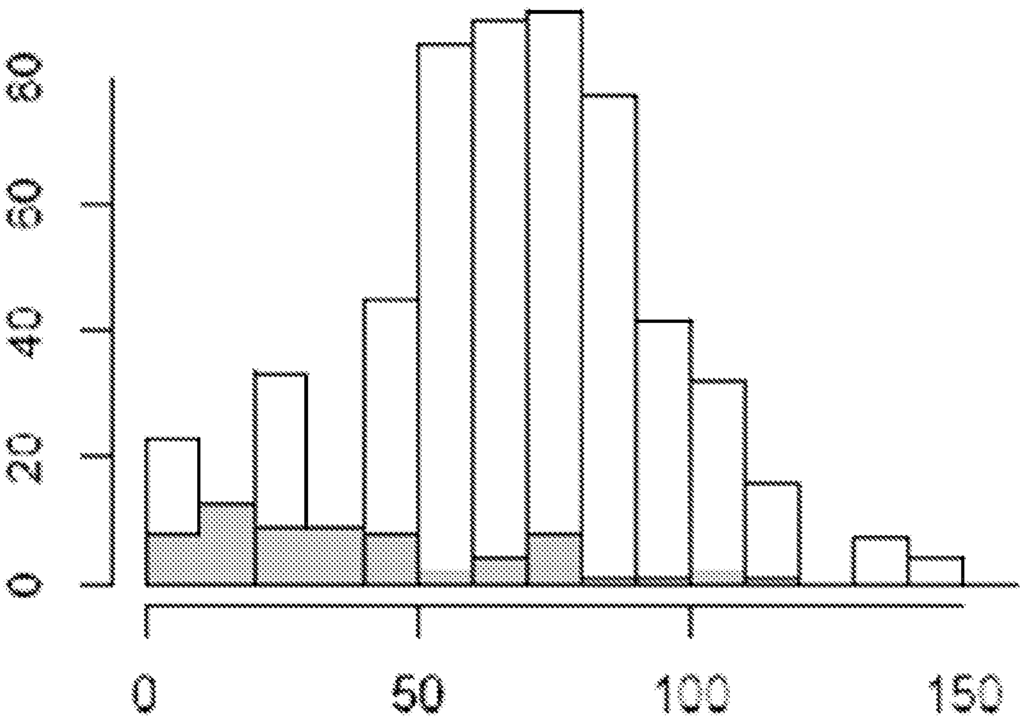


Figure 89

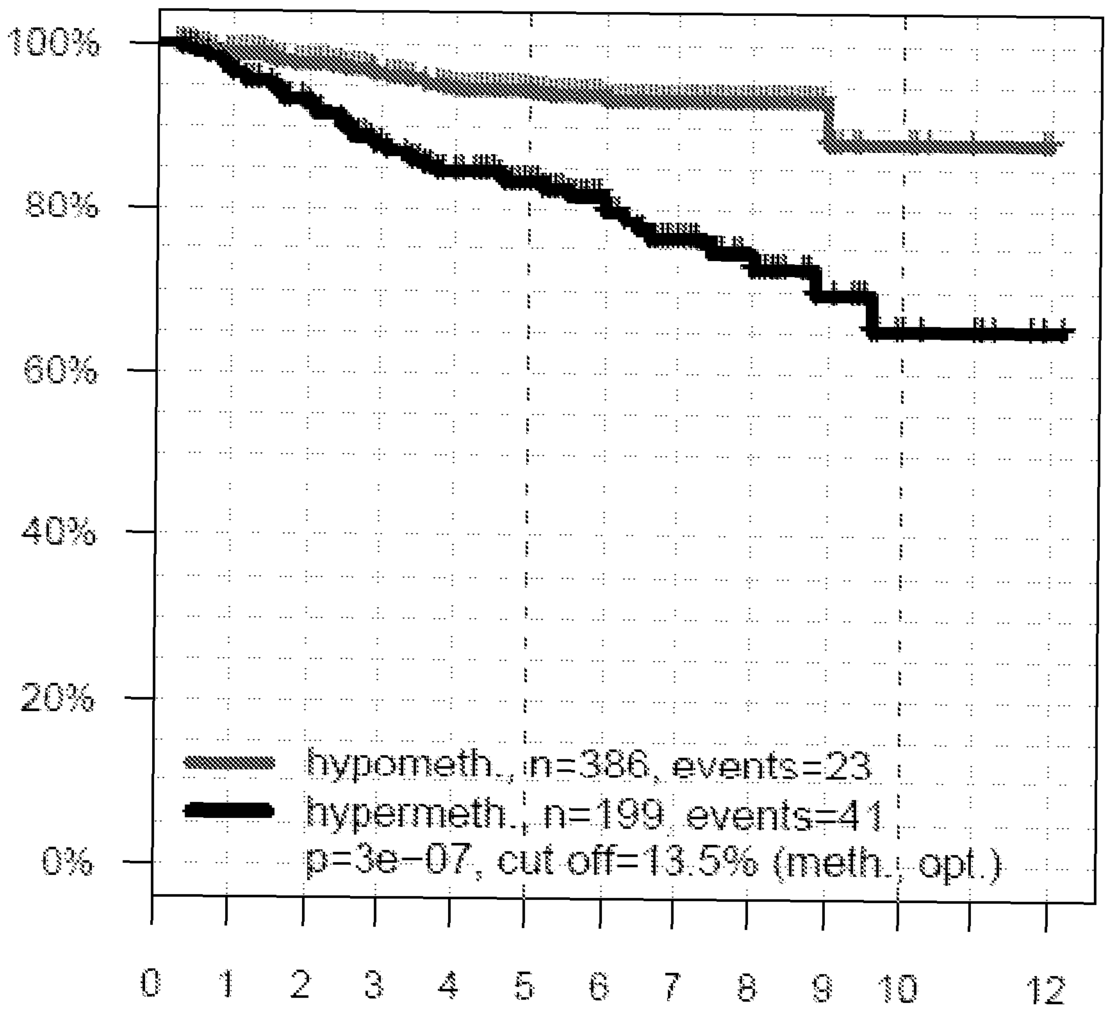
100%
80%
60%
40%
20%
0%
hypometh., n=386, events=23
hypermeth., n=199, events=41
p=3e−07, cut off=13.5% (meth., opt.)
0
1
2
3
4
5
6
7
8
9
10
12

Figure 90 A

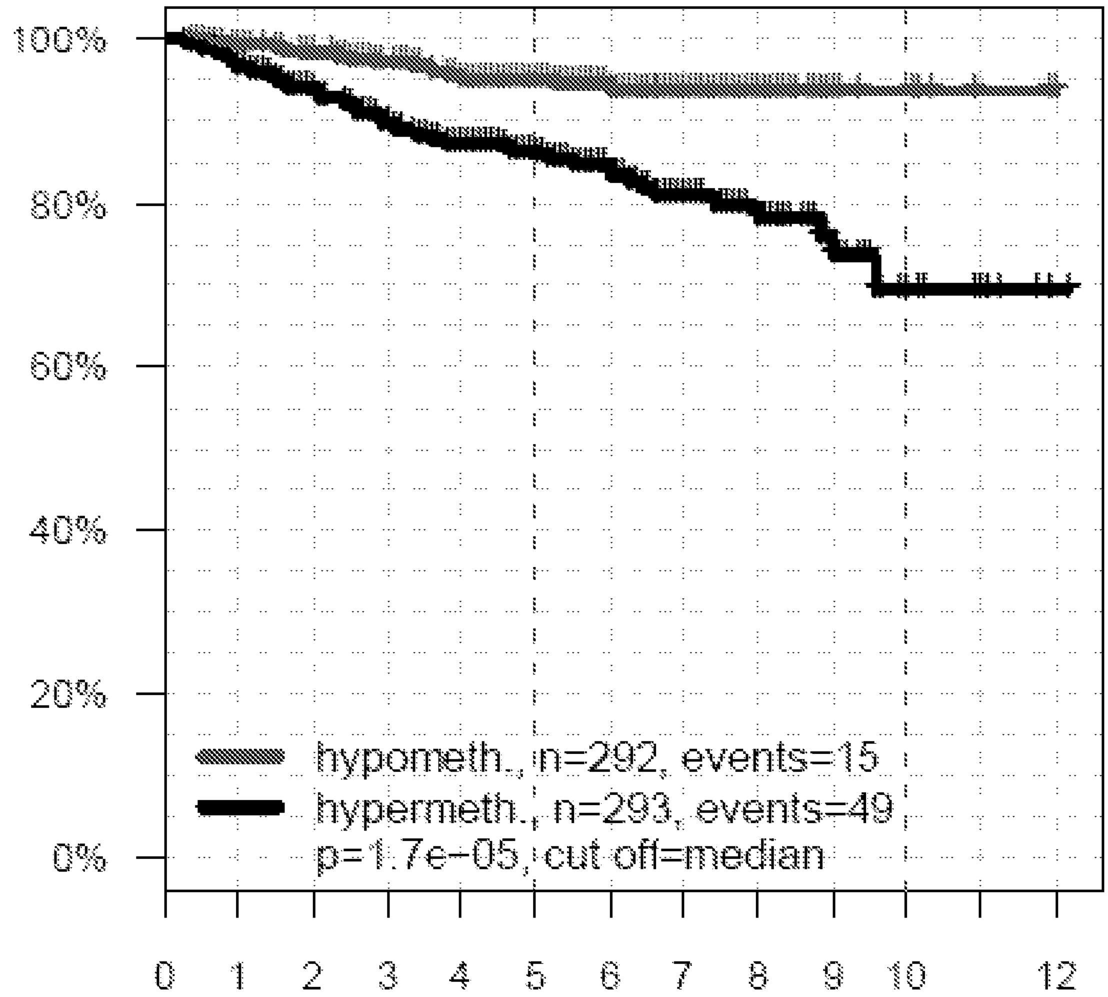
100%
80%
60%
40%
20%
0%
hypometh., n=292, events=15
hypermeth., n=293, events=49
p=1.7e−05, cut off=median
0
1
2
3
4
5
6
7
8
9
10
12

**Figure 90 B**

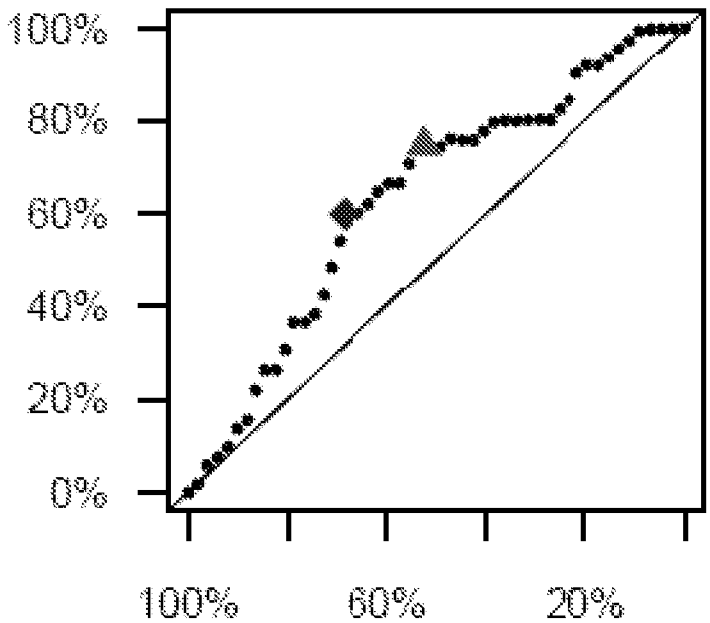


Figure 90 C

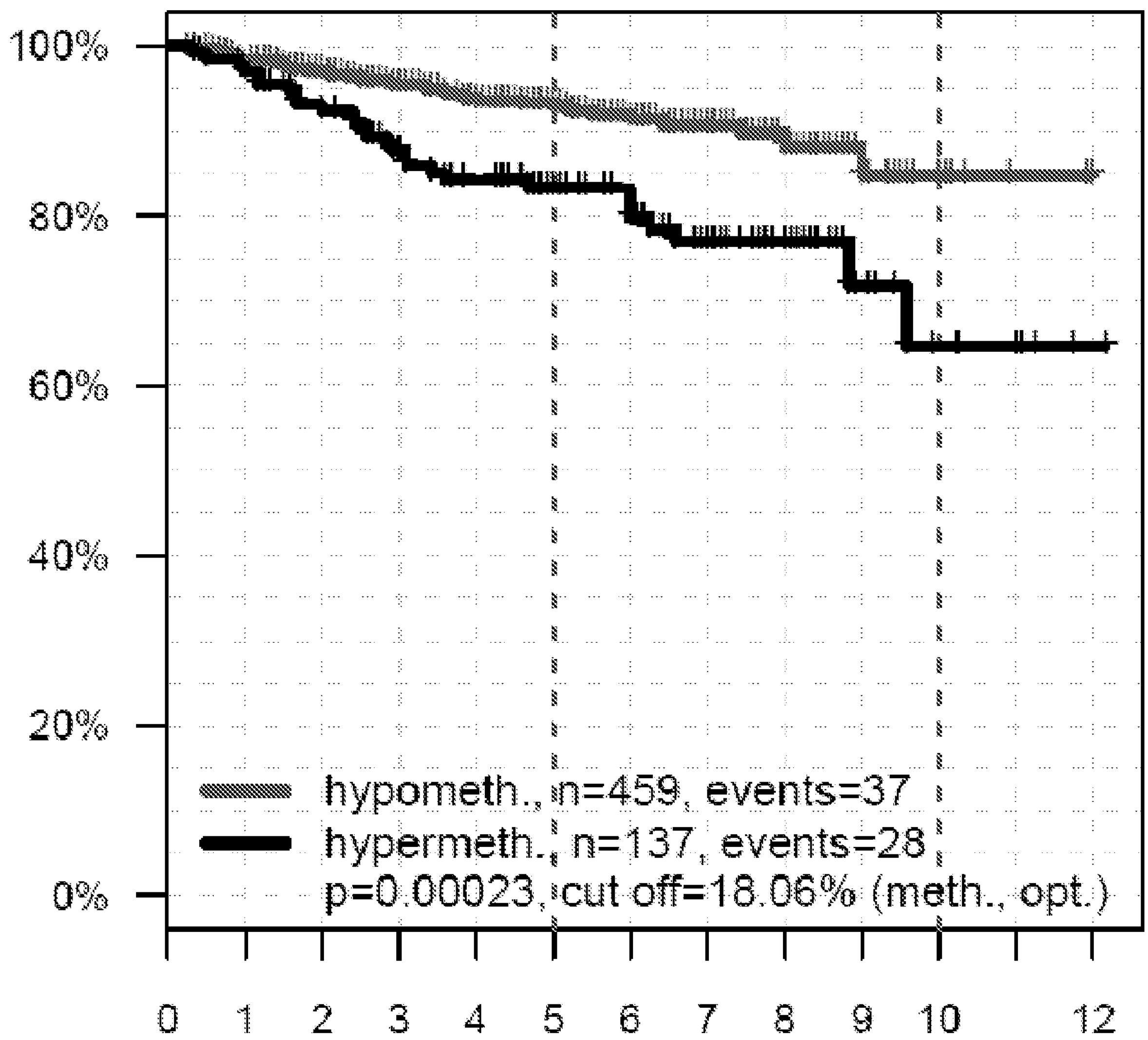
100%
80%
60%
40%
20%
0%
0
1
2
3
4
5
6
7
8
9
10
12
hypometh., n=459, events=37
hypermeth., n=137, events=28
p=0.00023, cut off=18.06% (meth., opt.)

Figure 91 A

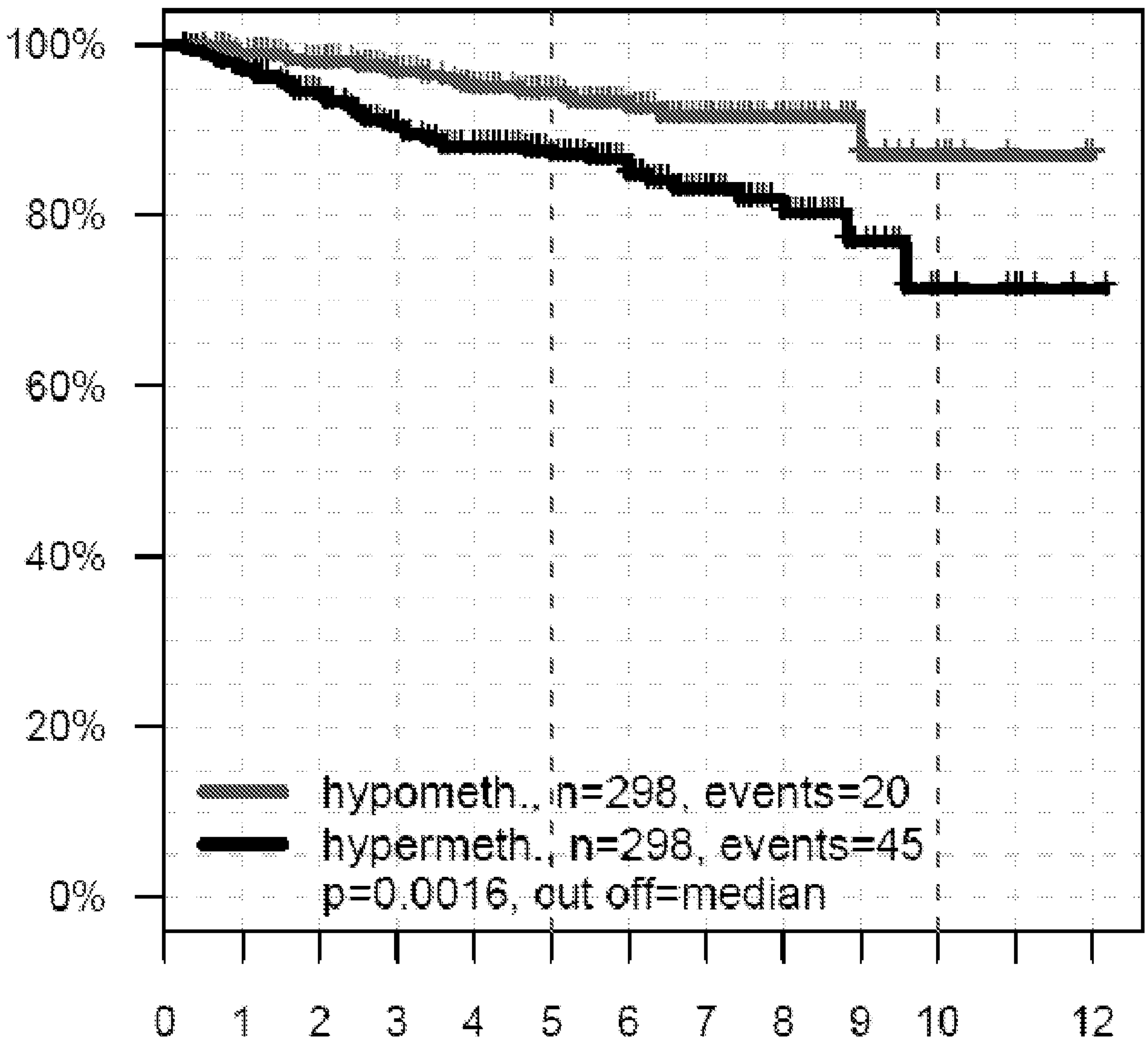
100%
80%
60%
40%
20%
0%
hypometh., n=298, events=20
hypermeth., n=298, events=45
p=0.0016, cut off=median
0 1 2 3 4 5 6 7 8 9 10 12

Figure 91 B

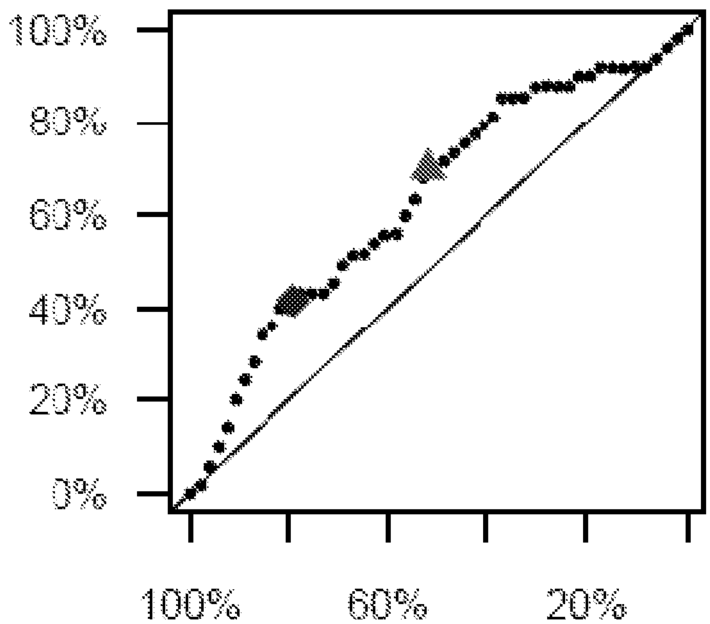


Figure 91 C

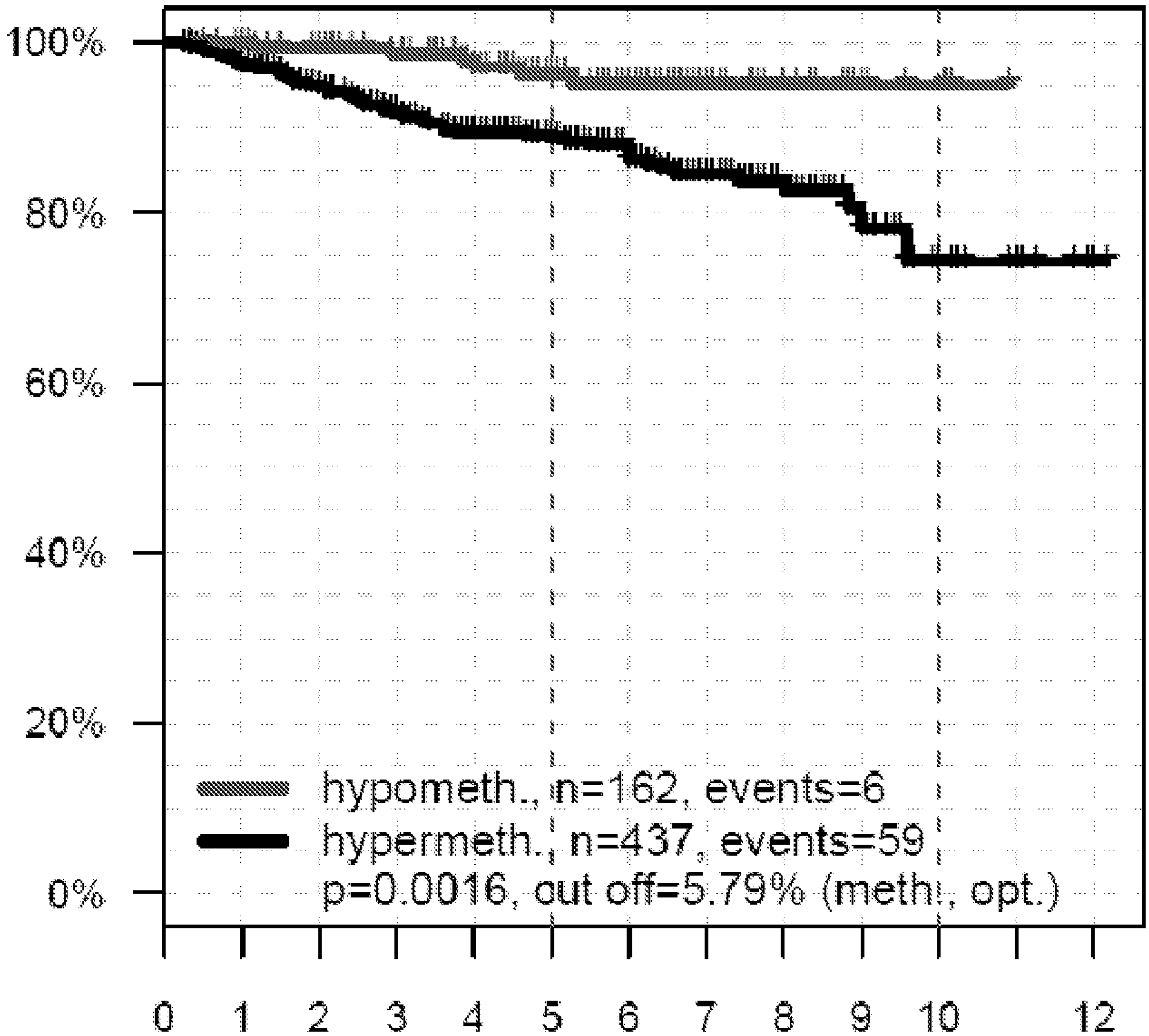
100%
80%
60%
40%
20%
0%
hypometh., n=162, events=6
hypermeth., n=437, events=59
p=0.0016, cut off=5.79% (meth., opt.)
0
1
2
3
4
5
6
7
8
9
10
12

Figure 92 A

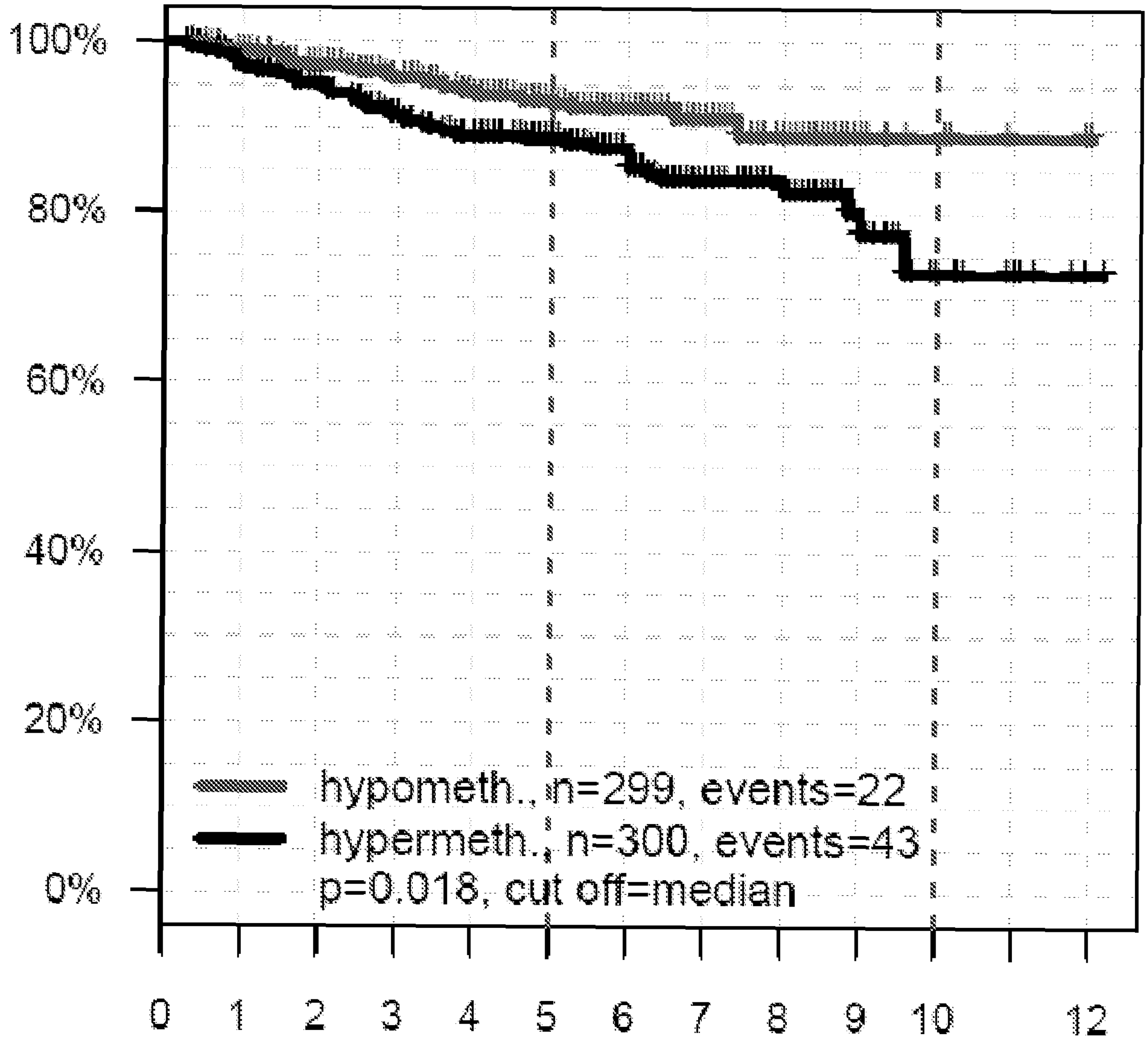
100%
80%
60%
40%
20%
0%
hypometh., n=299, events=22
hypermeth., n=300, events=43
p=0.018, cut off=median
0
1
2
3
4
5
6
7
8
9
10
12

Figure 92 B

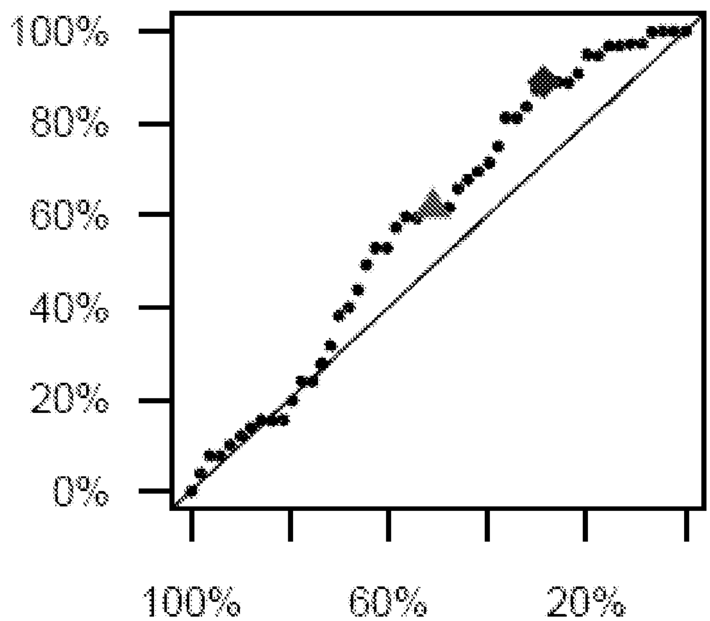


Figure 92 C

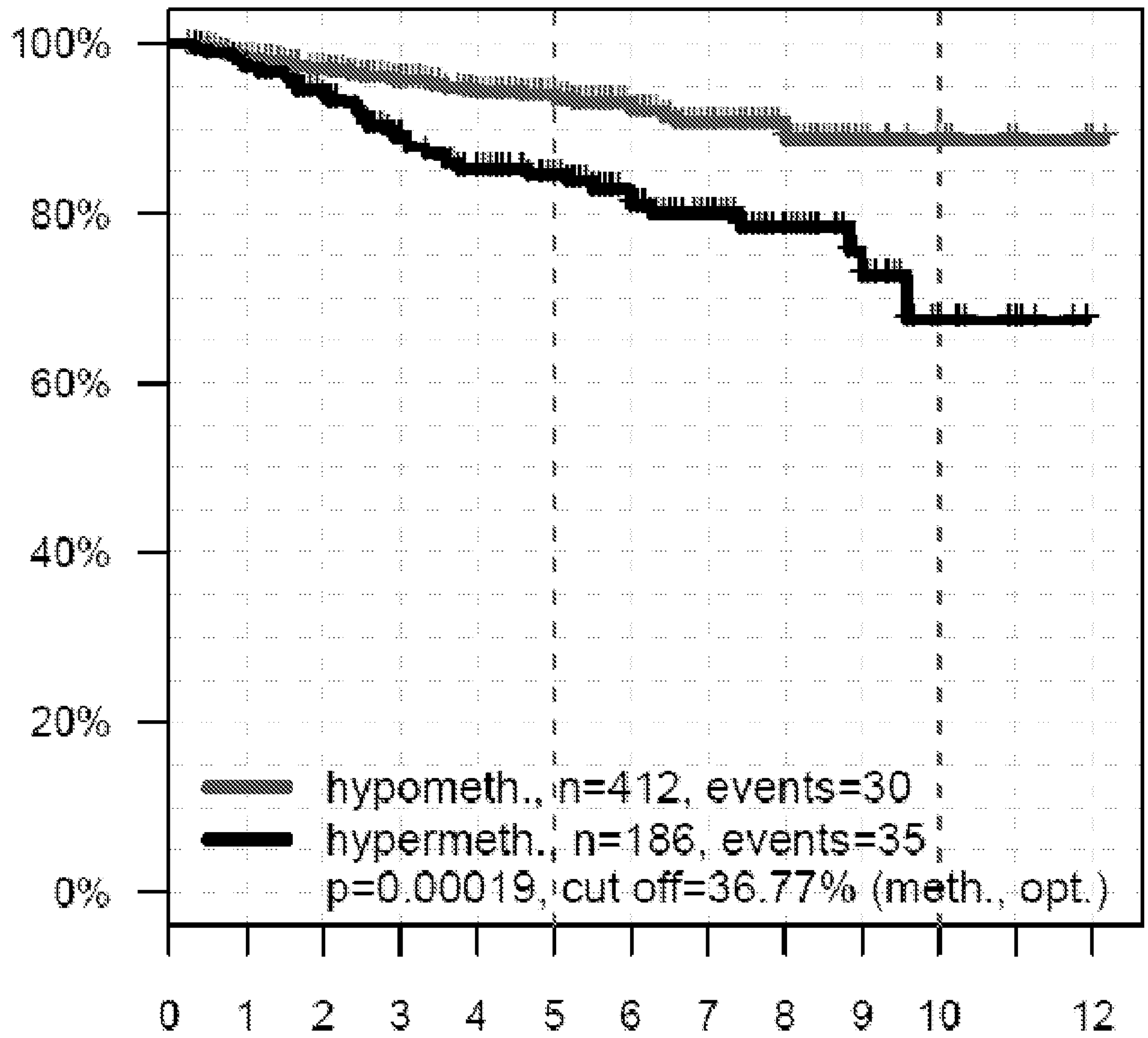
100%
80%
60%
40%
20%
0%
hypometh., n=412, events=30
hypermeth., n=186, events=35
p=0.00019, cut off=36.77% (meth., opt.)
0
1
2
3
4
5
6
7
8
9
10
12

Figure 93 A

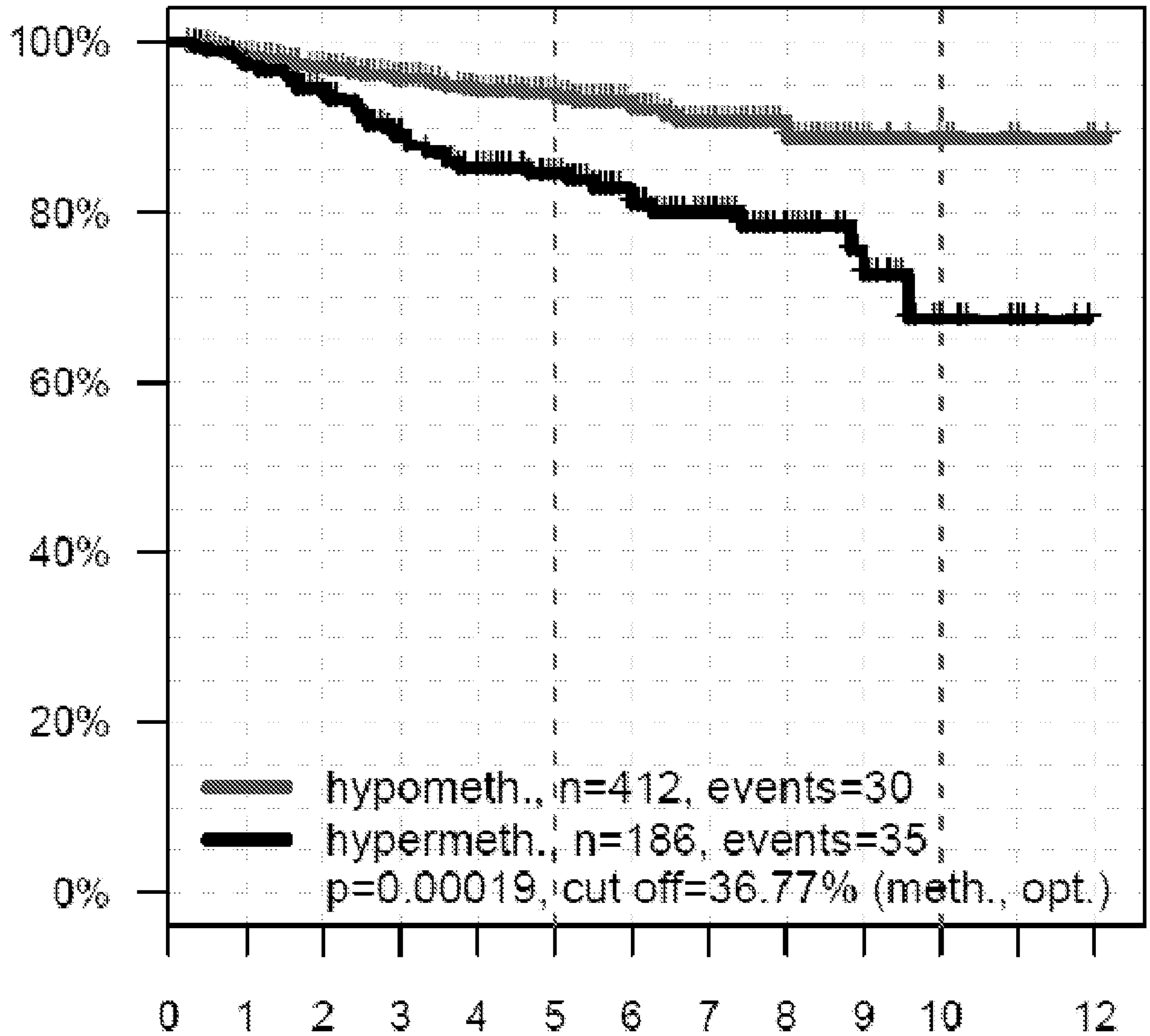
100%
80%
60%
40%
20%
0%
hypometh., n=412, events=30
hypermeth., n=186, events=35
p=0.00019, cut off=36.77% (meth., opt.)
0
1
2
3
4
5
6
7
8
9
10
12

Figure 93 B

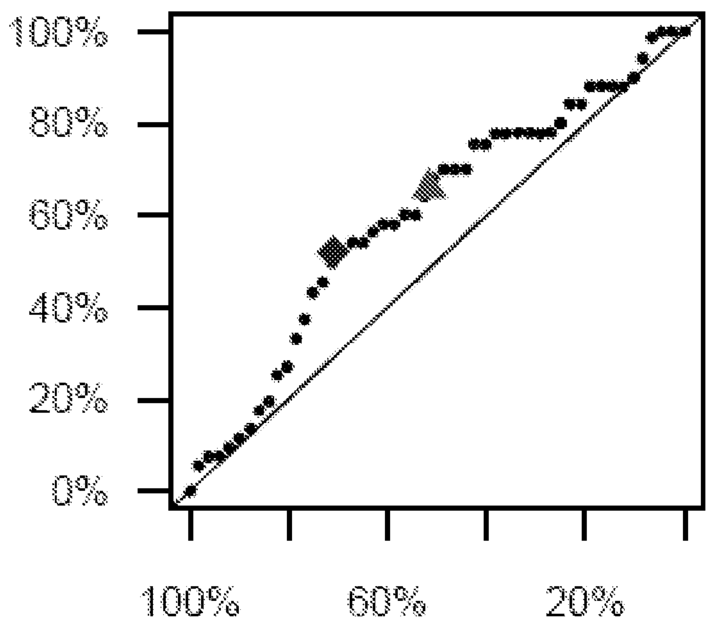


Figure 93 C

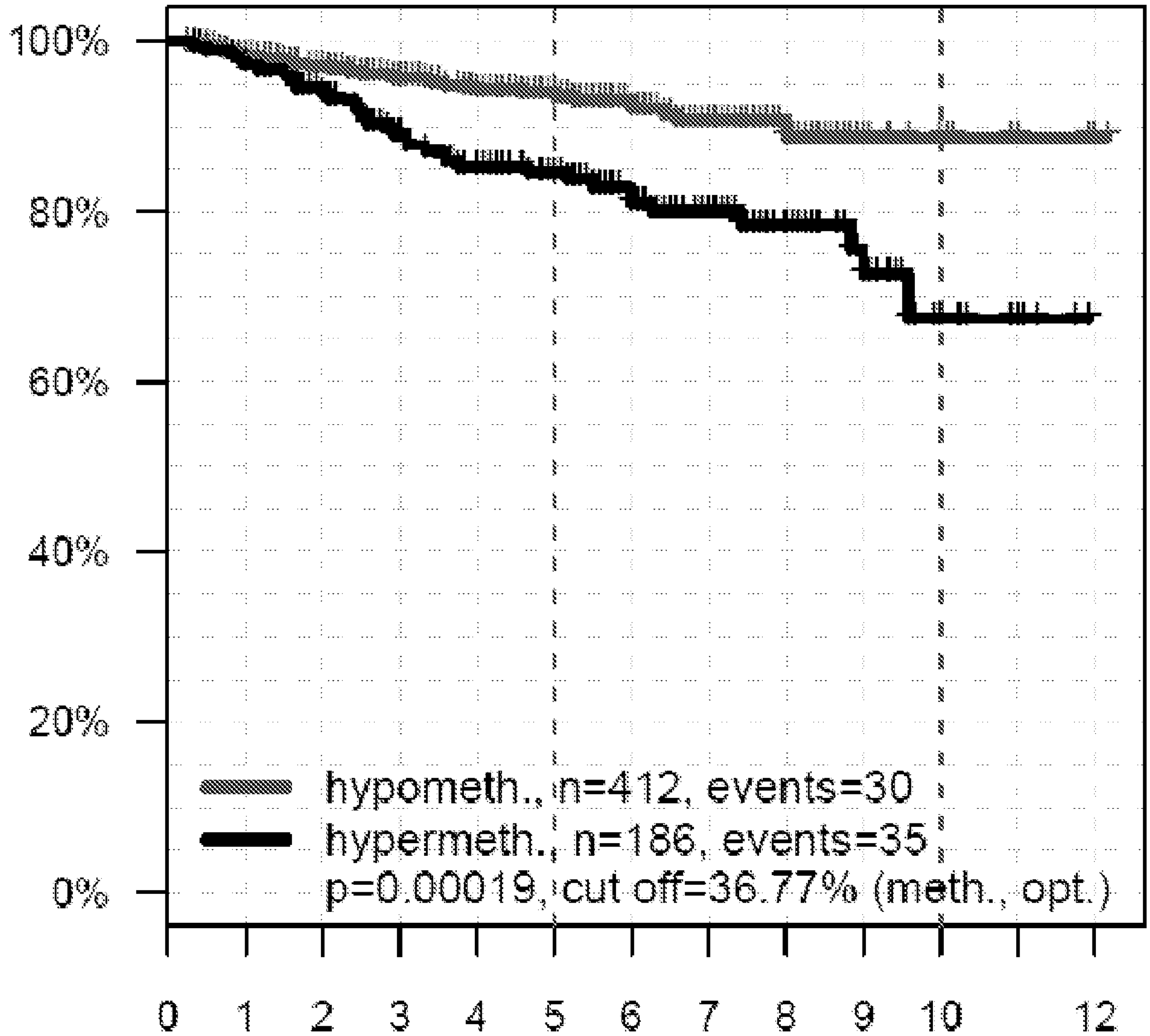
100%
80%
60%
40%
20%
0%
hypometh., n=412, events=30
hypermeth., n=186, events=35
p=0.00019, cut off=36.77% (meth., opt.)
0
1
2
3
4
5
6
7
8
9
10
12

Figure 94 A

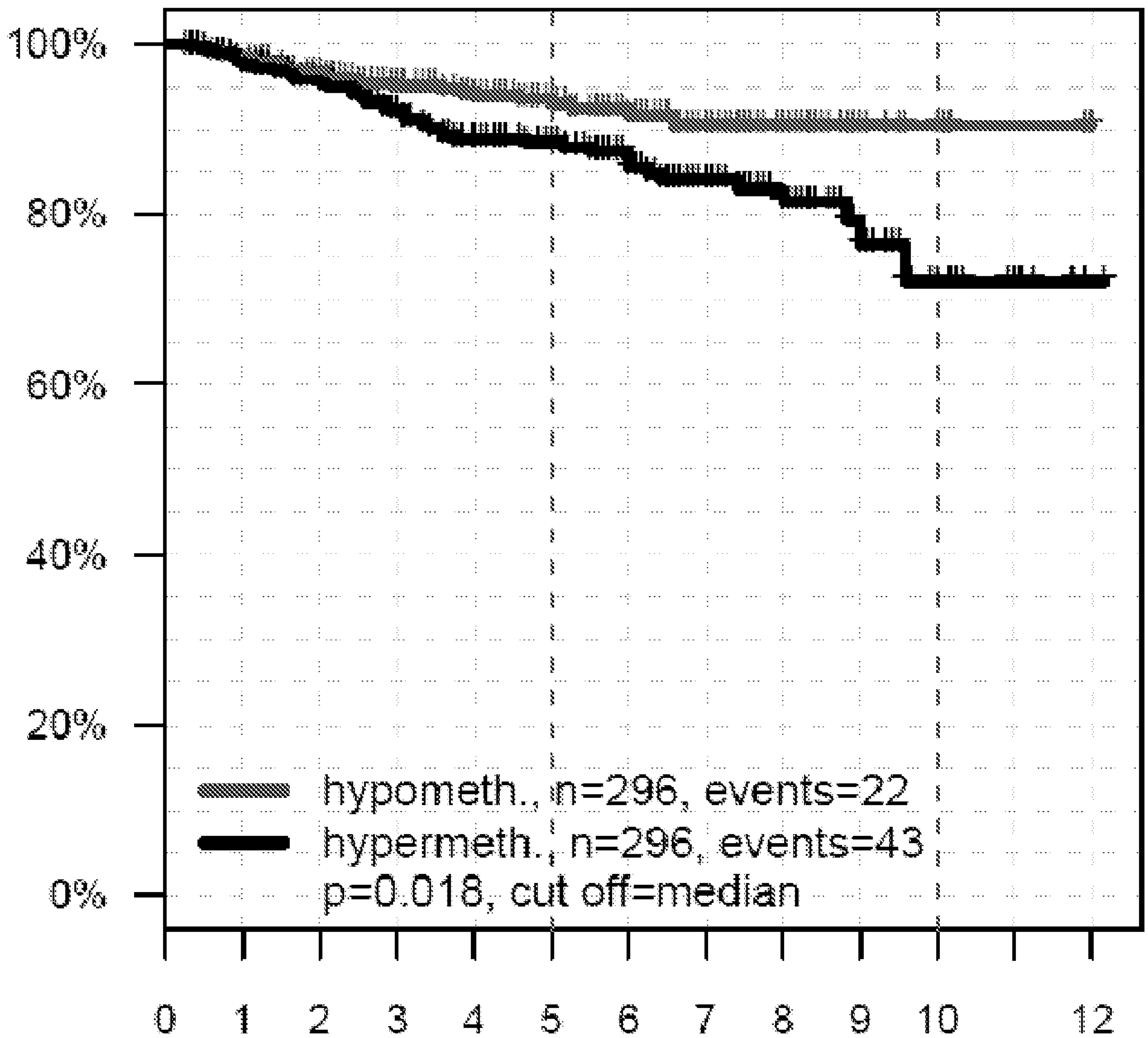
100%
80%
60%
40%
20%
0%
hypometh., n=296, events=22
hypermeth., n=296, events=43
p=0.018, cut off=median
0
1
2
3
4
5
6
7
8
9
10
12

Figure 94 B

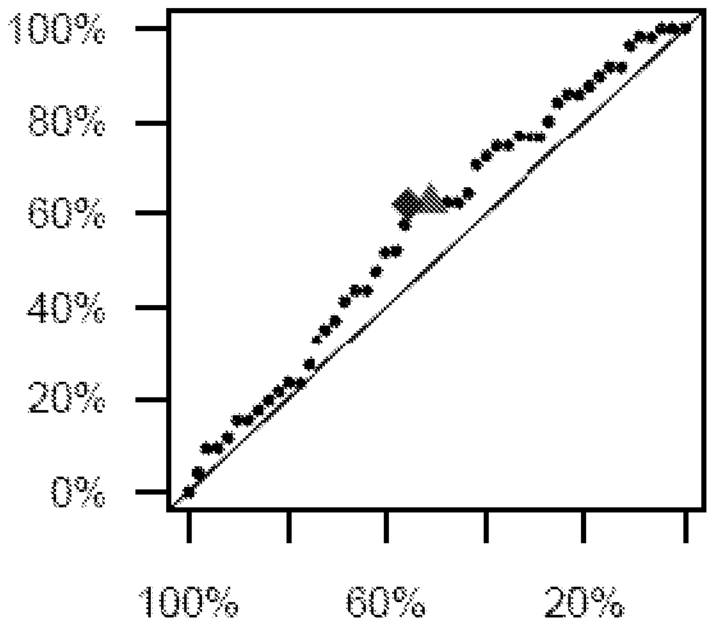


Figure 94 C

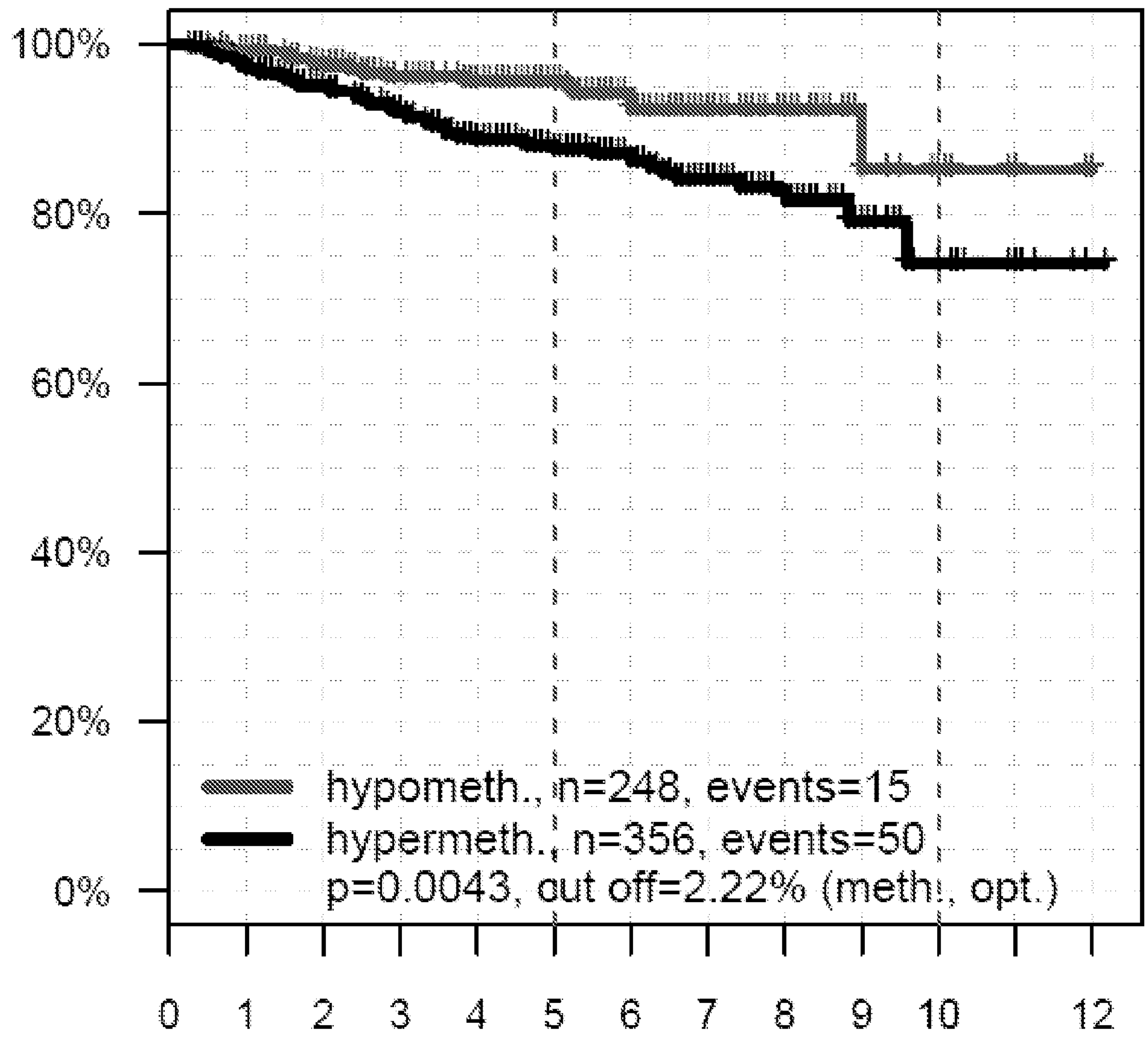
100%
80%
60%
40%
20%
0%
hypometh., n=248, events=15
hypermeth., n=356, events=50
p=0.0043, cut off=2.22% (meth., opt.)
0
1
2
3
4
5
6
7
8
9
10
12

**Figure 95 A**

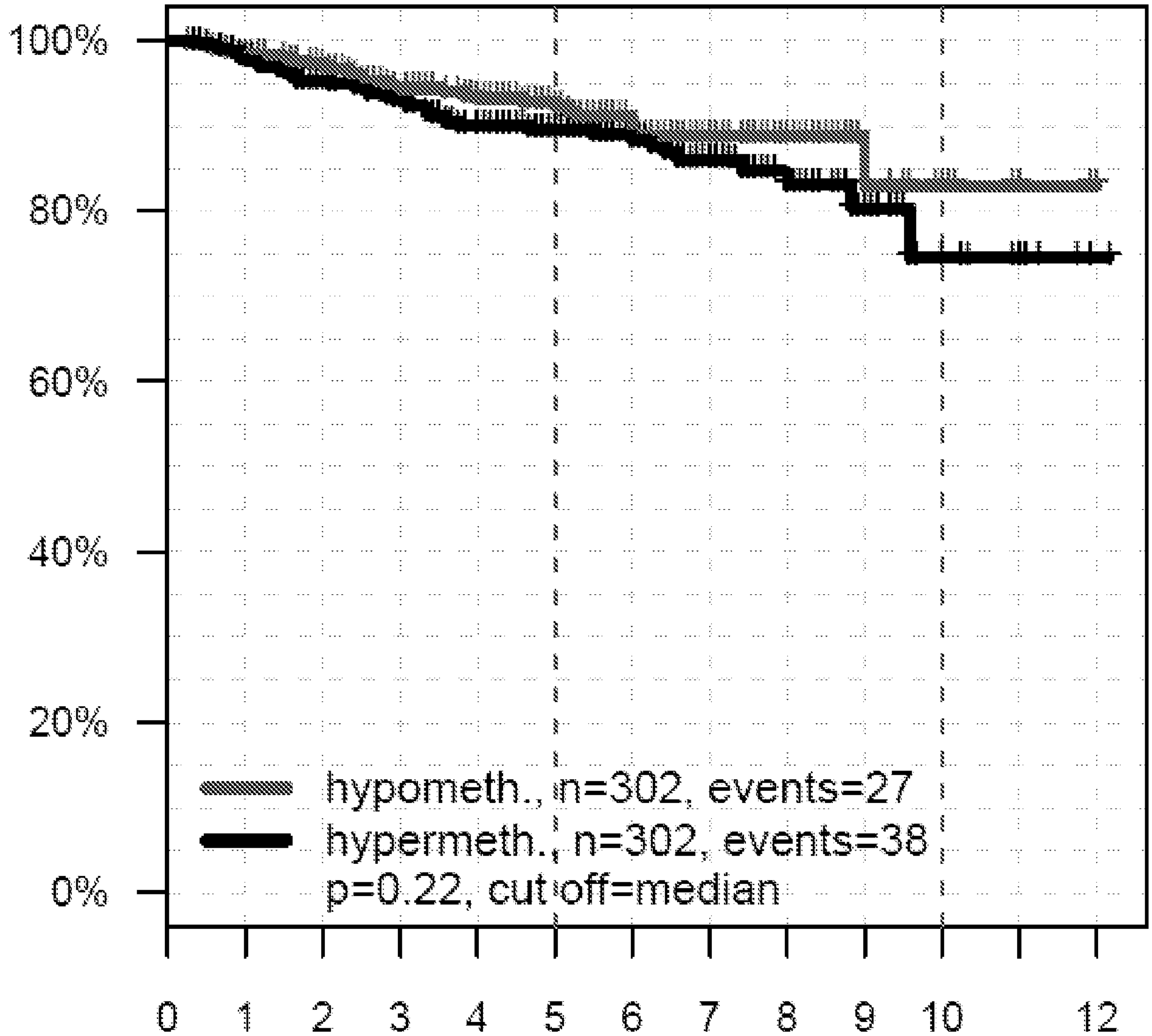
100%
80%
60%
40%
20%
0%
hypometh., n=302, events=27
hypermeth., n=302, events=38
p=0.22, cut off=median
0
1
2
3
4
5
6
7
8
9
10
12

Figure 95 B

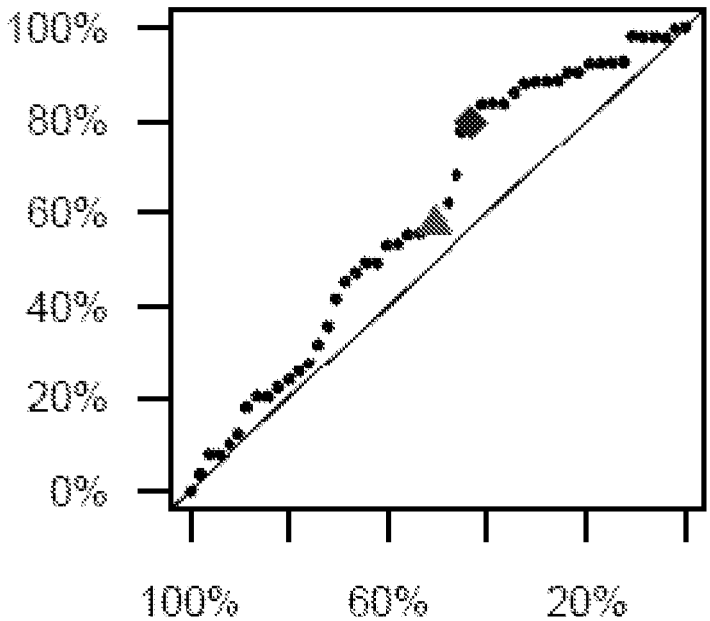


Figure 95 C

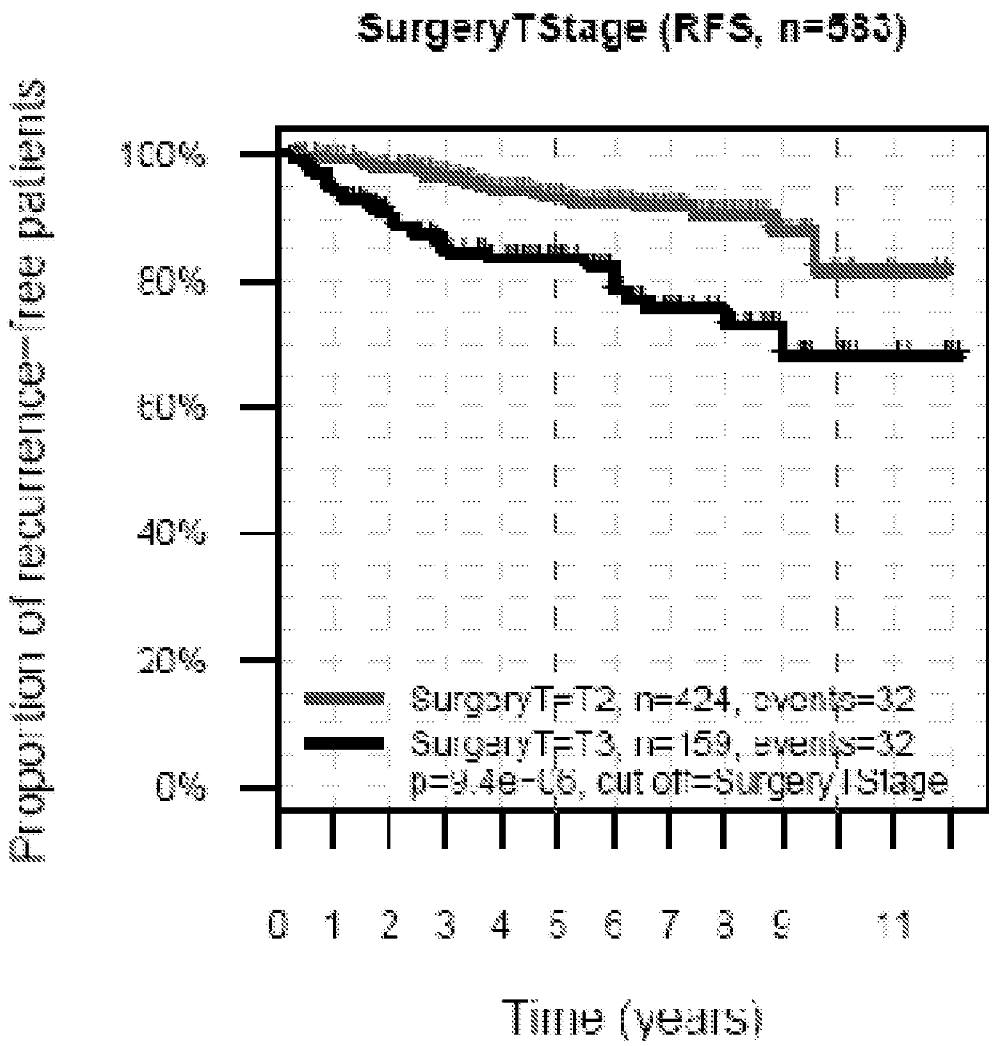
SurgeryTStage (RFS, n=583)
Proportion of recurrence-free patients
100%
80%
60%
40%
20%
0%
SurgeryT=T2, n=424, events=32
SurgeryT=T3, n=159, events=32
p=9.4e-06, cut off=SurgeryTStage
0 1 2 3 4 5 6 7 8 9 11
Time (years)

Figure 96/1

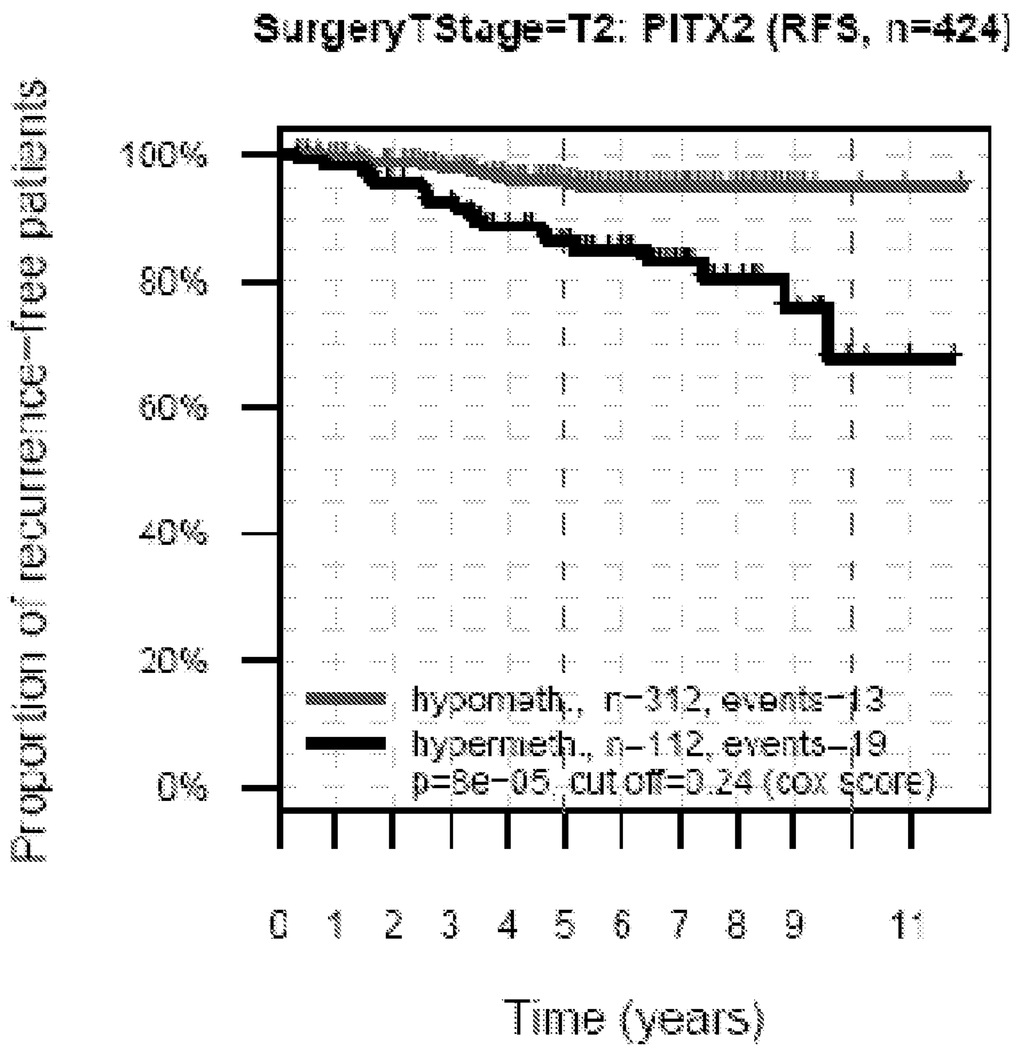
SurgeryTStage=T2: PITX2 (RFS, n=424)
Proportion of recurrence−free patients
100%
80%
60%
40%
20%
0%
hypometh., n=312, events=13
hypermeth., n=112, events=19
p=8e−05, cut off=0.24 (cox score)
0 1 2 3 4 5 6 7 8 9 11
Time (years)

Figure 96/2

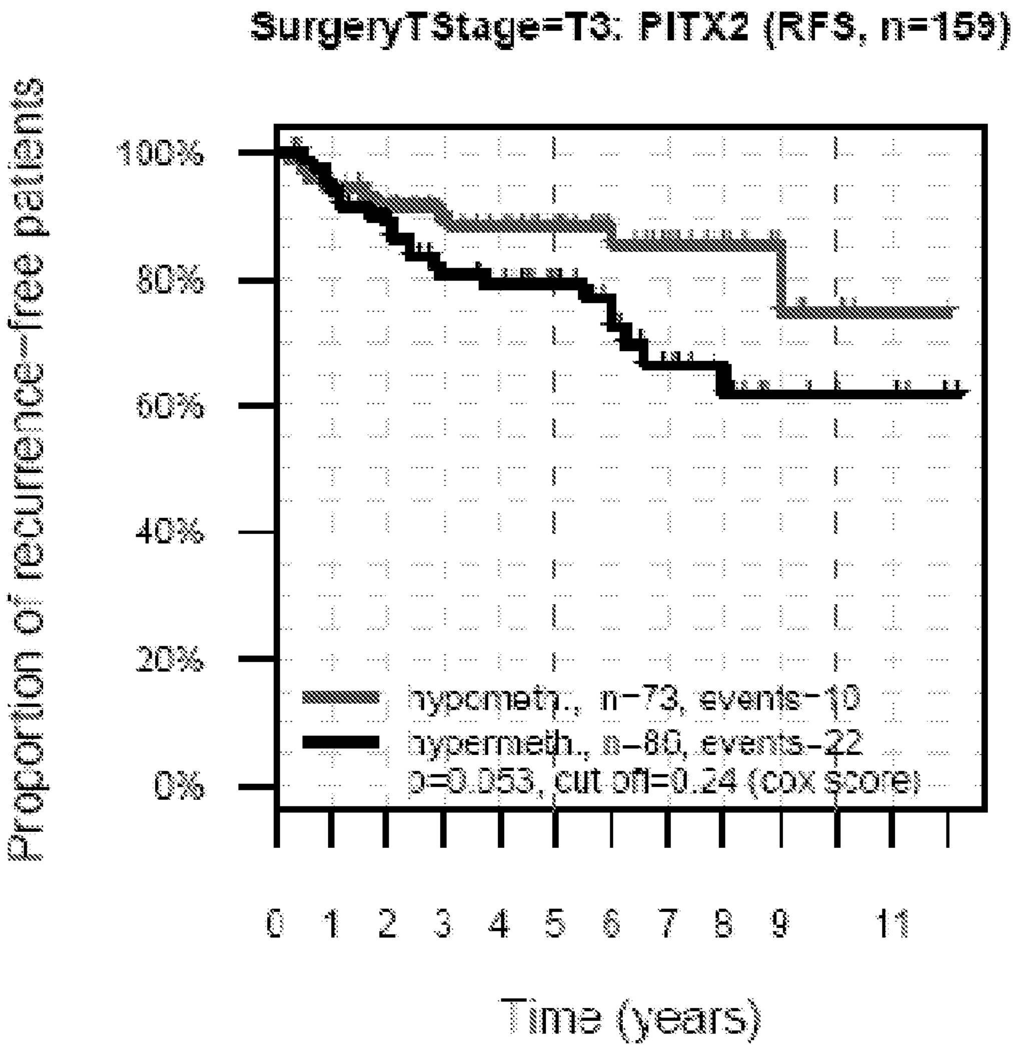
SurgeryTStage=T3: PITX2 (RFS, n=159)
Proportion of recurrence-free patients
100%
80%
60%
40%
20%
0%
hypometh., n=73, events=10
hypermeth., n=80, events=22
p=0.053, cut off=0.24 (cox score)
0 1 2 3 4 5 6 7 8 9 11
Time (years)

Figure 96/3

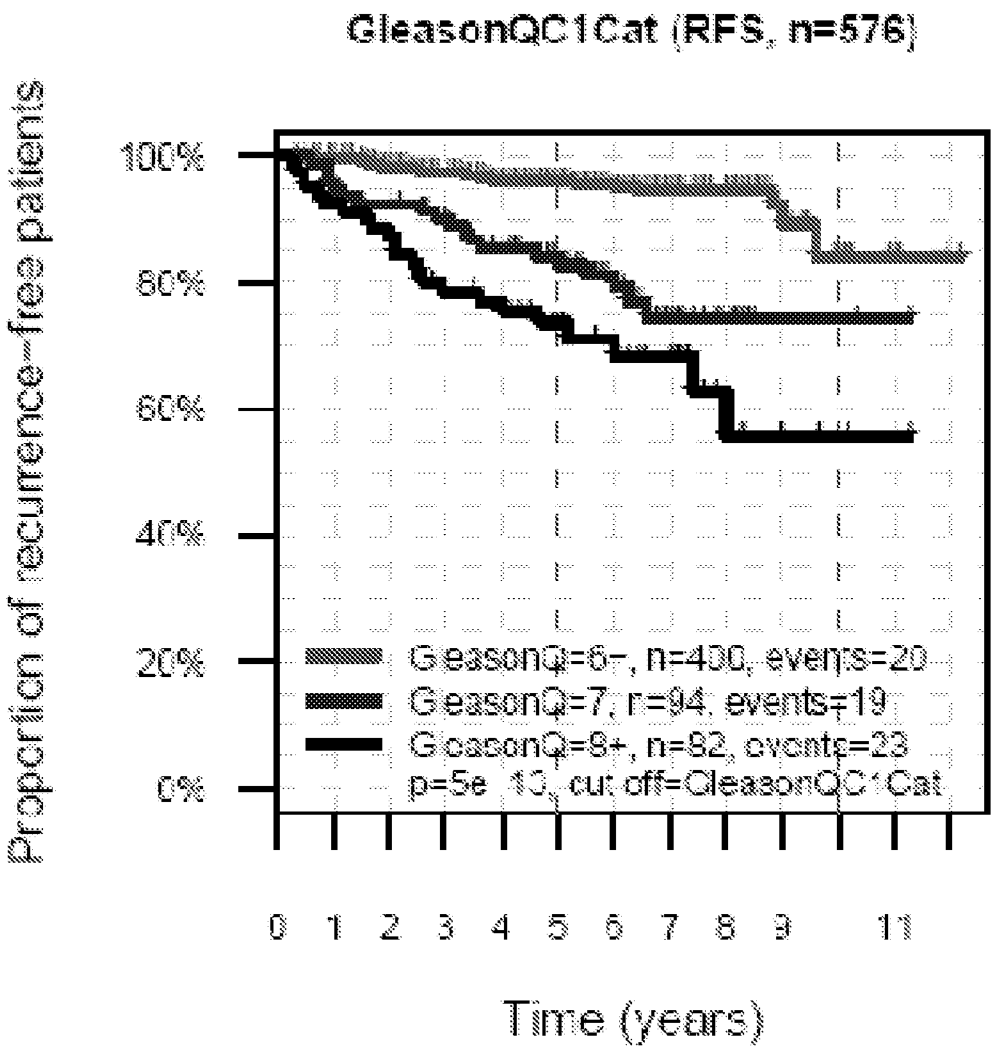
GleasonQC1Cat (RFS, n=576)
Proportion of recurrence-free patients
100%
80%
60%
40%
20%
0%
GleasonQ=6-, n=400, events=20
GleasonQ=7, n=94, events=19
GleasonQ=8+, n=82, events=23
p=5e-10, cut off=GleasonQC1Cat
0 1 2 3 4 5 6 7 8 9 11
Time (years)

**Figure 97/1**

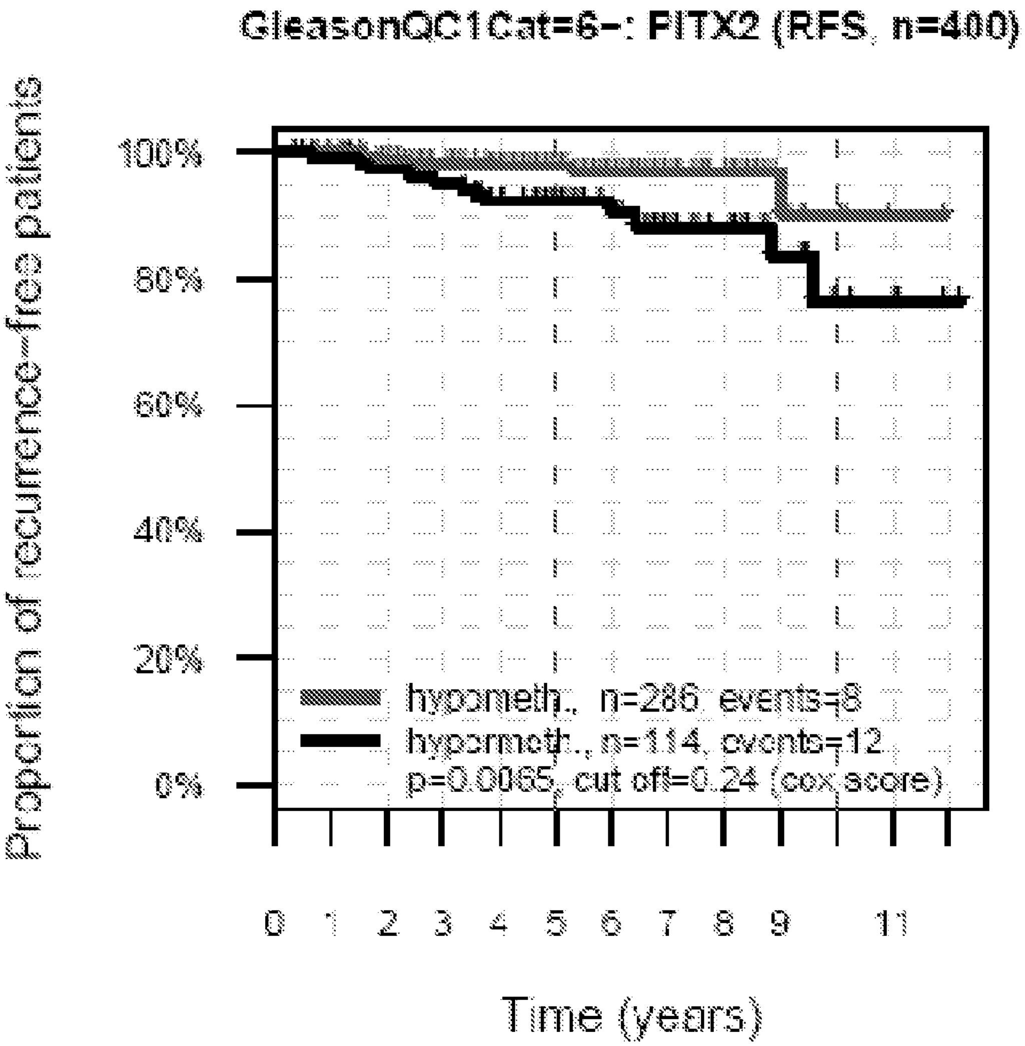
GleasonQC1Cat=6-: PITX2 (RFS, n=400)
Proportion of recurrence-free patients
100%
80%
60%
40%
20%
0%
hypometh., n=286, events=8
hypermeth., n=114, events=12
p=0.0065, cut off=0.24 (cox score)
0 1 2 3 4 5 6 7 8 9 11
Time (years)

Figure 97/2

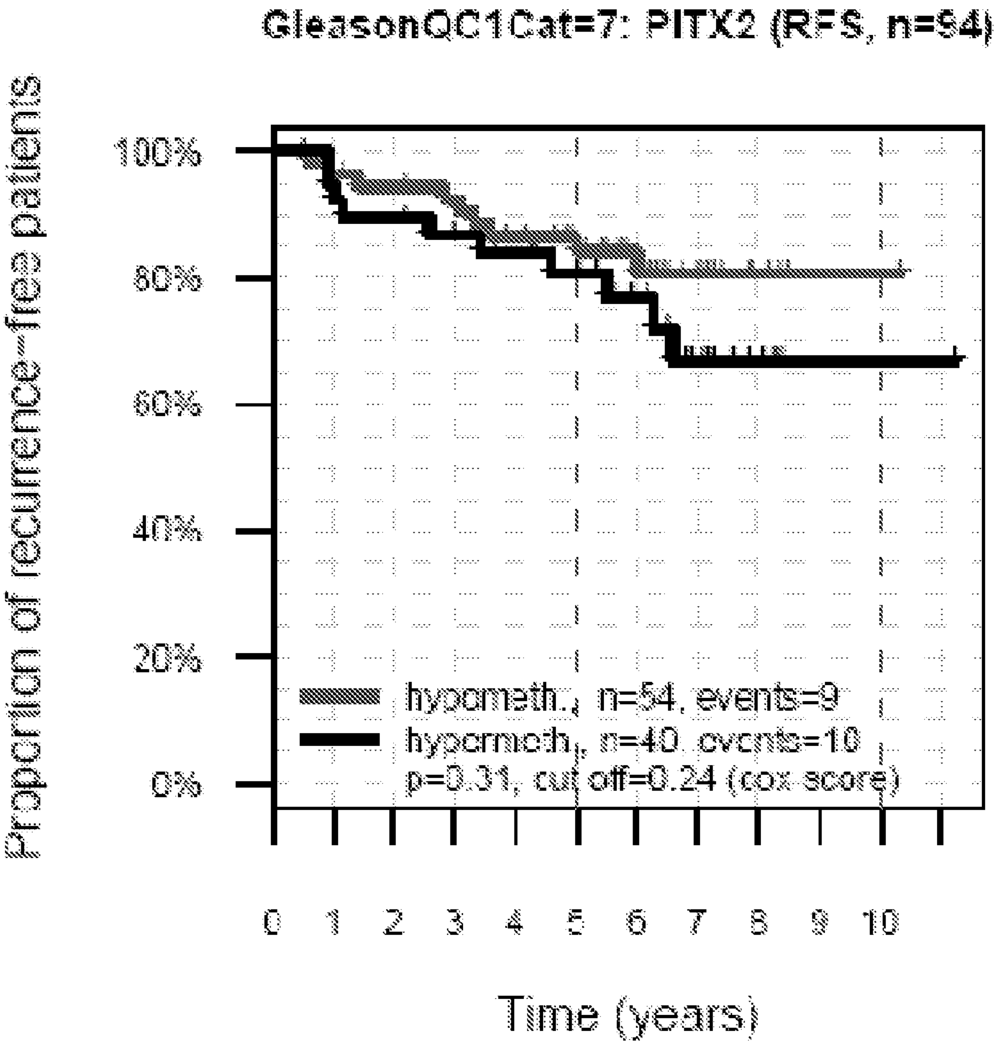
GleasonQC1Cat=7: PITX2 (RFS, n=94)
Proportion of recurrence-free patients
100%
80%
60%
40%
20%
0%
hypometh., n=54, events=9
hypermeth., n=40, events=10
p=0.31, cut off=0.24 (cox score)
0 1 2 3 4 5 6 7 8 9 10
Time (years)

Figure 97/3

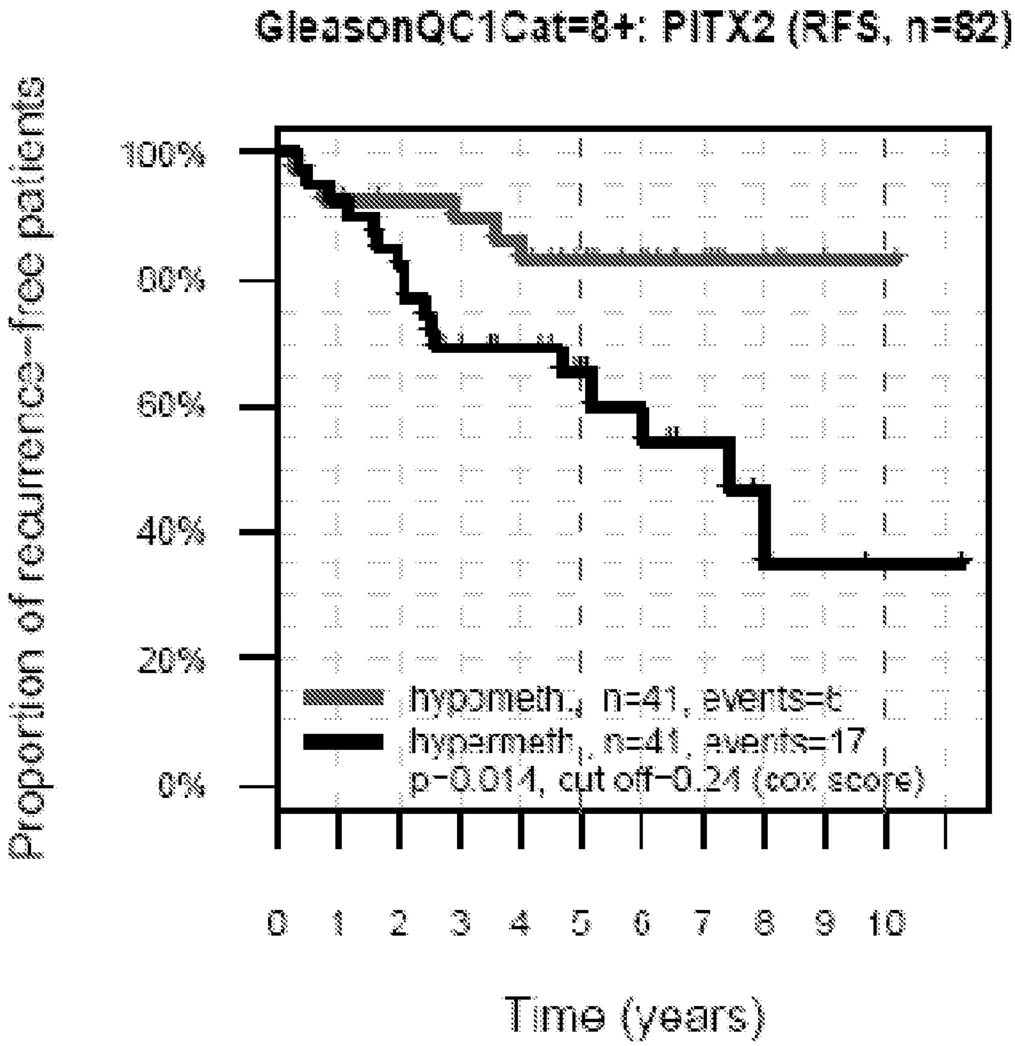
GleasonQC1Cat=8+: PITX2 (RFS, n=82)
Proportion of recurrence-free patients
100%
80%
60%
40%
20%
0%
hypometh., n=41, events=6
hypermeth., n=41, events=17
p=0.014, cut off=0.24 (cox score)
0 1 2 3 4 5 6 7 8 9 10
Time (years)

Figure 97/4

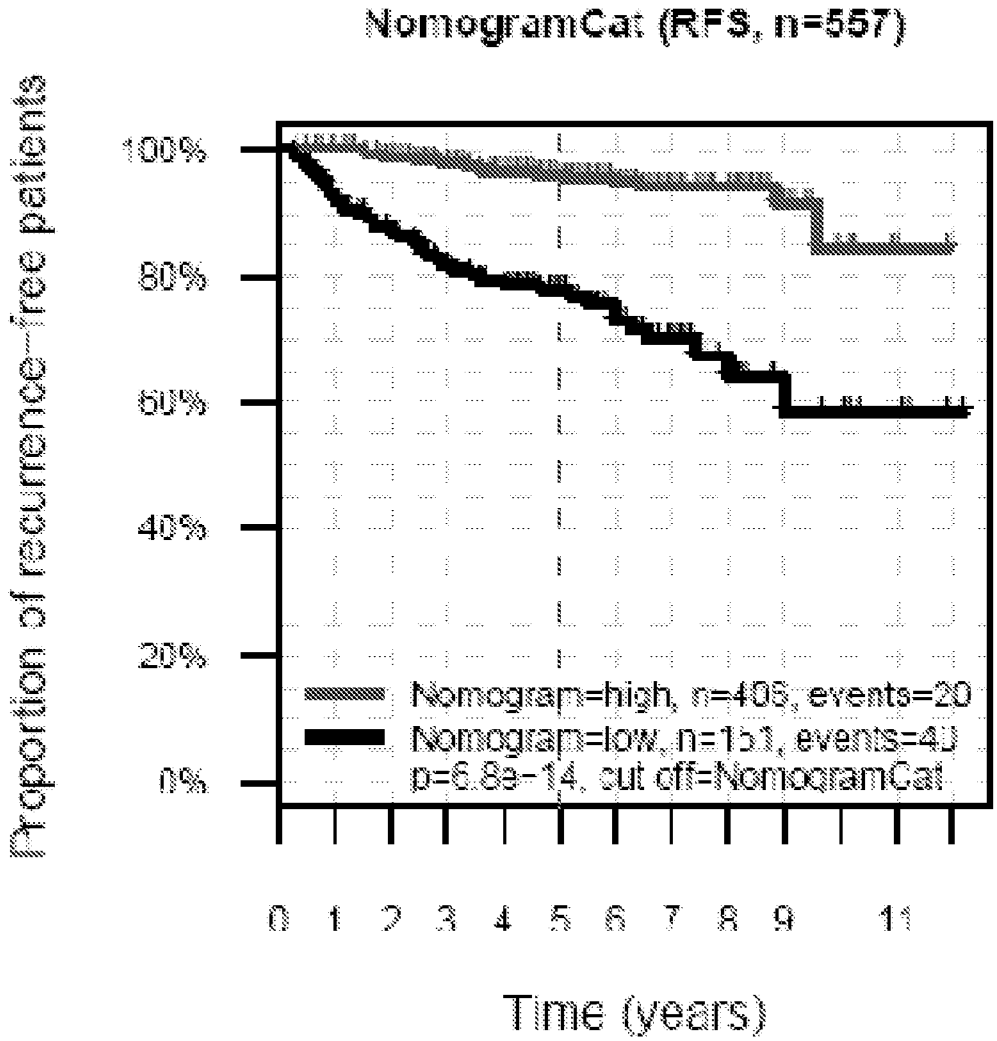
NomogramCat (RFS, n=557)
Proportion of recurrence−free patients
100%
80%
60%
40%
20%
0%
Nomogram=high, n=406, events=20
Nomogram=low, n=151, events=40
p=6.8e−14, cut off=NomogramCat
0 1 2 3 4 5 6 7 8 9 11
Time (years)

Figure 98/1

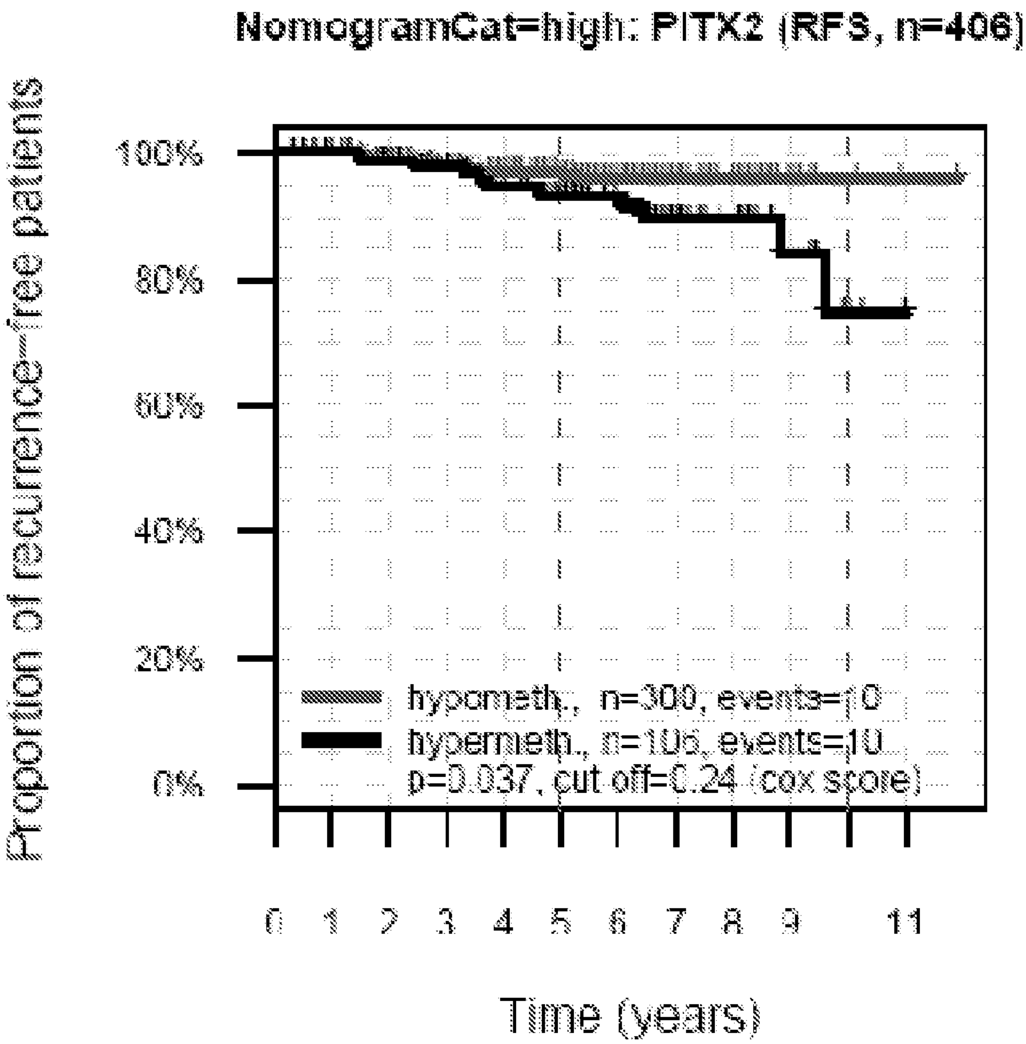
NomogramCat=high: PITX2 (RFS, n=406)
Proportion of recurrence-free patients
100%
80%
60%
40%
20%
0%
hypometh., n=300, events=10
hypermeth., n=106, events=10
p=0.037, cut off=0.24 (cox score)
0 1 2 3 4 5 6 7 8 9 11
Time (years)

Figure 98/2

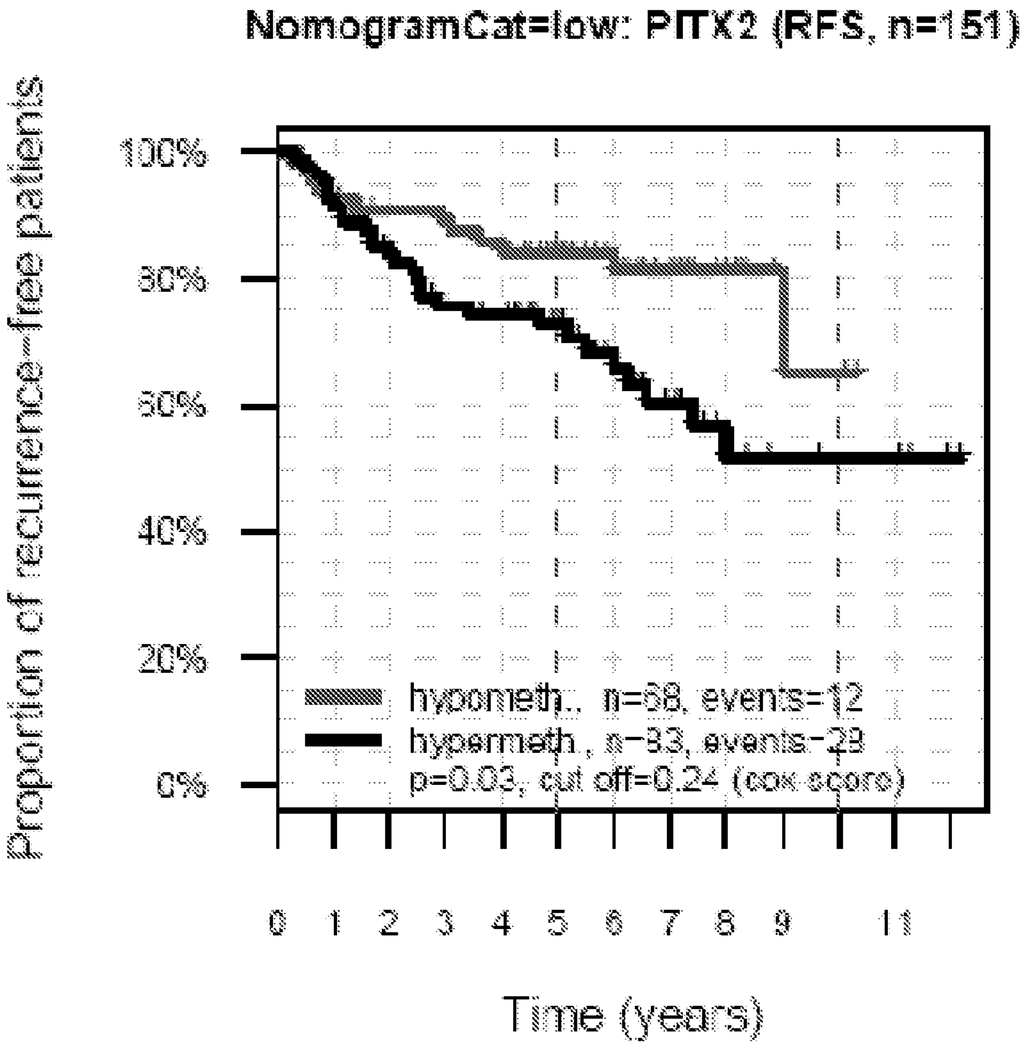
NomogramCat=low: PITX2 (RFS, n=151)
Proportion of recurrence-free patients
100%
80%
60%
40%
20%
0%
hypometh., n=68, events=12
hypermeth., n=83, events=29
p=0.03, cut off=0.24 (cox score)
0 1 2 3 4 5 6 7 8 9 11
Time (years)

Figure 98/3

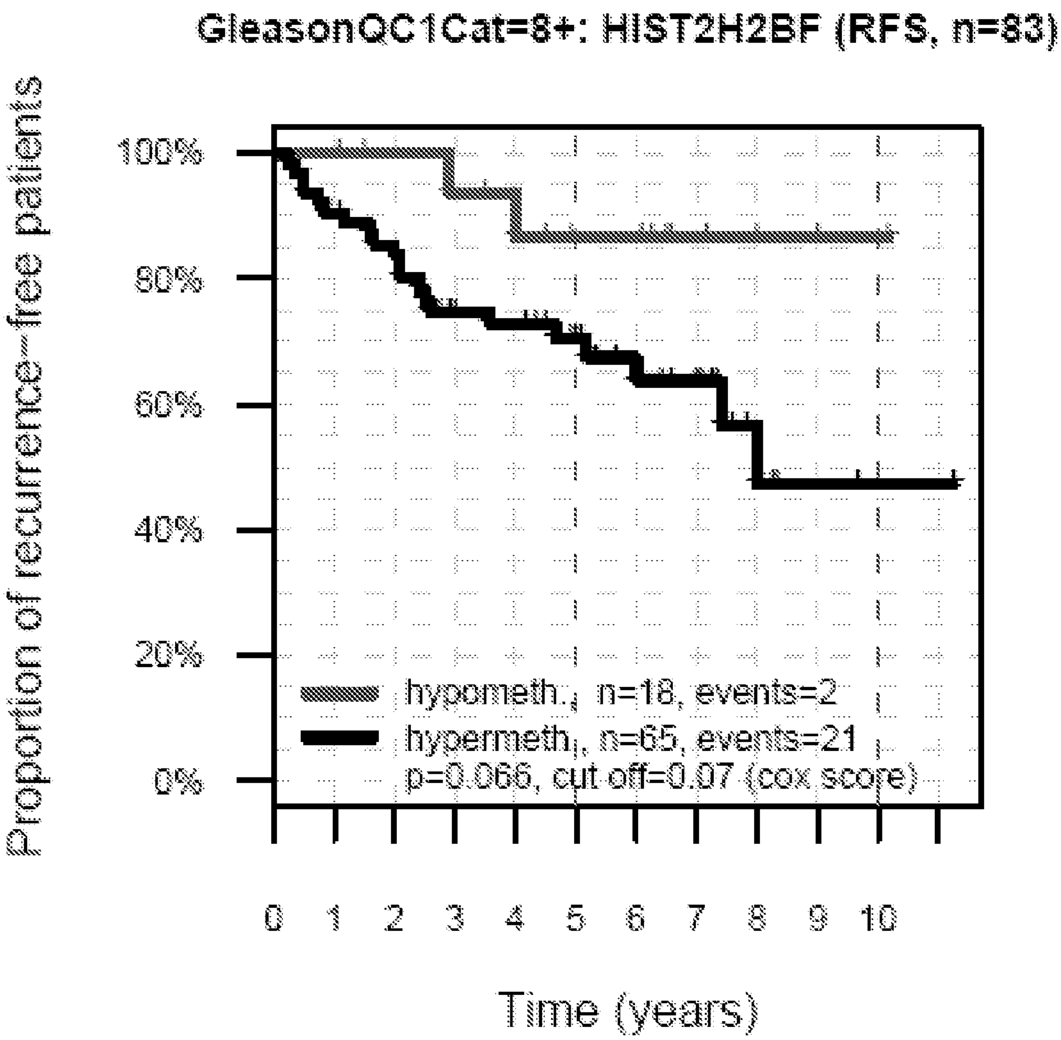
GleasonQC1Cat=8+: HIST2H2BF (RFS, n=83)
Proportion of recurrence−free patients
100%
80%
60%
40%
20%
0%
hypometh., n=18, events=2
hypermeth., n=65, events=21
p=0.066, cut off=0.07 (cox score)
0 1 2 3 4 5 6 7 8 9 10
Time (years)

Figure 99

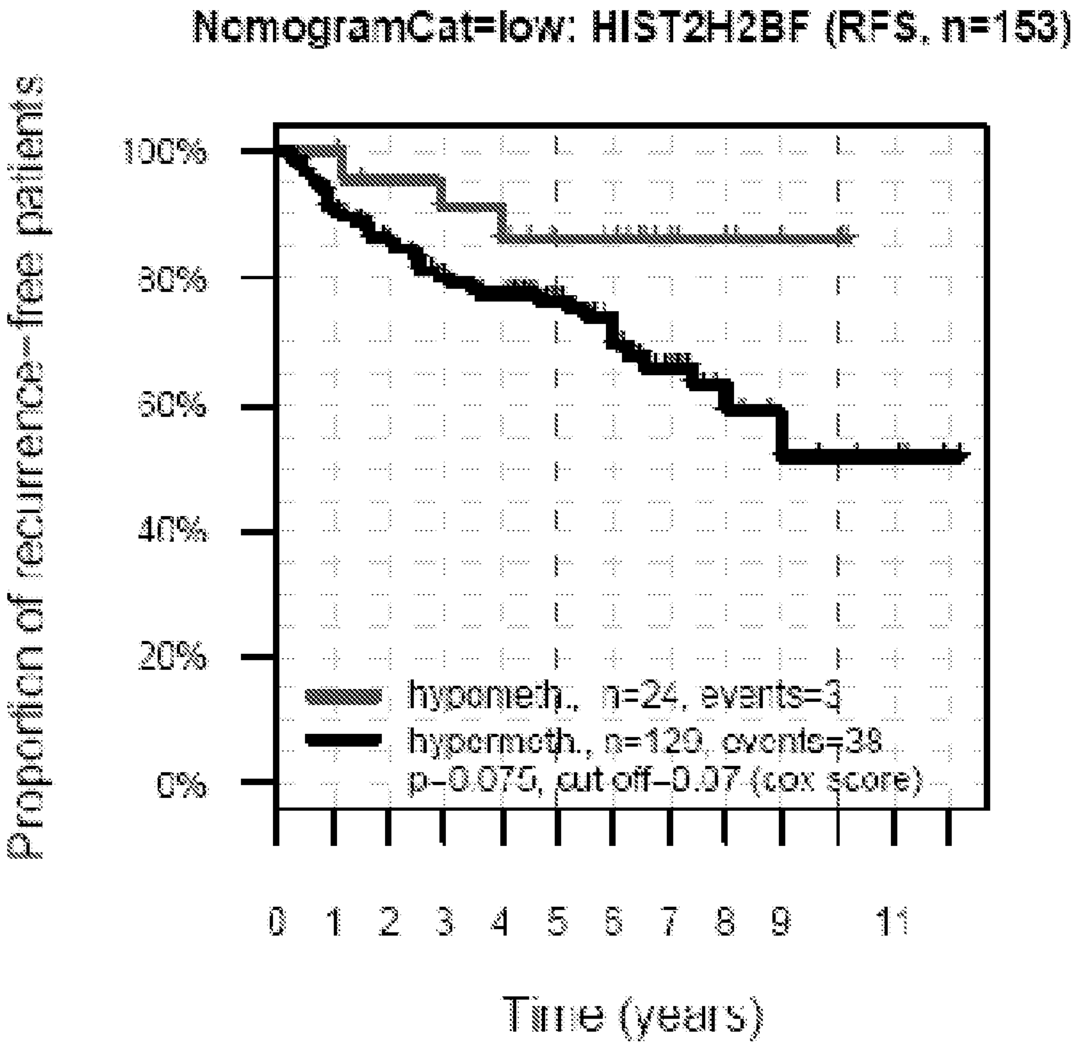
NomogramCat=low: HIST2H2BF (RFS, n=153)
Proportion of recurrence-free patients
100%
80%
60%
40%
20%
0%
hypometh., n=24, events=3
hypermeth., n=129, events=38
p=0.075, cut off=0.07 (cox score)
0 1 2 3 4 5 6 7 8 9 11
Time (years)

Figure 100

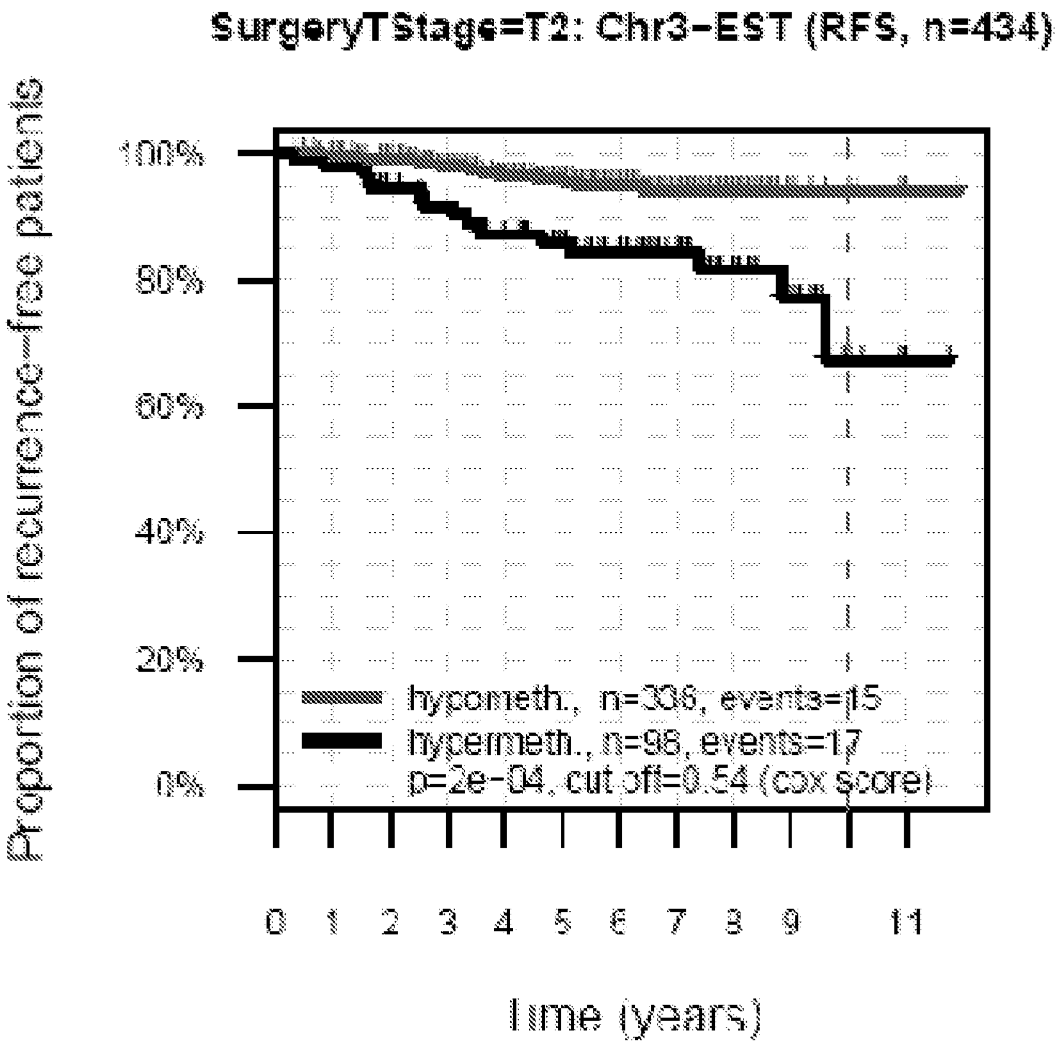
SurgeryTStage=T2: Chr3-EST (RFS, n=434)
Proportion of recurrence-free patients
100%
80%
60%
40%
20%
0%
hypometh., n=336, events=15
hypermeth., n=98, events=17
p=2e-04, cut off=0.54 (cox score)
0
1
2
3
4
5
6
7
8
9
11
Time (years)

Figure 101

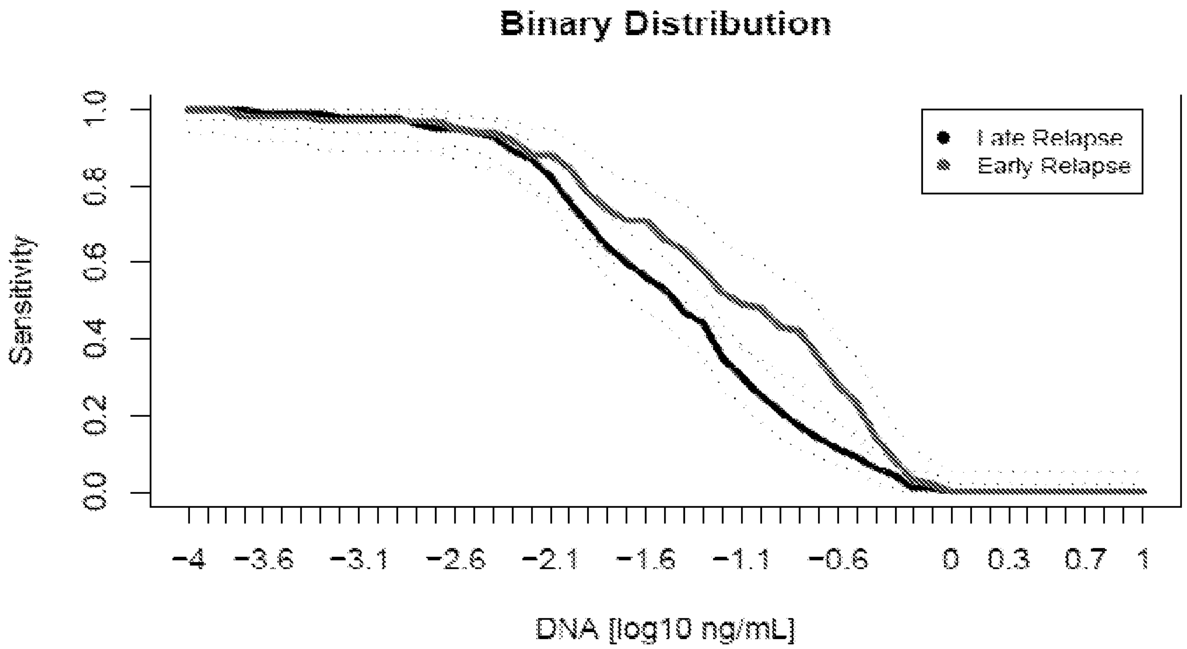
Binary Distribution
Sensitivity
1.0
0.8
0.6
0.4
0.2
0.0
Late Relapse
Early Relapse
-4
-3.6
-3.1
-2.6
-2.1
-1.6
-1.1
-0.6
0
0.3
0.7
1
DNA [log10 ng/mL]

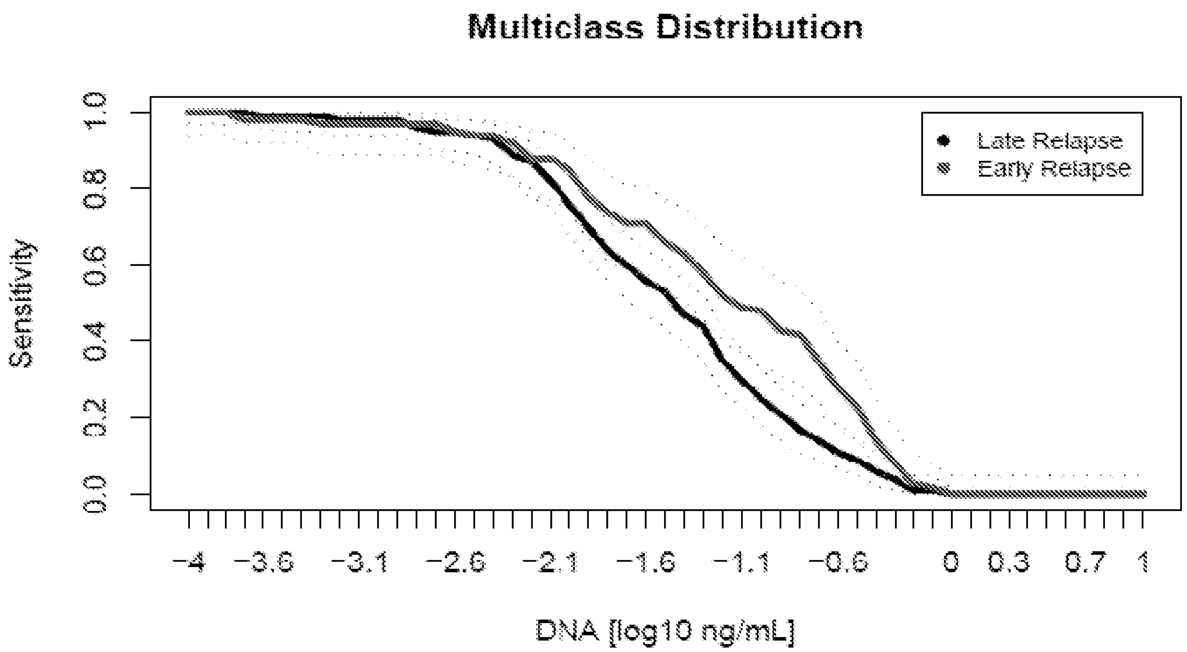
Multiclass Distribution
Sensitivity
1.0
0.8
0.6
0.4
0.2
0.0
Late Relapse
Early Relapse
-4
-3.6
-3.1
-2.6
-2.1
-1.6
-1.1
-0.6
0
0.3
0.7
1
DNA [log10 ng/mL]

Figure 102/1

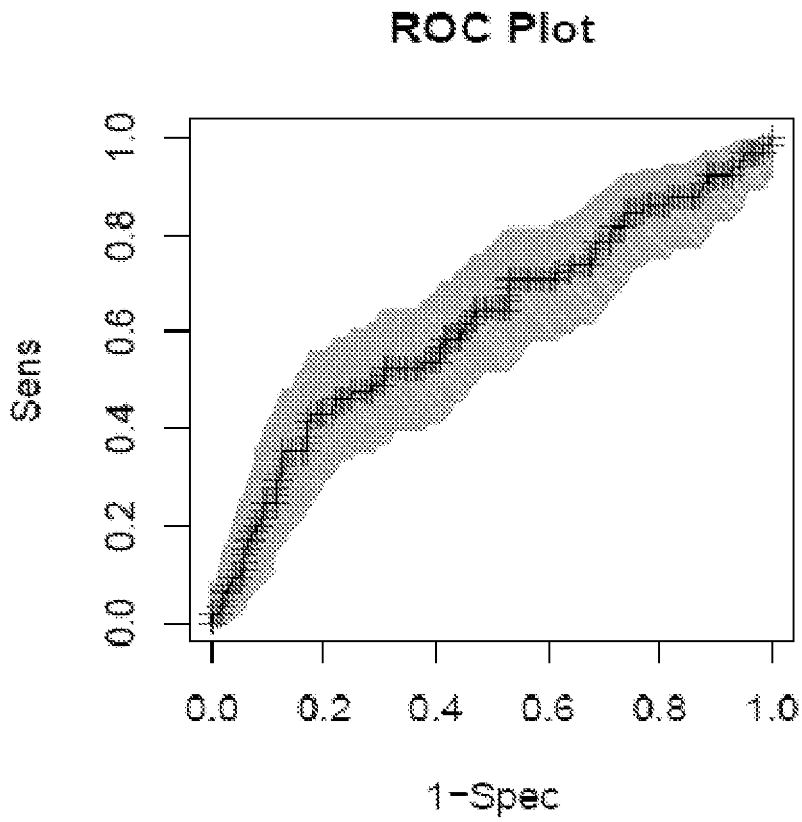


| | |
|---|---|
| AUC | 0.62 [0.54,0.68] |
| Sens / Spec | 0.35 / 0.86 |
| Wilcoxon *P* | 0.0072 |
| Early Relapse (pos) | 65 |
| Late Relapse (neg) | 140 |

**Figure 102/2**

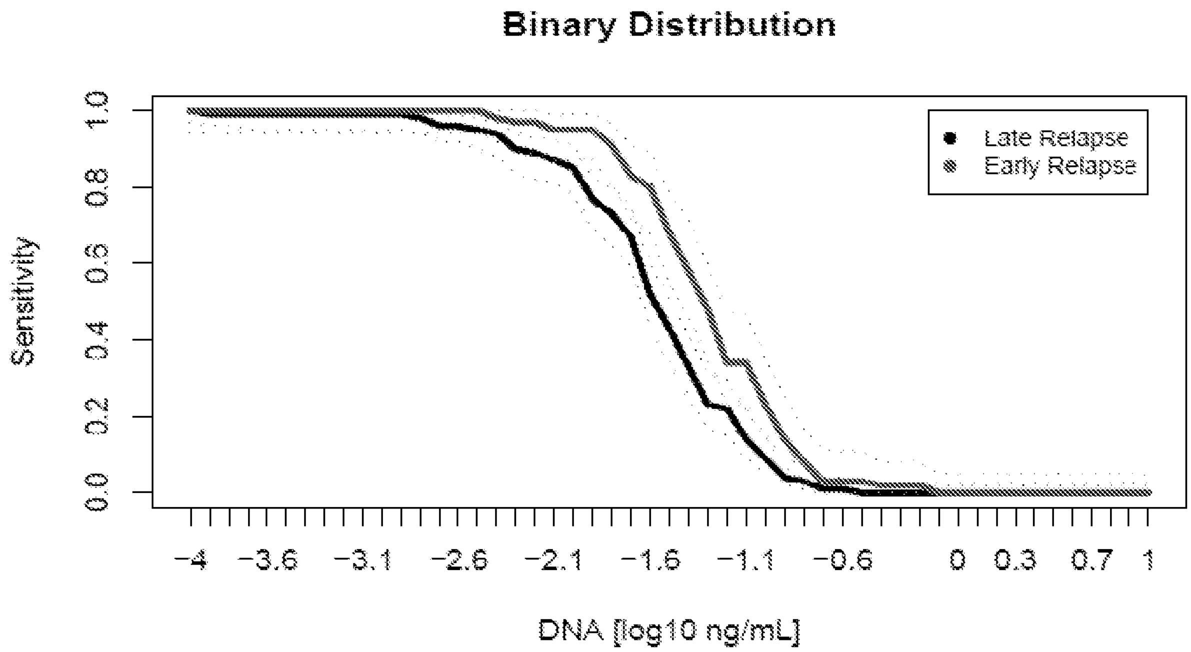
Binary Distribution
Late Relapse
Early Relapse
Sensitivity
0.0
0.2
0.4
0.6
0.8
1.0
-4
-3.6
-3.1
-2.6
-2.1
-1.6
-1.1
-0.6
0
0.3
0.7
1
DNA [log10 ng/mL]

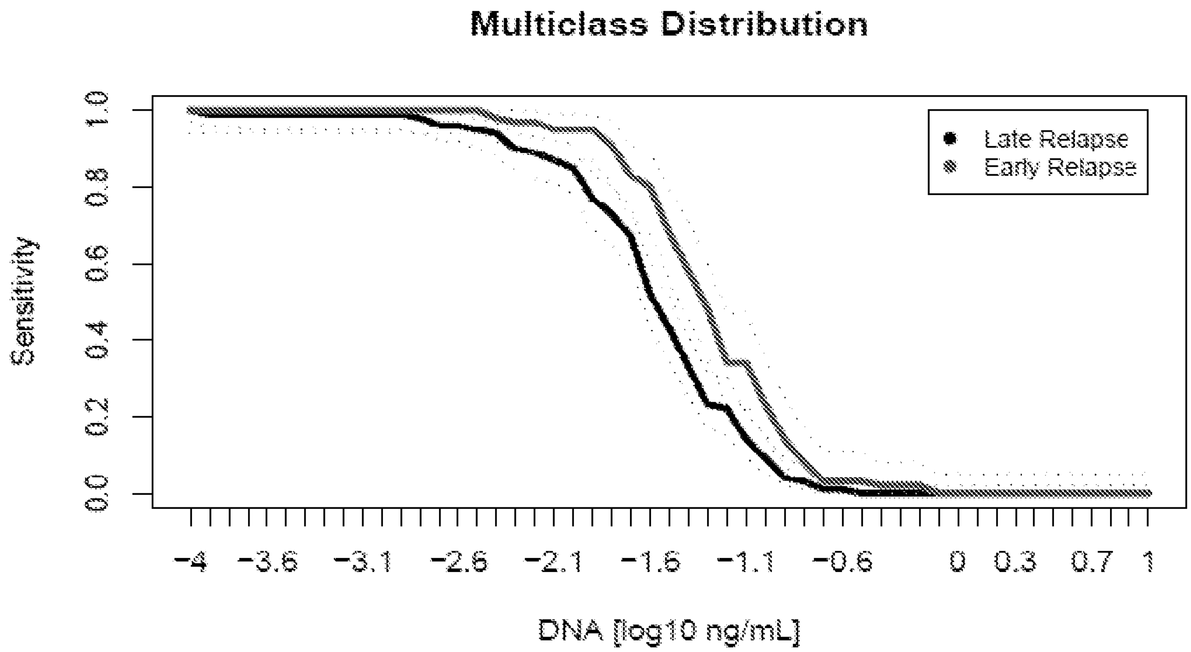
Multiclass Distribution
Late Relapse
Early Relapse
Sensitivity
0.0
0.2
0.4
0.6
0.8
1.0
-4
-3.6
-3.1
-2.6
-2.1
-1.6
-1.1
-0.6
0
0.3
0.7
1
DNA [log10 ng/mL]

Figure 103/1

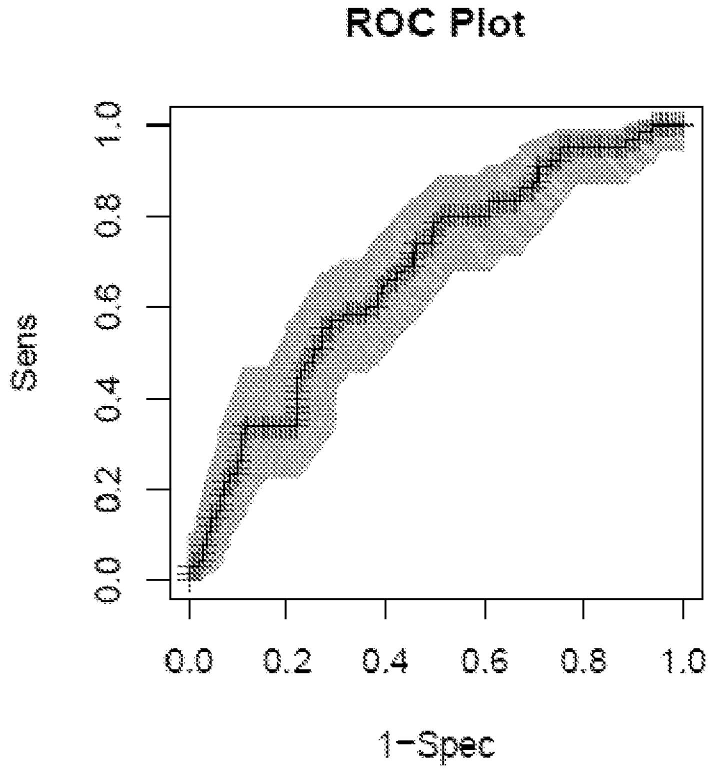


| | |
|---|---|
| AUC | 0.68 [0.61,0.74] |
| Sens / Spec | 0.34 / 0.85 |
| Wilcoxon *P* | 1e-04 |
| Early Relapse (pos) | 65 |
| Late Relapse (neg) | 141 |

**Figure 103/2**

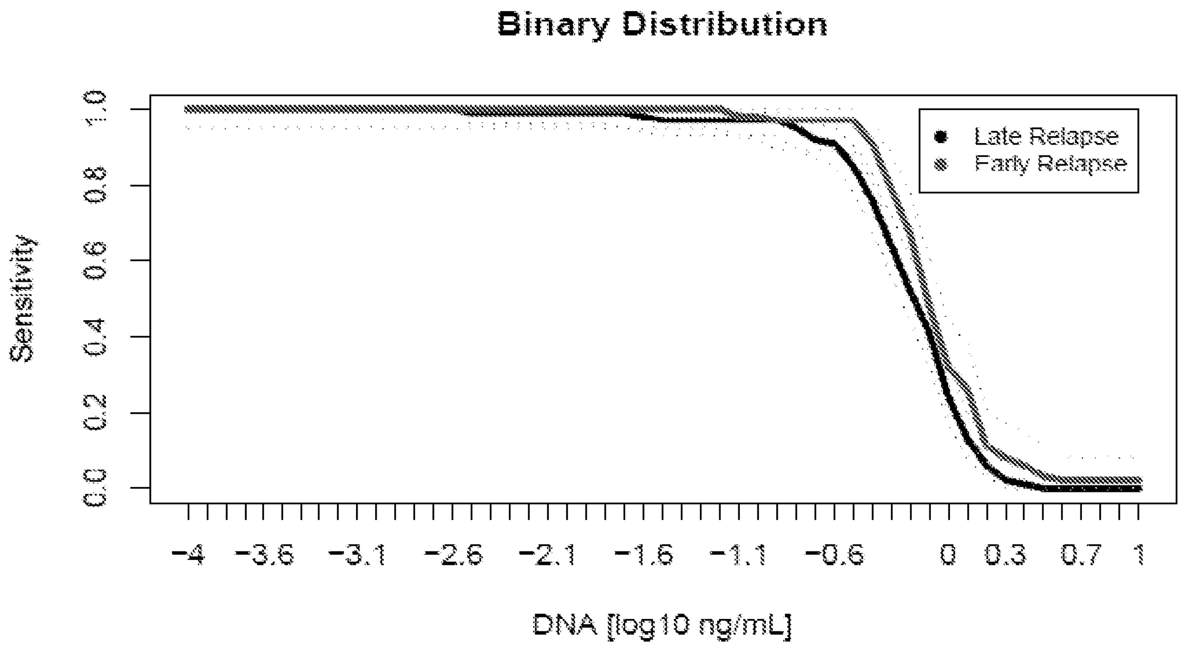
Binary Distribution
Sensitivity
1.0
0.8
0.6
0.4
0.2
0.0
Late Relapse
Early Relapse
-4
-3.6
-3.1
-2.6
-2.1
-1.6
-1.1
-0.6
0
0.3
0.7
1
DNA [log10 ng/mL]

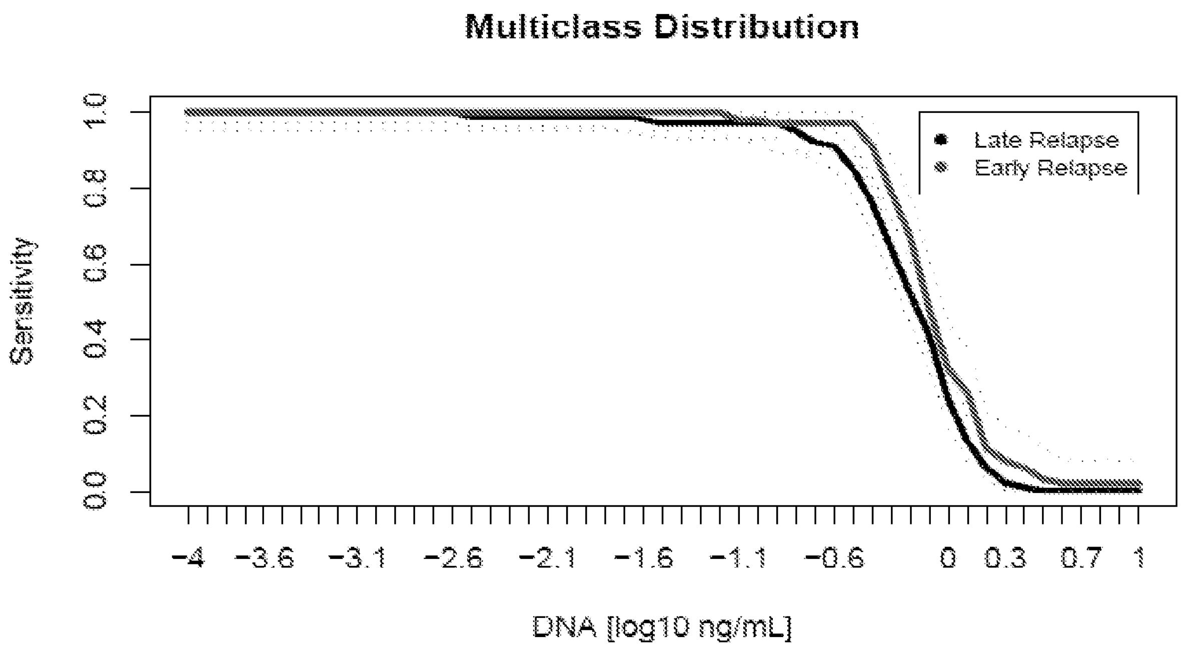
Multiclass Distribution
Sensitivity
1.0
0.8
0.6
0.4
0.2
0.0
Late Relapse
Early Relapse
-4
-3.6
-3.1
-2.6
-2.1
-1.6
-1.1
-0.6
0
0.3
0.7
1
DNA [log10 ng/mL]

Figure 104/1

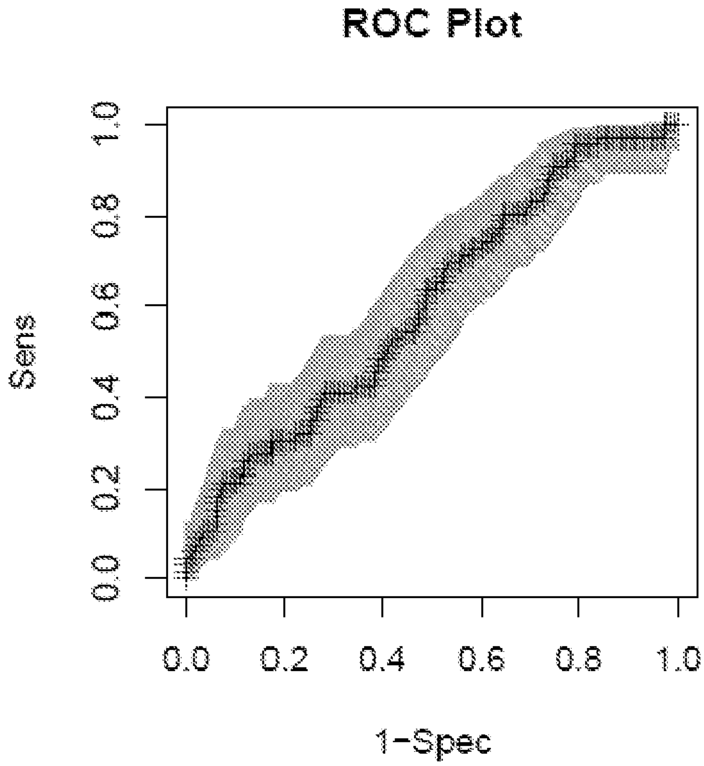


| | |
|---|---|
| AUC | 0.6 [0.53,0.67] |
| Sens / Spec | 0.27 / 0.85 |
| Wilcoxon *P* | 0.0178 |
| Early Relapse (pos) | 66 |
| Late Relapse (neg) | 143 |

**Figure 104/2**

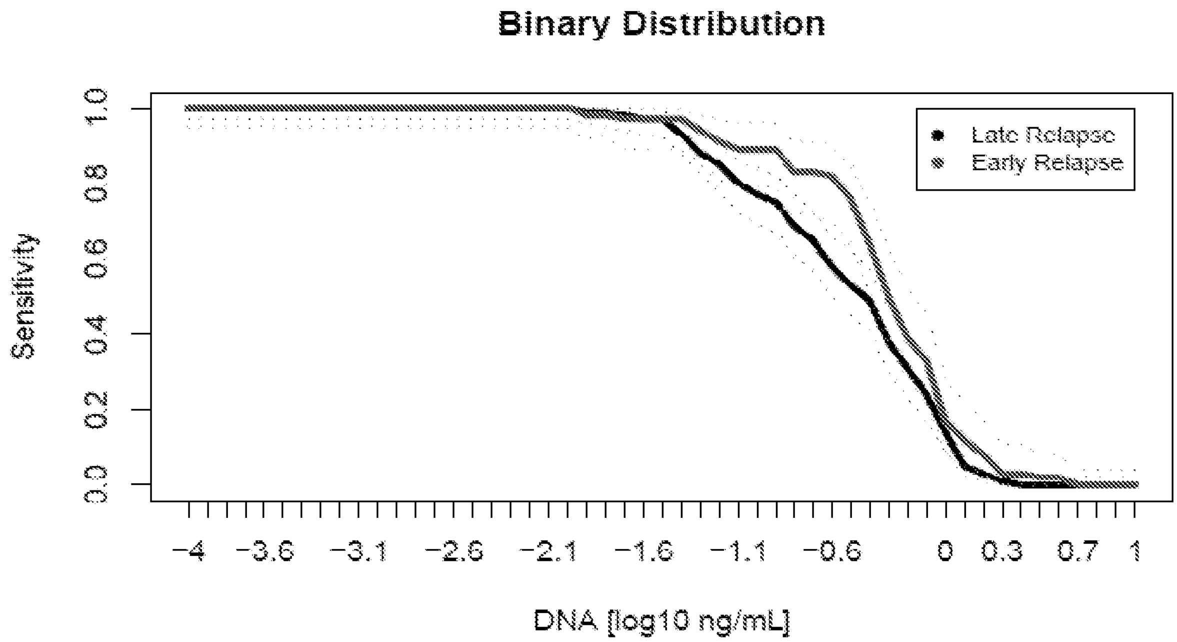
Binary Distribution
Sensitivity
1.0
0.8
0.6
0.4
0.2
0.0
Late Relapse
Early Relapse
-4
-3.6
-3.1
-2.6
-2.1
-1.6
-1.1
-0.6
0
0.3
0.7
1
DNA [log10 ng/mL]

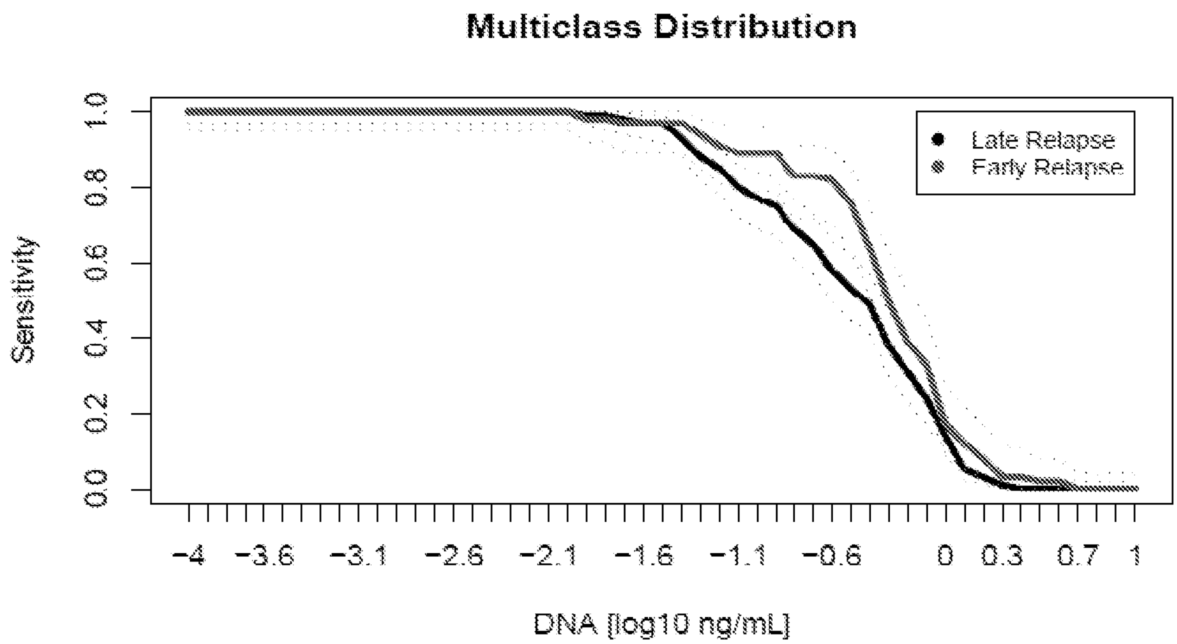
Multiclass Distribution
Sensitivity
1.0
0.8
0.6
0.4
0.2
0.0
Late Relapse
Early Relapse
-4
-3.6
-3.1
-2.6
-2.1
-1.6
-1.1
-0.6
0
0.3
0.7
1
DNA [log10 ng/mL]

Figure 105/1

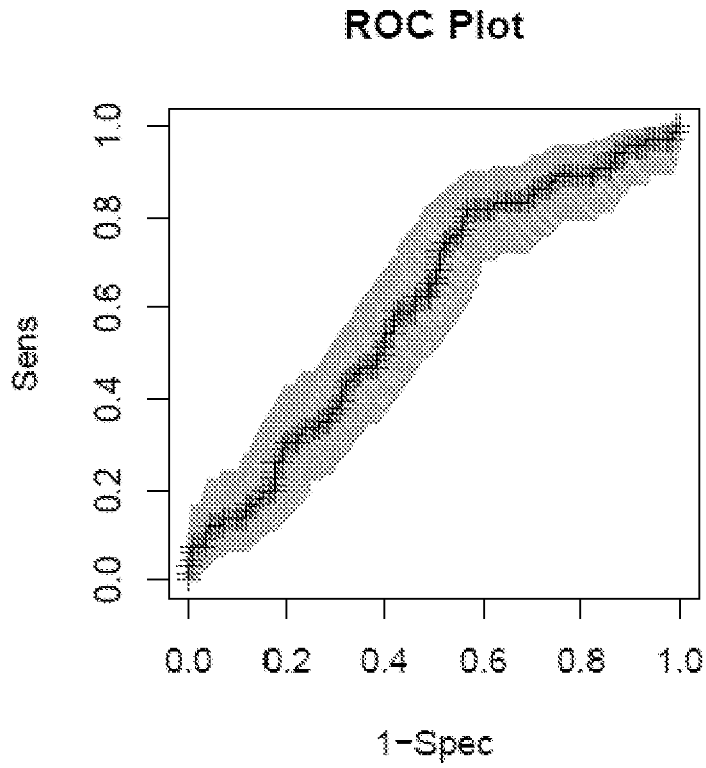


| | |
|---|---|
| AUC | 0.61 [0.53,0.67] |
| Sens / Spec | 0.18 / 0.85 |
| Wilcoxon *P* | 0.0146 |
| Early Relapse (pos) | 66 |
| Late Relapse (neg) | 143 |

**Figure 105/2**

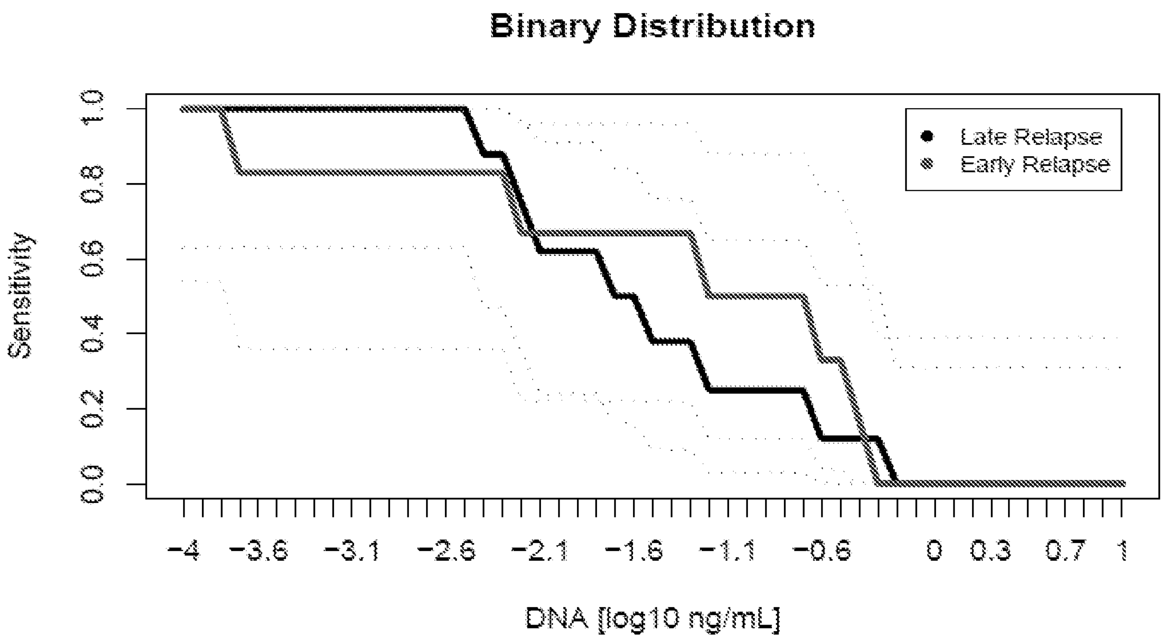
Binary Distribution
Late Relapse
Early Relapse
Sensitivity
0.0
0.2
0.4
0.6
0.8
1.0
-4
-3.6
-3.1
-2.6
-2.1
-1.6
-1.1
-0.6
0
0.3
0.7
1
DNA [log10 ng/mL]

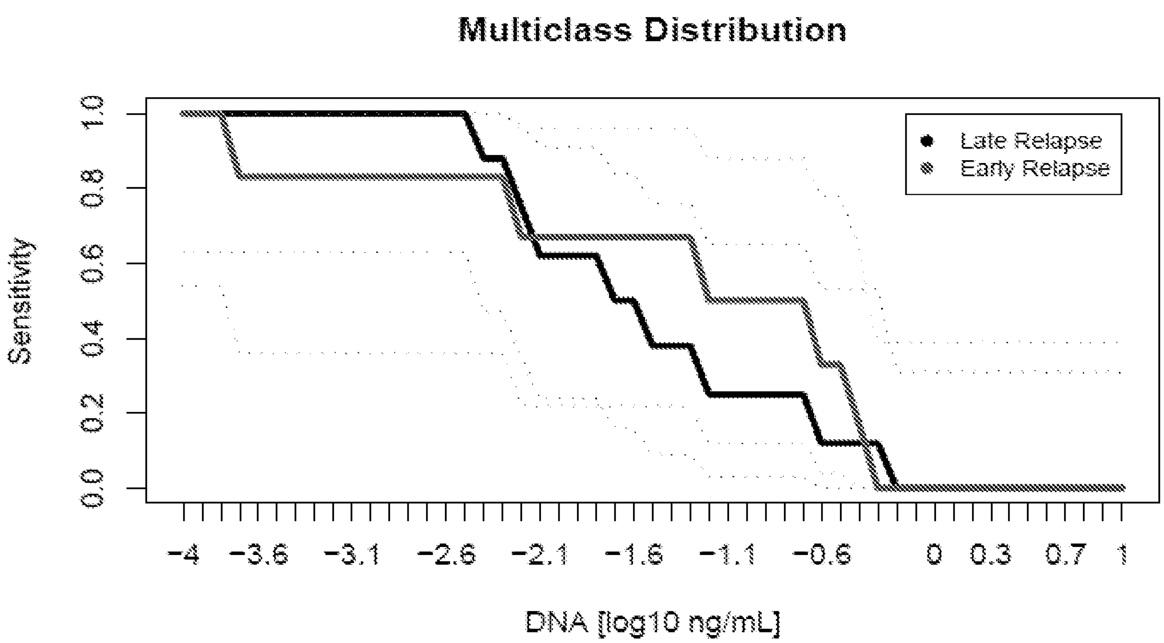
Multiclass Distribution
Late Relapse
Early Relapse
Sensitivity
0.0
0.2
0.4
0.6
0.8
1.0
-4
-3.6
-3.1
-2.6
-2.1
-1.6
-1.1
-0.6
0
0.3
0.7
1
DNA [log10 ng/mL]

Figure 106/1

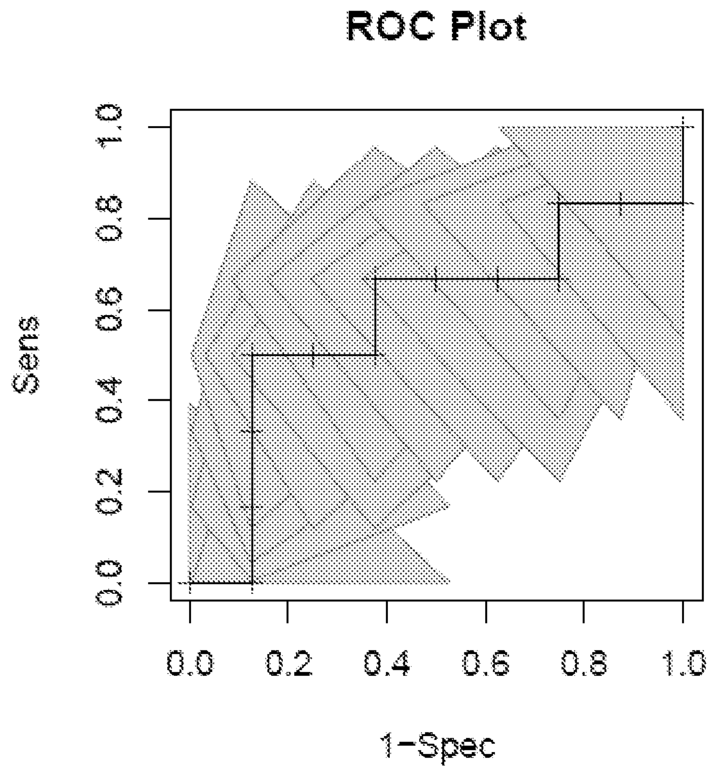


| | |
|---|---|
| AUC | 0.58 [0.29,0.82] |
| Sens / Spec | 0.5 / 0.88 |
| Wilcoxon *P* | 0.662 |
| Early Relapse (pos) | 6 |
| Late Relapse (neg) | 8 |

**Figure 106/2**

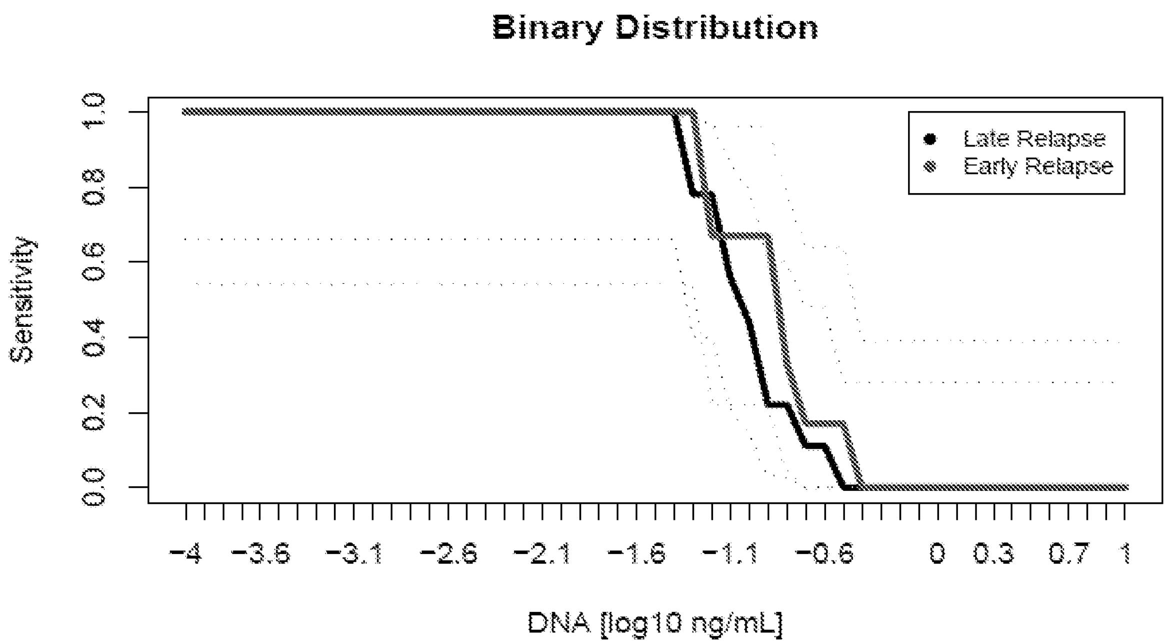
Binary Distribution
Sensitivity
1.0
0.8
0.6
0.4
0.2
0.0
Late Relapse
Early Relapse
-4
-3.6
-3.1
-2.6
-2.1
-1.6
-1.1
-0.6
0
0.3
0.7
1
DNA [log10 ng/mL]

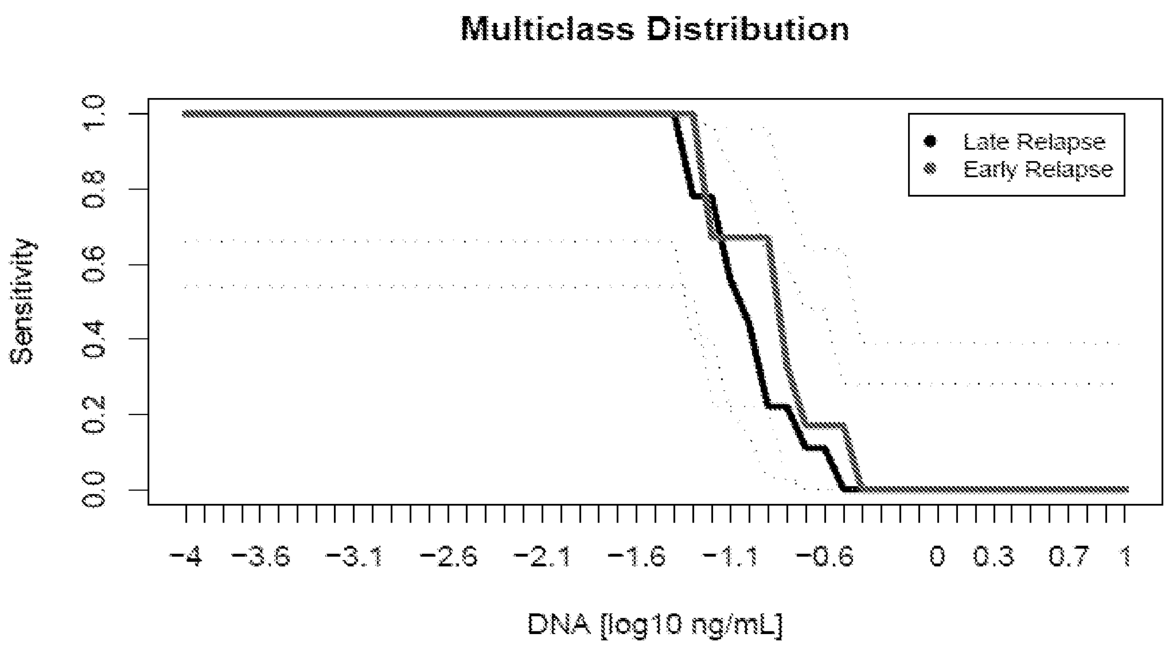
Multiclass Distribution
Sensitivity
1.0
0.8
0.6
0.4
0.2
0.0
Late Relapse
Early Relapse
-4
-3.6
-3.1
-2.6
-2.1
-1.6
-1.1
-0.6
0
0.3
0.7
1
DNA [log10 ng/mL]

Figure 107/1

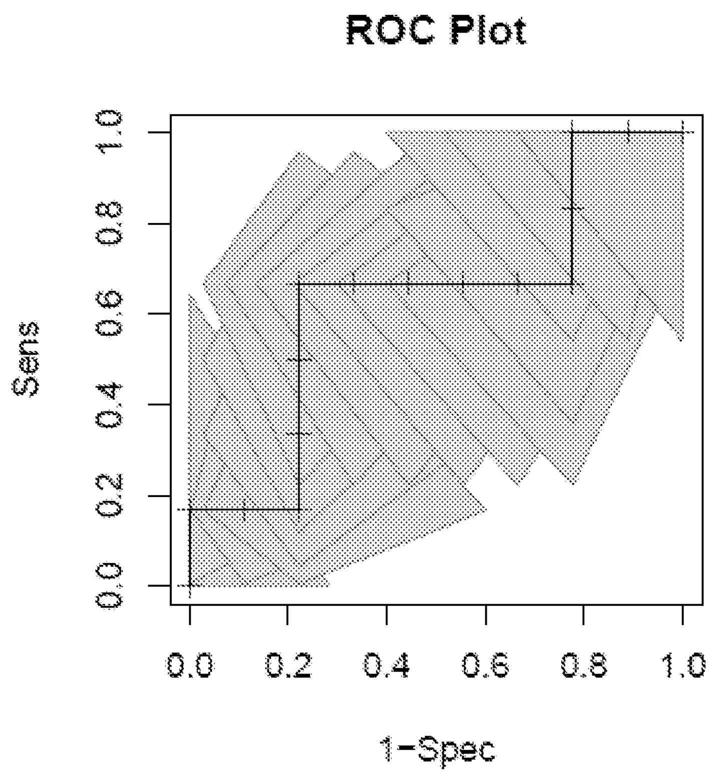


| | |
|---|---|
| AUC | 0.63 [0.32,0.84] |
| Sens / Spec | 0.17 / 0.89 |
| Wilcoxon *P* | 0.4559 |
| Early Relapse (pos) | 6 |
| Late Relapse (neg) | 9 |

**Figure 107/2**

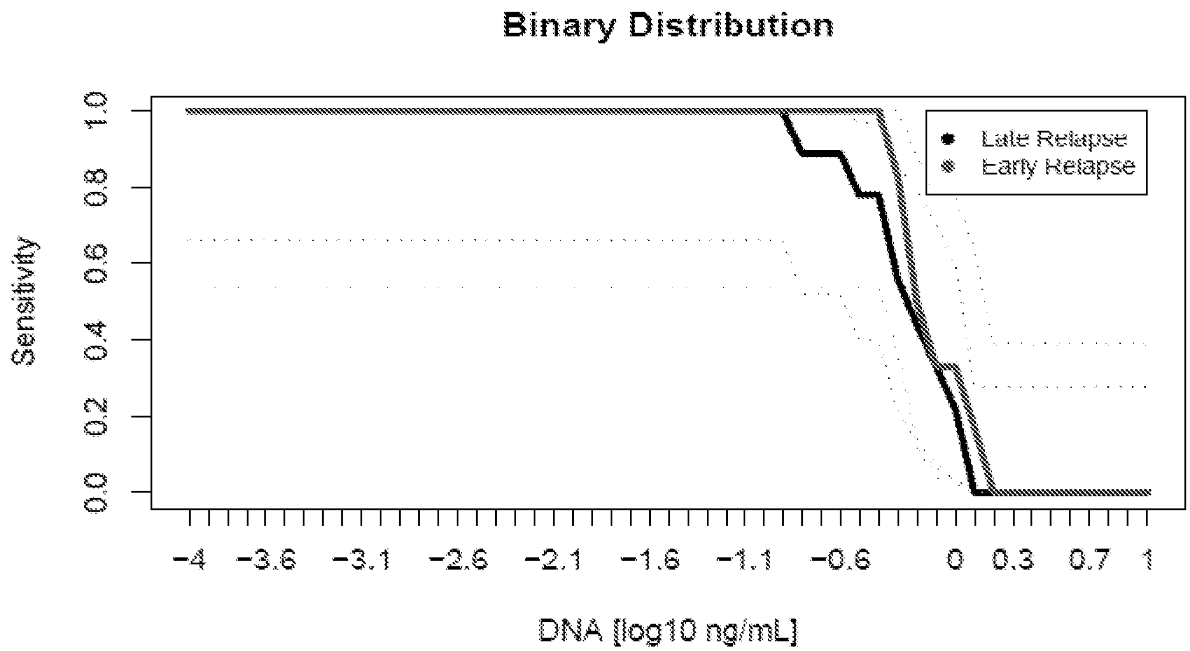

Binary Distribution
Sensitivity
1.0
0.8
0.6
0.4
0.2
0.0
Late Relapse
Early Relapse
-4
-3.6
-3.1
-2.6
-2.1
-1.6
-1.1
-0.6
0
0.3
0.7
1
DNA [log10 ng/mL]


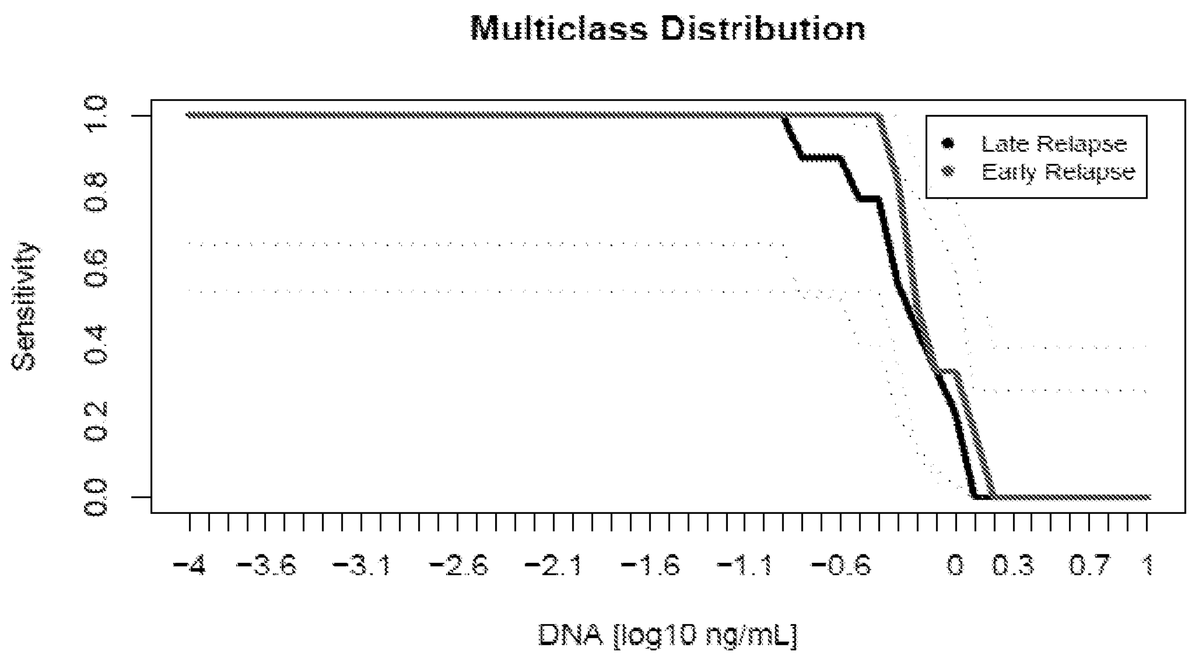

Multiclass Distribution
Sensitivity
1.0
0.8
0.6
0.4
0.2
0.0
Late Relapse
Early Relapse
-4
-3.6
-3.1
-2.6
-2.1
-1.6
-1.1
-0.6
0
0.3
0.7
1
DNA [log10 ng/mL]


Figure 108/1

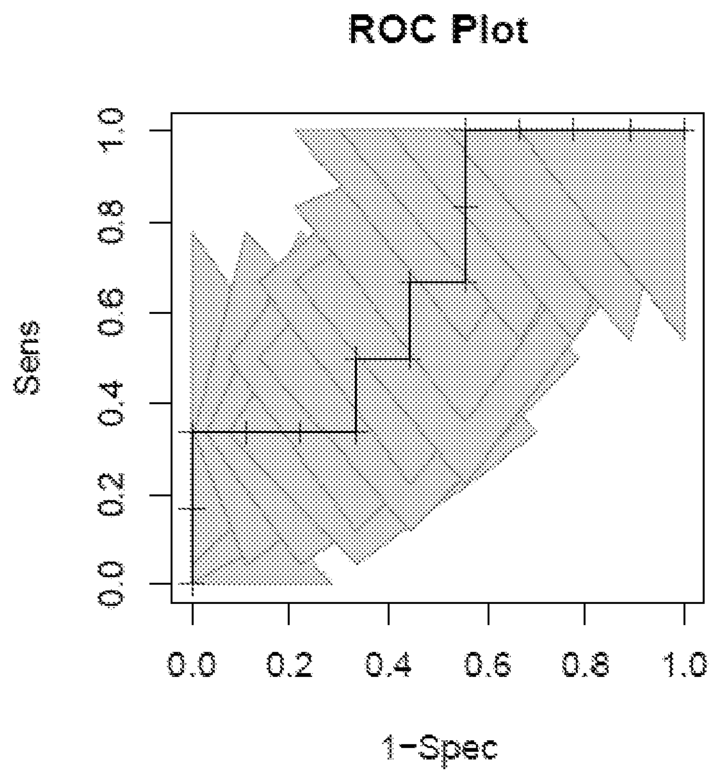


| | |
|---|---|
| AUC | 0.69 [0.38,0.88] |
| Sens / Spec | 0.33 / 0.89 |
| Wilcoxon *P* | 0.2721 |
| Early Relapse (pos) | 6 |
| Late Relapse (neg) | 9 |

**Figure 108/2**

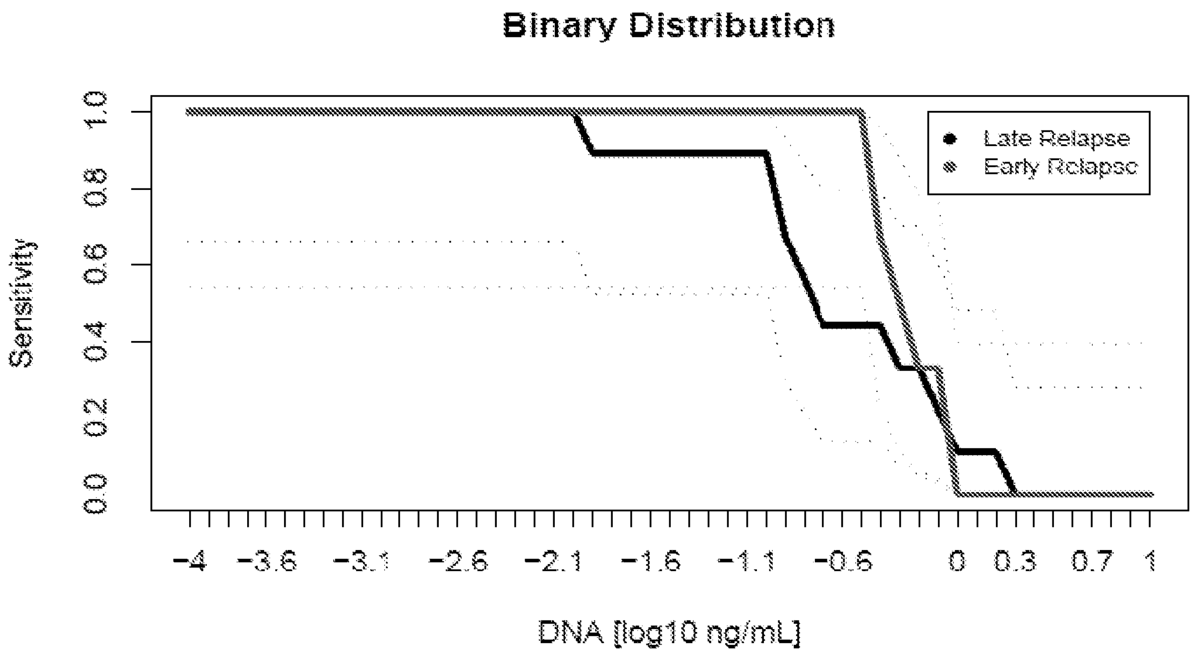
Binary Distribution
Late Relapse
Early Relapse
Sensitivity
1.0
0.8
0.6
0.4
0.2
0.0
-4
-3.6
-3.1
-2.6
-2.1
-1.6
-1.1
-0.6
0
0.3
0.7
1
DNA [log10 ng/mL]

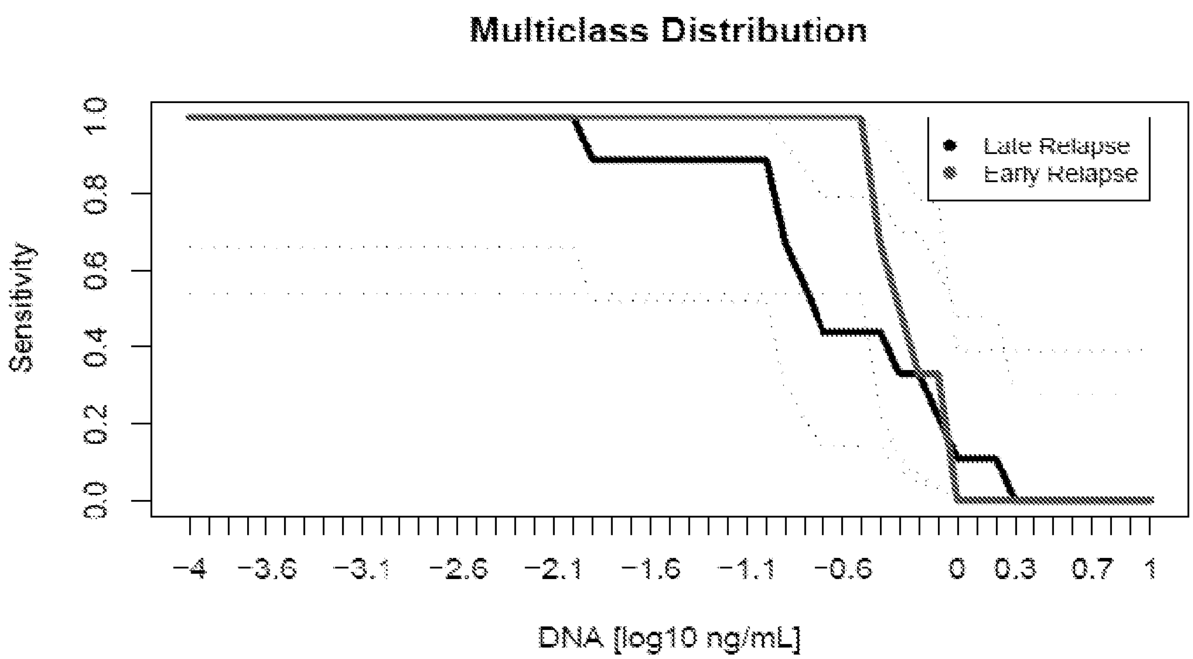
Multiclass Distribution
Late Relapse
Early Relapse
Sensitivity
1.0
0.8
0.6
0.4
0.2
0.0
-4
-3.6
-3.1
-2.6
-2.1
-1.6
-1.1
-0.6
0
0.3
0.7
1
DNA [log10 ng/mL]

Figure 109/1

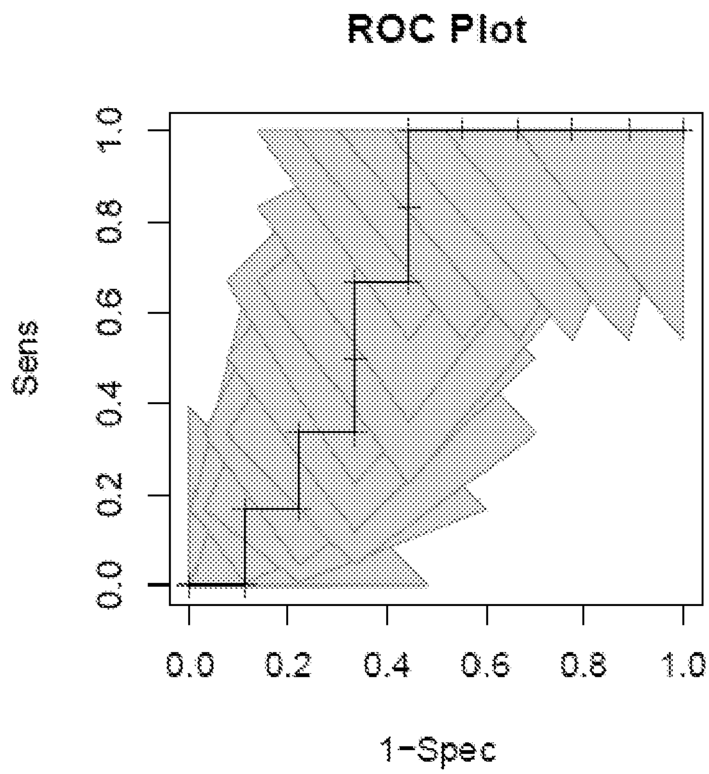


| | |
|---|---|
| AUC | 0.69 [0.38,0.88] |
| Sens / Spec | 0.17 / 0.89 |
| Wilcoxon *P* | 0.2721 |
| Early Relapse (pos) | 6 |
| Late Relapse (neg) | 9 |

**Figure 109/2**

Binary Distribution

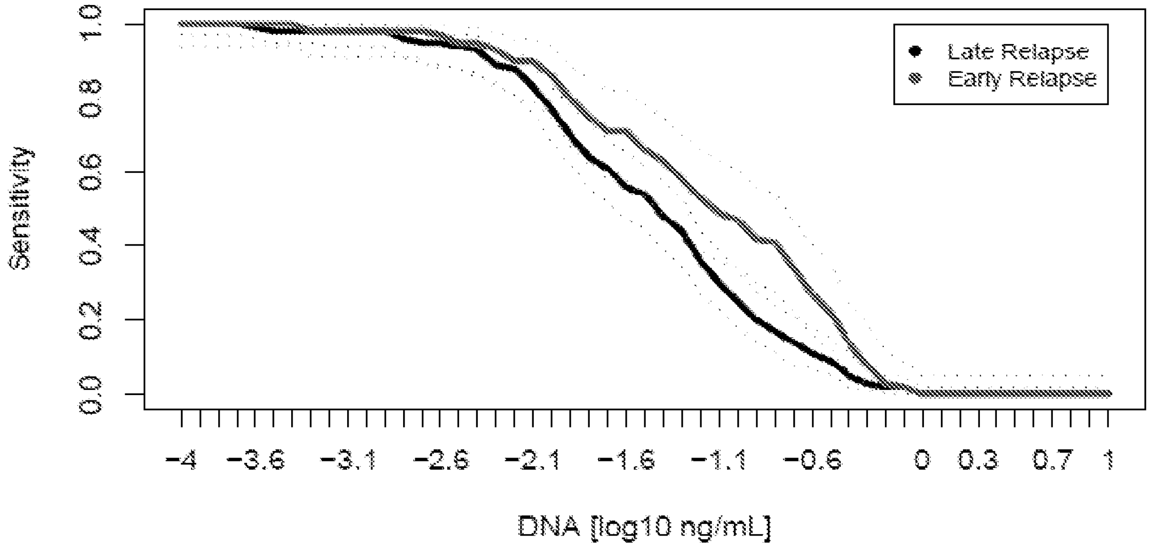


Multiclass Distribution

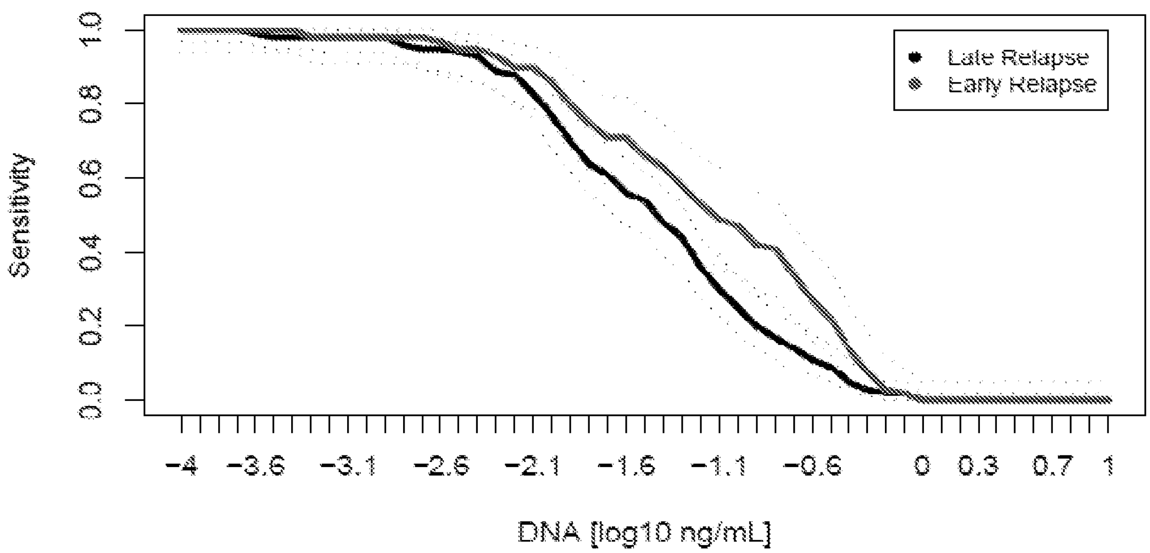


Figure 110/1

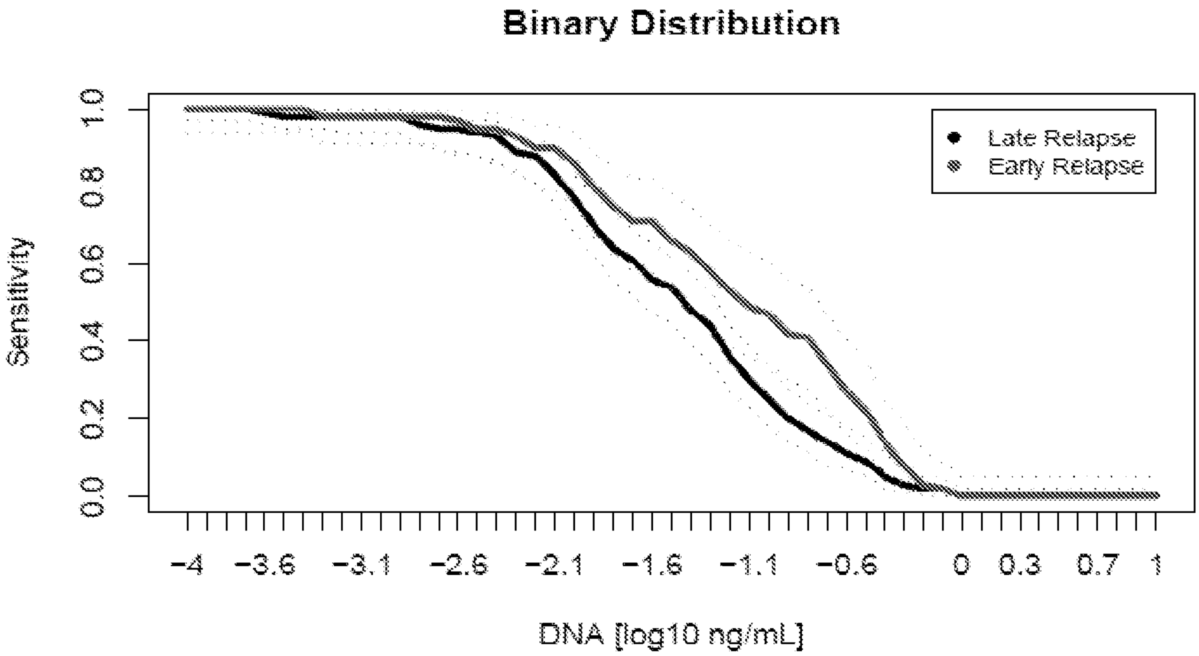
Binary Distribution
Late Relapse
Early Relapse
Sensitivity
0.0
0.2
0.4
0.6
0.8
1.0
-4
-3.6
-3.1
-2.6
-2.1
-1.6
-1.1
-0.6
0
0.3
0.7
1
DNA [log10 ng/mL]

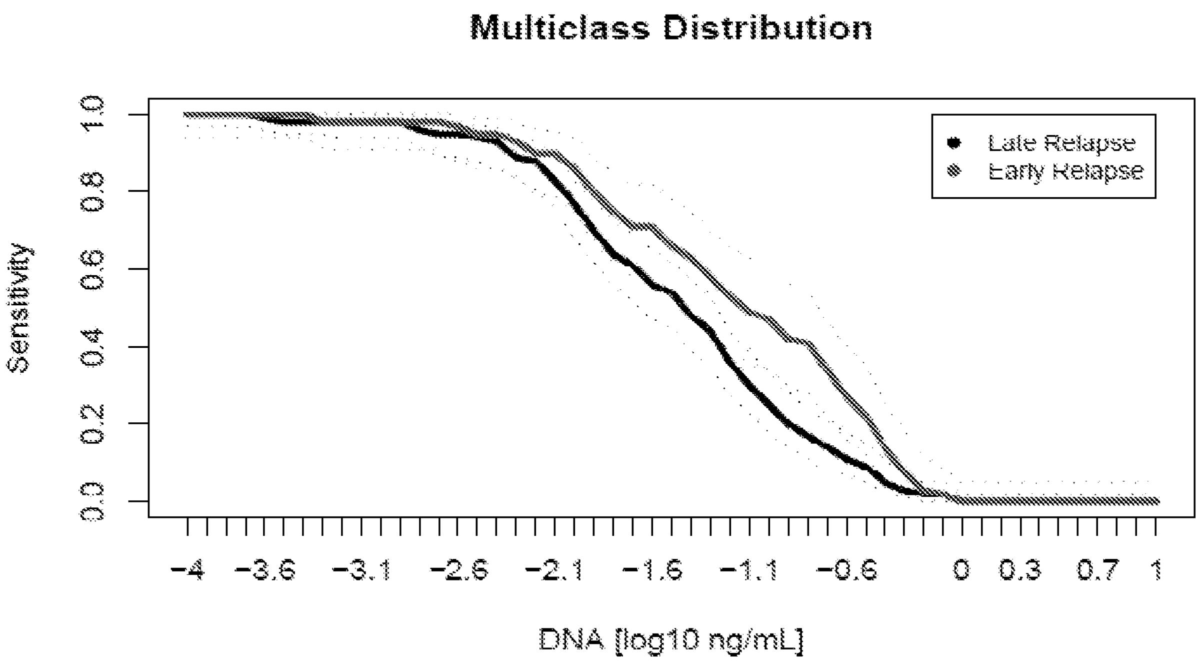
Multiclass Distribution
Late Relapse
Early Relapse
Sensitivity
0.0
0.2
0.4
0.6
0.8
1.0
-4
-3.6
-3.1
-2.6
-2.1
-1.6
-1.1
-0.6
0
0.3
0.7
1
DNA [log10 ng/mL]

Figure 110/1

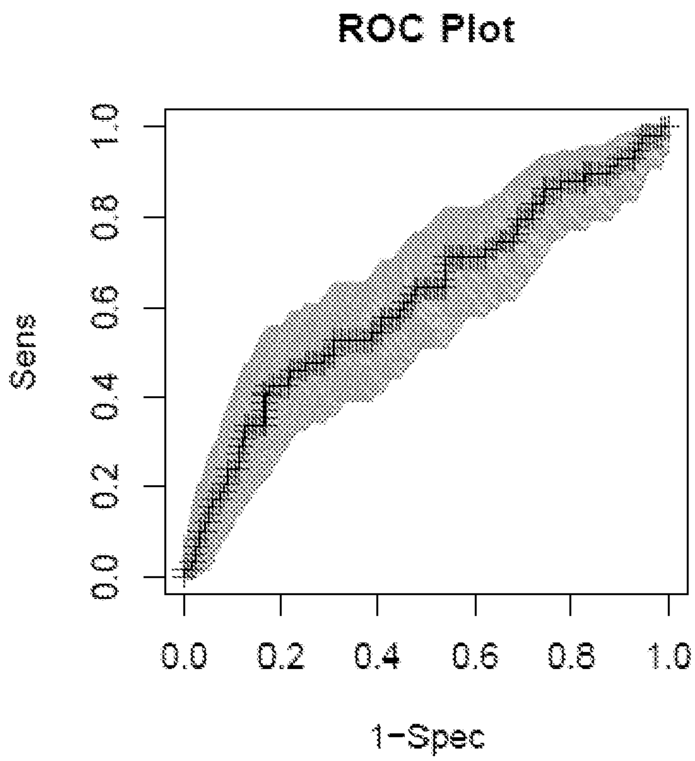


| | |
|---|---|
| AUC | 0.62 [0.54,0.69] |
| Sens / Spec | 0.34 / 0.86 |
| Wilcoxon *P* | 0.0085 |
| Early Relapse (pos) | 59 |
| Late Relapse (neg) | 132 |

Figure 110/2

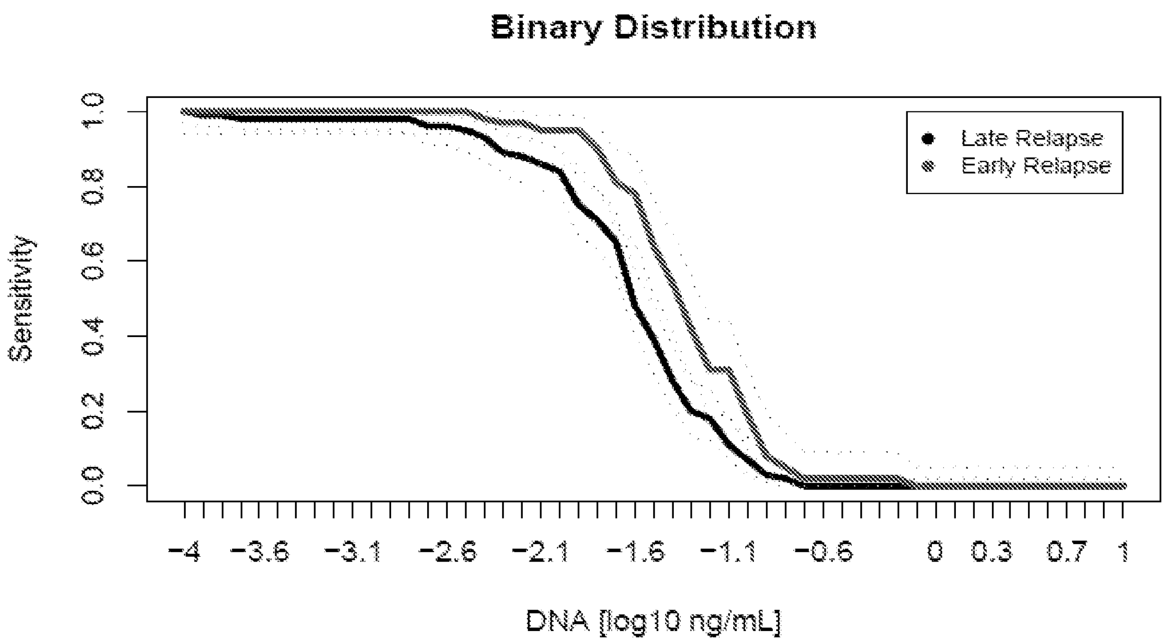
Binary Distribution
Late Relapse
Early Relapse
Sensitivity
0.0
0.2
0.4
0.6
0.8
1.0
-4
-3.6
-3.1
-2.6
-2.1
-1.6
-1.1
-0.6
0
0.3
0.7
1
DNA [log10 ng/mL]

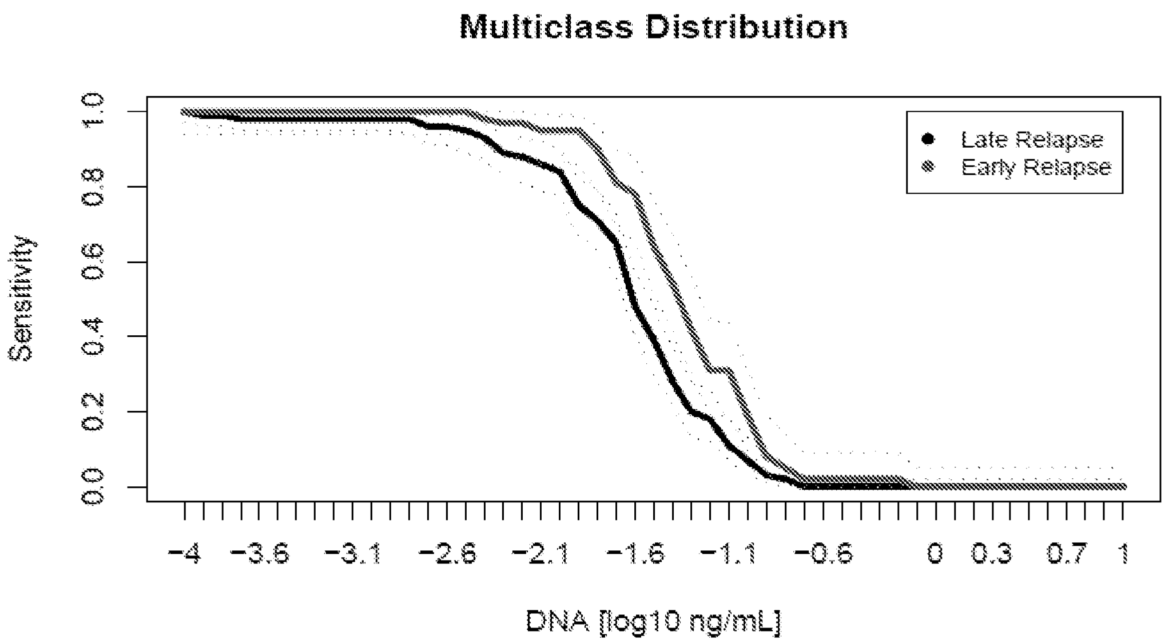
Multiclass Distribution
Late Relapse
Early Relapse
Sensitivity
0.0
0.2
0.4
0.6
0.8
1.0
-4
-3.6
-3.1
-2.6
-2.1
-1.6
-1.1
-0.6
0
0.3
0.7
1
DNA [log10 ng/mL]

Figure 111/1

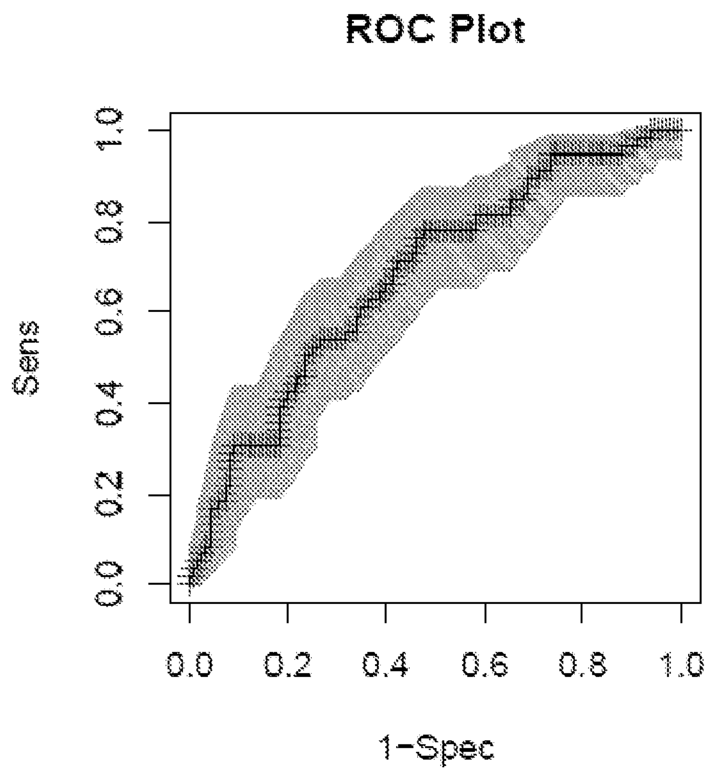


| | |
|---|---|
| AUC | 0.68 [0.61,0.75] |
| Sens / Spec | 0.31 / 0.86 |
| Wilcoxon *P* | 1e-04 |
| Early Relapse (pos) | 59 |
| Late Relapse (neg) | 132 |

**Figure 111/2**

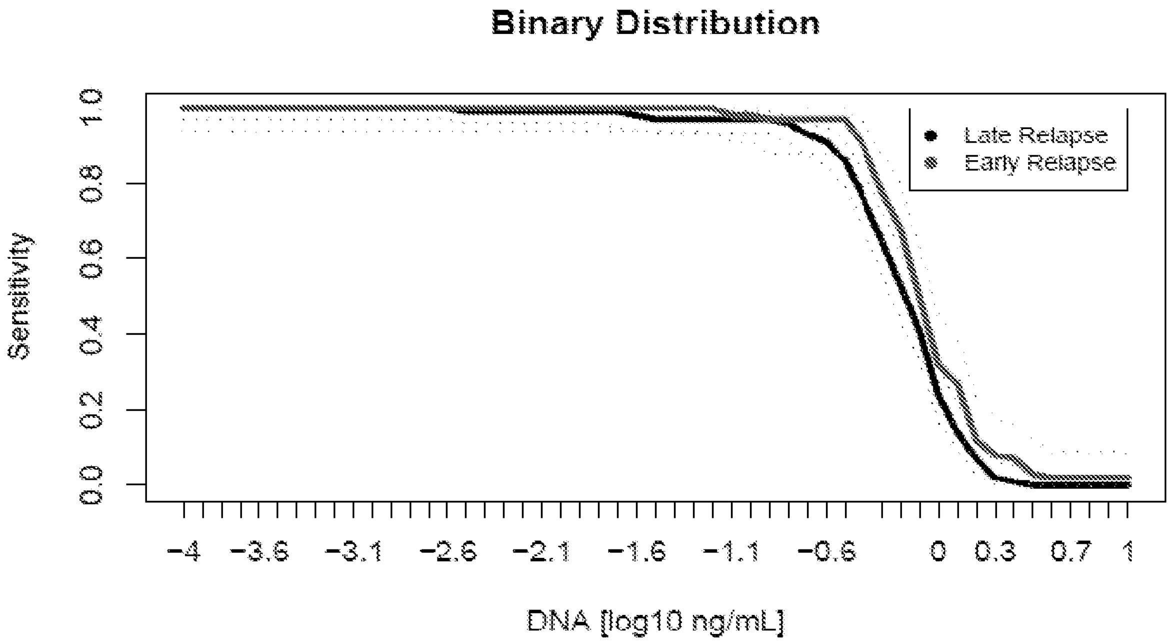
Binary Distribution
Sensitivity
0.0
0.2
0.4
0.6
0.8
1.0
Late Relapse
Early Relapse
-4
-3.6
-3.1
-2.6
-2.1
-1.6
-1.1
-0.6
0
0.3
0.7
1
DNA [log10 ng/mL]

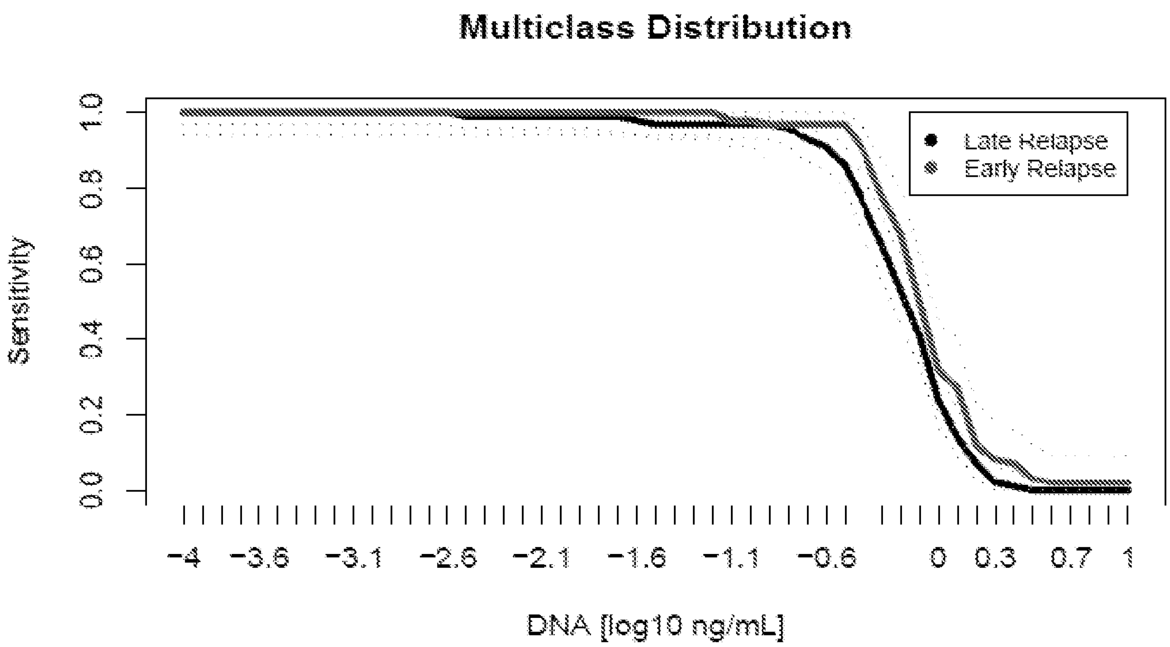
Multiclass Distribution
Sensitivity
0.0
0.2
0.4
0.6
0.8
1.0
Late Relapse
Early Relapse
-4
-3.6
-3.1
-2.6
-2.1
-1.6
-1.1
-0.6
0
0.3
0.7
1
DNA [log10 ng/mL]

Figure 112/1

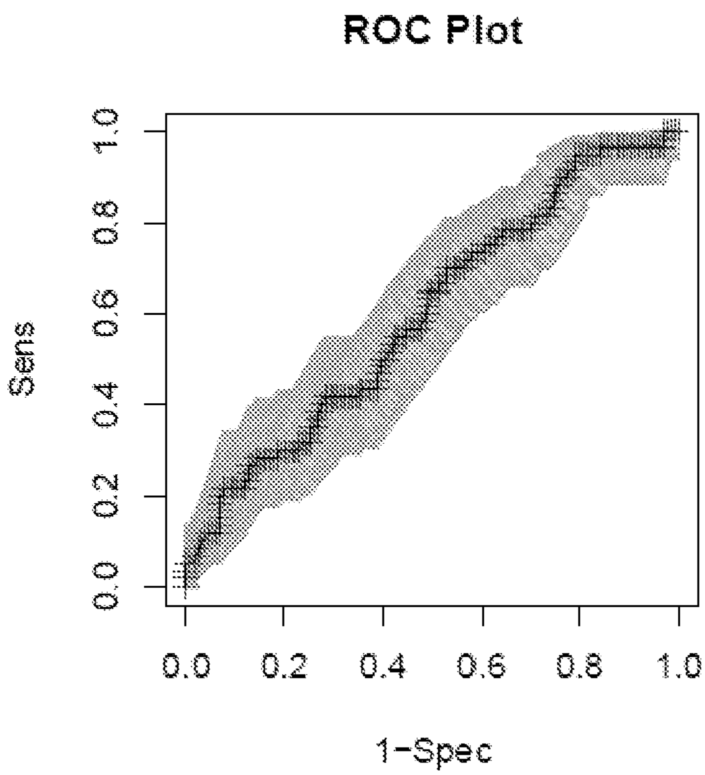


| | |
|---|---|
| AUC | 0.6 [0.53,0.67] |
| Sens / Spec | 0.28 / 0.85 |
| Wilcoxon *P* | 0.0264 |
| Early Relapse (pos) | 60 |
| Late Relapse (neg) | 134 |

**Figure 112/2**

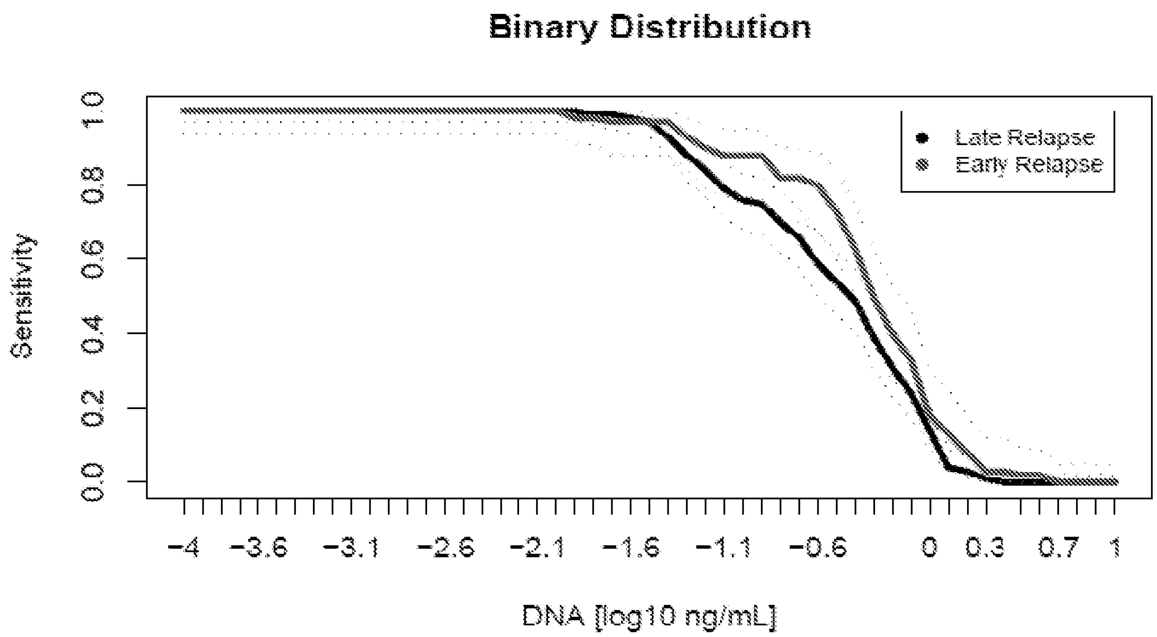
Binary Distribution
Sensitivity
1.0
0.8
0.6
0.4
0.2
0.0
Late Relapse
Early Relapse
-4
-3.6
-3.1
-2.6
-2.1
-1.6
-1.1
-0.6
0
0.3
0.7
1
DNA [log10 ng/mL]

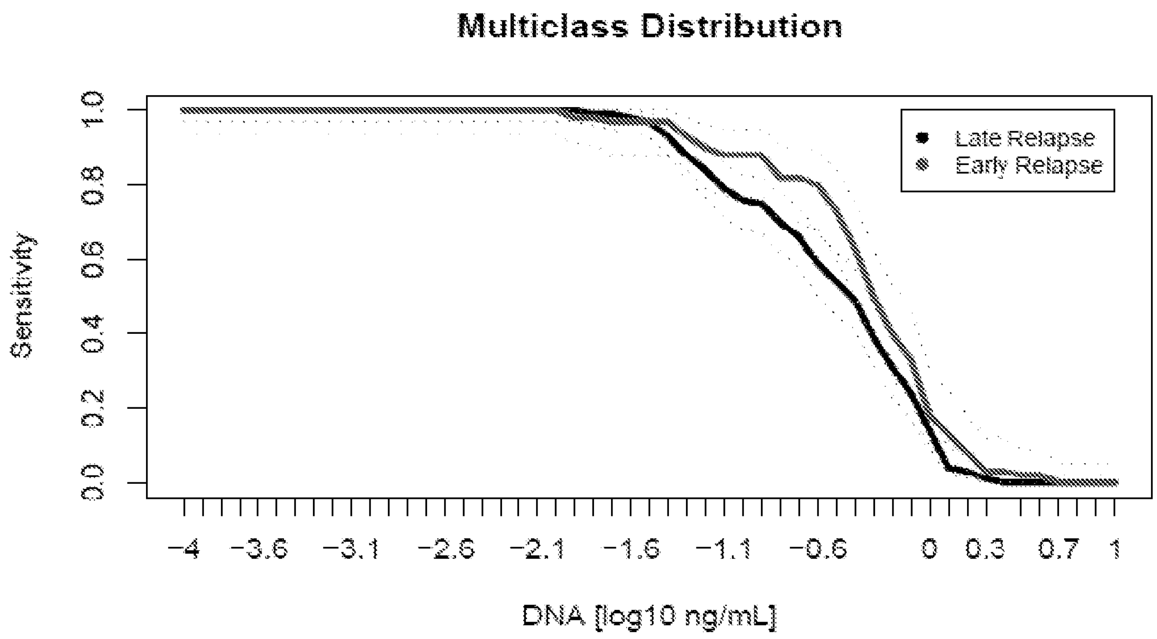
Multiclass Distribution
Sensitivity
1.0
0.8
0.6
0.4
0.2
0.0
Late Relapse
Early Relapse
-4
-3.6
-3.1
-2.6
-2.1
-1.6
-1.1
-0.6
0
0.3
0.7
1
DNA [log10 ng/mL]

Figure 113/1

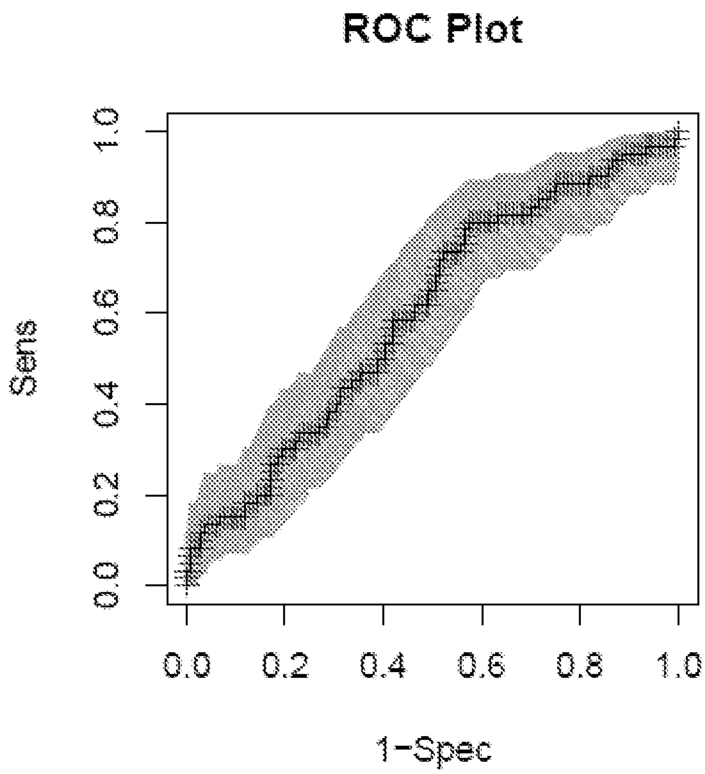


| | |
|---|---|
| AUC | 0.6 [0.53,0.67] |
| Sens / Spec | 0.2 / 0.85 |
| Wilcoxon *P* | 0.0272 |
| Early Relapse (pos) | 60 |
| Late Relapse (neg) | 134 |

**Figure 113/2**

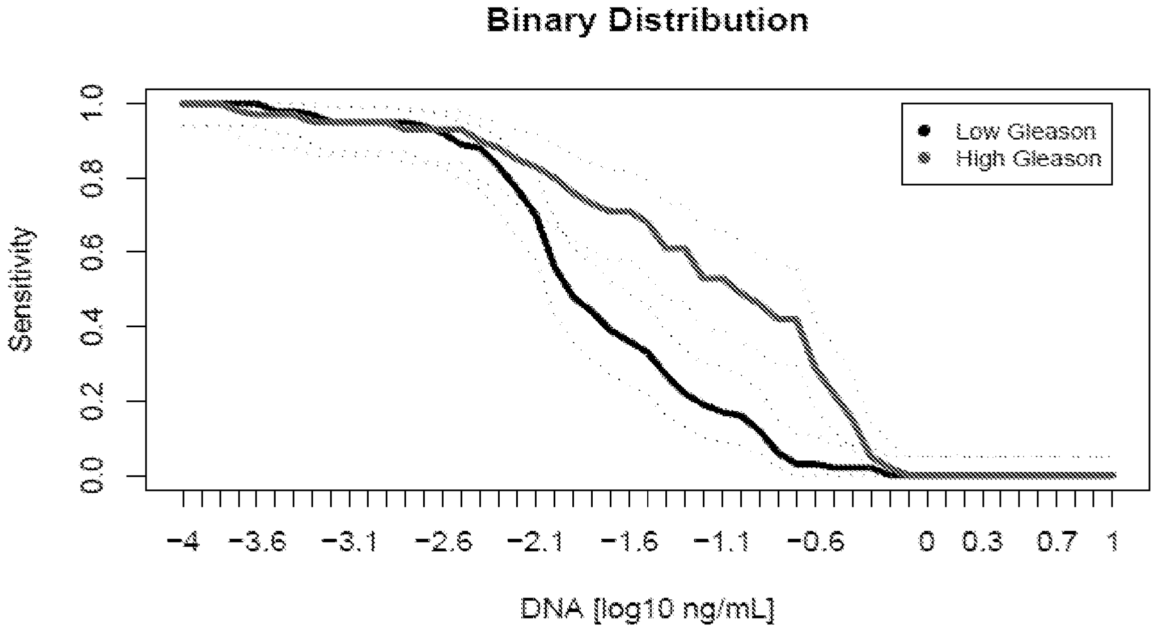
Binary Distribution
Low Gleason
High Gleason
Sensitivity
0.0
0.2
0.4
0.6
0.8
1.0
-4
-3.6
-3.1
-2.6
-2.1
-1.6
-1.1
-0.6
0
0.3
0.7
1
DNA [log10 ng/mL]

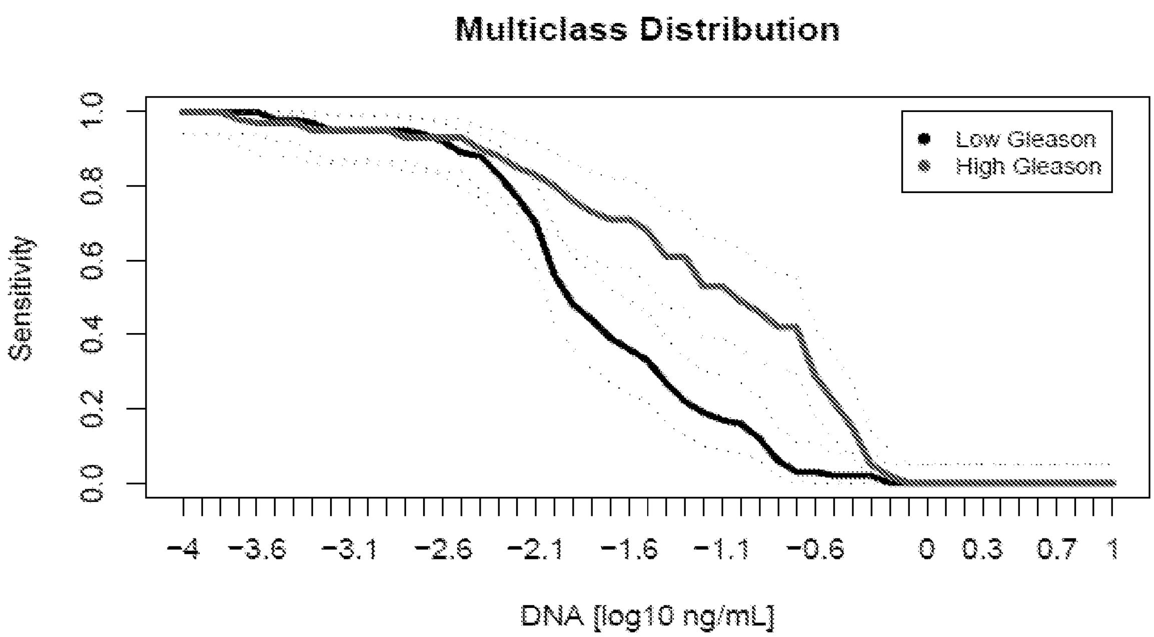
Multiclass Distribution
Low Gleason
High Gleason
Sensitivity
0.0
0.2
0.4
0.6
0.8
1.0
-4
-3.6
-3.1
-2.6
-2.1
-1.6
-1.1
-0.6
0
0.3
0.7
1
DNA [log10 ng/mL]

Figure 114/1

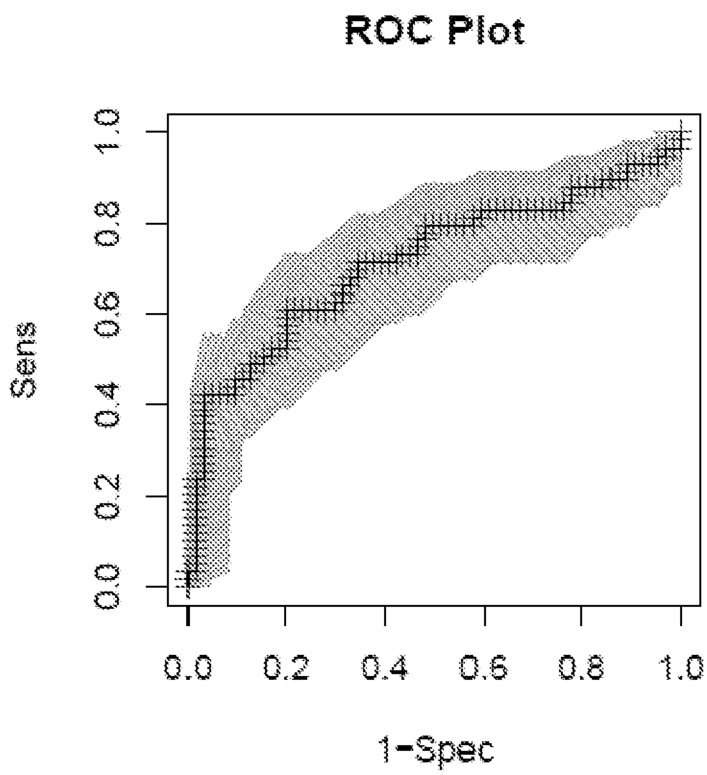


| | |
|---|---|
| AUC | 0.72 [0.63,0.79] |
| Sens / Spec | 0.49 / 0.86 |
| Wilcoxon *P* | 0 |
| High Gleason (pos) | 59 |
| Low Gleason (neg) | 64 |

**Figure 114/2**

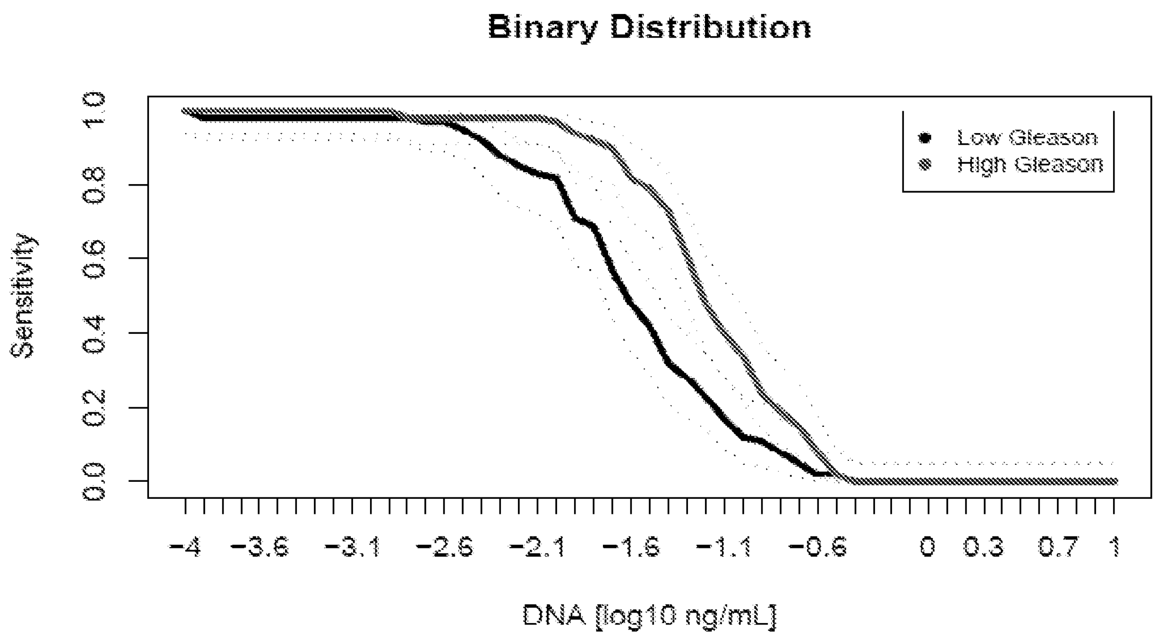
Binary Distribution
Low Gleason
High Gleason
Sensitivity
0.0
0.2
0.4
0.6
0.8
1.0
-4
-3.6
-3.1
-2.6
-2.1
-1.6
-1.1
-0.6
0
0.3
0.7
1
DNA [log10 ng/mL]

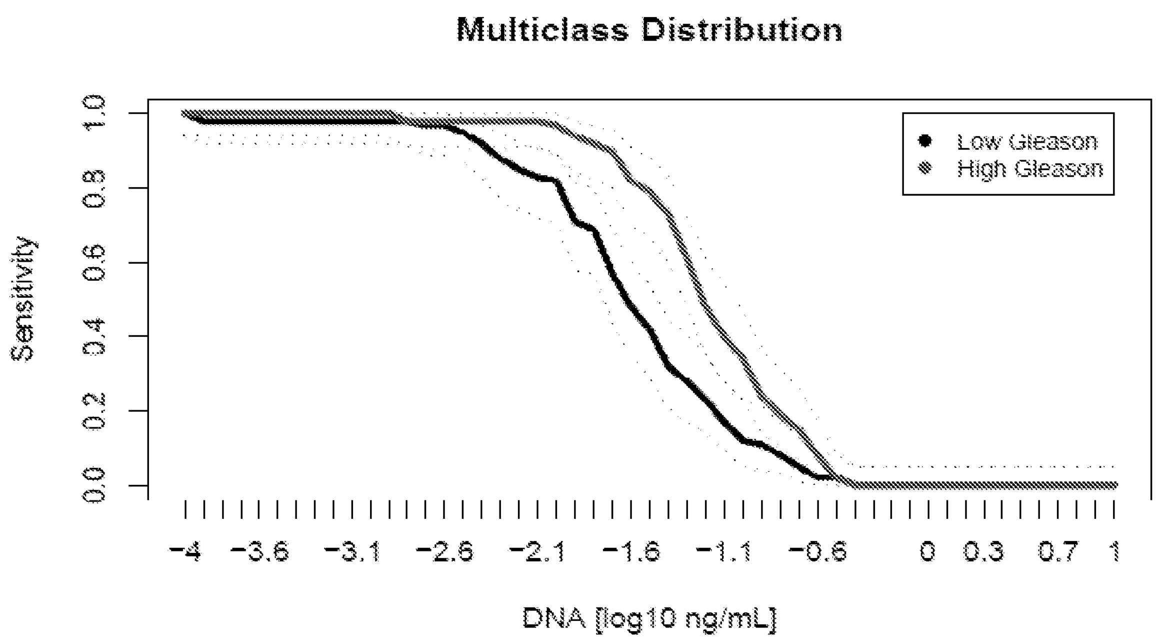
Multiclass Distribution
Low Gleason
High Gleason
Sensitivity
0.0
0.2
0.4
0.6
0.8
1.0
-4
-3.6
-3.1
-2.6
-2.1
-1.6
-1.1
-0.6
0
0.3
0.7
1
DNA [log10 ng/mL]

Figure 115/1

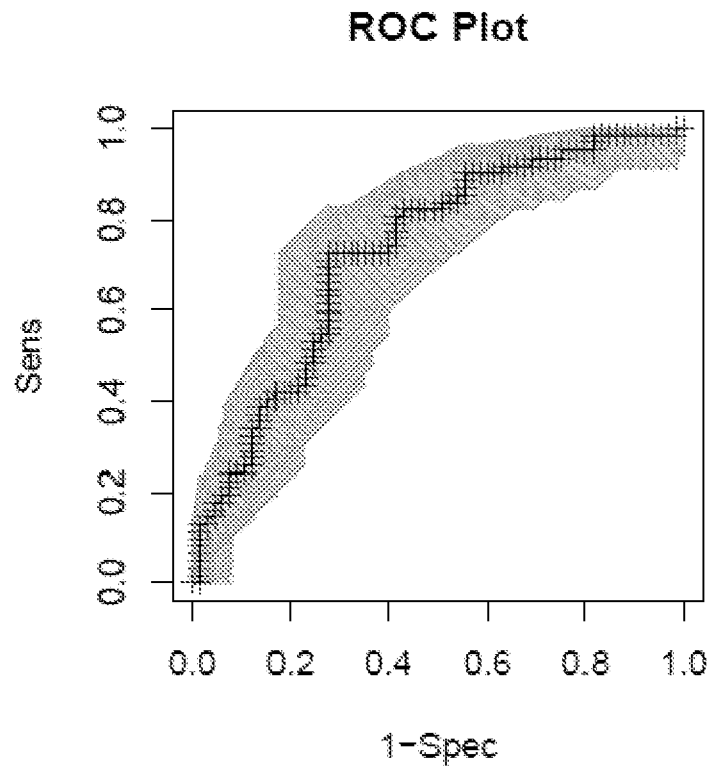


| | |
|---|---|
| AUC | 0.73 [0.64,0.8] |
| Sens / Spec | 0.39 / 0.86 |
| Wilcoxon *P* | 0 |
| High Gleason (pos) | 62 |
| Low Gleason (neg) | 65 |

**Figure 115/2**

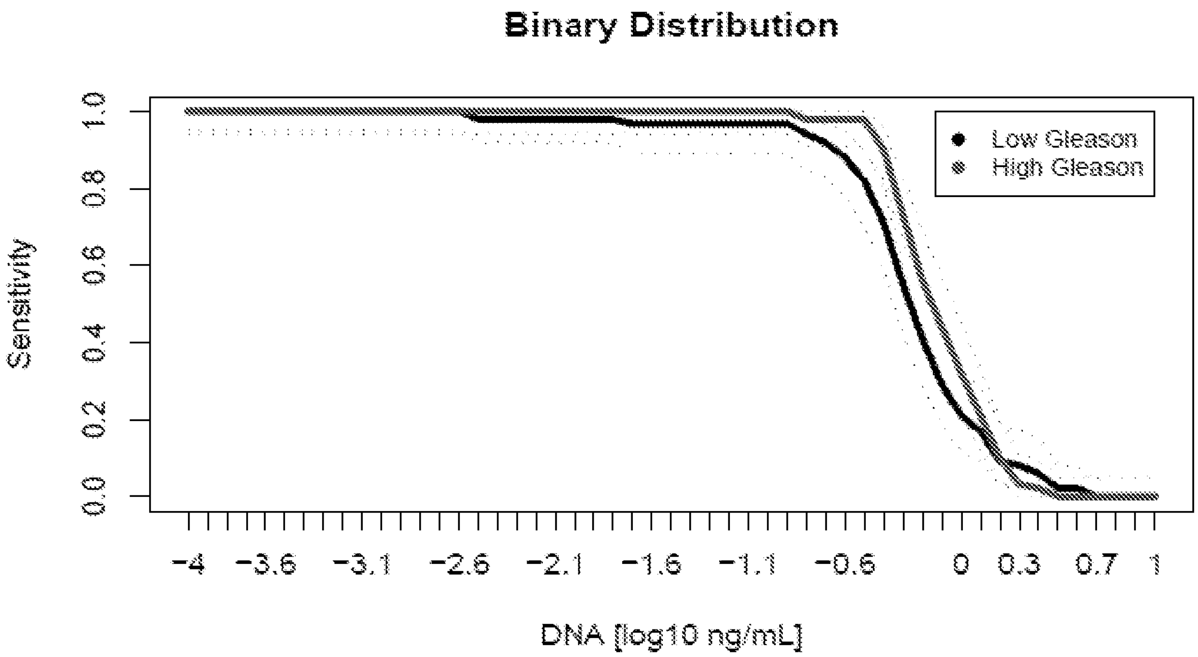
Binary Distribution
Low Gleason
High Gleason
Sensitivity
0.0
0.2
0.4
0.6
0.8
1.0
-4
-3.6
-3.1
-2.6
-2.1
-1.6
-1.1
-0.6
0
0.3
0.7
1
DNA [log10 ng/mL]

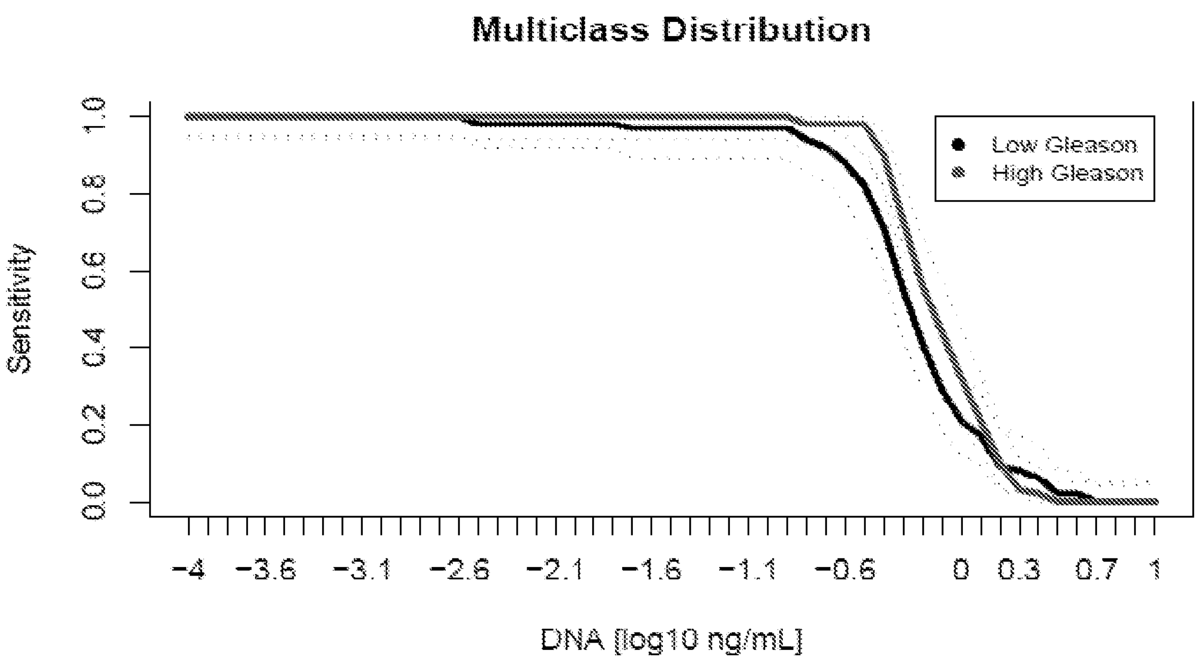
Multiclass Distribution
Low Gleason
High Gleason
Sensitivity
0.0
0.2
0.4
0.6
0.8
1.0
-4
-3.6
-3.1
-2.6
-2.1
-1.6
-1.1
-0.6
0
0.3
0.7
1
DNA [log10 ng/mL]

Figure 116/1

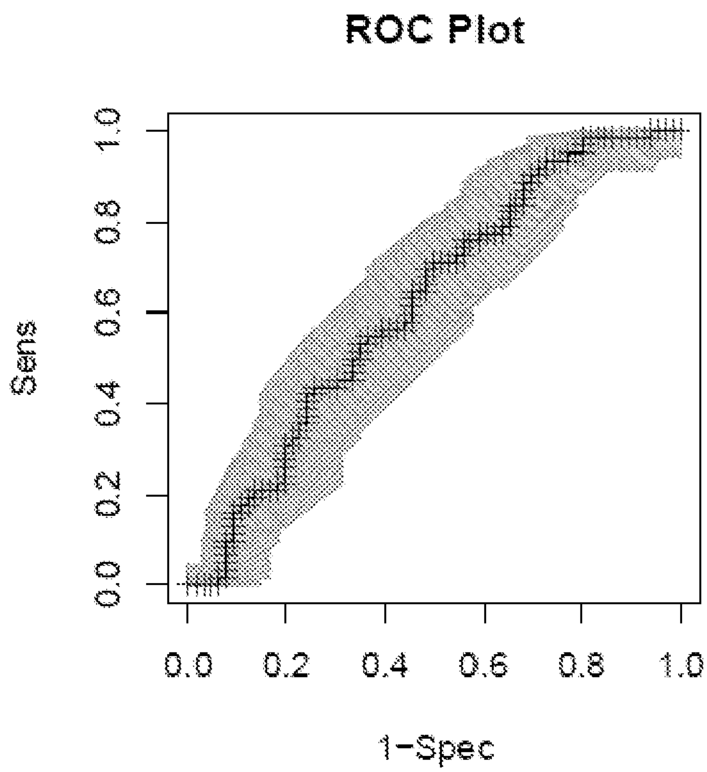


| | |
|---|---|
| AUC | 0.62 [0.53,0.7] |
| Sens / Spec | 0.21 / 0.86 |
| Wilcoxon *P* | 0.0191 |
| High Gleason (pos) | 62 |
| Low Gleason (neg) | 66 |

**Figure 116/2**

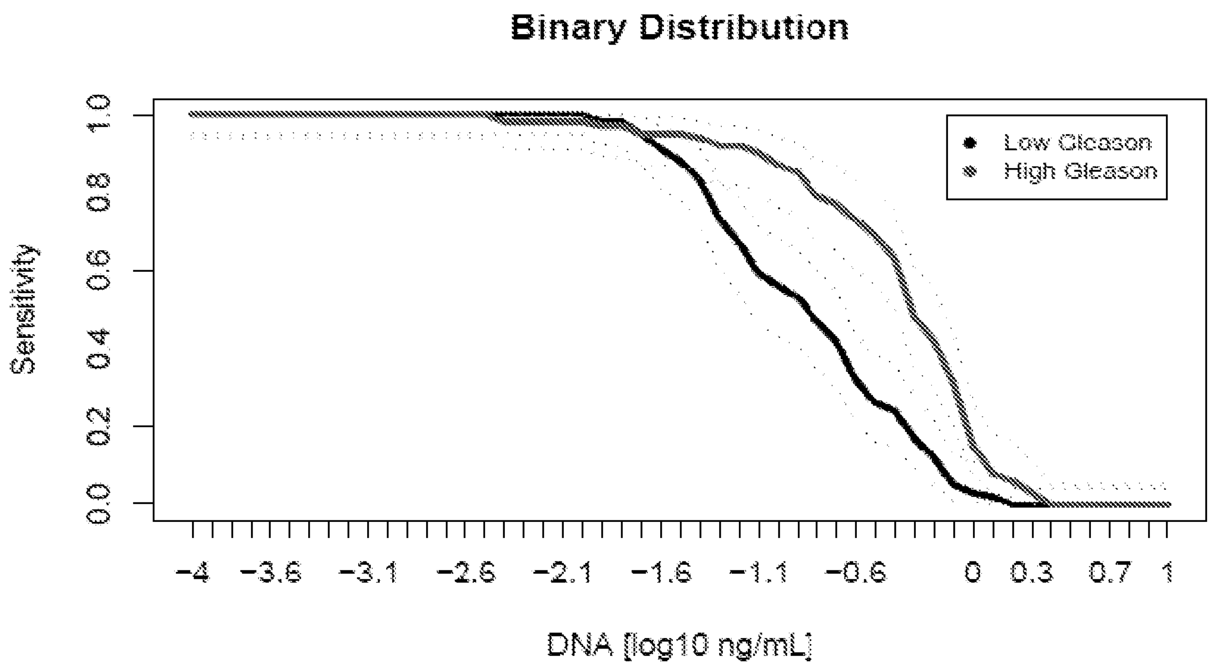
Binary Distribution
Low Gleason
High Gleason
Sensitivity
0.0
0.2
0.4
0.6
0.8
1.0
-4
-3.6
-3.1
-2.6
-2.1
-1.6
-1.1
-0.6
0
0.3
0.7
1
DNA [log10 ng/mL]

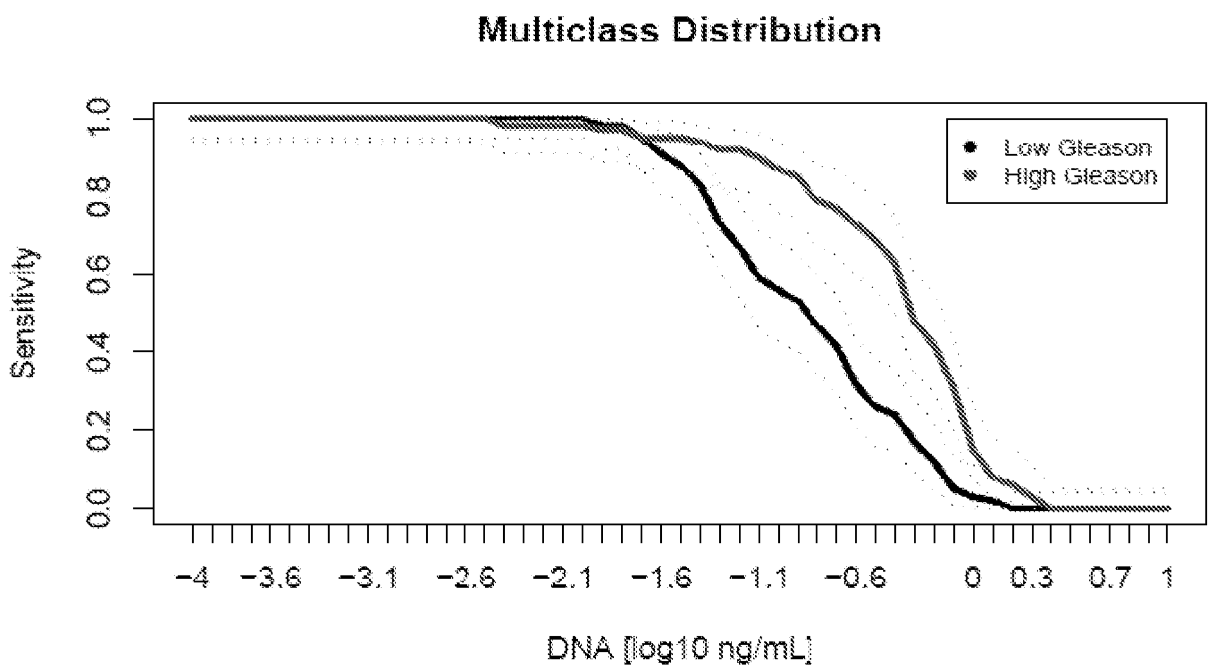
Multiclass Distribution
Low Gleason
High Gleason
Sensitivity
0.0
0.2
0.4
0.6
0.8
1.0
-4
-3.6
-3.1
-2.6
-2.1
-1.6
-1.1
-0.6
0
0.3
0.7
1
DNA [log10 ng/mL]

Figure 117/1

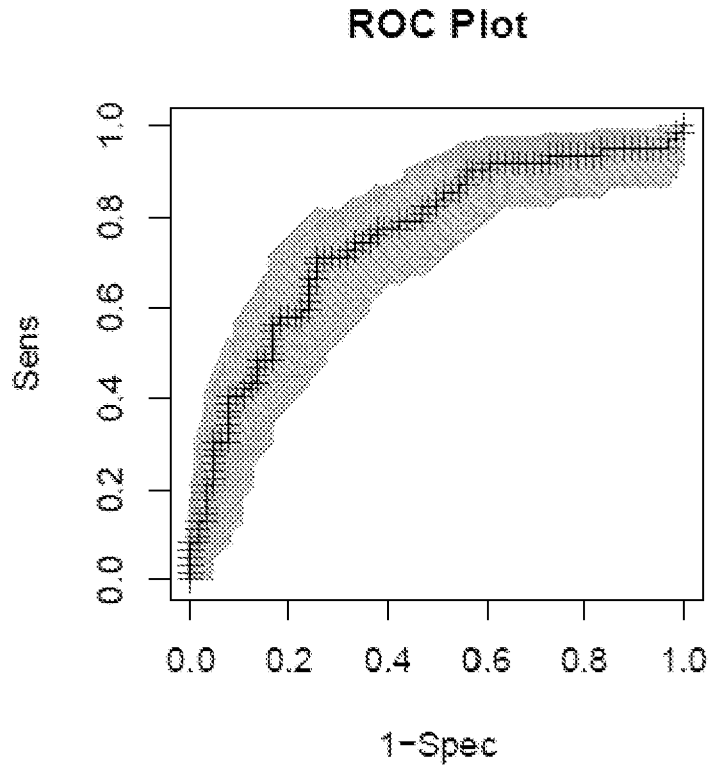


| | |
|---|---|
| AUC | 0.76 [0.67,0.83] |
| Sens / Spec | 0.48 / 0.86 |
| Wilcoxon *P* | 0 |
| High Gleason (pos) | 62 |
| Low Gleason (neg) | 66 |

**Figure 117/2**

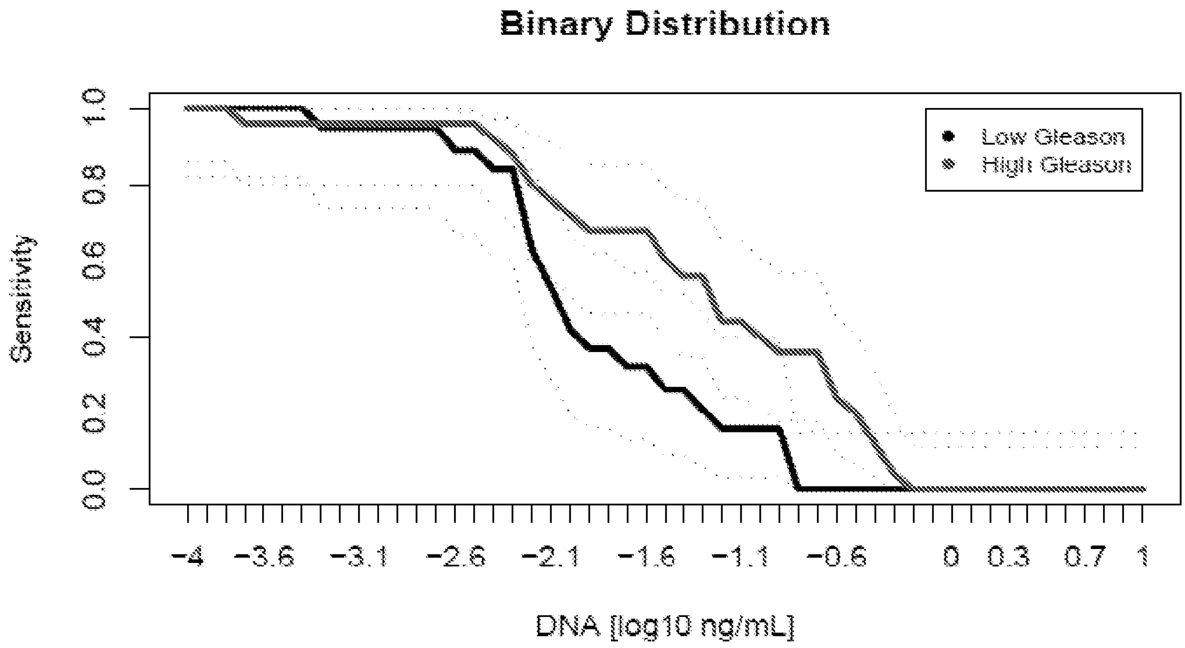
Binary Distribution
Sensitivity
0.0
0.2
0.4
0.6
0.8
1.0
Low Gleason
High Gleason
-4
-3.6
-3.1
-2.6
-2.1
-1.6
-1.1
-0.6
0
0.3
0.7
1
DNA [log10 ng/mL]

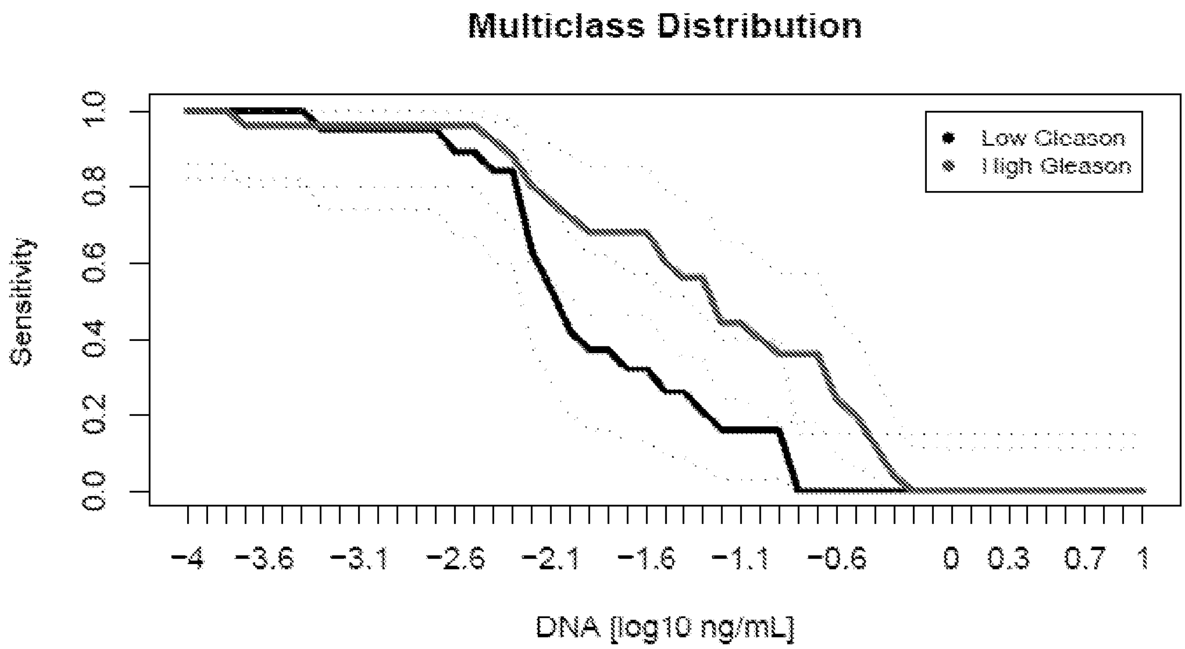
Multiclass Distribution
Sensitivity
0.0
0.2
0.4
0.6
0.8
1.0
Low Gleason
High Gleason
-4
-3.6
-3.1
-2.6
-2.1
-1.6
-1.1
-0.6
0
0.3
0.7
1
DNA [log10 ng/mL]

Figure 118/1

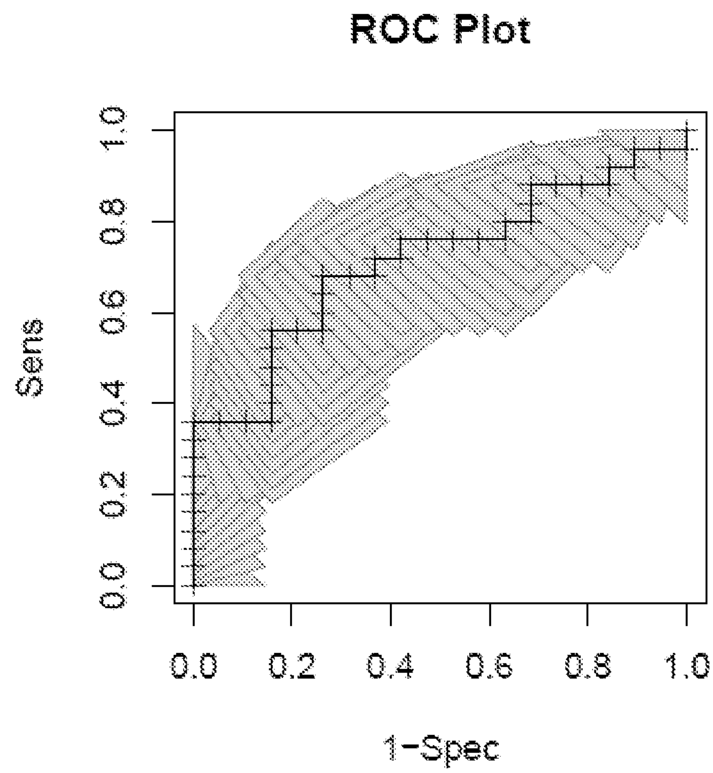


| | |
|---|---|
| AUC | 0.72 [0.55,0.83] |
| Sens / Spec | 0.36 / 0.89 |
| Wilcoxon *P* | 0.0146 |
| High Gleason (pos) | 25 |
| Low Gleason (neg) | 19 |

**Figure 118/2**

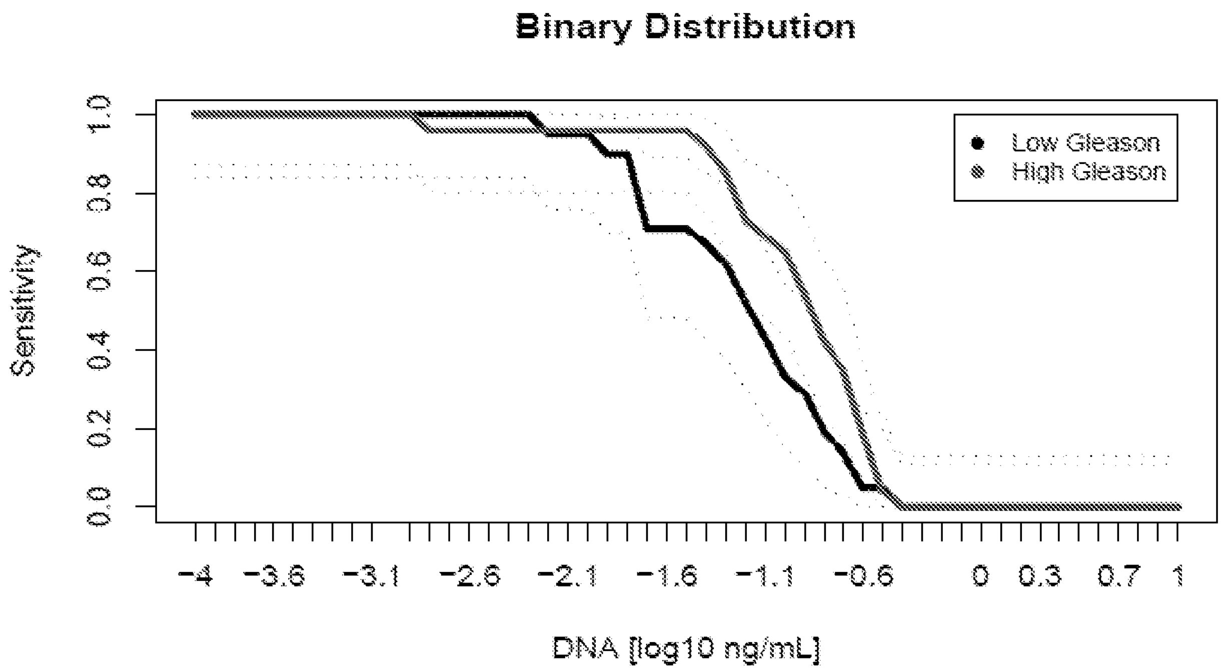
Binary Distribution
Low Gleason
High Gleason
Sensitivity
0.0
0.2
0.4
0.6
0.8
1.0
-4
-3.6
-3.1
-2.6
-2.1
-1.6
-1.1
-0.6
0
0.3
0.7
1
DNA [log10 ng/mL]

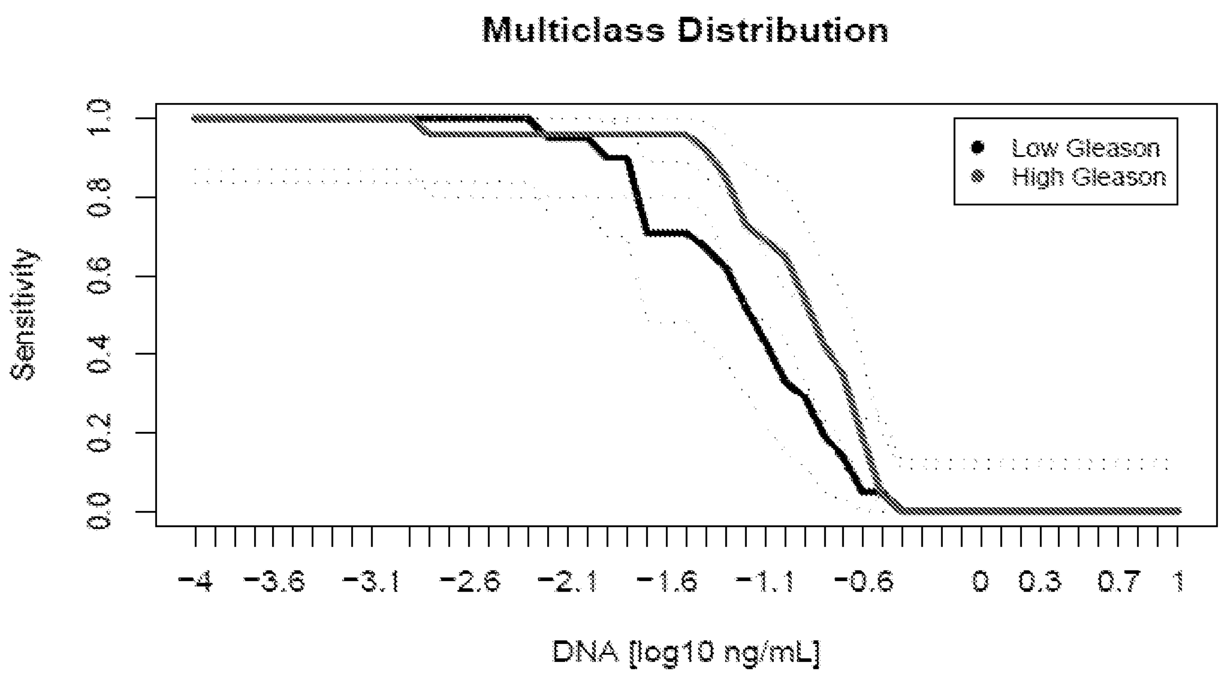
Multiclass Distribution
Low Gleason
High Gleason
Sensitivity
0.0
0.2
0.4
0.6
0.8
1.0
-4
-3.6
-3.1
-2.6
-2.1
-1.6
-1.1
-0.6
0
0.3
0.7
1
DNA [log10 ng/mL]

Figure 119/1

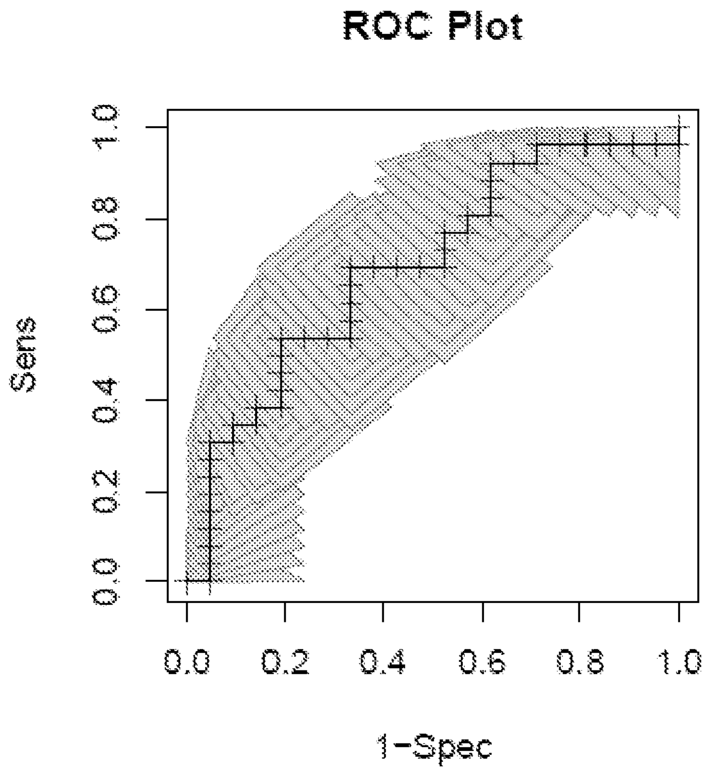


| | |
|---|---|
| AUC | 0.7 [0.55,0.83] |
| Sens / Spec | 0.38 / 0.86 |
| Wilcoxon *P* | 0.0217 |
| High Gleason (pos) | 26 |
| Low Gleason (neg) | 21 |

**Figure 119/2**

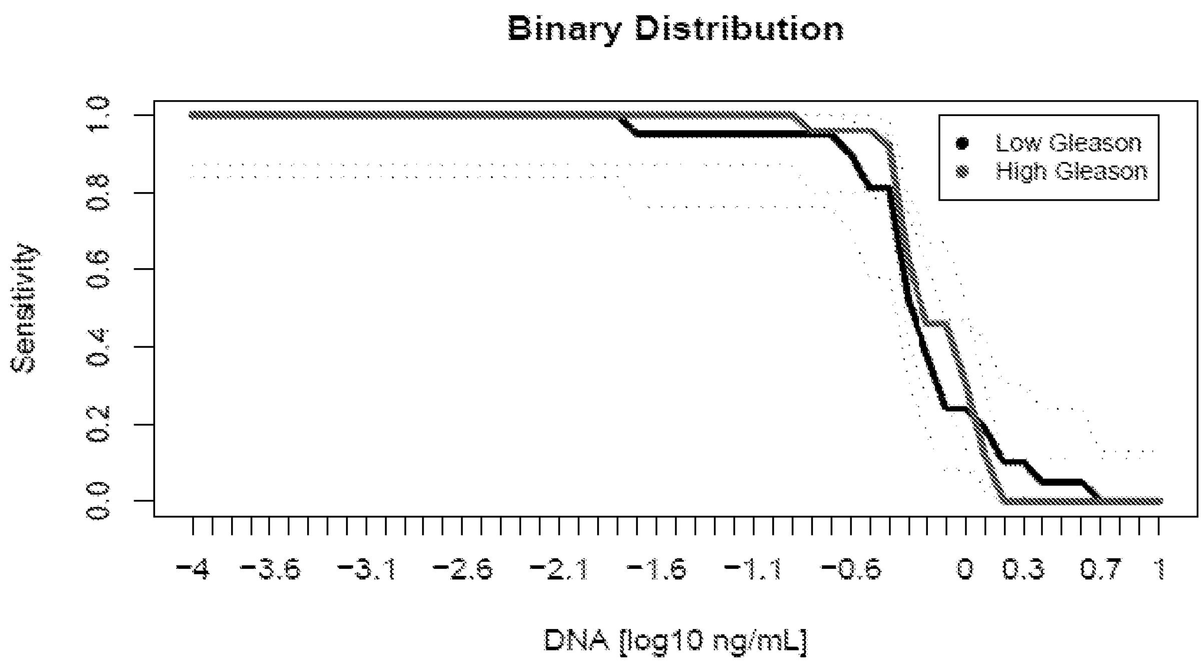
Binary Distribution
Low Gleason
High Gleason
Sensitivity
0.0
0.2
0.4
0.6
0.8
1.0
-4
-3.6
-3.1
-2.6
-2.1
-1.6
-1.1
-0.6
0
0.3
0.7
1
DNA [log10 ng/mL]

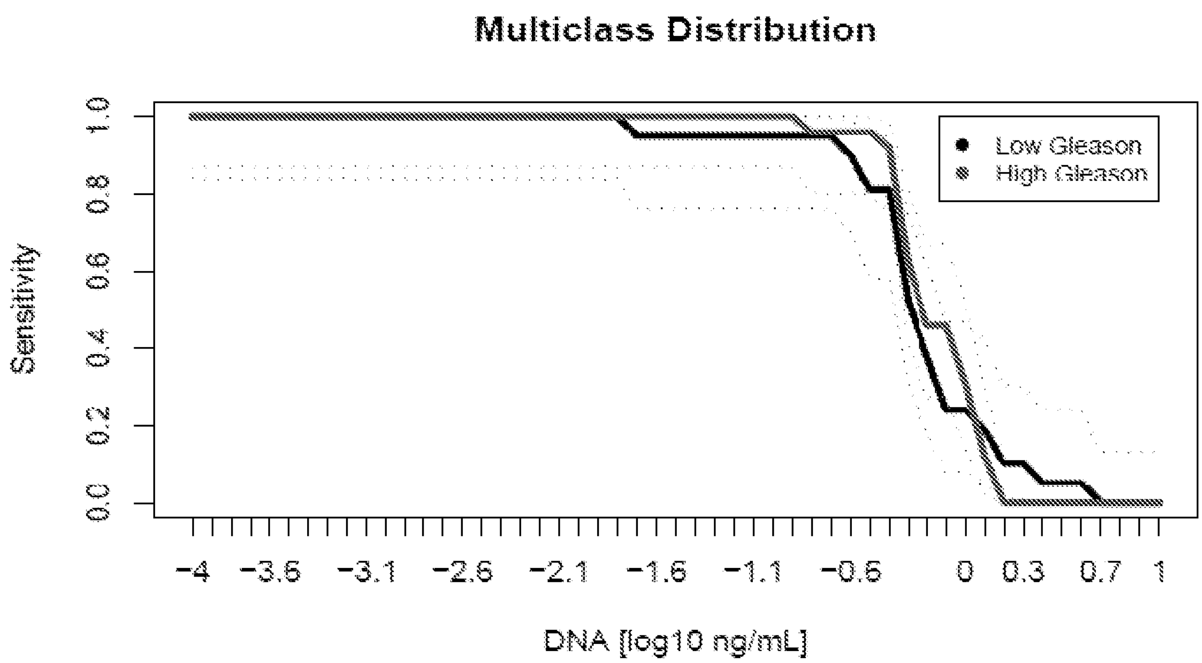
Multiclass Distribution
Low Gleason
High Gleason
Sensitivity
0.0
0.2
0.4
0.6
0.8
1.0
-4
-3.6
-3.1
-2.6
-2.1
-1.6
-1.1
-0.6
0
0.3
0.7
1
DNA [log10 ng/mL]

Figure 120/1

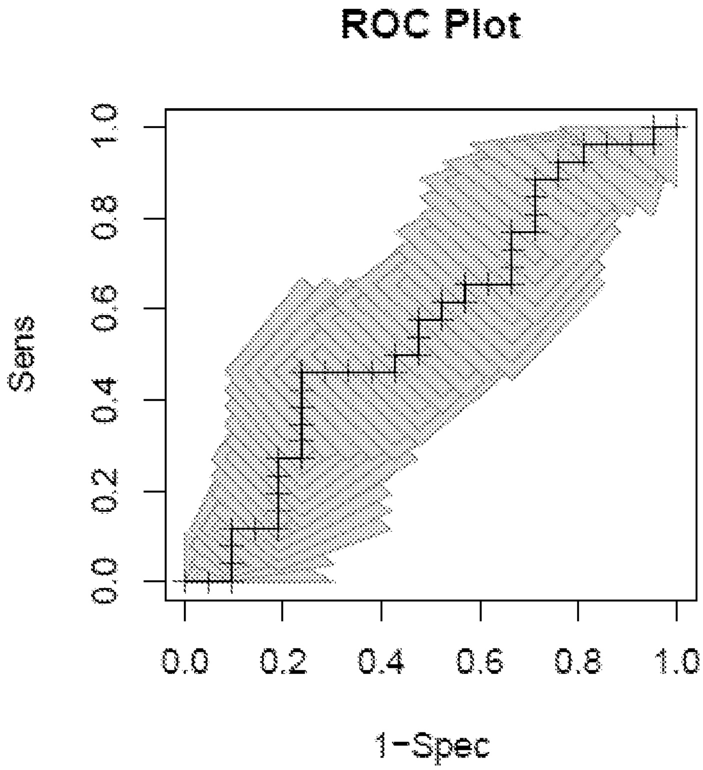


| | |
|---|---|
| AUC | 0.56 [0.4,0.7] |
| Sens / Spec | 0.12 / 0.86 |
| Wilcoxon *P* | 0.4771 |
| High Gleason (pos) | 26 |
| Low Gleason (neg) | 21 |

**Figure 120/2**

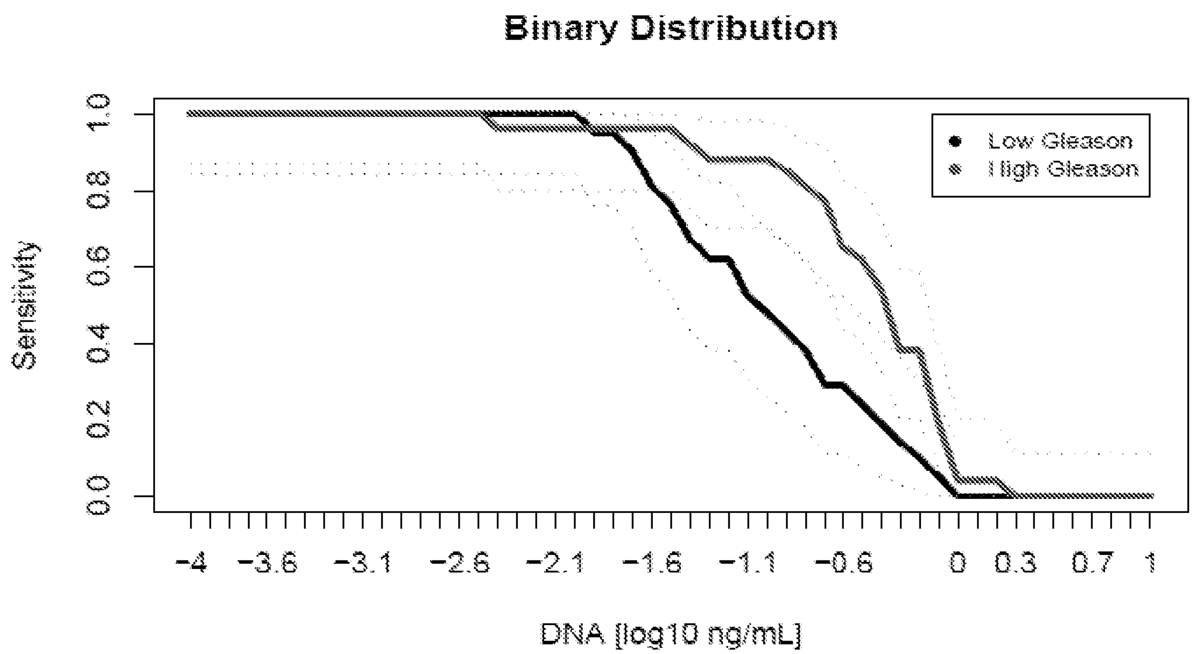
Binary Distribution
Low Gleason
High Gleason
Sensitivity
0.0
0.2
0.4
0.6
0.8
1.0
-4
-3.6
-3.1
-2.6
-2.1
-1.6
-1.1
-0.6
0
0.3
0.7
1
DNA [log10 ng/mL]

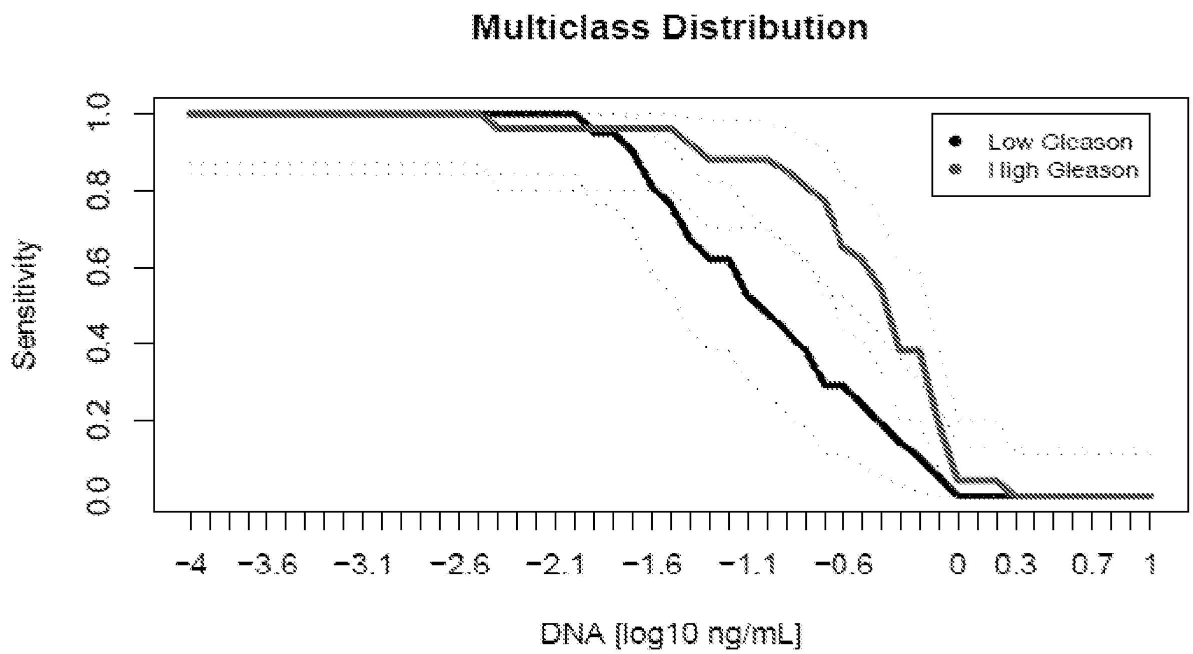
Multiclass Distribution
Low Gleason
High Gleason
Sensitivity
0.0
0.2
0.4
0.6
0.8
1.0
-4
-3.6
-3.1
-2.6
-2.1
-1.6
-1.1
-0.6
0
0.3
0.7
1
DNA [log10 ng/mL]

Figure 121/1

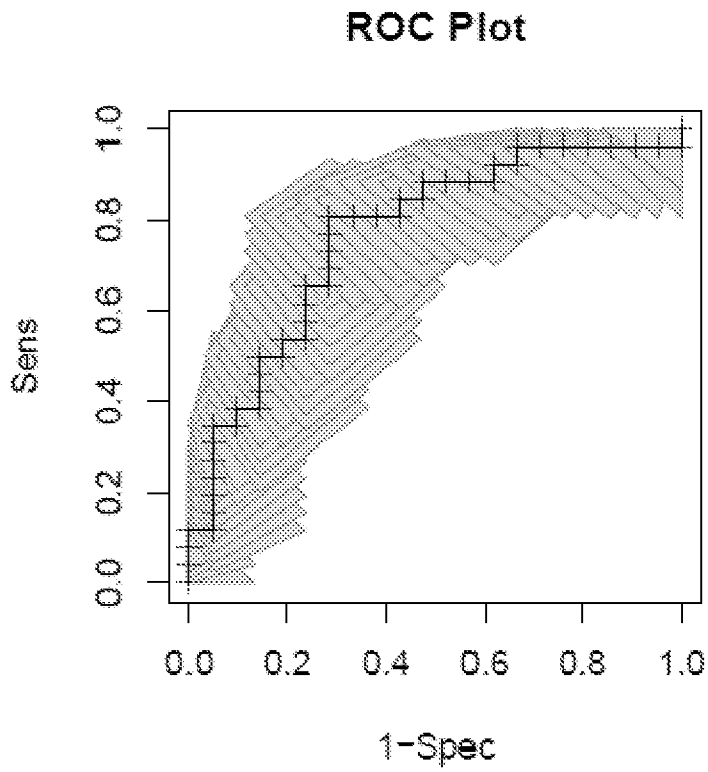


| | |
|---|---|
| AUC | 0.77 [0.62,0.88] |
| Sens / Spec | 0.5 / 0.86 |
| Wilcoxon *P* | 0.0014 |
| High Gleason (pos) | 26 |
| Low Gleason (neg) | 21 |

**Figure 121/2**

**Binary Distribution**

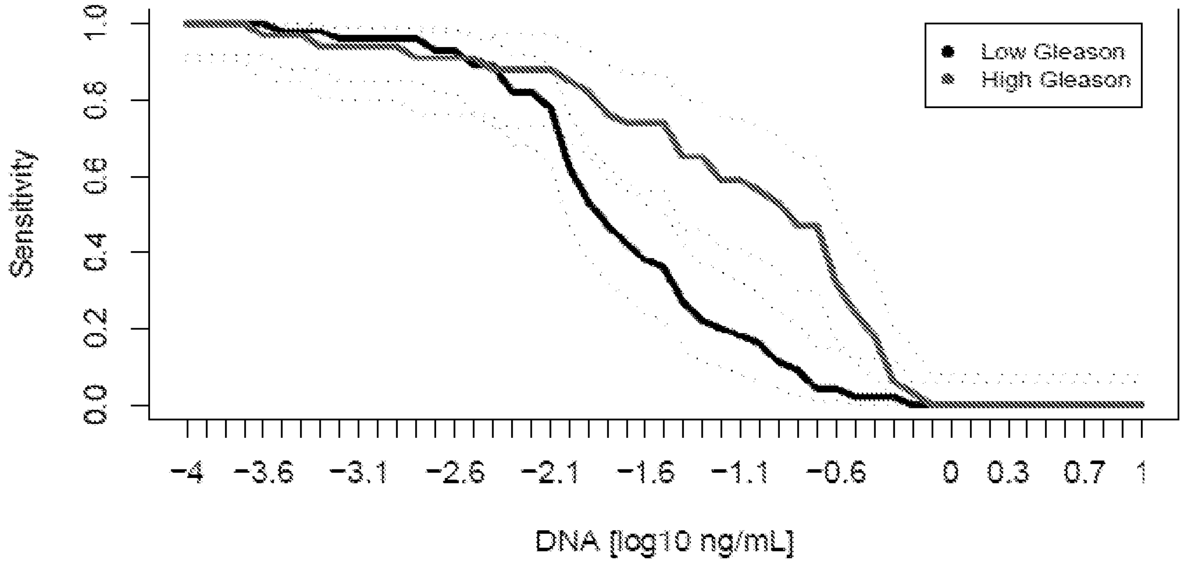


**Multiclass Distribution**

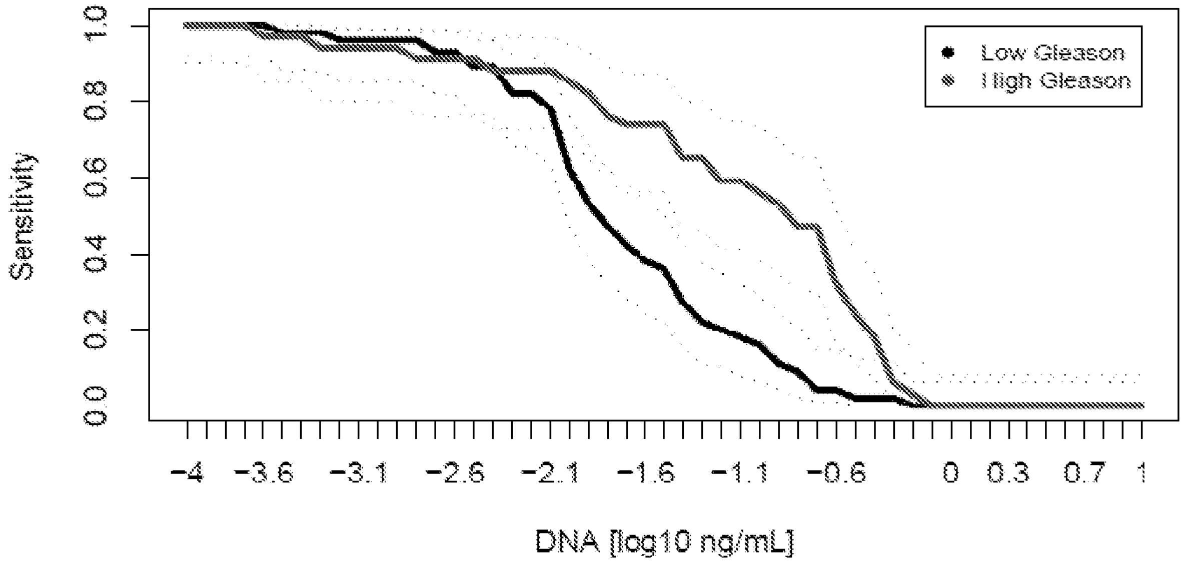


**Figure 122/1**

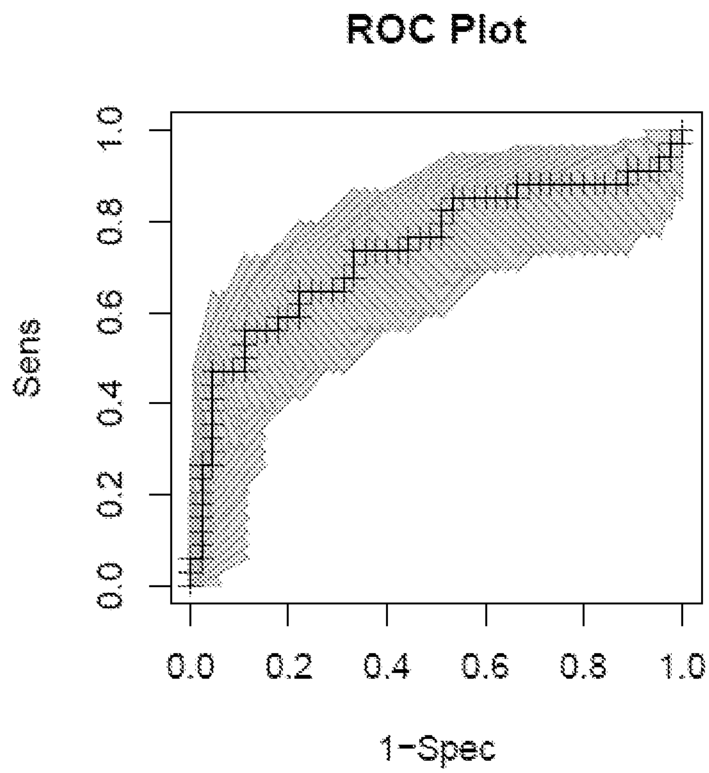


| | |
|---|---|
| AUC | 0.74 [0.62,0.83] |
| Sens / Spec | 0.56 / 0.87 |
| Wilcoxon *P* | 2e-04 |
| High Gleason (pos) | 34 |
| Low Gleason (neg) | 45 |

**Figure 122/2**

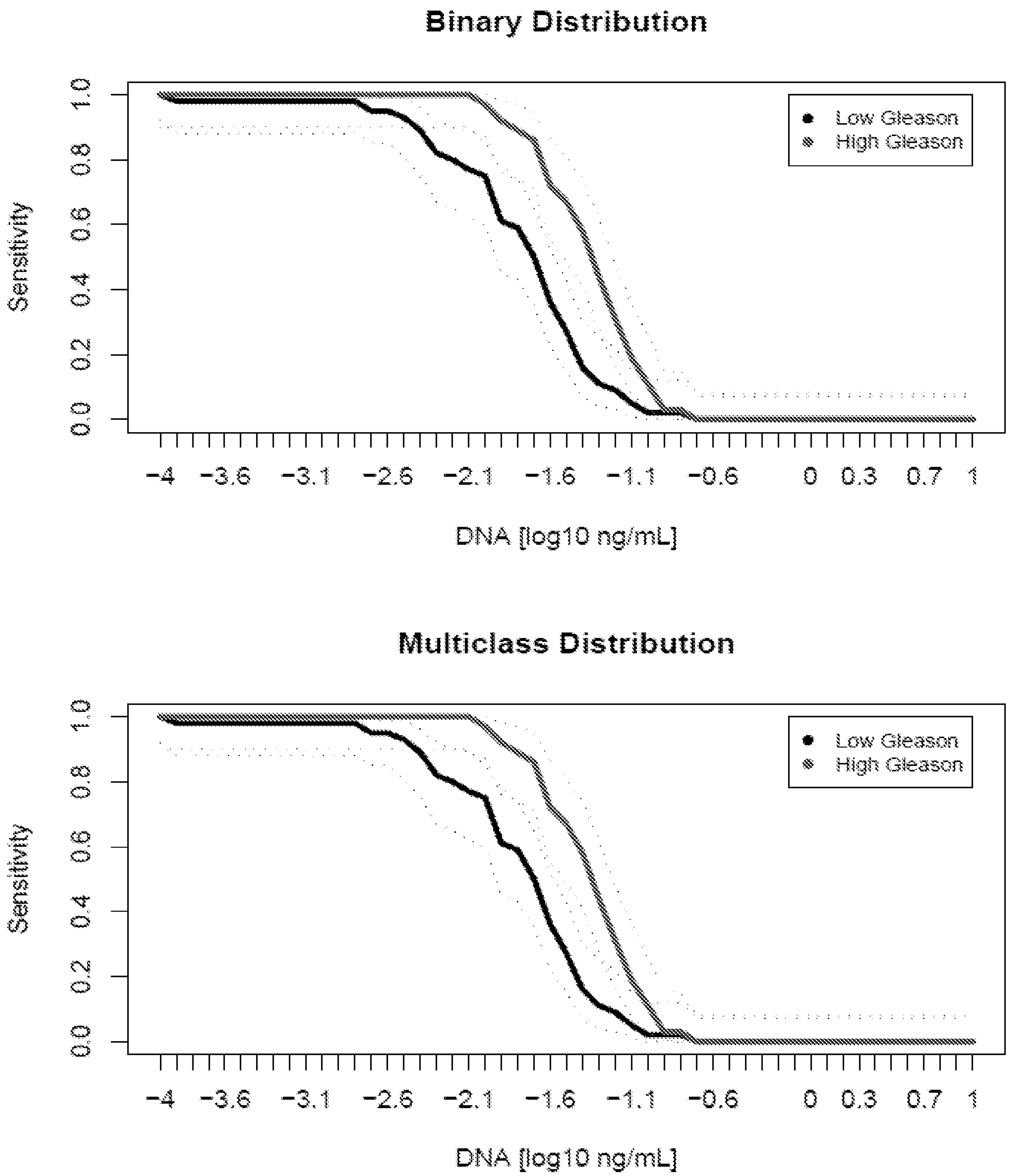
Binary Distribution
Low Gleason
High Gleason
Sensitivity
0.0
0.2
0.4
0.6
0.8
1.0
-4
-3.6
-3.1
-2.6
-2.1
-1.6
-1.1
-0.6
0
0.3
0.7
1
DNA [log10 ng/mL]
Multiclass Distribution
Low Gleason
High Gleason
Sensitivity
0.0
0.2
0.4
0.6
0.8
1.0
-4
-3.6
-3.1
-2.6
-2.1
-1.6
-1.1
-0.6
0
0.3
0.7
1
DNA [log10 ng/mL]

Figure 123/1

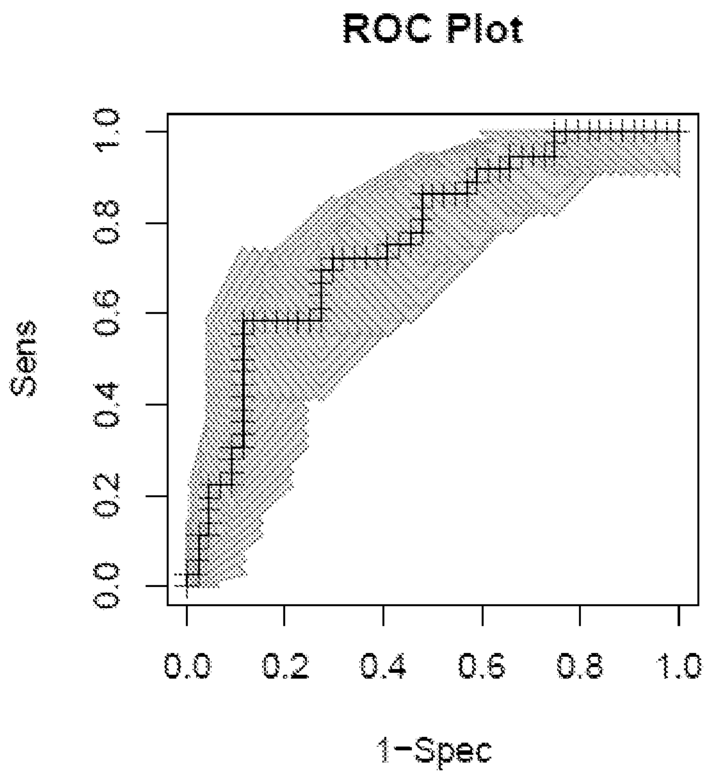


| | |
|---|---|
| AUC | 0.76 [0.65,0.85] |
| Sens / Spec | 0.58 / 0.86 |
| Wilcoxon *P* | 0 |
| High Gleason (pos) | 36 |
| Low Gleason (neg) | 44 |

**Figure 123/2**

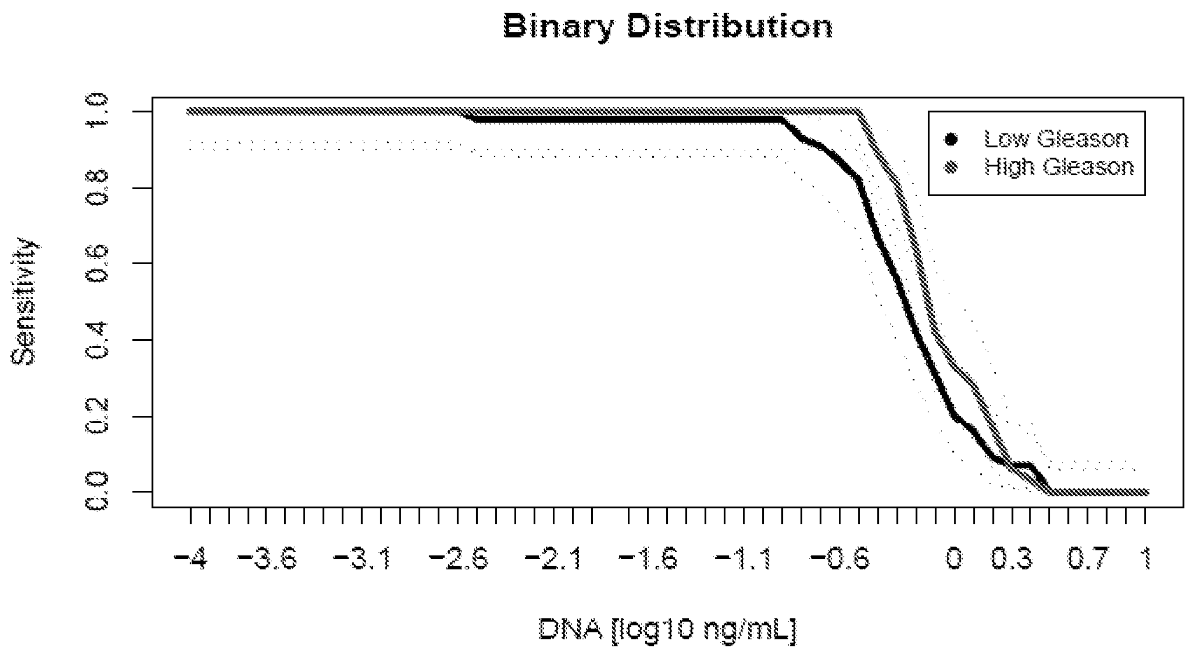
Binary Distribution
Low Gleason
High Gleason
Sensitivity
0.0
0.2
0.4
0.6
0.8
1.0
-4
-3.6
-3.1
-2.6
-2.1
-1.6
-1.1
-0.6
0
0.3
0.7
1
DNA [log10 ng/mL]

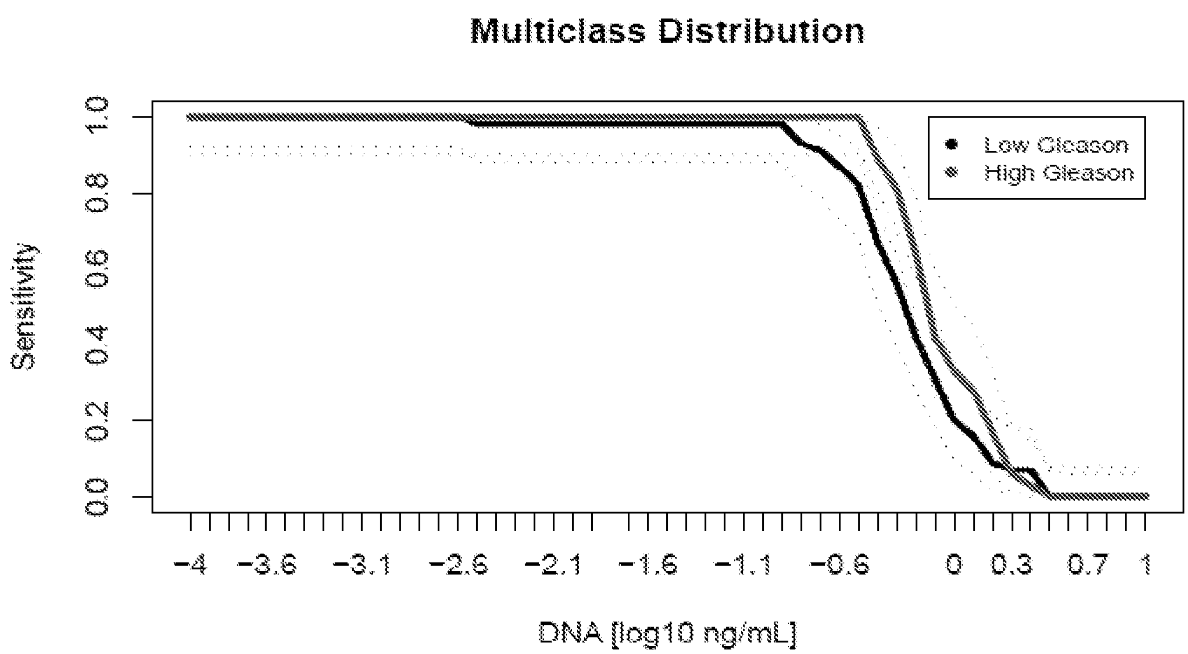
Multiclass Distribution
Low Gleason
High Gleason
Sensitivity
0.0
0.2
0.4
0.6
0.8
1.0
-4
-3.6
-3.1
-2.6
-2.1
-1.6
-1.1
-0.6
0
0.3
0.7
1
DNA [log10 ng/mL]

Figure 124/1

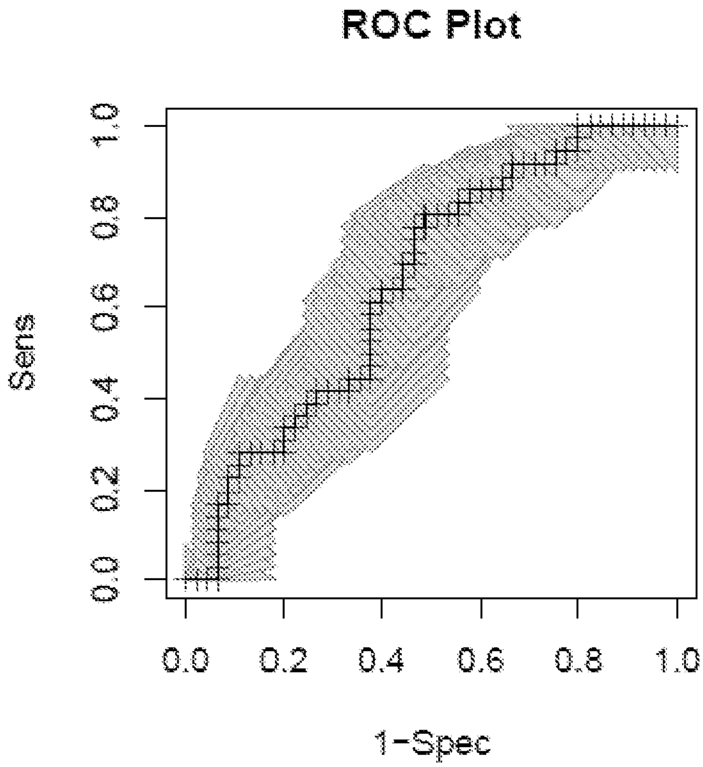


| | |
|---|---|
| AUC | 0.65 [0.54,0.76] |
| Sens / Spec | 0.28 / 0.87 |
| Wilcoxon *P* | 0.0186 |
| High Gleason (pos) | 36 |
| Low Gleason (neg) | 45 |

**Figure 124/2**

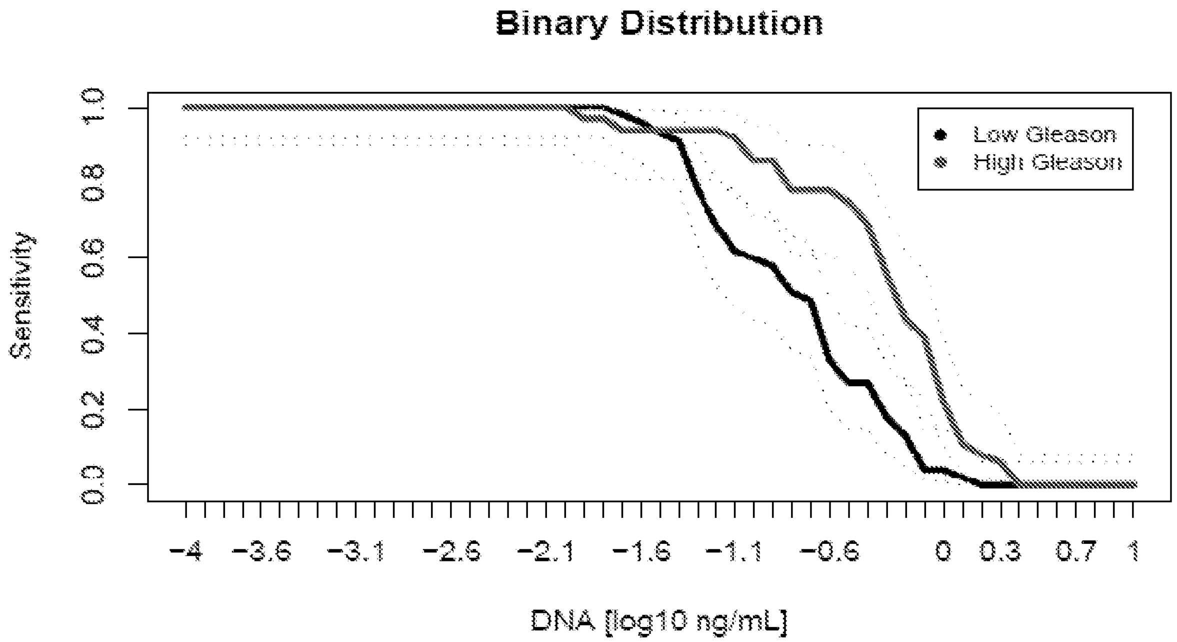
Binary Distribution
Low Gleason
High Gleason
Sensitivity
1.0
0.8
0.6
0.4
0.2
0.0
-4
-3.6
-3.1
-2.6
-2.1
-1.6
-1.1
-0.6
0
0.3
0.7
1
DNA [log10 ng/mL]

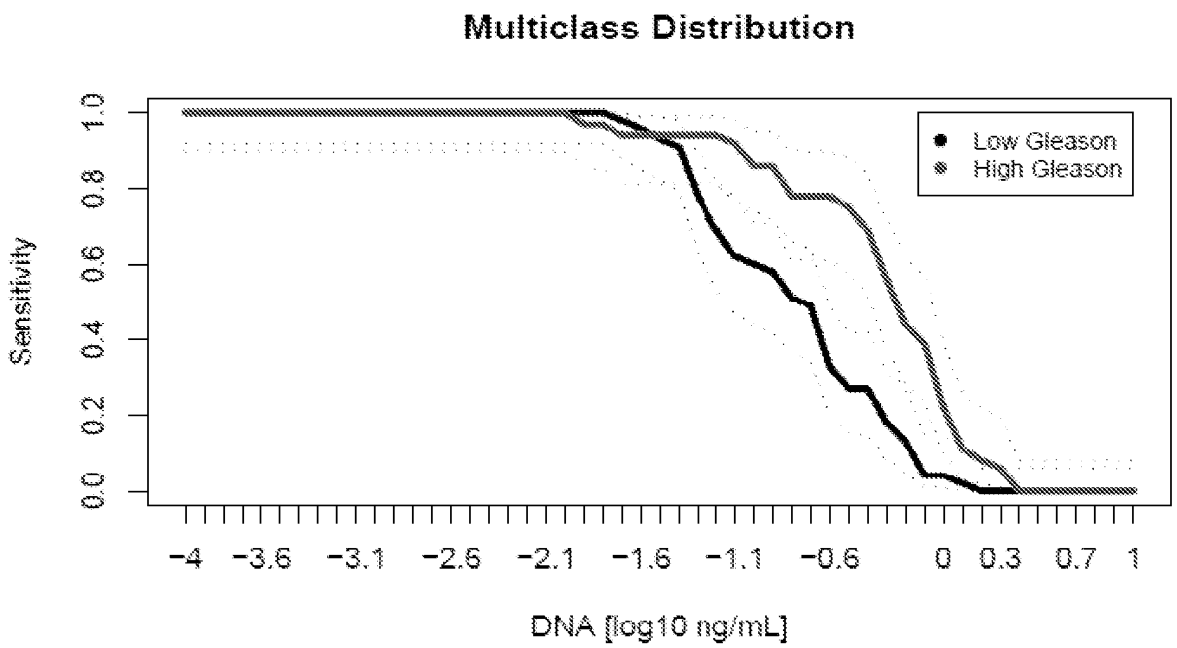
Multiclass Distribution
Low Gleason
High Gleason
Sensitivity
1.0
0.8
0.6
0.4
0.2
0.0
-4
-3.6
-3.1
-2.6
-2.1
-1.6
-1.1
-0.6
0
0.3
0.7
1
DNA [log10 ng/mL]

**Figure 125/1**

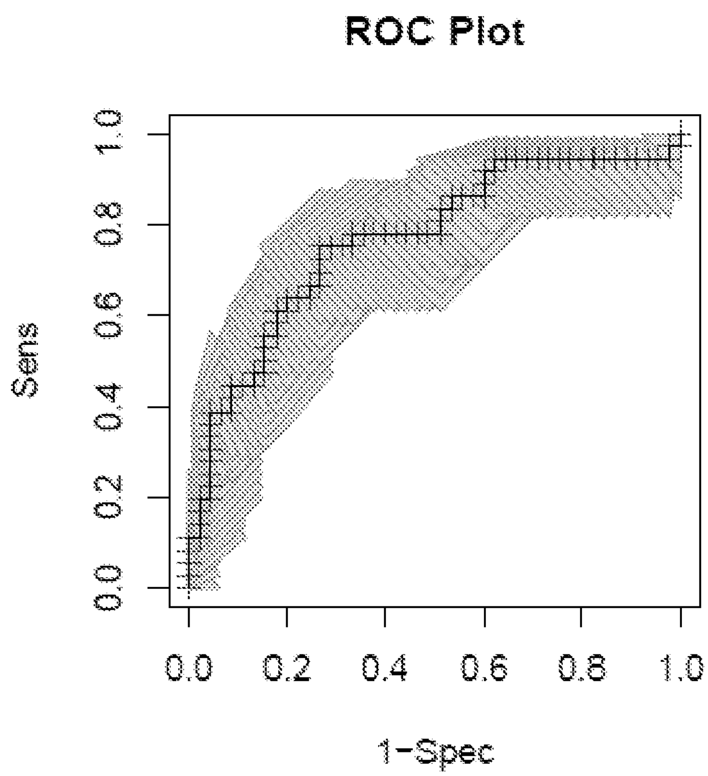


| | |
|---|---|
| AUC | 0.77 [0.66,0.85] |
| Sens / Spec | 0.47 / 0.87 |
| Wilcoxon *P* | 0 |
| High Gleason (pos) | 36 |
| Low Gleason (neg) | 45 |

**Figure 125/2**

Europäisches Patentamt
European Patent Office
Office européen des brevets

# EUROPEAN SEARCH REPORT

Application Number
EP 10 17 7065

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X,P | WO 2005/054517 A2 (EPIGENOMICS AG [US]; DAY KEVIN J [US]; COTTRELL SUSAN [US]; DISTLER JU) 16 June 2005 (2005-06-16)<br>* SEQ ID NO:1057 comprises SEQ ID NO:317claims 38-46 *<br>----- | 6-13 | INV.<br>C12Q1/68 |
| T | WEISS G ET AL: "DNA Methylation of the PITX2 Gene Promoter Region is a Strong Independent Prognostic Marker of Biochemical Recurrence in Patients With Prostate Cancer After Radical Prostatectomy",<br>JOURNAL OF UROLOGY, LIPPINCOTT WILLIAMS & WILKINS, BALTIMORE, MD, US,<br>vol. 181, no. 4, 1 April 2009 (2009-04-01), pages 1678-1685, XP026013957,<br>ISSN: 0022-5347, DOI: DOI:10.1016/J.JURO.2008.11.120<br>[retrieved on 2009-02-23]<br>* the whole document *<br>----- | 1-14 | |

TECHNICAL FIELDS SEARCHED (IPC)

C12Q

The present search report has been drawn up for all claims

| Place of search | Date of completion of the search | Examiner |
|---|---|---|
| The Hague | 6 January 2011 | Gabriels, Jan |

3

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

## ANNEX TO THE EUROPEAN SEARCH REPORT ON EUROPEAN PATENT APPLICATION NO. EP 10 17 7065

This annex lists the patent family members relating to the patent documents cited in the above-mentioned European search report. The members are as contained in the European Patent Office EDP file on
The European Patent Office is in no way liable for these particulars which are merely given for the purpose of information.

06-01-2011

| Patent document cited in search report | Publication date | Patent family member(s) | Publication date |
|---|---|---|---|
| WO 2005054517 A2 | 16-06-2005 | AU 2004295712 A1 | 16-06-2005 |
| | | CA 2548528 A1 | 16-06-2005 |
| | | JP 2007525208 T | 06-09-2007 |
| | | US 2008286761 A1 | 20-11-2008 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 60632426 B
- US 60662220 B
- US 60723125 B
- US 60633250 B
- US 60723054 B
- US 350763 A
- WO 2005059172 A
- US 5786146 A
- WO 0026401 A1
- WO 9500669 A
- US 6251594 B
- WO 9928498 A
- WO 9746705 A
- WO 9515373 A
- US 5514758 A
- US 5565552 A
- US 5567810 A
- US 5574142 A
- US 5585481 A
- US 5587371 A
- US 5597696 A
- US 5958773 A
- US 20030013091 A
- US 09898743 B
- US 6265171 B, Herman
- US 6331393 B


### Non-patent literature cited in the description


- **Lipshutz, R. J. et al.** *Nature Genetics,* 1999, vol. 21, 20-24
- **Bowtell, D. D. L.** *Nature Genetics,* 1999, vol. 21, 25-32
- **Santourlidis, S. et al.** *Prostate,* 1999, vol. 39, 166-74
- *Jpn J Clin Oncol,* 2004, vol. 4, 414-9
- *J Urol,* 2004, vol. 171, 187-91
- **Zhou et al.** *J Urol,* 2004, vol. 171, 2195-8
- **Rabbani et al.** *The FASEB Journal,* 2003, vol. 17, 1081-1088
- **J.P. Egan.** Signal Detection Theory and ROC Analysis. Academic Press, 1975
- **Gonzalgo et al.** *Cancer Research,* 1997, vol. 57, 594-599
- **Eads et al.** *Cancer Res.,* 1999, vol. 59, 2302-2306
- **Gonzalgo ; Jones.** *Nucleic Acids Res.,* 1997, vol. 25, 2529-2531
- **Herman et al.** *Proc. Natl. Acad. Sci. USA,* 1996, vol. 93, 9821-9826
- **Xiong ; Laird.** *Nucleic Acids Res.,* 1997, vol. 25, 2532-2534
- **Toyota et al.** *Cancer Res.,* 1999, vol. 59, 2307-12
- **Sambrook et al.** Molecular Cloning, A Laboratory Manual. Cold Spring Harbor Press, 1989
- Current Protocols in Molecular Biology. John Wiley & Sons, 1995
- **Olek A et al.** A modified and improved method for bisulfite based cytosine methylation analysis. *Nucleic Acids Res.,* 1996, vol. 24, 5064-6
- **Rein, T. et al.** *Nucleic Acids Res.,* 1998, vol. 26, 2255
- **Zeschnigk M et al.** *Eur J Hum Genet.,* 1997, vol. 5, 94-98
- **Olek ; Walter.** *Nat Genet.,* 1997, vol. 17, 275-6
- **Gonzalgo ; Jones.** *Nucleic Acids Res,* 1997, vol. 25, 2529-31
- **Xiong ; Laird.** *Nucleic Acids Res.,* 1997, vol. 25, 2532-4
- **Grigg ; Clark.** *Bioessays,* 1994, vol. 16, 431-6
- **Zeschnigk M et al.** *Hum Mol Genet.,* vol. 6, 387-95
- **Feil R et al.** *Nucleic Acids Res.,* vol. 22, 695, 1994
- **Martin V et al.** *Gene,* 1995, vol. 157, 261-4
- **Frommer et al.** *Proc. Natl. Acad. Sci. USA,* 1992, vol. 89, 1827-1831
- **Sadri ; Hornsby.** *Nucl. Acids Res.,* 1996, vol. 24, 5058-5059
- **Xiong ; Laird.** *Nucleic Acids Res,* 1997, vol. 25, 2532-2534
- **Toyota et al.** *Cancer Res,* 1999, vol. 59, 2307-12
- **Belyavsky et al.** *Nucl Acid Res,* 1989, vol. 17, 2919-2932
- **Krug ; Berger.** Methods in Enzymology. Academic Press, 1987, vol. 152, 316-325
- *Basic and Clinical Immunology,* 1991, 217-262

- **Milstein ; Kohler.** *Nature,* 1975, vol. 256, 495-497
- **Gulfre ; Milstein.** Methods in Enzymology: Immunochemical Techniques. Academic Press, 1981, vol. 73, 1-46
- **Sambrook et al.** Molecular Cloning: A Laboratory Manual. 1989
- Basic and Clinical Immunology. Appleton & Lange, 1991, 217-262
- **Krol et al.** *BioTechniques,* 1988, vol. 6, 958-976
- **Zon.** *Pharm. Res.,* 1988, vol. 5, 539-549
- *Nature Genetics Supplement,* January 1999, vol. 21
- **Yu et al.** *BioTechniques,* 1997, vol. 23, 714-720
- **Karas ; Hillenkamp.** *Anal Chem.,* 1988, vol. 60, 2299-301
- **Gut ; Beck.** *Current Innovations and Future Trends,* 1995, vol. 1, 147-57
- **Gut ; Beck.** *Nucleic Acids Res.,* 1995, vol. 23, 1367-73
- **Heid et al.** *Genome Res.,* 1996, vol. 6, 986-994
- **Sanger F. et al.** *Proc Natl Acad Sci USA,* 1977, vol. 74, 5463-5467
- **Olek et al.** *Nucleic Acids Res.,* 15 December 1996, vol. 24 (24), 5064-6